(19) Europäisches Patentamt
European Patent Office
Office européen des brevets

(11) EP 1 561 821 B1

(12) **EUROPEAN PATENT SPECIFICATION**

(45) Date of publication and mention of the grant of the patent:
**16.02.2011 Bulletin 2011/07**

(51) Int Cl.:
***C12Q 1/68*** *(2006.01)*

(21) Application number: **04029486.0**

(22) Date of filing: **13.12.2004**

(54) **Prognostic markers for prediction of treatment response and/or survival of breast cell proliferative disorder patients**

Marker zur Prognose der Reaktion auf Therapie bzw. der Überlebensrate von Brustkrebspatienten

Marqueurs pour le pronostic de la réponse à la thérapie et/ou de la survie chez les patients du cancer du sein

(84) Designated Contracting States:
**AT BE BG CH CY CZ DE DK EE ES FI FR GB GR HU IE IS IT LI LT LU MC NL PL PT RO SE SI SK TR**

(30) Priority:
**11.12.2003 EP 03090432**
**10.02.2004 EP 04090041**
**01.04.2004 EP 04090127**
**05.06.2004 EP 04013328**
**30.09.2004 EP 04090380**
**16.11.2004 EP 04027213**

(43) Date of publication of application:
**10.08.2005 Bulletin 2005/32**

(73) Proprietor: **Epigenomics AG**
**10178 Berlin (DE)**

(72) Inventors:
- **Foekens, John**
**2908 XA Rotterdam (NL)**
- **Harbeck, Nadia**
**83624 Otterfing (DE)**
- **König, Thomas**
**10999 Berlin (DE)**
- **Maier, Sabine**
**12163 Berlin (DE)**
- **Martens, John**
**3033 BK Rotterdam (NL)**
- **Model, Fabian**
**12683 Berlin (DE)**
- **Nimmrich, Inko**
**10407 Berlin (DE)**
- **Rujan, Tamas**
**13189 Berlin (DE)**
- **Schmitt, Manfred**
**81669 München (DE)**
- **Lesche, Ralf**
**10439 Berlin (DE)**
- **Dietrich, Dimo**
**10711 Berlin (DE)**
- **Müller, Volkmar**
**c/o Department of Gynecology**
**20246 Hamburg (DE)**
- **Kluth, Antje**
**10407 Berlin (DE)**
- **Schwope, Ina**
**10439 Berlin (DE)**
- **Hartmann, Oliver**
**10435 Berlin (DE)**
- **Höfler, Heinz**
**81675 München (DE)**
- **Adorjan, Peter**
**10437 Berlin (DE)**

(74) Representative: **Krauss, Jan**
**Forrester & Boehmert**
**Pettenkoferstrasse 20-22**
**80336 München (DE)**

(56) References cited:
**WO-A-01/19845** **WO-A-2004/035803**

- **TOYOTA MINORU ET AL: "Methylation profiling in acute myeloid leukemia" BLOOD, vol. 97, no. 9, 1 May 2001 (2001-05-01), pages 2823-2829, XP002226261 ISSN: 0006-4971**
- **CARVALHO RALPH ET AL: "Loss of heterozygosity and promoter methylation, but not mutation, may underlie loss of TFF1 in gastric carcinoma" LABORATORY INVESTIGATION, vol. 82, no. 10, October 2002 (2002-10), pages 1319-1326, XP002361843 ISSN: 0023-6837**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**(Cont. next page)**

- DATABASE EMBL [Online] 13 January 1996 (1996-01-13), "yy06g01.s1 Soares melanocyte 2NbHM Homo sapiens cDNA clone IMAGE: 270480 3' similar to gb:D29805 N-ACETYLLACTOSAMINE SYNTHASE (HUMAN);, mRNA sequence." XP002356708 retrieved from EBI accession no. EM_PRO:N33150 Database accession no. N33150
- DATABASE EMBL [Online] 7 October 2000 (2000-10-07), "1M0258D16F Mouse 10kb plasmid UUGC1M library Mus musculus genomic clone UUGC1M0258D16 F, DNA sequence." XP002356711 retrieved from EBI accession no. EM_PRO:AZ455741 Database accession no. AZ455741

## Description

The present invention relates to methods for predicting the survival and/or treatment response of a patient diagnosed with a cell proliferative disorder of the breast tissues, characterized in that the expression level of the gene PITX2 or the genetic or the epigenetic modifications of the genomic DNA associated with the gene PITX2 and/or regulatory or promoter regions thereof are determined. The invention also relates to nucleic acid sequences, oligonucleotides and antibodies which can be used in the described methods.

## Field of the Invention

BREAST CANCER.

In European and American women breast cancer is the most frequently diagnosed cancer and the second leading cause of cancer death. In women aged 40-55, breast cancer is the leading cause of death (Greenlee *et al.,* 2000). In 2002 there were 204,000 new cases of breast cancer in the US, and a comparable number in Europe.

Breast cancer is defined as the uncontrolled proliferation of cells within breast tissues. Breasts are comprised of 15 to 20 lobes joined together by ducts. Cancer arises most commonly in the duct, but is also found in the lobes with the rarest type of cancer termed inflammatory breast cancer. It will be appreciated by those skilled in the art that there exists a continuing need to improve methods of early detection, classification and treatment of breast cancers. In contrast to the detection of some other common cancers such as cervical and dermal there are inherent difficulties in classifying and detecting breast cancers.

BREAST CANCER TREATMENT.

The first step of any treatment is the assessment of the patient's condition comparative to defined classifications of the disease. However the value of such a system is inherently dependent upon the quality of the classification. Breast cancers are staged according to their size, location and occurrence of metastasis. Methods of treatment include the use of surgery, radiation therapy, chemotherapy and endocrine therapy, which are also used as adjuvant therapies to surgery. In general, more aggressive diseases should be treated with more aggressive therapies.

Although the vast majority of early cancers are operable, i.e. the tumor can be completely removed by surgery, about one third of the patients with lymph-node negative diseases and about 50-60% of patients with node-positive disease will develop metastases during follow-up.

Based on this observation, systemic adjuvant treatment has been introduced for both node-positive and node-negative breast cancers. Systemic adjuvant therapy is administered after surgical removal of the tumor, and has been shown to reduce the risk of recurrence significantly. Several types of adjuvant treatment are available: endocrine treatment, also called hormone treatment (for hormone receptor positive tumors), different chemotherapy regimens, and antibody treatments based on novel agents like Herceptin (an antibody to an epidermal growth factor receptor).

The growth of the majority of breast cancers (app. 70-80%) is dependent on the presence of estrogen. Therefore, one important target for adjuvant therapy is the removal of estrogen (e.g. by ovarian ablation) or the blocking of its synthesis or the blocking of its actions on the tumor cells either by blocking the receptor with competing substances (e.g. Tamoxifen) or by inhibiting the conversion of androgen into estrogen (e.g. aromatase inhibitors). This type of treatment is called "endocrine treatment". Endocrine treatment is thought to be efficient only in tumors that express hormone receptors (the estrogen receptor (ER) and/or the progesterone receptor (PR)). Currently, the vast majority of women with hormone receptor positive breast cancer receive some form of endocrine treatment, independent of their nodal status. The most frequently used drug in this scenario is Tamoxifen.

However, even in hormone receptor positive patients, not all patients benefit from endocrine treatment. Adjuvant endocrine therapy reduces mortality rates by 22% while response rates to endocrine treatment in the metastatic (advanced) setting are 50 to 60%.

Since Tamoxifen has relatively few side effects, treatment may be justified even for patients with low likelihood of benefit. However, these patients may require additional, more aggressive adjuvant treatment. Even in earliest and least aggressive tumors, such as node-negative, hormone receptor positive tumors, about 21% of patients relapse within 10 years after initial diagnosis if they receive Tamoxifen monotherapy only, as adjuvant treatment (Lancet. 1998 May 16;351(9114):1451-67. Tamoxifen for early breast cancer: an overview of the randomized trials. Early Breast Cancer Trialists' Collaborative Group.). Similarly, some patients with hormone receptor negative disease may be treated sufficiently with surgery and potentially radiotherapy alone, whereas others may require additional chemotherapy.

Several cytotoxic regimens have shown to be effective in reducing the risk of relapse in breast cancer (Mansour *et al.,* 1998). According to current treatment guidelines, most node-positive patients receive adjuvant chemotherapy both in the US and Europe, since the risk of relapse is considerable. Nevertheless, not all patients do relapse, and there is a proportion of patients who would never have relapsed even without chemotherapy, but who nevertheless receive chemotherapy due to the currently used criteria. In hormone receptor positive patients, chemotherapy is usually given before endocrine treatment, whereas hormone receptor negative patients receive only chemotherapy.

The situation for node-negative patients is particularly complex. In the US, cytotoxic chemotherapy is recommended for node-negative patients, if the tumor is larger than 1 cm. In Europe, chemotherapy is considered for the node-negative cases if one or more risk factors such as tumor size larger than 2 cm, negative hormone receptor status, or tumor grading of three or age <35 is present. In general, there is a tendency to select premenopausal women for additional chemotherapy whereas for postmenopausal women, chemotherapy is often omitted. Compared to endocrine treatment, in particular Tamoxifen or aromatase inhibitors, chemotherapy is highly toxic, with short-term side effects such as nausea, vomiting, bone marrow depression, and long-term effects such as cardiotoxicity and an increased risk for secondary cancers.

LONGFELT NEED IN THE ART.

It is currently not clear which breast cancer patients should be selected for more aggressive therapy and which would do well without additional aggressive treatment, and clinicians agree that there is a large need for proper selection of patients. The difficulty of selecting the right patients for adjuvant treatment and selecting the right adjuvant treatment, and the lack of suitable criteria is also reflected by a recent study which showed that chemotherapy is used much less frequently than recommended, based on data from the New Mexico Tumor registry (Du *et al.,* 2003). This study provided substantial evidence that there is a need for better selection of patients for chemotherapy or other, more aggressive forms of breast cancer therapy.

PRIOR ART OF THE GENE PITX2

PITX2 (also known as PTX2, RS, RGS, ARP1, Brx1, IDG2, IGDS, IHG2, RIEG, IGDS2, IRID2, Otlx2, RIEG1, MGC20144) is known to belong to the PTX subfamily of PTX1, PTX2, and PTX3 genes which define a novel family of transcription factors, within the paired-like class of homeodomain factors. The gene PITX2 (according to NM_153426) encodes the paired-like homeodomain transcription factor 2, which is known to be expressed during development of anterior structures such as the eye, teeth, and anterior pituitary.

Toyota et al., (2001) (Blood 97: p 2823-9.) found hypermethylation of the PITX2 gene in a large proportion of acute myeloid leukemia. Furthermore, in this study hypermethylation of PITX2 is positively correlated to methylation of the ER gene and to a reduced expression level. Means to analyze the methylation pattern of the PITX2 gene have been described in a number of patent applications, too (WO 02/077272 is related to the use of methylation markers to differentiate between AML and ALL, WO 01/19845 is related to several differentially methylated sequences useful for diagnosis of several cell proliferative disorders, WO 02/00927 and WO 01/092565 are related to the use of methylation markers to diagnose diseases associated with development genes or associated with DNA transcription, respectively.

Loss of heterozygosity (hereinafter also referred to as 'LOH')of chromosome 4 is a known characteristic of many tumor types. Shivapurkar et al. [Cancer Research 59, 3576-3580, August 1, 1999] have observed loss of heterozygosity at multiple regions of chromosome 4 in breast cancer samples and cell lines. Deletions at 4q25-26 were present in 67% of analyzed samples. However the analyzed region (between markers D4S1586 and D4S175) does not map to the PITX2 gene, and no inference concerning PITX2 expression was made. Furthermore, the investigation as carried out does not indicate the suitability of any genes or loci of the region for a prognostic use.

Although the methylation of PITX2 has been associated with development, transcription and disease such as cancer, it has no heretofore recognized role in the outcome prediction of breast cancer patients or responsiveness to endocrine treatment.

PRIOR ART IN EXPRESSSION ANALYSIS

The expression of a gene, or rather the protein encoded by the gene, can be studied on four different levels: firstly, protein expression levels can be determined directly, secondly, mRNA transcription levels can be determined, thirdly, epigenetic modifications, such as gene's DNA methylation profile or the gene's histone profile; can be analyzed, as methylation is often correlated with inhibited protein expression, and fourth, the gene itself may be analyzed for genetic modifications such as mutations, deletions, polymorphisms etc. influencing the expression of the gene product.

The levels of observation that have been studied by the methodological developments of recent years in molecular biology, are the genes themselves, the transcription of these genes into RNA, and the translation into the resulting proteins. However how the activation and inhibition of specific genes, in specific cells and tissues, at specific time points in the course of development of an individual are controlled, is correlatable to the degree and character of the methylation of the genes or respectively the genome. In this respect, pathogenic conditions may manifest themselves in a changed methylation pattern of individual genes or of the genome.

The four terms that apply to the fields of overall genome-wide analysis of all these biological processes are called: Proteomics, Transcriptomics, Epigenomics (or Methylomics) and Genomics. Methods and techniques that can be used for studying expression or studying the modifications responsible for expression on all of these levels are well described in the literature and therefore known to a person skilled in the art. They are described in text books of molecular biology and in a large number of scientific journals.

How to analyze the protein expression of a single gene is prior art. It usually requires an antibody specific for the gene product of interest. Appropriate technologies would be ELISA or Immunohistochemistry.

The analysis of the level of mRNA also has been described sufficiently. These days the gold standard is the reverse transcriptase PCR.

To avoid duplication a more detailed description of the prior art relating to existing and well known technologies is given within the description of the invention, as it is part of the invention.

US patent application 2003/0198970 by Gareth Roberts lists some of the technologies and methods on how to determine a person's "genetic make up", i.e. the genetic modifications, such as deletions, polymorphisms, mutations etc. that may vary between individuals and describes the potential role of this genetic sequence information in the individual's variability in disease, response to therapy and prognosis. Epigenetic differences however are not mentioned. The gene PITX2 is listed within this application as one gene name out of a long and comprehensive list of about 2.500 other gene names, suggesting its expression could play a role in some kind of treatment response. However, this is simply an assumption based on speculation only, as no experiments are disclosed, which demonstrate any kind of relation between genetic modifications of PITX2 and an individual's variation in treatment response.

A less established area in this context is the field of epigenomics or epigenetics, i.e. the field concerned with analysis of DNA methylation patterns.

PRIOR ART IN METHYLATION ANALYSIS

5-methylcytosine is the most frequent covalent base modification in the DNA of eukaryotic cells. Methylation of DNA can play an important role in the control of gene expression in mammalian cells. It plays a role, for example, in the regulation of the transcription, in genetic imprinting, and in tumorigenesis. DNA methyltransferases are involved in DNA methylation and catalyze the transfer of a methyl group from S-adenosylmethionine to cytosine residues to form 5-methylcytosine, a modified base that is found mostly at CpG sites in the genome. The presence of methylated CpG islands in the promoter region of genes can suppress their expression. This process may be due to the presence of 5-methylcytosine, which apparently interferes with the binding of transcription factors or other DNA-binding proteins to block transcription. In different types of tumors, aberrant or accidental methylation of CpG islands in the promoter region has been observed for many cancer-related genes, resulting in the silencing of their expression. Such genes include tumor suppressor genes, genes that suppress metastasis and angiogenesis, and genes that repair DNA (Momparler and Bovenzi (2000) J. Cell Physiol. 183:145-54). Therefore, the identification of 5-methylcytosine as a component of genetic information is of considerable interest. However, 5-methylcytosine positions cannot be identified by sequencing since 5-methylcytosine has the same base pairing behaviour as cytosine. Moreover, the epigenetic information carried by 5-methylcytosine is completely lost during PCR amplification.

METHYLATION ANALYSIS TECHNIQUES

Recently published was a study related to the prognostic power of methylation analysis in breast cancer patients. Müller et al. (Muller HM, Widschwendter A, Fiegl H, Ivarsson L, Goebel G, Perkmann E, Marth C, Widschwendter M. (2003) DNA methylation in serum of breast cancer patients: an independent prognostic marker. Cancer Res. 2003 Nov 15; 63(22): 7641-5.) described a set of genes, which can be used as prognostic biomarkers in breast cancer patients by analysis of pre-therapeutic sera. Specific aberrant methylation patterns of two genes found in DNA from pre-treatment serum of cancer patients indicated whether their prognosis was good or bad. The DNA analyzed was not tissue derived DNA but serum DNA. Most likely the presence of a tumor-specific pattern indicates that tumor derived DNA is present, however, the absence of a specific methylation pattern may be due to a tumor which does not show this methylation pattern, or a tumor which does not shed sufficient DNA into the blood stream. Good or bad prognosis was defined as long or short "overall survival" after surgery without adjuvant treatment. This result therefore relates to patients who do not receive a post surgical treatment. The markers are therefore (unless proven otherwise) considered to be purely prognostic. The markers provide no information concerning treatment response and can provide only a very basic guide as to the aggressiveness of the tumor. On this basis clinicians can only speculate on the suitability of treatment options. As it is however standard to provide Tamoxifen (or other endocrine therapies) as an adjuvant treatment to the majority of patients irrespectively of the aggressiveness of the tumor these markers are not applicable to most patients.

Therefore there is still a long felt need in the art for the improved treatment of breast cancer patients that are not fulfilled by the current state of the art:
More specifically, none of these markers is able to answer the specific problem as outlined above, namely whether a patient treated by means of a primary treatment (in most cases surgery) is a suitable candidate for treatment using only an endocrine treatment, for example but not limited to Tamoxifen, or aromatase inhibitors) or if said patient would have a better prognosis if treated with a further adjuvant treatment (for example but not limited to chemotherapy) instead of or in addition to said endocrine treatment.

A purely prognostic marker for cancer patients which is irrespective of treatment, is not the preferred solution for the need in the art as described above. Although said markers provide some indication of the aggressiveness of the tumor and therefore may guide the selection of treatment that may be required they do not take into account the heterogeneity of cancers with respect to treatment response. Therefore a patient with poor prognosis (determined using said purely prognostic markers) may respond well to adjuvant treatment with endocrine treatment, irrespective of the aggressiveness of the disease, however if a patient is a poor responder to said treatment an alternative and/or additional treatment will be suitable for treatment even if said patient has a good prognosis.

In one aspect the present invention provides a prognostic marker, PITX2 (which shall be recognized as the gene encoding for the protein PITX2; the mRNA transcript thereof being NM_153426), which however is not 'purely prognostic'. This marker provides a solution to the need in the art as outlined above, by providing guiding information on the question whether or not an adjuvant chemotoxic therapy shall be subscribed in addition to treatment with endocrines, like tamoxifen, or whether this is an unnecessary burden to the patient.

It is herein disclosed that aberrant expression of the gene PITX2 is correlated to prognosis and/or predicted outcome of treatment with estrogen treatment of breast cell proliferative disorder patients, in particular breast carcinoma.

This marker thereby provides a novel means for the characterization of breast carcinomas. Aberrant expression of the gene PITX2 is indicative of the relapse and/or survival of a breast carcinoma patient. The herein described invention is thereby particularly useful for making improved treatment decisions.

This invention also relates to the use of PITX2, as an 'prognostic marker', especially in hormone receptor negative women, which would not get any endocrine treatment at all.

**DESCRIPTION**

Characterization of a breast cancer in terms of prognosis and/or treatment outcome enables the physician to make an informed decision as to a therapeutic regimen with appropriate risk and benefit trade off's to the patient.

In the context of the present invention the terms "estrogen receptor positive" and/or "progesterone receptor positive" when used to describe a breast cell proliferative disorder are taken to mean that the proliferating cells express said hormone receptor.

In the context of the present invention the term 'aggressiveness' is taken to mean one or more of high likelihood of relapse post surgery; below average or below median patient survival; below average or below median disease free survival; below average or below median relapse-free survival; above average tumor-related complications; fast progression of tumor or metastases. According to the aggressiveness of the disease an appropriate treatment or treatments may be selected from the group consisting of chemotherapy, radiotherapy, surgery, biological therapy, immunotherapy, antibody treatments, treatments involving molecularly targeted drugs, estrogen receptor modulator treatments, estrogen receptor down-regulator treatments, aromatase inhibitors treatments, ovarian ablation, treatments providing LHRH analogues or other centrally acting drugs influencing estrogen production. Wherein a cancer is characterized as 'aggressive' it is particularly preferred that a treatment such as, but not limited to, chemotherapy is provided in addition to or instead of an endocrine targeting therapy. Indicators of tumor aggressiveness standard in the art include but are not limited to, tumor stage, tumor grade, nodal status and survival.

Unless stated otherwise as used herein the term "survival" shall be taken to include all of the following: survival until mortality, also known as overall survival (wherein said mortality may be either irrespective of cause or breast tumor related); "recurrence-free survival" (wherein the term recurrence shall include both localized and distant recurrence) ; metastasis free survival; disease free survival (wherein the term disease shall include breast cancer and diseases associated therewith). The length of said survival may be calculated by reference to a defined start point (e.g. time of diagnosis or start of treatment) and end point (e.g. death, recurrence or metastasis).

As used herein the term "prognostic marker" shall be taken to mean an indicator of the likelihood of progression of the disease, in particular aggressiveness and metastatic potential of a breast tumor.

As used herein the term 'predictive marker' shall be taken to mean an indicator of response to therapy, said response is preferably defined according to patient survival. It is preferably used to define patients with high, low and intermediate length of survival or recurrence after treatment, that is the result of the inherent heterogeneity of the disease process.

As defined herein the term predictive marker may in some situations fall within the remit of a herein described 'prognostic marker', for example, wherein a prognostic marker differentiates between patients with different survival outcomes pursuant to a treatment, said marker is also a predictive marker for said treatment. Therefore, unless otherwise stated the two terms shall not be taken to be mutually exclusive.

As used herein the term 'expression' shall be taken to mean the transcription and translation of a gene, as well as the genetic or the epigenetic modifications of the genomic DNA associated with the marker gene and/or regulatory or promoter regions thereof. Genetic modifications include SNPs, point mutations, deletions, insertions, repeat length, rearrangements and other polymorphisms. The analysis of either the expression levels of protein, or mRNA or the analysis of the patient's individual genetic or epigenetic modification of the marker gene are herein summarized as the analysis of 'expression of the gene.

The level of expression of a gene may be determined by the analysis of any factors associated with or indicative of the level of transcription and translation of a gene including but not limited to methylation analysis, loss of heterozygosity (hereinafter also referred to as LOH), RNA expression levels and protein expression levels.

Furthermore the activity of the transcribed gene may be affected by genetic variations such as but not limited to genetic modifications (including but not limited to SNPs, point mutations, deletions, insertions, repeat length, rearrangements and other polymorphisms).

The terms "endocrine therapy" or "endocrine treatment" are meant to comprise any therapy, treatment or treatments targeting the estrogen receptor pathway or estrogen synthesis pathway or estrogen conversion pathway, which is involved in estrogen metabolism, production or secretion. Said treatments include, but are not limited to estrogen receptor modulators, estrogen receptor down-regulators, aromatase inhibitors, ovarian ablation, LHRH analogues and other centrally acting drugs influencing estrogen production.

The term "monotherapy" shall be taken to mean the use of a single drug or other therapy.

In the context of the present invention the term "chemotherapy" is taken to mean the use of pharmaceutical or chemical substances to treat cancer. This definition excludes radiation therapy (treatment with high energy rays or particles), hormone therapy (treatment with hormones or hormone analogues) and surgical treatment.

The term "adjuvant treatment" is taken to mean a therapy of a cancer patient immediately following an initial non chemotherapeutical therapy, e.g. surgery. In general, the purpose of an adjuvant therapy is to decrease the risk of recurrence.

In the context of the present invention the term "determining a suitable treatment regimen for the subject" is taken to mean the determination of a treatment regimen (i.e. a single therapy or a combination of different therapies that are used for the prevention and/or treatment of the cancer in the patient) for a patient that is started, modified and/or ended based or essentially based or at least partially based on the results of the analysis according to the present invention. One example is starting an adjuvant endocrine therapy after surgery, another would be to modify the dosage of a particular chemotherapy. The determination can, in addition to the results of the analysis according to the present invention, be based on personal characteristics of the subject to be treated. In most cases, the actual determination of the suitable treatment regimen for the subject will be performed by the attending physician or doctor.

In the context of this invention the terms "obtaining a biological sample" or "obtaining a sample from a subject", shall not be taken to include the active retrieval of a sample from an individual, e.g. the performance of a biopsy. Said terms shall be taken to mean the obtainment of a sample previously isolated from an individual. Said samples may be isolated by any means standard in the art, including but not limited to biopsy, surgical removal, body fluids isolated by means of aspiration. Furthermore said samples may be provided by third parties including but not limited to clinicians, couriers, commercial sample providers and sample collections.

In the context of the present invention, the term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb in length.

In the context of the present invention the term "regulatory region" of a gene is taken to mean nucleotide sequences which affect the expression of a gene. Said regulatory regions may be located within, proximal or distal to said gene. Said regulatory regions include but are not limited to constitutive promoters, tissue-specific promoters, developmental-specific promoters, inducible promoters and the like. Promoter regulatory elements may also include certain enhancer sequence elements that control transcriptional or translational efficiency of the gene.

In the context of the present invention, the term "methylation" refers to the presence or absence of 5-methylcytosine ("5-mCyt") at one or a plurality of CpG dinucleotides within a DNA sequence.

In the context of the present invention the term "methylation state" is taken to mean the degree of methylation present in a nucleic acid of interest, this may be expressed in absolute or relative terms i.e. as a percentage or other numerical value or by comparison to another tissue and therein described as hypermethylated, hypomethylated or as having significantly similar or identical methylation status.

In the context of the present invention, the term "hemi-methylation" or "hemimethylation" refers to the methylation state of a CpG methylation site, where only a single cytosine in one of the two CpG dinucleotide sequences of the double stranded CpG methylation site is methylated (*e.g.*, 5'-NNC$^{M}$GNN-3' (top strand): 3'-NNGCNN-5' (bottom strand)).

In the context of the present invention, the term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

In the context of the present invention, the term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

In the context of the present invention, the term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as genetic modifications or mutations are, in particular, insertions, deletions, point mutations, inversions and polymorphisms and, particularly preferred, SNPs (single nucleotide polymorphisms).

"Epigenetic modifications" or "epigenetic parameters" are modifications of DNA bases of genomic DNA and sequences further required for their regulation, in particular, cytosine methylations thereof. Further epigenetic parameters include, for example, the acetylation of histones which, however, cannot be directly analyzed using the described method but which, in turn, correlate with the DNA methylation.

In the context of the present invention, the term "bisulfite reagent" refers to a reagent comprising bisulfite, disulfite, hydrogen sulfite or combinations thereof, useful as disclosed herein to distinguish between methylated and unmethylated CpG dinucleotide sequences.

In the context of the present invention, the term "Methylation assay" refers to any assay for determining the methylation state of one or more CpG dinucleotide sequences within a sequence of DNA.

In the context of the present invention, the term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

In the context of the present invention, the term "MethyLight" refers to the art-recognized fluorescence-based real-time PCR technique described by Eads et al., Cancer Res. 59:2302-2306, 1999.

In the context of the present invention, the term "HeavyMethyl™" assay, in the embodiment thereof implemented herein, refers to a methylation assay comprising methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

In the context of the present invention the term "MSP" (Methylation-specific PCR) refers to the art-recognized methylation assay described by Herman et al. Proc. Natl. Acad. Sci. USA 93:9821-9826, 1996, and by US Patent No. 5,786,146.

In the context of the present invention the term "COBRA" (Combined Bisulfite Restriction Analysis) refers to the art-recognized methylation assay described by Xiong & Laird, Nucleic Acids Res. 25:2532-2534, 1997.

In the context of the present invention the term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

"Background DNA" as used herein refers to any nucleic acids which originate from sources other than breast cells.

Using the methods and nucleic acids described herein, statistically significant models of patient relapse, disease free survival, metastasis free survival, overall survival and/or disease progression can be developed and utilized to assist patients and clinicians in determining suitable treatment options to be included in the therapeutic regimen.

In one aspect the method provides a prognostic marker for a cell proliferative disorder of the breast tissues. Preferably this prognosis is provided in terms of an outcome selected from the group consisting of likelihood of relapse;

overall patient survival; metastasis free survival; disease free survival or disease progression.

Using the methods and nucleic acids as described herein, patient survival can be evaluated before or during treatment for a cell proliferative disorder of the breast tissues, in order to provide critical information to the patient and clinician as to the likely progression of the disease. It will be appreciated, therefore, that the methods and nucleic acids exemplified herein can serve to improve a patient's quality of life and odds of treatment success by allowing both patient and clinician a more accurate assessment of the patient's treatment options.

The herein disclosed method may be used for the improved treatment of all breast cell proliferative disorder patients, both pre- and post- menopausal and independent of their node or estrogen receptor status. However, it is particularly preferred that said patients are node-negative and cells from breast tissue of said cell proliferative disorder are estrogen receptor positive.

The method according to the invention may be used for the analysis of a wide variety of cell proliferative disorders of the breast tissues including, but not limited to, ductal carcinoma *in situ,* invasive ductal carcinoma, invasive lobular carcinoma, lobular carcinoma *in situ,* come-docarcinoma, inflammatory carcinoma, mucinous carcinoma, scirrhous carcinoma, colloid carcinoma, tubular carcinoma, medullary carcinoma, metaplastic carcinoma, and papillary carcinoma and papillary carcinoma *in situ,* undifferentiated or anaplastic carcinoma and Paget's disease of the breast.

The method according to the invention may be used to provide a prognosis of breast cell proliferative disorder patients.

Wherein the herein disclosed markers, methods and nucleic acids are used as prognostic markers it is particularly preferred that said prognosis is defined in terms of patient survival and/or relapse. In this embodiment patients survival times and/or relapse are predicted according to their gene expression or genetic or epigenetic modifications thereof. In this aspect of the invention it is particularly preferred that said patients are tested prior to receiving any adjuvant endocrine treatment.

The herein described invention provides a novel breast cell proliferative disorder prognostic biomarker.

It is herein described that aberrant expression of the gene PITX2 and/or regulatory or promoter regions thereof is correlated to prognosis, in particular breast carcinoma.

This marker thereby provides a novel means for the characterization of breast cell proliferative disorders. As described herein determination of the expression of the gene PITX2 and/or regulatory or promoter regions thereof enables the prediction of prognosis of a patient with a proliferative disorder of the breast tissues. In an alternative embodiment the expression of the gene PITX2 and/or regulatory or promoter regions thereof enables the prediction of treatment response of a patient treated with one or more treatments which target the estrogen receptor, synthesis or conversion pathways or are otherwise involved in estrogen metabolism, production or secretion.

The herein described invention is thereby useful for the differentiation of individuals who may be appropriately treated with one or more treatments which target the estrogen receptor pathway or are involved in estrogen metabolism, production or secretion from those individuals, who would be optimally treated with other treatments in addition to said treatment. Preferred 'other treatments' include but are not limited to chemotherapy or radiotherapy. It is particularly preferred that said prognosis and/or treatment response is stated in terms of likelihood of relapse, survival or outcome.

In a further embodiment of the invention the aberrant expression of a plurality of genes comprising the gene PITX2 and/or regulatory or promoter regions thereof is analyzed. Said plurality of genes is hereinafter also referred to as a 'gene panel'. The analysis of multiple genes increases the accuracy of a provided prognosis.

It is preferred that the gene panel consists of up to seven genes and/or their promoter regions associated with prognosis and/or prediction of treatment response of breast carcinoma patients. It is further preferred that said panel consists of the gene PITX2 and one or more genes selected from the group consisting of PLAU and TFF1 and/or regulatory regions thereof. It is particularly preferred that the gene panel is selected from the group of gene panels consisting of:

- PITX2, PLAU & TFF1
- PITX2 & PLAU
- PITX2 & TFF1

It is particularly preferred that the gene panel consisting PITX2 & TFF1 is used to predict outcome of treatment of patients with an endocrine treatment. It is particularly preferred that the gene panel consisting PITX2 & PLAU is used to provide a prognosis of patients. It is preferred that said patients are analyzed prior to receiving any endocrine treatment.

In further embodiments this invention relates to new methods and sequences for the prognosis of patients diagnosed with breast cell proliferative disease. In a further aspect the invention relates to new methods and sequences, which may be used as tools for the selection of suitable treatments of patients diagnosed with breast cell proliferative disease based on a prediction of likelihood of relapse, survival or outcome.

One aspect of the invention is the provision of methods for providing a prognosis of a patient with a cell proliferative disorder of the breast tissues. Preferably said prognosis is provided in terms of likelihood of relapse or the survival of said patient. It is further preferred that said survival is disease free survival or metastasis free survival. It is also preferred that said disease is breast cancer. These methods comprise the analysis of the expression levels of the gene PITX2 and/or regulatory regions thereof.

In further embodiments the method comprises analysis of the expression of a 'gene panel' comprising the gene PITX2 and one or more genes selected from the group consisting of PLAU and TFF1 and/or regulatory regions thereof. It is particularly preferred that said gene panels are selected from the group of gene panels consisting of:

- PITX2, PLAU & TFF1
- PITX2 & PLAU
- PITX2 & TFF1

It is particularly preferred that the expression of the gene panel consisting PITX2 & TFF1 is determined in order to predict outcome of treatment of patients with an endocrine treatment. It is also particularly preferred that the expression of the gene panel consisting PITX2 & PLAU is determined in order to provide a prognosis of patients. It is preferred that said patients are analyzed prior to receiving any endocrine treatment.

Determination of expression may be achieved by any means standard in the art, however it is most preferably achieved by analysis of LOH, methylation, protein expression, mRNA expression, genetic or other epigenetic modifications of the genomic sequences.

Especially preferred is the analysis of the DNA methylation profile of the genomic sequence of the gene PITX2 and/or regulatory or promoter regions thereof as given in SEQ ID NO: 149. Further preferred is the analysis of the methylation status of CpG positions within the following sections of SEQ ID NO: 149 nucleotide 2,700-nucleotide 3,000; nucleotide 3,900-nucleotide 4,200; nucleotide 5,500-nucleotide 8,000; nucleotide 13,500-nucleotide 14,500; nucleotide 16,500-nucleotide 18,000; nucleotide 18,500-nucleotide 19,000; nucleotide 21,000-nucleotide 22,500. Especially preferred is the analysis of the methylation status of eight specific CpG dinucleotides, covered in the four sub-sequences of said SEQ ID NO: 149 given in SEQ ID NOs: 1, 13, 18 and 19. Wherein the method comprises analysis of a gene panel comprising the PITX2 and one or more genes selected from the group consisting PLAU and TFF1 and/or regulatory or promoter regions thereof it is preferred that the sequence of said genes is selected from the group consisting of SEQ ID NO: 73, SEQ ID NO: 75 and SEQ ID NO: 150 according to Table 1.

This methodology presents further improvements over the state of the art in that the method may be applied to any subject, independent of the estrogen and/or progesterone receptor status. Therefore in a preferred embodiment, the subject is not required to have been tested for estrogen or progesterone receptor status.

In further aspects of the invention, the disclosed matter provides novel nucleic acid sequences useful for the analysis of methylation within said gene, other aspects provide novel uses of the gene and the gene product as well as methods, assays and kits directed to providing a prognosis of a patient diagnosed with breast cell proliferative disease.

In one embodiment the invention discloses a method for providing the prognosis of a patient suffering from a breast cell proliferative disease, by analysis of expression of the gene PITX2 and/or regulatory regions thereof. Preferably said endocrine treatment is an adjuvant endocrine monotherapy. Said method may be enabled by means of any analysis of the expression of the gene, including but not limited to mRNA expression analysis or protein expression analysis or by analysis of its genetic modifications leading to an altered expression (including LOH). However, in the most preferred embodiment of the invention, said expression is determined by means of analysis of the methylation status of CpG sites within the gene PITX2 and its promoter or regulatory elements.

In one embodiment of the method aberrant expression of the gene PITX2 and/or panels thereof may be detected by analysis of loss of heterozygosity of the gene. In a first step genomic DNA is isolated from a biological sample of the patient's tumor. The isolated DNA is then analyzed for LOH by any means standard in the art including but not limited to amplification of the gene locus or associated microsatellite markers. Said amplification may be carried out by any means standard in the art including polymerase chain reaction (PCR), strand displacement amplification (SDA)and isothermal amplification.

The level of amplificate is then detected by any means known in the art including but not limited to gel electrophoresis and detection by probes (including Real Time PCR). Furthermore the amplificates may be labeled in order to aid said detection. Suitable detectable labels include but are not limited to fluorescence label, radioactive labels and mass labels the suitable use of which shall be described herein.

The detection of a decreased amount of an amplificate corresponding to one of the amplified alleles in a test sample as relative to that of a heterozygous control sample is indicative of LOH.

To detect the levels of mRNA encoding PITX2 and/or panels comprising said gene in a detection system for breast cancer relapse, a sample is obtained from a patient. Said obtaining of a sample is preferably not meant to be retrieving of a sample, as in performing a biopsy, but rather directed to the availability of an isolated biological material representing a specific tissue, relevant for the intended use. The sample can be a tumor tissue sample from the surgically removed tumor, a biopsy sample as taken by a surgeon and provided to the analyst or a sample of blood, plasma, serum or the like. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridization is determined by a number of factors during hybridization and during the washing procedure, including temperature, ionic strength, length of time and concentration of formamide. These factors are outlined in, for example, Sambrook et al. (Molecular Cloning: A Laboratory Manual, 2nd ed., 1989). Detection of the resulting duplex is usually accomplished by the use of labeled probes. Alternatively, the probe may be unlabeled, but may be detectable by specific binding with a ligand which is labeled, either directly or indirectly. Suitable labels and methods for labeling probes and ligands are known in the art, and include, for example, radioactive labels which may be incorporated by known methods (e.g., nick translation or kinasing), biotin, fluorescent groups, chemiluminescent groups (e.g., dioxetanes, particularly triggered dioxetanes), enzymes, antibodies, and the like.

In order to increase the sensitivity of the detection in a sample of mRNA encoding PITX2 and/or panels comprising said gene, the technique of reverse transcription/polymerization chain reaction can be used to amplify cDNA transcribed from mRNA encoding PITX2 and/or panels comprising said gene. The method of reverse transcription/PCR is well known in the art (for example, see Watson and Fleming, supra).

The reverse transcription/PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and PITX2 and/or panels comprising said gene specific primers. (Belyavsky et al, Nucl Acid Res 17:2919-2932, 1989; Krug and Berger, Methods in Enzymology, Academic Press,N.Y., Vol.152, pp. 316-325, 1987 which are incorporated by reference)

Disclosed here is a kit for use in predicting the likelihood of relapse and/or survival of a breast cancer patient before or after surgical tumor removal with or without adjuvant endocrine monotherapy state through testing of a biological sample. A representative kit may comprise one or more nucleic acid segments as described above that selectively hybridize to PITX2 mRNA and/or mRNA from genes of a panel comprising said PITX2 gene, and a container for each of the one or more nucleic acid segments. In certain cases the nucleic acid segments may be combined in a single tube. In further-examples, the nucleic acid segments may also include a pair of primers for amplifying the target mRNA. Such kits may also include any buffers, solutions, solvents, enzymes, nucleotides, or other components for hybridization, amplification or detection reactions. Preferred kit components include reagents for reverse transcription-PCR, in situ hybridization, Northern analysis and/or RPA.

The present invention further provides for methods to detect the presence of the polypeptide(s) of, PITX2 and/or panels comprising said protein, in a sample obtained from a patient. It is preferred that said sequence is essentially the same as the sequence as given in figure 10. Any method known in the art for detecting proteins can be used. Such methods include, but are not limited to immunodiffusion, immunoelectrophoresis, immunochemical methods, binder-ligand assays, immunohistochemical techniques, agglutination and complement assays. (for example see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes of PITX2 and/or panels thereof and competitively displacing a labeled PITX2 protein and/or panels thereof or derivatives thereof.

Certain embodiments of the present invention comprise the use of antibodies specific to the polypeptide encoded by the gene PITX2 and/or panels comprising said gene. Such antibodies may be useful for providing a prognosis of the likelihood of relapse and/or survival of a breast cancer patient preferably under adjuvant endocrine monotherapy by comparing a patient's levels of PITX2 marker expression and/or the expression of panels comprising PITX2 to expression of the same marker(s) in normal individuals. In certain embodiments the production of monoclonal or polyclonal antibodies can be induced by the use of the PITX2 and/or other polypeptides of the panels as antigene. Such antibodies may in turn be used to detect expressed proteins as markers for prognosis of relapse of a breast cancer patient under adjuvant endocrine monotherapy. The levels of such proteins present in the peripheral blood of a patient may be quantified by conventional methods. Antibody-protein binding may be detected and quantified by a variety of means known in the art, such as labeling with fluorescent or radioactive ligands. The invention further comprises kits for performing the above-mentioned procedures, wherein such kits contain antibodies specific for the PITX2 and/or panels thereof polypeptides.

Numerous competitive and non-competitive protein binding immunoassays are well known in the art. Antibodies employed in such assays may be unlabeled, for example as used in agglutination tests, or labeled for use a wide variety of assay methods. Labels that can be used include radionuclides, enzymes, fluorescers, chemiluminescers, enzyme substrates or co-factors, enzyme inhibitors, particles, dyes and the like for use in radioimmunoassay (RIA), enzyme immunoassays, e.g., enzyme-linked immunosorbent assay (ELISA), fluorescent immunoassays and the like. Polyclonal or monoclonal antibodies to PITX2 and/or panels thereof or an epitope thereof can be made for use in immunoassays by any of a number of methods known in the art. One approach for preparing antibodies to a protein is the selection and preparation of an amino acid sequence of all or part of the protein, chemically synthesising the sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of PITX2 and/or panels thereof or an epitope thereof include, but are not limited to chemical synthesis, recombinant DNA techniques or isolation from biological samples.

In the most preferred embodiment of the invention the analysis of expression is carried out by means of methylation analysis. It is further preferred that the methylation state of the CpG dinucleotides within the genomic sequence according to SEQ ID NO: 149 and sequences complementary thereto is analyzed. SEQ ID NO: 149 discloses the gene PITX2 and its promoter and regulatory elements thereof, wherein said fragment comprises CpG dinucleotides exhibiting a prognosis and/or predicting outcome of endocrine treatment specific methylation pattern. Further preferred is the analysis of the methylation status of CpG positions within the following sections of SEQ ID NO: 149 nucleotide 2,700-nucleotide 3,000; nucleotide 3,900-nucleotide 4,200; nucleotide 5,500-nucleotide 8,000; nucleotide 13,500-nucleotide 14,500; nucleotide 16,500-nucleotide 18,000; nucleotide 18,500-nucleotide 19,000; nucleotide 21,000-nucleotide 22,500. Also preferred is the analysis of the sub-sequence of the gene PITX2 as shown in SEQ ID NO: 1.

Wherein the method comprises analysis of the expression of a 'gene panel' comprising the gene and/or regulatory or promoter regions thereof and one or more genes selected from the group consisting PLAU and TFF1 it is almost most preferred that said analysis of expression is carried out by means of methylation analysis. It is particularly preferred that the CpG methylation of the gene panels selected from the group of gene panels consisting:

- PITX2, PLAU & TFF1
- PITX2 & PLAU
- PITX2 & TFF1

is analyzed.

It is also particularly preferred that the methylation of the gene panel consisting PITX2 & PLAU is determined in order to provide a prognosis of patients. It is preferred that said patients are analyzed prior to receiving any endocrine treatment.

Hypermethylation of PITX2 and selected other genes as herein and/or sequences thereof are associated with poor prognosis and/or outcome of endocrine treatment of breast cell proliferative disorders, most preferably breast carcinoma.

The methylation pattern of the gene PITX2 and its promoter and regulatory elements have heretofore not been analyzed with regard to prognosis of a patient diagnosed with a breast cell proliferative disorder. Due to the degeneracy of the genetic code, the sequence as identified in SEQ ID NO: 149 should be interpreted so as to include all substantially similar and equivalent sequences upstream of the promoter region of a gene which encodes a polypeptide with the biological activity of that encoded by PITX2.

Most preferably the following method is used to detect methylation within the gene PITX2 and/or regulatory or promoter regions thereof wherein said methylated nucleic acids are present in an excess of background DNA, wherein the background DNA is present in 100 to 1000 times the concentration of the DNA to be detected.

The method for the analysis of methylation comprises contacting a nucleic acid sample obtained from a subject with at least one reagent or a series of reagents, wherein said reagent or series of reagents, distinguishes between methylated and non-methylated CpG dinucleotides within the target nucleic acid.

Preferably, said method comprises the following steps: In the first *step,* a sample of the tissue to be analyzed is obtained. The source may be any suitable source, preferably, the source of the sample is selected from the group consisting of histological slides, biopsies, paraffin-embedded tissue, bodily fluids, plasma, serum, stool, urine, blood, nipple aspirate and combinations thereof. Preferably, the source is tumor tissue, biopsies, serum, urine, blood or nipple aspirate. The most preferred source, is the tumor sample, surgically removed from the patient or a biopsy sample of said patient.

The DNA is then isolated from the sample. Genomic DNA may be isolated by any means standard in the art, including the use of commercially available kits. Briefly, wherein the DNA of interest is encapsulated in/by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

The genomic DNA sample is then treated in such a manner that cytosine bases which are unmethylated at the 5'-position are converted to uracil, thymine, or another base which is dissimilar to cytosine in terms of hybridization behavior. This will be understood as "treatment" or "pre-treatment" herein.

The above described pre-treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior. Enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996) is one preferred example how to perform said pre-treatment. It is further preferred that the bisulfite treatment is carried out in the presence of a radical scavenger or DNA denaturing agent.

The treated DNA is then analyzed in order to determine the methylation state of the gene PITX2 and/or regulatory regions thereof (prior to the treatment) associated with prognosis and/or outcome of endocrine treatment. In a further embodiment of the method the methylation state of the gene PITX2 and/or regulatory regions thereof and the methylation state of one or more genes selected from the group consisting PLAU and TFF1 and/or regulatory or promoter regions thereof is determined. It is particularly preferred that methylation status of a gene panel selected from the group of gene panels consisting PITX2, PLAU & TFF1; PITX2 & PLAU; PITX2 & TFF1 is determined. It is further preferred that the sequences of said genes as described in the accompanying sequence listing (see Table 3) are analyzed.

In the third step of the method, fragments of the pretreated DNA are amplified. Wherein the source of the DNA is free DNA from serum, or DNA extracted from paraffin it is particularly preferred that the size of the amplificate fragment is between 100 and 200 base pairs in length, and wherein said DNA source is extracted from cellular sources (e.g. tissues, biopsies, cell lines) it is preferred that the amplificate is between 100 and 350 base pairs in length. It is particularly preferred that said amplificates comprise at least one 20 base pair sequence comprising at least three CpG dinucleotides. Said amplification is carried out using sets of primer oligonucleotides according to the present invention, and a preferably heat-stable polymerase. The amplification of several DNA segments can be carried out simultaneously in one and the same reaction vessel, in one embodiment of the method preferably six or more fragments are amplified simultaneously. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 18-base-pair long segment of the base sequences of SEQ ID NO: 2-5, SEQ ID NO: 84, 85, 98, 99, SEQ ID NO: 88, 89, 102, 103 and SEQ ID NO: 151 to SEQ ID NO: 158 and sequences complementary thereto.

In a preferred embodiment of the method the primers may be selected from the group consisting to SEQ ID NO: 6 to SEQ ID NO: 10.

In an alternate embodiment of the method, the methylation status of preselected CpG positions within the nucleic acid sequences comprising SEQ ID NO: 1, SEQ ID NO: 73, SEQ ID NO: 75, SEQ ID NO: 149 and SEQ ID NO: 150 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG , TpG or CpA dinucleotide. MSP primers specific for non-methylated DNA contain a "T' at the 3' position of the C position in the CpG. Preferably, therefore, the base sequence of said primers is required to comprise a sequence having a length of at least 18 nucleotides which hybridizes to a pretreated nucleic acid sequence according to SEQ ID NO: 2 to SEQ ID NO: 5 and SEQ ID NO: 151, 152, 155 and 156 and sequences complementary thereto, wherein the base sequence of said oligomers comprises at least one CpG, tpG or Cpa dinucleotide. In this embodiment of the method according to the invention it is particularly preferred that the MSP primers comprise between 2 and 4 CpG , tpG or Cpa dinucleotides. It is further preferred that said dinucleotides are located within the 3' half of the primer e.g. wherein a primer is 18 bases in length the specified dinucleotides are located within the first 9 bases form the 3'end of the molecule. In addition to the CpG , tpG or Cpa dinucleotides it is further preferred that said primers should further comprise several bisulfite converted bases (i.e. cytosine converted to thymine, or on the hybridizing strand, guanine converted to adenosine). In a further preferred embodiment said primers are designed so as to comprise no more than 2 cytosine or guanine bases.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, *e.g.*, matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates.

Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionisation process via the matrix is considerably less efficient. In MALDI-TOF spectrometry, the selection of the matrix plays an eminently important role. For the desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In a particularly preferred embodiment of the method the amplification of step three is carried out in the presence of at least one species of blocker oligonucleotides. The use of such blocker oligonucleotides has been described by Yu et al., BioTechniques 23:714-720, 1997. The use of blocking oligonucleotides enables the improved specificity of the amplification of a sub-population of nucleic acids. Blocking probes hybridized to a nucleic acid suppress, or hinder the polymerase mediated amplification of said nucleic acid. In one embodiment of the method blocking oligonucleotides are designed so as to hybridize to background DNA. In a further embodiment of the method said oligonucleotides are designed so as to hinder or suppress the amplification of unmethylated nucleic acids as opposed to methylated nucleic acids or vice versa.

Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'TpG' at the position in question, as opposed to a 'CpG.' In one embodiment of the method the sequence of said blocking oligonucleotides should be identical or complementary to molecule is complementary or identical to a sequence at least 18 base pairs in length selected from the group consisting of SEQ ID NOs: 2 to 5, 151, 152, 155 and 156 preferably comprising one or more CpG, TpG or CpA dinucleotides. In one embodiment of the method the sequence of said oligonucleotides is selected from the group consisting SEQ ID NO: 15 and SEQ ID NO: 16 and sequences complementary thereto.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivatised at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-termini thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker - a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

In one embodiment of the method, the binding site of the blocking oligonucleotide is identical to, or overlaps with that of the primer and thereby hinders the hybridization of the primer to its binding site. In a further preferred embodiment of the method, two or more such blocking oligonucleotides are used. In a particularly preferred embodiment, the hybridization of one of the blocking oligonucleotides hinders the hybridization of a forward primer, and the hybridization of another of the probe (blocker) oligonucleotides hinders the hybridization of a reverse primer that binds to the amplificate product of said forward primer.

In an alternative embodiment of the method, the blocking oligonucleotide hybridizes to a location between the reverse and forward primer positions of the treated background DNA, thereby hindering the elongation of the primer oligonucleotides.

It is particularly preferred that the blocking oligonucleotides are present in at least 5 times the concentration of the primers.

In the fourth step of the method, the amplificates obtained during the third step of the method are analyzed in order to ascertain the methylation status of the CpG dinucleotides prior to the treatment.

In embodiments where the amplificates are obtained by means of MSP amplification and/or blocking oligonucleotides, the presence or absence of an amplificate is in itself indicative of the methylation state of the CpG positions covered by the primers and or blocking oligonucleotide, according to the base sequences thereof. All possible known molecular biological methods may be used for this detection, including, but not limited to gel electrophoresis, sequencing, liquid chromatography, hybridizations, real time PCR analysis or combinations thereof. This step of the method further acts as a qualitative control of the preceding steps.

In the fourth step of the method amplificates obtained by means of both standard and methylation specific PCR are further analyzed in order to determine the CpG methylation status of the genomic DNA isolated in the first step of the method. This may be carried out by means of hybridization-based methods such as, but not limited to, array technology and probe based technologies as well as by means of techniques such as sequencing and template directed extension.

In one embodiment of the method, the amplificates synthesized in step three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the SEQ ID NO: 2 to SEQ ID NO: 5 and SEQ ID Nos: 151, 152, 155 and 156 and the segment comprises at least one CpG , TpG or CpA dinucleotide. In further embodiments said oligonucleotides contain at least one base sequence having a length of at least 9 nucleotides which is reverse complementary or identical to a segment of the base sequences specified in the SEQ ID NO: 2-5, SEQ ID NO: 151 to SEQ ID NO: 158, SEQ ID NO: 84, 85, 98, 99 and SEQ ID NO: 88, 89, 102, 103; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. In a further embodiment one oligonucleotide exists for the analysis of each CpG dinucleotide within the sequence according to SEQ ID NO: 1 and 149, and the equivalent positions within SEQ ID NO: 2 to 5 and SEQ ID NO:151, 152, 155 and 156. One oligonucleotide exists for the analysis of each CpG dinucleotide within the sequence according to SEQ ID NO: 1, SEQ ID NOS. 149, 150, and SEQ ID NO: 73, SEQ ID NO: 75, and the equivalent positions within SEQ ID NO: 2-5, SEQ ID NO: 151 to SEQ ID NO: 158, SEQ ID NO: 84 85, 98, 99 and SEQ ID NO: 88. 89. 102, 103. Said oligonucleotides may also be present in the form of peptide nucleic acids. The non-hybridized amplificates are then removed. The hybridized amplificates are detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393). There are two preferred embodiments of utilizing this method. One embodiment, known as the TaqMan™ assay employs a dual-labeled fluorescent oligonucleotide probe. The TaqMan™ PCR reaction employs the use of a non-extendible interrogating oligonucleotide, called a TaqMan™ probe, which is designed to hybridize to a CpG-rich sequence located between the forward and reverse amplification primers. The TaqMan™ probe further comprises a fluorescent "reporter moiety" and a "quencher moiety" covalently bound to linker moieties (*e.g.*, phosphoramidites) attached to the nucleotides of the TaqMan™ oligonucleotide. Hybridized probes are displaced and broken down by the polymerase of the amplification reaction thereby leading to an increase in fluorescence. For analysis of methylation within nucleic acids subsequent to bisulfite treatment, it is required that the probe be methylation specific, as described in United States Patent No. 6,331,393, (hereby incorporated by reference in its entirety) also known as the MethyLight assay. The second preferred embodiment of this MethyLight technology is the use of dual-probe technology (Lightcycler®), each probe carrying donor or recipient fluorescent moieties, hybridization of two probes in proximity to each other is indicated by an increase or fluorescent amplification primers. Both these techniques may be adapted in a manner suitable for use with bisulfite treated DNA, and moreover for methylation analysis within CpG dinucleotides.

Also any combination of these probes or combinations of these probes with other known probes may be used.

In a further preferred embodiment of the method, the fourth step of the method comprises the use of template-directed oligonucleotide extension, such as MS-SNuPE as described by Gonzalgo & Jones, Nucleic Acids Res. 25: 2529-2531, 1997. In said embodiment it is preferred that the methylation specific single nucleotide extension primer (MS-SNuPE primer) is identical or complementary to a sequence at least nine but preferably no more than twenty five nucleotides in length of one or more of the sequences taken from the group of SEQ ID NO: 2 to SEQ ID NO: 5 and SEQ ID NOS: 151, 152, 155 and 156. However it is preferred to use fluorescently labeled nucleotides, instead of radiolabeled nucleotides.

In yet a further embodiment of the method, the fourth step of the method comprises sequencing and subsequent sequence analysis of the amplificate generated in the third step of the method (Sanger F., et al., Proc Natl Acad Sci USA 74:5463-5467, 1977).

In the most preferred embodiment of the methylation analysis method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or blocking oligonucleotides, and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO: 2 to SEQ ID NO: 5 and SEQ ID Nos: 151, 152, 155 and 156 and sequences complementary thereto, wherein the base sequence of said oligomers comprises at least one CpG dinucleotide. Additionally, further methylation specific primers may also be used for the analysis of a gene panel as described above wherein said primers comprise a sequence having a length of at least 9 nucleotides which hybridizes to a treated nucleic acid sequence according to one of SEQ ID NO: 84, 85, 98, 99, SEQ ID NO: 88, 89, 102, 103 and SEQ ID Nos: 153, 154, 157 and 158 and sequences complementary thereto, wherein the base sequence of said oligomers comprises at least one CpG dinucleotide.

In an alternative most preferred embodiment of the method, the subsequent amplification of d) is carried out in the presence of blocking oligonucleotides, as described above. It is particularly preferred that said blocking oligonucleotides comprise a sequence having a length of at least 9 nucleotides which hybridizes to a treated nucleic acid sequence according to one of SEQ ID NO: 2 to SEQ ID NO: 5 SEQ ID Nos: 151, 152, 155 and 156 and sequences complementary thereto, wherein the base sequence of said oligomers comprises at least one CpG, TpG or CpA dinucleotide.

Additionally, further blocking oligonucleotides may also be used for the analysis of a gene panel as described above wherein said blocking oligonucleotides comprising a sequence having a length of at least 9 nucleotides which hybridizes to a treated nucleic acid sequence according to one of SEQ ID NO: 84, 85, 98, 99, SEQ ID NO: 88, 89, 102, 103 and SEQ ID Nos: 153, 154, 157 and 158 and sequences complementary thereto, wherein the base sequence of said oligomers comprises at least one CpG, TpG or CpA dinucleotide.

Step e) of the method, namely the detection of the specific amplificates indicative of the methylation status of one or more CpG positions according to SEQ ID NO: 2-5, SEQ ID NO: 151 to SEQ ID NO: 158, and SEQ ID NO: 84, 85, 98, 99, SEQ ID NO: 88, 89, 102, 103, and most preferably SEQ ID NO: 2 to SEQ ID NO: 5 and SEQ ID Nos: 151, 152, 155 and 156 is carried out by means of real-time detection methods as described above.

Additional embodiments of the invention provide a method for the analysis of the methylation status of the gene PITX2 and/or regulatory regions thereof without the need for pre-treatment. Furthermore said method may also be used for the methylation analysis of the gene PITX2 and/or regulatory regions thereof and the methylation state of one or more genes selected from the group consisting PLAU and TFF1 and/or regulatory or promoter regions thereof is determined. It is particularly preferred that methylation status of a gene panel selected from the group of gene panels consisting PITX2, PLAU and TFF1; PITX2 and PLAU; PITX2 and TFF1 is determined.

In the *first step* of such additional embodiments, the genomic DNA sample is isolated from tissue or cellular sources. Preferably, such sources include cell lines, histological slides, biopsy tissue, body fluids, or breast tumor tissue embedded in paraffin. Extraction may be by means that are standard to one skilled in the art, including but not limited to the use of detergent lysates, sonification and vortexing with glass beads. Once the nucleic acids have been extracted, the genomic double-stranded DNA is used in the analysis.

In a preferred embodiment, the DNA may be cleaved prior to the treatment, and this may be by any means standard in the state of the art, but preferably with methylation-sensitive restriction endonucleases.

In the *second step,* the DNA is then digested with one or more methylation sensitive restriction enzymes. The digestion is carried out such that hydrolysis of the DNA at the restriction site is informative of the methylation status of a specific CpG dinucleotide.

In the *third step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.

In the *fourth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

In the final step of the method the prognosis is determined. Preferably, the correlation of the expression level of the genes with the prognosis is done substantially without human intervention. Poor prognosis is determined by aberrant levels of mRNA and/or protein, and hypermethylation. It is particularly preferred that said hypermethylation is above average or above median of said disease in said specific setting.

It is particularly preferred that the classification of the sample is carried out by algorithmic means.

In one embodiment machine learning predictors are trained on the methylation patterns at the investigated CpG sites of the samples with known status. A selection of the CpG positions which are discriminative for the machine learning predictor are used in the panel. In a particularly preferred embodiment of the method, both methods are combined; that is, the machine learning classifier is trained only on the selected CpG positions that are significantly differentially methylated between the classes according to the statistical analysis.

The development of algorithmic methods for the classification of a sample based on the methylation status of the CpG positions within the panel are demonstrated in the examples.

The disclosed invention provides treated nucleic acids, derived from genomic SEQ ID NO: 1, SEQ ID NO: 149, SEQ ID NO: 150, SEQ ID NO: 73 and SEQ ID NO: 75, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the group consisting of SEQ ID NO: 1, SEQ ID NO: 149, SEQ ID NO: 150, SEQ ID NO: 73 and SEQ ID NO: 75, wherein said sequence comprises at least one CpG, TpA or CpA dinucleotide and sequences complementary thereto. The sequences of SEQ ID NO: 2-5, SEQ ID NO: 151 to SEQ ID NO: 158 AND SEQ ID NO: 84, 85, 98, 99 and SEQ ID NO: 88, 89, 102, 103 provide non-naturally occurring modified versions of the nucleic acid according to SEQ ID NO: 1, SEQ ID NO: 149, SEQ ID NO: 150, SEQ ID NO: 73 and SEQ ID NO: 75, wherein the modification of each genomic sequence results in the synthesis of a nucleic acid having a sequence that is unique and distinct from said genomic sequence as follows. For each sense strand genomic DNA, e.g., SEQ ID NO: 1, four converted versions are disclosed. A first version wherein "C" to "T," but "CpG" remains "CpG" (i.e., corresponds to case where, for the genomic sequence, all "C" residues of CpG dinucleotide sequences are methylated and are thus not converted); a second version discloses the complement of the disclosed genomic DNA sequence (i.e. antisense strand), wherein "C" to "T," but "CpG" remains "CpG" (i.e., corresponds to case where, for all "C" residues of CpG dinucleotide sequences are methylated and are thus not converted). The ’upmethylated’ converted sequences of SEQ ID NO: 1, SEQ ID NO: 149, SEQ ID NO: 150, SEQ ID NO: 73 and SEQ ID NO: 75 correspond to SEQ ID NO: 2-5, SEQ ID NO: 151 to SEQ ID NO: 158, SEQ ID NO: 84, 85, 98, 99 and SEQ ID NO: 88. 89. 102, 103. A third chemically converted version of each genomic sequences is provided, wherein "C" to "T" for all "C" residues, including those of "CpG" dinucleotide sequences (i.e., corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" to "T" for all "C" residues, including those of "CpG" dinucleotide sequences (i.e., corresponds to case where, for the complement (antisense strand) of each genomic sequence, all "C" residues of CpG dinucleotide sequences are unmethylated). The ’downmethylated’ converted sequences of SEQ ID NO: 1, SEQ ID NO: 149, SEQ ID NO: 150, SEQ ID NO: 73 and SEQ ID NO: 75 correspond to SEQ ID NO: 2-5, SEQ ID NO: 151 to SEQ ID NO: 158, SEQ ID NO: 84, 85, 98, 99 and SEQ ID NO: 88. 89. 102, 103.

The invention further discloses oligonucleotide or oligomer for detecting the cytosine methylation state within genomic or pre-treated DNA, according to SEQ ID NO: 1, SEQ ID NO:149 to SEQ ID NO: 158, SEQ ID NO: 84, 85, 98, 99 and SEQ ID NO: 88, 89, 102, 103. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO: 2-5, SEQ ID NO: 151 to SEQ ID NO: 158, SEQ ID NO: 84, 85, 98, 99 and SEO ID NO: 88. 89. 102, 103 and/or sequences complementary thereto, or to a genomic sequence according to SEQ ID NO: 1, SEQ ID NO: 149, SEQ ID NO: 150, SEQ ID NO: 73 and SEQ ID NO: 75 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 2-5, SEQ ID NO: 151 to SEQ ID NO: 158, SEQ ID NO: 84, 85, 98, 99 and SEQ ID NO: 88, 89, 102, 103 or to the complements thereof. The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 2-5, SEQ ID NO: 151 to SEQ ID NO: 158, SEQ ID NO: 84, 85, 98, 99 and SEQ ID NO: 88, 89. 102, 103, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 1, SEQ ID NO: 149, SEQ ID NO: 150, SEQ ID NO: 73 and SEQ ID NO: 75 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridisation reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, e.g., SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

n to (n + (X-1));

where n=1, 2, 3,...(Y-(X-1));

where Y equals the length (nucleotides or base pairs) of SEQ ID NO: 1 (9001);

where X equals the common length (in nucleotides) of each oligonucleotide in the set (e.g., X=20 for a set of consecutively overlapping 20-mers); and

where the number (Z) of consecutively overlapping oligomers of length X for a given SEQ ID NO of length Y is equal to Y-(X-1). For example Z= 9001-19= 8,982 for either sense or antisense sets of SEQ ID NO: 1, where X=20.

Preferably, the set is limited to those oligomers that comprise at least one CpG, TpG or CpA dinucleotide.

Examples of inventive 20-mer oligonucleotides include the following set of oligomers (and the antisense set complementary thereto), indicated by polynucleotide positions with reference to SEQ ID NO: 1: 1-20, 2-21, 3-22, 4-23, 5-24, ...... and 8,982 - 9,001.

Preferably, the set is limited to those oligomers that comprise at least one CpG, TpG or CpA dinucleotide.

Likewise, examples of inventive 25-mer oligonucleotides include the following set of oligomers (and the antisense set complementary thereto), indicated by polynucleotide positions with reference to SEQ ID NO: 1: 1-25, 2-26, 3-27, 4-28, 5-29, ............ and 8,977-9,001.

Preferably, the set is limited to those oligomers that comprise at least one CpG TpG or CpA dinucleotide.

The present invention encompasses, for each of SEQ ID NO: 1-5, SEQ ID NO: 149 to SEQ ID NO: 158 and SEQ ID NO: 60 to SEQ ID NO: 103 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO: 1, SEQ ID NO: 149, SEQ ID NO: 150, SEQ ID NO: 73 and SEQ ID NO: 75. Preferred sets of such oligonucleotides or modified oligonucleotides of length X are those consecutively overlapping sets of oligomers corresponding to SEQ ID NO: 1-5, SEQ ID NO: 149 to SEQ ID NO: 158 and SEQ ID NO: 60 to SEQ ID NO: 103 (and to the complements thereof). Preferably, said oligomers comprise at least one CpG. TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophores, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridisation-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of genomic sequences SEQ ID NO: 1, SEQ ID NO: 149, SEQ ID NO: 150, SEQ ID NO: 73 and SEQ ID NO: 75 and sequences complementary thereto, or to the corresponding CpG, TpG or CpA dinucleotide within a sequence of the treated nucleic acids according to SEQ ID NO: 2-5, SEQ ID NO: 151 to SEQ ID NO: 158, SEQ ID NO: 84, 85, 98, 99 and SEQ ID NO: 88, 89, 102, 103 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state of treated genomic DNA (SEQ ID NO: 2-5, SEQ ID NO: 151 to SEQ ID NO: 158 and SEQ ID NO: 76 to SEQ ID NO: 103 ), or in genomic DNA (SEQ ID NO: 1, SEQ ID NO: 149, SEQ ID NO: 150, SEQ ID NO: 73 and SEQ ID NO: 75 and sequences complementary thereto). These probes enable diagnosis, and/or classification of genetic and epigenetic parameters of lung cell proliferative disorders. The set of oligomers may also be used for detecting single nucleotide polymorphisms (SNPs) in treated genomic DNA (SEQ ID NO: 2-5, SEQ ID NO: 151 to SEQ ID NO: 158, SEQ ID NO: 84, 85, 98, 99 and SEQ ID NO: 88. 89. 102, 103), or in genomic DNA (SEQ ID NO: 1, SEQ ID NO: 149, SEQ ID NO: 150, SEQ ID NO: 73 and SEQ ID NO: 75 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO: 2-5, SEQ ID NO: 151 to SEQ ID NO: 158, SEQ ID NO: 84, 85, 98, 99 and SEQ ID NO: 88. 89. 102, 103 and sequences complementary thereto, or segments thereof.

It is anticipated that the oligonucleotides may constitute all or part of an "array" or "DNA chip" (i.e., an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the prior art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital read-out of each molecular species in the mixture).

The described invention further provides a composition of matter useful for providing a prognosis of breast cancer patients. Said composition comprising at least one nucleic acid 18 base pairs in length of a segment of the nucleic acid sequence disclosed in SEQ ID NO: 2 to 5 and SEQ ID NO: 151, 152, 155 and 156, and one or more substances taken from the group comprising : magnesium chloride, dNTP, taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 2 to SEQ ID NO: 5 and SEQ ID NO: 151, 152, 155 and 156 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

Disclosed here additionally is a kit comprising, for example: a bisulfite-containing reagent as well as at least one oligonucleotide whose sequences in each case correspond, are complementary, or hybridize under stringent or highly stringent conditions to a 18-base long segment of the sequences SEQ ID NO: 2 to 5 and SEQ ID NO: 151, 152, 155 and 156. Said kit may further comprise at least one oligonucleotide whose sequences in each case correspond, are complementary, or hybridize under stringent or highly stringent conditions to a 18-base long segment of the sequences SEQ ID NO: 2-5, SEQ ID NO: 151-158 and SEQ ID NO: 76 to 103. Said kit may further comprise instructions for carrying out and evaluating the described method. Said kit may further comprise standard reagents for performing a CpG position-specific methylation analysis, wherein said analysis comprises one or more of the following techniques: MS-SNuPE, MSP, MethyLight®, HeavyMethyl®, COBRA, and nucleic acid sequencing. However, a kit can also contain only part of the aforementioned components.

Typical reagents (*e.g.*, as might be found in a typical COBRA-based kit) for COBRA analysis may include, but are not limited to: PCR primers for specific gene (or methylation-altered DNA sequence or CpG island); restriction enzyme and appropriate buffer; gene-hybridization oligo; control hybridization oligo; kinase labeling kit for oligonucleotide probe; and radioactive nucleotides. Additionally, bisulfite conversion reagents may include: DNA denaturation buffer; sulfonation buffer; DNA recovery reagents or kits (*e.g.*, precipitation, ultrafiltration, affinity column); desulfonation buffer; and DNA recovery components.

Typical reagents (*e.g.*, as might be found in a typical MethyLight®-based kit) for MethyLight® analysis may include, but are not limited to: PCR primers for specific gene (or methylation-altered DNA sequence or CpG island); TaqMan® probes; optimized PCR buffers and deoxynucleotides; and Taq polymerase.

Typical reagents (*e.g.*, as might be found in a typical Ms-SNuPE-based kit) for Ms-SNuPE analysis may include, but are not limited to: PCR primers for specific gene (or methylation-altered DNA sequence or CpG island); optimized PCR buffers and deoxynucleotides; gel extraction kit; positive control primers; Ms-SNuPE primers for specific gene; reaction buffer (for the Ms-SNuPE reaction); and radioactive nucleotides. Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

Typical reagents (*e.g.*, as might be found in a typical MSP-based kit) for MSP analysis may include, but are not limited to: methylated and unmethylated PCR primers for specific gene (or methylation-altered DNA sequence or CpG island), optimized PCR buffers and deoxynucleotides, and specific probes.

SEQ ID NO: 6 to 9 provide the nucleic acid sequences of those primers and probes useful to predict the survival of breast cancer patients according to the invention as described in Example 4.

SEQ ID NO: 10 to 12 provide the nucleic acid sequences of primers and probes according to a control gene used in the example 4 and 5.

SEQ ID NO: 13 provides a sub-sequence of SEQ ID NO: 1, which represents the nucleic acid sequence of the human gene PITX2.

SEQ ID NO: 14 to 17 provide the nucleic acid sequences of those primers and probes useful to predict the survival of breast cancer patients according to the invention as described in example 5.

SEQ ID NO: 21 provides an amino acid sequence of the polypeptide encoded by the gene PITX2. The amino acid sequence of the polypeptide encoded by the gene PITX2 is also illustrated in figure 10.

FIGURES

Figure 1 illustrates a simplified model of a Stage 1-3 breast tumor wherein primary treatment was surgery (at point 1), followed by adjuvant therapy with Tamoxifen, as an example for an endocrine treatment.. The Y axis represents tumor(s) mass (or size), wherein the line '3' indicates the limit of detectability of said tumor mass. The X axis represents time. In a first scenario a patient without relapse during endocrine treatment (4) is shown as remaining below the limit of detectability for the duration of the observation. A patient with relapse of the cancer (5) has a period of disease free survival (2) followed by relapse when the carcinoma mass reaches the level of detectability.

Figure 2 shows the result of the assay (QM assay) as described in Example 4: A Kaplan-Meier estimated metastasis-free survival curve for three CpG sites of the PITX2 gene by means of Real-Time methylation specific probe analysis (QM assay). The lower curve shows the proportion of metastasis free patients in the population with above median methylation levels, the upper curve shows the proportion of metastasis free patients in the population with below median methylation levels. The X axis shows the metastasis free survival times of the patients in months, and the Y axis shows the proportion of metastasis free survival patients.

Figure 3 shows the result of the chip hybridization experiment as described in Example 2. A Kaplan-Meier estimated metastasis-free survival curves for two CpG positions of the PITX2 gene by means of methylation specific detection oligonucleotide hybridization analysis. The lower curve shows the proportion of metastasis free patients in the population with above median methylation levels, the upper curve shows the proportion of metastasis free patients in the population with below median methylation levels. The X axis shows the metastasis free survival times of the patients in months, and the Y axis shows the proportion of metastasis free survival patients.

Figure 4 shows the Kaplan-Meier estimated metastasis-free survival curves for two CpG positions of the PITX2 gene by means of methylation specific detection oligonucleotide hybridization analysis. The lower line shows the proportion of metastasis free patients in the population of 55 patients with above median methylation levels, the upper curve shows the proportion of metastasis free patients in the population of 54 patients with below median methylation levels. The X axis shows the metastasis free survival times of the patients in years, and the Y axis shows the proportion of metastasis free survival patients in %. This resulted from a first data set that was achieved in a first study.

Figure 5 shows the Kaplan-Meier estimated metastasis-free survival curves for six different CpG positions located within the preferred region of the PITX2 gene (SEQ ID NO: 13) by means of methylation specific detection oligonucleotide hybridization analysis. The lower line shows the proportion of metastasis free patients in the population of 118 patients with above median methylation levels, the upper curve shows the proportion of metastasis free patients in the population of 118 patients with below median methylation levels. The X axis shows the metastasis free survival times of the patients in years, and the Y axis shows the proportion of metastasis free survival patients in %. This resulted from a second data set that was achieved in a second study.

Figure 6 shows the Kaplan-Meier estimated metastasis-free survival curves for 6 different CpG positions located within the preferred region of the PITX2 gene (SEQ ID NO: 13) by means of methylation specific detection oligonucleotide hybridization analysis. This time only a sub-population of 148 patients, characterized by a tumor at grade G1 or G2, was analyzed: The lower curve shows the proportion of metastasis free patients in the population of 74 patients with above median methylation levels, the upper curve shows the proportion of metastasis free patients in the population of 74 patients with below median methylation levels. The X axis shows the metastasis free survival times of the patients in years, and the Y axis shows the proportion of metastasis free survival patients in %. This resulted from a second data set as shown in the Example 2.

Figure 7 shows the Kaplan-Meier estimated metastasis-free survival curves for 4 different CpG positions located within the preferred region of the PITX2 gene (SEQ ID NO: 13) by means of methylation specific detection oligonucleotide hybridization analysis. This time a sub-population of 224 patients, characterized by a tumor of stage 1 or 2 (T1 or T2), was analyzed: The lower curve shows the proportion of metastasis free patients in the population of 112 patients with above median methylation levels, the upper curve shows the proportion of metastasis free patients in the population of 112 patients with below median methylation levels. The X axis shows the metastasis free survival times of the patients in years, and the Y axis shows the proportion of metastasis free survival patients in %. This resulted from the second data set that was achieved in the second example.

Figure 8 shows the disease-free survival curves of a combination of two oligonucleotides each from the genes TBC1D3 and CDK6, and one oligonucleotide from the gene PITX2 covering two CpG sites. The black curve shows the proportion of disease free patients in the population with above median methylation scores, the gray curve shows the proportion of disease free patients in the population with below median methylation scores.

Figure 9 shows the plot according to Figure 8 and the classification of the sample set by means of the St. Gallen method. The unbroken lines represent the methylation analysis wherein the black curve shows the proportion of disease free patients in the population with above median methylation scores, the gray curve shows the proportion of disease free patients in the population with below median methylation scores. The broken lines represent the St. Gallen classification of the sample set wherein the black curve shows the disease free survival time of the high risk group and the gray curve shows the disease free survival of the low risk group.

Figure 10 illustrates the amino acid sequence of the polypeptide encoded by the gene PITX2.

Figure 11 illustrates the positions of the amplificates sequenced in Example 7. 'A' shows an illustration of the gene with the major exons annotated, 'B' shows annotated mRNA transcript variants and 'C' shows CpG rich regions of the gene. The positions of Amplificates 1 to 11 are shown to the right of the illustrations.

Figure 12 shows the sequencing data of 11 amplificates of the gene PITX2 according to Example 7. Each column of the matrices of columns 'A' and 'B' represent the sequencing data for one amplificate. The amplificate number is shown to the left of the matrices. Each row of a matrix represents a single CpG site within the fragment and each column represents an individual DNA sample. The matrices in the column marked 'A' showed below median methylation as measured by QM assays, the matrices in the column marked 'B' showed below median methylation as measured by QM assays.

The bar on the left represents a scale of the percent methylation, with the degree of methylation represented by the shade of each position within the column from black representing 100% methylation to light gray representing 0% methylation. White positions represented a measurement for which no data was available.

Figure 13 shows a schematic view of mRNA transcript variants of PITX2, as annotated in the on-line Ensembl database.

Figure 14 shows the Kaplan-Meier estimated disease-free survival curves for a CpG position of the ERBB2 gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 15 shows the Kaplan-Meier estimated metastasis-free survival curves for a CpG position of the ERBB2 gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 16 shows the Kaplan-Meier estimated metastasis-free survival curves for a CpG position of the TFF1 gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 17 shows the Kaplan-Meier estimated metastasis-free survival curves for a CpG position of the TFF1 gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 18 shows the Kaplan-Meier estimated disease-free survival curves for a CpG position of the PLAU gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 19 shows the Kaplan-Meier estimated metastasis-free survival curves for a CpG position of the PLAU gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 20 shows the Kaplan-Meier estimated disease-free survival curves for a CpG position of the PITX2 gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut offpoint's methylation levels.

Figure 21 shows the Kaplan-Meier estimated metastasis-free survival curves for a CpG position of the PITX2 gene by means of Real-Time methylation specific probe analysis according to Example 8. The X axis shows the disease free survival times of the patients in years, and the Y- axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 22 shows the Kaplan-Meier estimated disease-free survival curves for a CpG position of the TBC1D3 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 23 shows the Kaplan-Meier estimated metastasis-free survival curves for a CpG position of the TBC1D3 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 24 shows the Kaplan-Meier estimated disease-free survival curves for a CpG position of the ERBB2 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 25 shows the Kaplan-Meier estimated metastasis-free survival curves for a CpG position of the ERBB2 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 26 shows the Kaplan-Meier estimated disease-free survival curves for a CpG position of the TFF1 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 27 shows the Kaplan-Meier estimated metastasis-free survival curves for a CpG position of the TFF1 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 28 shows the Kaplan-Meier estimated disease-free survival curves for a CpG position of the PLAU gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 29 shows the Kaplan-Meier estimated metastasis-free survival curves for a CpG position of the PLAU gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 30 shows the Kaplan-Meier estimated disease-free survival curves for a CpG position of the PITX gene by means of Real-Time methylation specific probe analysis according to

Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 31 shows the Kaplan-Meier estimated metastasis-free survival curves for a CpG position of the PITX gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 32 shows the Kaplan-Meier estimated disease-free survival curves for a CpG position of the PITX gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 33 shows the Kaplan-Meier estimated metastasis-free survival curves for a CpG position of the PITX gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 34 shows the Kaplan-Meier estimated disease-free survival curves for a CpG position of the ONECUT gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 35 shows the Kaplan-Meier estimated metastasis-free survival curves for a CpG position of the ONECUT gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 36 shows the Kaplan-Meier estimated disease-free survival curves for a CpG position of the TBC1D3 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 37 shows the Kaplan-Meier estimated metastasis-free survival curves for a CpG position of the TBC1D3 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 38 shows the Kaplan-Meier estimated disease-free survival curves for a CpG position of the ABCA8 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 39 shows the Kaplan-Meier estimated metastasis-free survival curves for a CpG position of the ABCA8 gene by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 40 shows the Kaplan-Meier estimated disease-free survival curves for a CpG position of a combination of the TFF1 & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 41 shows the Kaplan-Meier estimated metastasis-free survival curves for a CpG position of a combination of the TFF1 & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 42 shows the Kaplan-Meier estimated disease-free survival curves for a CpG position of a combination of the TFF1 & PLAU & PITX genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 43 shows the Kaplan-Meier estimated metastasis-free survival curves for a CpG position of a combination of the TFF1 & PLAU & PITX genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 44 shows the Kaplan-Meier estimated disease-free survival curves for a CpG position of a combination of the PITX & TFF1 genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 45 shows the Kaplan-Meier estimated metastasis-free survival curves for a CpG position a combination of the PITX & TFF1 genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 46 shows the Kaplan-Meier estimated disease-free survival curves for a CpG position of a combination of the PITX & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 47 shows the Kaplan-Meier estimated metastasis-free survival curves for a CpG position of a combination of the PITX & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 48 shows the Kaplan-Meier estimated disease-free survival curves for a CpG position of a combination of the TFF1 & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 49 shows the Kaplan-Meier estimated metastasis-free survival curves for a CpG position of a combination of the TFF1 & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 50 shows the Kaplan-Meier estimated disease-free survival curves for a CpG position of a combination of the TFF1 & PLAU & PITX genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 51 shows the Kaplan-Meier estimated metastasis-free survival curves for a CpG position of a combination of the TFF1 & PLAU & PITX genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 52 shows the Kaplan-Meier estimated disease-free survival curves for a CpG position of a combination of the PITX & TFF1 genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 53 shows the Kaplan-Meier estimated metastasis-free survival curves for a CpG position of a combination of the PITX & TFF1 genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels.

Figure 54 shows the Kaplan-Meier estimated disease-free survival curves for a CpG position of a combination of the PITX & PLAU genes by means of Real-Time methylation specific probe analysis according to Example 8. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

Figure 55 shows the Kaplan-Meier estimated metastasis-free survival curves for a CpG position of a combination of the PITX & PLAU genes by means of Real-Time methylation specific probe analysis according to Example 8. The X axis shows the disease free survival times of the patients in years, and the Y-axis shows the proportion of patients with metastasis free survival. The black plot shows the proportion of metastasis free patients in the population with above an optimized cut off point's methylation levels, the gray plot shows the proportion of disease free patients in the population with below an optimized cut off point's methylation levels.

Figure 56 shows a scatter plot of matched pair PET and fresh frozen tissues analyzed using PITX2 gene assay 1 according to Example 8. Quantitative methylation CT scores of PET samples are shown on the Y-axis, and quantitative methylation CT scores of fresh frozen samples are shown on the X-axis. The association between the paired samples is 0.81 (Spearman's rho). This analysis is based on n=89 samples.

Figure 57 shows the Disease free survival (DFS) of randomly selected ER+, N0, untreated patient population in Kaplan-Meier survival plot according to Example 8. Proportion of disease free patients is shown on the Y-axis and time in years is shown on the X-axis. 139 events were observed (observed event rate=33%). Disease free survival after 5 years: 74.5% [70.3%, 78.9%], after 10 years 59.8% [54.2%, 66%]. 95% confidence intervals are plotted.

Figure 58 shows the distribution of follow-up times in ER+, N0, untreated population according to Example 8. Frequency is shown on the Y-axis and time in months is shown on the X-axis. The figure on the left shows patients with event (all kinds of relapses). Mean follow-up time 45.8 months (standard deviation=31), median=38 (range=[2, 123]). The figure on the right shows censored patients. Mean follow up time 93 months (standard deviation=35.6), median=94 (range=[1, 190]).

Figure 59 shows the Disease free survival (DFS) of ER+, N0, TAM treated population in Kaplan-Meier plot according to Example 8. Proportion of disease free patients is shown on the Y-axis and time in years is shown on the X-axis. 56 events were observed (observed event rate=10 %). DFS after 5 years: 92.4% [90%, 94.9%], after 10 years: 82.1% [77.3%,87.2%]. 95% confidence intervals are plotted.

Figure 60 shows the distribution of follow-up times in ER+, N0, untreated population according to Example 8. Frequency is shown on the Y-axis and time in months is shown on the X-axis. The figure on the left shows patients with all events (all kinds of relapses). Mean follow-up time 47.9 months (standard deviation=24.4), median=45 (range=[2, 98]). The figure on the right shows censored patients. Mean follow up time 65.3 months (standard deviation=31.6), median=64 (range=[0, 158]).

Figure 61 shows the ROC plot at different times for marker model 3522 (Assay 1) and 2265 on ER+N0 TAM treated population according to Example 8. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates, and the estimated area under the curve (AUC) is calculated. Values for median cut off (triangle) and best cut off (diamond, 0.32 quantile) are plotted.

Figure 62 shows the ROC plot at different times for marker model 3522 (Assay 1) alone on ER+N0 TAM treated population according to Example 8. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates, and the estimated area under the curve (AUC) is calculated. Values for median cut off (triangle) and best cut off (diamond, 0.42 quantile) are plotted.

Figure 63 shows the ROC plot at different times for marker model 2265 on ER+N0 TAM treated population according to Example 8. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates for different thresholds (= 5, 6, 7 , 8 years) and the estimated area under the curve (AUC) is calculated. Values for median cut off (triangle) and best cut off (diamond, 0.78 quantile) are plotted.

Figure 64 shows the ROC plot at different times for marker model 2395 on ER+N0 TAM treated population according to Example 8. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates for different thresholds (= 5, 6, 7 , 8 years), and the estimated area under the curve (AUC) is calculated. Values for median cut off (triangle) and best cut off (diamond, 0.77 quantile) are plotted.

Table 1: Genomic sequences and treated variants thereof according to the invention

| Gene name | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|
| PITX2 | 1 | 2 | 3 | 4 | 5 |
| PITX2 | 13 | | | | |
| PITX2 | 18 | | | | |
| PITX2 | 19 | | | | |
| ABCA8 | 69 | 76 | 77 | 90 | 91 |
| CDK6 | 70 | 78 | 79 | 92 | 93 |
| ERBB2 | 71 | 80 | 81 | 94 | 95 |
| ONECUT | 72 | 82 | 83 | 96 | 97 |
| PLAU | 73 | 84 | 85 | 98 | 99 |
| TBC1D3 | 74 | 86 | 87 | 100 | 101 |
| TFF1 | 75 | 88 | 89 | 102 | 103 |
| PITX2 | 149 | 151 | 152 | 155 | 156 |
| TFF1 | 150 | 153 | 154 | 157 | 158 |

**EXAMPLES**

EXAMPLE 1 : Study 1

The first study was based on a population of 109 patients, comprising patients of both nodal statuses N0 and N+. Cells from breast cell proliferative disorder tissue of all patients were ER+ (estrogen receptor positive). All patients received Tamoxifen monotherapy immediately after surgery or diagnosis. The samples were analyzed using the applicant's chip technology with two chip panels representing 117 candidate genes. For further details see examples in the published patent applications WO 04/035803 and EP 03 090 432.0, which are hereby incorporated by reference. In this study one of the most significant marker gene was PITX2. The methylation status of PITX2, coding for a transcription factor, was statistically significantly correlated with disease-free survival of patients undergoing adjuvant Tamoxifen treatment. This was calculated using the Cox regression model taking into account the nodal status of the patient at the time of diagnosis.

The result from this study - with respect to PITX2 - is illustrated in Figure 4. The X axis shows the metastasis free survival times of the patients in years, and the Y axis shows the proportion of metastasis free survival patients in %. Amongst the 54 patients (upper line) with below median methylation levels have a significantly longer metastasis free survival time than the 55 patients with above median methylation levels (lower line). To illustrate the result, at 10 years after surgery combined with Tamoxifen monotherapy, more than 75% of the patients with below median methylation in PITX2 were still metastasis free, as compared to less than 60% of the patients with above median methylation in PITX2.

As the survival of a breast cancer patient is known to also be correlated to the patient's nodal status, the differentiating power of the marker in this mixed population is expected to be less than in a homogenous population.

Another study was performed to analyze whether the same marker can be identified independently, in a completely different set of patient samples and also to characterize the differential power towards predicting survival for a sub-group of patients, all being N0.

EXAMPLE 2 : Study 2

The second study was based on samples from 236 patients from 5 different sample providers, wherein all patients were NO (nodal status negative), and older than 35 years. In all cases surgery was performed before 1998. Cells from breast cell proliferative disorder tissue of all patients were ER+ (estrogen receptor positive), and the tumors were graded to be T1-3, G1-3. In this study all patients received Tamoxifen directly after surgery, and the outcome was assessed according to the length of disease-free survival. In order to be as representative as possible for the final target group, the patients and their tumor samples had to fulfil the following criteria:

The range and median follow-up of patients were the following:

Median: 64.5 months
Range: 3 months to 142 months
(calculated based on patients who were disease-free at end of observation time).

Analysis of the methylation patterns of patient samples treated with Tamoxifen as an adjuvant therapy immediately following surgery (see Figure 1) is shown in the plots according to Figures 5 to 7. For the amplificate, the mean methylation over 4 oligo-pairs for that amplificate was calculated and the population split into groups according to their mean methylation values, wherein one group was composed of individuals with a methylation score higher than the median and a second group composed of individuals with a methylation score lower than the median.

The primer oligonucleotides used to generate the amplificate, that was analyzed in the array experiment were:
Array Primer PITX2_Q21: GTAGGGGAGGGAAGTAGATGT (SEQ ID NO: 22)
Array Primer PITX2_R23: TCCTCAACTCTACAAACCTAAAA (SEQ ID NO: 23)
The corresponding genomic region of said amplificate is given in SEQ ID NO: 13.

The sequences of the oligonucleotides used in this array experiment were the following:

SEQ ID NO xx: AGTCGGGAGAGCGAAA
SEQ ID NO xx: AGTTGGGAGAGTGAAA
SEQ ID NO xx: AAGAGTCGGGAGTCGGA
SEQ ID NO xx: AAGAGTTGGGAGTTGGA
SEQ ID NO xx: GGTCGAAGAGTCGGGA
SEQ ID NO xx: GGTTGAAGAGTTGGGA
SEQ ID NO xx: ATGTTAGCGGGTCGAA
SEQ ID NO xx: TAGTGGGTTGAAGAGT

When the data derived from analyzing 6 different CpG sites, located within the preferred amplified region of the PITX2 gene by means of methylation specific detection oligonucleotide hybridization analysis were plotted as Kaplan-Meier estimated metastasis-free survival curves, it can be seen that the differential power of the marker PITX2 increased with selecting for N0 patients. This is shown in figures 5 to 7. The X axis shows the metastasis free survival times of the patients in years, and the Y axis shows the proportion of metastasis free survival patients in %. The lower curve shows the proportion of metastasis free patients in the population with above median methylation levels, and the upper curve shows the proportion of metastasis free patients in the population with below median methylation levels.

For example, as illustrated in figure 5, 10 years after surgery only about 65% of the patients of the 118 patients with the higher methylation status are metastasis free, whereas about 90% of the 118 patients with lower methylation status are metastasis free.

As illustrated in Figure 6 the analogous Kaplan-Meier analysis for a sub-population of 148 patients, characterized by a tumor at stage G1 or G2 this differential power increases again: 10 years after surgery only about 60% of the 74 patients with the higher methylation status are metastasis free, whereas about 95% of the 74 patients with lower methylation status are metastasis free.

Figure 7 illustrates how the survival is also correlated to the tumor stage at surgery by showing the analogous Kaplan-Meier analysis for a sub-population of 150 patients, characterized by a tumor stage of T1 or T2: The number of patients with 10 years MFS is about 68% of patients of the 112 with the higher methylation status, whereas about 95% of the 112 patients with lower methylation status are metastasis free.

EXAMPLE 3:

The accuracy of the differentiation between the different groups was further increased by combining multiple oligonucleotides from different genes. As described in the text it was recognized that adding additional informative markers to the analysis could potentially increase the prognostic power of a survival test. Therefore it was calculated how a combination of two methylation specific oligonucleotides each from the genes TBC1D3 and CDK6, and one oligonucleotide from the gene PITX2 would differentiate the groups of good or bad prognosis. The result is shown in figure 8 as the according Kaplan-Meier curve.

Figure 9 shows -on top of Figure 8- the classification of the patients from the sample set by means of the St. Gallen method (the current method of choice for estimating disease free survival), thereby showing the improved effectiveness of methylation analysis over current methods, in particular post 80 months.

EXAMPLE 4: Real time quantitative methylation analysis

Genomic DNA was analyzed using the Real Time PCR technique after bisulfite conversion. In this analysis four oligonucleotides were used in each reaction. Two non methylation specific PCR primers were used to amplify a segment of the treated genomic DNA containing a methylation variable oligonucleotide probe binding site. Two oligonucleotide probes competitively hybridize to the binding site, one specific for the methylated version of the binding site, the other specific to the unmethlyated version of the binding site. Accordingly, one of the probes comprises a CpG at the methylation variable position (i.e. anneals to methylated bisulphite treated sites) and the other comprises a TpG at said position (i.e. anneals to unmethylated bisulphite treated sites). Each species of probe is labeled with a 5' fluorescent reporter dye and a 3' quencher dye wherein the CpG and TpG oligonucleotides are labeled with different dyes.

The reactions are calibrated by reference to DNA standards of known methylation levels in order to quantify the levels of methylation within the sample. The DNA standards were composed of bisulfite treated phi29 amplified genomic DNA (i.e. unmethlyated), and/or phi29 amplified genomic DNA treated with Sss1 methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only).

The amount of sample DNA amplified is quantified by reference to the gene (β-actin (ACTB)) to normalize for input DNA. For standardization the primers and the probe for analysis of the ACTB gene lack CpG dinucleotides so that amplification is possible regardless of methylation levels. As there are no methylation variable positions, only one probe oligonucleotide is required.

The following oligonucleotides were used in the reaction to amplify the control amplificate:
Control Primer1: TGGTGATGGAGGAGGTTTAGTAAGT (SEQ ID NO: 10)
Control Primer2: AACCAATAAAACCTACTCCTCCCTTAA (SEQ ID NO: 11)
Control Probe: 6FAM-ACCACCACCCAACACACAATAACAAACACA-TAMRA or Dabcyl (SEQ ID NO: 12)

The nucleic acid sequence of the gene PITX2 is given in (SEQ ID NO: 1), after treatment with bisulfite two different strands are generated, and each of the strands is represented twice, once in a prior to treatment methylated version (SEQ ID NO: 2 and 3) and once in the prior to treatment unmethylated form (SEQ ID NO: 4 and 5), which are characterized as containing no cytosine bases (despite of those 5' adjacent to a guanine and methylated before treatment). The following primers are used to generate an amplificate within the PITX2 sequence comprising the CpG sites of interest:

Primers for PITX bisulfite amplificate length : 144 bp
PITX2: GTAGGGGAGGGAAGTAGATGTT (SEQ ID NO: 6)
PITX2: TTCTAATCCTCCTTTCCACAATAA (SEQ ID NO: 7)

The genomic region according to the generated amplificate of 144 bp in length is given in SEQ ID NO: 18.
Probes:
PITX2cg1: FAM-AGTCGGAGTCGGGAGAGCGA-Darquencher (SEQ ID NO: 8)
As an alternative quencher TAMRA was also used in additional experiments:
FAM-AGTCGGAGTCGGGAGAGCGA-TAMRA
PITX2tg1: YAKIMA YELLOW-AGTTGGAGTTGGGAGAGTGAAAGGAGA-Darquencher (SEQ ID NO: 9)
In additional experiments we also used :
VIC- AGTTGGAGTTGGGAGAGTGAAAGGAGA -TAMRA

The extent of methylation at a specific locus was determined by the following formula:

$$\text{methylation rate} = 100 * I\,(CG) / (I(CG) + I(TG))$$

(I = Intensity of the fluorescence of CG-probe or TG-probe)
PCR components were ordered from Eurogentec:
3 mM MgCl2 buffer, 10x buffer, Hotstart TAQ
Program (45 cycles): 95 °C, 10 min; 95 °C, 15 sec; 62 °C, 1 min

This assay was performed on 236 samples identical to those used in Example 2. The result is shown in figure 2. Figure 2 shows the Kaplan-Meier estimated disease-free survival curves for 3 CpG positions of the PITX2 gene by means of Real-Time methylation specific probe analysis, as described above. The lower curve shows the proportion of disease free patients in the population with above median methylation levels, the upper curve shows the proportion of disease free patients in the population with below median methylation levels. The X axis shows the disease free survival times of the patients in months, and the Y- axis shows the proportion of disease free survival patients. The p-value (probability that the observed distribution occurred by chance) was calculated as 0.0031, thereby confirming the data obtained by means of array analysis.

For comparison, figure 3 illustrates the result from the array analysis of said gene, according to the chip hybridization experiment described in Example 2, wherein detection oligos were used (for details see EP 03 090 432.0, which is incorporated by reference). The p-value (probability that the observed distribution occurred by chance) was calculated as 0.0011.

EXAMPLE 5

Another QM assay was developed, which also performed very well. The following PITX2 specific oligonucleotides were employed to generate an amplificate of 164 bp. The oligonucleotides are specific for three co-methylated CpG positions:
Primers for PITX2 bisulfite amplificate with a length of 162 bp :
PITX2: AACATCTACTTCCCTCCCCTAC (SEQ ID NO: 14)
PITX2: GTTAGTAGAGATTTTATTAAATTTTATTGTAT (SEQ ID NO: 15)
The genomic region according to the generated amplificate of 162 bp in length is given in SEQ ID NO: 19.

Probes (from ABI):
PITX2-IIcg1 : FAM-TTCGGTTGCGCGGT-MGBNQF (SEQ ID NO: 16)
PITX2-IItg1: VIC-TTTGGTTGTGTGGTTG- MGBNQF (SEQ ID NO: 17)

The extent of methylation at a specific locus was determined by the following formula:

$$\text{methylation rate} = 100 * I\,(CG) / (I(CG) + I(TG))$$

(I = Intensity of the fluorescence of CG-probe or TG-probe)
PCR components were ordered from Eurogentec : 2,5 mM MgCl2 buffer, 10x buffer, Hotstart TAQ
Program (45 cycles): 95 °C, 10 min; 95 °C, 15 sec; 60 °C, 1 min

EXAMPLE 6: LOH analysis

Patient material

The material to be used in this study, consists of fresh frozen healthy breast tissue, fresh frozen breast tumor tissue from untreated breast cancer patients (follow up over >10 years) and samples from Tamoxifen treated patients (follow up over >10 years from Tamoxifen treatment). Aliquots of DNA from these micro-dissected lesions are used as the source template for PCR-based LOH (Loss of heterozygosity) analysis. All tumor samples were derived from ER+ node negative patients.

LOH analysis

DNA from all tissue samples is subjected to PCR-based LOH analysis using two 4q25-26 markers (D4S1284 and D4S406). These markers define a region on chromosome 4 comprising the gene PITX2 gene said region but being more than 8.5 kbp distant of a region previously shown to undergo LOH in breast carcinomas [Cancer Research 59, 3576-3580, August 1, 1999].

DNA Extraction

Extract DNA from samples using the Wizzard Kit (Promega).

PCR reaction

See Clin. Cancer Res., 5: 17-23, 1999 for further details.
Analyze each sample by means of single-plex PCR using the following primers:

D4S406

Forward primer: GAAAGGCAGAGTCATAACAGGAAG (SEQ ID NO: 32)
Reverse primer: TAAGGATAGAGTGATTTCCAAGAAAG (SEQ ID NO: 33)
PCR product size:205 (bp)
GenBank Accession:Z16728

D4S1284

Forward primer: CTTATCTGACAACAAGCGAGTATG (SEQ ID NO: 34)
Reverse primer: CAATTATTGTATTGTAGCATCGGAG (SEQ ID NO: 35)
PCR product size: 172 (bp)
GenBank Accession:L14168
Synthesize forward primers with either a fluorescent FAM tag (D4S1284) or a fluorescent TET tag (D4S406) at the 5' end.
Prepare a suitable quantity of nucleotide mixture according to Table 2.
Aliquot 1 μl of each DNA sample into separate PCR tubes, add 9 μl reaction mixture according to Table 3 and thermal cycle according to the following conditions.
Thermal cycling conditions:
95°C for 15 min; 39 cycles: 95°C for 1 min; 55°C for 0:45; 72°C for 1:15; and 72°C for 10 min

Gel electrophoresis

Horizontal ultrathin, high throughput fluorescence-based DNA fragment gel electrophoresis is the preferred technique to separate and analyze the PCR-generated alleles. Combine one microliter of amplified material with 2 μl formamide loading dye (APB) prior to electrophoresis. Add ROX 350 fluorescent size markers (0.7 μl; ABI) to amplified tumor DNA to allow sizing of alleles. Heat samples to 95°C, load on 70 μm, 5% horizontal polyacrylamide gel and electrophorese for 1 h and 15 min at 30 W in 1 x TBE.
Data may be collected as commonly known in the art (see for example Clin. Cancer Res., 5: 17-23, 1999).
To determine whether allelic deletion had occurred at individual markers, calculate allelic ratios and express as a percentage of loss of intensity for the treated and untreated tumor samples compared with the corresponding normal samples (D-value) after normalization. When the allelic ratio in the tumor DNA is reduced by greater than 40% (DO.40) from that found in the normal DNA, the sample is denoted as having LOH at that locus.

Table 2: Nucleotide Mix

| |
|---|
| 10 μl dATP, 10 mM |
| 10 μl dGTP, 10 mM |
| 10 μl dTTP, 10 mM |
| 2.0 μl dCTP, 10 mM |
| 288 μl DEPC-treated $H_2O$ |

Table 3: Reaction mixture

| | |
|---|---|
| 1.0 µl | Taq Buffer |
| 0.8 µl | Reduced nucleotide mixture |
| 0.2 µl | Forward primer, 20 µM |
| 0.2 µl | Reverse primer, 20 µM |
| 6.6 µl | DEPC treated $H_2O$ |
| 0.1 µl | α-32P dCTP |
| 0.1 µl | AmpliTaq Gold Polymerase |
| | Total volume = 9 µl |

**Example 7: Sequencing of gene PITX2**

Sequencing of the gene PITX2 was carried out in order to confirm that co-methylation of CpG positions correlated across all exons. For bisulfite sequencing amplification primers were designed to cover 11 sequences within the gene PITX2, see Figure 11 for further details. Sixteen samples analyzed in Example 4 were utilized for amplicon production. Each sample was treated with sodium bisulfite and sequenced. Sequence data was obtained using ABI 3700 sequencing technology. Obtained sequence traces were normalized and percentage methylation calculated using the Applicant's proprietary bisulphite sequence sequencing trace analysis program (See WO 2004/000463 for further information).

Samples

Eight samples displayed hypermethylation and eight samples displayed hypomethylation in analysis using QM assay as described in example 4.

Amplification

Fragments of interest were amplified using the following conditions

PCR Reaction solution:

| | |
|---|---|
| Taq 5U/µl | 0,2 |
| dNTPs 25mM each | 0,2 |
| 10x buffer | 2,5 |
| water | 10,1 |
| primer (6,25µM | 2 |
| DNA (1ng/µl) | 10 |

Cycling conditions:
15min 95°C; 30s 95°C; 30s 58°C; 1:30min 72°C (40 cycles)

Table 4: Primers and Amplificates

| Forward primer SEQ ID NO: | Reverse primer SEQ ID NO: | Amplificate SEQ ID NO: | Amplificate number |
|---|---|---|---|
| 36 | 37 | 38 | 1 |
| 39 | 40 | 41 | 2 |
| 42 | 43 | 44 | 3 |
| 45 | 46 | 47 | 4 |
| 48 | 49 | 50 | 5 |
| 51 | 52 | 53 | 6 |

(continued)

| Forward primer SEQ ID NO: | Reverse primer SEQ ID NO: | Amplificate SEQ ID NO: | Amplificate number |
|---|---|---|---|
| 54 | 55 | 56 | 7 |
| 57 | 58 | 59 | 8 |
| 60 | 61 | 62 | 9 |
| 63 | 64 | 65 | 10 |
| 66 | 67 | 68 | 11 |

Sequencing

Only g-rich primers were used for sequencing with one exception: Amplificate Number 2 was sequenced using both forward and reverse primer.

ExoSAP-IT Reaction solution:

4μl PCR product + 2μl ExoSAP-IT

45min/37°C and 15min/95°C

Cycle sequencing:
1 μl BigDye v.1.1
1 μl water
4 μl Sanger buffer
4 μl dNTP mix (0,025 mM each)
10μl
+
5 μl Primer (2pmol/μl)
+
6μl ExoSAP-IT product

Cycling

2 min 96°C, 26 cycles a (30 s/96°C, 15s/55°C, 4 min/60°C)

Purification

A 96 well MultiScreen (Millipore) plate was filled with Sephadex G50 (Amersham) using an appropriate admeasure device. 300μl water were added to each well and incubated 3h at 4°C. Water was removed by spinning for 5minutes at 910g. Cycle sequencing product was loaded to the plate and purified by spinning for 5min at 910g. 10μl of formamide was added to each eluate.

Results:

All PCRs yielded a product.

Figure 12 provides matrices produced from bisulfite sequencing data analyzed by the applicant's proprietary software (See WO 2004/000463 for further information). Each column of the matrices of columns 'A' and 'B' represent the sequencing data for one amplificate. The amplificate number is shown to the left of the matrices. Each row of a matrix represents a single CpG site within the fragment and each column represents an individual DNA sample. The matrices in the column marked 'A' showed below median methylation as measured by QM assays (see example 4), the matrices in the column marked 'B' showed below median methylation as measured by QM assays.

The bar on the left represents a scale of the percent methylation, with the degree of methylation represented by the shade of each position within the column from black representing 100% methylation to light gray representing 0% methylation. White positions represented a measurement for which no data was available.

Bisulfite sequencing indicated differential methylation of CpG sites between the two selected classes of samples, furthermore co-methylation was observed across the gene. In particular amplificates 4 to 7 showed a high level of differential methylation between the two analyzed groups.

Example 8

In order to validate the most promising marker panels from the set of ERBB2, TFF1, PLAU, PITX2, ONECUT, TBC1D3, and ABCA8 Real-Time assays were designed and optimized in order to provide assays of optimum accuracy. The assays were run on a combination of paraffin embedded tissue (hereinafter also referred to as PET) and fresh frozen tissue samples. DNA derived from PET is often of 'lower quality' (e.g. higher degree of DNA fragmentation and low DNA yield from samples), thus confirmation of assay results on PET demonstrates the robustness of the assay and increased utility of the marker.

Quantitative methylation assays were designed for the genes ERBB2, TFF1, PLAU, PITX2, ONECUT, TBC1D3, and ABCA8 and tested using a sample set of 415 estrogen receptor positive node negative samples untreated breast cancer patients and 541 estrogen receptor positive node negative samples Tamoxifen treated samples. Approximately 100 of these samples were previously analyzed in the microarray study.

The QM assay (= Quantitative Methylation Assay) is a Real-time PCR based method for quantitative DNA methylation detection. The assay principle is based on non-methylation specific amplification of the target region and a methylation specific detection by competitive hybridization of two different probes specific for the CG or the TG status, respectively. For the present study, TaqMan probes were used that were labeled with two different fluorescence dyes ("FAM" for CG specific probes, "VIC" for TG specific probes) and were further modified by a quencher molecule ("TAMRA" or "Minor Groove Binder/non-fluorescent quencher").

Evaluation of the QM assay raw data is possible with two different methods:

1. Measuring absolute fluorescence intensities (FI) in the logarithmic phase of amplification
2. Difference in threshold cycles (Ct) of CG and TG specific probe.

Results of this study were generated by using the Ct method.

In the following series of quantitative methylation assays the amount of sample DNA amplified is quantified by reference to the gene GSTP1 to normalize for input DNA. For standardization, the primers and the probe for analysis of the GSTP1 gene lack CpG dinucleotides so that amplification is possible regardless of methylation levels. As there are no methylation variable positions, only one probe oligonucleotide is required.

Sample Sets

ER+ N0 Untreated Population

To demonstrate that the markers identified have a strong prognostic component, ER+ N0 tumor samples from patients not treated with any adjuvant therapy were analyzed. Markers that are able to show a significant survival difference in this population are considered to be prognostic. All 508 samples of this set were obtained from an academic collaborator as cell nuclei pellets (fresh frozen samples). The sample population can be divided into two subsets: One with 415 randomly selected samples (from both censored and relapsing patients), representing a population with a natural distribution of relapses, and additional 93 samples from relapsing patients only. The latter samples were used for sensitivity/specificity analyses only.

Figure 16 shows the disease-free survival of the randomly selected population in a Kaplan-Meier plot and Figure 17 the distribution of follow-up times for the relapsed and censored patients in histograms. Table 6 lists the number of events broken down by different kinds of relapse. In summary, the survival of this population is comparable to the expected one from the literature.

ER+ N0 TAM treated Population

One intended target population of the invention is patients with ER+ N0 tumors that are treated with hormone therapy. To check the performance of the marker candidates in this population, 589 samples from ER+ N0 tumors from patients treated with Tamoxifen were analyzed. All samples were received as Paraffin-embedded tissues (PET). Three to ten 10 μm sections were provided.

In addition, for 89 PET patient samples matching fresh frozen samples from the same tumor were included into the study as controls. As these samples were already used in phase 1, they allowed for two kinds of concordance studies :

- Chip *versus* QM assay
- Fresh frozen *versus* PET samples

Samples of the ER+, N0, TAM treated population were received from eight different providers. Altogether 589 samples were processed, 48 of which had to be excluded from the study due to various reasons (e.g. two samples from same tumor, samples from patients that did not fulfill inclusion criteria etc.).

Figure 18 shows the disease-free survival of the total population in a Kaplan-Meier plot and Figure 19 the distribution of follow-up times for the relapsed and censored patients in histograms. Table 5 lists the number of events broken down by different kinds of relapse. In summary, the survival of this population (82.1 % after 10 years) is comparable to the expected one from the literature (79.2 %).

<u>DNA Extraction</u>

DNA extraction from Fresh Frozen Samples

From a total of 508 fresh frozen samples available as cell nuclei pellets, genomic DNA was isolated using the QIAamp Kit (Qiagen, Hilden, Germany). The extraction was done according to the Cell Culture protocol using Proteinase K with few modifications.

DNA extraction from PET Samples

589 provided PET samples were deparaffinated directly in the tube in which they were delivered by the providers. The tissue was then lysed and DNA extracted using the QIAGEN DNeasy Tissue kit.

<u>Bisulfite treatment</u>

Bisulfite treatment was carried out based on the method disclosed by Olek et al. Nucleic Acids Res. 1996 Dec 15;24(24):5064-6, and optimized to the applicant's laboratory workflow.

Quantification Standards

The reactions are calibrated by reference to DNA standards of known methylation levels in order to quantify the levels of methylation within the sample. The DNA standards were composed of bisulfite treated phi29 amplified human genomic DNA (Promega) (i.e. unmethlyated), and/or phi29 amplified genomic DNA treated with Sss1 Methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only). 2000 ng batches of human genomic DNA (Promega) were treated with bisulfite. To generate methylated MDA DNA, 13 tubes of 4.5 μg MDA-DNA (700ng/μl) was treated with Sss1.

Control assay

The GSTP1-C3 assay design makes it suitable for quantitating DNAs from different sources, including fresh/frozen samples, remote samples such as plasma or serum, and DNA obtained from archival specimen such as paraffin embedded material. The following oligonucleotides were used in the reaction to amplify the control amplificate:

| | |
|---|---|
| Control Primer1 : | GGAGTGGAGGAAATTGAGAT (SEQ ID NO: 104) |
| Control Primer2: | CCACACAACAAATACTCAAAAC (SEQ ID NO: 105) |
| Control Probe: | FAM-TGGGTGTTTGTAATTTTTGTTTTGTGTTAGGTT-TAMRA (SEQ ID NO: 106) |

| | |
|---|---|
| Cycle program (40 cycles): | 95 °C, 10 min |
| | 95 °C, 15 sec |
| | 58 °C, 1 min |

Assay design and reaction conditions

Two assays were developed for the analysis of the gene PITX2(SEQ ID NO: 23) Assay 1:

Primers: GTAGGGGAGGGAAGTAGATGTT (SEQ ID NO:107)

TTCTAATCCTCCTTTCCACAATAA (SEQ ID NO:108)
Probes: FAM-AGTCGGAGTCGGGAGAGCGA-TAMRA (SEQ ID NO:109)
VIC-AGTTGGAGTTGGGAGAGTGAAAGGAGA -TAMRA (SEQ ID NO:110)

Amplicon (SEQ ID NO:111):

GtAGGGGAGGGAAGtAGATGttAGCGGGtCGAAGAGTCGGGAGtCGGAGtCGGGA-GAGCGAAAGGAGAGGGGAttTGGCGGGGtAtTTAGGAGttAAtCGAGGAGtAGGAG-tACGGAtTttAtTGTGGAAAGGAGGAttAGAA

Length of fragment: 143 bp

Positions of primers, probes and CpG dinucleotides ar highlighted.

PCR components (supplied by Eurogentec) : 3 mM MgCl2 buffer, 10x buffer, Hotstart TAQ, 200 μM dNTP, 625 nM each primer, 200 nM each probe

Cycle program (45 cycles): 95 °C, 10 min
95 °C, 15 sec
62 °C, 1 min

Assay 2 :

Primers: AACATCTACTTCCCTCCCCTAC (SEQ ID NO: 112)
GTTAGTAGAGATTTTATTAAATTTTATTGTAT (SEQ ID NO: 113)
Probes: FAM-TTCGGTTGCGCGGT-MGBNQF (SEQ ID NO: 114)
VIC-TTTGGTTGTGTGGTTG- MGBNQF (SEQ ID NO: 115)

Amplicon (SEQ ID NO: 116):

GTtAGtAGAGATTttAttAAAtTttAtTGtA-tAGTGGCGCGCGGGCGGtCGGtCGAGttCGGtTGCGCGGtTGGCGATttAGGAGC-GAGtAtAGCGttCGGGCGAGCGtCGGGGGGAGCGAGtAGGGGCGACGAGAAAC-GAGGtAGGGGAGGGAAGtAGATGtt

Length of fragment: 164 bp

The positions of probes, primers and CpG positions are highlighted.

The probes cover three co-methylated CpG positions.

PCR components (supplied by Eurogentec): 2,5 mM MgCl2 buffer, 10x buffer, Hotstart TAQ, 200 μM dNTP, 625 nM each primer, 200 nM each probe

Program (45 cycles): 95 °C, 10 min
95 °C, 15 sec
60 °C, 1 min

The extent of methylation at a specific locus was determined by the following formulas:

Using absolute fluorescence intensity: methylation rate= 100 * I (CG) / (I(CG) + I(TG))
(I = Intensity of the fluorescence of CG-probe or TG-probe)

Using threshold cycle Ct: methylation rate= 100*CG/(CG+TG)= 100/(1+TG/CG)= 100/(1+2^delta(ct))

(assuming PCR efficiency E=2; delta (Ct)= Ct (methylated) - Ct (unmethylated) )

Gene PLAU

Primer: GTTAGGTGTATGGGAGGAAGTA (SEQ ID NO: 117)
TCCCTCCCCTATCTTACAA (SEQ ID NO: 118)
Probes: FAM-ACCCGAACCCCGCGTACTTC-TAMRA (SEQ ID NO: 119)
VIC-ACCCAAACCCCACATACTTCCACA-TAMRA (SEQ ID NO: 120)

Amplicon (SEQ ID NO: 121):
GttAGGTGtATGGGAGGAAGtA**CG**GAGAATTTAtAAGttTt**CG**ATTttTtAGTttA-
GA**CG**tTGTTGGGTttttTtCGtTGGA-
GATCGCGtTTtttttAAATtTTTGTGAG**CG**TTG**CG**GAAG-
tA**CG**C**G**GGGTtC**G**GGT**CG**tTGAG**CG**tTGtAAGAtAGGGGAGGGA

Length of fragment: 166 bp

The positions of probes, primers and CpG positions are highlighted.

PCR components were supplied by Eurogentec : 2,5 mM MgCl2 buffer, 10x buffer, Hotstart TAQ, 200 µM dNTP, 625 nM each primer, 200 nM each probe

Program (45 cycles): 95 °C, 10 min
95 °C, 15 sec
60 °C, 1 min

Gene ONECUT2

Primer: GTAGGAAGAGGTGTTGAGAAATTAA (SEQ ID NO: 122)
CCACACAAAAAATTTCTATACTCCT (SEQ ID NO:123)
Probes: FAM- ACGGGTAGAGGCGCGGGT -TAMRA (SEQ ID NO: 124)
VIC- ATGGGTAGAGGTGTGGGTTATATTGTTTTG-TAMRA (SEQ ID NO:125)

Amplicon (SEQ ID NO:126):

GtAGGAAGAGGTGtTGAGAAATTAAAAATTtAGGTTAGTTAATG-tATtttTGtCGtCGGtTGtAGGtTtCGttTTTGtAT-TAAGCGGGCGtTGATTGTGCGCGttTGGCGAtCGCGGGGAG-GAtTGGCGGttCGCGGGAGGGGACGGGTAGAGGCGCGGGTTA-

tATTGTTtTGGAGtCGGtTCGGtTtTTTGTGttTttTtTAGCGGttAAGtTGCGAGGTA-tAGtttTtTATTGTTtTAGGAGtAtAGAAAttTttTGTGTGG

Length of fragment: 266 bp

The positions of probes, primers and CpG positions are highlighted.

PCR components were supplied by Eurogentec : 3 mM MgCl2 buffer, 10x buffer, Hotstart TAQ, 200 µM dNTP, 625 nM each primer, 200 nM each probe

Program (45 cycles): 95 °C, 10 min
95 °C, 15 sec
60 °C, 1 min

Gene ABCA8

| | |
|---|---|
| Primer: | GTGAGGTATTGGATTTAGTTTATTTG (SEQ ID NO: 127) |
| | CCCTAAATCTCATCCTAAAAACAC (SEQ ID NO: 128) |
| Probes: | FAM- TGAGGTTTCGGTTTTTAACGGTGG -TAMRA (SEQ ID NO: 129) |
| | VIC- TGAGGTTTTGGTTTTTAATGGTGGGAT -TAMRA (SEQ ID NO: 130) |

Amplicon (SEQ ID NO: 131):

GTGAGGTAtTGGATT-tAGtttATTTGGtttCGAAGttTtTGTTtTCGGAATtCGGGTGtTGTGGGTTGAGGTttCGGTTttTAACGGTGGGAtTGGTGTttTCGAGAT-GAAATTTGGGGTTTttTCGGGGtTTTGGTGGGATCGGTGTttTtAGGATGAGATT-TAGGG

Length of fragment: 168 bp

The positions of probes, primers and CpG positions are highlighted.

PCR components were supplied by Eurogentec : 3 mM MgCl2 buffer, 10x buffer, Hotstart TAQ, 200 µM dNTP, 625 nM each primer, 200 nM each probe

Program (45 cycles): 95 °C, 10 min
95 °C, 15 sec
62 °C, 1 min

Gene ERBB2

Primer: GGAGGGGGTAGAGTTATTAGTTTT (SEQ ID NO: 134)
ACTCCCAACTTCACTTTCTCC (SEQ ID NO: 135)
Probes: FAM- TAATTTAGGCGTTTCGGCGTTAGG -TAMRA (SEQ ID NO: 136)
VIC- TAATTTAGGTGTTTTGGTGTTAGGAGGGA -TAMRA (SEQ ID NO:137)

Amplicon (SEQ ID NO:138):

GGAGGGGGTAGAGTTATTAGTTTTTGTATTTAGGGATTTTTCGAGGAAAAGTGTG
AGAACGGTTGTAGGTAATTTAGGCGTTTCGGCGTTAGGAGGGACGTATTTAGGT
TTGCGCGAAGAGAGGGAGAAAGTGAAGTTGGGAGT

Length of fragment: 144 bp

The positions of probes, primers and CpG positions are highlighted.

PCR components were supplied by Eurogentec: 2,5 mM MgCl2 buffer, 10x buffer, Hotstart TAQ, 200 µM dNTP, 625 nM each primer, 200 nM each probe

Program (45 cycles): 95 °C, 10 min
95 °C, 15 sec
62 °C, 1 min

Gene TFF1

Primer: AGTTGGTGATGTTGATTAGAGTT (SEQ ID NO: 139)
CCCTCCCAATATACAAATAAAAACTA (SEQ ID NO: 140)
Probes: FAM- ACACCGTTCGTAAAA-MGBNFQ (SEQ ID NO: 141)
VIC- ACACCATTCATAAAAT-MGBNFQ (SEQ ID NO: 142)

Amplicon (SEQ ID NO: 143):

AGTTGGTGATGTTGATTAGAGTTTTTGTAGTTTTAAATGATTTTTTTAATTAATTTT
AAATTTTTAGAATTTATCGTATAAAAAGGTTATATTTTTTGGAGGGACGTCGATG
GTATTAGGATAGAAGTATTAGGGGATTTTACGAACGGTGTCGTCGAAATAGTAG
TTTTTATTTGTATATTGGGAGGG

Length of fragment: 189 bp

The positions of probes, primers and CpG positions are highlighted.

PCR components were supplied by Eurogentec: 2,5 mM MgCl2 buffer, 10x buffer, Hotstart TAQ, 200 µM dNTP, 625 nM each primer, 200 nM each probe

Program (45 cycles): 95 °C, 10 min
95 °C, 15 sec
60 °C, 1 min

Gene TBC1D3

Primer: TTTTTAGTTGGTTTTTATTAGGGTTTT (SEQ ID NO: 144)
CCAACATATCCACCCACTTACT (SEQ ID NO: 145)
Probes: FAM- TTTCGACTAATCTCCCGCCGA-TAMRA (SEQ ID NO: 146)
VIC- TTTCAACTAATCTCCCACCAAATTTACTATCA-TAMRA (SEQ ID NO: 147)

Amplicon(SEQ ID NO: 148):

tTTttAGtTGGtTttAttAGGGtTttAGAGtttAAGAtttAG-
tATt**CG**C**G**GG**CG**GtTtTGGGAAGttTGGtAGtTt**CG**tTAAtTttAAtATGttTtATTTGAtAG-
tAAATT**CG**G**CG**GGAGATtAGt**CG**AAAGAGtAAGTGGGTGGATATGtTGG

Length of fragment: 142 bp
The positions of probes, primers and CpG positions are highlighted.
PCR components were supplied by Eurogentec: 4,5 mM MgCl2 buffer, 10x buffer, Hotstart TAQ, 200 μM dNTP, 625 nM each primer, 200 nM each probe
Program (45 cycles): 95 °C, 10 min; 95 °C, 15 sec; 60 °C, 1 min
Each of the designed assays was tested on the following sets of samples:

- Tamoxifen treated patients who relapsed during treatment (all relapses).
- Tamoxifen treated patients who relapsed during treatment with distant metastases only.
- Non-Tamoxifen treated patients who relapsed during treatment (all relapses).
- Non-Tamoxifen treated patients who relapsed during treatment with distant metastases only.

Raw Data Processing

All analyses were based on CT evaluation (evaluation using fluorescence intensities are available upon request). Assuming optimal real-time PCR conditions in the exponential amplification phase, the concentration of methylated DNA ($C_{meth}$) can be determined by

$$C_{meth} = \frac{100}{1+2^{(CT_{CG}-CT_{TG})}}[\%],$$

where
$CT_{CG}$ denotes the threshold cycle of the CG reporter (FAM channel) and
$CT_{TG}$ denotes the threshold cycle of the TG reporter (VIC channel).
The thresholds for the cycles were determined by human experts after a visual inspection of the Amplification Plots [ABI PRISM 7900 HT Sequence Detection System User Guide]. The values for the cycles ($CT_{CG}$ and $CT_{TG}$) were calculated with these thresholds by the ABI 7900 software. Whenever the amplification curve did not exceed the threshold, the value of the cycle was set to the maximum cycle, i.e. 50.

Statistical Methods

Cox Regression

The relation between disease-free survival times (DFS) (or metastasis free survival, MFS) and covariates are modeled using Cox Proportional Hazard models (Cox and Oates, 1984; Harrel, 2001). The hazard, i.e. the instantaneous risk of a relapse, is modeled as

$$h(t \mid x) = h_0(t) \cdot exp(\beta x) \quad (3)$$

and

$$h(t \mid x_1, \ldots, x_k) = h_0(t) \cdot exp(\beta_1 x_1 + \ldots + \beta_k x_k) \quad (4)$$

for univariate and multiple regression analyses, respectively, where $t$ is the time measured in months after surgery, $h_0(t)$ is the baseline hazard, x is the vector of covariates (e.g. measurements of the assays) and β is the vector of regression coefficients (parameters of the model). β will be estimated by maximizing the partial likelihood of the Cox proportional hazard model Likelihood ratio tests are performed to test whether methylation is related to the hazard. The difference between 2Log(Likelihood) of full model and null-model is approximately □$^2$-distributed with $k$ degrees of freedom under the null hypotheses $□_1 = ... = □_k = 0$.

The assumption of proportional hazards were checked by scaled Schoenfeld residuals (Thernau *et al.,* 2000).

For the calculation, analysis and diagnostic of the Cox Proportional Hazard Model the R functions coxph, coxph.zph of the "survival" package were used.

Stepwise Regression Analysis

For multivariate Cox regression models a stepwise procedure (Venables *et al.,* 1999; Harrel, 2001) was used in order to find sub-models including only relevant variables. Two effects are usually achieved by these procedures:

- Variables (methylation rates) that are basically unrelated to the dependent variable (DFS/MFS) are excluded as they do not add relevant information to the model.
- Out of a set of highly correlated variables, only the one with the best relation to the dependent variable is retained.

Inclusion of both types of variables can lead to numerical instabilities and a loss of power. Moreover, the predictory performance can be low due to overfitting.

The applied algorithm aims at minimizing the Akaike information criterion (AIC) which is defined as

AIC = 2□maximized log-likelihood + 2□#parameters.

The AIC is related to the predictory performance of a model, smaller values promise better performance. Whereas the inclusion of additional variables always improves the model fit and thus increases the likelihood, the second term penalizes the estimation of additional parameters. The best model will present a compromise model with good fit and usually a small or moderate number of variables.

Stepwise regression calculation with AIC was done with the R function "step".

Kaplan-Meier Survival Curves and Log-Rank Tests

Survival curves are estimated from DFS/MFS data using the Kaplan-Meier method (Kaplan and Meier, 1958). Log-rank tests were used to test for differences of two survival curves, e.g. survival in hyper- vs. hypomethylated groups. For a description of this test see (Cox and Oates, 1984).

For the Kaplan Meier Analysis the functions "survfit" and "survdiff' of the "survival" package were used.

Independence of markers from other covariates

To check whether our marker panel gives additional and independent information, other relevant clinical factors were included in the cox proportional hazard model and the p-values for the weights for every factor were calculated (Wald-Test) (Thernau *et al.,* 2000). For the analysis of additional factors in the Cox Proportional Hazard model, the R function "coxph" was used.

Correlation Analysis

Pearson and Spearman correlation coefficients are calculated to estimate the concordance between measurements (e.g. methylation in matched fresh frozen and PET samples).

Density Estimation

For numerical variables, kernel density estimation was performed with a gaussian kernel and variable bandwidth. The bandwidth is determined using Silverman's "rule-of-thumb" (Silverman, 1986). For the calculation of the densities the R function "density" was used.

Analysis of Sensitivity and Specificity

For the analysis of sensitivity and specificity of single assays and marker panels ROCs were calculated. The calculation of the ROCs was done with two methods: The first method is to calculate sensitivity and specificity for a given threshold for the time $T_{Threshold}$. With that threshold, true positives, false positives, true negatives and false negatives were defined and the values for sensitivity and specificity were calculated for different cutoffs of the model. Patients censored before $T_{Threshold}$ were excluded. The ROCs were calculated for different times $T_{Threshold}$ (3 year, 4 years, ..., 10 years). The second method is to calculate sensitivity and specificity by using the Bayes-formula based on the Kaplan-Meier estimates (Heagerty *et al.,* 2000) for the survival probabilities in the marker positive and marker negative groups for a given time $T_{Threshold}$. The ROCs were calculated for different times $T_{Threshold}$ (3 year, 4 years, ... , 10 years).

k-fold Crossvalidation

For the analysis of model selection and model robustness k-fold crossvalidation (Hastie *et al.,* 2001) was used. The set of observation was split in k chunks by random. In turn, every chunk was used as a test set and the remaining k-1 chunks were used as training set. This procedure was repeated n times.

Population Charts

For the description of the relation between censoring and a covariate Population Charts (Möcks *et al.,* 2002) were used. The baseline of the covariate was calculated including all observations with event. For a given time t, the mean (in case of real variables like age) or the fraction (in case of categorical variables) for all censored patients in the risk set at time t was calculated and added to the baseline value.

Technical Performance

Comparison of Assay Replicates

Each marker was measured in at least three replicates, variability between assay replicates was observed to be higher for PET than for fresh frozen samples.

Concordance Study Fresh Frozen *versus* PET Samples

Markers analyzed in this study (Example 2)were initially identified on a chip platform (Example 1) using fresh frozen samples. The ER+ N0 untreated population was also analyzed on fresh frozen samples in Example 2. A concordance study should demonstrate that measured methylation ratios are comparable for fresh frozen and PET samples. For this purpose, 89 fresh frozen samples from three different providers already used in the chip study were processed again in parallel with a matching PET sample originating from the same tumor.

Figure 15 shows such a concordance study for marker candidate PITX2 assay 1 as a scatter plot between fresh frozen and PET samples (using the QM assay). The association between the paired samples is 0.81 (Spearman's rho). This analysis is based on n=89 samples.

Results

Evaluation of Single Markers

Each of the eight established QM assays was used to measure the 508 samples from the N0, ER+ untreated patient population (random selection and additional relapses) in three replicates. After filtering of measuring points not fulfilling quality criteria and performing a Cox analyses, Kaplan-Meier survival curves and ROC curves for each single marker were generated.

Two different clinical endpoints were used for analyses:

- Disease-free survival, i.e. using all kinds of relapses (distant metastasis, loco-regional relapses, relapses at contralateral breast) as event.
- Metastasis-free survival, i.e. treating only distant metastasis as an event.

For analyzing the ER+, N0, TAM treated population, five marker candidates were analyzed on 541 samples from the N0, ER+ untreated patient population. Assays were measured in three replicates. Three assays that were measured on the untreated population (PITX-2, ONECUT, and ABCA8) were not measured due to the limited material that was available for the TAM treated population. These assays were rejected either because they performed bad in the untreated population (ONECUT and ABCA8) or in case of PITX2-II it performed significantly worse than the other assay of this marker (PITX2-I). After filtering of measuring points not fulfilling quality criteria Kaplan-Meier survival curves and ROC curves for each single marker were generated.

Two different clinical endpoints were used:

- Disease-free survival, i.e. using all kinds of relapses (distant metastasis, locoregional relapses, relapses at contralateral breast) as event.
- Metastasis-free survival, i.e. treating only distant metastasis as an event.

The Kaplan-Meier estimated disease-free survival or metastasis-free survival curves of each single assay are shown in Figures 14 to 39, and combinations of assays are shown in Figures 40 to 55. The X axis shows the disease free survival times of the patients in years, and the Y-axis shows the proportion of patients with disease free survival. The black plot shows the proportion of disease free patients in the population with above an optimized cut off point's methylation levels, the gray plot shows the proportion of disease free patients in the population with below an optimized cut off point's methylation levels.

The following p-values (probability that the observed distribution occurred by chance) were calculated when the cut off was optimized. For cut-off optimization, the quantiles of both groups were shifted between 0.2 and 0.8 and the p-value for the separation of the curves was calculated for each quantile. The quantile with the lowest p-value was then the best cut-off. Percentage values refer to the methylation ratios at the cut-off point.

<u>Single gene assays</u>

<u>Tamoxifen treated</u>

TAM treated (all relapses) ERBB2 (Figure 14) : p-value 0.089; cut off point: 1.3%
TAM treated (distant only) ERBB2 (Figure 15): p-value 0.084; cut off point: 0.1%
TAM treated (all relapses) TFF1 (Figure 16): p-value 0.037; cut off point: 50.9%
TAM treated (distant only) TFF1 (Figure 17): p-value 0.029; cut off point: 52.9%
TAM treated (all relapses) PLAU (Figure 18): p-value 0.056; cut off point: 4.8%
TAM treated (distant only) PLAU (Figure 19): p-value 0.065; cut off point: 4.8%
TAM treated (all relapses) PITX2 (Figure 20): p-value 0.01; cut off point: 13.1%
TAM treated (distant only) PITX2 (Figure 21): p-value 0.0012; cut off point: 14.3%
TAM treated (all relapses) TBC1D3 (assay II) (Figure 22): p-value 0.28; cut off point: 94.6%
TAM treated (distant only) TBC1D3 (assay II) (Figure 23): p-value 0.078; cut off point: 97%

Figure 62 shows the ROC plot at different times for marker model PITX2 (Assay 1) alone on ER+N0 TAM treated population. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates, and the estimated area under the curve (AUC) is calculated. Values for median cut off (triangle) and best cut off (diamond, 0.42 quantile) are plotted.

AUC 60 months: 0.6
AUC 72 months: 0.69
AUC 84 months: 0.69
AUC 96 months: 0.67

Figure 63 shows the ROC plot at different times for marker model TFF1 on ER+N0 TAM treated population. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates for different thresholds (= 5, 6, 7 , 8 years) and the estimated area under the curve (AUC) is calculated. Values for median cut off (triangle) and best cut off (diamond, 0.78 quantile) are plotted.

AUC 60 months: 0.7
AUC 72 months: 0.65
AUC 84 months: 0.61
AUC 96 months: 0.64

Figure 64 shows the ROC plot at different times for marker model PLAU on ER+N0 TAM treated population. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates for different thresholds (= 5, 6, 7 , 8 years), and the estimated area under the curve (AUC) is calculated. Values for median cut off (triangle) and best cut off (diamond, 0.77 quantile) are plotted.

AUC 60 months: 0.6
AUC 72 months: 0.63
AUC 84 months: 0.57
AUC 96 months: 0.6

Non Tamoxifen treated

Non Tamoxifen treated (all relapses) ERBB2 (Figure 24): p-value 0.21; cut off point: 0%;
Non Tamoxifen treated (distant only) ERBB2 (Figure 25): p-value 0.23; cut off point: 0.6%,
Non Tamoxifen treated (all relapses) TFF1 (Figure 26) : p-value 0.012; cut off point: 49.6%;
Non Tamoxifen treated (distant only) TFF1 (Figure 27): p-value 0.016; cut off point: 45.4%;
Non Tamoxifen treated (all relapses) PLAU (Figure 28): p-value 0.011; cut off point: 3.2%;
Non Tamoxifen treated (distant only) PLAU (Figure 29): p-value 0.0082; cut off point: 5.5%;
Non Tamoxifen treated (all relapses) PITX2 (I) (Figure 30): p-value 1.4e-06; cut off point: 35.4%;
Non Tamoxifen treated (distant only) PITX2 (I) (Figure 31): p-value 1.7 e-05; cut off point: 41.2%;
Non Tamoxifen treated (all relapses) PITX2 (II) (Figure 32): p-value 0.00026; cut off point: 56.1%;
Non Tamoxifen treated (distant only) PITX2 (II) (Figure 33): p-value 0.0026; cut off point: 61.9%;
Non Tamoxifen treated (all relapses) ONECUT (Figure 34): p-value 0.26; cut off point: 0%;
Non Tamoxifen treated (distant only) ONECUT (Figure 35): p-value 0.77; cut off point: 0%;
Non Tamoxifen treated (all relapses) TBC1D3 (Figure 36): p-value 0.004; cut off point: 98.6%;
Non Tamoxifen treated (distant only) TBC1D3 (Figure 37): p-value 0.00022; cut off point: 98.6%;
Non Tamoxifen treated (all relapses) ABCA8 (Figure 38): p-value 0.0065; cut off point: 60.9%;
Non Tamoxifen treated (distant only) ABCA8 (Figure 39): p-value 0.15; cut off point: 49.2%

Panels

Based on the results of the single marker evaluations, it was decided to build models using the marker candidates PITX2-Assay I, TFF1, and PLAU. All possible combinations of these markers were evaluated.

Tamoxifen treated

TAM treated (all relapses) TFF1 & PLAU (Figure 40): p-value 0.023; cut off point: 0.7 quantile;
TAM treated (distant only) TFF1 & PLAU (Figure 41): p-value 0.00084; cut off point: 0.72 quantile;

TAM treated (all relapses) TFF1 & PLAU & PITX2 (Figure 42): p-value 0.037; cut off point: 0.72 quantile;
TAM treated (distant only) TFF1 & PLAU & PITX2 (Figure 43): p-value 0.0014; cut off point: 0.4 quantile;
TAM treated (all relapses) PITX2 & TFF1 (Figure 44): p-value 0.17; cut off point: 0.78 quantile;
TAM treated (distant only) PITX2 & TFF1 (Figure 45): p-value 0.0048; cut off point: 0.32 quantile;
TAM treated (all relapses) PITX2 & PLAU (Figure 46): p-value 0.1; cut off point: 0.74 quantile;
TAM treated (distant only) PITX2 & PLAU (Figure 47): p-value 0.0081; cut off point: 0.44 quantile.

Figure 61 shows the ROC plot at different times for marker model PITX2 (Assay 1) and TFF1 on ER+N0 TAM treated population. Figure A shows the plot at 60 months, figure B shows the plot at 72 months, figure C shows the plot at 84 months and figure D shows the plot at 96 months. Only distant metastasis are defined as events. Sensitivity (proportion of all relapsed patients in poor prognostic group) shown on the X-axis and specificity (proportion of all relapse free patients in good prognostic group) shown on the Y-axis are calculated from KM estimates, and the estimated area under the curve (AUC) is calculated. Values for median cut off (triangle) and best cut off (diamond, 0.32 quantile) are plotted.

AUC 60 months: 0.62
AUC 72 months: 0.67
AUC 84 months: 0.63
AUC 96 months: 0.65

Non Tamoxifen treated

Non Tamoxifen treated (all relapses) TFF1 & PLAU (Figure 48): p-value 0.0015; cut off point: 0.78 quantile;
Non Tamoxifen treated (distant only) TFF1 & PLAU (Figure 49): p-value 0.003; cut off point: 0.8 quantile;
Non Tamoxifen treated (all relapses) TFF1 & PLAU & PITX2 (Figure 50): p-value 8.9e-07; cut off point: 0.64 quantile;
Non Tamoxifen treated (distant only) TFF1 & PLAU & PITX2 (Figure 51): p-value 5.4e-05; cut off point: 0.66 quantile;
Non Tamoxifen treated (all relapses) PITX2 & TFF1 (Figure 52): p-value 1.9e-06; cut off point: 0.72 quantile;
Non Tamoxifen treated (distant only) PITX2 & TFF1 (Figure 53): p-value 3.5e-05; cut off point: 0.76 quantile;
Non Tamoxifen treated (all relapses) PITX2 & PLAU (Figure 54): p-value 1.1e-06; cut off point: 0.68 quantile;
Non Tamoxifen treated (distant only) PITX2 & PLAU (Figure 55): p-value 1.5e-05; cut off point: 0.64 quantile.

Robustness of marker models

To evaluate the robustness of the models, a crossvalidation was performed on model marker panel PITX2 (Assay 1) plus TFF1 and marker panel PITX2 (Assay 1) alone, with 200 replicates. The stability of the assignment of one certain patient to the bad or good outcome group is illustrated in Figure 65, the left hand figure shows model marker panel PITX2 (Assay 1) plus TFF1 and the right hand figure shows model marker panel PITX2 (Assay 1) alone. The plot illustrates in how many crossvalidation replicates each patient get's assigned to group 1 (light gray) or group 2 (dark gray).

Table 4: Numbers of censored and relapsed patients in randomly selected sample set of ER+, N0, untreated population.

| | **Frequency** | **Percentage** |
|---|---|---|
| Censored | 276 | 66.5 |
| Distant metastasis | 66 | 15.9 |
| Locoregional relapse | 49 | 11.8 |
| Contralateral breast | 24 | 5.8 |
| **Sum** | **415** | **100.0** |

Table 5: Numbers of censored and relapsed patients in ER+, N0, TAM treated population.

| | **Frequency** | **Percentage** |
|---|---|---|
| Censored | 485 | 89.6 |

(continued)

| | Frequency | Percentage |
|---|---|---|
| Distant metastasis | 31 | 5.7 |
| Locoregional relapse | 20 | 3.7 |
| Contralateral breast | 5 | 0.9 |
| **Sum** | **541** | **100.0** |

Sequence listing <110> Epigenomics AG

<120> PROGNOSTIC MARKERS FOR PREDICTION OF TREATMENT RESPONSE AND/OR SURVIVAL OF BREAST CELL PROLIFERATIVE DISORDER PATIENTS.

<130> FB14771

<160> 158

<210> 1
<211> 9001
<212> DNA
<213> Homo Sapiens

<400> 1

```
agctgatgga cttgctaaat ttctttcttc ttttttcttt ttcatattat ttgctagcca     60
taatggaatc ctctaggttt aagccaaaga aaaattggag agacaaaatt agattttgta    120
gccctttcc cccccgggaa tgccttttt tttcttttta gtttctgatg aatggctatc    180
atttatttct accaaattta aataaggact gctgccttgt atgtttaact aggcaggcag    240
agggaactgg tttgtttagg aagcagtgac tgagatgtcc tggccaagtt agtgacagag    300
gaggggagaa agaatccaga ccaatttgta tgcagtatat tttactccca tgaaataaaa    360
cacatttgtt tcatatttgc tgaaaagtaa aacaataata ttgtacgaaa tgttatacac    420
agggtaggtt gtacatagca gtttcagaaa catcattgca tccaccagag aaactattct    480
aaaactgata ttcacacatt ttttataata ataataatat gttagaaaca tacagtgtgg    540
catttagtat atacactccc ttgctcgcaa gcgaaaaatc ctaatcgctt ctgtataaca    600
tgctttattt taaagcctaa cctttaaaaa cactgttgtg atattactaa caactgcttt    660
tataaaatta atttgacatt tcgatatata tacatccttt cagtcattta aatgttaaca    720
atgctaaact taaaaaataa caagcttata gtaatgttaa aatgtcatat ccagtcaaac    780
atttgtttgt gtatgtgtcc ttgcaactgt tagaaatact tgtagtgaaa gatgtcagac    840
actgaggaca tccctttgaa atcaaaggag ctctctcttt gattcagtgg tttccttttc    900
tctatatagc ttctctttct ctccctttct ttagtgccca cgaccttcta gcataattcc    960
cagtctttca agggcggagt tgccccatcc ggcaaggtcc taggatcccg gcgctgtggg   1020
tgcggctcac acgggccggt ccactgcata ctggcaagca ctcaggttgg aggccgggtt   1080
ctgcacgctg gcgtagccga agctggagtg ctgctttgct ttcagtctca ggctggccag   1140
gctcgagtta cacgtgtccc tataaacata cggaggagtc ggcggcgcgt aaggacaggc   1200
aggcgtcggc accgcggaat tcagcgacgg gctactcagg ttgttcaagt tattcaggct   1260
gttgagactg gagcccggga cgcctgtcac tgctgagggc accatgctgg acgacatgct   1320
catggacgag atagagttgg gtggggaaaa catgctctgt gatgacaggg ggttgacgtt   1380
catagagttg aagaagggga agctcttggt ggatagggag gcggatgtaa ggcccttggc   1440
ggcccagttg ttgtaggaat agcctgggta catgtcgtcg tagggctgca tgagcccatt   1500
gaactgcggc ccgaagccat tcttgcatag ctcggcctgc tggttgcgct ccctctttct   1560
ccatttggcc cgacgattct tgaaccaaac ctgggggcgg ttggggcaag ggagcaaaca   1620
gatgccacag tgcagattac taaaacttcc atcggaggcc aacccccgcc ttcccccgac   1680
acacacgcta gcgcactcac acaccctggc ctcgcttcac tgcaccgccc tgcacaccaa   1740
gataccaggg ccagctttca gttactggcc cgggtctcca ccaagcgcag gagacctggt   1800
ctgctctggc ctgcgagctg ggactcggag ctacgccaca aacctcagcc gaacgcatgg   1860
agacctgcgg acggtttgat cactcagcca ggcgtttctc caggtccaaa aacacttaat   1920
gtaaaacaaa cgcgggggcag caggcttttc caacccttcc cggggcacct tgcaaacttg   1980
cttccattcc aaagccacag acccacggat gaggagaagg ggctggaagg gcactagagg   2040
atcgctcttt ctcccacgca attcctccct tccttccctg acctccactg tcgtccccca   2100
cccccctggta cgtgctccct taacagggac taggccgcca acactctttc tcgcctagca   2160
aaacaaccaa ataaagagca aaagaccacc tcttcgtcag ctcgttaact ccaggagctt   2220
ggcatattaa actccgggaa cccggaaagg gtagttttgg agattccccc ttctttcgct   2280
ctgcctcttc tttaccctaa gcccaccaca ggcctgtccg cgcgccaggc ccagccgggt   2340
cgtttggctt tgcaggcggc cacccaggcc ggccggcttc cacccgtgtc cggtggccca   2400
gccgcaaccc cgatcccaat ccacatcggg cctccctgtc gccccagacg gcggcttttg   2460
tgtattggag agaggcctgg cctgagatat ccgagctgac accagtgatg tttcacatta   2520
cacatctccg ccgggcccag ccgtgtaatc cgctttttct ctttttcctt tcattcttga   2580
tttccttttt atccccccttc ctctttgcac ccgactgcta taaaaagcac gcctcactcc   2640
cacttggctc gacaagcagc cgccctggaa ggagaggcag ctgcaaggag agcccagcgc   2700
```

cgcggctaca aagcactagg gtggagctgc ggaatagcgg gcggggtggg agggcgtttt 2760
cgaaggatcc cagaaaaccc atagactctg tctttaatta cttgccattt ctaccctagg 2820
ccatctaaac tttgctcagg cgagaagagt acgtgagagg cccgttccct tgatgtgcaa 2880
gagagctaat gaaagactga ccttgctcaa aaccacgccg cccaggaccc agctctggct 2940
ctggacagtt aaactaaaac cattttcaac ttcttcccgg ccttttatcc accagcatag 3000
cctcatgcct tgcacaaatg ccacccagag agtgtcttca ttccctctga tttgggagag 3060
cattttggtc tttattcttt ttatcgttgt tttcttcttt ttgtttgctc tgctctaacc 3120
gggggcttta tttttctac ccagagcact taattttttt tttttaacag caaagcctct 3180
ggatgccgct tgatttgctt gattctgttt tctgcttcca gaatcctaac aaatttggaa 3240
tcttccaccg accagcataa accaggacgt tgctattggg ttatttattt gagctcattt 3300
ttgccaatcc ataaagtaca gatttgctac aaagttaagg taagcccttt ttacaaaact 3360
atgattataa tttagaagag ggggtgtgag tttcaatttc cagagttcaa ctcctgagag 3420
aagataaata aaccaagcag aaaagtcttt cttctttttt tctttctcct tctaagagga 3480
ctagtagttg tgtattaaaa ctttgctccc ggagatcaca aaactaggaa atagggtgtg 3540
tgggagagac ctgaatggcc gaaacaaccg taaagaaggt gtaagaagcg cgagcccagg 3600
agggaaaaag ctgggccagg gccgggacaa aggtttccca gggagggcca actcttccgt 3660
gtctctggcg ggttttcctt gttaaaggct cacaggttgg agcctgttcg cggctcttgg 3720
cctggtaggg atttattag ctctgctctg gcaactgcaa gccaggaaca caatgtcctg 3780
tgcaggggat tgcccatgca gcccagctcg tgagatcgcg ggatggcggg gcagtgagcc 3840
ggtgccgctc tgggagcctg agccagggcg gcagtcctgt cggcctcgga gagggaactg 3900
taatctcgca accaggccgc cgcgaggcct tctgcctttg caaagctgcg ccccaccggc 3960
gccctcccag gcggcgctgc cttccacatt ctctcctggt ctacttggcc tgtacctcca 4020
caacatcctc cccccatccc tcccagactc cgtgctggct cctacccgga ctcgggcttc 4080
cgtaaggttg gtccacacag cgatttcttc gcgtgtggac atgtccgggt agcggttcct 4140
ctggaaagtg gcctccagct cctggagctg ctggctggta aagtgagtcc gctgccgcct 4200
ttgccgcttc ttcttagacg ggtcctcggc gcccacgtcc tcattcttcc cctgctggct 4260
tttatctttc tctgaaaacg aaacacacac actttcccgt cagcatgccc acctgcaacg 4320
cggacgccaa ctggaccggc ggcagaagcc gtggaagagc tgggctgcct ggcgccggag 4380
gagggtgcgc gcggcggctc cgggccgcga ggagcgctgc gcctgtgggg tgtgcaggcg 4440
caagtgtggg tgtccgcgcc ccatttcctc ccctccccca gcgccgcacg ttttatttac 4500
atgtttatct cactgcagcg gcacattcac ttttatagcc tgtgctttca agtatattta 4560
tacacctctg cgcagacaca ccaaatctcc tgggacgcgc acacgcgcgt ggtttacaga 4620
ccccccctccc cctcgcagaa agctcagatt tccatgcggt ttgggaaggc taggaaaaga 4680
tgtggggatt cggttgggca ccgaagttcg ccggcccttt cccaaaaaaa aaaaaaaaat 4740
gcctcttcgc gaagggcatt tctgagtggt ttcaggcaat ttcctaacga gtggagctcc 4800
tcgggagctg aaagccgaga ggaaaacagg gacagaggtc ggcggcctct gaaggtcctc 4860
gaatcaagat gctgggattt ttgtgaccca ggaaacagaa gggaggccag ggtacgaata 4920
gagagggcgg cagaattgct cgcgccctta gcgccccagg agccgggccg gtcgagggag 4980
aactaaaggg atgcggggta gtcaaaattc cggctcccgg aagttctgcg gggagccagg 5040
cgaacgacca ctcccaccac gcctcccccc ggaggggctg acttccttgg ggcgagaggg 5100
agcgggtggc gcagagcagc tgagcgggaa tgtctgcagg gcggcgcggc gccttacctg 5160
cggcctccgg gctggaggtg tcggagatgg tgtgcacctc cagcctgtgc ttggaggagt 5220
ccagcgaccg gggctgaccg ggagccagaa ccgaagccat ggctaacggc tggggatggt 5280
gacaggaaga tgaggagacg gccgacagct tggtccccgc tgctcggtgc tccaagtgaa 5340
gcgggccttt catgcagttc atggacgagg gagcgcgacg ctctactagt ccttggctac 5400
tgccccgccg agcccccgta gccgccgctg cccgctccgg gtcgcgctct aggcgcggag 5460
tttccccgct gcgggggagag ccaggggacg caaccccgc cgagttctca agccaagctg 5520
cccccgtctc ctccggaagg ctcaagcgaa aaagtccgga gacggaaagt cagcgggcaa 5580
acgaagacat gggatgtggg cagaagggca ccactcagag cgtctttagg gagcaggctt 5640
ccaagctcca aagcgaaaca agagtgggca aagacccccct tcttctctcc ctccctcccc 5700
caagaacccc tccaataagg aaagctaacg ccgaccgcgc tctgcccgcc cccccccac 5760
gcggcagccc tgacagagaa gtgtcaagag tgacagggac aggtaggtga tattagatcc 5820
cctgcggcgg cagcagccgc tgcagccacg acgcggccct ctgagcgcac cctccgcaac 5880
gcgcacacgc acacccctcg ggcggtcgaa caggagccgg gccttgccgc agctcagctc 5940
caggcaccca ggcgagcgac ggaccagatc tgcggctccg cgcttccctg ttggcctaac 6000
atcttaaaac cagaggcggg cttcctggtg ccgagacgtc actccgccgc ggccctcccc 6060
agccctctcc gcctccgcct cctcccagac ccttctccgg gtgcgactga cgtggctccg 6120
caccaatcag gacgccccga gccgcggtgg agggactgtc ctgcctgcac ctatcagcag 6180
tgcggggccg ggctactgcc tcgccgtgcg cactgggtct acacaggcaa gctcccggga 6240
attcagctcc tgcccagccc aaggcgatcc ggcttttagt acgaacccaa aggtgaagag 6300
atgaggctag gagtcgaagg cttgggagaa gagagtggaa tggtcaagaa gagaaaggta 6360
caaggatcaa caagacaccc actctttgtg tctcactaca tccatttcca atcccccacc 6420
ccatataaaa aggagacacg ttacttaaaa ctagaaaatt tgaaaaacag caacaaatca 6480

```
cctctccgat cttaaatttt ccaaacagcc tgtcaagtga atgctgcgct aatctgaaga    6540
agctttaatt gcaaagaaga cagagccctg aaaaggcagg ctaataaatt agaaatcgag    6600
aagcaaatgg acccgtcaaa agaaaattac cttgacttta aacgaacaac tgtttggtgg    6660
ttcactctgg atttatacaa gaataaaaag tcgcctcaga tcacgttctc tgtgatgctt    6720
attagtcccc agacagaaaa cacacaatag aagagaaacc ctaacccagc gttttcaaaa    6780
tgctgaaagc ttatccattc tacttaacgt tgattaagac acatatccta gatctttcaa    6840
attccttgta cactgtatta agctcgtcct aacccgagag agccacgctt taaattcgac    6900
tctcttgttt actttattat caatcagatt taaatccata aagcctgtag aatcaacaac    6960
cttgagctaa ttatatatga aatatgcctt aatgaatttc catacaatta agaatgttgc    7020
caaataacca atttcaagga taatttttaa cagtcatttt cttttcccag tgagctcaag    7080
gctgtcttga gccattaaag tccaagcagg cagaaggggt gtgtgtgagc taagggcgaa    7140
aagcctagaa ctgcgctcaa ctagcaaaag caaaacctta tttatataaa acaaaaaaaa    7200
tcacctttgg agacatcaac tctttatagc actgtttcca agcaaattta atttccaaag    7260
aaattaaaga aagaaatcca aacatattca aaataatttt tgaaagtcct tttgtccccc    7320
agcataggtc agctggagag gacaaactaa tctcctctgg gtttctgcat gggcgattgt    7380
tttactatgg agttagtgtt atcatctctg aatgtgtatt tgtttgacat tacagtcaat    7440
gatttgcaat gccagcatga agtatcttta aaacactccc tccttgtcct tgttcacaag    7500
attgggaaac ttattcgatg tggaacaaag tggatgaagc agactacaaa tatatttgca    7560
acttatgtgt ctctcctttg ccctgaccac ccccaaaccc tatctgcaac tcctccccat    7620
tttaaacttg cagtccaaag acgcacatga gaattgtttt tcagtctttc ttcaccagta    7680
tcatcccact ttaagaataa tttagctgca agggaggaat ttcttcatag taagctttaa    7740
atcagcattt ctgcttttaa ccttttattc cactttaccc cattccacac atacagacac    7800
ctgctcagag taaaacacat cctcatgtga caggtctgca ttagctgagg ctcatacatc    7860
cagctatatt aggtcctgca atcttatcac taaattatac acattacact agcagcctgt    7920
tggtaaagaa ggttaaatta atttacattc tgctcattat ctggtgctta aatgacgcat    7980
tttatcccgg agatttggcg gagaatctcc ttctcagacc ccacagcgtt tcactgaaga    8040
caatgctctt acatttgtag tggtttttaa tctgataaga ctctaatttg cttaagtctt    8100
ttaaataagg gttttaaatg tttctagccg ttttcttatt gaatttcctc taattccccc    8160
aagatcataa agtatatgtg taaagtaaat atttcctccc attgcactgc cagccgatga    8220
cctataacta agtcaataag aatccagctc ttttctgctg aatgtgttta ctaatcatat    8280
tccagtttct tcttttaaac ctcagaatag ctgtggtccc cacaatacca tgccccttaa    8340
agcttcattt catgaaggga ctccatcaca ttaaagaatg aaaaaaatct ccactgtagt    8400
tagtatacac agcccctcac tccttgtttt tcaagattca aaccccagag ctgcaaatat    8460
ttttggaagc ttgggtgtta atgccttatt ttagaaagcc gagaagcccc acagagccat    8520
atagatttct aaacccatct ctcataaacc cacagaattt tgataaaagc tctggtggct    8580
ccactctacc gatggaactt tcatcacgac aaatatacat gtatgaagga cctcaatcag    8640
cctccaaagt ggttgaaaaa cccaagggca cgtgactgct cctcatagtg ccaacgtgtg    8700
cgagatgttg gaagcactgg ggatcagcag cagcctagat gcctaaaaag ataaggtgtc    8760
ctaatttgtg tggacccatt gaagtcaagt ggtgaataaa gacaattatc tagataattc    8820
agattaaagt aaaagcaaaa ccatatctat ttgtatatat atattcacat ccattttata    8880
ttacagacac acacacgcac acacacactg gctctgtaaa caactgactc aaagtgagga    8940
ttttctttgc atttttccag caggagtttc aacattctcc taatctccta atcactttac    9000
a                                                                    9001
```

<210> 2
<211> 9001
<212> DNA
<213> Artificial Sequence

<220>
<223> chemically treated genomic DNA (Homo sapiens)

<400> 2

```
agttgatgga tttgttaaat tttttttttt tttttttttt tttatattat ttgttagtta      60
taatggaatt ttttaggttt aagttaaaga aaaattggag agataaaatt agattttgta     120
gttttttttt ttttcgggaa tgtttttttt tttttttta gtttttgatg aatggttatt     180
atttattttt attaaattta aataaggatt gttgttttgt atgtttaatt aggtaggtag     240
agggaattgg tttgtttagg aagtagtgat tgagatgttt tggttaagtt agtgatagag     300
gaggggagaa agaatttaga ttaatttgta tgtagtatat tttattttta tgaaataaaa     360
tatatttgtt ttatatttgt tgaaaagtaa aataataata ttgtacgaaa tgttatatat     420
agggtaggtt gtatatagta gttttagaaa tattattgta tttattagag aaattatttt     480
aaaattgata tttatatatt ttttataata ataataatat gttagaaata tatagtgtgg     540
```

```
tatttagtat atatattttt ttgttcgtaa gcgaaaaatt ttaatcgttt ttgtataata    600
tgttttattt taaagtttaa tttttaaaaa tattgttgtg atattattaa taattgtttt    660
tataaaatta atttgatatt tcgatatata tatatttttt tagttattta aatgttaata    720
atgttaaatt taaaaaataa taagtttata gtaatgttaa aatgttatat ttagttaaat    780
atttgtttgt gtatgtgttt ttgtaattgt tagaaatatt tgtagtgaaa gatgttagat    840
attgaggata tttttttgaa attaaaggag tttttttttt gatttagtgg tttttttttt    900
tttatatagt tttttttttt tttttttttt ttagtgttta cgatttttta gtataatttt    960
tagtttttta agggcggagt tgttttattc ggtaaggttt taggatttcg gcgttgtggg   1020
tgcggtttat acgggtcggt ttattgtata ttggtaagta tttaggttgg aggtcgggtt   1080
ttgtacgttg gcgtagtcga agttggagtg ttgttttgtt tttagtttta ggttggttag   1140
gttcgagtta tacgtgtttt tataaatata cggaggagtc ggcggcgcgt aaggataggt   1200
aggcgtcggt atcgcggaat ttagcgacgg gttatttagg ttgtttaagt tatttaggtt   1260
gttgagattg gagttcggga cgtttgttat tgttgagggt attatgttgg acgatatgtt   1320
tatggacgag atagagttgg gtggggaaaa tatgttttgt gatgataggg ggttgacgtt   1380
tatagagttg aagaagggga agtttttggt ggatagggag gcggatgtaa ggtttttggc   1440
ggtttagttg ttgtaggaat agtttgggta tatgtcgtcg tagggttgta tgagtttatt   1500
gaattgcggt tcgaagttat ttttgtatag ttcggtttgt tggttgcgtt tttttttttt   1560
ttatttggtt cgacgatttt tgaattaaat ttgggggcgg ttggggtaag ggagtaaata   1620
gatgttatag tgtagattat taaaattttt atcggaggtt aattttcgtt tttttcgat    1680
atatacgtta gcgtatttat atattttggt ttcgttttat tgtatcgttt tgtatattaa   1740
gatattaggg ttagttttta gttattggtt cgggttttta ttaagcgtag gagatttggt   1800
ttgttttggt ttgcgagttg ggattcggag ttacgttata aattttagtc gaacgtatgg   1860
agatttgcgg acggtttgat tatttagtta ggcgtttttt taggtttaaa aatatttaat   1920
gtaaaataaa cgcggggtag taggtttttt taattttttt cggggtattt tgtaaatttg   1980
tttttatttt aaagttatag atttacggat gaggagaagg ggttggaagg gtattagagg   2040
atcgtttttt tttttacgta attttttttt ttttttttg attttttattg tcgtttttta   2100
ttttttggta cgtgtttttt taatagggat taggtcgtta atattttttt tcgtttagta   2160
aaataattaa ataaagagta aaagattatt ttttcgttag ttcgttaatt ttaggagttt   2220
ggtatattaa atttcgggaa ttcggaaagg gtagttttgg agattttttt tttttcgtt    2280
ttgttttttt tttattttaa gtttattata ggtttgttcg cgcgttaggt ttagtcgggt   2340
cgtttggttt tgtaggcggt tatttaggtc ggtcggtttt tattcgtgtt cggtggttta   2400
gtcgtaattt cgattttaat ttatatcggg tttttttgtc gttttagacg gcggtttttg   2460
tgtattggag agaggtttgg tttgagatat tcgagttgat attagtgatg ttttatatta   2520
tatattttcg tcgggtttag tcgtgtaatt cgtttttttt tttttttttt ttatttttga   2580
tttttttttt attttttttt ttttttgtat tcgattgtta taaaaagtac gttttatttt   2640
tatttggttc gataagtagt cgttttggaa ggagaggtag ttgtaaggag agtttagcgt   2700
cgcggttata aagtattagg gtggagttgc ggaatagcgg gcggggtggg agggcgtttt   2760
cgaaggattt tagaaaattt atagatttg ttttttaatta tttgttattt ttattttagg   2820
ttatttaaat tttgtttagg cgagaagagt acgtgagagg ttcgtttttt tgatgtgtaa   2880
gagagttaat gaaagattga ttttgtttaa aattacgtcg tttaggattt agttttggtt   2940
ttggatagtt aaattaaaat tatttttaat tttttttcgg tttttttattt attagtatag   3000
ttttatgttt tgtataaatg ttatttagag agtgttttta ttttttttga tttgggagag   3060
tattttggtt tttatttttt ttatcgttgt tttttttttt ttgtttgttt tgttttaatc   3120
gggggtttta ttttttttat ttagagtatt taattttttt tttttaatag taaagttttt   3180
ggatgtcgtt tgatttgttt gattttgttt tttgtttttta gaattttaat aaatttggaa   3240
tttttttatcg attagtataa attaggacgt tgttattggg ttatttattt gagtttattt   3300
ttgttaattt ataaagtata gatttgttat aaagttaagg taagtttttt ttataaaatt   3360
atgattataa tttagaagag ggggtgtgag ttttaatttt tagagtttaa tttttgagag   3420
aagataaata aattaagtag aaaagttttt tttttttttt tttttttttt tttaagagga   3480
ttagtagttg tgtattaaaa ttttgttttc ggagattata aaattaggaa atagggtgtg   3540
tgggagagat ttgaatggtc gaaataatcg taaagaaggt gtaagaagcg cgagtttagg   3600
agggaaaaag ttgggttagg gtcgggataa aggtttttta gggagggtta atttttttcgt   3660
gtttttggcg ggtttttttt gttaaaggtt tataggttgg agtttgttcg cggtttttgg   3720
tttggtaggg atttattag ttttgttttg gtaattgtaa gttaggaata taatgttttg    3780
tgtaggggat tgtttatgta gtttagttcg tgagatcgcg ggatggcggg gtagtgagtc   3840
ggtgtcgttt tgggagtttg agttagggcg gtagttttgt cggtttcgga gagggaattg   3900
taatttcgta attaggtcgt cgcgaggttt tttgtttttg taaagttgcg ttttatcggc   3960
gtttttttag gcggcgttgt tttttatatt tttttttggt ttatttggtt tgtattttta   4020
taatattttt tttttatttt ttttagattt cgtgttggtt tttattcgga ttcgggtttt   4080
cgtaaggttg gtttatatag cgatttttttc gcgtgtggat atgttcgggt agcggttttt   4140
ttggaaagtg gtttttagtt tttggagttg ttggttggta aagtgagttc gttgtcgttt   4200
ttgtcgtttt tttttagacg ggttttcggc gtttacgttt ttattttttt tttgttggtt   4260
tttatttttt tttgaaaacg aaatatatat attttttcgt tagtatgttt atttgtaacg   4320
```

cggacgttaa ttggatcggc ggtagaagtc gtggaagagt tgggttgttt ggcgtcggag 4380
gagggtgcgc gcggcggttt cgggtcgcga ggagcgttgc gtttgtgggg tgtgtaggcg 4440
taagtgtggg tgttcgcgtt ttattttttt ttttttttta gcgtcgtacg ttttatttat 4500
atgtttattt tattgtagcg gtatatttat ttttatagtt tgtgttttta agtatattta 4560
tatatttttg cgtagatata ttaaattttt tgggacgcgt atacgcgcgt ggtttataga 4620
tttttttttt tttcgtagaa agtttagatt tttatgcggt ttgggaaggt taggaaaaga 4680
tgtggggatt cggttgggta tcgaagttcg tcggtttttt tttaaaaaaa aaaaaaaaat 4740
gtttttttcgc gaagggtatt tttgagtggt tttaggtaat tttttaacga gtggagtttt 4800
tcgggagttg aaagtcgaga ggaaaatagg gatagaggtc ggcggttttt gaaggttttc 4860
gaattaagat gttgggattt ttgtgattta ggaaatagaa gggaggttag ggtacgaata 4920
gagagggcgg tagaattgtt cgcgttttta gcgttttagg agtcgggtcg gtcgagggag 4980
aattaaaggg atgcggggta gttaaaattt cggttttcgg aagttttgcg gggagttagg 5040
cgaacgatta tttttattac gttttttttc ggagggggttg atttttttgg ggcgagaggg 5100
agcgggtggc gtagagtagt tgagcgggaa tgtttgtagg gcggcgcggc gttttatttg 5160
cggttttcgg gttggaggtg tcggagatgg tgtgtatttt tagtttgtgt ttggaggagt 5220
ttagcgatcg gggttgatcg ggagttagaa tcgaagttat ggttaacggt tggggatggt 5280
gataggaaga tgaggagacg gtcgatagtt tggttttcgt tgttcggtgt tttaagtgaa 5340
gcgggttttt tatgtagttt atggacgagg gagcgcgacg ttttattagt ttttggttat 5400
tgtttcgtcg agttttcgta gtcgtcgttg ttcgtttcgg gtcgcgtttt aggcgcggag 5460
ttttttcgtt gcggggagag ttaggggacg taattttcgt cgagttttta agttaagttg 5520
ttttcgtttt tttcggaagg tttaagcgaa aaagttcgga gacggaaagt tagcgggtaa 5580
acgaagatat gggatgtggg tagaagggta ttatttagag cgtttttagg gagtaggttt 5640
ttaagtttta aagcgaaata agagtgggta aagatttttt tttttttttt tttttttttt 5700
taagaatttt tttaataagg aaagttaacg tcgatcgcgt tttgttcgtt tttttttttac 5760
gcggtagttt tgatagagaa gtgttaagag tgatagggat aggtaggtga tattagattt 5820
tttgcggcgg tagtagtcgt tgtagttacg acgcggtttt ttgagcgtat ttttcgtaac 5880
gcgtatacgt atatttttcg ggcggtcgaa taggagtcgg gttttgtcgt agtttagttt 5940
taggtattta ggcgagcgac ggattagatt tgcggtttcg cgtttttttg ttggtttaat 6000
attttaaaat tagaggcggg ttttttggtg tcgagacgtt atttcgtcgc ggtttttttt 6060
agtttttttc gttttcgttt ttttttagat tttttttcgg gtgcgattga cgtggtttcg 6120
tattaattag gacgtttcga gtcgcggtgg agggattgtt ttgtttgtat ttattagtag 6180
tgcggggtcg ggttattgtt tcgtcgtgcg tattgggttt atataggtaa gttttcggga 6240
atttagtttt tgtttagttt aaggcgattc ggtttttagt acgaatttaa aggtgaagag 6300
atgaggttag gagtcgaagg tttgggagaa gagagtggaa tggttaagaa gagaaaggta 6360
taaggattaa taagatattt atttttttgtg ttttattata tttattttta atttttttatt 6420
ttatataaaa aggagatacg ttatttaaaa ttagaaaatt tgaaaaatag taataaatta 6480
tttttttcgat tttaaatttt ttaaatagtt tgttaagtga atgttgcgtt aatttgaaga 6540
agttttaatt gtaaagaaga tagagttttg aaaaggtagg ttaataaatt agaaatcgag 6600
aagtaaatgg attcgttaaa agaaaattat tttgatttta aacgaataat tgtttggtgg 6660
tttattttgg atttatataa gaataaaaag tcgttttaga ttacgttttt tgtgatgttt 6720
attagttttt agatagaaaa tatataatag aagagaaatt ttaatttagc gtttttaaaa 6780
tgttgaaagt ttatttattt tatttaacgt tgattaagat atatatttta gattttttaa 6840
atttttttgta tattgtatta agttcgtttt aattcgagag agttacgttt taaattcgat 6900
tttttttgttt atttttattat taattagatt taaatttata aagtttgtag aattaataat 6960
tttgagttaa ttatatatga aatatgtttt aatgaatttt tatataatta agaatgttgt 7020
taaataatta atttttaagga taattttttaa tagttatttt tttttttttag tgagtttaag 7080
gttgttttga gttattaaag tttaagtagg tagaaggggt gtgtgtgagt taagggcgaa 7140
aagtttagaa ttgcgtttaa ttagtaaaag taaaatttta tttatataaa ataaaaaaaa 7200
ttatttttgg agatattaat tttttatagt attgttttta agtaaattta atttttaaag 7260
aaattaaaga aagaaattta aatatattta aaataatttt tgaaagtttt tttgtttttt 7320
agtataggtt agttggagag gataaattaa tttttttttgg gtttttgtat gggcgattgt 7380
tttattatgg agttagtgtt attattttttg aatgtgtatt tgtttgatat tatagttaat 7440
gatttgtaat gttagtatga agtattttta aaatattttt tttttgtttt tgtttataag 7500
attgggaaat ttattcgatg tggaataaag tggatgaagt agattataaa tatatttgta 7560
atttatgtgt tttttttttg ttttgattat ttttaaattt tatttgtaat tttttttttat 7620
tttaaatttg tagtttaaag acgtatatga gaattgtttt ttagtttttt tttattagta 7680
ttattttatt ttaagaataa tttagttgta agggaggaat ttttttatag taagttttaa 7740
attagtattt ttgtttttaa tttttttattt tattttattt tattttatat atatagatat 7800
ttgtttagag taaaatatat ttttatgtga taggtttgta ttagttgagg tttatatatt 7860
tagttatatt aggtttgtta atttttattat taaattatat atattatatt agtagtttgt 7920
tggtaaagaa ggttaaatta atttatattt tgtttattat ttggtgttta aatgacgtat 7980
tttatttcgg agatttggcg gagaattttt tttttagatt ttatagcgtt ttattgaaga 8040
taatgttttt atatttgtag tggtttttaa tttgataaga ttttaatttg tttaagtttt 8100

```
ttaaataagg gttttaaatg tttttagtcg tttttttatt gaatttttt taattttttt    8160
aagattataa agtatatgtg taaagtaaat attttttttt attgtattgt tagtcgatga    8220
tttataatta agttaataag aatttagttt ttttttgttg aatgtgttta ttaattatat    8280
tttagttttt ttttttaaat tttagaatag ttgtggtttt tataatatta tgttttttaa    8340
agttttattt tatgaaggga ttttattata ttaaagaatg aaaaaaattt ttattgtagt    8400
tagtatatat agttttttat tttttgtttt ttaagattta aattttagag ttgtaaatat    8460
ttttggaagt ttgggtgtta atgttttatt ttagaaagtc gagaagtttt atagagttat    8520
atagattttt aaatttattt tttataaatt tatagaattt tgataaaagt tttggtggtt    8580
ttattttatc gatggaattt ttattacgat aaatatatat gtatgaagga ttttaattag    8640
tttttaaagt ggttgaaaaa tttaagggta cgtgattgtt ttttatagtg ttaacgtgtg    8700
cgagatgttg gaagtattgg ggattagtag tagtttagat gtttaaaaag ataaggtgtt    8760
ttaatttgtg tggatttatt gaagttaagt ggtgaataaa gataattatt tagataattt    8820
agattaaagt aaaagtaaaa ttatatttat ttgtatatat atatttatat ttattttata    8880
ttatagatat atatacgtat atatatattg gttttgtaaa taattgattt aaagtgagga    8940
tttttttgt attttttag taggagtttt aatatttttt taatttttta attattttat    9000
a                                                                   9001
```

<210> 3
<211> 9001
<212> DNA
<213> Artificial Sequence

<220>
<223> chemically treated genomic DNA (Homo sapiens)

<400> 3

```
tgtaaagtga ttaggagatt aggagaatgt tgaaattttt gttggaaaaa tgtaaagaaa     60
atttttattt tgagttagtt gtttatagag ttagtgtgtg tgtgcgtgtg tgtgtttgta    120
atataaaatg gatgtgaata tatatatata aatagatatg gttttgtttt tattttaatt    180
tgaattattt agataattgt ttttatttat tatttgattt taatgggttt atataaatta    240
ggatatttta tttttttagg tatttaggtt gttgttgatt tttagtgttt ttaatatttc    300
gtatacgttg gtattatgag gagtagttac gtgtttttgg gtttttttaat tattttggag    360
gttgattgag gtttttttata tatgtatatt tgtcgtgatg aaagttttat cggtagagtg    420
gagttattag agtttttatt aaaattttgt gggtttatga gagatgggtt tagaaattta    480
tatggttttg tggggttttt cggtttttta aaataaggta ttaatattta agtttttaaa    540
aatatttgta gttttggggt ttgaattttg aaaaataagg agtgaggggt tgtgtatatt    600
aattatagtg gagatttttt ttattttttta atgtgatgga gtttttttat gaaatgaagt    660
tttaaggggt atggtattgt ggggattata gttattttga ggtttaaaag aagaaattgg    720
aatatgatta gtaaatatat ttagtagaaa agagttggat ttttattgat ttagttatag    780
gttatcggtt ggtagtgtaa tgggaggaaa tatttatttt atatatatat tttatgattt    840
tgggggaatt agaggaaatt taataagaaa acggttagaa atatttaaaa tttttattta    900
aaagatttaa gtaaattaga gttttattag attaaaaatt attataaatg taagagtatt    960
gtttttagtg aaacgttgtg gggtttgaga aggagatttt tcgttaaatt ttcgggataa   1020
aatgcgttat ttaagtatta gataatgagt agaatgtaaa ttaatttaat ttttttttatt   1080
aataggttgt tagtgtaatg tgtataattt agtgataaga ttgtaggatt taatatagtt   1140
ggatgtatga gttttagtta atgtagattt gttatatgag gatgtgtttt attttgagta   1200
ggtgtttgta tgtgtggaat ggggtaaagt ggaataaaag gttaaaagta gaaatgttga   1260
tttaaagttt attatgaaga aatttttttt ttgtagttaa attattttta aagtgggatg   1320
atattggtga agaaagattg aaaaataatt tttatgtgcg tttttggatt gtaagtttaa   1380
aatgggggagg agttgtagat agggtttggg ggtggttagg gtaaaggaga gatatataag   1440
ttgtaaatat atttgtagtt tgttttattt attttgtttt atatcgaata agttttttaa   1500
ttttgtgaat aaggataagg agggagtgtt ttaaagatat tttatgttgg tattgtaaat   1560
tattgattgt aatgttaaat aaatatatat ttagagatga taatattaat tttatagtaa   1620
aataatcgtt tatgtagaaa tttagaggag attagtttgt ttttttttagt tgatttatgt   1680
tgggggataa aaggattttt aaaaattatt ttgaatatgt ttggattttt tttttttaatt   1740
tttttggaaa ttaaatttgt ttggaaatag tgttataaag agttgatgtt tttaaaggtg   1800
atttttttttg ttttatataa ataaggtttt gtttttgtta gttgagcgta gttttaggtt   1860
tttcgttttt agtttatata tatttttttt gtttgtttgg attttaatgg tttaagatag   1920
ttttgagttt attgggaaaa gaaaatgatt gttaaaaatt atttttgaaa ttggttattt   1980
ggtaatattt ttaattgtat ggaaatttat taaggtatat tttatatata attagtttaa   2040
ggttgttgat tttataggtt ttatggattt aaatttgatt gataataaag taaataagag   2100
agtcgaattt aaagcgtggt tttttcgggt taggacgagt ttaatatagt gtataaggaa   2160
```

```
tttgaaagat ttaggatatg tgttttaatt aacgttaagt agaatggata agtttttagt    2220
attttgaaaa cgttgggtta gggtttttt tttattgtgt gtttttgtt tggggattaa     2280
taagtattat agagaacgtg atttgaggcg atttttatt tttgtataaa tttagagtga    2340
attattaaat agttgttcgt ttaaagttaa ggtaatttt ttttgacggg tttatttgtt    2400
tttcgatttt taatttatta gtttgttttt ttagggtttt gttttttttg taattaaagt   2460
ttttttagat tagcgtagta tttatttgat aggttgtttg gaaaatttaa gatcggagag   2520
gtgatttgtt gttgtttttt aaatttttta gtttaagta acgtgtttt tttttatatg     2580
gggtggggga ttggaaatgg atgtagtgag atataaagag tgggtgtttt gttgattttt   2640
gtattttttt tttttgatt atttattttt ttttttttaa gttttcgatt tttagtttta    2700
ttttttttatt tttgggttcg tattaaaagt cggatcgttt tgggttgggt aggagttgaa  2760
ttttcgggag tttgtttgtg tagatttagt gcgtacggcg aggtagtagt tcggtttcgt   2820
attgttgata ggtgtaggta ggatagtttt tttatcgcgg ttcggggcgt tttgattggt   2880
gcggagttac gttagtcgta ttcggagaag ggtttgggag gaggcggagg cggagagggt   2940
tggggagggt cgcggcggag tgacgtttcg gtattaggaa gttcgtttt ggttttaaga    3000
tgttaggtta atagggaagc gcggagtcgt agatttggtt cgtcgttcgt ttgggtgttt   3060
ggagttgagt tgcggtaagg ttcggtttt gttcgatcgt tcgaggggtg tgcgtgtgcg    3120
cgttgcggag ggtgcgttta gagggtcgcg tcgtggttgt agcggttgtt gtcgtcgtag   3180
gggatttaat attatttatt tgtttttgtt atttttgata ttttttttgtt agggttgtcg  3240
cgtggggggg gggcgggtag agcgcggtcg gcgttagttt tttttattgg aggggttttt   3300
gggggaggga gggagagaag aagggggttt ttgtttattt ttgtttcgtt ttggagtttg   3360
gaagtttgtt ttttaaagac gtttgagtg gtgttttttt gtttatattt tatgttttcg    3420
tttgttcgtt gatttttcgt tttcggattt tttcgtttga gttttttcgga ggagacgggg  3480
gtagtttggt ttgagaattc ggcgggggtt gcgtttttttg gttttttttcg tagcggggaa 3540
atttcgcgtt tagagcgcga ttcggagcgg gtagcggcgg ttacgggggt tcggcggggt   3600
agtagttaag gattagtaga gcgtcgcgtt ttttcgttta tgaattgtat gaaaggttcg   3660
ttttatttgg agtatcgagt agcggggatt aagttgtcgg tcgtttttttt atttttttgt  3720
tattattttt agtcgttagt tatggtttcg gttttggttt tcggttagtt tcggtcgttg   3780
gatttttta agtataggtt ggaggtgtat attattttcg atatttttag ttcggaggtc    3840
gtaggtaagg cgtcgcgtcg ttttgtagat attttcgttt agttgtttg cgttattcgt    3900
tttttttcgt tttaaggaag ttagtttttt cggggggagg cgtggtggga gtggtcgttc   3960
gtttggtttt tcgtagaatt ttcgggagtc ggaattttga ttatttcgta ttttttttagt  4020
ttttttttcga tcggttcggt ttttggggcg ttaagggcgc gagtaatttt gtcgtttttt  4080
ttattcgtat tttggttttt ttttttgtttt ttgggttata aaaattttag tattttgatt  4140
cgaggatttt tagaggtcgt cgatttttgt ttttgttttt ttttcggttt ttagttttcg   4200
aggagtttta ttcgttagga aattgtttga aattatttag aaatgtttt cgcgaagagg    4260
tatttttttt ttttttttgg gaaagggtcg gcgaatttcg gtgtttaatc gaatttttat   4320
attttttttt agtttttttta aatcgtatgg aaatttgagt tttttgcgag ggggaggggg  4380
gtttgtaaat tacgcgcgtg tgcgcgtttt aggagatttg gtgtgtttgc gtagaggtgt   4440
ataaatatat ttgaaagtat aggttataaa agtgaatgtg tcgttgtagt gagataaata   4500
tgtaaataaa acgtgcggcg ttgggggagg ggaggaaatg gggcgcggat atttatattt   4560
gcgtttgtat attttatagg cgtagcgttt ttcgcggttc ggagtcgtcg cgcgtatttt   4620
ttttcggcgt taggtagttt agtttttta cggtttttgt cgtcggttta gttggcgttc    4680
gcgttgtagg tgggtatgtt gacgggaaag tgtgtgtgtt tcgtttttag agaaagataa   4740
aagttagtag gggaagaatg aggacgtggg cgtcgaggat tcgtttaaga agaagcggta   4800
aaggcggtag cggatttatt ttattagtta gtagttttag gagttggagg ttattttttta  4860
gaggaatcgt tattcggata tgtttatacg cgaagaaatc gttgtgtgga ttaattttac   4920
ggaagttcga gttcgggtag gagttagtac ggagtttggg agggatgggg ggaggatgtt   4980
gtggaggtat aggttaagta gattaggaga gaatgtggaa ggtagcgtcg tttgggaggg   5040
cgtcggtggg gcgtagtttt gtaaaggtag aaggtttcgc ggcggtttgg ttgcgagatt   5100
atagtttttt tttcgaggtc gataggattg tcgttttggt ttaggttttt agagcggtat   5160
cggtttattg tttcgttatt tcgcgatttt acgagttggg ttgtatgggt aatttttttgt  5220
ataggatatt gtgtttttgg tttgtagttg ttagagtaga gttaataaaa tttttattag   5280
gttaagagtc gcgaataggt tttaatttgt gagtttttaa taaggaaaat tcgttagaga   5340
tacggaagag ttggtttttt ttgggaaatt tttgtttcgg ttttggttta gttttttttt   5400
ttttgggttc gcgtttttta tatttttttt acggttgttt cggttattta ggtttttttt   5460
atatatttta ttttttagtt ttgtgatttt cgggagtaaa gttttaatat ataattatta   5520
gtttttttag aaggagaaag aaaaaaagaa gaaagatttt tttgtttggt ttatttattt   5580
ttttttagga gttgaatttt ggaaattgaa atttatattt ttttttttaa attataatta   5640
tagttttgta aaaagggttt attttaattt tgtagtaaat ttgtatttta tggattggta   5700
aaaatgagtt taaataaata atttaatagt aacgttttgg tttatgttgg tcggtggaag   5760
attttaaatt tgttaggatt ttggaagtag aaaatagaat taagtaaatt aagcggtatt   5820
tagaggtttt gttgttaaaa aaaaaaaatt aagtgttttg ggtagaaaaa ataaagtttt   5880
cggttagagt agagtaaata aaaagaagaa aataacgata aaaagaataa agattaaaat   5940
```

```
gtttttttaa attagaggga atgaagatat tttttgggtg gtatttgtgt aaggtatgag    6000
gttatgttgg tggataaaag gtcgggaaga agttgaaaat ggttttagtt taattgttta    6060
gagttagagt tgggttttgg gcggcgtggt tttgagtaag gttagttttt tattagtttt    6120
tttgtatatt aagggaacgg gtttttttacg tattttttttc gtttgagtaa agtttagatg    6180
gtttagggta gaaatggtaa gtaattaaag atagagttta tgggtttttt gggatttttc    6240
gaaaacgttt ttttatttcg ttcgttattt cgtagtttta ttttagtgtt ttgtagtcgc    6300
ggcgttgggt ttttttttgta gttgtttttt tttttagggc ggttgtttgt cgagttaagt    6360
gggagtgagg cgtgtttttt atagtagtcg ggtgtaaaga ggaaggggga taaaaaggaa    6420
attaagaatg aaaggaaaaa gagaaaaagc ggattatacg gttgggttcg gcggagatgt    6480
gtaatgtgaa atattattgg tgttagttcg gatattttag gttaggtttt tttttaatat    6540
ataaaagtcg tcgtttgggg cgatagggag gttcgatgtg gattgggatc ggggttgcgg    6600
ttgggttatc ggatacgggt ggaagtcggt cggtttgggt ggtcgtttgt aaagttaaac    6660
gattcggttg ggtttggcgc gcggataggt ttgtggtggg tttagggtaa agaagaggta    6720
gagcgaaaga agggggaatt tttaaaatta ttttttttcgg gttttcggag tttaatatgt    6780
taagtttttg gagttaacga gttgacgaag aggtggtttt ttgtttttta tttggttgtt    6840
ttgttaggcg agaaagagtg ttggcggttt agtttttgtt aagggagtac gtattagggg    6900
gtgggggacg atagtggagg ttagggaagg aagggaggaa ttgcgtggga gaaagagcga    6960
ttttttagtg ttttttttagt ttttttttttt tattcgtggg tttgtggttt tggaatggaa    7020
gtaagtttgt aaggtgtttc gggaagggtt ggaaaagttt gttgtttcgc gtttgtttta    7080
tattaagtgt ttttggattt ggagaaacgt ttggttgagt gattaaatcg ttcgtaggtt    7140
tttatgcgtt cggttgaggt ttgtggcgta gtttcgagtt ttagttcgta ggttagagta    7200
gattaggttt tttgcgtttg gtggagattc gggttagtaa ttgaaagttg gttttggtat    7260
tttggtgtgt agggcggtgt agtgaagcga ggttagggtg tgtgagtgcg ttagcgtgtg    7320
tgtcgggggga aggcgggggt tggttttcga tggaagtttt agtaatttgt attgtggtat    7380
ttgtttgttt ttttgtttta atcgttttta ggtttggttt aagaatcgtc gggttaaatg    7440
gagaaagagg gagcgtaatt agtaggtcga gttatgtaag aatggtttcg ggtcgtagtt    7500
taatgggttt atgtagtttt acgacgatat gtatttaggt tatttttata ataattgggt    7560
cgttaagggt tttatattcg ttttttttatt tattaagagt ttttttttttt ttaattttat    7620
gaacgttaat tttttgttat tatagagtat gttttttttta tttaatttta tttcgtttat    7680
gagtatgtcg tttagtatgg tgtttttagt agtgataggc gtttcgggtt ttagttttaa    7740
tagtttgaat aatttgaata atttgagtag ttcgtcgttg aatttcgcgg tgtcgacgtt    7800
tgtttgtttt tacgcgtcgt cgattttttc gtatgtttat agggatacgt gtaattcgag    7860
tttggttagt ttgagattga aagtaaagta gtattttagt ttcggttacg ttagcgtgta    7920
gaattcggtt tttaatttga gtgtttgtta gtatgtagtg gatcggttcg tgtgagtcgt    7980
atttatagcg tcgggatttt aggattttgt cggatggggt aatttcgttt ttgaaagatt    8040
gggaattatg ttagaaggtc gtgggtatta aagaaaggga gagaaagaga agttatatag    8100
agaaaaggaa attattgaat taaagagaga gttttttttga ttttaaaggg atgtttttag    8160
tgtttgatat tttttattat aagtattttt aatagttgta aggatatata tataaataaa    8220
tgtttgattg gatatgatat tttaatatta ttataagttt gttatttttt aagtttagta    8280
ttgttaatat ttaaatgatt gaaaggatgt atatatatcg aaatgttaaa ttaattttat    8340
aaaagtagtt gttagtaata ttataatagt gtttttaaag gttaggtttt aaaataaagt    8400
atgttatata gaagcgatta ggatttttcg tttgcgagta agggagtgta tatattaaat    8460
gttatattgt atgtttttaa tatattatta ttattataaa aaatgtgtga atattagttt    8520
tagaatagtt tttttggtgg atgtaatgat gtttttgaaa ttgttatgta taatttattt    8580
tgtgtataat atttcgtata atattattgt tttatttttt agtaaatatg aaataaatgt    8640
gttttatttt atgggagtaa aatatattgt atataaattg gtttggattt ttttttttttt    8700
tttttgttat taatttggtt aggatatttt agttattgtt ttttaaataa attagttttt    8760
tttgtttgtt tagttaaata tataaggtag tagtttttat ttaaatttgg tagaaataaa    8820
tgatagttat ttattagaaa ttaaaaagaa aaaaaaaggt attttcgggg gggaaaaggg    8880
ttataaaatt taattttgtt tttttaattt ttttttggtt taaatttaga ggattttatt    8940
atggttagta aataatatga aaaagaaaaa agaagaaaga aatttagtaa gtttattagt    9000
t                                                                    9001
```

<210> 4
<211> 9001
<212> DNA
<213> Artificial Sequence

<220>
<223> chemically treated genomic DNA (Homo sapiens)

<400> 4

agttgatgga tttgttaaat tttttttttt tttttttttt tttatattat ttgttagtta 60
taatggaatt ttttaggttt aagttaaaga aaaattggag agataaaatt agattttgta 120
gttttttttt tttttgggaa tgtttttttt ttttttttta gtttttgatg aatggttatt 180
atttattttt attaaattta aataaggatt gttgttttgt atgtttaatt aggtaggtag 240
agggaattgg tttgtttagg aagtagtgat tgagatgttt tggttaagtt agtgatagag 300
gaggggagaa agaatttaga ttaatttgta tgtagtatat tttattttta tgaaataaaa 360
tatatttgtt ttatatttgt tgaaaagtaa aataataata ttgtatgaaa tgttatatat 420
agggtaggtt gtatatagta gttttagaaa tattattgta tttattagag aaattatttt 480
aaaattgata tttatatatt ttttataata ataataatat gttagaaata tatagtgtgg 540
tatttagtat atatattttt ttgtttgtaa gtgaaaaatt ttaattgttt ttgtataata 600
tgttttattt taaagtttaa tttttaaaaa tattgttgtg atattattaa taattgtttt 660
tataaaatta atttgatatt ttgatatata tatatttttt tagttattta aatgttaata 720
atgttaaatt taaaaaataa taagtttata gtaatgttaa aatgttatat ttagttaaat 780
atttgtttgt gtatgtgttt ttgtaattgt tagaaatatt tgtagtgaaa gatgttagat 840
attgaggata ttttttttgaa attaaaggag tttttttttt gatttagtgg tttttttttt 900
tttatatagt tttttttttt tttttttttt ttagtgttta tgatttttta gtataatttt 960
tagtttttta agggtggagt tgttttattt ggtaaggttt taggattttg gtgttgtggg 1020
tgtggtttat atgggttggt ttattgtata ttggtaagta tttaggttgg aggttgggtt 1080
ttgtatgttg gtgtagttga agttggagtg ttgttttgtt tttagtttta ggttggttag 1140
gtttgagtta tatgtgtttt tataaatata tggaggagtt ggtggtgtgt aaggataggt 1200
aggtgttggt attgtggaat ttagtgatgg gttatttagg ttgtttaagt tatttaggtt 1260
gttgagattg gagtttggga tgtttgttat tgttgagggt attatgttgg atgatatgtt 1320
tatggatgag atagagttgg gtggggaaaa tatgttttgt gatgataggg ggttgatgtt 1380
tatagagttg aagaagggga agtttttggt ggatagggag gtggatgtaa ggtttttggt 1440
ggtttagttg ttgtaggaat agtttgggta tatgttgttg tagggttgta tgagtttatt 1500
gaattgtggt ttgaagttat ttttgtatag tttggtttgt tggttgtgtt tttttttttt 1560
ttatttggtt tgatgatttt tgaattaaat ttgggggtgg ttggggtaag ggagtaaata 1620
gatgttatag tgtagattat taaaattttt attggaggtt aatttttgtt tttttttgat 1680
atatatgtta gtgtatttat atattttggt tttgttttat tgtattgttt tgtatattaa 1740
gatattaggg ttagttttta gttattggtt tgggttttta ttaagtgtag gagatttggt 1800
ttgttttggt ttgtgagttg ggatttggag ttatgttata aattttagtt gaatgtatgg 1860
agatttgtgg atggtttgat tatttagtta ggtgtttttt taggtttaaa aatatttaat 1920
gtaaaataaa tgtgggggtag taggtttttt taattttttt tggggtattt tgtaaatttg 1980
tttttatttt aaagttatag atttatggat gaggagaagg ggttggaagg gtattagagg 2040
attgtttttt tttttatgta attttttttt ttttttttttg atttttattg ttgtttttta 2100
tttttttggta tgtgtttttt taatagggat taggttgtta atattttttt ttgtttagta 2160
aaataattaa ataaagagta aaagattatt tttttgttag tttgttaatt ttaggagttt 2220
ggtatattaa atttgggaa tttggaaagg gtagttttgg agattttttt tttttttgtt 2280
ttgttttttt tttattttaa gtttattata ggtttgtttg tgtgttaggt ttagttgggt 2340
tgtttggttt tgtaggtggt tatttaggtt ggttggtttt tatttgtgtt tggtggttta 2400
gttgtaattt tgattttaat ttatattggg ttttttttgtt gttttagatg gtggttttttg 2460
tgtattggag agaggtttgg tttgagatat ttgagttgat attagtgatg ttttatatta 2520
tatattttttg ttgggtttag ttgtgtaatt tgtttttttt tttttttttt ttatttttga 2580
tttttttttt attttttttt tttttgtat ttgattgtta taaaaagtat gttttatttt 2640
tatttggttt gataagtagt tgttttggaa ggagaggtag ttgtaaggag agtttagtgt 2700
tgtggttata aagtattagg gtggagttgt ggaatagtgg gtggggtggg agggtgtttt 2760
tgaaggattt tagaaaattt atagatttttg tttttaatta tttgttattt ttattttagg 2820
ttatttaaat tttgtttagg tgagaagagt atgtgagagg tttgtttttt tgatgtgtaa 2880
gagagttaat gaaagattga ttttgtttaa aattatgttg tttaggattt agttttggtt 2940
ttggatagtt aaattaaaat tatttttaat ttttttttgg tttttttattt attagtatag 3000
ttttatgttt tgtataaatg ttatttagag agtgttttta tttttttttga tttgggagag 3060
tattttggtt tttattttttt ttattgttgt tttttttttt ttgtttgttt tgttttaatt 3120
gggggtttta ttttttttat ttagagtatt taattttttt tttttaatag taaagttttt 3180
ggatgttgtt tgatttgttt gattttgttt tttgtttttta gaattttaat aaatttggaa 3240
tttttttattg attagtataa attaggatgt tgttattggg ttatttattt gagtttattt 3300
ttgttaattt ataaagtata gatttgttat aaagttaagg taagtttttt ttataaaatt 3360
atgattataa tttagaagag gggtgtgag ttttaatttt tagagtttaa tttttgagag 3420
aagataaata aattaagtag aaaagttttt tttttttttt tttttttttt tttaagagga 3480
ttagtagttg tgtattaaaa ttttgttttt ggagattata aaattaggaa atagggtgtg 3540
tgggagagat ttgaatggtt gaaataattg taaagaaggt gtaagaagtg tgagtttagg 3600
agggaaaaag ttgggttagg gttgggataa aggtttttta gggagggtta atttttttgt 3660
gtttttggtg ggtttttttt gttaaaggtt tataggttgg agtttgtttg tggtttttgg 3720
tttggtaggg atttattag ttttgttttg gtaattgtaa gttaggaata taatgttttg 3780

tgtaggggat tgtttatgta gtttagtttg tgagattgtg ggatggtggg gtagtgagtt 3840
ggtgttgttt tgggagtttg agttagggtg gtagttttgt tggttttgga gagggaattg 3900
taattttgta attaggttgt tgtgaggttt tttgtttttg taaagttgtg ttttattggt 3960
gtttttttag gtggtgttgt tttttatatt tttttttggt ttatttggtt tgtattttta 4020
taatattttt tttttatttt ttttagattt tgtgttggtt tttatttgga tttgggtttt 4080
tgtaaggttg gtttatatag tgattttttt gtgtgtggat atgtttgggt agtggttttt 4140
ttggaaagtg gtttttagtt tttggagttg ttggttggta aagtgagttt gttgttgttt 4200
ttgttgtttt tttttagatg ggtttttggt gtttatgttt ttattttttt tttgttggtt 4260
tttatttttt tttgaaaatg aaatatatat atttttttgt tagtatgttt atttgtaatg 4320
tggatgttaa ttggattggt ggtagaagtt gtggaagagt tgggttgttt ggtgttggag 4380
gagggtgtgt gtggtggttt tgggttgtga ggagtgttgt gtttgtgggg tgtgtaggtg 4440
taagtgtggg tgtttgtgtt ttattttttt ttttttttta gtgttgtatg ttttatttat 4500
atgtttattt tattgtagtg gtatatttat ttttatagtt tgtgttttta agtatattta 4560
tatattttg tgtagatata ttaaattttt tgggatgtgt atatgtgtgt ggtttataga 4620
ttttttttt ttttgtagaa agtttagatt tttatgtggt ttgggaaggt taggaaaaga 4680
tgtggggatt tggttgggta ttgaagtttg ttggtttttt tttaaaaaaa aaaaaaaaat 4740
gtttttttgt gaagggtatt tttgagtggt tttaggtaat tttttaatga gtggagtttt 4800
ttgggagttg aaagttgaga ggaaaatagg gatagaggtt ggtggttttt gaaggttttt 4860
gaattaagat gttgggattt ttgtgattta ggaaatagaa gggaggttag ggtatgaata 4920
gagagggtgg tagaattgtt tgtgttttta gtgttttagg agttgggttg gttgagggag 4980
aattaaaggg atgtggggta gttaaaattt tggtttttgg aagttttgtg gggagttagg 5040
tgaatgatta tttttattat gttttttttt ggaggggttg atttttttgg ggtgagaggg 5100
agtgggtggt gtagagtagt tgagtgggaa tgtttgtagg gtggtgtggt gttttatttg 5160
tggtttttgg gttggaggtg ttggagatgg tgtgtatttt tagtttgtgt ttggaggagt 5220
ttagtgattg gggttgattg ggagttagaa ttgaagttat ggttaatggt tggggatggt 5280
gataggaaga tgaggagatg gttgatagtt tggtttttgt tgtttggtgt tttaagtgaa 5340
gtgggttttt tatgtagttt atggatgagg gagtgtgatg ttttattagt ttttggttat 5400
tgttttgttg agtttttgta gttgttgttg tttgttttgg gttgtgtttt aggtgtggag 5460
tttttttgtt gtggggagag ttaggggatg taatttttgt tgagttttta agttaagttg 5520
tttttgtttt ttttggaagg tttaagtgaa aaagtttgga gatggaaagt tagtgggtaa 5580
atgaagatat gggatgtggg tagaagggta ttatttagag tgtttttagg gagtaggttt 5640
ttaagtttta aagtgaaata agagtgggta aagatttttt tttttttttt tttttttttt 5700
taagaatttt tttaataagg aaagttaatg ttgattgtgt tttgtttgtt tttttttat 5760
gtggtagttt tgatagagaa gtgttaagag tgatagggat aggtaggtga tattagattt 5820
tttgtggtgg tagtagttgt tgtagttatg atgtggtttt ttgagtgtat tttttgtaat 5880
gtgtatatgt atattttttg ggtggttgaa taggagttgg gttttgttgt agtttagttt 5940
taggtattta ggtgagtgat ggattagatt tgtggttttg tgttttttg ttggtttaat 6000
attttaaaat tagaggtggg tttttggtg ttgagatgtt attttgttgt ggtttttttt 6060
agtttttttt gtttttgttt tttttagat tttttttgg gtgtgattga tgtggttttg 6120
tattaattag gatgttttga gttgtggtgg agggattgtt ttgtttgtat ttattagtag 6180
tgtggggttg ggttattgtt ttgttgtgtg tattgggttt atataggtaa gtttttggga 6240
atttagtttt tgtttagttt aaggtgattt ggtttttagt atgaatttaa aggtgaagag 6300
atgaggttag gagttgaagg tttgggagaa gagagtggaa tggttaagaa gagaaaggta 6360
taaggattaa taagatattt attttttgtg ttttattata tttattttta attttttatt 6420
ttatataaaa aggagatatg ttatttaaaa ttagaaaatt tgaaaaatag taataaatta 6480
tttttttgat tttaaatttt ttaaatagtt tgttaagtga atgttgtgtt aatttgaaga 6540
agttttaatt gtaaagaaga tagagttttg aaaaggtagg ttaataaatt agaaattgag 6600
aagtaaatgg atttgttaaa agaaaattat tttgatttta aatgaataat tgtttggtgg 6660
tttattttgg atttatataa gaataaaaag ttgttttaga ttatgttttt tgtgatgttt 6720
attagttttt agatagaaaa tatataatag aagagaaatt ttaatttagt gtttttaaaa 6780
tgttgaaagt ttatttattt tatttaatgt tgattaagat atatatttta gatttttttaa 6840
atttttgta tattgtatta agtttgtttt aatttgagag agttatgttt taaatttgat 6900
ttttttgttt attttattat taattagatt taaatttata aagtttgtag aattaataat 6960
tttgagttaa ttatatatga aatatgtttt aatgaatttt tatataatta agaatgttgt 7020
taaataatta attttaagga taattttaa tagttatttt ttttttttag tgagtttaag 7080
gttgttttga gttattaaag tttaagtagg tagaaggggt gtgtgtgagt taagggtgaa 7140
aagtttagaa ttgtgtttaa ttagtaaaag taaaatttta tttatataaa ataaaaaaaa 7200
ttatttttgg agatattaat tttttatagt attgttttta agtaaattta atttttaaag 7260
aaattaaaga aagaaattta aatatattta aaataatttt tgaaagtttt tttgtttttt 7320
agtataggtt agttggagag gataaattaa tttttttgg gtttttgtat gggtgattgt 7380
tttattatgg agttagtgtt attattttg aatgtgtatt tgtttgatat tatagttaat 7440
gatttgtaat gttagtatga agtattttta aaatattttt tttttgtttt tgtttataag 7500
attgggaaat ttatttgatg tggaataaag tggatgaagt agattataaa tatatttgta 7560

```
atttatgtgt ttttttttg ttttgattat ttttaaattt tatttgtaat ttttttttat 7620
tttaaatttg tagtttaaag atgtatatga gaattgtttt ttagtttttt tttattagta 7680
ttattttatt ttaagaataa tttagttgta agggaggaat ttttttatag taagttttaa 7740
attagtattt ttgtttttaa tttttttattt tattttattt tattttatat atatagatat 7800
ttgtttagag taaaatatat ttttatgtga taggtttgta ttagttgagg tttatatatt 7860
tagttatatt aggttttgta atttattat taaattatat atattatatt agtagtttgt 7920
tggtaaagaa ggttaaatta atttatattt tgtttattat ttggtgttta aatgatgtat 7980
tttattttgg agatttggtg gagaattttt tttttagatt ttatagtgtt ttattgaaga 8040
taatgttttt atatttgtag tggtttttaa tttgataaga ttttaatttg tttaagtttt 8100
ttaaataagg gttttaaatg tttttagttg tttttttatt gaattttttt taattttttt 8160
aagattataa agtatatgtg taaagtaaat attttttttt attgtattgt tagttgatga 8220
tttataatta agttaataag aatttagttt ttttttgttg aatgtgttta ttaattatat 8280
tttagttttt ttttttaaat tttagaatag ttgtggtttt tataatatta tgttttttaa 8340
agttttattt tatgaaggga ttttattata ttaaagaatg aaaaaaattt ttattgtagt 8400
tagtatatat agttttttat tttttgtttt ttaagattta aattttagag ttgtaaatat 8460
ttttggaagt ttgggtgtta atgttttatt ttagaaagtt gagaagtttt atagagttat 8520
atagattttt aaatttattt tttataaatt tatagaattt tgataaaagt tttggtggtt 8580
ttattttatt gatggaattt ttattatgat aaatatatat gtatgaagga ttttaattag 8640
tttttaaagt ggttgaaaaa tttaagggta tgtgattgtt ttttatagtg ttaatgtgtg 8700
tgagatgttg gaagtattgg ggattagtag tagtttagat gtttaaaaag ataaggtgtt 8760
ttaatttgtg tggatttatt gaagttaagt ggtgaataaa gataattatt tagataattt 8820
agattaaagt aaaagtaaaa ttatatttat ttgtatatat atatttatat ttattttata 8880
ttatagatat atatatgtat atatatattg gttttgtaaa taattgattt aaagtgagga 8940
ttttttttgt attttttttag taggagtttt aatattttttt taattttttta attattttat 9000
a 9001
```

<210> 5
<211> 9001
<212> DNA
<213> Artificial Sequence

<220>
<223> chemically treated genomic DNA (Homo sapiens)

<400> 5

```
tgtaaagtga ttaggagatt aggagaatgt tgaaattttt gttggaaaaa tgtaaagaaa      60
atttttattt tgagttagtt gtttatagag ttagtgtgtg tgtgtgtgtg tgtgtttgta     120
atataaaatg gatgtgaata tatatatata aatagatatg gttttgtttt tattttaatt     180
tgaattattt agataattgt ttttatttat tatttgattt taatgggttt atataaatta     240
ggatatttta tttttttagg tatttaggtt gttgttgatt tttagtgttt ttaatatttt     300
gtatatgttg gtattatgag gagtagttat gtgtttttgg gtttttttaat tattttggag     360
gttgattgag gtttttttata tatgtatatt tgttgtgatg aaagttttat tggtagagtg     420
gagttattag agtttttatt aaaattttgt gggtttatga gagatgggtt tagaaattta     480
tatggttttg tggggttttt tggtttttta aaataaggta ttaatattta agtttttaaa     540
aatatttgta gttttggggt ttgaattttg aaaaataagg agtgaggggt tgtgtatatt     600
aattatagtg gagattttttt ttattttta atgtgatgga gttttttttat gaaatgaagt     660
tttaaggggt atggtattgt ggggattata gttattttga ggtttaaaag aagaaattgg     720
aatatgatta gtaaatatat ttagtagaaa agagttggat ttttattgat ttagttatag     780
gttattggtt ggtagtgtaa tgggaggaaa tatttatttt atatatatat tttatgattt     840
tgggggaatt agaggaaatt taataagaaa atggttagaa atatttaaaa tttttattta     900
aaagatttaa gtaaattaga gttttattag attaaaaatt attataaatg taagagtatt     960
gtttttagtg aaatgttgtg gggtttgaga aggagatttt ttgttaaatt tttgggataa    1020
aatgtgttat ttaagtatta gataatgagt agaatgtaaa ttaatttaat tttttttatt    1080
aataggttgt tagtgtaatg tgtataattt agtgataaga ttgtaggatt taatatagtt    1140
ggatgtatga gttttagtta atgtagattt gttatatgag gatgtgtttt attttgagta    1200
ggtgtttgta tgtgtggaat ggggtaaagt ggaataaaag gttaaaagta gaaatgttga    1260
tttaaagttt attatgaaga aattttttttt ttgtagttaa attattttta aagtgggatg    1320
atattggtga agaaagattg aaaaataatt tttatgtgtg tttttggatt gtaagtttaa    1380
aatggggagg agttgtagat agggtttggg ggtggttagg gtaaaggaga gatatataag    1440
ttgtaaatat atttgtagtt tgttttattt attttgtttt atattgaata agtttttttaa    1500
ttttgtgaat aaggataagg agggagtgtt ttaaagatat tttatgttgg tattgtaaat    1560
tattgattgt aatgttaaat aaatatatat ttagagatga taatattaat tttatagtaa    1620
```

aataattgtt tatgtagaaa tttagaggag attagtttgt tttttttagt tgatttatgt 1680
tggggataa aaggattttt aaaaattatt ttgaatatgt ttggattttt tttttaatt 1740
tttttggaaa ttaaatttgt ttggaaatag tgttataaag agttgatgtt tttaaaggtg 1800
atttttttg ttttatataa ataaggtttt gtttttgtta gttgagtgta gttttaggtt 1860
ttttgttttt agtttatata tattttttt gtttgtttgg attttaatgg tttaagatag 1920
ttttgagttt attgggaaaa gaaaatgatt gttaaaaatt atttttgaaa ttggttattt 1980
ggtaatattt ttaattgtat ggaatttat taaggtatat tttatatata attagtttaa 2040
ggttgttgat tttataggtt ttatggattt aaatttgatt gataataaag taaataagag 2100
agttgaattt aaagtgtggt ttttttgggt taggatgagt ttaatatagt gtataaggaa 2160
tttgaaagat ttaggatatg tgttttaatt aatgttaagt agaatggata agtttttagt 2220
attttgaaaa tgttgggtta gggtttttt tttattgtgt gtttttttgtt tggggattaa 2280
taagtattat agagaatgtg atttgaggtg attttttatt tttgtataaa tttagagtga 2340
attattaaat agttgtttgt ttaaagttaa ggtaattttt ttttgatggg tttatttgtt 2400
ttttgatttt taatttatta gtttgttttt ttagggtttt gttttttttg taattaaagt 2460
ttttttagat tagtgtagta tttatttgat aggttgtttg gaaaatttaa gattggagag 2520
gtgatttgtt gttgtttttt aaatttttta gttttaagta atgtgttttt tttttatatg 2580
gggtggggga ttggaaatgg atgtagtgag atataaagag tgggtgtttt gttgattttt 2640
gtattttttt ttttttgatt attttatttt ttttttttaa gtttttgatt tttagtttta 2700
ttttttttatt tttgggtttg tattaaaagt tggattgttt tgggttgggt aggagttgaa 2760
tttttgggag tttgtttgtg tagatttagt gtgtatggtg aggtagtagt ttggttttgt 2820
attgttgata ggtgtaggta ggatagtttt tttattgtgg tttggggtgt tttgattggt 2880
gtggagttat gttagttgta tttggagaag ggtttgggag gaggtggagg tggagagggt 2940
tggggagggt tgtggtggag tgatgttttg gtattaggaa gtttgttttt ggttttaaga 3000
tgttaggtta atagggaagt gtggagttgt agatttggtt tgttgtttgt ttgggtgttt 3060
ggagttgagt tgtggtaagg tttggttttt gtttgattgt ttgaggggtg tgtgtgtgtg 3120
tgttgtggag ggtgtgttta gagggttgtg ttgtggttgt agtggttgtt gttgttgtag 3180
gggatttaat attatttatt tgtttttgtt atttttgata tttttttgtt agggttgttg 3240
tgtggggggg gggtgggtag agtgtggttg gtgttagttt tttttattgg aggggttttt 3300
gggggaggga gggagagaag aagggggttt ttgtttattt ttgttttgtt ttggagtttg 3360
gaagtttgtt ttttaaagat gttttgagtg gtgttttttt gtttatattt tatgtttttg 3420
tttgtttgtt gatttttttgt ttttggattt ttttgtttga gtttttttgga ggagatgggg 3480
gtagtttggt ttgagaattt ggtgggggtt gtgtttttttg gttttttttg tagtggggaa 3540
atttgtgtt tagagtgtga tttggagtgg gtagtggtgg ttatgggggt ttggtggggt 3600
agtagttaag gattagtaga gtgttgtgtt tttttgttta tgaattgtat gaaaggtttg 3660
ttttatttgg agtattgagt agtggggatt aagttgttgg ttgttttttt atttttttgt 3720
tattattttt agttgttagt tatggttttg gttttggttt ttggttagtt ttggttgttg 3780
gatttttttta agtataggtt ggaggtgtat attattttttg atatttttag tttggaggtt 3840
gtaggtaagg tgttgtgttg ttttgtagat atttttgttt agttgttttg tgttatttgt 3900
tttttttttgt tttaaggaag ttagtttttt tgggggggagg tgtggtggga gtggttgttt 3960
gtttggtttt ttgtagaatt tttgggagtt ggaattttga ttattttgta ttttttttagt 4020
tttttttttga ttggtttggt ttttggggtg ttaagggtgt gagtaatttt gttgtttttt 4080
ttatttgtat tttggttttt tttttgtttt ttgggttata aaaattttag tattttgatt 4140
tgaggatttt tagaggttgt tgatttttgt ttttgttttt tttttggttt ttagtttttg 4200
aggagtttta tttgttagga aattgtttga aattatttag aaatgttttt tgtgaagagg 4260
tattttttttt tttttttttgg gaaagggttg gtgaattttg gtgtttaatt gaatttttat 4320
atttttttttt agtttttttta aattgtatgg aaatttgagt tttttgtgag ggggaggggg 4380
gtttgtaaat tatgtgtgtg tgtgtgtttt aggagatttg gtgtgtttgt gtagaggtgt 4440
ataaatatat ttgaaagtat aggttataaa agtgaatgtg ttgttgtagt gagataaata 4500
tgtaaataaa atgtgtggtg ttgggggagg ggaggaaatg gggtgtggat atttatattt 4560
gtgtttgtat attttatagg tgtagtgttt tttgtggttt ggagttgttg tgtgtatttt 4620
tttttggtgt taggtagttt agtttttttta tggtttttgt tgttggttta gttggtgttt 4680
gtgttgtagg tgggtatgtt gatgggaaag tgtgtgtgtt ttgtttttag agaaagataa 4740
aagttagtag gggaagaatg aggatgtggg tgttgaggat ttgtttaaga agaagtggta 4800
aaggtggtag tggatttatt ttattagtta gtagttttag gagttggagg ttattttttta 4860
gaggaattgt tatttggata tgtttatatg tgaagaaatt gttgtgtgga ttaattttat 4920
ggaagtttga gtttgggtag gagttagtat ggagtttggg agggatgggg ggaggatgtt 4980
gtggaggtat aggttaagta gattaggaga gaatgtggaa ggtagtgttg tttgggaggg 5040
tgttggtggg gtgtagtttt gtaaaggtag aaggttttgt ggtggtttgg ttgtgagatt 5100
atagtttttt ttttgaggtt gataggattg ttgttttggt ttaggttttt agagtggtat 5160
tggtttattg ttttgttatt ttgtgatttt atgagttggg ttgtatgggt aatttttttgt 5220
ataggatatt gtgtttttgg tttgtagttg ttagagtaga gttaataaaa tttttattag 5280
gttaagagtt gtgaataggt tttaatttgt gagtttttaa taaggaaaat ttgttagaga 5340
tatggaagag ttggtttttt ttgggaaatt tttgttttgg ttttggttta gttttttttt 5400

```
ttttgggttt gtgtttttta tatttttttt atggttgttt tggttattta ggtttttttt 5460
atatatttta ttttttagtt ttgtgatttt tgggagtaaa gttttaatat ataattatta 5520
gtttttttag aaggagaaag aaaaaaagaa gaaagatttt tttgtttggt ttatttattt 5580
tttttttagga gttgaatttt ggaaattgaa atttatattt ttttttttaa attataatta 5640
tagttttgta aaaagggttt attttaattt tgtagtaaat ttgtatttta tggattggta 5700
aaaatgagtt taaataaata atttaatagt aatgttttgg tttatgttgg ttggtggaag 5760
attttaaatt tgttaggatt ttggaagtag aaaatagaat taagtaaatt aagtggtatt 5820
tagaggtttt gttgttaaaa aaaaaaaatt aagtgttttg ggtagaaaaa ataaagtttt 5880
tggttagagt agagtaaata aaaagaagaa aataatgata aaaagaataa agattaaaat 5940
gtttttttaa attagaggga atgaagatat tttttgggtg gtatttgtgt aaggtatgag 6000
gttatgttgg tggataaaag gttgggaaga agttgaaaat ggttttagtt taattgttta 6060
gagttagagt tgggttttgg gtggtgtggt tttgagtaag gttagttttt tattagtttt 6120
tttgtatatt aagggaatgg gtttttttatg tatttttttt gtttgagtaa agtttagatg 6180
gtttagggta gaaatggtaa gtaattaaag atagagttta tgggtttttt gggatttttt 6240
gaaaatgttt ttttattttg tttgttattt tgtagtttta ttttagtgtt ttgtagttgt 6300
ggtgttgggt tttttttgta gttgtttttt tttttagggt ggttgtttgt tgagttaagt 6360
gggagtgagg tgtgtttttt atagtagttg ggtgtaaaga ggaaggggga taaaaaggaa 6420
attaagaatg aaaggaaaaa gagaaaaagt ggattatatg gttgggtttg gtggagatgt 6480
gtaatgtgaa atattattgg tgttagtttg gatattttag gttaggtttt tttttaatat 6540
ataaaagttg ttgtttgggg tgatagggag gtttgatgtg gattgggatt ggggttgtgg 6600
ttgggttatt ggatatgggt ggaagttggt tggtttgggt ggttgtttgt aaagttaaat 6660
gatttggttg ggtttggtgt gtggataggt ttgtggtggg tttagggtaa agaagaggta 6720
gagtgaaaga aggggggaatt tttaaaatta tttttttttgg gtttttggag tttaatatgt 6780
taagtttttg gagttaatga gttgatgaag aggtggtttt ttgtttttta tttggttgtt 6840
ttgttaggtg agaaagagtg ttggtggttt agtttttgtt aagggagtat gtattagggg 6900
gtgggggatg atagtggagg ttagggaagg aagggaggaa ttgtgtggga gaaagagtga 6960
tttttttagtg tttttttagt tttttttttt tatttgtggg tttgtggttt tggaatggaa 7020
gtaagtttgt aaggtgtttt gggaagggtt ggaaaagttt gttgttttgt gtttgtttta 7080
tattaagtgt ttttggattt ggagaaatgt ttggttgagt gattaaattg tttgtaggtt 7140
tttatgtgtt tggttgaggt ttgtggtgta gttttgagtt ttagtttgta ggttagagta 7200
gattaggttt tttgtgtttg gtggagattt gggttagtaa ttgaaagttg gttttggtat 7260
tttggtgtgt agggtggtgt agtgaagtga ggttagggtg tgtgagtgtg ttagtgtgtg 7320
tgttggggga aggtggggggt tggtttttga tggaagtttt agtaatttgt attgtggtat 7380
ttgtttgttt ttttgtttta attgttttta ggtttggttt aagaattgtt gggttaaatg 7440
gagaaagagg gagtgtaatt agtaggttga gttatgtaag aatggttttg ggttgtagtt 7500
taatgggttt atgtagtttt atgatgatat gtatttaggt tatttttata ataattgggt 7560
tgttaagggt tttatatttg ttttttttatt tattaagagt tttttttttt ttaattttat 7620
gaatgttaat tttttgttat tatagagtat gtttttttta tttaatttta ttttgtttat 7680
gagtatgttg tttagtatgg tgtttttagt agtgataggt gttttgggtt ttagttttaa 7740
tagtttgaat aatttgaata atttgagtag tttgttgttg aattttgtgg tgttgatgtt 7800
tgtttgtttt tatgtgttgt tgattttttt gtatgtttat agggatatgt gtaatttgag 7860
tttggttagt ttgagattga aagtaaagta gtattttagt tttggttatg ttagtgtgta 7920
gaatttggtt tttaatttga gtgtttgtta gtatgtagtg gattggtttg tgtgagttgt 7980
atttatagtg ttgggatttt aggattttgt tggatggggt aattttgttt ttgaaagatt 8040
gggaattatg ttagaaggtt gtgggtatta aagaaaggga gagaaagaga agttatatag 8100
agaaaaggaa attattgaat taaagagaga gttttttttga ttttaaaggg atgtttttag 8160
tgtttgatat tttttattat aagtattttt aatagttgta aggatatata tataaataaa 8220
tgtttgattg gatatgatat tttaatatta ttataagttt gttatttttt aagtttagta 8280
ttgttaatat ttaaatgatt gaaaggatgt atatatattg aaatgttaaa ttaattttat 8340
aaaagtagtt gttagtaata ttataatagt gtttttaaag gttaggtttt aaaataaagt 8400
atgttatata gaagtgatta ggattttttg tttgtgagta agggagtgta tatattaaat 8460
gttatattgt atgtttttaa tatattatta ttattataaa aaatgtgtga atattagttt 8520
tagaatagtt tttttggtgg atgtaatgat gtttttgaaa ttgttatgta taatttattt 8580
tgtgtataat attttgtata atattattgt tttatttttt agtaaatatg aaataaatgt 8640
gttttatttt atgggagtaa aatatattgt atataaattg gtttggattt tttttttttt 8700
tttttgttat taatttggtt aggatatttt agttattgtt ttttaaataa attagttttt 8760
tttgtttgtt tagttaaata tataaggtag tagtttttat ttaaatttgg tagaaataaa 8820
tgatagttat ttattagaaa ttaaaaagaa aaaaaaaggt atttttgggg gggaaaaggg 8880
ttataaaatt taattttgtt tttttaattt ttttttggtt taaatttaga ggattttatt 8940
atggttagta aataatatga aaaagaaaaa agaagaaaga aatttagtaa gtttattagt 9000
t 9001
```

<210> 6
<211> 22
<212> DNA
<213> Artificial Sequence

<220>
<223> PRIMER

<400> 6
gtaggggagg gaagtagatg tt 22

<210> 7
<211> 24
<212> DNA
<213> Artificial Sequence

<220>
<223> PRIMER

<400> 7
ttctaatcct cctttccaca ataa 24

<210> 8
<211> 20
<212> DNA
<213> Artificial Sequence

<220>
<223> PROBE

<400> 8
agtcggagtc gggagagcga 20

<210> 9
<211> 27
<212> DNA
<213> Artificial Sequence

<220>
<223> PROBE

<400> 9
agttggagtt gggagagtga aaggaga 27

<210> 10
<211> 25
<212> DNA
<213> Artificial Sequence

<220>
<223> PRIMER CONTROL

<400> 10
tggtgatgga ggaggtttag taagt 25

<210> 11
<211> 27
<212> DNA

<213> Artificial Sequence

<220>
<223> PRIMER CONTROL

<400> 11
aaccaataaa acctactcct cccttaa 27

<210> 12
<211> 30
<212> DNA
<213> Artificial Sequence

<220>
<223> PROBE CONTROL

<400> 12
accaccaccc aacacacaat aacaaacaca 30

<210> 13
<211> 408
<212> DNA
<213> Homo Sapiens

<400> 13

```
tcctcaactc tgcaggcctg aaagaaggtc acacacgcac gctcacaccc acactccaca      60
cgcctcgtcc caaacaaccc catgaacatt gtcctttgtt ccgtctcttg ggccactttc     120
cctgtcgctt cctcccagcc cgtcctgatt tgctccccaa aagtacgttt ctgtctcccc     180
gctgccctgg cgctccccct ttgatttatt agggctgccg ggttggcgca gattgctttt     240
tcttctcttc catcccatcc tcccttctgg tcctcctttc cacagtggga gtccgtgctc     300
ctgctcctcg gttggctcct aagtgccccg ccaggtcccc tctcctttcg ctctcccggc     360
tccggctccc gactcttcgg cccgctggca tctgcttccc tcccctgc                  408
```

<210> 14
<211> 22
<212> DNA
<213> Artificial Sequence

<220>
<223> chemically treated genomic DNA (Homo sapiens)

<400> 14
aacatctact tccctcccct ac 22

<210> 15
<211> 32
<212> DNA
<213> Artificial Sequence

<220>
<223> chemically treated genomic DNA (Homo sapiens)

<400> 15
gttagtagag attttattaa attttattgt at 32

<210> 16

<211> 14
<212> DNA
<213> Artificial Sequence

<220>
<223> chemically treated genomic DNA (Homo sapiens)

<400> 16
ttcggttgcg cggt 14

<210> 17
<211> 16
<212> DNA
<213> Artificial Sequence

<220>
<223> chemically treated genomic DNA (Homo sapiens)

<400> 17
tttggttgtg tggttg 16

<210> 18
<211> 144
<212> DNA
<213> Homo Sapiens

<400> 18

```
ttctggtcct cctttccaca gtgggagtcc gtgctcctgc tcctcggttg gctcctaagt     60
gccccgccag gtcccctctc ctttcgctct cccggctccg gctcccgact cttcggcccg    120
ctggcatctg cttccctccc ctgc                                           144
```

<210> 19
<211> 162
<212> DNA
<213> Homo Sapiens

<400> 19

```
tggcatctgc ttccctcccc tgcctcgttt ctcgtcgccc ctgctcgctc cccccggcgc     60
tcgcccgggc gctgtgctcg ctcctggatc gccagccgcg cagcgggctc gccggcgccc    120
gcgcgccact gtgcagtgga gtttggtgga atctctgctg ac                       162
```

<210> 20
<211> 2235
<212> DNA
<213> Homo Sapiens

<400> 20

```
tgggagtccg tgctcctgct cctcggttgg ctcctaagtg ccccgccagg tcccctctcc      60
tttcgctctc ccggctccgg ctcccgactc ttcggcccgc tggcatctgc ttccctcccc     120
tgcctcgttt ctcgtcgccc ctgctcgctc cccccggcgc tcgcccgggc gctgtgctcg     180
ctcctggatc gccagccgcg cagccgggct cggccggccg cccgcgcgcc actgtgcagt     240
ggagtttggt ggaatctctg ctgacgtcac gtcactcccc acacggagta ggagcagagg     300
gaagagagag ggatgagagg gagggagagg agagagagtg cgagaccgag cgagaaagct     360
ggagaggagc agaaagaaac tgccagtggc ggctagattt cggaggcccc agtgcacccg     420
tggactcctt cggaacttgg caccctcagg agccctgcag tcctctcagg cccggctttc     480
gggcgcttgc cgtgcagccg gaggctcggc tcgctggaaa tcgccccggg aagcagtggg     540
acgcggagac agcagctctc tcccggtagc cgataacggg gaatggagac caactgccgc     600
aaactggtgt cggcgtgtgt gcaattaggc gtgcagccgg cggccgttga atgtctcttc     660
tccaaagact ccgaaatcaa aaaggtcgag ttcacggact ctcctgagag ccgaaaagag     720
gcagccagca gcaagttctt cccgcggcag catcctggcg ccaatgagaa agataaaagc     780
cagcagggga agaatgagga cgtgggcgcc gaggacccgt ctaagaagaa gcggcaaagg     840
```

```
cggcagcgga ctcactttac cagccagcag ctccaggagc tggaggccac tttccagagg     900
aaccgctacc cggacatgtc cacacgcgaa gaaatcgctg tgtggaccaa ccttacggaa     960
gcccgagtcc gggtttggtt caagaatcgt cgggccaaat ggagaaagag ggagcgcaac    1020
cagcaggccg agctatgcaa gaatggcttc gggccgcagt tcaatgggct catgcagccc    1080
tacgacgaca tgtacccagg ctattcctac aacaactggg ccgccaaggg ccttacatcc    1140
gcctccctat ccaccaagag cttccccttc ttcaactcta tgaacgtcaa cccccctgtca   1200
tcacagagca tgttttcccc acccaactct atctcgtcca tgagcatgtc gtccagcatg    1260
gtgccctcag cagtgacagg cgtcccgggc tccagtctca acagcctgaa taacttgaac    1320
aacctgagta gcccgtcgct gaattccgcg gtgccgacgc ctgcctgtcc ttacgcgccg    1380
ccgactcctc cgtatgttta tagggacacg tgtaactcga gcctggccag cctgagactg    1440
aaagcaaagc agcactccag cttcggctac gccagcgtgc agaacccggc ctccaacctg    1500
agtgcttgcc agtatgcagt ggaccggccc gtgtgagccg cacccacagc gccgggatcc    1560
taggaccttg ccggatgggg caactccgcc cttgaaagac tgggaattat gctagaaggt    1620
cgtgggcact aaagaaaggg agagaaagag aagctatata gagaaaagga aaccactgaa    1680
tcaaagagag agctcctttg atttcaaagg gatgtcctca gtgtctgaca tctttcacta    1740
caagtatttc taacagttgc aaggacacat acacaaacaa atgtttgact ggatatgaca    1800
ttttaacatt actataagct tgttattttt taagtttagc attgttaaca tttaaatgac    1860
tgaaaggatg tatatatatc gaaatgtcaa attaatttta taaaagcagt tgttagtaat    1920
atcacaacag tgtttttaaa ggttaggctt taaaataaag catgttatac agaagcgatt    1980
aggatttttc gcttgcgagc aagggagtgt atatactaaa tgccacactg tatgtttcta    2040
acatattatt attattataa aaaatgtgtg aatatcagtt ttagaatagt ttctctggtg    2100
gatgcaatga tgtttctgaa actgctatgt acaacctacc ctgtgtataa catttcgtac    2160
aatattattg ttttactttt cagcaaatat gaaacaaatg tgttttattt catgggagta    2220
aaatatactg catac                                                     2235
```

<210> 21
<211>
<212> protein
<213> polypeptide Sequence

<220>
<223> amino acid sequence (Homo sapiens)

<400> 450

```
METNCRKLVSACVQLGVQPAAVECLFSKDSEIKKVEFTDSPESRKEAASSKFFP
RQHPGANEKDKSQQGKNEDVGAEDPSKKKRQRRQRTHFTSQQLQELEATFQRNR
YPDMSTREEIAVWTNLTEARVRVWFKNRRAKWRKRERNQQAELCKNGFGPQFNG
LMQPYDDMYPGYSYNNWAAKGLTSASLSTKSFPFFNSMNVNPLSSQSMFSPPNS
ISSMSMSSSMVPSAVTGVPGSSLNSLNNLNNLSSPSLNSAVPTPACPYAPPTPP
YVYRDTCNSSLASLRLKAKQHSSFGYASVQNPASNLSACQYAVDRPV             450
```

<210> 22
<211> 21
<212> DNA
<213> Artificial Sequence

<220>
<223> chemically treated genomic DNA (Homo sapiens)

<400> 22
gtaggggagg gaagtagatg t 21

<210> 23
<211> 23
<212> DNA
<213> Artificial Sequence

<220>
<223> chemically treated genomic DNA (Homo sapiens)

<400> 23
tcctcaactc tacaaaccta aaa 23

<210> 24
<211> 16
<212> DNA
<213> Artificial Sequence

<220>
<223> chemically treated genomic DNA (Homo sapiens)

<400> 24
agtcgggaga gcgaaa 16

<210> 25
<211> 16
<212> DNA
<213> Artificial Sequence

<220>
<223> chemically treated genomic DNA (Homo sapiens)

<400> 25
agttgggaga gtgaaa 16

<210> 26
<211> 17
<212> DNA
<213> Artificial Sequence

<220>
<223> chemically treated genomic DNA (Homo sapiens)

<400> 26
aagagtcggg agtcgga 17

<210> 27
<211> 17
<212> DNA

<213> Artificial Sequence

<220>
<223> chemically treated genomic DNA (Homo sapiens)

<400> 27
aagagttggg agttgga 17

<210> 28
<211> 16
<212> DNA
<213> Artificial Sequence

<220>
<223> chemically treated genomic DNA (Homo sapiens)

<400> 28
ggtcgaagag tcggga 16

<210> 29
<211> 16
<212> DNA
<213> Artificial Sequence

<220>
<223> chemically treated genomic DNA (Homo sapiens)

<400> 29
ggttgaagag ttggga 16

<210> 30
<211> 16
<212> DNA
<213> Artificial Sequence

<220>
<223> chemically treated genomic DNA (Homo sapiens)

<400> 30
atgttagcgg gtcgaa 16

<210> 31
<211> 16
<212> DNA
<213> Artificial Sequence

<220>
<223> chemically treated genomic DNA (Homo sapiens)

<400> 31
tagtgggttg aagagt 16

<210> 32
<211> 24
<212> DNA
<213> Homo Sapiens

<400> 32

gaaaggcaga gtcataacag gaag 24

<210> 33
<211> 26
<212> DNA
<213> Homo Sapiens

<400> 33
taaggataga gtgatttcca agaaag 26

<210> 34
<211> 24
<212> DNA
<213> Homo Sapiens

<400> 34
cttatctgac aacaagcgag tatg 24

<210> 35
<211> 25
<212> DNA
<213> Homo Sapiens

<400> 35
caattattgt attgtagcat cggag 25

<210> 36
<211> 24
<212> DNA
<213> Artificial Sequence

<220>
<223> chemically treated genomic DNA (Homo sapiens)

<400> 36
ttctaatcct cctttccaca ataa 24

<210> 37
<211> 22
<212> DNA
<213> Artificial Sequence

<220>
<223> chemically treated genomic DNA (Homo sapiens)

<400> 37
gtaggggagg gaagtagatg tt 22

<210> 38
<211> 144
<212> DNA
<213> Artificial Sequence

<220>
<223> Example 7 amplificate 1

<400> 38

```
gcaggggagg gaagcagatg ccagcgggcc gaagagtcgg gagccggagc cgggagagcg      60
aaaggagagg ggacctggcg gggcacttag gagccaaccg aggagcagga gcacggactc     120
ccactgtgga aaggaggacc agaa                                            144
```

<210> 39
<211> 24
<212> DNA
<213> Artificial Sequence

<220>
<223> chemically treated genomic DNA (Homo sapiens)

<400> 39
ttctaatcct cctttccaca ataa 24

<210> 40
<211> 30
<212> DNA
<213> Artificial Sequence

<220>
<223> chemically treated genomic DNA (Homo sapiens)

<400> 40
gagattttat taaattttat tgtatagtgg 30

<210> 41
<211> 279
<212> DNA
<213> Artificial Sequence

<220>
<223> Example 7 amplificate 2

<400> 41

```
gagattccac caaactccac tgcacagtgg cgcgcgggcg gccggccgag cccggctgcg      60
cggctggcga tccaggagcg agcacagcgc ccgggcgagc gccgggggga gcgagcaggg     120
gcgacgagaa acgaggcagg ggagggaagc agatgccagc gggccgaaga gtcgggagcc     180
ggagccggga gagcgaaagg agaggggacc tggcggggca cttaggagcc aaccgaggag     240
caggagcacg gactcccact gtggaaagga ggaccagaa                            279
```

<210> 42
<211> 24
<212> DNA
<213> Artificial Sequence

<220>
<223> chemically treated genomic DNA (Homo sapiens)

<400> 42
ttttggaaag tggtttttag tttt 24

<210> 43
<211> 19
<212> DNA

<213> Artificial Sequence

<220>
<223> chemically treated genomic DNA (Homo sapiens)

<400> 43
ccaaacaacc caactcttc 19

<210> 44
<211> 234
<212> DNA
<213> Artificial Sequence

<220>
<223> Example 7 amplificate 3

<400> 44

```
ctctggaaag tggcctccag ctcctggagc tgctggctgg taaagtgagt ccgctgccgc      60
ctttgccgct tcttcttaga cgggtcctcg gcgcccacgt cctcattctt cccctgctgg     120
cttttatctt tctctgaaaa cgaaacacac acactttccc gtcagcatgc ccacctgcaa     180
cgcggacgcc aactggaccg gcggcagaag ccgtggaaga gctgggctgc ctgg           234
```

<210> 45
<211> 22
<212> DNA
<213> Artificial Sequence

<220>
<223> chemically treated genomic DNA (Homo sapiens)

<400> 45
gatgttggga tttttgtgat tt 22

<210> 46
<211> 20
<212> DNA
<213> Artificial Sequence

<220>
<223> chemically treated genomic DNA (Homo sapiens)

<400> 46
cccaaaaaaa tcaacccctc 20

<210> 47
<211> 224
<212> DNA
<213> Artificial Sequence

<220>
<223> Example 7 amplificate 4

<400> 47

```
gatgctggga tttttgtgac ccaggaaaca gaagggaggc cagggtacga atagagaggg     60
cggcagaatt gctcgcgccc ttagcgcccc aggagccggg ccggtcgagg gagaactaaa    120
gggatgcggg gtagtcaaaa ttccggctcc cggaagttct gcggggagcc aggcgaacga    180
ccactcccac cacgcctccc cccggagggg ctgacttcct tggg                     224
```

<210> 48
<211> 22
<212> DNA
<213> Artificial Sequence

<220>
<223> chemically treated genomic DNA (Homo sapiens)

<400> 48
gagatggtgt gtatttttag tt 22

<210> 49
<211> 23
<212> DNA
<213> Artificial Sequence

<220>
<223> chemically treated genomic DNA (Homo sapiens)

<400> 49
tccataaact acataaaaaa ccc 23

<210> 50
<211> 182
<212> DNA
<213> Artificial Sequence

<220>
<223> Example 7 amplificate 5

<400> 50

```
gagatggtgt gcacctccag cctgtgcttg gaggagtcca gcgaccgggg ctgaccggga     60
gccagaaccg aagccatggc taacggctgg ggatggtgac aggaagatga ggagacggcc    120
gacagcttgg tccccgctgc tcggtgctcc aagtgaagcg ggcctttcat gcagttcatg    180
ga                                                                   182
```

<210> 51
<211> 20
<212> DNA
<213> Artificial Sequence

<220>
<223> chemically treated genomic DNA (Homo sapiens)

<400> 51
gatgtgggta gaagggtatt 20

<210> 52
<211> 23
<212> DNA

<213> Artificial Sequence

<220>
<223> chemically treated genomic DNA (Homo sapiens)

<400> 52
ctcttaacac ttctctatca aaa 23

<210> 53
<211> 198
<212> DNA
<213> Artificial Sequence

<220>
<223> Example 7 amplificate 6

<400> 53

```
gatgtgggca gaagggcacc actcagagcg tctttaggga gcaggcttcc aagctccaaa      60
gcgaaacaag agtgggcaaa gacccccttc ttctctccct ccctccccca agaacccctc     120
caataaggaa agctaacgcc gaccgcgctc tgcccgcccc ccccccacgc ggcagccctg     180
acagagaagt gtcaagag                                                   198
```

<210> 54
<211> 24
<212> DNA
<213> Artificial Sequence

<220>
<223> chemically treated genomic DNA (Homo sapiens)

<400> 54
gataggtagg tgatattaga tttt 24

<210> 55
<211> 24
<212> DNA
<213> Artificial Sequence

<220>
<223> chemically treated genomic DNA (Homo sapiens)

<400> 55
cctaaatacc taaaactaaa ctac 24

<210> 56
<211> 155
<212> DNA
<213> Artificial Sequence

<220>
<223> Example 7 amplificate 7

<400> 56

gacaggtagg tgatattaga tcccctgcgg cggcagcagc cgctgcagcc acgacgcggc 60
cctctgagcg caccctccgc aacgcgcaca cgcacacccc tcgggcggtc gaacaggagc 120
cgggccttgc cgcagctcag ctccaggcac ccagg 155

<210> 57
<211> 23
<212> DNA
<213> Artificial Sequence

<220>
<223> chemically treated genomic DNA (Homo sapiens)

<400> 57
ggaagaattt gttgttggtt gtt 23

<210> 58
<211> 22
<212> DNA
<213> Artificial Sequence

<220>
<223> chemically treated genomic DNA (Homo sapiens)

<400> 58
tcaactcttt cctctaacta aa 22

<210> 59
<211> 265
<212> DNA
<213> Artificial Sequence

<220>
<223> Example 7 amplificate 8

<400> 59

ggaagaactt gctgctggct gcctcttttc ggctctcagg agagtccgtg aactcgacct 60
ttttgatttc ggagtctttg gagaagagac attcaacggc cgccggctgc acgcctgggc 120
cacgcgcgcg cccgccccac gtgcggagag aggcgtcccg gaccgcggcc gaaaggagcc 180
ggggacggga ggagggggag gggcgaggca ggccggagga gaaagaggga caaagagcaa 240
agacccagtt agaggaaaga gctga 265

<210> 60
<211> 23
<212> DNA
<213> Artificial Sequence

<220>
<223> chemically treated genomic DNA (Homo sapiens)

<400> 60
agagggataa agagtaaaga ttt 23

<210> 61
<211> 21

<212> DNA
<213> Artificial Sequence

<220>
<223> chemically treated genomic DNA (Homo sapiens)

<400> 61
ccaataacct ctccctataa c 21

<210> 62
<211> 213
<212> DNA
<213> Artificial Sequence

<220>
<223> Example 7 amplificate 9

<400> 62

```
agagggacaa agagcaaaga cccagttaga ggaaagagct gacggcatcc ccgtcccccg      60
gaccctctgg caacccgggg caggatggcg tactctttct gcgcctcccc ggttgccggc     120
ggttccagcc gggaggagta ggctgggggg cttcggtaca cagcgcgccg ctgcttccta     180
gtccaccgcc ccgccacagg gagaggccac tgg                                  213
```

<210> 63
<211> 23
<212> DNA
<213> Artificial Sequence

<220>
<223> chemically treated genomic DNA (Homo sapiens)

<400> 63
agagggataa agagtaaaga ttt 23

<210> 64
<211> 21
<212> DNA
<213> Artificial Sequence

<220>
<223> chemically treated genomic DNA (Homo sapiens)

<400> 64
acaaaaaaac actaacccta c 21

<210> 65
<211> 304
<212> DNA
<213> Artificial Sequence

<220>
<223> Example 7 amplificate 10

<400> 65

```
agagggacaa agagcaaaga cccagttaga ggaaagagct gacggcatcc ccgtccccg      60
gaccctctgg caacccgggg caggatggcg tactctttct gcgcctcccc ggttgccggc    120
ggttccagcc gggaggagta ggctgggggg cttcggtaca cagcgcgccg ctgcttccta    180
gtccaccgcc ccgccacagg gagaggccac tggcgatttg gttctgattt ccttcctgcc    240
caggctggcc cctcggggaa gcgcccctcg ctggggcctc gccgcagggc cagtgccctc    300
ttgc                                                                  304
```

<210> 66
<211> 22
<212> DNA
<213> Artificial Sequence

<220>
<223> chemically treated genomic DNA (Homo sapiens)

<400> 66
gtaggagaag ggggttttta tt 22

<210> 67
<211> 23
<212> DNA
<213> Artificial Sequence

<220>
<223> chemically treated genomic DNA (Homo sapiens)

<400> 67
ttataatcct ctaacctcta aac 23

<210> 68
<211> 329
<212> DNA
<213> Artificial Sequence

<220>
<223> Example 7 amplificate 11

<400> 68

```
gcaggagaag ggggttctta cctaattgca cacacgccga caccagtttg cggcagttgg     60
tctccattcc ccgttatctg caaaacagaa gagaaggaag gctgtaagaa gcggcggccg    120
tcgagtgagc agggctcaga tgagactacg ttacactagc tgttaggcgc ctactgtgtg    180
ctaggcttca ggcgtgtttt tccgacctga cagcctctgg ctgtgcagca gcatctccag    240
cctagcctcg ggcccaggac atttatttat caagagggga tctccctcca gagttgccgc    300
aaaagtgccc agaggttaga ggactataa                                      329
```

<210> 69
<211> 6499
<212> DNA
<213> Homo Sapiens

<400> 69

```
tactgcatat tctcacttat aactggcagc taatcattga atacacatag acataaatgt      60
attttaacac attattattc ccacttagat tctgtgtaat atttaaccat ttaattacat     120
caacaaataa aagcctttag aaattgatgt atctagcaca attaaggggt aatttgttta     180
tgattacagg agtcatttca tttacactaa tttgatgtga ggagacaatc aaattccttt     240
tttcctataa gaaaaactta attccgggag gcttaggcag gagaatctct tgaacccagg     300
aggcagaagt tgcagtgagc cgagattgca ccattgcact ccagcctggg caacaagagt     360
gaaactctgt ctcaaaaaaa taaataaata aaataaaata aaaagtaaaa cttaaccctc     420
acattttttc tttaaaaaca ttacactatt gaaatagaat agaattttgg acctcaatgt     480
ttctatgctt tttgacaatg taaagattat atcaagaata acattgactc cttgtgcaca     540
tgtgtgaaac tatgtcgagc tgatgtatcc agaaaagaaa ttgcagcgat attgggtata     600
gaatttccaa tctcaacctc cttcttgttg tggaatagaa atttagttcc accttctctg     660
ttcataatac ataagagtag ataatcagat atgaggtgat taaatatata aactgatttc     720
acagttacca tggaaagacc ataatatgtt tggacgaggt gggggaaaga gtctccctct     780
gaactgagat ttgaataggc tttagataga ctaaaaaccc atggcctgtc ttcttcaact     840
tcccatcatt gatcatctgg gagaatcaaa atggcctgga ttataaagtg attacaatta     900
gtagggcagg catactaaat aaaaatgggg tgagtgtgct tggatgggaa aaactggggt     960
gcagagaaat atttcctgtt ttggaggctt cgttcatagc gagcctgagg atgcagatga    1020
tatggaacat gagccccacc aggatataca tcctcctgca cagtcttccc tgatcagtgc    1080
tgtctattgc cctaggcacc atcctcagcc actctgtgtt aggcacttag gacagttctg    1140
ttcaaagcat gattgctttc tggtcagtga agaaatagta aagaaatgag agtctgcttt    1200
tgaagctttt gtagcaactt aacatttctg caatgtcgtg gtacatgttt tatatttttt    1260
aaaagtattc atctgcagac tagaaataaa agagtccttc agcacagata gtttgagaga    1320
tgctactgta gagtgccctg ggaggatggg attcttctag acattgacgg ctccctggag    1380
tggtgctgga gcttgctgag atagggacgc ggggattgct gtggaacagc atgttgtgaa    1440
aacaggcaca gatgggctgt tgccacgtac tcagtatttg gcaggcccag gcaccctgcc    1500
ccggggagac agagcaacag ctgtcatttc caaaaatgtg acacaaatat gactttcgtt    1560
ttttattgta tgtgtatgca tgtgtgcatg cgcatgtgtg gttggtgtgt gggtaaataa    1620
tattggtgag ggagttagca gtgcagagac gcagagatga atctaatgaa tttatatttg    1680
gaggcccttg aaaggagccc tgtgcatgca taatcaggaa gcagaagtgc agtcgtaggc    1740
cagtcccatc ctctggtgga aacagcaaat tacagcctta gcacttctca cctgtagcaa    1800
aagaaatgta tgctgcacct gggaggggta ggagaacttc ttagaatttg gtgggtttat    1860
tagtcagggt tgtgtagaga aacagaacca atgagataaa tatgttagat agatgataga    1920
tagatagata gatagataga tagatagata gatagataga gatgatagat gatatagata    1980
gatgatagat gatagataat agatgataca tagatagata tagcaaatag atctatagat    2040
atgacagaga gagaatgaga gagatttaca ttaaagaatt ggctcacatg attgtggggg    2100
ctgcaaatcc tacatctgta gaatttgctg gcaggatgga aattcaggta agagtggata    2160
ttgtagcctt gagtcgaaag tccacagggc agcaagctgg aaactcaggc aaggtttcta    2220
ggttgcagtc ttaagaagaa tttcatattc tttaggaaac ctcagtctgt gttcttaggg    2280
tcttcaactg attgaatgaa gcacaaacat tatggaagga aatccggttt actcaaagtc    2340
tgctggatta agcattaatc ccaactaaaa aacgccttca cagcaacatt tagactactg    2400
ttcagccaag tatctgggca ccatagctca gataagttga cgtataaaat tcaccatcac    2460
aagggaggaa tttatatata cactttattg cagcaaaatc tttacagttt aacgttttgt    2520
acttttccca gaaggaaacg tttcagtgca gagttgaata tcgcatcttt ccgtagctca    2580
tacaaatccc atacaacttt gatttgctga gcctctcctg aaaactggac aacttaagtg    2640
ttcatgaaag gttttaattg cttaagaaaa tagactgttt gtgtgaatta caaagaaaaa    2700
gggattttag aaggaacatt ggtactccgg gaagcaggtt ggggcaaggc ttgcataagt    2760
gaatcagaag ttttaggcac gaagtcagca cctctgtcat ggcctatgtc agttgagtct    2820
ttacctttgc ctcgtgcctg gccttcagac cctgtgagcc cgctccggga cagggttacg    2880
gccaatccag cagagattct ggcaaagcat cccgggaatg agagtgagaa aggcccaaag    2940
cagtcaaggc ccaaagacaa ccctcgaccc catctctcca aatgtcagcc ctcaagatct    3000
caggctctct ggacctcaat ctccatgcca cttcaaaggc cccaaatttc agccccagca    3060
gctccagctc atagccctag gtcttcagag atcactccag taactcccaa atatcacccc    3120
agggatccag ttctgacagc caagaccact cctctcataa aataatcgca gatattaggt    3180
gaagatcccc aaagccccaa ggaaacccta aatctcatcc tgaggacacc gatcccacca    3240
aagccccgag gaaaccccaa atttcatctc gaggacacca gtcccaccgt taggaaccgg    3300
```

gacctcaacc cacagcaccc ggattccgag aacagaggct tcggggccaa atgggctgaa 3360
tccagtacct cactcccacg cccccgggtg gacagcaacc ctcctccacc gcgtcccctc 3420
gtgggtttta ggtcctatat ttgagggatg tggcctcctc cttttatatc atgttggcca 3480
agaatgatct acacagtcat catggaatat tgcatggaga taaggagtgg ctgtgcccct 3540
gcctgaaggg ctcatcccat tcatgaggca ggatgaccct caaggcagtg ctactcacta 3600
gagtcaaagc tcaggtaagg tgtttagggg ccagttgcaa catgaagcac attagccaag 3660
gcagtctcaa agagtattgc cagaggaaga atctacacct ggctgtttaa tgaggttgaa 3720
aggcaaatct aggtcaagca ggccaagtaa agaggtcagc ccagcacttt gggaggcaga 3780
ggcaggtgga tcacctgagg tcaggagttc gagaccagcc tggccaacat ggtgaaacct 3840
tgtctttact aaaaatacaa aaattagctg ggtgtggtgg cacgcgcctg taatcccagc 3900
tattcaggag gctgaggcag gagaattgct tgaaccaggg aggcggaggt tgtggtgagc 3960
caagatcagg ccactgcact ccagcctggg tgacagagtg aaactccgcc tcaaaaaata 4020
aataaataaa taaataaaaa taaaaaagaa gtcagaaagc ccatgaaaaa catttggagg 4080
gaagaagtta gtccaagaat aaaagcatta ggtcagggcg tctaagatcc ccaaggattg 4140
gtcctggctg tgtagacact taaagagatt gtgcaaggct ggacaggagg gtgagctggc 4200
caaaaaggtg atctgctaac tgtggaatca accaccgccc aatgtcctgg ataactgagt 4260
ctgactcgca atggttaggc ttaccattgt aaatcagtct tgaagtataa acaagttcat 4320
taaactaccc ctcctgttta cattttcttt tattccctga ttcatgtaat ctctgtagct 4380
catatctaaa taaaaaataa taacatggac ttgttattat gcatgtcgtg gtttacttct 4440
aactgtaaag tttctgatta ttctcctgtc atgctgatct tcatgtcaga agaaaacgct 4500
ttgctattca ccatgagtgc agaaaactgc ttactagaaa cgattctaaa cagcaaagga 4560
tgtatatttg aaataatagg gttgtaatga aaaagtacat atttctcaat ctcattcaat 4620
acattgtacc cgtagagtaa aagatataga aaagcatctt atgttttaaa aatgtaagtc 4680
tgagagtgaa aaacagagac aataaaaacc tttctctaaa tattattggt ggaaaagtct 4740
caaagcaata gaaagtatat ataaaatgcc tgtgaatttt tgtgcaataa aatcatatat 4800
atatatgtgt gtgtgtatgt atatttgttt tttttgtttt gttttgtttt tttttttttt 4860
tttgagacgg agtctcgctc tgtcgcccag gctggagtgc agtggcggga tctcggctca 4920
ctgcaagctc cgcctcccgg gttcacgcca ttctcctgcc tcagcctccc aagtagctgg 4980
gactacaggc gcccgccact acgcccggct aatttttttgt atttttagta gagacggggt 5040
ttcaccgttt tagcggggtt ggtctcgatc tcctgacctc gtgatctgcc cgcctcggcc 5100
tcccaaagtg ctgggattac aggcgtgagc caccgcgccc ggccaatgta tgtatatttg 5160
taactcagat ttgtaaatgc agccacattc atatccacac atcaaatgca ccaaaaagat 5220
tcactaagtt cctcatagtc agtccataaa ttcccagctt cttcacatat tcgcattttt 5280
tgtcttaaca tgatataata gttaaaaaaa tttaaaaatt aataattaac caagtttttt 5340
tttcttgtag actatatcag ccattattca gaaacattaa ttcattgttt taaaaataat 5400
ttctctagag atgtttggca cttagtatat gaaatcattt ataattgaat aatacaaaca 5460
aaacagaaaa aagaacttac ttttgggaca tctgaagaaa gtgacctaca aactcagttt 5520
atccctgagt ctcattattt ttaggaaaat ggtctctatg tcatctctat gttatgtttg 5580
acttgtcttg cttgagtctg ttttccagaa agattgtgat ccttggcctg ttgaatatga 5640
aatttacatg atcactcttg ggaaaaattc cttttttagt gactgcaagc caggtcctgt 5700
ggccgagaag catcagctgt acttggttga ctgactgtat tactctgttt tcatgctgct 5760
cataaagaca tacctgagac ttggcaattt acaaaagagg tttaacagac tcacagtgtc 5820
aagtgggtgg gggtgcctca caatcatggc ggaaggtgaa aggcatgtct cacatggtgg 5880
cagacaagag aagagagctt gcgcaggaaa atttcctctt ataaaaccat cagacctcat 5940
gagacttact cactatcaca agaatagcac gggaaggacc cacccccatg attcaattac 6000
ctccaaccag gaccctccac aacatgtggg aattatggga actacaattc aagataagat 6060
ttgggtgagg tcacagccaa ccatatcact gactttcatc atattataaa gaactatctt 6120
gcagagagaa tctgctttcc atctctaata atttgtttat tctggatatt tcacataaat 6180
agagttatat aatatttgtt gctggctttt tcatttagtg taatgttttc aaggttcatc 6240
tgtgttagag tatgtacctt tgcttcattc ctttacatca ctaaataata ttcgattgta 6300
tggatgtgcc acatttattt tgatctctta tcagttgaca gactgttggg tttttctact 6360
ttttggctat tacgaatgat ggtactatat gaacattcac acacaagttt tcatgggtat 6420
atgtttttat ctttcttggg tatataccta ggtgggggat tgctgtcata tggtaacttt 6480
atgtttaaca tcctgagga 6499

<210> 70
<211> 6456
<212> DNA
<213> Homo Sapiens

<400> 70

caagaatttc aagtcattat ccctgtgcat gcaggcagta ccagtgccag caataacaag 60

tcagcttttc tggcaaatcc atcaaaatta aatcttaaac ttttgaaaaa actaatatgt 120
ttattatgct cctgttagct tagaggctta actatctgtg ggggagggcc tataattatg 180
atcaattact tatgacttga aggtacttat aaaagcatct gcccttttaa gaaaccatta 240
aagaaccagg cttaagactg taaaactaat cccaggggca atagtaaatc acaggtttgg 300
ggacccaact ttgacaactc tcccttggaa agaaaagaat tgctgtcaac tgtccttatt 360
tcctgaccct gtctgagcaa atattcgcag cctctaaaat ccagaattct tagtctggcc 420
aagcaagtca tggggaccaa tggttgtttc aaggcagagg cagaagagaa gaaacaggaa 480
ggaaacaaaa agggaatgaa gatctgtagc tcatgagggt gacatcatca ataatttcct 540
caagtatcac tgaatttcat ggctcgtgac tgagagtctt ttctgacatc ttacaggcta 600
attatcctca aggatcttct tggttccaga gcctgtaatg tctgggtttg aatatgattt 660
gttctacaaa aaaggtcagg tgaattgacc cctccctgtg aagctgctcc atggcaagag 720
accatcaatc tgctgaaacg caaacaatcc tttagatatc tgaagtactt tcaaatttac 780
aaagtgcttt cacatacgcc atctcatttt attctcacga ctttaacagg taggtgccat 840
catcacctta acacgtaaaa ataattttag aaaacaatgc ctttgtcttc agttcataag 900
aatatcctcc tgaaattcac agtacagaat ccaagtcatg ggtcagttag aaacgtctag 960
aaacacatca atctcaggag gataaatcaa gagaatagga tggatcagac aaaatctaca 1020
agcacaatgg cagaaagtgt gcctctggta taagaatggc gtaagctttg taaacaatag 1080
tgttatacag gggtgcctct gtagtacctc tacgtttgga aggacactat tcattcatat 1140
acatgataag caccccttaac tggatactcc ataggtaaca gacaaaataa aacaagaact 1200
tccgtcttaa aattgtagtg gtggaattca tccttcggga tgtattaggt gattccccta 1260
ggacgacagc tgagttacag gggctaaat ataccctccc cttttccctc caatatcaca 1320
aagagcccca aaaacggatt ttgttgtttt taacaaataa aatacacatg aaaaaaagtc 1380
atctataggt ttttatatat aatctcatct tctaaaacac tcattctcct tcactagcta 1440
ggccgaggta taatgcggtc ttaactttat aaccatttac tgccttattt atatatttat 1500
gattctttga tgccttctag cttgagtcta aactgcgttg ggaacaaagc accgcacaaa 1560
tcaccagcct gggataagag gagacaagtt aaaacctccc aagaagattc tgtagatatt 1620
tggtctctct gagtctgtag agagtttggt atttcaaagg tcagtcttgg gtctctccac 1680
tttgatgact aatgttaaat tgtttgactt tttacaaaaa tatttagctg tgcttattta 1740
catcatttca tcctcagcgc acacagtagg catgtatttg ttgaacagaa gccctaaatg 1800
gatgagaaaa aagatatttc tgctacacat tttctcaaga tcgagaaagg taaaatcaca 1860
acccaatttg cagaagtttc taggcattcc ttttcaatgt aaagctatgc atctctgttt 1920
aaaaaagaaa aggatgacac aaccaaacaa tgtcaaccac acaatgggga aattccagga 1980
tcactaagaa aacaccatta tcctaagtca cacattttca tttaatactg gttaccacac 2040
aggagagagt aagaactgct gtctccccac ccactcgcac aatccatctg ggaaaagcat 2100
gggcagaaca tttatgatct aaccatacca catcttggac tcacatttaa ccctttggag 2160
aattagtcta aagagtatgt ccgcccagag atataaagta aaacagacac agggataaca 2220
gttttggaag aaatttttat gacgctgttt gagattgggc atcagcactc aaactcctta 2280
gaccaccagg agtcattctt actacgcaac tttaataaag agaaactgag agtatgactg 2340
gcaaaaatat tatgatgttt ctgcatcttg ttaacctgat ttacacttgg ctccacccat 2400
aaagttccca ctgattatca cacggcatcc tcattctcca gtctcaccta ctctgtcttt 2460
caaaaattcc ccaacggcat ggccacgaaa aatatgcggg ggctagcggg gcatgtctca 2520
ggttggctta tcctgtccct aaaacgcacc cacttccccc agaccctcct tcagggtggg 2580
cagggagaaa acgcttctaa gttgcacagc tgcagggaag cctgctcagg tcatgttttt 2640
ttgacttgtc tttcggctcg ctgtctggca aggtcaccgg ctctggctcg cggctctgtc 2700
gacaaaactt aactctcact ctgtaagacc aacaggtcgc gcaggacctc cccagtcgcc 2760
ctctgccccg cacctttctg ggcctgagga ttcccggctc ggccctcccc gcgcgcgcga 2820
ggccccagat ggcgagggcg cagctccctg gctcacctga ccacgttggg gtgctcgaag 2880
gtctccaggt gcctcagcac cgccacctcg cggatggtgg agagcggcat gccctcctcg 2940
ccggtctgca cccgcacgcg cttcaacgcc acgaaacggc ctccgttctt caagtcgcgg 3000
gccttgaaca ccttcccata ggcgccctcc ccgatctccg ccacgcattc gtactgctgg 3060
tcagcgcggc acaggccgtc cttctccatg ccgcctggac gccgcccgcc gcggcgccgc 3120
tggggcgggc gggggtgcg ctcaactagc tggcggccgc cgctcgccta ctccggggct 3180
ccccggagat cggtctagct ttacttgctc cccgccggct caggcgctcg ggcgctgggg 3240
ctttcgccgc tgcagaagct ggatggagag acctccccgc ggggctggcg taaccctggt 3300
gccgccgccg cgaaactccg cctgcagagt cgccgccgcc gccgccgccg gaggagcgag 3360
ccgatccctc ctcttccctc ctcgaagcga agtcctcaac acagacacga ttacatagcc 3420
tctgcccaag cgcgtctcag tccagaatca ttgcacctaa aggaggagac gggaggataa 3480
gaagaaagtg caatcagaca gcccagaagc cttcctcggg gttcctccag actcccctcc 3540
tcctccttta cgaagcctcc atcgctaccc tccgcgcgct cctgccctct cccaagccgc 3600
ttaatccttc ctggttcctc cgagaaaagc gaagttactt ttctttccct gcagggctga 3660
agccgtcttc gcgcggagag gttgcagggg cccctcgggg atgagcgagc ggcgcgggac 3720
gcagtggaac gggaggggc gtgccgagca gcccagagtg tgccgggagc gcgggggagg 3780
ggaggcgccg ggcacgtcaa tgtcacggct taatatttat ccctatatca ttgtgttgcg 3840

```
ccgctaccct ccccgcctcc tgaggcccgg acgtgcaggg agacggggtc caggggtgcc     3900
ggagggcgtt caggggtcgc gcacaagctg gagcggaagg actgtgggtc catccgtgtg     3960
gggccgcaga atgtgggtgg gggctcccag gaccctgtaa cccgatgctg ggagtgtgcg     4020
agaaggggtg gtcagacatc tgctcagaaa ttgcttcctt tctttctact ttcagttttt     4080
ctacgaggag acatttaaaa acgactcgca tacacaaatg gtctttttgg ggtaaaggag     4140
tctcggttgg aaacggaatt ctctctccgg ctgggaggac ctccctggtg gaaaccttgg     4200
gaagaccagc catctggtcg gggagggttg gggcttatcg gggtgggcg agcgagcggt     4260
cctgggggag gggagacacc gtccccacg ggatcccaag ggtgggagaa aggggtgttc     4320
gccacaattt cgcttgtcgt ctccttaaag aataacttca ggaaggggat agcataatcg     4380
cgcctcgtcc ttaagtttta aactgatttc gacctgagca aaactcaacc tccctttcaa     4440
agggtggggg gtgggggtt aaatcctgcg cctccagggg tgagagagaa ggtctctgtc     4500
ctcggggccc ggactcgcga acctttccc attcccagga cctccccgct gtaggtagca     4560
gaggtggctg ccccattccc cctccggcta aaggcccggt ccagtctgca gccggacgcc     4620
cccggagccc tctgcacaac aaagcgccag agtagggtac cctaactttc cctccccatt     4680
cagcagctat ctgggaccta gggggcctcc ctcctccagg gccgccgcgg cctcggcagg     4740
actttcacca cgacggccgc atgtccggaa ttcccggggc acctcggaat tacctgcctc     4800
gcaaccgctg gcccgctcgc cttttgccag gaattaaaca aacggcgcga cccccacgat     4860
gagcgggtgg agcggaccgc ggcgagagca gagcttctgg cactcactac attcagaact     4920
ttccttaaaa gccgaggaaa gagcccccag ggactgcccg gggtctgag ccaggtcgag     4980
ggccacacag gtagccgggg agccgccgcc ggccgcccaa cccgcctgtc ctgcatcccc     5040
ccaaccccct cccgctcgag tctagccccg ggctccgcag cgaaggaagc gtcggggacg     5100
tcccacccc gcagggaact gcgcctgctg cccccgccgg gccccgcact gccctggctc     5160
tccccccact cgcccccag acggtcggct tcggcggaga gaaacgggag cagcaatgcc     5220
ttttcccccc ctctcctcca ctttttttg ttgttgttgt tgctttccca cgctggctga     5280
atgtgacttg accccatttc aaaaaaagtt tgacatagtg cttcaacatt tccgcattcc     5340
tccgcgacta caaaggctgc agccgcctcc ttctgctttc tgtctctgct ctctgtcttc     5400
acactcaatg aaatccttca tgcgcctctc ccgaagcctg ccaagcacca caagggtcgc     5460
caccagcaca gcgacaagag gccacaccgg ggaccgcgac tcccgcgtgt gcgcacacgc     5520
agatgcgccc atatgcacac ggacgggcgc gctgcgggga ccagggctgc tcactgcggg     5580
gcgtgtgttt aactccaata ttttaaatta taaaagtaaa cttggaaact acagcaaacg     5640
tgcgtcttcg cggggctcca agcctgggca ctggccggct gcgtgcactt ttctgtgtat     5700
aacacgcccg caggaagcct gcgttaacat ttatctcgtg gaggggaagg tggagcccac     5760
acccacacct cagcgagctg gagagggagt tttcgagggg gaaatgcaag cacattctcc     5820
agcatggagt ccctagaggc agtgcatttt aaaccctaaa tgtgaattat tatgtgagtg     5880
tccgagtaga gttccagttc cgtttggaga aactgtatga ggcgtgttcc gtgtgggtgt     5940
gtgtgcgtac ggatacgtgg aagcaggatc tcggtgtcgc gggtgtccct aggctgggat     6000
ctccccctct catcagataa ggagaatcct ctagctcccc aaaactggcc cccataacca     6060
atcccgtctc agttcgtaag ggttaacca aagccgattc caaggaaaca caggtccccc     6120
ccacccccg ccaccctcca cttaccccac ccacagttct gcctcggacc ccggccaatc     6180
ccccagatct cccccgggac cccccacccc gcagcccacc cacccgagcg cacagctcct     6240
cacctgaggg gcccagtcgc gtcgggcctc ccgagggggc tgcgagtgtc agtcggctct     6300
ccgcacgtgt ccgcggcctc gcggagcagg taatcagact ctggggaagg agttaccagc     6360
actctctccg gcgaggggt gggcacagcg gcggagggcg gagggacggc ggagggcgcc     6420
gcccgcgccg ctcgctgggg gcggacgggg gccgct                               6456
```

<210> 71
<211> 6257
<212> DNA
<213> Homo Sapiens

<400> 71

```
ccacagtagc tttcctgact ggactaacct ttccggacac aacagcaggg caggggtggg      60
gcctggggag aaaggacacc tctaaccctg atcctaacat cccgatggcc tctaaggctg     120
cctgcacact catccaggtg caagccctcc aaggtgtggt gtgatgaacc agtgactcct     180
ggagccaggt cagcgcatcc tcttcccgca gggctgtaag ctgcaggact gagaggcagg     240
ttgaccaggt cctgggctgg atgatggggt gagagtaagg ggtcagtttt gatacatgcc     300
caacttttct ctctagccct aagacatcct gggcaaattg cttacctcag ttcccctgat     360
cctcacccta accctaacac cagctcaaga gaaaataggg atattgatgg ccatccagaa     420
gggctgctgt gttccataca cagcaatatt tctcgaatgt ttgtgacagc ggtccaagga     480
ataagttaat tttacattat cactctggat acctgtacaa aactccacct tatccttact     540
atatgaatgt gctagggttg tttttttgtt ttgttttttt tttttttttt tgagacagag     600
tttcgctctt gttgcccagg ctggagtaca atggcgcgat cttggctcac cgcaacctcc     660
```

```
gcttcccagg ttcaagcgat tcacctgcct cagccttccc gagtagctgg gattacaggc     720
atgcgccacc atgcccggct aattttgtgt ttttagtaga gacagggttt ctccatgttg     780
gtcaggctgg taccaaactc ccgacctcag gtgatccacc tgccttggcc tcccaaagtg     840
ctgcaattac aggcatgagc caccgcaccc agccgtgcta gggtcttttt ctgttcaatt     900
cctttctctc tcttgctctc tttctttctt tcaatggagt cttactctgt cacccaggct     960
ggagtgcagt ggcaagatct cagctcactg caacctctgc cctctgagtt caagcaattc    1020
tcctgcctca gcctcccgag tagctgggat tacaggtgcc tgccaccaca cctagttaat    1080
ttttgtactt ttagtagaga tggggttttg tcatgttggc caggctggtc tcgaactcct    1140
gacctcgtga tctgcctgtc ttggcctccc aaagtgctgg gattacaggc atgagccgcc    1200
atactcggcc aactttgtat tactttctta aagagagttt cccaaattat ataagcttca    1260
ggccccacaa aacctagatc tgccccagta taactaaatc tgggaccatt tattgagcaa    1320
ttattatgtg ccaagtattg cgctgagtgc ttccagagca ttatctcctt taaccccagc    1380
atagtatgtc agatgctgtt ttacagatga gccaactgag accagagatg ctcagtcact    1440
tgcccaaggt gacatgactg atatggaata gagtcaagat tttttttttt tttttgaca    1500
cggagtctca ctctgtctcc caggctggag tgcagaggcg caatctcagc tcactgcaag    1560
ctctgcctcc caggttcacg ccattctcct gcctcagcct cctgagtagc tgggactaca    1620
ggcacccgcc accacacctg gctaattttt tgtattttta gcagagacag ggtttcaccg    1680
tgttagccag gatggtctcg atctcctgac ctcgtgatct gcctgcctcg gcctcccaaa    1740
gtgctggaat tacaggtgtg agccaccgcg actggccaga ttcaagattt gaacccaggt    1800
cctcttggtc ccagaggccc ctgtttctca actccctagg atggcatagc aacctgtccc    1860
acaagaggtg cctgctttaa gtgtgctcag cacatggaag caagtttaga aatgcaagtg    1920
tatacctgta aagaggtgtg ggagatgggg gggagggaag agagaaagag atgctggtgt    1980
ccttcattct ccagtccctg ataggtgcct ttgatccctt cttgaccagt atagctgcat    2040
tcttggctgg ggcattccaa ctagaactgc caaatttagc acataaaaat aaggaggccc    2100
agttaaattt gaatttcaga taaacaatga ataatttgtt agtataaata tgtcccatgc    2160
aatatcttgt tgaattaaa aaaaaaaaaa aaagtcttcc ttccatcccc acccctacca    2220
ctaggcctaa ggaatagggt caggggctcc aaatagaatg tggttgagaa gtggaattaa    2280
gcaggctaat agaaggcaag gggcaaagaa gaaaccttga atgcattggg tgctgggtgc    2340
ctccttaaat aagcaagaag ggtgcatttt gaagaattga gatagaagtc tttttgggct    2400
gggtgcagtt gctcgtggtt gtaattccag cactttggga ggctgaggcg ggaggatcac    2460
ctgaggttgg gagttcaaga ccagcctcac caacgtggag aaaccctgtc tttactaaaa    2520
atacaaaaaa ttagctggtc atggtggcac atgcctgtaa tcccagctgc tcgggaggct    2580
gaggcaggag aatcacttga accagggagg cagaggttgt ggtgagcaga gatcgcgcca    2640
ttgctctcca gcctgggcaa caagagcaaa agttcgttta aaaaaaaaaa aaagtccttt    2700
cgatgtgact gtctcctccc aaatttgtag accctcttaa gatcatgctt ttcagatact    2760
tcaaagattc cagaagatat gccccggggg tcctggaagc cacaaggtaa acacaacaca    2820
tccccctcct tgactatcaa ttttactaga ggatgtggtg ggaaaaccat tatttgatat    2880
taaaacaaat aggcttggga tggagtagga tgcaagctcc ccaggaaagt ttaagataaa    2940
acctgagact taaaagggtg ttaagagtgg cagcctaggg aatttatccc ggactccggg    3000
ggagggggca gagtcaccag cctctgcatt tagggattct ccgaggaaaa gtgtgagaac    3060
ggctgcaggc aacccaggcg tcccggcgct aggagggacg cacccaggcc tgcgcgaaga    3120
gagggagaaa gtgaagctgg gagttgccac tcccagactt gttggaatgc agttggaggg    3180
ggcgagctgg gagcgcgctt gctcccaatc acaggagaag gaggaggtgg aggaggaggg    3240
ctgcttgagg aagtataaga atgaagttgt gaagctgaga ttcccctcca ttgggaccgg    3300
agaaaccagg ggagcccccc gggcagccgc gcgccccttc ccacggggcc ctttactgcg    3360
ccgcgcgccc ggcccccacc cctcgcagca ccccgcgccc cgcgccctcc cagccgggtc    3420
cagccggagc catggggccg gagccgcagt gagcaccatg gagctggcgg ccttgtgccg    3480
ctgggggctc ctcctcgccc tcttgccccc cggagccgcg agcacccaag gtgggtctgg    3540
tgtggggagg ggacggagca gcggcgggac cctgccctgt ggatgccccg ccgaggtccc    3600
gcggccggcg gggccagagg ggcccggacg agctctccta tcccgaagtt gtggacagtc    3660
gagacgctca gggcagccgg gccctggggc cctcgggcgg gagggggcag ttacacggca    3720
gcggctcgag atggcccatc caagagactg gcgctttcca ggctccgagg ggctccggga    3780
acttgtcaaa gaagttctct gaaattgttc agaaagtttt cccgcaaagg gtgtattgcg    3840
tagagcgcgc gcgcgcgttt ccccccttct tgagccccct caagctttct caaagccttt    3900
ccagttggca gcctccgcct ccggactggc ctgggctgga ttccttgggg gggtcctctg    3960
ccctgcccct cctccagccc ctccccgctc cccttcagac gattttggtt tggttgctcc    4020
tgcttctggc ggggtcgggt gtgtgtgtgt gtggtggagt ggagggtggc atagcaacct    4080
gtcccaacca gagccgggga ggaaagggtg gcccggaggg tggcctcttg ctggggtctg    4140
ggttgggggc gggggagacg tttgctttga acagattctt ggggccagct tagggactgt    4200
gctctgtgac ttttggagcg cgtggaccat ggaggggtgg gggtgggttt cttggggtgt    4260
aaagtgggag agttcccaga gaaggaagct aagaaataag gccagatggg agcctaggga    4320
gggctgcgtt gttctgctgc cttttccttg gtgctgtgcg tggggaaggg tgagtggggg    4380
cagtgtgtat cctgacccat ctgtccacct gtgtgcatta atcataaaag ctaacatata    4440
```

```
gcctgggcca ggtatactct gccaggaact gtttgtggtg ttttgcatgc attctccttt    4500
aatcctagaa cacccctata gtggaagttc tgccagcatt ctggactgag tagcagtcca    4560
gaggttgagt agcagctagt aagtggtggg gtcaagatgg gaccccaggc agtgcgaccc    4620
ccaaccatgc attcgaaatc gctatatgga tgagtgcacc tggagcaatg agggacactg    4680
ctccctgagt cactgggctg caggggagac aaaatgaaag tgttctggga gtcgtgggtg    4740
gtctccatag gtcagagggt ctggggaggg agtgggtgtc atcgtggctg tgtgttgccc    4800
gaggggccct ctgtgagtga gtgcatggcc gtgttatctc tgcaggtcta cgccagggtg    4860
ttcctcagtt gtgtggtctt tgtatttgtg tgtctgggct ttgtgttgcc aaacagcagt    4920
ctctctgctg acttggggac acaggctgaa ctctgtcctc tgcaggaact cccttaaggt    4980
gctgggccag atctgccata aacagaggga ggtagccttc tatggccacg ccttcttgct    5040
gaggaagaag gttcctctct tccagggagt acatccttgc cctccctgtt tcccagacaa    5100
gcatcttcac ctctcatctt ctgatgagaa gggtgaggcc atactgagct gtcaggctga    5160
gctgctgccc ttcctcacct tgggctggga gttgatcagg gaatggcagt tgctgcagag    5220
ctggatttga gggctgggtt ctctggatgg ggcctcctca tgtcctcacc cctcaacctg    5280
cactattgat tgtgttgtgc aggagttagt taaaaagtca ttgcacagcc tgggcaacaa    5340
ggcaaaactc tgtacaaaaa atacaaaaat tagttggatg tgattacacg tgcctgtagt    5400
cccagctact ccggaggctg aggcaggagg atcacctgag cccaggaagt tgaggcttgc    5460
agtgagctgt gattgcaaat gctctccagc ctgggtgaca gtgtgagact ccgtttcaga    5520
aaaaaagtat accacccagc tgcctccagc acccagattt tacccaaggg gtgaggtctg    5580
gggcaggaat gtgggggaag gggaggccta gggggagccc cagaggggtc aggatttttc    5640
tgaaatcctt tcttagaggt atgggtttta caaattgcag caaatacatc cttttaatct    5700
tgcagaactc cttcatattt taattccagt atgattcttc caacagcctc ctctctttac    5760
tatacttggg gaaagtactc attttatttg tcaagaaaaa aacaattgaa aagataggga    5820
tcaaatgtaa aaagaaaaaa tacgtggcat tccaaagtca aacacaaagc atgtttaatt    5880
ttctcgtggt ttgggattac ccatattcct gctgtatgaa cctgtcttgt cttaactttt    5940
aagaaatgta cggtgtactt cctatatgct aggtttttat ccatgctttc atttaatctc    6000
tgtgacagtc ctgtgaagta ggtgcacaga tgagaaaatg gaagttcaga gaaatgaagc    6060
aacttatcca aggctcccag ctacccagta atgtccaggg aatttttgga ctctgaagag    6120
gaggcattaa gaggtggtta gagtcttatt ccagccaaca ataatgggtt gaacaaagcc    6180
ttaggggcag gcaggtggcc agatgggagg agaagcgctc ctcttgttca ggcgaatgac    6240
ctttccatcc acttctc                                                   6257
```

<210> 72
<211> 8467
<212> DNA
<213> Homo Sapiens

<400> 72

```
atgaatcaaa cctttctctg gcaccaagaa gggacataca gtattccgag ctctgccctt 60
gttttcttat caagttttct ctaacagctg gaagtggttg aagggggctg tcttaaggag 120
accccctgct cttcttttga cagctgatca aaagaaaata ggtcacgctt ctcaaactta 180
tgtccaccct gtccttaatt actgtctctt taaacaaagg caacatctgc gcaacctcag 240
ctagcttgaa tcttcggagg caaacagagc gcaacctgct ttggaagaat cttgttacgt 300
ttaaggcttt atgccgggtg ttggcctctc tgtcttcaac tacccctcca ccccgactcg 360
gactgcagaa atctccaact ctctgtcccc cagttccact cccacctcca gacctggggt 420
tgccaaacaa cggaaattct aggaagacgt aggtgcggtt tttaaagcct tggcttgtga 480
gcgttctcag gctggccggg gcgctggtct tttcagaaac aaggtttgaa tatgcaggtg 540
tcagccagga ttccttgtcg tctgcccacc gccgtttctc tcccgggatt tgagagaagc 600
gggagtgtgt gtgtgtgtgt gtgtgtgtgt gtgtgcgcgt gtgtgtgtct cgtgttagga 660
gaaaggtccg tggttgccgt ctcagctggt ttgcttactc tctgccccca ggagaagttt 720
gctcacttgg gacaccagcg accctcctca cctccacctc accggttcta cactctcccc 780
acttctttcc aagtcgctga tgcagaaagc acttggtcac cttgaaacgg cagctcccgg 840
aattctagtt ttgttttgca atctagcagg gctcttcgga agcctcataa ctgagattta 900
tgggctcacc gggtaattaa atgatgttat tgtgttaact ttgcatgcat tactgctgcc 960
cgcgccgccc gtgggccgcc ggccggccgc agctcgctgg cgacgagggc actacagttg 1020
ctctgaccgc gtagattatg catgtcccgg cctcgggaat ttaccatgca ttagaataca 1080
ttagcgcctg cattttaaaa ggctaaacta ttggctccca gctagggact ctcggtaagt 1140
ggcttgttag tgacgagtgt ttgtctatac tggcacatag cggagtcttt tgctcccggc 1200
ttactcgcct ccaggaaagc tttggggtga ggcgaaggcg attgaagcaa tgccccttcc 1260
cccagatcgc agctgctcag gggggacaca gcacggcatc tttcaccgaa tctctctcgc 1320
tcgctcgcac tccagcctcc ctctccccag cgaccccccc accttctcct ccctccctct 1380
ccttgacgtt tgattccagt agcaaaggag gtaaaaaagg caccgagccg tcagccaaac 1440
```

ctgaaaagtg cggccccgcc ccctccacag ccactggtag cttcccgtgg aaggcccgcc 1500
tcccggggca gctgcggcct cggagtggtt gcgcttggcg cccgtcgggc gtggccccgc 1560
cccaggtccg ggagggtagg ttggctgccc cggcgagcgg cagagccctt ctggacagct 1620
cccgctcacc caaacagaag acgtcggcgc cggagcgggc tcggacatgg cgaggctgcg 1680
agccggcccg agcggcgggg cccggtgatc cctccctccc tccccgtccc ctcccctctc 1740
ccgcacgcac gccccgtccg cccccacccc gcccccaccc cgggcgagcc cgcccgcagc 1800
ccggggcgca cacccgcacg cgcactcctc tccactcact cccgcgcccg cccccactcc 1860
cgcagccgag ccccgccacg cgcgccttgc ccgcccgccg gccgcccccg ccgcccccgc 1920
cgcccccggg ccctgatgga ctgaatgaag gctgcctaca ccgcctatcg atgcctcacc 1980
aaagacctag aaggctgcgc catgaacccg gagctgacaa tggaaagtct gggcactttg 2040
cacgggccgg ccggcggcgg cagtggcggg ggcggcggcg ggggcggcgg gggcggcggc 2100
gggggcccgg gccatgagca ggagctgctg gccagcccca gcccccacca cgcgggccgc 2160
ggcgccgctg gctcgctgcg gggccctccg ccgcctccaa ccgcgcacca ggagctgggc 2220
acggcggcag cggcggcagc ggcggcgtcg cgctcggcca tggtcaccag catggcctcg 2280
atcctggacg gcggcgacta ccggcccgag ctctccatcc cgctgcacca cgccatgagc 2340
atgtcctgcg actcgtctcc gcctggcatg ggcatgagca acacctacac cacgctgaca 2400
ccgctccagc cgctgccacc catctccacc gtgtctgaca agttccacca ccctcacccg 2460
caccaccatc cgcaccacca ccaccaccac caccaccagc gcctgtccgg caacgtcagc 2520
ggcagcttca ccctcatgcg cgacgagcgc gggctcccgg ccatgaacaa cctctacagt 2580
ccctacaagg agatgcccgg catgagccag agcctgtccc cgctggccgc cacgccgctg 2640
ggcaacgggc taggcggcct ccacaacgcg cagcagagtc tgcccaacta cggtccgccg 2700
ggccacgaca aaatgctcag ccccaacttc gacgcgcacc acactgccat gctgacccgc 2760
ggtgagcaac acctgtcccg cggcctgggc accccacctg cggccatgat gtcgcacctg 2820
aacggcctgc accacccggg ccacactcag tctcacgggc cggtgctggc acccagtcgc 2880
gagcggccac cctcgtcctc atcgggctcg caggtggcca cgtcgggcca gctggaagaa 2940
atcaacacca aagaggtggc ccagcgcatc acagcggagc tgaagcgcta cagtatcccc 3000
caggcgatct ttgcgcagag ggtgctgtgc cggtctcagg ggactctctc cgacctgctc 3060
cggaatccaa aaccgtggag taaactcaaa tctggcaggg agaccttccg caggatgtgg 3120
aagtggcttc aggagcccga gttccagcgc atgtccgcct tacgcctggc aggtaaggcc 3180
ggggctagcc aggggccagg ctgctgggaa gagggctccg ggtccggtgc ttgtggccca 3240
agtctgcgcg ccgagtcact tctcttgatt ctttccttct ctttcctata cacgtcctct 3300
ttcttctcgt ttttatttct tcttccattt tctctttctc ttccgctctt cccctacttt 3360
cccttctccc ttttcttttt ctttcttact ctctccttgt ccctgagctt tcattgaccg 3420
accccccccc atttcattcg ccctcccctc aatgtgccaa cctttgccct atttccgatc 3480
ttcccaggta ctgggaggcg ggatgggggt gtgcgttttc ctctaggagc cctgtctttc 3540
caagacccac agaaaccagg acctgccctt attcaaaacc ccatgcactt caagtctctt 3600
ttagacaaca catttcaatt ttccgggctg actagtctcc ctgtgcagag gcagttgaga 3660
ggctttgctc tgcagaggga aaagagctct ctactctccc acccaccata taggcaaact 3720
tatttggtca ttggctgaag gcacagcctt gcccccgcgg ggaaccggcg gccaggatac 3780
aacagcgctc ctggagccca tctctggcct tggcgttggc gcagggactt tctgaccggg 3840
cttgaggggc tcgggccagc tccaatgtca ctacctacag cgagggcagg gtgtaaggtt 3900
gagaaggtca cattcaccgc tttgggagga cgtgggagaa gagactgagg tggaaagcgc 3960
tttgccttgc tcaccggccg tccttgcccc ggtcccagcg tttgctggga tttgccagga 4020
tttgccgggg ctccgggaga ccctgagcac tcgcaggaag aggtgctgag aaattaaaaa 4080
ttcaggttag ttaatgcatc cctgccgccg gctgcaggct ccgcctttgc attaagcggg 4140
cgctgattgt gcgcgcctgg cgaccgcggg gaggactggc ggcccgcggg aggggacggg 4200
tagaggcgcg ggttacattg ttctggagcc ggctcggctc tttgtgcctc ctctagcggc 4260
caagctgcga ggtacagccc tctattgttc taggagcaca gaaacctcct gtgtgggcgg 4320
cgggtgcgcg agctagaggg aaagatgcag tagttactgc gactggcacg cagttgcgcg 4380
cttttgtgcg cacggacccc gcgcggtgtg cgtggcgact gcgctgcccc taggagcaag 4440
ccacgggccc agagggggcaa aatgtccagg tcccccgctg ggaaggacac actataccct 4500
atggcaagcc agggtgggcg acttcccatg gatcgggtgg agggggtat ctttcaggat 4560
cggcgggcgg tctaggggaa caattcgtgg tggcgatgat ttgcatagcg cgggtcttgg 4620
gatgcgcgcg gttccgagcc agcctcgcac agctcgcttc cggagctgcg agctcaggtt 4680
tccacccccg atcccccggg ctttcctcgc accgctgagc ccagcttgtg gggtgcactc 4740
gaccaacgcc cgacagggct ggggaatgtg acaggcagca ggttcacccg ggcttgggga 4800
gggggagttt ccgctttgac agcattttcc tttgccgtct gctggtggat tcctattccc 4860
agtcggtaat cgccccgcag tgttgatcta agaaggtaaa gaaaactagg tttccctgca 4920
aagagcctcc cccaaatcgg cggactccgg atactttgag tggatttaga aatttatgta 4980
atctttctcc tttagtttat ttttcatcct ctcctacagt tttctctgat ttgctgttgg 5040
ttcggggcaa gataaagcag ccagtagaga gcgataataa tagcggcggg aaatgaactg 5100
gagactggct gacagttctt aacattttgt catagatccc cccgaatgtc ccaggctgtc 5160
tctggtgggt tttagtaccc gccggcttct tgggcaccgg ggaccagaag gaacttggca 5220

```
gctggtctta ggggtacagt taaaggcagg atgacagcta ttctcctgct catctcagag     5280
cgctgccgcc ccctcatgcc ggtcgcgcaa agaacacagc ttttaaaaaa cacgtgcctt     5340
ctgcccatat aggtctgaaa gtgatgagga aagtaatgct tcgcctatta gcgagtttca     5400
gcttttaaaa tgatcccaag cgttgctgag atgagaaagc gtggcatccc gggggtcctc     5460
agccccaccc gcgcccatgg tgcaagtctg cagggacagg cccgggacag cactgcccac     5520
gctgctagat tttccgcaga ggatcgctga agctgccttc gtgggagaca gaatgcctcc     5580
tccagcgagt ggaaaaggcc tgctgaggac cccgctttgc tcgagcattc aaatgtgtgt     5640
ctgttttatt accctgggtt gaaaagggac aagagcttta gcctttttat ctggccattt     5700
tatcagcaac tacaagtgtg ttgagtggtt attattacat aggaggcttt tcagtttggg     5760
gtcagtagat cagtctcttc agacactgat gcagaagctg ggactggtaa gtaggtatta     5820
tgtgctcgga gcgctagggg acaggagcaa atggagaaga aaagcggagg ctttctccgc     5880
ccggagtatc gatcggaatc cccgccggta cgccgcagag ggccctcgcc gttgggcccc     5940
gggggtttaa caagcccagc cgctccgcag gcggctcggc cggactctca gaccggtgcc     6000
tggaagacac cgtccctgcc cccctcccgc caaacctgcc tcttctcttt ctctcatagg     6060
ttataggttc cctttctctc tcattttggc cccgcccccg ggtcctgcca aacagccaag     6120
caggccgggg tttagggggc tcagaatgaa gaggtctgat ttggccagcg ccggcaaagc     6180
tcacccttag gcgaggtcac aacagaggca ggtccttcct gcccagcctg ccggtgtagt     6240
cacagccaag ggtggcactt gaaaggaaaa gggagaaaac ttcggagaaa tttagattgc     6300
cccaacgtta gatttcagag aaattgactc caaatgcacg gattcgttcg gaaagggcgg     6360
ctaagtggca ggtggttgca accccgcccg gtcgggcctt cgcagaggtt ccccaagacc     6420
agcccttgca gggcggtttt cagcaacctg acaagaggcg gccaagacaa atttctgcgg     6480
gttcgagcac acactctcgg gcgttgggcc ccagagacct ctaaaccaag cacaaacaag     6540
aagggagtga gagaacccag gctagaactt gcacgggcat cccactgagg aaaagcgagg     6600
cctcggtggc aggcatgttt tcttccgacg cccgaaaatc gagccgagcg cccgactaca     6660
tttactgcag aggtttccgc ctccagtgag cccggatccc ccagcggcct gcccggagct     6720
ggtctccagt ccccgccgta gtccgacgca cggccctctc ctggcagcaa gctcccagcg     6780
gccagtctga agccaattct gttcaggcgg ccgagggccc ttagccaacc caccatgatg     6840
tcgcctgggc cacctgatgc ccgcagcggc gggacacggc ccgggcagtg cgcagtggct     6900
cctgctaggg gcaccgcgtg cgtgcttgtc tcccgctgcg ccggggacgt ccttgggtga     6960
cacgggccgc tgggcacctc ccaagccgag gaaacggacc cccttcgcag agtctcgcgc     7020
ccacccccca acctcccacc tcgtttctcg ctgctagggc tcccgactca gcccacctct     7080
cctggcggtt tagttaggga tcagagctgg agaggctgaa cgcaacccgt gccagtacgg     7140
aacagacgat atgtttgcct gctagctgct tggatgaata attgaaaagt tcgctgcagt     7200
ctgtgcttcg tcaagtcccg ggtgccggga gaacaccttc ccaacacgca tcagggtggg     7260
cgggagcggg cagaggaggc gggacccgag ggaggagagt gaacccgagc aggagaagca     7320
gcccaggcag ccaggcgccc tcgatgcgag aggctgggca tttattttta ttccaggctt     7380
tccactgtgt ggttatgtca ctttctcaaa caaatgtgta tatggaggga gatcgatgct     7440
gataatgttt agaagattaa aagagcatta atgctggcaa caataacgta aacgtgtgga     7500
cccagatttc attgatctgg aacttgatcc ggcgcgtttc cagtaagccc gacggcgcgc     7560
tcttcccagc agagcgctca ccagcgccac ggccccgcgg ttttccagcg gtgccgcttc     7620
gccagctctg cgcgggttct cccgtctgac cgcagctcct cccccgcgag gccccagccc     7680
gccttacttc cccgaggttt tctcctcctc tcgcggggct ctctgccctc tgcacccccct     7740
cccccgacct ctgcaccacc cgcccctgtg cgcacacacc gctacttgcg cttccggcga     7800
tccgcctggg cggctgggtc cgcgaagcca atgcgctgaa cggtgcccga gtcttcctaa     7860
ctatcctgtg cttggccgtt gccactgggc cctggtgact aagcccaagt ttgaaaatga     7920
cgtggctaaa gctgcctgct aacggcagag cttaatcagc cgcagatccc ctcctatcct     7980
catcggttct cgtactaaaa aggctcacgc gcagactttt tacgcaggtg cattcgttgg     8040
acaattaaac gtggccctag taacaaaagc ctgagcttca tccctccgag tagcaggctc     8100
tgcgctggac tggtcgggtc taacagggag aatcttgtgt cctaccttgg ctgggacagg     8160
aagtaagaga agcaaactgg ggaacccctg ccccacccct tcccaccccc tggacgtcct     8220
gggccgaggc ccgggtcact ggcccatcgc gggtaaggag gtgtcctgtg gaacacctgc     8280
attgactgga aaagaaagaa ctttaaagct cttccttccc gcttgtgggg gacaccacca     8340
caccctgcca accactcccc tggccaaatg gtggcttgtt tttccagaag gagcactaag     8400
gtgaatttta tggaaaaaaa ataaaagatc agggacctaa gtgggagtag gatgagaact     8460
aattctt                                                               8467
```

<210> 73
<211> 4453
<212> DNA
<213> Homo Sapiens

<400> 73

```
ttggctctga agcctatagc atcgctgact cagtctgtcc cctggaaggc tggcagctca     60
gcaagcacag aagtctctcc agaagacagt gggtcacctg cctcccaaaa gctgaaaggc    120
taacttgtac tttccccagc aggcagctgg caccctgagc cctcggctgg ggcagagcaa    180
aggagccttc ctccttccta ccttcctggc actctccctg ccttccttct gtcactctca    240
ggtggaccca gacccaaggt ccagatttgc aaggcaggaa aatgctgcag gcctaggctg    300
ggaaagggcc caaagccgct agtggattgc tgggactcag cctcctcctt cccactaaga    360
gagcgagtcc tactgggttc aaaatgaccc caagccctgg ttcctgacac taggggaaag    420
agatgggggt gacagaatca cagaatccct gctatgttcc tccaagtgtg cccagagatg    480
cgtgtgtgtg tgtgtgtgta tacacaaatg tctgcttatc ctcaggcagg aagggtggat    540
gcagtcattt acacatggtc tgtttttctg gaggacaatt ttatttgata aacaattgtt    600
tctatctgaa tagaataaac aaggctctat gatgaagtaa aacactaaat acacatgcat    660
taaaaaatgc ataattatct ttttggaatg ggctatacag agatgtgctt tttaaaatgt    720
taagagtgta aaaggacaaa cagtgaaaaa taaatcttcc tcttattttg tcctccagtc    780
tcccaattcc tctactcaga ggtgagaaca gaacttccac accctccaga acctccacag    840
ttagaactgt ctacatgttt ccattgtctt tacttttatt cttgcctgca caaataaatg    900
aattgctcat tatggaaact tcccaaaaga cccgttaaca cttcaatagg aagcaccaac    960
agtttatgcc ctaggacttt gttcccacaa tcctgtaaca tcatatcacg acacctaacc   1020
caatccttat caagccctgt caaaaacgga ctttaaacca agctgcaaat tttcagtaat   1080
ctggccttgc ctttccccct ctgatagcac catcaaacaa accccttac tgccgaaagc   1140
aataagcccg gctttgttcc atccactggt tgtgttggtg atatctgggg actgccactg   1200
aacagacgca cagagggagc cctacaggc aggggttttt ctgtctgtgc ttctgggaga   1260
gtatgtctcg tacatttgtc gcgttgatga agacttcaca gctccatcag ctgcgggcaa   1320
gggggtctga ggcagtctta ggcaagttgg ggcccagcgg ggagaagttg cagaagaact   1380
gattagagga ccccaggagg cttcagagct gggcgaggta gagagtctcc tgtgcgcctt   1440
ctctcctctc tgcaattcgg ggactccttg cactggggca ggcccccggc caggtgcatg   1500
ggaggaagca cggagaattt acaagcctct cgattcctca gtccagacgc tgttgggtcc   1560
cctccgctgg agatcgcgct tcccccaaat ctttgtgagc gttgcggaag cacgcggggt   1620
ccgggtcgct gagcgctgca agacagggga gggagccggg cgggagaggg aggggcggcg   1680
ccggggcggg ccctgatata gagcaggcgc cgcgggtcgc agcacagtgc ggagaccgca   1740
gccccggagc ccgggccagg gtccacctgt ccccgcagcg ccggctcgcg ccctcctgcc   1800
gcagccaccg gtgagtgccg cggtcctgag atccccgggc cggatgcgcg gcggccccag   1860
ctcccgagcg tctgcctccc ccgccctggg ctgcccgggc tccctgggct ccccggcggc   1920
tgcacggagt caaggcgccc cgtcccgggc gtcccccgcg ggtgccgatc caggctgccc   1980
ggagtccgga gcccagagag gagagagaca gctggggagc ctggtcaccg cgggcatctc   2040
ccctgcgctg cagtcgcccg cctggcctgc cttcccgttc ctccgcctct tgccctgact   2100
tctccttcct ttgcagagcc gccgtctagc gccccgacct cgccaccatg agagccctgc   2160
tggcgcgcct gcttctctgc gtcctggtcg tgagcgactc caaagtgagt gcgctcttgc   2220
tttgactgat gctgcccaag gacctctgat cagcaccagg ggagaggagg ggctgctcag   2280
ggagctgggg tcctccggat tccatccaca gcagggccag actctcccca ggaaatggga   2340
cagggtggca gcggaggctt gagaaccacg gggttggca ctggctggca agggaggaag   2400
aggccgccgg gactgcccca gcctgcgggc atctggtaga tgaagcttgc ttgggtcaat   2460
ccatttctcc tggctggaaa cccatggtct tccatttgag aactagatac gaacagggtg   2520
aggcgagagg gagagggaag agtgggtttt gggattgggg ccagtttacc ctcaccctgg   2580
agtccctgga gcatgggacc tttgatgaag cctcctcccg aatctcttcc agggcagcaa   2640
tgaacttcat caagttccat gtgagtatcc acccctacaa cagttggctg cacagacaag   2700
ttgggaaggc ttcaggggac atcccctccc tgccctctgc tgcagggctg cgccacccct   2760
taccacttcc actccccctc gcttacccca cctttgttct ctccagcgaa ctgtgactgt   2820
ctaaatggag gaacatgtgt gtccaacaag tacttctcca acattcactg gtgcaactgc   2880
ccaaagaaat tcggagggca gcactgtgaa ataggtatgg ggatctccac tgcaactggg   2940
agagaaattt ggggacaggg agggatgggt gggaggcaag agcaggcagg agttaggagc   3000
tggaggtagg gtgggtgaca tcttcatccc tatgtgacaa gcataaacac acacacacgc   3060
tcacgaaaca gtggccacac aaatgtgagg tggggttgga aggagaccct gtccagtctt   3120
ctggcaggtc tgaaacgaca tctttaaaat gtccgttggc agccgggcat ggtggctcac   3180
gcttgtaatc ccagcatttt gagaggtcaa ggtgagtgga tcatttgagg tcaggagttc   3240
aagaccagcc tggacaacat ggtgtaaccc tgcctctact aaaaatgcaa aaatcagcct   3300
ggcatggtag tggatgcctg tagtcccagc tacttgggag gctgaggcag gagaattgct   3360
tgaacctggg aggcagagat ctcagtgagc tgagatcaca ccactgcact ccaactgggc   3420
gacagagcaa gactccatct caaaaaaaaa aaataaaagt tagttggaat gttcttctct   3480
ttctcatatt ctctcatcct cctgtcccct tgtagataag tcaaaaacct gctatgaggg   3540
gaatggtcac ttttaccgag gaaaggccag cactgacacc atgggccggc cctgcctgcc   3600
ctggaactct gccactgtcc ttcagcaaac gtaccatgcc cacagatctg atgctcttca   3660
gctgggcctg gggaaacata attactgcag gtgaggtggg ggcaacaagg accaaaagcc   3720
ctccctacag cttcccagaa accttgttac catccccttc tcccagaggg ctggccatag   3780
```

```
cacaagagaa gtgcggcctc tggttgagtc ttccctgagg ggaggaggca gggaaggccc    3840
tctgggttgg aatgacatcc cctatctttc tgtgttgcca ggaacccaga caaccggagg    3900
cgaccctggt gctatgtgca ggtgggccta aagctgcttg tccaagagtg catggtgcat    3960
gactgcgcag atggtgagca tcactgacct gctgatgaca gtggggtgga aggggacaaa    4020
cttacatgtc cccttattcc atcacaggag gactgaggag gtggggggtg cccgagaggg    4080
atgctttctc ctacctgcct ccctaagaca tccctctgtt tgtcctccag gaaaaaagcc    4140
ctcctctcct ccagaagaat taaaatttca gtgtggccaa aagactctga ggccccgctt    4200
taagattatt gggggagaat tcaccaccat cgagaaccag ccctggtttg cggccatcta    4260
caggaggcac cggggggggct ctgtcaccta cgtgtgtgga ggcagcctca tcagcccttg    4320
ctgggtgatc agcgccacac actgcttcat gtacggccct gggtttctcc tcttcgactc    4380
ttctgcccca ccccaagcac atccctttct ccttcccagc aaagtgttcc gcctcatttc    4440
tccctcatct gcc                                                       4453
```

<210> 74
<211> 10490
<212> DNA
<213> Homo Sapiens

<400> 74

```
agaaggtgcc tgcttcccct tcgccttctg ccatgactgt aagttccctg aactgggagt      60
cgattaaacc tctttccttt ataaattacc taggctcaag tatttcttta tagcagtgtg     120
aaaacaaact aatacccctt ccctgaggcg ccttctcctt aggcaacccg ctgcccccat     180
gctcctcctc tgccccctgt tctttctttt cccctcatga ggcccaagtg ataaacgggg     240
ccagccccag tcccagcccc agccccagcc ccatcctact gcaggcctgt gtggctgctg     300
gagaggccgt gttcctttcc tctccccgag cctgcctgat atgctttctg gatcctggag     360
gaaactgacc ccctattctc atactggtgc aacatcttcc aagacctcaa agctgtacca     420
tttgagccag tcttttttct tatctccact tgctagggct gtcattggga cagtcctaga     480
gggtggtgcc aatggatgaa tggatggatg gacagtagtc cagggatgat gtccctgtct     540
gtcctgaacc gggcctttcc tccaatgaga agccttcctg agtgagtata tacagtcatc     600
ccttggtatc catggaggat tagttctagg gtccccggga atgccaaaat ccatggatgc     660
tcaagtctct gatataacgt ggcctagtat ttacgtataa gctatgcaca tcctcccgta     720
tacgttagac cgttactaga ttatttatga tgtgtaatac aatgcagatg ctacataaat     780
ggtcgtgata ctgtattctt tagggaatga tgacaagaac aaagtctgca catgttcaat     840
agaaacataa ccgtccaatt tattttctga atattttcca tctgctgttg ctgaatctac     900
agatgcagag ctcctggata cgagagccaa gtgtgctttg agagtagggt gggtgaggtt     960
gctaatgagt acaggggagc aggtgttgat caggaggacc ctgcactggg gcatctggac    1020
gtcctgcctc aggacttgag actccagttg gatggcacag gtagactcag cccaggtcaa    1080
agccgtcccc ttgaagtttc tttttatccc aagctctttc tggaccctgg aattcggcat    1140
cccctaggcc ctgcgtggaa ggacagatga accaggtttt agataacatg tctagaagag    1200
tgagccccta ctgtgtgccc ggcactttcc ccacaggatc ctctagctag aatatccaag    1260
ggtcatggag agaaataccc agttaaaata tcagaaaaga aaaagtgata ccattagaga    1320
cactaaaaag accattaggt aatagtatta gcttttgtat tctgagatcc aatagcagca    1380
gtcacttccc tccaccgcta tgtgtatccc aggaccaccc tgggcgggga gggctgcggt    1440
tagggagcag ccatggatgc tctgatgctg gccctgggcc tcgggggtga cagtgatgag    1500
gaactgggtg cacacatgag tggggcagcc gggcctggcc agagaagcag cacacacgtg    1560
cacagacgtg tttacccaca tacacatgtg cacgcacgtg cacaaacaca ttgcaggcag    1620
gcatgttgac gcctcaggca gcggaggacc ctgactctgg gcgctgctga cccgggcaag    1680
gccccactgt gattcgtgcc atgacctcag aatgtcactg gtgcttagca cctgtctgct    1740
ctctggcctg cctcagtggt ctacagcagt tacacacagg cagtggtatc tgtgagcagc    1800
tctgtggact caaaggtttt ctccctgaga ggcatgaccc aggccagctg attcatcaga    1860
atcaggtgag cgtgacctgc tctcttccct ccaggcggac ttggggacag tggctacggt    1920
gcgggcggtg ttggcctctg tggggcagct accgaggagg gtcatccctg agcactcacc    1980
aggcgcccgt tctacactgc ccgtgtagac gattggctct ttcgtctcca tggtggcttc    2040
gtagagtggg tgctgttccc aaatgtcccc attcgacaga tgagacgtct ggggtcagag    2100
aggcagtaac cggcctggga atccggacat gaccctgagt tttgctctca gccctgccgt    2160
gtgctgtgct ggaattcagg cctgaaccct gtgacctccc tgccctagat cccaaatctg    2220
cccaggtttc ccatcccgat ggggcagagc ctggtcctgg cagagccact ggtatagagc    2280
cactggtaca gatccactga cggtcctcag aacacctctg tgccctaagc tgggtcctga    2340
tggtcgctgt gggccccact gaacacacat ggtcccttgt ccaggggagc ctgctgccct    2400
tgggcagctg tggaaaatga aggagccctg gagggctggc tgaggggaga ctatcttccc    2460
ttgtgttcaa aggggtccgg gcactagggt tctccccagg tatttcttgc tctgcgtggt    2520
cctcttgagg cctcgccctc cttttgcctc gagtattccc aggagggacg gtccatccag    2580
```

ctgttctcca ggaccaagga cccactgttc ttcctcagtg acccaggaaa atgaagcctc 2640
ctcctgttgg gacggctcag aatggtggac tccacagtcc ctccgcgaga gacgtggttt 2700
ccatgcgtac aatagatctt tctcatcccc caaacccaac accctcctgc tcaacaggcg 2760
ttattcctaa agtggcttca ctgttcagac tgaagagcca cggtagccaa agtgatgagc 2820
ggagtagaac cgagcagtcg ggagagatct tgttccctgt aggaaactgg gcatcgctga 2880
ggccctgagc atcccaggag gccgattgca cagagacctc tggtcgctga ccccagtctg 2940
cctccacatc cctggaatag cccatcatgg gcccttcacc cttggcaggt ggaaaccatt 3000
caacctgctg gggccggtgt gtccccattt catggcattg gggacaaca ggattctctg 3060
tctaggtccc actgtactca agtccttggg aagatgccca cccctgcttg ggacttgaga 3120
ctccagagac tggagcagct gtgggccact gggtctggcc ccttttttccc tgggggcggc 3180
ggtggaatgg gggttacgca gccagccagc atctgggagc ccggcgagag cggttcaggt 3240
gttctccgaa gccgccgcgt acagtgtgac ctttagacaa ttctgtctca caggatggac 3300
gtggtagagg tcgcgggcag ttggtgggca caagagcgag aggacatcat tatgaaatac 3360
gaaaaggtac aagtcggtct gcttcttgga gggaggcctc ttccagtgtg ccctggtcaa 3420
agggtcctgg gctccctagg agcacagggc agggacgggt ggccaatgcc cccaggccct 3480
tgcacccttt accttggacc cctcaccaag gctccctctg ggctacaggg acaccgagct 3540
gggctgccag aggacaaggg gcctaagcct tttcgaagct acaacaacaa cgtcgatcat 3600
ttggggattg tacagtgagt cctctgcact cccctcaccc ctaaagcacc tgtctcagct 3660
cagggatggg tttgctttta gaaaggcctt tctgacgcag gacatgtctc accaggtcgg 3720
gtcaacctcc tttccaggga cagaactcct ccctgactcc cctgcaggtc cagcccgagg 3780
ttgttaggcc agaggtgtgg ggcccatcta gggagccggt gggaatggag actgggctag 3840
gtcaggcccc tgggcgctca gcagttctgt cggcaagtga gcacaagagg agcggggcag 3900
cctgagggtc tggccctgtc tacttggaga caaccccggt gagatgcaag ggttatggcc 3960
acagggtgag gggacgcctg gcccagcctc agggctgttg tccagcaggt ctctgagggc 4020
ccacctgccc ctgttctccc ccattcccct agagctacag ccctcactgt cccgtgaggg 4080
gaaaaggcat ggtgacaatg gggctgtag ccctaggaga acgggggaga agatgggcag 4140
ggccccgttc tgggcatctc acggtgaggc cagggaggca gcagggctcg cggctaaaga 4200
cctgggtctg gtgctgggaa gggatctggg gccgggtaag aggagcccag ccaggagccc 4260
atccctcagg gatcacagga tggagagaca gaggatccct ggggaggtag ggcgggaggg 4320
agctgacgag ccgtgccact tctgaaacgc agggtgtgtg gctcgggtgc agggagaggc 4380
aggtggatgc tgggaggtca gaacctgcaa gggccttggg gctgtcaagt ggggtgggcc 4440
cctggtgcag ccagagtaca ccgggcaggt ctcagggcag gctcccttga ccctggcggg 4500
gggatgtggt cactccctga gggactcctg tcagggcccg gtcgcccacc ctgggcggcc 4560
cccatcccat ctcagggcta acctttctca gctccagcag aaagcaccac ctcgagtcca 4620
ggacgggcag ccccactggg cagcctgacc gccccccacg ccagggggccc cagtaacccc 4680
ggccaggctg tccctacact ccttcttctc ccaggtcctg cccctcctgg gagtcagccc 4740
cacaggaagg cccttgtcct cccttccctg tgccttctcc tgggctgagc cctgagctgg 4800
aaagggacag agccagtcct ttctgggggt cggcacccag gctggggccg ctccaggccc 4860
cgtgcagttc ctcagctctg cctgggttgc cttacagtga gacggagctg cctcctctga 4920
ctgcgcggga ggcgaaggta agagcctgat gcgtggaggg gctggtccag ggacgtaggg 4980
actgggcggg tggtcagtga ggcagaggaa gcagctggcc tgagcggtgg cgggtgaggg 5040
caacacgctg tcactgggag gggcagcagt ccctgctgga cctgacccca ggttgctgtt 5100
cactttggca gtttgataaa attccaaaag gagaaccaca gtcctggctt gggggtggct 5160
gcgcgcttgt gtcaggaccc cacctagagg ctgggaccta agactggtgt gtctgtggcc 5220
tgaggatggt acatcccggg gtcccaaagc cagcccactg gtgctcattt gctcaaaggc 5280
tctcagccct tgaggtctgc ccttccctgg ctccttccag ctggctccca ccagggctcc 5340
agagcccaag acccagcatc cgcgggcggc tctgggaagc ctggcagctc cgctaactcc 5400
aacatgcctc atttgacagc aaattcggcg ggagatcagc cgaaagagca agtgggtgga 5460
tatgctggga gactgggaga aatacaaaag cagcagaaag gtaacgtgtg gagggaggaa 5520
gcactctctg cagagacagg ggacaggcac ccatggctgt ggcctggcac catcagcctc 5580
tcagagggtg ggcggcacac tgtcctcgcc cagaggactg caggcctggt cgccagattt 5640
cctgcctatt cgtgcaagcg tcaccttgca gggagggaat ctgaatctag ggctgggact 5700
acccggagct caaggctagg gatgccctgg tgacctgaag gaaggaaaag gttcagatca 5760
gagtttcgac tctgagtgtc catccactct ttcagtcctg ggaagggaga ccctgtccca 5820
gcttgatctc acctctactg aggaatcatg gggccaaaac cgacaatttc cagaatcccc 5880
gggctctggt cctcactggg gtcaccccgt ggcctgtgac accagatcgt tttctgccca 5940
cagctcatag atcgagcgta caagggaatg cccatgaaca tccggggccc gatgtggtca 6000
gtcctcctga acattgagga aatgaagttg aaaaaccccg gaagatacca ggtacgctca 6060
gccagagcac aacaaacagg acaggccgtg tcggggccca ggtctccagc tggagggaac 6120
gtcaagacca ccctggggag ctgggggtga aggtcagatg aacaccctgg gcacagatgg 6180
tgacacagtc accacagaca aactcagctc tggtgaccct ccctggcttc agtaacaagc 6240
caaaatgcag ctttctgcag aaggaaacct tccttctgtc cttccttccc gaagtgctga 6300
ctgtgggctg actgccactg gggcaggga gtcttccatc tgttctgaga ctgcttcctc 6360

```
ctcttggccc tgccctacag atcatgaagg agaagggcaa gaggtcatct gagcacatcc    6420
agcgcatcga ccgggacgta agcgggacat taaggaagca tatattcttc agggatcgat    6480
acggaaccaa gtaagcctac gggagccaca gggtcccagc agagatgggg tgaatgagag    6540
ggatgggggc ttccccggag cagaagccag ggtcacccag gagggatgac acagctgcca    6600
agagctctcc cggcccaggg agcagccggc accatgaacc gagcacctcc ctggttccaa    6660
gccctgggcc agactggaac atgtggggcc agaacccagg aggatcctga ggagatggaa    6720
ggcagcaaac aaaatcatgc acaatggtga agggtgctct ccctgaccca tggggaccca    6780
tggtaggacc cacgggaggg tggcaggata gagggcccat gagccccccc caggcaacag    6840
tgacagcacc aaatgctggg agaattaggg gtcctggaaa ctctcatcca ggtccgctgg    6900
gaacatgaca tggcacagcc acgttggcag cccgttgggc agtggctcac aaagctcgat    6960
ggacttgaac cacacatccc caaagtgtca cagatattga acccactgat ttgcaaactg    7020
acatccacat gaaaccagca tgccaggttc actgcttgac tcctcgtcac tcacacacgg    7080
agccttcggg gacggccttc aacacgggga tggggagagc aaggctggtc ctcccttcaa    7140
acggaagacc cagtgagaaa agggaacgag ccggtgatgc ccgcacgaac gtgggtggat    7200
cctagatgca ttttgctgag ggacagaagc cagacccaat aagctaccac agtaggattc    7260
ccattcctag gccattctgg aaaaggccaa accacaggga ctgagaagca gtctgggtgg    7320
ccaggggctg acggatcggg gagaggctgg gtgcataggg gccaccctgg agacttggag    7380
gatgaaggag tcgccccagg aggggctgga gcggtggccg ggagactctg cacattggtt    7440
tggaaccgtg gaggaactgt acacccacag actgaactgg cgtgtgtgca aactgaaaaa    7500
aaaaaaaaaa aatcattcag agtgaaaagg atcaggcaag tcactgtaca actgggctat    7560
ttgcatgtca cagatgtgga ttttactgaa acatttcttc aagagtctca ggccctgaag    7620
agctcactgc ttatctggtg aaacatctga acctgaaatg ggatttgctg ttaggctttg    7680
tagacaaagt gaaattaaca acatctgcac aaaacaaacc aaagcccccct ttctctgttt    7740
cctaggcagc gggaactact ccacatcctc ctggcatatg aggagtacaa cccggtgagt    7800
attcccggca gtgaggttcc cgggccatat ttccatattg acaggagtgg gtgtctggtg    7860
ggggtgtcgt tgcttctttt aaagttagta tttgtgaccc accaggatat aggaggtagg    7920
atgtcagctc accgctggca taaacctcca aggaaggggg tggtctcaag ggtcaagct    7980
gagacacaga ggagtcaggg cctggactcc tggtgtcacc tgggcctgac caccacttct    8040
cagaacaaga aatgacgccc tcctcctggg gctgccccaa agcccaggag cttggcagca    8100
tcgcacacag gatggtgcta tcagcagaca ttttggacaa ggtgctgaag tgcctgatgg    8160
acttggctct tgtcatgaaa tgaatgtgca tcctgaggaa gcctcttttt cagaggaagc    8220
ctctccttca gaggaagcct ctccagtcac ctctgccctc tccaatgaca tgagtcctcc    8280
caggtgacct cagccctccc aggtgatgtc cttccatggt gactctggct cttgcaggag    8340
gtgggctact gcagggacct gagccacatc gccgccttgt tcctcctcta tcttcctgag    8400
gaggatgcat tctgggcact ggtgcagctg ctggccagtg agaggcactc cctgcagggt    8460
aagtgaacag ctgccccggg gacctcctgc agccagacct ggggatggcc accctggcca    8520
ggtgatcaca gctttcagcc aaggcaccct ccttgtgtcg ccagcttgtt gggagacttt    8580
aggatgtctc tgctgagggt cccacaggag tccacggctg acccccaaag cccaaatcag    8640
acgcctctca tccccatcag cagagggcat ctcatcctcc ccgtggccac cctctgtgtc    8700
ctggagccac gccctccggc tctgattctg tgcagctgac tctcccctcc ctgagagtcc    8760
tcctgccctc cagctgcccg ggctcctgct gccatcggtg cccacgaatg ggccgaccaa    8820
gcccaggtgg cagcatctcc ccatcccctg ttccctggcc cgaccccact accaggagat    8880
gaccgggaag cccagcgccc acccagttcc ggccaccctg tcgtggcctg aaagtcaggc    8940
ttgccctttt tgcaccctgg cccaggaggc ctccagggga acctccagcc aggctccagg    9000
gaatgttccc gccccacctc cccagggtaa aggccgcatg ttggggtcac cagatgggag    9060
ggtgggaggc cttggggttt gggggcctct ccagctgccc agctcttgca gctgatggct    9120
ccacatcttg ggggaaggct ctgatttcat gatgggctgg gggcttctca ggatttcaca    9180
gcccaaatgg cgggaccgtc caggggctcc aagaccaaca ggagcatgtg gtagccacgt    9240
cacaacccaa gaccatgggg catcaggtga gtttatggtc ccctcagctc ttcccagagg    9300
ccctgcctcc cgtggggctg taggagcagg ggggctggag cccctcgtgg ggctggtgac    9360
tggctgagtc ccagccaggg cctgacctgg gacgtcgggt tctccatggg ctgggagttg    9420
gtttcctttc ctgccctgga ggagacagag gcacagggat gggggcccag ctcccgcaga    9480
gcagggcaaa gggcagtgtg tccaccggga gtgtgggaag gtgacagtgt tgtggggagc    9540
tctggacacc gcccagtgtt ctgcactagg ggaagggtct tcagaggccc tggaagaggg    9600
aggtttttag ggcagcccag tggcctgagc acctctgttg cttccatcag gacaagaaag    9660
atctatgtgg gcagtgttcc ccgttaggct gcctcatccg gatattgatt gacggggtaa    9720
ggaggcatag ggagaccctg gctcagggac cttccttgcc ctgcagtgcc ctgcttcccc    9780
agcccggggg tctggctcac tcccagccca caggaggctc aggcgggtcc ccaaaggaca    9840
cacaagcaaa accctctgcc caaggggggt catcccaggg ccatggctgg ggctcaggcc    9900
cagcctcatg ggcagactgg gccaggaccc gacttgagag ggctcaggga agcctcaagc    9960
cctgggcaag cccctctctc caggagccac atccccactc aaatgagtgc cccccatgag   10020
gagcttcaag accttgtctg acccagcgtc ctggagggct caggcgaccc tcatggggaa   10080
ggtcactgac tctggagact gaagccccag tgtgcgcagc tcgagccacc agccccagcc   10140
```

```
tggaaggacc aggttctttc acacctgctg tccccacaga tctctctcgg gctcaccctg   10200
cgcctgtggg acgtgtatct ggtagaaggc gaacaggcgt tgatgccgat aacaagaatc   10260
gcctttaagg ttcagcagag taagtctacg tgtgcccagc ggggcctggg gagccctggg   10320
gtcagacccc gactggcccg agggcagctt cctcacactg tcctcatgat cctctgttct   10380
ggcccagagg gaggtctggc caggtgggct gggcaggaca ctgtgacacc gagcccatcc   10440
cccacatgac ccagatgaaa gtcgagagtg tggtgagcac ttccctgtcc               10490
```

<210> 75
<211> 7001
<212> DNA
<213> Homo Sapiens

<400> 75

```
atgacatcag caactgtcca gtgaggcgga tataaaaccc tcaggacatg agagggagac      60
gtggtcctca catcctgatg tgcaaacatt acgctcaggg aaaatgcaag gtgccccagg     120
tttgtggact ttgcatcttt ctaggtaact tatttattca ctttttaatt tcaacaaatg     180
attattaaat tttactcaat acataaatat ttactgagca ccatttgtgt gcatgagaag     240
tgggagctag catggcaaaa gccaggcact gtgccaggtg agagagaccc agaaattaaa     300
accagagaag tcattaataa gagtctaaat atctgggccc aggctcatgc ctgtaatccc     360
agcactttgg gaggctgaag gaggtgaatc acttgaggtc aggagttcaa gaccagcctg     420
accaaaatgg tgaagcccca tctctactaa aaatacaaaa aattaggcgg gcattgtggc     480
acacgtctgt aatcccagct acttgggagg ctgaggcagg agaatcactt gaacccagga     540
ggcagaggtt gtagtgagcc aagatcgcac cattgcactc cagcctgagt gacagagcaa     600
gactccatct caaaaaaaaa aaaaaaagag tctaaggatc tgatggagga gaaaggcaag     660
aacatgtgcg agacaacgca aggccatcgt cccagggtgc ccagggtaac cacgggggta     720
gggcactccc cggagaggcc aatgacaagc aggttgaaca aagcaggggg gctccctgca     780
ggaggaggtt caccaggtga agatggagcc gcatgggcaa aggccattcc agagacccag     840
gtgtgttcag gaggtggaaa cccattgcag gtaaggtgag aggaccgggt ggggtggtct     900
aggaggagcc gacagagggt acaagctgtg aaacagcttg aagcagggca gtgaggaaag     960
ggatctagag gaggaagaca cgtggacaga tggggctggc tgggggctgc cgcaggatct    1020
tatgcaagag gttctaacac cagagcttca ggctctgagc tccgcggaat caaaggtctc    1080
agaaagcaac ctattaggat ctggtggttg acagcctgca gcaggggtg aaagaggagc    1140
ccagagcacc ctccggtccc tgtccctgcc ttggggcata ggaggggagg aactcagtct    1200
ggccacactg gctcaggtga gggcgcccca ggggaggccg agaggggctg ttctcttgtc    1260
tgctttgcta tcagggactg cctcgagatg tctttggaga aagtgttcct ggcttgctgg    1320
gaaggatccg tgttcagctc ccgctgccca gcagcttcca tgggaacctt gccctcctgg    1380
ggtctggatc catcgcgacg tgaaggtgat catcgcccac ccccgggaca ctgtggggt     1440
caagagaggc cccgtggtga gggaggatca tcatcctggg ggtcgggggg gtttcctcct    1500
cagggaggaa gatttccagc cccggtgctc tgcctccggc agactctgtc ttgcccaccc    1560
gctttgtctt tgtcgcatga tggaaataaa tggaaatggt ttccacacac gaatgcacta    1620
aactgtaatc acaattttct agatttcagt tgtaacagga tcatggacct gagtgacccg    1680
caaagacgct ctgagccttg agccagaggg tgtgtggggt ggggagggga gtttgccacg    1740
gcctctgtga tcccacagca caggggcag agctggggc tggggtgggg gcaagggcgc      1800
aggcagatgg gcctggggt ggccagcacg gggacccacc cagttcgttc tgtacaccga     1860
ggccacttcc tctctcgggt tccaaagctc ccccgctccc tgccatgggc ttggggtct     1920
tcccactgca gcccaatgct ggctgctctt ctacgctgcc caagcccacc cctcaggccc    1980
ggtaccccac agcagagatc aagaggtggc tggagggcag tgggggcacg gacagcacca    2040
ctgggcgctc cctcttcagg cctctctgga aacccttgct ttggaaacgt agaaagccct    2100
ttctcctgcc cccaccttga aactgtacct aataacggca aataagttat tcagctcccc    2160
acagctggca aagtgcaagt cgcagatgct gccacacagc atttcctgac agccctaggg    2220
cagaccgttg atccattctg caggtaaagg agttgagatg caaacacttc ccaaggaagc    2280
aagaatggcc tcctcctcca ctccctagaa ggacccaggg caaccgatcc acttgacaac    2340
accagggaat atgggttgcc agccacgcac tcatgagaga ggtggctttg actcccagag    2400
catggatccc aggggagccc aggaacctgt aggagagggt ctcccagttg gccaccactg    2460
ggacgggcat tcgggcctgt gggagagggt ctcccaggtg gccatcactg ggaccggcat    2520
ttgggcctat ctggatgggc cttgggaggg tctgcccctg ggggagatgt tggcatgaac    2580
agtcaaaagc actattctga gaccccacgc caacacttcc tctttgaaac atgacacttg    2640
ggaggattgt atagtcttcc taacacagga aaacagcccc gactgaaggc agcccctcc     2700
tcgtcgcact tctcgaaggt ctccgggggc cctgccaccc tgagttactc tccacctgct    2760
tttgccgatc ctgtaaattg gatatacttt taagggccta gaacagcacc tggcacaaaa    2820
caggtgctca aaaatatgta tgtaaaacag tggctcctgg cggaggctgc cagagctggc    2880
tgtggcccca cagagcagga agaagcacgc cttacctgtc tgggcctcgg ccagggtgcc    2940
```

```
gagggccagc atggacacca ggaccagggc gcagatcacc ttgttctcca tggtggccat   3000
tgcctcctct ctgctccaaa ggcgaccccg agtcagggat gagaggccgc ccgagccccg   3060
gattttatag ggcaggctct gtttgcttaa agagcgttag ataacatttg cctaaggagg   3120
cccggggatc ctctgagaca ataatctcca ctgattttta tcaaaggtgt ttcctagaca   3180
tggtcaagct acatggaagg atttgctgat agacagagac gacatgtggt gaggtcatct   3240
tggctgaggg atctgagatt cagaaagtcc ctctttccca tgggagtctc ctccaacctg   3300
accttaatcc aggtcctact catatctgag aggccctccc gccagggtaa atactgtact   3360
cactgcagaa gtgattcata gtgagagatg gccggaaaaa ggcttggccg tgacaacagt   3420
ggctcacggg gtggccaccg tgaccttgca gggggaaggg aaggagctca tgaagcccat   3480
tccgtctagg cctaagctaa ttcttttaca gtggaattgt tttaataatg aaattgtagg   3540
cctggcgcag tggctcacgc ctgtaatccc aacactttgg gaggccaaag caggcggatc   3600
acttaaagtc aggagtttga gactagcctg gccaacatgg cgaaaccccg tctctacaaa   3660
aaaaaaaaat acaaaaatta gccaggcatg gtggcgggtg cctgtaatcc tagctactct   3720
ggaggttaag tcaggagaat cacttgaacc tgggaggcgg aggttgcagt gagtcgagat   3780
caccctactg cactccagcc tgagcgacag attaagtctc cttctgggag gggctggggg   3840
caggagggag aaaaaaatag tatatacgag ctgccatcaa aaactgacaa agtgaacgtg   3900
gttcactccc ctgtgtctat ggggctccct ccctccccct cccacacctt cccagttggt   3960
cccctggact cctgtatttt agaggacagc ctcctgctct cctgtaccat gcctcactcc   4020
cctcccctgc tcggagcttt ctctttcata gctcttatta cctcctggtg gataaaacaa   4080
gtgactgaca cattatgttg gttatttatt cattgcactc ctccctgcag cagacgggag   4140
ggcgcatggg agcagaagtc ctcatctcct agtgccttga ggtggcccca gaccctagaa   4200
cagtggcttt gattggcatt cgagaaacag ctgttgaaga gctgcatgaa gaaatggaca   4260
acaggagaaa tcttttcttc ttcagtgaga aattaggccc tgaggaaaat gcatgctagc   4320
ctaacaaagg tactatcttt tcttgggtga cctagtcagt acatttctgc agcccctctg   4380
ggaggggagc ctgggccaga atgggggcgg ccatgttcct cttcgctcca tcccaggacc   4440
cagcccctcc tccagggctg ccactcctgc accggggtct ccactcctgg gccagggtct   4500
ccaggagggg cctgaccgca ggacccaggg aggggccgag gcctggtgtc ctctgtggtc   4560
agcttaagtt tattgaaaac aggacggggg caccaggccc agggtggtgc tcatagtgac   4620
ggtggttttc gctgctctaa tttctcggat caaaatgggg gctcttggaa atgggatgta   4680
aattgcacaa tcatttggag aaaaggtggg cagtgttcgg tgaggtttaa gccatacaga   4740
ttttacagct cagcaaattc ttctgtttcc aggcctggtc tcttaatccc agaaacatgt   4800
tcatacttgg gcagaagaaa acacaggcaa agatattctt tttagcattg tttgtaacag   4860
ctggaaacag ggaaagaagg aaggaaggaa aggagggaaa gaaaagcaaa ataaatattc   4920
attaaccagg gaaagggaaa atgaacttaa aatacaatgc agtcgtccct tagtacccct   4980
gggggactgg tttcaggacc ccccacccag ggatgccaaa atccacaaat gctcaagtcc   5040
ctggtataaa atgacatagt gcgcagaaac cacgtgcatc ctctcctaca ctttaaccat   5100
ctctaggtta gttataatac ctgatgcaat acctacacct catttcattc tgcagattct   5160
acatagtact tgacttgcgg cacacccaag ttttgatctt taagatgttg taaataagat   5220
tttaaaatac tttttttttt tgagacagag tcttgctctg tcgcccaggc tggagtgcgg   5280
tggcatgatc tcagcttact gcaacctcca cctcccaggt ttaagtgatt ctcctgcctc   5340
agcctcctga gtagctggga ttacaggcat gtgccaccac gcccagctaa tttttgtgtt   5400
tttagtagag acagggcttc accatgttgg ccaggctggt ctcgaactcc tgacctcagg   5460
tgatgcaccc gcctcagcct cccaaagtgc tgggattaca agtgtgagcc actgtgccca   5520
gctggatttt aaaacagttt tgatctgaca ttactggaat ccatggatgt agaacccatg   5580
aatatagaga actaattgca tacatgtatt atactttaca tatattatat atgtatgtat   5640
gtgagtatct ttcagaagat gaaaatgaat aaataatgag aaatattgaa aacaaagcga   5700
agtgaaaaaa tccgttaaaa tggtttgtaa agtaggatat catttataat gtagatttcc   5760
aaactatgtg aaacaatgtg atatactgtt ggaactgcat ccagatgaac aaaatgatga   5820
cagtgtagac aggaacgaga cactgacttc agcatgatgg gtctgtctag gaaggaggaa   5880
gcaggaaaag gtggcggggt aggcgcaaa ggggccttcg atgtctccgt aatgttttct   5940
tttgttgaag aaaatctgag ggaaataagg taaaaaggtt agcatccatt aatgaggggc   6000
agcgtagatt cacggatgtt gttctagcat tctgtacatg tttgcaattg ctttcttgga   6060
aagcccagct cctatctcct tgggagagct gtgagataac taccaagtgc cggagtttgg   6120
cctcccacat tagggttgga gcggctggag ctgcccccgg gactctccgt ttctcgggac   6180
aagagcttta tccctgtgag agtgggcgga acccaagacc acaccataca taaccttatc   6240
cataaaaact tgttgaagac aattccagtg tggcgaggga gagaatgcag aggcaagcgc   6300
cagtccatca tcagtggggg accctgtgcc agcattttca cagctctggg tcttagtttt   6360
cttatctgaa agacgcagtg gctgggccac aggacatctg aagactccgc acctcagacc   6420
ctctccggca ggcatctatg gccgtggggg cgtctgtggc tgtgcaggca ttgcttgttt   6480
cctgcgccca gccgacctcc ctgcggtctg gcgagagccc ctcattttca gtgggggcc    6540
ccgcagccgc ccagattccc cacaaggggg acgcgcagtt ggggagtcag tctcctcctg   6600
acgctcccca cctgcccgct gcccctgacc tgtgccttgg caggaggggg ttctgtaggt   6660
ggccctggag ggagggagga gtttgggagg aagtggatcc gggctctcct ccaggatccc   6720
```

```
tcctgggatc acctgggaca ggccacgtcc ccctctcagc cacagcccag gtgggcgtcc    6780
tctccacaca ccatcttccc caggccccag agcagacccc tcacccccct gccccagcct    6840
gggggggttta ccagccgcaa ggagactttt acagtcttca ctctcctcgc attgcccaga    6900
tccgctgtgg tgaagcccgt ttcctgccag gcctctggtc aaaaaagcaa ctgccacccc    6960
agggaccctt tacaaggaca cccagctgag gccctggggt t                        7001
```

<210> 76
<211> 6499
<212> DNA
<213> Artificial Sequence

<220>
<223> chemically treated genomic DNA (Homo sapiens)

<400> 76

```
tattgtatat ttttatttat aattggtagt taattattga atatatatag atataaatgt      60
attttaatat attattattt ttatttagat tttgtgtaat atttaattat ttaattatat     120
taataaataa aagtttttag aaattgatgt atttagtata attaaggggt aatttgttta     180
tgattatagg agttatttta tttatattaa tttgatgtga ggagataatt aaattttttt     240
tttttataa gaaaaattta atttcgggag gtttaggtag gagaattttt tgaatttagg     300
aggtagaagt tgtagtgagt cgagattgta ttattgtatt ttagtttggg taataagagt     360
gaaattttgt tttaaaaaaa taaataaata aaataaaata aaaagtaaaa tttaattttt     420
atattttttt tttaaaaata ttatattatt gaaatagaat agaattttgg attttaatgt     480
ttttatgttt tttgataatg taaagattat attaagaata atattgattt tttgtgtata     540
tgtgtgaaat tatgtcgagt tgatgtattt agaaaagaaa ttgtagcgat attgggtata     600
gaattttaa ttttaatttt ttttttgttg tggaatagaa atttagtttt attttttttg     660
tttataatat ataagagtag ataattagat atgaggtgat taaatatata aattgatttt     720
atagttatta tggaaagatt ataatatgtt tggacgaggt gggggaaaga gttttttttt     780
gaattgagat ttgaataggt tttagataga ttaaaaattt atggtttgtt ttttttaatt     840
ttttattatt gattatttgg gagaattaaa atggtttgga ttataaagtg attataatta     900
gtagggtagg tatattaaat aaaaatgggg tgagtgtgtt tggatgggaa aaattggggt     960
gtagagaaat atttttgtt ttggaggttt cgtttatagc gagtttgagg atgtagatga    1020
tatggaatat gagttttatt aggatatata tttttttgta tagttttttt tgattagtgt    1080
tgtttattgt tttaggtatt atttttagtt attttgtgtt aggtatttag gatagttttg    1140
tttaaagtat gattgttttt tggttagtga agaaatagta aagaaatgag agtttgtttt    1200
tgaagttttt gtagtaattt aatattttttg taatgtcgtg gtatatgttt tatattttttt    1260
aaaagtattt atttgtagat tagaaataaa agagtttttt agtatagata gtttgagaga    1320
tgttattgta gagtgttttg ggaggatggg attttttttag atattgacgg tttttttggag    1380
tggtgttgga gtttgttgag atagggacgc ggggattgtt gtggaatagt atgttgtgaa    1440
aataggtata gatgggttgt tgttacgtat ttagtatttg gtaggtttag gtattttgtt    1500
tcggggagat agagtaatag ttgttatttt taaaaatgtg atataaatat gattttcgtt    1560
ttttattgta tgtgtatgta tgtgtgtatg cgtatgtgtg gttggtgtgt gggtaaataa    1620
tattggtgag ggagttagta gtgtagagac gtagagatga atttaatgaa tttatatttg    1680
gaggttttg aaaggagttt tgtgtatgta taattaggaa gtagaagtgt agtcgtaggt    1740
tagttttatt ttttggtgga aatagtaaat tatagtttta gtattttttta tttgtagtaa    1800
aagaaatgta tgttgtattt gggaggggta ggagaatttt ttagaatttg gtgggtttat    1860
tagttagggt tgtgtagaga aatagaatta atgagataaa tatgttagat agatgataga    1920
tagatagata gatagataga tagatagata gatagataga gatgatagat gatatagata    1980
gatgatagat gatagataat agatgatata tagatagata tagtaaatag atttatagat    2040
atgatagaga gagaatgaga gagatttata ttaaagaatt ggtttatatg attgtggggg    2100
ttgtaaattt tatatttgta gaatttgttg gtaggatgga aatttaggta agagtggata    2160
ttgtagtttt gagtcgaaag tttatagggt agtaagttgg aaatttaggt aaggttttta    2220
ggttgtagtt ttaagaagaa ttttatattt tttaggaaat tttagtttgt gtttttaggg    2280
tttttaattg attgaatgaa gtataaatat tatggaagga aattcggttt atttaaagtt    2340
tgttggatta agtattaatt ttaattaaaa aacgttttta tagtaatatt tagattattg    2400
tttagttaag tatttgggta ttatagttta gataagttga cgtataaaat ttattattat    2460
aagggaggaa tttatatata tattttattg tagtaaaatt tttatagttt aacgttttgt    2520
atttttttta gaaggaaacg ttttagtgta gagttgaata tcgtattttt tcgtagttta    2580
tataaatttt atataatttt gatttgttga gtttttttttg aaaattggat aatttaagtg    2640
tttatgaaag gttttaattg tttaagaaaa tagattgttt gtgtgaatta taaagaaaaa    2700
gggattttag aaggaatatt ggtatttcgg gaagtaggtt ggggtaaggt ttgtataagt    2760
gaattagaag ttttaggtac gaagttagta tttttgttat ggtttatgtt agttgagttt    2820
```

```
ttatttttgt ttcgtgtttg gtttttagat tttgtgagtt cgtttcggga tagggttacg    2880
gttaatttag tagagatttt ggtaaagtat ttcgggaatg agagtgagaa aggtttaaag    2940
tagttaaggt ttaaagataa ttttcgattt tattttttta aatgttagtt tttaagattt    3000
taggtttttt ggattttaat ttttatgtta ttttaaaggt tttaaatttt agttttagta    3060
gttttagttt atagttttag gtttttagag attattttag taatttttaa atattatttt    3120
agggatttag ttttgatagt taagattatt ttttttataa aataatcgta gatattaggt    3180
gaagattttt aaagttttaa ggaaatttta aattttattt tgaggatatc gattttatta    3240
aagtttcgag gaaattttaa attttatttc gaggatatta gttttatcgt taggaatcgg    3300
gattttaatt tatagtattc ggatttcgag aatagaggtt tcggggttaa atgggttgaa    3360
tttagtattt tatttttacg ttttcgggtg gatagtaatt tttttttatc gcgtttttttc    3420
gtgggtttta ggttttatat ttgagggatg tggttttttt tttttatatt atgttggtta    3480
agaatgattt atatagttat tatggaatat tgtatggaga taaggagtgg ttgtgttttt    3540
gtttgaaggg tttattttat ttatgaggta ggatgatttt taaggtagtg ttatttatta    3600
gagttaaagt ttaggtaagg tgtttagggg ttagttgtaa tatgaagtat attagttaag    3660
gtagttttaa agagtattgt tagaggaaga atttatattt ggttgtttaa tgaggttgaa    3720
aggtaaattt aggttaagta ggttaagtaa agaggttagt ttagtatttt gggaggtaga    3780
ggtaggtgga ttatttgagg ttaggagttc gagattagtt tggttaatat ggtgaaattt    3840
tgtttttatt aaaaatataa aaattagttg ggtgtggtgg tacgcgtttg taattttagt    3900
tatttaggag gttgaggtag gagaattgtt tgaattaggg aggcggaggt tgtggtgagt    3960
taagattagg ttattgtatt ttagtttggg tgatagagtg aaatttcgtt ttaaaaaata    4020
aataaataaa taaataaaaa taaaaaagaa gttagaaagt ttatgaaaaa tatttggagg    4080
gaagaagtta gtttaagaat aaaagtatta ggttagggcg tttaagattt ttaaggattg    4140
gttttggttg tgtagatatt taaagagatt gtgtaaggtt ggataggagg gtgagttggt    4200
taaaaaggtg atttgttaat tgtggaatta attatcgttt aatgttttgg ataattgagt    4260
ttgattcgta atggttaggt ttattattgt aaattagttt tgaagtataa ataagtttat    4320
taaattattt tttttgttta tatttttttt tatttttttga tttatgtaat ttttgtagtt    4380
tatatttaaa taaaaaataa taatatggat ttgttattat gtatgtcgtg gtttattttt    4440
aattgtaaag tttttgatta tttttttgtt atgttgattt ttatgttaga agaaaacgtt    4500
ttgttattta ttatgagtgt agaaaattgt ttattagaaa cgatttttaaa tagtaaagga    4560
tgtatatttg aaataatagg gttgtaatga aaaagtatat atttttttaat tttatttaat    4620
atattgtatt cgtagagtaa aagatataga aaagtatttt atgttttaaa aatgtaagtt    4680
tgagagtgaa aaatagagat aataaaaatt tttttttaaa tattattggt ggaaaagttt    4740
taaagtaata gaaagtatat ataaaatgtt tgtgaatttt tgtgtaataa aattatatat    4800
atatatgtgt gtgtgtatgt atatttgttt tttttgtttt gttttgtttt tttttttttt    4860
tttgagacgg agtttcgttt tgtcgtttag gttggagtgt agtggcggga tttcggttta    4920
ttgtaagttt cgtttttcgg gtttacgtta ttttttttgtt ttagtttttt aagtagttgg    4980
gattataggc gttcgttatt acgttcggtt aatttttttgt atttttagta gagacggggt    5040
tttatcgttt tagcggggtt ggtttcgatt ttttgatttc gtgatttgtt cgtttcggtt    5100
ttttaaagtg ttgggattat aggcgtgagt tatcgcgttc ggttaatgta tgtatatttg    5160
taatttagat ttgtaaatgt agttatattt atatttatat attaaatgta ttaaaaagat    5220
ttattaagtt ttttatagtt agtttataaa tttttagttt ttttatatat tcgtattttt    5280
tgttttaata tgatataata gttaaaaaaa tttaaaaatt aataattaat taagtttttt    5340
ttttttgtag attatattag ttattattta gaaatattaa tttattgttt taaaaataat    5400
ttttttagag atgtttggta tttagtatat gaaattattt ataattgaat aatataaata    5460
aaatagaaaa aagaatttat ttttgggata tttgaagaaa gtgatttata aatttagttt    5520
atttttgagt tttattattt ttaggaaaat ggtttttatg ttatttttat gttatgtttg    5580
atttgttttg tttgagtttg tttttttagaa agattgtgat ttttggtttg ttgaatatga    5640
aatttatatg attattttttg ggaaaaattt tttttttagt gattgtaagt taggttttgt    5700
ggtcgagaag tattagttgt atttggttga ttgattgtat tattttgttt ttatgttgtt    5760
tataaagata tatttgagat ttggtaattt ataaaagagg tttaatagat ttatagtgtt    5820
aagtgggtgg gggtgtttta taattatggc ggaaggtgaa aggtatgttt tatatggtgg    5880
tagataagag aagagagttt gcgtaggaaa attttttttt ataaaattat tagattttat    5940
gagatttatt tattattata agaatagtac gggaaggatt tatttttatg atttaattat    6000
ttttaattag gatttttttat aatatgtggg aattatggga attataattt aagataagat    6060
ttgggtgagg ttatagttaa ttatattatt gatttttatt atattataaa gaattatttt    6120
gtagagagaa tttgtttttt atttttaata atttgtttat tttggatatt ttatataaat    6180
agagttatat aatatttgtt gttggttttt ttatttagtg taatgttttt aaggtttatt    6240
tgtgttagag tatgtatttt tgttttattt ttttatatta ttaaataata ttcgattgta    6300
tggatgtgtt atattttatt tgattttttta ttagttgata gattgttggg ttttttttatt    6360
ttttggttat tacgaatgat ggtattatat gaatatttat atataagttt ttatgggtat    6420
atgtttttat ttttttttggg tatatattta ggtgggggat tgttgttata tggtaatttt    6480
atgtttaata ttttgagga                                                 6499
```

<210> 77
<211> 6499
<212> DNA
<213> Artificial Sequence

<220>
<223> chemically treated genomic DNA (Homo sapiens)

<400> 77

```
tttttaggat gttaaatata aagttattat atgatagtaa ttttttattt aggtatatat     60
ttaagaaaga taaaaatata tatttatgaa aatttgtgtg tgaatgttta tatagtatta    120
ttattcgtaa tagttaaaaa gtagaaaaat ttaatagttt gttaattgat aagagattaa    180
ataaaatgtg gtatatttat ataatcgaat attatttagt gatgtaaagg aatgaagtaa    240
aggtatatat tttaatatag atgaattttg aaaatattat attaaatgaa aaagttagta    300
ataaatatta tataatttta tttatgtgaa atatttagaa taaataaatt attagagatg    360
gaaagtagat ttttttgta agatagtttt ttataatatg atgaaagtta gtgatatggt    420
tggttgtgat tttatttaaa ttttattttg aattgtagtt tttataattt ttatatgttg    480
tggagggttt tggttggagg taattgaatt atgggggtgg gttttttcg tgttattttt    540
gtgatagtga gtaagtttta tgaggtttga tggttttata agaggaaatt tttttgcgta    600
agtttttttt ttttgtttgt tattatgtga gatatgtttt ttatttttcg ttatgattgt    660
gaggtatttt tatttatttg atattgtgag tttgttaaat ttttttgta aattgttaag    720
ttttaggtat gtttttatga gtagtatgaa aatagagtaa tatagttagt taattaagta    780
tagttgatgt ttttcggtta taggatttgg tttgtagtta ttaaaaaagg aatttttttt    840
aagagtgatt atgtaaattt tatatttaat aggttaagga ttataatttt tttggaaaat    900
agatttaagt aagataagtt aaatataata tagagatgat atagagatta ttttttaaa    960
aataatgaga tttagggata aattgagttt gtaggttatt tttttagat gttttaaaag   1020
taagtttttt tttttgtttt gtttgtatta tttaattata aatgatttta tatattaagt   1080
gttaaatatt tttagagaaa ttatttttaa aataatgaat taatgttttt gaataatggt   1140
tgatatagtt tataagaaaa aaaaatttgg ttaattatta atttttaaat tttttaatt   1200
attatattat gttaagataa aaaatgcgaa tatgtgaaga agttgggaat ttatggattg   1260
attatgagga atttagtgaa ttttttggt gtatttgatg tgtggatatg aatgtggttg   1320
tatttataaa tttgagttat aaatatatat atattggtcg ggcgcggtgg tttacgtttg   1380
taattttagt attttgggag gtcgaggcgg gtagattacg aggttaggag atcgagatta   1440
atttcgttaa aacggtgaaa tttcgttttt attaaaaata taaaaaatta gtcgggcgta   1500
gtggcgggcg tttgtagttt tagttatttg ggaggttgag gtaggagaat ggcgtgaatt   1560
cgggaggcgg agtttgtagt gagtcgagat ttcgttattg tattttagtt tggcgatag   1620
agcgagattt cgttttaaaa aaaaaaaaaa aaataaaata aaataaaaaa aataaatata   1680
tatatatata tatatatata tatatgattt tattgtataa aaatttatag gtattttata   1740
tatatttttt attgttttga gatttttta ttaataatat ttagagaaag gtttttattg   1800
tttttgtttt ttatttttag atttatattt ttaaaatata agatgttttt ttatattttt   1860
tattttacgg gtataatgta ttgaatgaga ttgagaaata tgtatttttt tattataatt   1920
ttattatttt aaatatatat tttttgttgt ttagaatcgt ttttagtaag tagtttttg    1980
tatttatggt gaatagtaaa gcgttttttt ttgatatgaa gattagtatg ataggagaat   2040
aattagaaat tttatagtta gaagtaaatt acgatatgta taataataag tttatgttat   2100
tatttttat ttagatatga gttatagaga ttatatgaat tagggaataa aagaaaatgt    2160
aaataggagg ggtagtttaa tgaatttgtt tatattttaa gattgattta taatggtaag   2220
tttaattatt gcgagttaga tttagttatt taggatattg ggcggtggtt gattttatag   2280
ttagtagatt attttttgg ttagtttatt tttttgttta gttttgtata atttttttaa    2340
gtgtttatat agttaggatt aatttttggg gattttagac gttttgattt aatgtttta    2400
tttttggatt aatttttttt ttttaaatgt tttttatggg ttttttgatt ttttttttat   2460
ttttatttat ttatttattt atttttgag gcggagtttt attttgttat ttaggttgga    2520
gtgtagtggt ttgattttgg tttattataa ttttcgtttt ttttggtttaa gtaatttttt  2580
tgttttagtt ttttgaatag ttgggattat aggcgcgtgt tattatattt agttaatttt   2640
tgtattttta gtaaagataa ggttttatta tgttggttag gttggtttcg aatttttgat   2700
tttaggtgat ttatttgttt ttgtttttta aagtgttggg ttgatttttt tatttggttt   2760
gtttgattta gatttgtttt ttaattttat taaatagtta ggtgtagatt ttttttttgg   2820
taatattttt tgagattgtt ttggttaatg tgttttatgt tgtaattggt ttttaaatat   2880
tttatttgag ttttgatttt agtgagtagt attgttttga gggttatttt gttttatgaa   2940
tgggatgagt tttttaggta ggggtatagt tattttttat ttttatgtaa tattttatga   3000
tgattgtgta gattattttt ggttaatatg atataaaagg aggaggttat atttttaaa    3060
tataggattt aaaatttacg aggggacgcg gtggaggagg gttgttgttt attcgggggc   3120
gtgggagtga ggtattggat ttagtttatt tggtttcgaa gtttttgttt tcggaattcg   3180
```

ggtgttgtgg gttgaggttt cggtttttaa cggtgggatt ggtgttttcg agatgaaatt 3240
tggggttttt tcggggtttt ggtgggatcg gtgtttttag gatgagattt agggtttttt 3300
tggggttttg gggattttta tttaatattt gcgattattt tatgagagga gtggttttgg 3360
ttgttagaat tggattttg gggtgatatt tgggagttat tggagtgatt tttgaagatt 3420
tagggttatg agttggagtt gttggggttg aaatttgggg tttttgaagt ggtatggaga 3480
ttgaggttta gagagtttga gattttgagg gttgatattt ggagagatgg ggtcgagggt 3540
tgtttttggg ttttgattgt tttgggtttt ttttattttt attttcggga tgttttgtta 3600
gaatttttgt tggattggtc gtaattttgt ttcggagcgg gtttataggg tttgaaggtt 3660
aggtacgagg taaaggtaaa gatttaattg atataggtta tgatagaggt gttgatttcg 3720
tgtttaaaat ttttgattta tttatgtaag ttttgtttta atttgttttt cggagtatta 3780
atgttttttt taaaattttt ttttttttgt aatttatata aatagtttat ttttttaagt 3840
aattaaaatt ttttatgaat atttaagttg tttagttttt aggagaggtt tagtaaatta 3900
aagttgtatg ggatttgtat gagttacgga aagatgcgat atttaatttt gtattgaaac 3960
gttttttttt gggaaaagta taaaacgtta aattgtaaag attttgttgt aataaagtgt 4020
atatataaat tttttttttg tgatggtgaa ttttatacgt taatttattt gagttatggt 4080
gtttagatat ttggttgaat agtagtttaa atgttgttgt gaaggcgttt tttagttggg 4140
attaatgttt aatttagtag attttgagta aatcggattt ttttttataa tgtttgtgtt 4200
ttatttaatt agttgaagat tttaagaata tagattgagg ttttttaaag aatatgaaat 4260
ttttttttaag attgtaattt agaaatttttg tttgagtttt tagtttgttg ttttgtggat 4320
tttcgattta aggttataat atttattttt atttgaattt ttattttgtt agtaaatttt 4380
atagatgtag gatttgtagt ttttataatt atgtgagtta attttttaat gtaaattttt 4440
tttatttttt ttttgttata tttatagatt tatttgttat atttatttat gtattattta 4500
ttatttatta tttattattt atttatatta tttattattt ttatttattt atttatttat 4560
ttatttattt atttatttat ttattattta tttaatatat ttattttatt ggttttgttt 4620
ttttatataa ttttgattaa taaatttatt aaattttaag aagttttttt atttttttta 4680
ggtgtagtat atattttttt tgttataggt gagaagtgtt aaggttgtaa tttgttgttt 4740
ttattagagg atgggattgg tttacgattg tatttttgtt ttttgattat gtatgtatag 4800
ggtttttttt aagggttttt aaatataaat ttattagatt tatttttgcg tttttgtatt 4860
gttaattttt ttattaatat tatttattta tatattaatt atatatgcgt atgtatatat 4920
gtatatatat ataataaaaa acgaaagtta tatttgtgtt atatttttgg aaatgatagt 4980
tgttgttttg ttttttcggg gtagggtgtt tgggtttgtt aaatattgag tacgtggtaa 5040
tagtttattt gtgtttgttt ttataatatg ttgttttata gtaattttcg cgtttttatt 5100
ttagtaagtt ttagtattat tttagggagt cgttaatgtt tagaagaatt ttattttttt 5160
agggtatttt atagtagtat tttttaaatt atttgtgttg aaggattttt ttatttttag 5220
tttgtagatg aatattttta aaaaatataa aatatgtatt acgatattgt agaaatgtta 5280
agttgttata aaagttttaa aagtagattt ttattttttt attatttttt tattgattag 5340
aaagtaatta tgttttgaat agaattgttt taagtgttta atatagagtg gttgaggatg 5400
gtgtttaggg taatagatag tattgattag ggaagattgt gtaggaggat gtatattttg 5460
gtggggttta tgttttatat tatttgtatt tttaggttcg ttatgaacga agtttttaaa 5520
ataggaaata tttttttgta ttttagtttt ttttatttaa gtatatttat tttattttta 5580
tttagtatgt ttgttttatt aattgtaatt attttataat ttaggttatt ttgatttttt 5640
tagatgatta atgatgggaa gttgaagaag ataggttatg ggtttttagt ttatttaaag 5700
tttatttaaa ttttagttta gagggagatt tttttttta tttcgtttaa atatattatg 5760
gtttttttat ggtaattgtg aaattagttt atatatttaa ttattttata tttgattatt 5820
tatttttatg tattatgaat agagaaggtg gaattaaatt tttattttat aataagaagg 5880
aggttgagat tggaaatttt atatttaata tcgttgtaat ttttttttg gatatattag 5940
ttcgatatag ttttatatat gtgtataagg agttaatgtt atttttgata taatttttat 6000
attgttaaaa agtatagaaa tattgaggtt taaaatttta ttttatttta atagtgtaat 6060
gtttttaaag aaaaaatgtg agggttaagt tttatttttt attttatttt atttatttat 6120
tttttttgaga tagagtttta tttttgttgt ttaggttgga gtgtaatggt gtaatttcgg 6180
tttattgtaa tttttgtttt ttgggtttaa gagatttttt tgtttaagtt tttcggaatt 6240
aagttttttt tataggaaaa aaggaatttg attgtttttt tatattaaat tagtgtaaat 6300
gaaatgattt ttgtaattat aaataaatta tttttttaatt gtgttagata tattaatttt 6360
taaaggtttt tatttgttga tgtaattaaa tggttaaata ttatatagaa tttaagtggg 6420
aataataatg tgttaaaata tatttatgtt tatgtgtatt taatgattag ttgttagtta 6480
taagtgagaa tatgtagta 6499

<210> 78
<211> 6456
<212> DNA
<213> Artificial Sequence

<220>
<223> chemically treated genomic DNA (Homo sapiens)

<400> 78

```
taagaatttt aagttattat ttttgtgtat gtaggtagta ttagtgttag taataataag     60
ttagtttttt tggtaaattt attaaaatta aattttaaat ttttgaaaaa attaatatgt    120
ttattatgtt tttgttagtt tagaggttta attatttgtg ggggagggtt tataattatg    180
attaattatt tatgatttga aggtatttat aaaagtattt gttttttaa gaaattatta    240
aagaattagg tttaagattg taaaattaat tttaggggta atagtaaatt ataggtttgg    300
ggatttaatt ttgataattt tttttggaa agaaaagaat tgttgttaat tgtttttatt    360
ttttgatttt gtttgagtaa atattcgtag tttttaaaat ttagaatttt tagtttggtt    420
aagtaagtta tggggattaa tggttgtttt aaggtagagg tagaagagaa gaaataggaa    480
ggaaataaaa agggaatgaa gatttgtagt ttatgagggt gatattatta ataatttttt    540
taagtattat tgaattttat ggttcgtgat tgagagtttt ttttgatatt ttataggtta    600
attattttta aggatttttt tggtttagag gtttgtaatg tttgggtttg aatatgattt    660
gttttataaa aaaggttagg tgaattgatt ttttttgtg aagttgtttt atggtaagag    720
attattaatt tgttgaaacg taaataattt tttagatatt tgaagtattt ttaaatttat    780
aaagtgtttt tatatacgtt attttatttt atttttacga ttttaatagg taggtgttat    840
tattatttta atacgtaaaa ataattttag aaaataatgt ttttgttttt agtttataag    900
aatatttttt tgaaatttat agtatagaat ttaagttatg ggttagttag aaacgtttag    960
aaatatatta attttaggag gataaattaa gagaatagga tggattagat aaaatttata   1020
agtataatgg tagaaagtgt gtttttggta taagaatggc gtaagttttg taaataatag   1080
tgttatatag gggtgttttt gtagtatttt tacgtttgga aggatattat ttatttatat   1140
atatgataag tatttttaat tggatatttt ataggtaata gataaaataa aataagaatt   1200
ttcgttttaa aattgtagtg gtggaattta tttttcggga tgtattaggt gatttttttta   1260
ggacgatagt tgagttatag gggttaaat atattttttt ttttttttt taatattata   1320
aagagtttta aaaacggatt ttgttgtttt taataaataa aatatatatg aaaaaaagtt   1380
atttataggt ttttatatat aattttattt tttaaaatat ttattttttt ttattagtta   1440
ggtcgaggta taatgcggtt ttaattttat aattatttat tgttttattt atatatttat   1500
gattttttga tgttttttag tttgagttta aattgcgttg ggaataaagt atcgtataaa   1560
ttattagttt gggataagag gagataagtt aaaatttttt aagaagattt tgtagatatt   1620
tggtttttt gagtttgtag agagtttggt attttaaagg ttagttttgg gttttttat   1680
tttgatgatt aatgttaaat tgtttgattt tttataaaaa tatttagttg tgtttattta   1740
tattatttta tttttagcgt atatagtagg tatgtatttg ttgaatagaa gttttaaatg   1800
gatgagaaaa aagatatttt tgttatatat ttttttaaga tcgagaaagg taaaattata   1860
atttaatttg tagaagtttt taggtatttt tttttaatgt aaagttatgt atttttgttt   1920
aaaaaagaaa aggatgatat aattaaataa tgttaattat ataatgggga aattttagga   1980
ttattaagaa aatattatta ttttaagtta tatattttta tttaatattg gttattatat   2040
aggagagagt aagaattgtt gtttttttat ttattcgtat aatttatttg ggaaaagtat   2100
gggtagaata tttatgattt aattatatta tattttggat ttatatttaa tttttttggag   2160
aattagttta aagagtatgt tcgtttagag atataaagta aaatagatat agggataata   2220
gttttggaag aaatttttat gacgttgttt gagattgggt attagtattt aaattttttta   2280
gattattagg agttattttt attacgtaat tttaataaag agaaattgag agtatgattg   2340
gtaaaaatat tatgatgttt ttgtattttg ttaatttgat ttatatttgg ttttatttat   2400
aaagttttta ttgattatta tacggtattt ttatttttta gttttattta ttttgttttt   2460
taaaaatttt ttaacggtat ggttacgaaa aatatgcggg ggttagcggg gtatgtttta   2520
ggttggttta ttttgttttt aaaacgtatt tattttttttt agattttttt ttagggtggg   2580
tagggagaaa acgtttttaa gttgtatagt tgtagggaag tttgtttagg ttatgttttt   2640
ttgatttgtt tttcggttcg ttgtttggta aggttatcgg ttttggttcg cggttttgtc   2700
gataaaattt aatttttatt ttgtaagatt aataggtcgc gtaggatttt tttagtcgtt   2760
ttttgtttcg tattttttttg ggtttgagga ttttcggttc ggttttttttc gcgcgcgcga   2820
ggttttagat ggcgagggcg tagtttttttg gtttatttga ttacgttggg gtgttcgaag   2880
gtttttaggt gttttagtat cgttatttcg cggatggtgg agagcggtat gtttttttcg   2940
tcggtttgta ttcgtacgcg ttttaacgtt acgaaacggt tttcgttttt taagtcgcgg   3000
gttttgaata tttttttata ggcgtttttt tcgattttcg ttacgtattc gtattgttgg   3060
ttagcgcggt ataggtcgtt tttttttatg tcgtttggac gtcgttcgtc gcggcgtcgt   3120
tggggcgggc gggggtgcg tttaattagt tggcggtcgt cgttcgttta tttcggggtt   3180
tttcggagat cggtttagtt ttatttgttt ttcgtcggtt taggcgttcg ggcgttgggg   3240
ttttcgtcgt tgtagaagtt ggatggagag atttttttcgc ggggttggcg taattttggt   3300
gtcgtcgtcg cgaaatttcg tttgtagagt cgtcgtcgtc gtcgtcgtcg gaggagcgag   3360
tcgatttttt tttttttttt ttcgaagcga agtttttaat atagatacga ttatatagtt   3420
tttgtttaag cgcgttttag tttagaatta ttgtatttaa aggaggagac gggaggataa   3480
gaagaaagtg taattagata gtttagaagt tttttcggg gttttttttag atttttttttt   3540
```

```
ttttttttta cgaagttttt atcgttattt ttcgcgcgtt tttgtttttt tttaagtcgt     3600
ttaattttt ttggtttttt cgagaaaagc gaagttattt tttttttttt gtagggttga     3660
agtcgttttc gcgcggagag gttgtagggg tttttcgggg atgagcgagc ggcgcgggac     3720
gtagtggaac gggagggggc gtgtcgagta gtttagagtg tgtcgggagc gcgggggagg     3780
ggaggcgtcg ggtacgttaa tgttacggtt taatatttat ttttatatta ttgtgttgcg     3840
tcgttatttt tttcgttttt tgaggttcgg acgtgtaggg agacggggtt taggggtgtc     3900
ggagggcgtt taggggtcgc gtataagttg gagcggaagg attgtgggtt tattcgtgtg     3960
gggtcgtaga atgtgggtgg gggtttttag gattttgtaa ttcgatgttg ggagtgtgcg     4020
agaaggggtg gttagatatt tgtttagaaa ttgttttttt tttttttatt tttagttttt     4080
ttacgaggag atatttaaaa acgattcgta tatataaatg gtttttttgg ggtaaaggag     4140
tttcggttgg aaacggaatt tttttttcgg ttgggaggat ttttttggtg gaaattttgg     4200
gaagattagt tatttggtcg gggagggttg gggtttatcg ggggtgggcg agcgagcggt     4260
tttgggggag gggagatatc gtttttttacg ggattttaag ggtgggagaa aggggtgttc     4320
gttataattt cgtttgtcgt tttttaaag aataatttta ggaaggggat agtataatcg     4380
cgtttcgttt ttaagtttta aattgatttc gatttgagta aaatttaatt ttttttttaa     4440
agggtggggg gtggggggtt aaattttgcg tttttagggg tgagagagaa ggtttttgtt     4500
ttcggggttc ggattcgcga atttttttttt atttttagga ttttttcgtt gtaggtagta     4560
gaggtggttg ttttattttt ttttcggtta aaggttcggt ttagtttgta gtcggacgtt     4620
ttcggagttt tttgtataat aaagcgttag agtagggtat tttaattttt tttttttatt     4680
tagtagttat ttgggattta ggggtttttt ttttttttagg gtcgtcgcgg tttcggtagg     4740
atttttatta cgacggtcgt atgttcggaa ttttcggggt atttcggaat tatttgtttc     4800
gtaatcgttg gttcgttcgt tttttgttag gaattaaata aacggcgcga tttttacgat     4860
gagcgggtgg agcggatcgc ggcgagagta gagtttttgg tatttattat atttagaatt     4920
tttttttaaaa gtcgaggaaa gagtttttag ggattgttcg ggggtttgag ttaggtcgag     4980
ggttatatag gtagtcgggg agtcgtcgtc ggtcgtttaa ttcgtttgtt ttgtattttt     5040
ttaattttt ttcgttcgag tttagtttcg ggtttcgtag cgaaggaagc gtcggggacg     5100
ttttatttc gtagggaatt gcgtttgttg ttttcgtcgg gtttcgtatt gttttggttt     5160
ttttttttatt cgttttttag acggtcggtt tcggcggaga gaaacgggag tagtaatgtt     5220
tttttttttt ttttttttta ttttttttttg ttgttgttgt tgtttttttta cgttggttga     5280
atgtgatttg attttatttt aaaaaaagtt tgatatagtg ttttaatatt ttcgtatttt     5340
ttcgcgatta taaaggttgt agtcgttttt ttttgttttt tgtttttgtt ttttgttttt     5400
atatttaatg aaattttttta tgcgtttttt tcgaagtttg ttaagtatta taagggtcgt     5460
tattagtata gcgataagag gttatatcgg ggatcgcgat tttcgcgtgt gcgtatacgt     5520
agatgcgttt atatgtatac ggacgggcgc gttgcgggga ttagggttgt ttattgcggg     5580
gcgtgtgttt aattttaata ttttaaatta taaaagtaaa tttggaaatt atagtaaacg     5640
tgcgttttcg cggggtttta agtttgggta ttggtcggtt gcgtgtattt ttttgtgtat     5700
aatacgttcg taggaagttt gcgttaatat ttatttcgtg gaggggaagg tggagtttat     5760
atttatattt tagcgagttg gagagggagt tttcgagggg gaaatgtaag tatattttttt     5820
agtatggagt ttttagaggt agtgtatttt aaattttaaa tgtgaattat tatgtgagtg     5880
ttcgagtaga gttttagttt cgtttggaga aattgtatga ggcgtgtttc gtgtgggtgt     5940
gtgtgcgtac ggatacgtgg aagtaggatt tcggtgtcgc gggtgttttt aggttgggat     6000
tttttttttt tattagataa ggagaatttt ttagtttttt aaaattggtt tttataatta     6060
atttcgtttt agttcgtaag ggttaatta aagtcgattt taaggaaata taggtttttt     6120
ttattttttcg ttatttttta tttattttat ttatagtttt gtttcggatt tcggttaatt     6180
ttttagattt ttttcgggat tttttatttc gtagtttatt tattcgagcg tatagttttt     6240
tatttgaggg gtttagtcgc gtcgggtttt tcgagggggt tgcgagtgtt agtcggtttt     6300
tcgtacgtgt tcgcggtttc gcggagtagg taattagatt ttggggaagg agttattagt     6360
attttttttcg gcgagggggt gggtatagcg gcggagggcg gagggacggc ggagggcgtc     6420
gttcgcgtcg ttcgttgggg gcggacgggg gtcgtt                               6456
```

<210> 79

<211> 6456

<212> DNA

<213> Artificial Sequence

<220>

<223> chemically treated genomic DNA (Homo sapiens)

<400> 79

```
agcggttttc gttcgttttt agcgagcggc gcgggcggcg tttttcgtcg ttttttcgtt      60
tttcgtcgtt gtgtttattt tttcgtcgga gagagtgttg gtaatttttt ttttagagtt     120
tgattatttg tttcgcgagg tcgcggatac gtgcggagag tcgattgata ttcgtagttt     180
```

```
tttcgggagg ttcgacgcga ttgggttttt taggtgagga gttgtgcgtt cgggtgggtg      240
ggttgcgggg tgggggggttt cgggggagat ttgggggatt ggtcggggtt cgaggtagaa      300
ttgtgggtgg ggtaagtgga gggtggcggg gggtgggggg gatttgtgtt tttttggaat      360
cggttttggt taattttta cgaattgaga cgggattggt tatgggggtt agttttgggg      420
agttagagga ttttttttat ttgatgagag ggggagattt tagtttaggg atattcgcga      480
tatcgagatt ttgtttttac gtattcgtac gtatatatat ttatacggaa tacgttttat      540
atagtttttt taaacggaat tggaatttta ttcggatatt tatataataa tttatattta      600
gggtttaaaa tgtattgttt ttagggattt tatgttggag aatgtgtttg tatttttttt      660
tcgaaaattt ttttttagt tcgttgaggt gtgggtgtgg gttttatttt ttttttttacg      720
agataaatgt taacgtaggt tttttgcggg cgtgttatat atagaaaagt gtacgtagtc      780
ggttagtgtt taggtttgga gtttcgcgaa gacgtacgtt tgttgtagtt tttaagttta      840
tttttataat ttaaaatatt ggagttaaat atacgtttcg tagtgagtag ttttggtttt      900
cgtagcgcgt tcgttcgtgt gtatatgggc gtatttgcgt gtgcgtatac gcgggagtcg      960
cggttttcgg tgtggttttt tgtcgttgtg ttggtggcga tttttgtggt gtttggtagg     1020
tttcgggaga ggcgtatgaa ggattttatt gagtgtgaag atagagagta gagatagaaa     1080
gtagaaggag gcggttgtag tttttgtagt cgcggaggaa tgcggaaatg ttgaagtatt     1140
atgttaaatt tttttttgaaa tggggttaag ttatatttag ttagcgtggg aaagtaataa     1200
taataataaa aaaaagtgga ggagaggggg ggaaaaggta ttgttgtttt cgtttttttt     1260
cgtcgaagtc gatcgtttgg ggggcgagtg gggggagagt tagggtagtg cggggttcgg     1320
cgggggtagt aggcgtagtt ttttgcgggg gtgggacgtt ttcgacgttt ttttcgttgc     1380
ggagttcggg gttagattcg agcgggaggg ggttgggggg atgtaggata ggcgggttgg     1440
gcggtcggcg gcggtttttc ggttatttgt gtggttttcg atttggttta gattttcggg     1500
tagtttttgg gggttttttt ttcggttttt aaggaaagtt ttgaatgtag tgagtgttag     1560
aagttttgtt ttcgtcgcgg ttcgttttat tcgtttatcg tgggggtcgc gtcgtttgtt     1620
taatttttgg taaaaggcga gcgggttagc ggttgcgagg taggtaattt cgaggtgttt     1680
cgggaatttc ggatatgcgg tcgtcgtggt gaaagttttg tcgaggtcgc ggcggttttg     1740
gaggagggag gtttttttagg ttttagatag ttgttgaatg gggagggaaa gttagggtat     1800
tttattttgg cgttttgttg tgtagagggt ttcggggggcg ttcggttgta gattggatcg     1860
ggtttttagt cggaggggga atggggtagt tatttttgtt atttatagcg gggaggtttt     1920
gggaatggga aaaggttcgc gagttcgggt ttcgaggata gagattttttt tttttatttt     1980
tggaggcgta ggatttaatt ttttattttt tatttttttga aagggaggtt gagttttgtt     2040
taggtcgaaa ttagtttaaa atttaaggac gaggcgcgat tatgttattt ttttttttgaa     2100
gttatttttt aaggagacga taagcgaaat tgtggcgaat attttttttt tttattttttg     2160
ggatttcgtg ggggacggtg tttttttttt tttaggatcg ttcgttcgtt tattttcgat     2220
aagttttaat tttttttcgat tagatggttg gtttttttaa ggtttttatt agggaggttt     2280
ttttagtcgg agagagaatt tcgtttttaa tcgagatttt tttattttaa aaagattatt     2340
tgtgtatgcg agtcgttttt aaatgttttt tcgtagaaaa attgaaagta gaaagaaagg     2400
aagtaatttt tgagtagatg tttgattatt tttttttcgta tatttttagt atcgggttat     2460
agggttttgg gagtttttat ttatattttg cggttttata cggatggatt tatagttttt     2520
tcgttttagt ttgtgcgcga tttttgaacg tttttcggta tttttggatt tcgttttttt     2580
gtacgttcgg gttttaggag gcggggaggg tagcggcgta atataatgat atagggataa     2640
atattaagtc gtgattattga cgtgttcggc gtttttttttt tttcgcgttt tcggtatatt     2700
ttgggttgtt cggtacgttt tttttcgttt tattgcgttt cgcgtcgttc gtttattttc     2760
gaggggtttt tgtaattttt tcgcgcgaag acggttttag ttttgtaggg aaagaaaagt     2820
aatttcgttt ttttcggagg aattaggaag gattaagcgg tttgggagag ggtaggagcg     2880
cgcggagggt agcgatggag gtttcgtaaa ggaggaggag gggagtttgg aggaatttcg     2940
aggaaggttt ttgggttgtt tgattgtatt ttttttttat tttttcgttt tttttttttag     3000
gtgtaatgat tttggattga gacgcgtttg ggtagaggtt atgtaatcgt gtttgtgttg     3060
aggatttcgt ttcgaggagg gaagaggagg gatcggttcg ttttttcggc ggcggcggcg     3120
gcggcgattt tgtaggcgga gtttcgcggc ggcggtatta gggttacgtt agtttcgcgg     3180
ggaggttttt ttatttagtt tttgtagcgg cgaaagtttt agcgttcgag cgtttgagtc     3240
ggcggggagt aagtaaagtt agatcgattt tcggggagtt tcggagtagg cgagcggcgg     3300
tcgttagtta gttgagcgta tttttcgttc gttttagcgg cgtcgcggcg ggcggcgttt     3360
aggcggtatg gagaaggacg gtttgtgtcg cgttgattag tagtacgaat gcgtggcgga     3420
gatcggggag ggcgtttatg ggaaggtgtt taaggttcgc gatttgaaga acggaggtcg     3480
tttcgtggcg ttgaagcgcg tgcgggtgta gatcggcgag gagggtatgt cgtttttttat     3540
tattcgcgag gtggcggtgt tgaggtattt ggagattttc gagtatttta acgtggttag     3600
gtgagttagg gagttgcgtt ttcgttattt ggggtttcgc gcgcgcgggg agggtcgagt     3660
cgggaatttt taggtttaga aaggtgcggg gtagagggcg attggggagg ttttgcgcga     3720
tttgttggtt ttatagagtg agagttaagt tttgtcgata gagtcgcgag ttagagtcgg     3780
tgattttgtt agatagcgag tcgaaagata agttaaaaaa atatgatttg agtaggtttt     3840
tttgtagttg tgtaatttag aagcgttttt tttttgttta ttttgaagga gggtttgggg     3900
gaagtgggtg cgttttaggg ataggataag ttaatttgag atatgtttcg ttagttttcg     3960
```

```
tatatttttc gtggttatgt cgttggggaa tttttgaaag atagagtagg tgagattgga    4020
gaatgaggat gtcgtgtgat aattagtggg aattttatgg gtggagttaa gtgtaaatta    4080
ggttaataag atgtagaaat attataatat ttttgttagt tatattttta gttttttttt    4140
attaaagttg cgtagtaaga atgattttg gtggtttaag gagtttgagt gttgatgttt    4200
aattttaaat agcgttataa aaattttttt taaaattgtt atttttgtgt ttgttttatt    4260
ttatattttt gggcggatat atttttaga ttaattttt aaagggttaa atgtgagttt    4320
aagatgtggt atggttagat tataaatgtt ttgtttatgt ttttttaga tggattgtgc    4380
gagtgggtgg ggagatagta gtttttattt tttttgtgt ggtaattagt attaaatgaa    4440
aatgtgtgat ttaggataat ggtgtttttt tagtgatttt ggaatttttt tattgtgtgg    4500
ttgatattgt ttggttgtgt tattttttt tttttaaat agagatgtat agttttatat    4560
tgaaaaggaa tgtttagaaa tttttgtaaa ttgggttgtg attttatttt tttcgatttt    4620
gagaaaatgt gtagtagaaa tattttttt tttatttatt tagggttttt gtttaataaa    4680
tatatgttta ttgtgtgcgt tgaggatgaa atgatgtaaa taagtatagt taaatatttt    4740
tgtaaaaagt taaataattt aatattagtt attaaagtgg agagatttaa gattgatttt    4800
tgaaatatta aatttttat agatttagag agattaaata tttatagaat tttttggga    4860
ggttttaatt tgttttttt tattttaggt tggtgatttg tgcggtgttt tgtttttaac    4920
gtagtttaga tttaagttag aaggtattaa agaattataa atatataaat aaggtagtaa    4980
atggttataa agttaagatc gtattatatt tcggtttagt tagtgaagga gaatgagtgt    5040
tttagaagat gagattatat ataaaaattt atagatgatt tttttatg tgtattttat    5100
ttgttaaaaa taataaaatt cgtttttggg gtttttgtg atattggagg gaaaaggga    5160
gggtatattt agtttttgt aatttagttg tcgttttagg ggaattattt aatatatttc    5220
gaaggatgaa ttttattatt ataattttaa gacggaagtt tttgttttat tttgtttgtt    5280
atttatggag tatttagtta agggtgttta ttatgtatat gaatgaatag tgttttttta    5340
aacgtagagg tattatagag gtatttttgt ataatattat tgtttataaa gtttacgtta    5400
tttttatatt agaggtatat tttttgttat tgtgtttgta gattttgttt gatttatttt    5460
attttttga tttatttttt tgagattgat gtgtttttag acgtttttaa ttgatttatg    5520
atttggattt tgtattgtga attttaggag gatattttta tgaattgaag ataaaggtat    5580
tgtttttaa aattattttt acgtgttaag gtgatgatgg tatttatttg ttaaagtcgt    5640
gagaataaaa tgagatggcg tatgtgaaag tattttgtaa atttgaaagt attttagata    5700
tttaaaggat tgtttgcgtt ttagtagatt gatggttttt tgttatggag tagttttata    5760
gggaggggtt aatttatttg atttttttg tagaataaat tatatttaaa tttagatatt    5820
ataggttttg gaattaagaa gatttttgag gataattagt ttgtaagatg ttagaaaaga    5880
tttttagtta cgagttatga aatttagtga tatttgagga aattattgat gatgttattt    5940
ttatgagtta tagattttta tttttttttt gtttttttt tgtttttttt tttttgtttt    6000
tgttttgaaa taattattgg tttttatgat ttgtttggtt agattaagaa ttttggattt    6060
tagaggttgc gaatatttgt ttagataggg ttaggaaata aggatagttg atagtaattt    6120
tttttttttt aagggagagt tgttaaagtt gggtttttaa atttgtgatt tattattgtt    6180
tttgggatta gttttatagt tttaagtttg gtttttaat ggtttttaa aagggtagat    6240
gtttttataa gtattttaa gttataagta attgattata attataggtt tttttttata    6300
gatagttaag tttttaagtt aataggagta taataaatat attagttttt ttaaaagttt    6360
aagatttaat tttgatggat ttgttagaaa agttgatttg ttattgttgg tattggtatt    6420
gtttgtatgt atagggataa tgatttgaaa tttttg                            6456
```

<210> 80
<211> 6257
<212> DNA
<213> Artificial Sequence

<220>
<223> chemically treated genomic DNA (Homo sapiens)

<400> 80

```
ttatagtagt ttttttgatt ggattaattt tttcggatat aatagtaggg taggggtggg      60
gtttggggag aaaggatatt tttaattttg attttaatat ttcgatggtt tttaaggttg     120
tttgtatatt tatttaggtg taagtttttt aaggtgtggt gtgatgaatt agtgattttt     180
ggagttaggt tagcgtattt ttttttcgta gggttgtaag ttgtaggatt gagaggtagg     240
ttgattaggt tttgggttgg atgatggggt gagagtaagg ggttagtttt gatatatgtt     300
taattttttt ttttagtttt aagatatttt gggtaaattg tttattttag tttttttgat     360
ttttatttta attttaatat tagtttaaga gaaaataggg atattgatgg ttatttagaa     420
gggttgttgt gttttatata tagtaatatt tttcgaatgt ttgtgatagc ggtttaagga     480
ataagttaat tttatattat tattttggat atttgtataa aattttattt tatttttatt     540
atatgaatgt gttagggttg tttttttgtt ttgttttttt tttttttttt tgagatagag     600
```

```
tttcgttttt gttgtttagg ttggagtata atggcgcgat tttggtttat cgtaattttc      660
gttttttagg tttaagcgat ttatttgttt tagtttttc gagtagttgg gattataggt      720
atgcgttatt atgttcggtt aattttgtgt ttttagtaga gatagggttt ttttatgttg      780
gttaggttgg tattaaattt tcgattttag gtgatttatt tgttttggtt ttttaaagtg      840
ttgtaattat aggtatgagt tatcgtattt agtcgtgtta gggtttttt ttgtttaatt      900
ttttttttt ttttgttttt ttttttttt ttaatggagt tttattttgt tatttaggtt      960
ggagtgtagt ggtaagattt tagtttattg taatttttgt tttttgagtt taagtaattt     1020
ttttgtttta gtttttcgag tagttgggat tataggtgtt tgttattata tttagttaat     1080
ttttgtattt ttagtagaga tggggttttg ttatgttggt taggttggtt tcgaattttt     1140
gatttcgtga tttgtttgtt ttggtttttt aaagtgttgg gattataggt atgagtcgtt     1200
atattcggtt aattttgtat tattttttta aagagagttt tttaaattat ataagtttta     1260
ggttttataa aatttagatt tgttttagta taattaaatt tgggattatt tattgagtaa     1320
ttattatgtg ttaagtattg cgttgagtgt ttttagagta ttattttttt taattttagt     1380
atagtatgtt agatgttgtt ttatagatga gttaattgag attagagatg tttagttatt     1440
tgtttaaggt gatatgattg atatggaata gagttaagat ttttttttt tttttgata      1500
cggagtttta ttttgttttt taggttggag tgtagaggcg taattttagt ttattgtaag     1560
ttttgttttt taggtttacg ttattttttt gttttagttt tttgagtagt tgggattata     1620
ggtattcgtt attatatttg gttaattttt tgtattttta gtagagatag ggttttatcg     1680
tgttagttag gatggtttcg atttttgat ttcgtgattt gtttgtttcg gttttttaaa      1740
gtgttggaat tataggtgtg agttatcgcg attggttaga tttaagattt gaatttaggt     1800
ttttttggtt ttagaggttt ttgtttttta atttttagg atggtatagt aatttgtttt      1860
ataagaggtg tttgttttaa gtgtgtttag tatatggaag taagtttaga aatgtaagtg     1920
tatatttgta aagaggtgtg ggagatgggg gggagggaag agagaaagag atgttggtgt     1980
tttttatttt ttagtttttg ataggtgttt ttgattttt tttgattagt atagttgtat      2040
ttttggttgg ggtattttaa ttagaattgt taaatttagt atataaaaat aaggaggttt     2100
agttaaattt gaattttaga taaataatga ataatttgtt agtataaata tgttttatgt     2160
aatattttgt tgaaattaaa aaaaaaaaaa aaagtttttt ttttattttt atttttatta     2220
ttaggtttaa ggaatagggt taggggtttt aaatagaatg tggttgagaa gtggaattaa     2280
gtaggttaat agaaggtaag gggtaaagaa gaaattttga atgtattggg tgttgggtgt     2340
tttttaaat aagtaagaag ggtgtatttt gaagaattga gatagaagtt tttttgggtt      2400
gggtgtagtt gttcgtggtt gtaattttag tattttggga ggttgaggcg ggaggattat     2460
ttgaggttgg gagtttaaga ttagttttat taacgtggag aaattttgtt tttattaaaa     2520
atataaaaaa ttagttggtt atggtggtat atgtttgtaa ttttagttgt tcgggaggtt     2580
gaggtaggag aattatttga attagggagg tagaggttgt ggtgagtaga gatcgcgtta     2640
ttgtttttta gtttgggtaa taagagtaaa agttcgttta aaaaaaaaaa aaagtttttt     2700
cgatgtgatt gtttttttt aaatttgtag attttttaa gattatgttt tttagatatt       2760
ttaaagattt tagaagatat gtttcggggg ttttggaagt tataaggtaa atataatata     2820
ttttttttt tgattattaa ttttattaga ggatgtggtg ggaaaattat tatttgatat      2880
taaaataaat aggtttggga tggagtagga tgtaagtttt ttaggaaagt ttaagataaa     2940
atttgagatt taaaagggtg ttaagagtgg tagtttaggg aatttatttc ggatttcggg     3000
ggagggggta gagttattag tttttgtatt tagggatttt tcgaggaaaa gtgtgagaac     3060
ggttgtaggt aatttaggcg tttcggcgtt aggagggacg tatttaggtt tgcgcgaaga     3120
gagggagaaa gtgaagttgg gagttgttat ttttagattt gttggaatgt agttggaggg     3180
ggcgagttgg gagcgcgttt gtttttaatt ataggagaag gaggaggtgg aggaggaggg     3240
ttgtttgagg aagtataaga atgaagttgt gaagttgaga ttttttttta ttgggatcgg     3300
agaaattagg ggagttttc gggtagtcgc gcgtttttt ttacggggtt ttttattgcg       3360
tcgcgcgttc ggtttttatt tttcgtagta tttcgcgttt cgcgtttttt tagtcgggtt     3420
tagtcggagt tatggggtcg gagtcgtagt gagtattatg gagttggcgg ttttgtgtcg     3480
ttgggggttt tttttcgttt ttttgttttt cggagtcgcg agtatttaag gtgggtttgg     3540
tgtggggagg ggacggagta gcggcgggat tttgttttgt ggatgtttcg tcgaggtttc     3600
gcggtcggcg gggttagagg ggttcggacg agttttttta tttcgaagtt gtggatagtc     3660
gagacgttta gggtagtcgg gttttggggt tttcgggcgg gagggggtag ttatacggta     3720
gcggttcgag atggtttatt taagagattg gcgtttttta ggtttcgagg ggtttcggga     3780
atttgttaaa gaagtttttt gaaattgttt agaaagtttt ttcgtaaagg gtgtattgcg     3840
tagagcgcgc gcgcgcgttt ttttttttt tgagtttttt taagtttttt taaagttttt      3900
ttagttggta gttttcgttt tcggattggt ttgggttgga ttttttgggg gggttttttg     3960
ttttgttttt tttttagttt tttttcgttt ttttttagac gattttggtt tggttgtttt     4020
tgtttttggc ggggtcgggt gtgtgtgtgt gtggtggagt ggagggtggt atagtaattt     4080
gttttaatta gagtcgggga ggaaagggtg gttcggaggg tggtttttttg ttggggtttg    4140
ggttgggggc gggggagacg tttgttttga atagattttt ggggttagtt tagggattgt     4200
gttttgtgat ttttggagcg cgtggattat ggaggggtgg gggtgggttt tttggggtgt     4260
aaagtgggag agtttttaga gaaggaagtt aagaaataag gttagatggg agtttaggga     4320
gggttgcgtt gttttgttgt ttttttttg gtgttgtgcg tggggaaggg tgagtggggg      4380
```

```
tagtgtgtat tttgatttat ttgtttattt gtgtgtatta attataaaag ttaatatata    4440
gtttgggtta ggtatatttt gttaggaatt gtttgtggtg ttttgtatgt attttttttt    4500
aattttagaa tatttttata gtggaagttt tgttagtatt ttggattgag tagtagttta    4560
gaggttgagt agtagttagt aagtggtggg gttaagatgg gattttaggt agtgcgattt    4620
ttaattatgt attcgaaatc gttatatgga tgagtgtatt tggagtaatg agggatattg    4680
tttttttgagt tattgggttg taggggagat aaaatgaaag tgttttggga gtcgtgggtg    4740
gtttttatag gttagagggt ttggggaggg agtgggtgtt atcgtggttg tgtgttgttc    4800
gaggggtttt ttgtgagtga gtgtatggtc gtgttatttt tgtaggttta cgttagggtg    4860
ttttttagtt gtgtggtttt tgtatttgtg tgtttgggtt ttgtgttgtt aaatagtagt    4920
ttttttgttg atttggggat ataggttgaa ttttgttttt tgtaggaatt tttttaaggt    4980
gttgggttag atttgttata aatagaggga ggtagttttt tatggttacg tttttttgtt    5040
gaggaagaag gttttttttt tttagggagt atatttttgt ttttttttgtt ttttagataa    5100
gtatttttat tttttatttt ttgatgagaa gggtgaggtt atattgagtt gttaggttga    5160
gttgttgttt tttttatttt tgggttggga gttgattagg gaatggtagt tgttgtagag    5220
ttggatttga gggttgggtt ttttggatgg ggttttttta tgtttttatt ttttaatttg    5280
tattattgat tgtgttgtgt aggagttagt taaaaagtta ttgtatagtt tgggtaataa    5340
ggtaaaattt tgtataaaaa atataaaaat tagttggatg tgattatacg tgtttgtagt    5400
tttagttatt tcggaggttg aggtaggagg attatttgag tttaggaagt tgaggtttgt    5460
agtgagttgt gattgtaaat gtttttttagt ttgggtgata gtgtgagatt tcgttttaga    5520
aaaaaagtat attatttagt tgtttttagt atttagattt tatttaaggg gtgaggtttg    5580
gggtaggaat gtgggggaag gggaggttta gggggagttt tagaggggtt aggatttttt    5640
tgaaattttt ttttagaggt atgggtttta taaattgtag taaatatatt tttttaattt    5700
tgtagaattt ttttatattt taattttagt atgatttttt taatagtttt ttttttttat    5760
tatatttggg gaaagtattt attttatttg ttaagaaaaa aataattgaa aagataggga    5820
ttaaatgtaa aaagaaaaaa tacgtggtat tttaaagtta aatataaagt atgtttaatt    5880
ttttcgtggt ttgggattat ttatattttt gttgtatgaa tttgttttgt tttaattttt    5940
aagaaatgta cggtgtattt tttatatgtt aggtttttat ttatgttttt atttaatttt    6000
tgtgatagtt ttgtgaagta ggtgtataga tgagaaaatg gaagtttaga gaaatgaagt    6060
aatttattta aggtttttag ttatttagta atgtttaggg aatttttgga ttttgaagag    6120
gaggtattaa gaggtggtta gagttttatt ttagttaata ataatgggtt gaataaagtt    6180
ttaggggtag gtaggtggtt agatgggagg agaagcgttt tttttgttta ggcgaatgat    6240
tttttatttt attttt                                                    6257
```

<210> 81
<211> 6257
<212> DNA
<213> Artificial Sequence

<220>
<223> chemically treated genomic DNA (Homo sapiens)

<400> 81

```
gagaagtgga tggaaaggtt attcgtttga ataagaggag cgtttttttt tttatttggt   60
tatttgtttg tttttaaggt tttgtttaat ttattattgt tggttggaat aagattttaa  120
ttatttttta atgttttttt tttagagttt aaaaattttt tggatattat tgggtagttg  180
ggagttttgg ataagttgtt ttattttttt gaatttttat tttttttattt gtgtatttat  240
tttataggat tgttatagag attaaatgaa agtatggata aaaatttagt atataggaag  300
tatatcgtat atttttaaa agttaagata agataggttt atatagtagg aatatgggta  360
attttaaatt acgagaaaat taaatatgtt ttgtgtttga ttttggaatg ttacgtattt  420
ttttttttta tatttgattt ttattttttt aattgttttt tttttgataa ataaaatgag  480
tatttttttt aagtatagta aagagaggag gttgttggaa gaattatatt ggaattaaaa  540
tatgaaggag ttttgtaaga ttaaaaggat gtatttgttg taatttgtaa aatttatatt  600
tttaagaaag gattttagaa aaattttgat ttttttgggg ttttttttag gttttttttt  660
tttttatatt tttgttttag attttatttt ttgggtaaaa tttgggtgtt ggaggtagtt  720
gggtggtata tttttttttt gaaacggagt tttatattgt tatttaggtt ggagagtatt  780
tgtaattata gtttattgta agttttaatt ttttgggttt aggtgatttt tttgttttag  840
ttttcggagt agttgggatt ataggtacgt gtaattatat ttaattaatt tttgtatttt  900
ttgtatagag ttttgttttg ttgtttaggt tgtgtaatga ttttttaatt aatttttgta  960
taatataatt aatagtgtag gttgaggggt gaggatatga ggaggtttta tttagagaat 1020
ttagttttta aatttagttt tgtagtaatt gttatttttt gattaatttt tagtttaagg 1080
tgaggaaggg tagtagttta gtttgatagt ttagtatggt tttatttttt ttattagaag 1140
atgagaggtg aagatgtttg tttgggaaat agggagggta aggatgtatt ttttggaaga 1200
```

```
gaggaatttt ttttttagt aagaaggcgt ggttatagaa ggttattttt ttttgtttat     1260
ggtagatttg gtttagtatt ttaagggagt ttttgtagag gatagagttt agtttgtgtt     1320
tttaagttag tagagagatt gttgtttggt aatataaagt ttagatatat aaatataaag     1380
attatataat tgaggaatat tttggcgtag atttgtagag ataatacggt tatgtattta     1440
tttatagagg gtttttcggg taatatatag ttacgatgat atttattttt tttttagatt     1500
ttttgattta tggagattat ttacgatttt tagaatattt ttattttgtt ttttttgtag     1560
tttagtgatt tagggagtag tgttttttat tgttttaggt gtatttattt atatagcgat     1620
ttcgaatgta tggttggggg tcgtattgtt tggggtttta ttttgatttt attatttatt     1680
agttgttatt taatttttgg attgttattt agtttagaat gttggtagaa tttttattat     1740
aggggtgttt taggattaaa ggagaatgta tgtaaaatat tataaatagt ttttggtaga     1800
gtatatttgg tttaggttat atgttagttt ttatgattaa tgtatatagg tggatagatg     1860
ggttaggata tatattgttt ttatttattt ttttttacgt atagtattaa ggaaaaggta     1920
gtagaataac gtagtttttt ttaggttttt atttggtttt atttttttagt ttttttttt     1980
gggaattttt ttattttata ttttaagaaa tttattttta tttttttatg gtttacgcgt     2040
tttaaaagtt atagagtata gtttttaagt tggttttaag aatttgttta aagtaaacgt     2100
ttttttcgtt tttaatttag attttagtaa gaggttattt ttcgggttat ttttttttt     2160
tcggttttgg ttgggatagg ttgttatgtt attttttatt ttattatata tatatatatt     2220
cgatttcgtt agaagtagga gtaattaaat taaaatcgtt tgaaggggag cggggagggg     2280
ttggaggagg ggtagggtag aggatttttt taaggaattt agtttaggtt agttcggagg     2340
cggaggttgt taattggaaa ggttttgaga aagtttgagg gggtttaaga agggggggaaa     2400
cgcgcgcgcg cgttttacgt aatatatttt ttgcgggaaa atttttttgaa taattttaga     2460
gaattttttt gataagtttt cggagttttt cggagtttgg aaagcgttag tttttttggat     2520
gggttatttc gagtcgttgt cgtgtaattg tttttttttcg ttcgagggtt ttagggttcg     2580
gttgttttga gcgtttcgat tgtttataat ttcgggatag gagagttcgt tcgggttttt     2640
ttggtttcgt cggtcgcggg atttcggcgg ggtatttata gggtagggtt tcgtcgttgt     2700
ttcgtttttt ttttatatta gatttatttt gggtgttcgc ggtttcgggg ggtaagaggg     2760
cgaggaggag tttttagcgg tataaggtcg ttagttttat ggtgtttatt gcggtttcgg     2820
ttttatggtt tcggttggat tcggttggga gggcgcgggg cgcggggtgt tgcgaggggt     2880
gggggtcggg cgcgcggcgt agtaaagggt ttcgtgggaa ggggcgcgcg gttgttcggg     2940
gggttttttt ggtttttttcg gttttaatgg aggggaattt tagttttata attttatttt     3000
tatattttttt taagtagttt ttttttttta ttttttttt tttttgtgat tgggagtaag     3060
cgcgttttta gttcgttttt tttaattgta ttttaataag tttgggagtg gtaattttta     3120
gtttatttt ttttttttt tcgcgtaggt ttgggtgcgt ttttttttagc gtcgggacgt     3180
ttgggttgtt tgtagtcgtt tttatatttt ttttcggaga atttttaaat gtagaggttg     3240
gtgattttgt tttttttttc ggagttcggg ataaattttt taggttgtta tttttaatat     3300
tttttttaagt tttaggtttt atttttaaatt tttttgggga gtttgtattt tattttattt     3360
taagtttatt tgttttaata ttaaataatg gttttttttat tatattttttt agtaaaattg     3420
atagttaagg agggggatgt gttgtgttta ttttgtggtt tttaggattt tcggggtata     3480
tttttttggaa tttttgaagt atttgaaaag tatgatttta agagggttta taaatttggg     3540
aggagatagt tatatcgaaa ggattttttt tttttttttaa acgaattttt gtttttgttg     3600
tttaggttgg agagtaatgg cgcgattttt gtttattata atttttgttt ttttggttta     3660
agtgattttt ttgttttagt ttttcgagta gttgggatta taggtatgtg ttattatgat     3720
tagttaattt tttgtatttt tagtaaagat agggtttttt tacgttggtg aggttggttt     3780
tgaattttta attttaggtg atttttttcgt tttagttttt taaagtgttg gaattataat     3840
tacgagtaat tgtatttagt ttaaaaagat ttttatttta atttttttaaa atgtattttt     3900
tttgtttatt taaggaggta tttagtattt aatgtattta aggtttttttt tttgtttttt     3960
gtttttttatt agtttgttta attttatttt ttaattatat tttatttgga gtttttgatt     4020
ttattttttta ggtttagtgg taggggtggg gatggaagga agattttttt ttttttttt     4080
aatttaata agatattgta tgggatatat ttatattaat aaattattta ttgtttattt     4140
gaaatttaaa tttaattggg ttttttttatt tttatgtgtt aaatttggta gtttttagttg     4200
gaatgtttta gttaagaatg tagttatatt ggttaagaag ggattaaagg tatttattag     4260
ggattggaga atgaaggata ttagtatttt ttttttttt ttttttttt tatttttttat     4320
attttttttat aggtatatat ttgtattttt aaatttgttt ttatgtgttg agtatattta     4380
aagtaggtat tttttgtggg ataggttgtt atgttatttt agggagttga gaaatagggg     4440
tttttgggat taagaggatt tgggtttaaa ttttgaattt ggttagtcgc ggtggtttat     4500
atttgtaatt ttagtatttt gggaggtcga ggtaggtaga ttacgaggtt aggagatcga     4560
gattattttg gttaatacgg tgaaattttg tttttgttaa aaatataaaa aattagttag     4620
gtgtggtggc gggtgtttgt agttttagtt atttaggagg ttgaggtagg agaatggcgt     4680
gaatttggga ggtagagttt gtagtgagtt gagattgcgt ttttgtattt tagtttggga     4740
gatagagtga gatttcgtgt taaaaaaaaa aaaaaaaatt ttgattttat tttatattag     4800
ttatgttatt ttgggtaagt gattgagtat ttttggtttt agttggttta tttgtaaaat     4860
agtatttgat atattatgtt ggggttaaag gagataatgt tttggaagta tttagcgtaa     4920
tatttggtat ataataattg tttaataaat ggttttagat ttagttatat tggggtagat     4980
```

```
ttaggttttg tggggtttga agtttatata atttgggaaa ttttttttaa gaaagtaata     5040
taaagttggt cgagtatggc ggtttatgtt tgtaatttta gtattttggg aggttaagat     5100
aggtagatta cgaggttagg agttcgagat tagtttggtt aatatgataa aattttattt     5160
ttattaaaag tataaaaatt aattaggtgt ggtggtaggt atttgtaatt ttagttattc     5220
gggaggttga ggtaggagaa ttgtttgaat ttagagggta gaggttgtag tgagttgaga     5280
ttttgttatt gtattttagt ttgggtgata gagtaagatt ttattgaaag aaagaaagag     5340
agtaagagag agaaaggaat tgaatagaaa aagattttag tacggttggg tgcggtggtt     5400
tatgtttgta attgtagtat tttgggaggt taaggtaggt ggattatttg aggtcgggag     5460
tttggtatta gtttgattaa tatggagaaa ttttgttttt attaaaaata taaaattagt     5520
cgggtatggt ggcgtatgtt tgtaatttta gttattcggg aaggttgagg taggtgaatc     5580
gtttgaattt gggaagcgga ggttgcggtg agttaagatc gcgttattgt attttagttt     5640
gggtaataag agcgaaattt tgttttaaaa aaaaaaaaaa aaaataaaat aaaaaaataa     5700
ttttagtata tttatatagt aaggataagg tggagttttg tataggtatt tagagtgata     5760
atgtaaaatt aatttatttt ttggatcgtt gttataaata ttcgagaaat attgttgtgt     5820
atggaatata gtagtttttt tggatggtta ttaatatttt tatttttttt tgagttggtg     5880
ttagggttag ggtgaggatt aggggaattg aggtaagtaa tttgtttagg atgttttagg     5940
gttagagaga aaagttgggt atgtattaaa attgattttt tatttttatt ttattattta     6000
gtttaggatt tggttaattt gtttttttagt tttgtagttt atagttttgc gggaagagga     6060
tgcgttgatt tggttttagg agttattggt ttattatatt atattttgga gggtttgtat     6120
ttggatgagt gtgtaggtag ttttagaggt tatcgggatg ttaggattag ggttagaggt     6180
gtttttttttt tttaggtttt atttttgttt tgttgttgtg ttcggaaagg ttagtttagt     6240
taggaaagtt attgtgg                                                   6257
```

<210> 82
<211> 8467
<212> DNA
<213> Artificial Sequence

<220>
<223> chemically treated genomic DNA (Homo sapiens)

<400> 82

```
atgaattaaa ttttttttg gtattaagaa gggatatata gtatttcgag ttttgttttt      60
gttttttat taagtttttt ttaatagttg gaagtggttg aagggggttg ttttaaggag     120
atttttgtt tttttttga tagttgatta aaagaaaata ggttacgttt tttaaattta     180
tgtttatttt gtttttaatt attgtttttt taaataaagg taatatttgc gtaattttag     240
ttagtttgaa ttttcggagg taaatagagc gtaatttgtt ttggaagaat tttgttacgt     300
ttaaggtttt atgtcgggtg ttggtttttt tgtttttaat tatttttta tttcgattcg      360
gattgtagaa atttttaatt ttttgttttt tagttttatt tttattttta gatttggggt     420
tgttaaataa cggaaatttt aggaagacgt aggtgcggtt tttaaagttt tggtttgtga     480
gcgtttttag gttggtcggg gcgttggttt ttttagaaat aaggtttgaa tatgtaggtg     540
ttagttagga ttttttgtcg tttgtttatc gtcgtttttt tttcgggatt tgagagaagc     600
gggagtgtgt gtgtgtgtgt gtgtgtgtgt gtgtgcgcgt gtgtgtgttt cgtgttagga     660
gaaaggttcg tggttgtcgt tttagttggt ttgtttattt tttgttttta ggagaagttt     720
gtttatttgg gatattagcg attttttta tttttatttt atcggtttta tattttttt      780
atttttttt aagtcgttga tgtagaaagt atttggttat tttgaaacgg tagttttcgg     840
aattttagtt ttgttttgta atttagtagg gtttttcgga agttttataa ttgagattta     900
tgggtttatc gggtaattaa atgatgttat tgtgttaatt ttgtatgtat tattgttgtt     960
cgcgtcgttc gtgggtcgtc ggtcggtcgt agttcgttgg cgacgagggt attatagttg    1020
ttttgatcgc gtagattatg tatgtttcgg tttcgggaat ttattatgta ttagaatata    1080
ttagcgtttg tattttaaaa ggttaaatta ttggttttta gttagggatt ttcggtaagt    1140
ggtttgttag tgacgagtgt ttgtttatat tggtatatag cggagttttt tgttttcggt    1200
ttattcgttt ttaggaaagt tttggggtga ggcgaaggcg attgaagtaa tgtttttttt    1260
tttagatcgt agttgtttag gggggatata gtacggtatt ttttatcgaa tttttttcgt    1320
tcgttcgtat tttagttttt ttttttttag cgattttttt attttttttt tttttttttt    1380
ttttgacgtt tgattttagt agtaaaggag gtaaaaaagg tatcgagtcg ttagttaaat    1440
ttgaaaagtg cggtttcgtt ttttttatag ttattggtag tttttcgtgg aaggttcgtt    1500
tttcggggta gttgcggttt cggagtggtt gcgtttggcg ttcgtcgggc gtggtttcgt    1560
tttaggttcg ggagggtagg ttggttgttt cggcgagcgg tagagttttt ttggatagtt    1620
ttcgtttatt taaatagaag acgtcggcgt cggagcgggt tcggatatgg cgaggttgcg    1680
agtcggttcg agcggcgggg ttcggtgatt tttttttttt ttttcgtttt tttttttttt    1740
tcgtacgtac gtttcgttcg tttttatttc gtttttattt cgggcgagtt cgttcgtagt    1800
```

tcggggcgta tattcgtacg cgtatttttt tttatttatt ttcgcgttcg tttttatttt 1860
cgtagtcgag tttcgttacg cgcgttttgt tcgttcgtcg gtcgttttcg tcgttttcgt 1920
cgttttcggg ttttgatgga ttgaatgaag gttgtttata tcgtttatcg atgttttatt 1980
aaagatttag aaggttgcgt tatgaattcg gagttgataa tggaaagttt gggtattttg 2040
tacgggtcgg tcggcggcgg tagtggcggg ggcggcggcg ggggcggcgg gggcggcggc 2100
gggggttcgg gttatgagta ggagttgttg gttagtttta gttttttatta cgcgggtcgc 2160
ggcgtcgttg gttcgttgcg gggtttttcg tcgtttttaa tcgcgtatta ggagttgggt 2220
acggcggtag cggcggtagc ggcggcgtcg cgttcggtta tggttattag tatggtttcg 2280
atttggacg gcggcgatta tcggttcgag ttttttattt cgttgtatta cgttatgagt 2340
atgttttgcg attcgttttc gtttggtatg ggtatgagta atatttatat tacgttgata 2400
tcgttttagt cgttgttatt tatttttatc gtgtttgata agttttatta tttttattcg 2460
tattattatt cgtattatta ttattattat tattattagc gtttgttcgg taacgttagc 2520
ggtagtttta tttttatgcg cgacgagcgc gggttttcgg ttatgaataa tttttatagt 2580
ttttataagg agatgttcgg tatgagttag agtttgtttt cgttggtcgt tacgtcgttg 2640
ggtaacgggt taggcggttt ttataacgcg tagtagagtt tgtttaatta cggttcgtcg 2700
ggttacgata aaatgtttag ttttaatttc gacgcgtatt atattgttat gttgattcgc 2760
ggtgagtaat atttgtttcg cggtttgggt attttatttg cggttatgat gtcgtatttg 2820
aacggtttgt attattcggg ttatatttag ttttacgggt cggtgttggt atttagtcgc 2880
gagcggttat tttcgttttt atcgggttcg taggtggtta cgtcgggtta gttggaagaa 2940
attaatatta aagaggtggt ttagcgtatt atagcggagt tgaagcgtta tagtattttt 3000
taggcgattt ttgcgtagag ggtgttgtgt cggttttagg ggattttttt cgatttgttt 3060
cggaatttaa aatcgtggag taaatttaaa tttggtaggg agattttttcg taggatgtgg 3120
aagtggtttt aggagttcga gtttagcgt atgttcgttt tacgtttggt aggtaaggtc 3180
ggggttagtt aggggttagg ttgttgggaa gagggtttcg ggttcggtgt ttgtggttta 3240
agtttgcgcg tcgagttatt tttttgatt ttttttttt ttttttata tacgtttttt 3300
ttttttcgt ttttattttt tttttattt ttttttttt tttcgtttt tttttatttt 3360
ttttttttt ttttttttt ttttttatt tttttttgt ttttgagttt ttattgatcg 3420
atttttttt atttattcg ttttttttt aatgtgttaa tttttgtttt attttcgatt 3480
tttttaggta ttgggaggcg ggatgggggt gtgcgttttt ttttaggagt tttgtttttt 3540
taagatttat agaaattagg atttgttttt atttaaaatt ttatgtattt taagtttttt 3600
ttagataata tattttaatt tttcgggttg attagttttt ttgtgtagag gtagttgaga 3660
ggttttgttt tgtagaggga aaagagtttt ttattttttt atttattata taggtaaatt 3720
tatttggtta ttggttgaag gtatagtttt gtttcgcgg ggaatcggcg gttaggatat 3780
aatagcgttt ttggagttta tttttggttt tggcgttggc gtagggattt tttgatcggg 3840
tttgaggggt tcgggttagt tttaatgtta ttatttatag cgagggtagg gtgtaaggtt 3900
gagaaggtta tatttatcgt tttgggagga cgtgggagaa gagattgagg tggaaagcgt 3960
tttgttttgt ttatcggtcg tttttgtttc ggttttagcg tttgttggga tttgttagga 4020
tttgtcgggg tttcgggaga ttttgagtat tcgtaggaag aggtgttgag aaattaaaaa 4080
tttaggttag ttaatgtatt tttgtcgtcg gttgtaggtt tcgtttttgt attaagcggg 4140
cgttgattgt gcgcgtttgg cgatcgcggg gaggattggc ggttcgcggg aggggacggg 4200
tagaggcgcg ggttatattg ttttggagtc ggttcggttt tttgtgtttt ttttagcggt 4260
taagttgcga ggtatagttt tttattgttt taggagtata gaaatttttt gtgtgggcgg 4320
cgggtgcgcg agttagaggg aaagatgtag tagttattgc gattggtacg tagttgcgcg 4380
tttttgtgcg tacggatttc gcgcggtgtg cgtggcgatt gcgttgtttt taggagtaag 4440
ttacgggttt agaggggtaa aatgtttagg tttttcgttg ggaaggatat attatatttt 4500
atggtaagtt agggtgggcg attttttatg gatcgggtgg agggggggtat tttttaggat 4560
cggcgggcgg tttaggggaa taattcgtgg tggcgatgat ttgtatagcg cgggttttgg 4620
gatgcgcgcg gtttcgagtt agtttcgtat agttcgtttt cggagttgcg agtttaggtt 4680
tttattttcg atttttcggg tttttttcgt atcgttgagt ttagtttgtg gggtgtattc 4740
gattaacgtt cgatagggtt ggggaatgtg ataggtagta ggtttattcg ggtttgggga 4800
gggggagttt tcgttttgat agtatttttt tttgtcgttt gttggtggat ttttattttt 4860
agtcggtaat cgtttcgtag tgttgattta agaaggtaaa gaaaattagg tttttttgta 4920
aagagttttt tttaaatcgg cggatttcgg atattttgag tggatttaga aatttatgta 4980
attttttttt tttagtttat tttttatttt tttttatagt ttttttgat ttgttgttgg 5040
ttcggggtaa gataaagtag ttagtagaga gcgataataa tagcggcggg aaatgaattg 5100
gagattggtt gatagttttt aatattttgt tatagatttt ttcgaatgtt ttaggttgtt 5160
tttggtgggt tttagtattc gtcggttttt tgggtatcgg ggattagaag gaatttggta 5220
gttggtttta ggggtatagt taaaggtagg atgatagtta tttttttgtt tattttagag 5280
cgttgtcgtt tttttatgtc ggtcgcgtaa agaatatagt ttttaaaaaa tacgtgtttt 5340
ttgtttatat aggtttgaaa gtgatgagga aagtaatgtt tcgtttatta gcgagtttta 5400
gtttttaaaa tgattttaag cgttgttgag atgagaaagc gtggtatttc gggggttttt 5460
agttttattc gcgtttatgg tgtaagtttg tagggatagg ttcgggatag tattgtttac 5520
gttgttagat ttttcgtaga ggatcgttga agttgttttc gtgggagata gaatgttttt 5580

```
tttagcgagt ggaaaaggtt tgttgaggat ttcgttttgt tcgagtattt aaatgtgtgt    5640
ttgttttatt attttgggtt gaaaagggat aagagtttta gtttttttat ttggttattt    5700
tattagtaat tataagtgtg ttgagtggtt attattatat aggaggtttt ttagtttggg    5760
gttagtagat tagttttttt agatattgat gtagaagttg ggattggtaa gtaggtatta    5820
tgtgttcgga gcgttagggg ataggagtaa atggagaaga aaagcggagg ttttttttcgt   5880
tcggagtatc gatcggaatt ttcgtcggta cgtcgtagag ggttttcgtc gttgggtttc    5940
gggggtttaa taagtttagt cgtttcgtag gcggttcggt cggattttta gatcggtgtt    6000
tggaagatat cgtttttgtt ttttttcgt taaatttgtt tttttttttt tttttatagg     6060
ttataggttt tttttttttt ttattttggt ttcgttttcg ggttttgtta aatagttaag    6120
taggtcgggg tttagggggt ttagaatgaa gaggtttgat ttggttagcg tcggtaaagt    6180
ttatttttag gcgaggttat aatagaggta ggttttttttt gtttagtttg tcggtgtagt   6240
tatagttaag ggtggtattt gaaaggaaaa gggagaaaat ttcggagaaa tttagattgt    6300
tttaacgtta gattttagag aaattgattt taaatgtacg gattcgttcg gaaagggcgg    6360
ttaagtggta ggtggttgta atttcgttcg gtcgggtttt cgtagaggtt ttttaagatt    6420
agtttttgta gggcggtttt tagtaatttg ataagaggcg gttaagataa atttttgcgg    6480
gttcgagtat atattttcgg gcgttgggtt ttagagattt ttaaattaag tataaataag    6540
aagggagtga gagaatttag gttagaattt gtacgggtat tttattgagg aaaagcgagg    6600
tttcggtggt aggtatgttt tttttcgacg ttcgaaaatc gagtcgagcg ttcgattata    6660
tttattgtag aggttttcgt ttttagtgag ttcggatttt ttagcggttt gttcggagtt    6720
ggtttttagt tttcgtcgta gttcgacgta cggttttttt ttggtagtaa gtttttagcg    6780
gttagtttga agttaatttt gtttaggcgg tcgagggttt ttagttaatt tattatgatg    6840
tcgtttgggt tatttgatgt tcgtagcggc gggatacggt tcgggtagtg cgtagtggtt    6900
tttgttaggg gtatcgcgtg cgtgtttgtt tttcgttgcg tcggggacgt ttttgggtga    6960
tacgggtcgt tgggtatttt ttaagtcgag gaaacggatt tttttcgtag agtttcgcgt    7020
ttattttttta atttttttatt tcgtttttcg ttgttagggt tttcgattta gtttattttt  7080
tttggcggtt tagttaggga ttagagttgg agaggttgaa cgtaattcgt gttagtacgg    7140
aatagacgat atgtttgttt gttagttgtt tggatgaata attgaaaagt tcgttgtagt    7200
ttgtgtttcg ttaagtttcg ggtgtcggga gaatattttt ttaatacgta ttagggtggg    7260
cgggagcggg tagaggaggc gggattcgag ggaggagagt gaattcgagt aggagaagta    7320
gtttaggtag ttaggcgttt tcgatgcgag aggttgggta tttatttttta ttttaggttt   7380
tttattgtgt ggttatgtta ttttttttaaa taaatgtgta tatggaggga gatcgatgtt   7440
gataatgttt agaagattaa aagagtatta atgttggtaa taataacgta aacgtgtgga    7500
tttagatttt attgatttgg aatttgattc ggcgcgtttt tagtaagttc gacggcgcgt    7560
tttttttagt agagcgttta ttagcgttac ggtttcgcgg ttttttagcg gtgtcgtttc    7620
gttagttttg cgcgggtttt ttcgtttgat cgtagttttt ttttcgcgag gttttagttc    7680
gttttatttt ttcgaggttt tttttttttt tcgcggggtt ttttgttttt tgtatttttt    7740
ttttcgattt ttgtattatt cgtttttgtg cgtatatatc gttatttgcg ttttcggcga    7800
ttcgtttggg cggttgggtt cgcgaagtta atgcgttgaa cggtgttcga gttttttttaa   7860
ttattttgtg tttggtcgtt gttattgggt tttggtgatt aagtttaagt ttgaaaatga    7920
cgtggttaaa gttgtttgtt aacggtagag tttaattagt cgtagatttt tttttatttt    7980
tatcggtttt cgtattaaaa aggtttacgc gtagattttt tacgtaggtg tattcgttgg    8040
ataattaaac gtggttttag taataaaagt ttgagtttta tttttttcgag tagtaggttt   8100
tgcgttggat tggtcgggtt taatagggag aattttgtgt tttattttgg ttgggatagg    8160
aagtaagaga agtaaattgg ggaattttttg ttttattttt ttttattttt tggacgtttt   8220
gggtcgaggt tcgggttatt ggtttatcgc gggtaaggag gtgttttgtg gaatatttgt    8280
attgattgga aaagaaagaa ttttaaagtt ttttttttttc gtttgtgggg gatattatta   8340
tatttgtta attatttttt tggttaaatg gtggtttgtt tttttagaag gagtattaag     8400
gtgaatttta tggaaaaaaa ataaaagatt agggatttaa gtgggagtag gatgagaatt    8460
aattttt                                                             8467
```

<210> 83

<211> 8467

<212> DNA

<213> Artificial Sequence

<220>

<223> chemically treated genomic DNA (Homo sapiens)

<400> 83

```
aagaattagt ttttatttta tttttattta ggtttttgat tttttatttt ttttttataa      60
aatttatttt agtgtttttt ttggaaaaat aagttattat ttggttaggg gagtggttgg     120
tagggtgtgg tggtgttttt tataagcggg aaggaagagt tttaaagttt tttttttttt     180
```

```
agttaatgta ggtgttttat aggatatttt tttattcgcg atgggttagt gattcgggtt     240
tcggtttagg acgtttaggg ggtgggaagg ggtggggtag gggtttttta gtttgttttt     300
tttatttttt gtttagtta aggtaggata taagattttt tttgttagat tcgattagtt     360
tagcgtagag tttgttattc ggagggatga agtttaggtt tttgttatta gggttacgtt     420
taattgttta acgaatgtat ttgcgtaaaa agtttgcgcg tgagtttttt tagtacgaga     480
atcgatgagg ataggagggg atttgcggtt gattaagttt tgtcgttagt aggtagtttt     540
agttacgtta tttttaaatt tgggtttagt tattagggtt tagtggtaac ggttaagtat     600
aggatagtta ggaagattcg ggtatcgttt agcgtattgg tttcgcggat ttagtcgttt     660
aggcggatcg tcggaagcgt aagtagcggt gtgtgcgtat aggggcgggt ggtgtagagg     720
tcgggggagg gggtgtagag ggtagagagt ttcgcgagag gaggagaaaa tttcggggaa     780
gtaaggcggg ttggggtttc gcgggggagg agttgcggtt agacgggaga attcgcgtag     840
agttggcgaa gcggtatcgt tggaaaatcg cggggtcgtg gcgttggtga gcgttttgtt     900
gggaagagcg cgtcgtcggg tttattggaa acgcgtcgga ttaagtttta gattaatgaa     960
atttgggttt atacgtttac gttattgttg ttagtattaa tgttttttta atttttttaaa   1020
tattattagt atcgattttt ttttatatat atatttgttt gagaaagtga tataattata    1080
tagtggaaag tttggaataa aaataaatgt ttagtttttc gtatcgaggg cgtttggttg    1140
tttgggttgt ttttttgtt cgggtttatt tttttttttc gggtttcgtt tttttgttc      1200
gttttcgttt atttgatgc gtgttgggaa ggtgtttttt cggtattcgg gatttgacga     1260
agtatagatt gtagcgaatt ttttaattat ttatttaagt agttagtagg taaatatatc    1320
gtttgtttcg tattggtacg ggttgcgttt agtttttta gttttgattt ttaattaaat     1380
cgttaggaga ggtgggttga gtcgggagtt ttagtagcga gaaacgaggt gggaggttgg    1440
ggggtgggcg cgagattttg cgaagggggt tcgtttttc ggtttgggag gtgtttagcg     1500
gttcgtgtta tttaaggacg ttttcggcgt agcgggagat aagtacgtac gcggtgtttt    1560
tagtaggagt tattgcgtat tgttcgggtc gtgtttcgtc gttgcgggta ttaggtggtt    1620
taggcgatat tatggtgggt tggttaaggg ttttcggtcg tttgaataga attggtttta    1680
gattggtcgt tgggagtttg ttgttaggag agggtcgtgc gtcggattac ggcggggatt    1740
ggagattagt ttcgggtagg tcgttggggg attcgggttt attggaggcg gaaatttttg    1800
tagtaaatgt agtcgggcgt tcggttcgat tttcgggcgt cggaagaaaa tatgtttgtt    1860
atcgaggttt cgtttttttt tagtgggatg ttcgtgtaag ttttagtttg ggttttttta    1920
tttttttttt gtttgtgttt ggtttagagg tttttggggt ttaacgttcg agagtgtgtg    1980
ttcgaattcg tagaaatttg ttttggtcgt tttttgttag gttgttgaaa atcgttttgt    2040
aagggttggt tttggggaat ttttgcgaag gttcgatcgg gcggggttgt aattatttgt    2100
tatttagtcg ttttttcga acgaattcgt gtatttggag ttaatttttt tgaaatttaa     2160
cgttggggta atttaaattt tttcgaagtt tttttttttt ttttttaag tgttattttt     2220
ggttgtgatt atatcggtag gttgggtagg aaggatttgt ttttgttgtg atttcgttta    2280
agggtgagtt ttgtcggcgt tggttaaatt agattttttt attttgagtt ttttaaattt    2340
cggtttgttt ggttgtttgg taggattcgg gggcggggtt aaaatgagag agaaagggaa    2400
tttataattt atgagagaaa gagaagaggt aggtttggcg ggaggggggt agggacggtg    2460
tttttaggt atcggtttga gagttcggtc gagtcgtttg cggagcggtt gggtttgtta     2520
aattttcggg gtttaacggc gagggttttt tgcggcgtat cggcggggat ttcgatcgat    2580
atttcgggcg gagaaagttt tcgttttttt tttttatttg tttttgtttt ttagcgtttc    2640
gagtatataa tatttattta ttagttttag tttttgtatt agtgtttgaa gagattgatt    2700
tattgatttt aaattgaaaa gtttttatg taataataat tatttaatat atttgtagtt     2760
gttgataaaa tggttagata aaaaggttaa agtttttgtt tttttttaat ttagggtaat    2820
aaaatagata tatatttgaa tgttcgagta aagcggggtt tttagtaggt tttttttatt    2880
cgttggagga ggtattttgt tttttacgaa ggtagtttta gcgatttttt gcggaaaatt    2940
tagtagcgtg ggtagtgttg tttcgggttt gtttttgtag atttgtatta tgggcgcggg    3000
tggggttgag gattttcggg atgttacgtt tttttatttt agtaacgttt gggattattt    3060
taaaagttga aattcgttaa taggcgaagt attatttttt ttattatttt tagatttata    3120
tgggtagaag gtacgtgttt tttaaaagtt gtgtttttg cgcgatcggt atgagggggc     3180
ggtagcgttt tgagatgagt aggagaatag ttgttatttt gtttttaatt gtatttttaa    3240
gattagttgt taagtttttt ttggttttcg gtgtttaaga agtcggcggg tattaaaatt    3300
tattagagat agtttgggat attcgggggg atttatgata aaatgttaag aattgttagt    3360
tagtttttag tttattttc gtcgttatta ttatcgtttt ttattggttg ttttattttg     3420
tttcgaatta atagtaaatt agagaaaatt gtaggagagg atgaaaaata aattaaagga    3480
gaaagattat ataaattttt aaatttattt aaagtattcg gagttcgtcg atttgggga     3540
ggttttttgt agggaaattt agtttttttt attttttag attaatattg cggggcgatt     3600
atcgattggg aataggaatt tattagtaga cggtaaagga aaatgttgtt aaagcggaaa    3660
tttttttttt ttaagttcgg gtgaatttgt tgtttgttat atttttagt tttgtcgggc     3720
gttggtcgag tgtattttat aagttgggtt tagcggtgcg aggaaagttc gggggatcgg    3780
gggtggaaat ttgagttcgt agtttcggaa gcgagttgtg cgaggttggt tcggaatcgc    3840
gcgtatttta agattcgcgt tatgtaaatt atcgttatta cgaattgttt ttttagatcg    3900
ttcgtcgatt ttgaaagata ttttttttta ttcgatttat gggaagtcgt ttattttggt    3960
```

```
ttgttatagg gtatagtgtg ttttttttag cgggggattt ggatatttg ttttttt ggg 4020
ttcgtggttt gtttttaggg gtagcgtagt cgttacgtat atcgcgcggg gttcgtgcgt 4080
ataaaagcgc gtaattgcgt gttagtcgta gtaattattg tatttttttt tttagttcgc 4140
gtattcgtcg tttatatagg aggtttttgt gtttttagaa taatagaggg ttgtatttcg 4200
tagtttggtc gttagaggag gtataaagag tcgagtcggt tttagaataa tgtaattcgc 4260
gtttttattc gtttttttc gcgggtcgtt agttttttc gcggtcgtta ggcgcgtata 4320
attagcgttc gtttaatgta aaggcggagt ttgtagtcgg cggtagggat gtattaatta 4380
atttgaattt ttaatttttt agtatttttt tttgcgagtg tttagggttt ttcggagttt 4440
cggtaaattt tggtaaattt tagtaaacgt tgggatcggg gtaaggacgg tcggtgagta 4500
aggtaaagcg tttttttattt tagttttttt ttttacgttt ttttaaagcg gtgaatgtga 4560
ttttttaat tttatatttt gttttcgttg taggtagtga tattggagtt ggttcgagtt 4620
ttttaagttc ggttagaaag tttttgcgtt aacgttaagg ttagagatgg gttttaggag 4680
cgttgttgta ttttggtcgt cggtttttcg cgggggtaag gttgtgtttt tagttaatga 4740
ttaaataagt ttgtttatat ggtgggtggg agagtagaga gtttttttt ttttgtagag 4800
taaagttttt taattgtttt tgtataggga gattagttag ttcggaaaat tgaaatgtgt 4860
tgtttaaaag agatttgaag tgtatggggt tttgaataag ggtaggtttt ggtttttgtg 4920
ggttttggaa agatagggtt tttagaggaa aacgtatatt tttatttcgt tttttagtat 4980
ttgggaagat cggaaatagg gtaaaggttg gtatattgag gggagggcga atgaaatggg 5040
ggggggtcgg ttaatgaaag tttagggata aggagagagt aagaaagaaa aagaaaaggg 5100
agaagggaaa gtaggggaag agcggaagag aaagagaaaa tggaagaaga aataaaaacg 5160
agaagaaaga ggacgtgtat aggaaagaga aggaaagaat taagagaagt gattcggcgc 5220
gtagatttgg gttataagta tcggattcgg agtttttttt ttagtagttt ggtttttggt 5280
tagtttcggt tttatttgtt aggcgtaagg cggatatgcg ttggaattcg ggtttttgaa 5340
gttattttta tattttgcgg aaggtttttt tgttagattt gagtttattt tacggttttg 5400
gatttcggag taggtcggag agagtttttt gagatcggta tagtattttt tgcgtaaaga 5460
tcgtttgggg gatattgtag cgttttagtt tcgttgtgat gcgttgggtt atttttttgg 5520
tgttgatttt ttttagttgg ttcgacgtgg ttatttgcga gttcgatgag gacgagggtg 5580
gtcgttcgcg attgggtgtt agtatcggtt cgtgagattg agtgtggttc gggtggtgta 5640
ggtcgtttag gtgcgatatt atggtcgtag gtggggtgtt taggtcgcgg gataggtgtt 5700
gtttatcgcg ggttagtatg gtagtgtggt gcgcgtcgaa gttggggttg agtattttgt 5760
cgtggttcgg cggatcgtag ttgggtagat tttgttgcgc gttgtggagg tcgtttagtt 5820
cgttgtttag cggcgtggcg gttagcgggg ataggttttg gtttatgtcg ggtatttttt 5880
tgtagggatt gtagaggttg tttatggtcg ggagttcgcg ttcgtcgcgt atgagggtga 5940
agttgtcgtt gacgttgtcg gataggcgtt ggtggtggtg gtggtggtgg tggtgcggat 6000
ggtggtgcgg gtgagggtgg tggaatttgt tagatacggt ggagatgggt ggtagcggtt 6060
ggagcggtgt tagcgtggtg taggtgttgt ttatgtttat gttaggcgga gacgagtcgt 6120
aggatatgtt tatggcgtgg tgtagcggga tggagagttc gggtcggtag tcgtcgtcgt 6180
ttaggatcga ggttatgttg gtgattatgg tcgagcgcga cgtcgtcgtt gtcgtcgttg 6240
tcgtcgtgtt tagtttttgg tgcgcggttg gaggcggcgg agggtttcgt agcgagttag 6300
cggcgtcgcg gttcgcgtgg tgggggttgg ggttggttag tagtttttgt ttatggttcg 6360
ggttttcgtc gtcgttttcg tcgttttcgt cgtcgttttc gttattgtcg tcgtcggtcg 6420
gttcgtgtaa agtgtttaga ttttttattg ttagtttcgg gtttatggcg tagtttttta 6480
ggtttttggt gaggtatcga taggcggtgt aggtagtttt tatttagttt attagggttc 6540
gggggcggcg ggggcggcgg gggcggtcgg cgggcgggta aggcgcgcgt ggcggggttc 6600
ggttgcggga gtgggggcgg gcgcgggagt gagtggagag gagtgcgcgt gcgggtgtgc 6660
gtttcgggtt gcgggcgggt tcgttcgggg tgggggcggg gtgggggcgg acggggcgtg 6720
cgtgcgggag aggggagggg acggggaggg agggagggat tatcgggttt cgtcgttcgg 6780
gtcggttcgt agtttcgtta tgttcgagtt cgtttcggcg tcgacgtttt ttgtttgggt 6840
gagcgggagt tgtttagaag ggttttgtcg ttcgtcgggg tagttaattt atttttttcgg 6900
atttggggcg gggttacgtt cgacgggcgt taagcgtaat tatttcgagg tcgtagttgt 6960
ttcgggaggc gggtttttta cgggaagtta ttagtggttg tggagggggc ggggtcgtat 7020
tttttaggtt tggttgacgg ttcggtgttt tttttatttt ttttgttatt ggaattaaac 7080
gttaaggaga gggagggagg agaaggtggg ggggtcgttg gggagaggga ggttggagtg 7140
cgagcgagcg agagagattc ggtgaaagat gtcgtgttgt gtttttttttg agtagttgcg 7200
atttggggga aggggtattg ttttaatcgt tttcgtttta ttttaaagtt tttttggagg 7260
cgagtaagtc gggagtaaaa gatttcgtta tgtgttagta tagataaata ttcgttatta 7320
ataagttatt tatcgagagt ttttagttgg gagttaatag tttagttttt taaaatgtag 7380
gcgttaatgt attttaatgt atggtaaatt ttcgaggtcg ggatatgtat aatttacgcg 7440
gttagagtaa ttgtagtgtt ttcgtcgtta gcgagttgcg gtcggtcggc ggtttacggg 7500
cggcgcgggt agtagtaatg tatgtaaagt taatataata atattattta attattcggt 7560
gagtttataa attttagtta tgaggttttc gaagagtttt gttagattgt aaaataaaat 7620
tagaatttcg ggagttgtcg ttttaaggtg attaagtgtt ttttgtatta gcgatttgga 7680
aagaagtggg gagagtgtag aatcggtgag gtggaggtga ggagggtcgt tggtgtttta 7740
```

```
agtgagtaaa ttttttttgg gggtagagag taagtaaatt agttgagacg gtaattacgg    7800
attttttttt taatacgaga tatatatacg cgtatatata tatatatata tatatatata    7860
tattttcgtt ttttttaaat ttcgggagag aaacggcggt gggtagacga taaggaattt    7920
tggttgatat ttgtatattt aaattttgtt tttgaaaaga ttagcgtttc ggttagtttg    7980
agaacgttta taagttaagg ttttaaaaat cgtatttacg tttttttaga attttcgttg    8040
tttggtaatt ttaggtttgg aggtgggagt ggaattgggg gatagagagt tggagatttt    8100
tgtagttcga gtcggggtgg aggggtagtt gaagatagag aggttaatat tcggtataaa    8160
gttttaaacg taataagatt tttttaaagt aggttgcgtt ttgtttgttt tcgaagattt    8220
aagttagttg aggttgcgta gatgttgttt ttgtttaaag agatagtaat taaggatagg    8280
gtggatataa gtttgagaag cgtgatttat tttttttgga ttagttgtta aaagaagagt    8340
agggggtttt tttaagatag ttttttttaa ttatttttag ttgttagaga aaatttgata    8400
agaaaataag ggtagagttc ggaatattgt atgttttttt ttggtgttag agaaaggttt    8460
gatttat                                                              8467
```

<210> 84
<211> 4453
<212> DNA
<213> Artificial Sequence

<220>
<223> chemically treated genomic DNA (Homo sapiens)

<400> 84

```
ttggttttga agtttatagt atcgttgatt tagtttgttt tttggaaggt tggtagttta     60
gtaagtatag aagtttttt agaagatagt gggttatttg tttttaaaa gttgaaaggt     120
taatttgtat tttttagt aggtagttgg tattttgagt tttcggttgg ggtagagtaa     180
aggagttttt ttttttttta tttttttggt atttttttg ttttttttt gttattttta     240
ggtggattta gatttaaggt ttagatttgt aaggtaggaa aatgttgtag gtttaggttg     300
ggaaagggtt taaagtcgtt agtggattgt tgggatttag ttttttttt tttattaaga     360
gagcgagttt tattgggttt aaaatgattt taagttttgg tttttgatat taggggaaag     420
agatgggggt gatagaatta tagaattttt gttatgtttt tttaagtgtg tttagagatg     480
cgtgtgtgtg tgtgtgtgta tatataaatg tttgtttatt tttaggtagg aagggtggat     540
gtagttattt atatatggtt tgttttttg gaggataatt ttatttgata aataattgtt     600
tttatttgaa tagaataaat aaggttttat gatgaagtaa aatattaaat atatatgtat     660
taaaaaatgt ataattattt ttttggaatg ggttatatag agatgtgttt tttaaaatgt     720
taagagtgta aaaggataaa tagtgaaaaa taaatttttt ttttattttg ttttttagtt     780
ttttaatttt tttatttaga ggtgagaata gaatttttat atttttaga atttttatag     840
ttagaattgt ttatatgttt ttattgtttt tatttttatt tttgtttgta taaataaatg     900
aattgtttat tatggaaatt ttttaaaaga ttcgttaata ttttaatagg aagtattaat     960
agtttatgtt ttaggatttt gttttataa ttttgtaata ttatattacg atatttaatt    1020
taatttttat taagttttgt taaaaacgga ttttaaatta agttgtaaat ttttagtaat    1080
ttggttttgt ttttttttt ttgatagtat tattaaataa atttttttat tgtcgaaagt    1140
aataagttcg gttttgtttt atttattggt tgtgttggtg atatttgggg attgttattg    1200
aatagacgta tagagggagt ttttataggt aggggttttt ttgtttgtgt ttttgggaga    1260
gtatgtttcg tatatttgtc gcgttgatga agattttata gttttattag ttgcgggtaa    1320
gggggtttga ggtagtttta ggtaagttgg ggtttagcgg ggagaagttg tagaagaatt    1380
gattagagga ttttaggagg ttttagagtt gggcgaggta gagagttttt tgtgcgtttt    1440
ttttttttt tgtaattcgg ggattttttg tattggggta ggttttcggt taggtgtatg    1500
ggaggaagta cggagaattt ataagttttt cgatttttta gtttagacgt tgttgggttt    1560
ttttcgttgg agatcgcgtt ttttttaaat ttttgtgagc gttgcggaag tacgcggggt    1620
tcgggtcgtt gagcgttgta agatagggga gggagtcggg cgggagaggg aggggcggcg    1680
tcggggcggg ttttgatata gagtaggcgt cgcgggtcgt agtatagtgc ggagatcgta    1740
gtttcggagt tcgggttagg gtttatttgt tttcgtagcg tcggttcgcg tttttttgtc    1800
gtagttatcg gtgagtgtcg cggttttgag atttcgggt cggatgcgcg gcggttttag    1860
ttttcgagcg tttgtttttt tcgttttggg ttgttcgggt tttttgggtt tttcggcggt    1920
tgtacggagt taaggcgttt cgtttcgggc gtttttcgcg ggtgtcgatt taggttgttc    1980
ggagttcgga gtttagagag gagagagata gttggggagt ttggttatcg cgggtatttt    2040
ttttgcgttg tagtcgttcg tttggtttgt tttttcgttt tttcgttttt tgttttgatt    2100
ttttttttt ttgtagagtc gtcgtttagc gtttcgattt cgttattatg agagttttgt    2160
tggcgcgttt gtttttttgc gttttggtcg tgagcgattt taaagtgagt gcgtttttgt    2220
tttgattgat gttgtttaag gatttttgat tagtattagg ggagaggagg ggttgtttag    2280
ggagttgggg tttttcggat tttatttata gtagggttag atttttttta ggaaatggga    2340
```

```
tagggtggta gcggaggttt gagaattacg gggttggta ttggttggta agggaggaag 2400
aggtcgtcgg gattgtttta gtttgcgggt atttggtaga tgaagtttgt ttgggttaat 2460
ttattttttt tggttggaaa tttatggttt tttatttgag aattagatac gaatagggtg 2520
aggcgagagg gagagggaag agtgggtttt gggattgggg ttagtttatt tttattttgg 2580
agtttttgga gtatgggatt tttgatgaag ttttttttcg aatttttttt agggtagtaa 2640
tgaattttat taagttttat gtgagtattt atttttataa tagttggttg tatagataag 2700
ttgggaaggt tttaggggat attttttttt tgtttttgt tgtagggttg cgttattttt 2760
tattattttt atttttttc gtttatttta tttttgtttt ttttagcgaa ttgtgattgt 2820
ttaaatggag gaatatgtgt gtttaataag tattttttta atatttattg gtgtaattgt 2880
ttaaagaaat tcggagggta gtattgtgaa ataggtatgg ggatttttat tgtaattggg 2940
agagaaattt ggggataggg agggatgggt gggaggtaag agtaggtagg agttaggagt 3000
tggaggtagg gtgggtgata tttttatttt tatgtgataa gtataaatat atatatacgt 3060
ttacgaaata gtggttatat aaatgtgagg tggggttgga aggagatttt gtttagtttt 3120
ttggtaggtt tgaaacgata tttttaaaat gttcgttggt agtcgggtat ggtggtttac 3180
gtttgtaatt ttagtatttt gagaggttaa ggtgagtgga ttatttgagg ttaggagttt 3240
aagattagtt tggataatat ggtgtaattt tgtttttatt aaaaatgtaa aaattagttt 3300
ggtatggtag tggatgtttg tagttttagt tatttgggag gttgaggtag gagaattgtt 3360
tgaatttggg aggtagagat tttagtgagt tgagattata ttattgtatt ttaattgggc 3420
gatagagtaa gattttattt taaaaaaaaa aaataaaagt tagttggaat gttttttttt 3480
tttttatatt tttttatttt tttgtttttt tgtagataag ttaaaaattt gttatgaggg 3540
gaatggttat ttttatcgag gaaaggttag tattgatatt atgggtcggt tttgtttgtt 3600
ttggaatttt gttattgttt tttagtaaac gtattatgtt tatagatttg atgtttttta 3660
gttgggtttg gggaaatata attattgtag gtgaggtggg ggtaataagg attaaaagtt 3720
tttttttatag tttttttagaa attttgttat tattttttttt ttttagaggg ttggttatag 3780
tataagagaa gtgcggtttt tggttgagtt ttttttgagg ggaggaggta gggaaggttt 3840
tttgggttgg aatgatattt tttatttttt tgtgttgtta ggaatttaga taatcggagg 3900
cgattttggt gttatgtgta ggtgggttta aagttgtttg tttaagagtg tatggtgtat 3960
gattgcgtag atggtgagta ttattgattt gttgatgata gtggggtgga aggggataaa 4020
tttatatgtt tttttatttt attataggag gattgaggag gtggggggtg ttcgagaggg 4080
atgttttttt ttatttgttt ttttaagata ttttttttgtt tgtttttttag gaaaaaagtt 4140
tttttttttt ttagaagaat taaaatttta gtgtggttaa aagattttga ggtttcgttt 4200
taagattatt gggggagaat ttattattat cgagaattag ttttggtttg cggttattta 4260
taggaggtat cggggggggtt ttgttattta cgtgtgtgga ggtagtttta ttagtttttg 4320
ttgggtgatt agcgttatat attgttttat gtacggtttt gggttttttt ttttcgattt 4380
ttttgtttta ttttaagtat attttttttt tttttttagt aaagtgtttc gttttatttt 4440
ttttttattt gtt 4453
```

<210> 85

<211> 4453

<212> DNA

<213> Artificial Sequence

<220>

<223> chemically treated genomic DNA (Homo sapiens)

<400> 85

```
ggtagatgag ggagaaatga ggcggaatat tttgttggga aggagaaagg gatgtgtttg      60
gggtggggta gaagagtcga agaggagaaa tttagggtcg tatatgaagt agtgtgtggc     120
gttgattatt tagtaagggt tgatgaggtt gtttttatat acgtaggtga tagagttttt     180
tcggtgtttt ttgtagatgg tcgtaaatta gggttggttt tcgatggtgg tgaatttttt     240
tttaataatt ttaaagcggg gttttagagt tttttggtta tattgaaatt ttaatttttt     300
tggaggagag gagggttttt tttttggagg ataaatagag ggatgtttta gggaggtagg     360
taggagaaag tatttttttc gggtattttt tattttttta gtttttttgt gatggaataa     420
ggggatatgt aagtttgttt tttttatttt tattgttatt agtaggttag tgatgtttat     480
tatttgcgta gttatgtatt atgtattttt ggataagtag ttttaggttt atttgtatat     540
agtattaggg tcgttttcgg ttgtttgggt ttttggtaat atagaaagat aggggatgtt     600
attttaattt agagggtttt ttttgttttt ttttttttagg gaagatttaa ttagaggtcg     660
tatttttttt gtgttatggt tagtttttttg ggagaagggg atggtaataa ggtttttggg     720
aagttgtagg gagggttttt ggtttttgtt gtttttattt tatttgtagt aattatgttt     780
ttttaggttt agttgaagag tattagattt gtgggtatgg tacgtttgtt gaaggatagt     840
ggtagagttt tagggtaggt agggtcggtt tatggtgtta gtgttggttt tttttcggta     900
aaagtgatta tttttttttat agtaggtttt tgatttattt ataaggggat aggaggatga     960
```

```
gagaatatga gaaagagaag aatattttaa ttaattttta ttttttttt ttgagatgga 1020
gttttgtttt gtcgtttagt tggagtgtag tggtgtgatt ttagtttatt gagatttttg 1080
tttttaggt ttaagtaatt tttttgtttt agttttttaa gtagttggga ttataggtat 1140
ttattattat gttaggttga tttttgtatt tttagtagag gtagggttat attatgttgt 1200
ttaggttggt tttgaatttt tgattttaaa tgatttattt attttgattt tttaaaatgt 1260
tgggattata agcgtgagtt attatgttcg gttgttaacg gatattttaa agatgtcgtt 1320
ttagatttgt tagaagattg gatagggttt ttttttaatt ttattttata tttgtgtggt 1380
tattgtttcg tgagcgtgtg tgtgtgttta tgtttgttat atagggatga agatgttatt 1440
tattttattt ttagttttta atttttgttt gtttttgttt tttatttatt ttttttttgtt 1500
tttaaatttt tttttagtt gtagtggaga tttttatatt tattttatag tgttgttttt 1560
cgaatttttt tgggtagttg tattagtgaa tgttggagaa gtatttgttg gatatatatg 1620
ttttttatt tagatagtta tagttcgttg gagagaataa aggtggggta agcgaggggg 1680
agtggaagtg gtaaggggtg gcgtagtttt gtagtagagg gtagggaggg gatgtttttt 1740
gaagtttttt taatttgttt gtgtagttaa ttgttgtagg ggtggatatt tatatggaat 1800
ttgatgaagt ttattgttgt tttggaagag attcgggagg aggttttatt aaaggtttta 1860
tgttttaggg atttagggt gagggtaaat tggttttaat tttaaaattt atttttttt 1920
tttttttcg ttttattttg ttcgtattta gtttttaaat ggaagattat gggtttttag 1980
ttaggagaaa tggattgatt taagtaagtt ttatttatta gatgttcgta ggttggggta 2040
gtttcggcgg ttttttttt ttttgttagt tagtgttaat tttcgtggtt tttaagtttt 2100
cgttgttatt ttgttttatt ttttggggag agtttggttt tgttgtggat ggaattcgga 2160
ggattttagt tttttgagta gtttttttt ttttttggtg ttgattagag gtttttgggt 2220
agtattagtt aaagtaagag cgtatttatt ttggagtcgt ttacgattag gacgtagaga 2280
agtaggcgcg ttagtagggt ttttatggtg gcgaggtcgg ggcgttagac ggcggttttg 2340
taaaggaagg agaagttagg gtaagaggcg gaggaacggg aaggtaggtt aggcgggcga 2400
ttgtagcgta ggggagatgt tcgcggtgat taggtttttt agttgttttt ttttttttg 2460
ggtttcggat ttcgggtagt ttggatcggt attcgcgggg gacgttcggg acggggcgtt 2520
ttgatttcgt gtagtcgtcg gggagtttag ggagttcggg tagtttaggg cgggggaggt 2580
agacgttcgg gagttggggt cgtcgcgtat tcggttcggg gattttagga tcgcggtatt 2640
tatcggtggt tgcggtagga gggcgcgagt cggcgttgcg gggataggtg gattttggtt 2700
cgggtttcgg ggttgcggtt ttcgtattgt gttgcgattc gcggcgtttg ttttatatta 2760
gggttcgttt cggcgtcgtt ttttttttt tcgttcggtt ttttttttg ttttgtagcg 2820
tttagcgatt cggatttcgc gtgttttcgt aacgtttata aagatttggg ggaagcgcga 2880
tttttagcgg aggggattta atagcgtttg gattgaggaa tcgagaggtt tgtaaatttt 2940
tcgtgttttt ttttatgtat ttggtcgggg gtttgtttta gtgtaaggag ttttcgaatt 3000
gtagagagga gagaaggcgt ataggagatt ttttatttcg tttagttttg aagtttttttg 3060
gggtttttta attagttttt ttgtaatttt ttttcgttgg gttttaattt gtttaagatt 3120
gttttagatt tttttgttcg tagttgatgg agttgtgaag tttttattaa cgcgataaat 3180
gtacgagata tattttttta gaagtataga tagaaaaatt tttgtttgta ggggtttttt 3240
ttgtgcgttt gtttagtggt agtttttaga tattattaat ataattagtg gatggaataa 3300
agtcgggttt attgttttcg gtagtaaggg ggtttgtttg atggtgttat tagaggggga 3360
aaggtaaggt tagattattg aaaatttgta gtttggttta aagttcgttt ttgatagggt 3420
ttgataagga ttgggttagg tgtcgtgata tgatgttata ggattgtggg aataaagttt 3480
tagggtataa attgttggtg tttttattg aagtgttaac gggtttttttg ggaagttttt 3540
ataatgagta atttatttat ttgtgtaggt aagaataaaa gtaaagataa tggaaatatg 3600
tagatagttt taattgtgga ggttttggag ggtgtggaag ttttgttttt atttttgagt 3660
agaggaattg ggagattgga ggataaaata agaggaagat ttattttttta ttgtttgttt 3720
ttttatattt ttaatatttt aaaaagtata tttttgtata gtttatttta aaaagataat 3780
tatgtatttt ttaatgtatg tgtatttagt gttttatttt attatagagt tttgtttatt 3840
ttatttagat agaaataatt gtttattaaa taaaattgtt ttttagaaaa atagattatg 3900
tgtaaatgat tgtatttatt tttttgttt gaggataagt agatatttgt gtatatatat 3960
atatatatat acgtattttt gggtatattt ggaggaatat agtagggatt ttgtgatttt 4020
gttattttta tttttttttt ttagtgttag gaattagggt ttggggttat tttgaattta 4080
gtaggattcg tttttttagt gggaaggagg aggttgagtt ttagtaattt attagcggtt 4140
ttgggttttt ttttagttta ggtttgtagt atttttttgt tttgtaaatt tggattttgg 4200
gtttgggttt atttgagagt gatagaagga aggtagggag agtgttagga aggtaggaag 4260
gaggaaggtt tttttgtttt gttttagtcg agggtttagg gtgttagttg tttgttgggg 4320
aaagtataag ttagtttttt agtttttggg aggtaggtga tttattgttt tttggagaga 4380
tttttgtgtt tgttgagttg ttagtttttt aggggataga ttgagttagc gatgttatag 4440
gttttagagt taa                                                    4453
```

<210> 86

<211> 10490
<212> DNA
<213> Artificial Sequence

<220>
<223> chemically treated genomic DNA (Homo sapiens)

<400> 86

```
agaaggtgtt tgtttttttt tcgtttttg ttatgattgt aagtttttg aattgggagt    60
cgattaaatt tttttttttt ataaattatt taggtttaag tattttttta tagtagtgtg   120
aaaataaatt aatatttttt ttttgaggcg tttttttttt aggtaattcg ttgtttttat   180
gttttttttt tgttttttgt tttttttttt tttttatga ggtttaagtg ataaacgggg   240
ttagttttag ttttagtttt agttttagtt ttattttatt gtaggtttgt gtggttgttg   300
gagaggtcgt gttttttttt ttttttcgag tttgtttgat atgtttttg gattttggag   360
gaaattgatt ttttattttt atattggtgt aatattttt aagattttaa agttgtatta   420
tttgagttag tttttttttt tatttttatt tgttagggtt gttattggga tagttttaga   480
gggtggtgtt aatggatgaa tggatggatg gatagtagtt tagggatgat gtttttgttt   540
gttttgaatc gggttttttt tttaatgaga agtttttttg agtgagtata tatagttatt   600
ttttggtatt tatggaggat tagttttagg gttttcggga atgttaaaat ttatggatgt   660
ttaagttttt gatataacgt ggtttagtat ttacgtataa gttatgtata ttttttcgta   720
tacgttagat cgttattaga ttatttatga tgtgtaatat aatgtagatg ttatataaat   780
ggtcgtgata ttgtattttt tagggaatga tgataagaat aaagtttgta tatgtttaat   840
agaaatataa tcgtttaatt tattttttga atattttta tttgttgttg ttgaatttat   900
agatgtagag tttttggata cgagagttaa gtgtgttttg agagtagggt gggtgaggtt   960
gttaatgagt atagggggagt aggtgttgat taggaggatt ttgtattggg gtatttggac  1020
gttttgtttt aggatttgag atttagttg gatggtatag gtagatttag tttaggttaa  1080
agtcgttttt ttgaagtttt tttttatttt aagtttttt tggattttgg aattcggtat  1140
tttttaggtt ttgcgtggaa ggatagatga attaggtttt agataatatg tttagaagag  1200
tgagttttta ttgtgtgttc ggtatttttt ttataggatt ttttagttag aatatttaag  1260
ggttatggag agaaatattt agttaaaata ttagaaaaga aaaagtgata ttattagaga  1320
tattaaaaag attattaggt aatagtatta gtttttgtat tttgagattt aatagtagta  1380
gttatttttt tttatcgtta tgtgtatttt aggattattt tgggcgggga gggttgcggt  1440
tagggagtag ttatggatgt tttgatgttg gttttgggtt tcgggggtga tagtgatgag  1500
gaattgggtg tatatatgag tggggtagtc gggtttggtt agagaagtag tatatacgtg  1560
tatagacgtg tttatttata tatatatgtg tacgtacgtg tataaatata ttgtaggtag  1620
gtatgttgac gttttaggta gcggaggatt ttgattttgg gcgttgttga ttcgggtaag  1680
gttttattgt gattcgtgtt atgattttag aatgttattg gtgtttagta tttgtttgtt  1740
ttttggtttg ttttagtggt ttatagtagt tatatatagg tagtggtatt tgtgagtagt  1800
tttgtggatt taaaggtttt ttttttgaga ggtatgattt aggttagttg atttattaga  1860
attaggtgag cgtgatttgt tttttttttt ttaggcggat ttggggatag tggttacggt  1920
gcgggcggtg ttggtttttg tggggtagtt atcgaggagg gttatttttg agtatttatt  1980
aggcgttcgt tttatattgt tcgtgtagac gattggtttt ttcgttttta tggtggtttc  2040
gtagagtggg tgttgttttt aaatgttttt attcgataga tgagacgttt ggggttagag  2100
aggtagtaat cggtttggga attcggatat gattttgagt tttgttttta gttttgtcgt  2160
gtgttgtgtt ggaatttagg tttgaatttt gtgatttttt tgttttagat tttaaatttg  2220
tttaggtttt ttatttcgat ggggtagagt ttggttttgg tagagttatt ggtatagagt  2280
tattggtata gatttattga cggtttttag aatattttg tgttttaagt tgggttttga  2340
tggtcgttgt gggttttatt gaatatatat ggtttttttgt ttaggggagt ttgttgtttt  2400
tgggtagttg tggaaaatga aggagttttg gagggttggt tgaggggaga ttattttttt  2460
ttgtgtttaa aggggttcgg gtattagggt tttttttagg tatttttttgt tttgcgtggt  2520
ttttttgagg tttcgttttt tttttgtttc gagtattttt aggagggacg gtttatttag  2580
ttgtttttta ggattaagga tttattgttt tttttagtg atttaggaaa atgaagtttt  2640
tttttgttgg gacggtttag aatggtggat tttatagttt tttcgcgaga gacgtggttt  2700
ttatgcgtat aatagatttt ttttattttt taaatttaat atttttttgt ttaataggcg  2760
ttattttttaa agtggtttta ttgtttagat tgaagagtta cggtagttaa agtgatgagc  2820
ggagtagaat cgagtagtcg ggagagattt tgtttttttgt aggaaattgg gtatcgttga  2880
ggttttgagt atttaggag gtcgattgta tagagatttt tggtcgttga ttttagtttg  2940
tttttatatt tttggaatag tttattatgg gtttttatt tttggtaggt ggaaattatt  3000
taatttgttg gggtcggtgt gtttttattt tatggtattg ggggataata ggattttttg  3060
tttaggtttt attgtattta agtttttggg aagatgttta tttttgtttg ggatttgaga  3120
ttttagagat tggagtagtt gtgggttatt gggtttggtt tttttttttt tggggggcggc  3180
ggtggaatgg gggttacgta gttagttagt atttgggagt tcggcgagag cggtttaggt  3240
gtttttcgaa gtcgtcgcgt atagtgtgat ttttagataa ttttgtttta taggatggac  3300
gtggtagagg tcgcgggtag ttggtgggta taagagcgag aggatattat tatgaaatac  3360
```

gaaaaggtat aagtcggttt gtttttgga gggaggtttt ttttagtgtg ttttggttaa 3420
agggttttgg gtttttagg agtatagggt agggacgggt ggttaatgtt tttaggtttt 3480
tgtattttt attttggatt ttttattaag gtttttttg ggttataggg atatcgagtt 3540
gggttgttag aggataaggg gtttaagttt tttcgaagtt ataataataa cgtcgattat 3600
ttggggattg tatagtgagt tttttgtatt ttttttattt ttaaagtatt tgttttagtt 3660
tagggatggg tttgttttta gaaaggtttt tttgacgtag gatatgtttt attaggtcgg 3720
gttaattttt tttttaggga tagaattttt ttttgatttt tttgtaggtt tagttcgagg 3780
ttgttaggtt agaggtgtgg ggtttattta gggagtcggt gggaatggag attgggttag 3840
gttaggtttt tgggcgttta gtagttttgt cggtaagtga gtataagagg agcggggtag 3900
tttgagggtt tggttttgtt tatttggaga taatttcggt gagatgtaag ggttatggtt 3960
atagggtgag gggacgtttg gtttagtttt agggttgttg tttagtaggt ttttgagggt 4020
ttatttgttt ttgtttttt ttattttttt agagttatag tttttattgt ttcgtgaggg 4080
gaaaaggtat ggtgataatg ggggttgtag ttttaggaga acggggggaga agatgggtag 4140
ggtttcgttt tgggtatttt acggtgaggt tagggaggta gtagggttcg cggttaaaga 4200
tttgggtttg gtgttgggaa gggatttggg gtcgggtaag aggagtttag ttaggagttt 4260
attttttagg gattatagga tggagagata gaggattttt ggggaggtag ggcgggaggg 4320
agttgacgag tcgtgttatt tttgaaacgt agggtgtgtg gttcgggtgt agggagaggt 4380
aggtggatgt tgggaggtta gaatttgtaa gggttttggg gttgttaagt ggggtgggtt 4440
tttggtgtag ttagagtata tcgggtaggt tttagggtag gttttttga ttttggcggg 4500
gggatgtggt tatttttga gggattttg ttagggttcg gtcgtttatt ttgggcggtt 4560
tttattttat tttagggtta atttttttta gttttagtag aaagtattat ttcgagttta 4620
ggacgggtag ttttattggg tagtttgatc gttttttacg ttaggggttt tagtaatttc 4680
ggttaggttg tttttatatt ttttttttt ttaggttttg tttttttgg gagttagttt 4740
tataggaagg tttttgtttt tttttttttg tgttttttt tgggttgagt tttgagttgg 4800
aaagggatag agttagtttt ttttgggggt cggtatttag gttggggtcg ttttaggttt 4860
cgtgtagttt tttagttttg tttgggttgt tttatagtga gacggagttg ttttttttga 4920
ttgcgcggga ggcgaaggta agagtttgat gcgtggaggg gttggtttag ggacgtaggg 4980
attgggcggg tggttagtga ggtagaggaa gtagttggtt tgagcggtgg cgggtgaggg 5040
taatacgttg ttattgggag gggtagtagt ttttgttgga tttgatttta ggttgttgtt 5100
tattttggta gtttgataaa atttaaaag gagaattata gttttggttt gggggtggtt 5160
gcgcgtttgt gttaggattt tatttagagg ttgggattta agattggtgt gtttgtggtt 5220
tgaggatggt atatttcggg gtttaaagt tagtttattg gtgtttattt gtttaaaggt 5280
ttttagtttt tgaggtttgt tttttttgg tttttttag ttggttttta ttagggtttt 5340
agagtttaag atttagtatt cgcgggcggt tttgggaagt ttggtagttt cgttaatttt 5400
aatatgtttt atttgatagt aaattcggcg ggagattagt cgaaagagta agtgggtgga 5460
tatgttggga gattgggaga aatataaaag tagtagaaag gtaacgtgtg gagggaggaa 5520
gtattttttg tagagatagg ggataggtat ttatggttgt ggtttggtat tattagtttt 5580
ttagagggtg ggcggtatat tgttttcgtt tagaggattg taggtttggt cgttagattt 5640
tttgtttatt cgtgtaagcg ttattttgta gggagggaat ttgaatttag ggttgggatt 5700
attcggagtt taaggttagg gatgttttgg tgatttgaag gaaggaaaag gtttagatta 5760
gagtttcgat tttgagtgtt tatttatttt tttagttttg ggaagggaga ttttgtttta 5820
gtttgatttt atttttattg aggaattatg gggttaaaat cgataatttt tagaattttc 5880
gggttttggt ttttattggg gttatttcgt ggtttgtgat attagatcgt tttttgttta 5940
tagtttatag atcgagcgta taagggaatg tttatgaata ttcggggttc gatgtggtta 6000
gttttttga atattgagga aatgaagttg aaaaatttcg gaagatatta ggtacgttta 6060
gttagagtat aataaatagg ataggtcgtg tcggggttta ggtttttagt tggagggaac 6120
gttaagatta ttttggggag ttgggggtga aggttagatg aatattttgg gtatagatgg 6180
tgatatagtt attatagata aatttagttt tggtgatttt ttttggtttt agtaataagt 6240
taaaatgtag tttttgtag aaggaaattt ttttttgtt ttttttttc gaagtgttga 6300
ttgtgggttg attgttattg ggggtaggga gtttttatt tgttttgaga ttgtttttt 6360
tttttggttt tgttttatag attatgaagg agaagggtaa gaggttattt gagtatattt 6420
agcgtatcga tcgggacgta agcgggatat taaggaagta tatattttt agggatcgat 6480
acggaattaa gtaagtttac gggagttata gggttttagt agagatgggg tgaatgagag 6540
ggatgggggt tttttcggag tagaagttag ggttatttag gagggatgat atagttgtta 6600
agagtttttt cggtttaggg agtagtcggt attatgaatc gagtattttt ttggttttaa 6660
gttttgggtt agattggaat atgtggggtt agaatttagg aggattttga ggagatggaa 6720
ggtagtaaat aaaattatgt ataatggtga agggtgtttt ttttgattta tggggattta 6780
tggtaggatt tacgggaggg tggtaggata gagggtttat gagttttttt taggtaatag 6840
tgatagtatt aaatgttggg agaattaggg gttttggaaa tttttattta ggttcgttgg 6900
gaatatgata tggtatagtt acgttggtag ttcgttgggt agtggtttat aaagttcgat 6960
ggatttgaat tatatatttt taaagtgtta tagatattga atttattgat ttgtaaattg 7020
atatttatat gaaattagta tgttaggttt attgtttgat ttttcgttat ttatatacgg 7080
agttttcggg gacggtttttt aatacgggga tggggagagt aaggttggtt tttttttaa 7140

```
acggaagatt tagtgagaaa agggaacgag tcggtgatgt tcgtacgaac gtgggtggat      7200
tttagatgta ttttgttgag ggatagaagt tagatttaat aagttattat agtaggattt      7260
ttatttttag gttattttgg aaaaggttaa attatatagga ttgagaagta gtttgggtgg      7320
ttaggggttg acggatcggg gagaggttgg gtgtataggg gttattttgg agatttggag      7380
gatgaaggag tcgttttagg aggggttgga gcggtggtcg ggagattttg tatattggtt      7440
tggaatcgtg gaggaattgt atatttatag attgaattgg cgtgtgtgta aattgaaaaa      7500
aaaaaaaaaa aattatttag agtgaaaagg attaggtaag ttattgtata attgggttat      7560
ttgtatgtta tagatgtgga ttttattgaa atattttttt aagagtttta ggttttgaag      7620
agtttattgt ttatttggtg aaatatttga atttgaaatg ggatttgttg ttaggttttg      7680
tagataaagt gaaattaata atatttgtat aaaataaatt aaagtttttt tttttttgttt      7740
tttaggtagc gggaattatt ttatattttt ttggtatatg aggagtataa ttcggtgagt      7800
attttcggta gtgaggtttt cgggttatat ttttatattg ataggagtgg gtgtttggtg      7860
ggggtgtcgt tgtttttttt aaagttagta tttgtgattt attaggatat aggaggtagg      7920
atgttagttt atcgttggta taaattttta aggaaggggg tggttttaag gggttaagtt      7980
gagatataga ggagttaggg tttggatttt tggtgttatt tgggtttgat tattattttt      8040
tagaataaga aatgacgttt ttttttgggg gttgttttaa agtttaggag tttggtagta      8100
tcgtatatag gatggtgtta ttagtagata ttttggataa ggtgttgaag tgtttgatgg      8160
atttggtttt tgttatgaaa tgaatgtgta ttttgaggaa gttttttttt tagaggaagt      8220
ttttttttta gaggaagttt ttttagttat ttttgttttt tttaatgata tgagtttttt      8280
taggtgattt tagttttttt aggtgatgtt tttttatggt gattttggtt tttgtaggag      8340
gtgggttatt gtagggattt gagttatatc gtcgttttgt ttttttttta ttttttttgag      8400
gaggatgtat tttgggtatt ggtgtagttg ttggttagtg agaggtattt tttgtagggt      8460
aagtgaatag ttgtttcggg gattttttgt agttagattt ggggatggtt attttggtta      8520
ggtgattata gtttttagtt aaggtatttt ttttgtgtcg ttagtttgtt gggagatttt      8580
aggatgtttt tgttgagggt tttataggag tttacggttg atttttaaag tttaaattag      8640
acgtttttta tttttattag tagagggtat tttatttttt tcgtggttat tttttgtgtt      8700
ttggagttac gtttttcggt tttgattttg tgtagttgat tttttttttt ttgagagttt      8760
ttttgttttt tagttgttcg ggtttttgtt gttatcggtg tttacgaatg ggtcgattaa      8820
gtttaggtgg tagtattttt ttattttttg ttttttggtt cgattttatt attaggagat      8880
gatcgggaag tttagcgttt atttagtttc ggttattttg tcgtggtttg aaagttaggt      8940
ttgttttttt tgtattttgg tttaggaggt ttttaggggga atttttagtt aggtttttagg      9000
gaatgttttc gttttatttt tttagggtaa aggtcgtatg ttggggttat tagatgggag      9060
ggtgggaggt tttggggttt gggggttttt ttagttgttt agtttttgta gttgatggtt      9120
ttatattttg ggggaaggtt ttgattttat gatgggttgg gggtttttta ggattttata      9180
gtttaaatgg cgggatcgtt taggggtttt aagattaata ggagtatgtg gtagttacgt      9240
tataatttaa gattatgggg tattaggtga gtttatggtt tttttagttt tttttagagg      9300
ttttgttttt cgtggggttg taggagtagg ggggttggag tttttcgtgg ggttggtgat      9360
tggttgagtt ttagttaggg tttgatttgg gacgtcgggt tttttatggg ttgggagttg      9420
gttttttttt ttgttttgga ggagatagag gtatagggat gggggtttag ttttcgtaga      9480
gtagggtaaa gggtagtgtg tttatcggga gtgtgggaag gtgatagtgt tgtggggagt      9540
tttggatatc gtttagtgtt ttgtattagg ggaagggttt ttagaggttt tggaagaggg      9600
aggtttttag ggtagtttag tggtttgagt atttttgttg tttttattag gataagaaag      9660
atttatgtgg gtagtgtttt tcgttaggtt gttttattcg gatattgatt gacggggtaa      9720
ggaggtatag ggagattttg gtttagggat ttttttttgtt ttgtagtgtt ttgttttttt      9780
agttcggggg tttggtttat ttttagttta taggaggttt aggcgggttt ttaaaggata      9840
tataagtaaa atttttttgtt taaggggggt tattttaggg ttatggttgg ggtttaggtt      9900
tagttttatg ggtagattgg gttaggattc gatttgagag ggtttaggga agttttaagt      9960
tttgggtaag tttttttttt taggagttat atttttattt aaatgagtgt tttttatgag      10020
gagttttaag attttgtttg atttagcgtt ttggagggtt taggcgattt ttatggggaa      10080
ggttattgat tttggagatt gaagttttag tgtgcgtagt tcgagttatt agttttagtt      10140
tggaaggatt aggttttttt atatttgttg tttttataga tttttttcgg gtttattttg      10200
cgtttgtggg acgtgtattt ggtagaaggc gaataggcgt tgatgtcgat aataagaatc      10260
gtttttaagg tttagtagag taagtttacg tgtgtttagc ggggtttggg gagttttggg      10320
gttagatttc gattggttcg agggtagttt ttttatattg tttttatgat tttttgtttt      10380
ggtttagagg gaggtttggt taggtgggtt gggtaggata ttgtgatatc gagtttattt      10440
tttatatgat ttagatgaaa gtcgagagtg tggtgagtat ttttttgttt                 10490
```

<210> 87
<211> 10490
<212> DNA
<213> Artificial Sequence

<220>
<223> chemically treated genomic DNA (Homo sapiens)

<400> 87

```
ggatagggaa gtgtttatta tattttcgat ttttatttgg gttatgtggg ggatgggttc      60
ggtgttatag tgttttgttt agtttatttg gttagatttt tttttgggtt agaatagagg     120
attatgagga tagtgtgagg aagttgtttt cgggttagtc ggggtttgat tttagggttt     180
tttaggtttc gttgggtata cgtagattta ttttgttgaa ttttaaaggc gatttttgtt     240
atcggtatta acgtttgttc gtttttttatt agatatacgt tttataggcg tagggtgagt     300
tcgagagaga tttgtgggga tagtaggtgt gaaagaattt ggttttttta ggttggggtt     360
ggtggttcga gttgcgtata ttggggtttt agtttttaga gttagtgatt tttttttatga     420
gggtcgtttg agtttttttag gacgttgggt tagataaggt tttgaagttt tttatggggg     480
gtatttattt gagtggggat gtggtttttg gagagagggg tttgtttagg gtttgaggtt     540
tttttgagtt tttttaagtc gggttttggt ttagtttgtt tatgaggttg ggtttgagtt     600
ttagttatgg ttttgggatg atttttttttg ggtagagggt tttgtttgtg tgtttttttgg     660
ggattcgttt gagttttttg tgggttggga gtgagttaga ttttcgggtt ggggaagtag     720
ggtattgtag ggtaaggaag gtttttgagt tagggttttt ttatgttttt ttatttcgtt     780
aattaatatt cggatgaggt agtttaacgg ggaatattgt ttatatagat tttttttgtt     840
ttgatggaag taatagaggt gtttaggtta ttgggttgtt ttaaaaattt ttttttttta     900
gggtttttga agattttttt tttagtgtag aatattgggc ggtgtttaga gtttttttata     960
atattgttat tttttttatat tttcggtgga tatattgttt tttgttttgt tttgcgggag    1020
ttgggttttt atttttgtgt ttttgttttt tttagggtag gaaaggaaat taattttttag    1080
tttatggaga attcgacgtt ttaggttagg ttttggttgg gatttagtta gttattagtt    1140
ttacgagggg ttttagtttt tttgttttta tagttttacg ggaggtaggg tttttgggaa    1200
gagttgaggg gattataaat ttatttgatg ttttatggtt ttgggttgtg acgtggttat    1260
tatatgtttt tgttggtttt ggagttttttg gacggtttcg ttatttgggt tgtgaaattt    1320
tgagaagttt ttagtttatt atgaaattag agtttttttt taagatgtgg agttattagt    1380
tgtaagagtt gggtagttgg agaggttttt aaattttaag gttttttatt tttttatttg    1440
gtgattttaa tatgcggttt ttattttggg gaggtggggc gggaatattt tttggagttt    1500
ggttggaggt tttttttggag gttttttggg ttagggtgta aaaagggtaa gtttgatttt    1560
taggttacga tagggtggtc ggaattgggt gggcgttggg tttttcggtt atttttttggt    1620
agtggggtcg ggttagggaa taggggatgg ggagatgttg ttatttgggt ttggtcggtt    1680
tattcgtggg tatcgatggt agtaggagtt cgggtagttg gagggtagga ggatttttag    1740
ggaggggaga gttagttgta tagaattaga gtcggagggc gtggttttag gatatagagg    1800
gtggttacgg ggaggatgag atgttttttg ttgatgggga tgagaggcgt ttgatttggg    1860
ttttgggggt tagtcgtgga tttttgtggg atttttagta gagatatttt aaagtttttt    1920
aataagttgg cgatataagg agggtgtttt ggttgaaagt tgtgattatt tggttagggt    1980
ggttattttt aggtttggtt gtaggaggtt ttcggggtag ttgtttattt attttgtagg    2040
gagtgttttt tattggttag tagttgtatt agtgtttaga atgtattttt tttaggaaga    2100
tagaggagga ataaggcggc gatgtggttt aggtttttgt agtagtttat tttttgtaag    2160
agttagagtt attatggaag gatattattt gggagggttg aggttatttg ggaggattta    2220
tgttattgga gagggtagag gtgattggag aggttttttt tgaaggagag gtttttttttg    2280
aaaaagaggt tttttagga tgtatattta ttttatgata agagttaagt ttattaggta     2340
ttttagtatt ttgtttaaaa tgtttgttga tagtattatt ttgtgtgcga tgttgttaag    2400
tttttgggtt ttggggtagt tttaggagga gggcgttatt ttttgttttg agaagtggtg    2460
gttaggttta ggtgatatta ggagtttagg ttttgatttt tttgtgtttt agtttgattt    2520
tttgagatta ttttttttttt tggaggttta tgttagcggt gagttgatat tttatttttt    2580
atattttggt gggttataaa tattaatttt aaaagaagta acgatatttt tattagatat    2640
ttattttttgt taatatggaa atatggttcg ggaattttat tgtcgggaat atttatcggg    2700
ttgtattttt tatatgttag gaggatgtgg agtagtttttc gttgtttagg aaatagagaa    2760
agggggtttt ggtttgtttt gtgtagatgt tgttaatttt attttgttta taaagtttaa    2820
tagtaaattt tattttaggt ttagatgttt tattagataa gtagtgagtt ttttagggtt    2880
tgagattttt gaagaaatgt tttagtaaaa tttatatttg tgatatgtaa atagtttagt    2940
tgtatagtga tttgtttgat ttttttttatt ttgaatgatt tttttttttt tttttttagtt    3000
tgtatatacg ttagtttagt ttgtgggtgt atagtttttt tacggtttta aattaatgtg    3060
tagagttttt cggttatcgt tttagttttt tttggggcga ttttttttatt ttttaagttt    3120
ttagggtggt ttttatgtat ttagtttttt ttcgattcgt tagttttttgg ttatttagat    3180
tgttttttag tttttgtggt ttggtttttt ttagaatggt ttaggaatgg gaattttatt    3240
gtggtagttt attgggtttg gtttttgttt tttagtaaaa tgtatttagg atttatttac    3300
gttcgtgcgg gtattatcgg ttcgtttttt tttttattg ggtttttcgt ttgaagggag     3360
gattagtttt gttttttttta ttttcgtgtt gaaggtcgtt ttcgaaggtt tcgtgtgtga    3420
gtgacgagga gttaagtagt gaatttggta tgttggtttt atgtggatgt tagtttgtaa    3480
attagtgggt ttaatatttg tgatattttg gggatgtgtg gtttaagttt atcgagtttt    3540
```

gtgagttatt gtttaacggg ttgttaacgt ggttgtgtta tgttatgttt ttagcggatt 3600
tggatgagag tttttaggat ttttaatttt tttagtattt ggtgttgtta ttgttgtttg 3660
gggggggttt atgggttttt tattttgtta tttttcgtg ggttttatta tgggttttta 3720
tgggttaggg agagtatttt ttattattgt gtatgatttt gtttgttgtt ttttattttt 3780
ttaggatttt tttgggtttt ggttttatat gttttagttt ggtttagggt ttggaattag 3840
ggaggtgttc ggtttatggt gtcggttgtt ttttgggtcg ggagagtttt tggtagttgt 3900
gttatttttt ttgggtgatt ttggtttttg tttcggggaa gtttttattt tttttattta 3960
ttttattttt gttgggattt tgtggttttc gtaggtttat ttggtttcgt atcgattttt 4020
gaagaatata tgttttttta atgtttcgtt tacgtttcgg tcgatgcgtt ggatgtgttt 4080
agatgatttt ttgttttttt tttttatgat ttgtagggta gggttaagag gaggaagtag 4140
ttttagaata gatggaagat tttttgtttt tagtggtagt tagtttatag ttagtatttc 4200
gggaaggaag gatagaagga aggttttttt ttgtagaaag ttgtattttg gtttgttatt 4260
gaagttaggg agggttatta gagttgagtt tgtttgtggt gattgtgtta ttatttgtgt 4320
ttagggtgtt tatttgattt ttatttttag tttttttaggg tggttttgac gttttttttta 4380
gttggagatt tgggtttcga tacggtttgt tttgtttgtt gtgttttggt tgagcgtatt 4440
tggtattttt cggggttttt taattttatt tttttaatgt ttaggaggat tgattatatc 4500
gggtttcgga tgtttatggg tatttttttg tacgttcgat ttatgagttg tgggtagaaa 4560
acgatttggt gttataggtt acggggtgat tttagtgagg attagagttc ggggattttg 4620
gaaattgtcg gttttggttt tatgattttt tagtagaggt gagattaagt tgggataggg 4680
tttttttttt taggattgaa agagtggatg gatatttaga gtcgaaattt tgatttgaat 4740
tttttttttt ttttaggtta ttagggtatt tttagttttg agtttcgggt agttttagtt 4800
ttagatttag attttttttt tgtaaggtga cgtttgtacg aataggtagg aaatttggcg 4860
attaggtttg tagtttttttg ggcgaggata gtgtgtcgtt tattttttga gaggttgatg 4920
gtgttaggtt atagttatgg gtgtttgttt tttgttttttg tagagagtgt tttttttttt 4980
tatacgttat tttttttgttg tttttgtatt tttttttagtt ttttagtata tttatttatt 5040
tgtttttttcg gttgattttt cgtcgaattt gttgttaaat gaggtatgtt ggagttagcg 5100
gagttgttag gtttttttaga gtcgttcgcg gatgttgggt tttgggtttt ggagttttgg 5160
tgggagttag ttggaaggag ttagggaagg gtagatttta agggttgaga gtttttgagt 5220
aaatgagtat tagtgggttg gttttgggat ttcgggatgt attattttta ggttatagat 5280
atattagttt taggttttag tttttaggtg gggttttgat ataagcgcgt agttattttt 5340
aagttaggat tgtggttttt tttttggaat tttattaaat tgttaaagtg aatagtaatt 5400
tggggttagg tttagtaggg attgttgttt tttttagtga tagcgtgttg ttttttattcg 5460
ttatcgttta ggttagttgt ttttttttgtt ttattgatta ttcgtttagt ttttacgttt 5520
ttggattagt tttttttacgt attaggtttt tattttcgtt tttcgcgtag ttagaggagg 5580
tagtttcgtt ttattgtaag gtaatttagg tagagttgag gaattgtacg gggtttggag 5640
cggttttagt ttgggtgtcg atttttagaa aggattggtt ttgttttttt ttagtttagg 5700
gtttagttta ggagaaggta tagggaaggg aggataaggg tttttttgtg gggttgattt 5760
ttaggagggg taggatttgg gagaagaagg agtgtaggga tagtttggtc ggggttattg 5820
gggtttttgg cgtgggggc ggttaggttg tttagtgggg ttgttcgttt tggattcgag 5880
gtggtgtttt ttgttggagt tgagaaaggt tagttttgag atgggatggg ggtcgtttag 5940
ggtgggcgat cgggttttga taggagtttt ttagggagtg attatatttt ttcgttaggg 6000
ttaagggagt ttgttttgag atttgttcgg tgtattttgg ttgtattagg ggtttatttt 6060
atttgatagt tttaaggttt ttgtaggttt tgatttttta gtatttattt gttttttttt 6120
gtattcgagt tatatatttt gcgttttaga agtggtacgg ttcgttagtt ttttttcgtt 6180
ttattttttt aggattttt tgttttttta ttttgtgatt tttgagggat gggtttttgg 6240
ttgggttttt tttattcggt tttagatttt tttttagtat tagatttagg tttttagtcg 6300
cgagttttgt tgttttttttg gttttatcgt gagatgttta gaacggggtt ttgtttattt 6360
tttttttcgt tttttttaggg ttatagtttt tattgttatt atgttttttt tttttacggg 6420
atagtgaggg ttgtagtttt aggggaatgg gggagaatag gggtaggtgg gtttttagag 6480
atttgttgga taatagtttt gaggttgggt taggcgtttt tttattttgt ggttataatt 6540
tttgtatttt atcggggttg tttttaagta gatagggtta gatttttagg ttgtttcgtt 6600
tttttttgtgt ttatttgtcg atagaattgt tgagcgttta ggggtttgat ttagtttagt 6660
ttttattttt atcggttttt tagatgggtt ttatattttt ggtttaataa tttcgggttg 6720
gatttgtagg ggagttaggg aggagtttttg tttttggaaa ggaggttgat tcgatttggt 6780
gagatatgtt ttgcgttaga aaggtttttt taaaagtaaa tttattttttg agttgagata 6840
ggtgttttag ggggtgagggg agtgtagagg atttattgta taattttttaa atgatcgacg 6900
ttgttgttgt agtttcgaaa aggtttaggt tttttgtttt ttggtagttt agttcggtgt 6960
ttttgtagtt tagagggagt tttggtgagg ggtttaaggt aaagggtgta agggtttggg 7020
ggtattggtt attcgttttt gttttgtgtt tttagggagt ttaggatttt ttgattaggg 7080
tatattggaa gaggtttttt tttaagaagt agatcgattt gtattttttc gtattttata 7140
atgatgtttt ttcgttttttg tgtttattaa ttgttcgcga tttttattac gtttattttg 7200
tgagatagaa ttgtttaaag gttatattgt acgcggcggt ttcggagaat atttgaatcg 7260
ttttcgtcgg gtttttagat gttggttggt tgcgtaattt ttattttatc gtcgttttta 7320

gggaaaaagg ggttagattt agtggtttat agttgtttta gtttttggag ttttaagttt 7380
taagtagggg tgggtatttt tttaaggatt tgagtatagt gggatttaga tagagaattt 7440
tgttgttttt taatgttatg aaatggggat atatcggttt tagtaggttg aatggttttt 7500
atttgttaag ggtgaagggt ttatgatggg ttattttagg gatgtggagg tagattgggg 7560
ttagcgatta gaggtttttg tgtaatcggt tttttgggat gtttagggtt ttagcgatgt 7620
ttagtttttt atagggaata agattttttt cgattgttcg gttttatttc gtttattatt 7680
ttggttatcg tggtttttta gtttgaatag tgaagttatt ttaggaataa cgtttgttga 7740
gtaggagggt gttgggtttg ggggatgaga aagatttatt gtacgtatgg aaattacgtt 7800
tttcgcggag ggattgtgga gtttattatt ttgagtcgtt ttaataggag gaggttttat 7860
tttttttgggt tattgaggaa gaatagtggg tttttggttt tggagaatag ttggatggat 7920
cgtttttttt gggaatattc gaggtaaaag gagggcgagg ttttaagagg attacgtaga 7980
gtaagaaata tttgggggaga atttagtgt tcggattttt ttgaatataa gggaagatag 8040
tttttttttta gttagttttt tagggttttt ttattttta tagttgttta agggtagtag 8100
gttttttttgg ataagggatt atgtgtgttt agtggggttt atagcgatta ttaggattta 8160
gtttagggta tagaggtgtt ttgaggatcg ttagtggatt tgtattagtg gttttatatt 8220
agtggttttg ttaggattag gttttgtttt atcgggatgg gaaatttggg tagatttggg 8280
atttagggta gggaggttat agggtttagg tttgaatttt agtatagtat acggtagggt 8340
tgagagtaaa atttagggtt atgttcggat ttttaggtcg gttattgttt ttttgatttt 8400
agacgtttta tttgtcgaat ggggatattt gggaatagta tttattttac gaagttatta 8460
tggagacgaa agagttaatc gtttatacgg gtagtgtaga acgggcgttt ggtgagtgtt 8520
tagggatgat ttttttcggt agttgtttta tagaggttaa tatcgttcgt atcgtagtta 8580
ttgtttttaa gttcgtttgg agggaagaga gtaggttacg tttatttgat tttgatgaat 8640
tagttggttt gggttatgtt ttttagggag aaaatttttg agtttataga gttgtttata 8700
gatattattg tttgtgtgta attgttgtag attattgagg taggttagag agtagatagg 8760
tgttaagtat tagtgatatt ttgaggttat ggtacgaatt atagtggggt tttgttcggg 8820
ttagtagcgt ttagagttag ggtttttcgt tgtttgaggc gttaatatgt ttgtttgtaa 8880
tgtgtttgtg tacgtgcgtg tatatgtgta tgtgggtaaa tacgtttgtg tacgtgtgtg 8940
ttgttttttt ggttaggttc ggttgtttta tttatgtgtg tatttagttt tttattattg 9000
ttattttcga ggtttagggt tagtattaga gtatttatgg ttgtttttta atcgtagttt 9060
ttttcgttta gggtggtttt gggatatata tagcggtgga gggaagtgat tgttgttatt 9120
ggattttaga atataaaagt taatattatt atttaatggt ttttttagtg tttttaatgg 9180
tattattttt ttttttttga tattttaatt gggtattttt ttttatgatt tttggatatt 9240
ttagttagag gattttgtgg ggaaagtgtc gggtatatag taggggttta ttttttttaga 9300
tatgttattt aaaatttggt ttatttgttt ttttacgtag ggtttagggg atgtcgaatt 9360
ttagggttta gaaagagttt gggataaaaa gaaattttaa ggggacggtt ttgatttggg 9420
ttgagtttat ttgtgttatt taattggagt tttaagtttt gaggtaggac gtttagatgt 9480
tttagtgtag ggtttttttg attaatattt gttttttttgt atttattagt aattttattt 9540
attttatttt taaagtatat ttggttttcg tatttaggag ttttgtattt gtagatttag 9600
taatagtaga tggaaaatat ttagaaaata aattggacgg ttatgttttt attgaatatg 9660
tgtagatttt gtttttgtta ttattttta aagaatatag tattacgatt atttatgtag 9720
tatttgtatt gtattatata ttataaataa tttagtaacg gtttaacgta tacgggagga 9780
tgtgtatagt ttatacgtaa atattaggtt acgttatatt agagatttga gtatttatgg 9840
atttggtat tttcggggat tttagaatta attttttatg gatattaagg gatgattgta 9900
tatatttatt taggaaggtt ttttattgga ggaaaggttc ggtttaggat agatagggat 9960
attattttttg gattattgtt tatttattta tttatttatt ggtattattt tttaggattg 10020
ttttaatgat agttttagta agtggagata agaaaaaaga ttggtttaaa tggtatagtt 10080
ttgaggtttt ggaagatgtt gtattagtat gagaataggg ggttagtttt ttttaggatt 10140
tagaaagtat attaggtagg ttcggggaga ggaaaggaat acggtttttt tagtagttat 10200
ataggtttgt agtaggatgg ggttggggtt ggggttggga ttggggttgg tttcgtttat 10260
tatttgggtt ttatgagggg aaaagaaaga atagggggta gaggaggagt atgggggtag 10320
cgggttgttt aaggagaagg cgttttaggg aaggggtatt agtttgtttt tatattgtta 10380
taaagaaata tttgagttta ggtaatttat aaaggaaaga ggtttaatcg atttttagtt 10440
tagggaattt atagttatgg tagaaggcga aggggaagta ggtatttttt 10490

<210> 88
<211> 7001
<212> DNA
<213> Artificial Sequence

<220>
<223> chemically treated genomic DNA (Homo sapiens)

<400> 88

```
atgatattag taattgttta gtgaggcgga tataaaattt ttaggatatg agagggagac   60
gtggttttta tattttgatg tgtaaatatt acgtttaggg aaaatgtaag gtgttttagg  120
tttgtggatt ttgtattttt ttaggtaatt tatttattta tttttaatt ttaataaatg   180
attattaaat tttatttaat atataaatat ttattgagta ttatttgtgt gtatgagaag  240
tgggagttag tatggtaaaa gttaggtatt gtgttaggtg agagagattt agaaattaaa  300
attagagaag ttattaataa gagtttaaat atttgggttt aggtttatgt ttgtaatttt  360
agtattttgg gaggttgaag gaggtgaatt atttgaggtt aggagtttaa gattagtttg  420
attaaaatgg tgaagtttta tttttattaa aaatataaaa aattaggcgg gtattgtggt  480
atacgtttgt aattttagtt atttgggagg ttgaggtagg agaattattt gaatttagga  540
ggtagaggtt gtagtgagtt aagatcgtat tattgtattt tagtttgagt gatagagtaa  600
gattttattt taaaaaaaaa aaaaaaagag tttaaggatt tgatggagga gaaaggtaag  660
aatatgtgcg agataacgta aggttatcgt tttagggtgt ttagggtaat tacgggggta  720
gggtattttt cggagaggtt aatgataagt aggttgaata aagtaggggg gtttttttgta 780
ggaggaggtt tattaggtga agatggagtc gtatgggtaa aggttatttt agagatttag  840
gtgtgtttag gaggtggaaa tttattgtag gtaaggtgag aggatcgggt ggggtggttt  900
aggaggagtc gatagagggt ataagttgtg aaatagtttg aagtagggta gtgaggaaag  960
ggatttagag gaggaagata cgtggataga tggggttggt tgggggttgt cgtaggattt 1020
tatgtaagag gttttaatat tagagtttta ggttttgagt ttcgcggaat taaaggtttt 1080
agaaagtaat ttattaggat ttggtggttg atagtttgta gtagggggtg aaagaggagt 1140
ttagagtatt tttcggtttt tgtttttgtt ttggggtata ggaggggagg aatttagttt 1200
ggttatattg gtttaggtga gggcgtttta ggggaggtcg agaggggttg tttttttgtt 1260
tgttttgtta ttagggattg tttcgagatg tttttggaga aagtgttttt ggtttgttgg 1320
gaaggattcg tgtttagttt tcgttgttta gtagttttta tgggaatttt gtttttttgg 1380
ggtttggatt tatcgcgacg tgaaggtgat tatcgtttat tttcgggata ttgtgggggt 1440
taagagaggt ttcgtggtga gggaggatta ttattttggg ggtcgggggg gttttttttt 1500
tagggaggaa gatttttagt ttcggtgttt tgttttcggt agattttgtt ttgtttattc 1560
gttttgtttt tgtcgtatga tggaaataaa tggaaatggt ttttatatac gaatgtatta 1620
aattgtaatt ataatttttt agattttagt tgtaatagga ttatggattt gagtgattcg 1680
taaagacgtt ttgagttttg agttagaggg tgtgtggggt ggggaggggga gtttgttacg 1740
gtttttgtga ttttatagta taggggggtag agttggggggt tggggtgggg gtaagggcgt 1800
aggtagatgg gtttgggggt ggttagtacg gggatttatt tagttcgttt tgtatatcga 1860
ggttattttt tttttcgggt tttaaagttt tttcgttttt tgttatgggt ttgggggttt 1920
ttttattgta gtttaatgtt ggttgttttt ttacgttgtt taagtttatt ttttaggttc 1980
ggtattttat agtagagatt aagaggtggt tggagggtag tgggggtacg gatagtatta 2040
ttgggcgttt ttttttttagg ttttttttgga aatttttgtt ttggaaacgt agaaagtttt 2100
ttttttttgtt tttattttga aattgtattt aataacggta aataagttat ttagtttttt 2160
atagttggta aagtgtaagt cgtagatgtt gttatatagt atttttttgat agttttaggg 2220
tagatcgttg atttattttg taggtaaagg agttgagatg taaatatttt ttaaggaagt 2280
aagaatggtt ttttttttta tttttttagaa ggatttaggg taatcgattt atttgataat 2340
attagggaat atgggttgtt agttacgtat ttatgagaga ggtggttttg atttttagag 2400
tatggatttt aggggagttt aggaatttgt aggagagggt tttttagttg gttattattg 2460
ggacgggtat tcgggtttgt gggagagggt tttttaggtg gttattattg ggatcggtat 2520
ttgggtttat ttggatgggt tttgggaggg tttgtttttg ggggagatgt tggtatgaat 2580
agttaaaagt attattttga gattttacgt taatattttt tttttgaaat atgatatttg 2640
ggaggattgt atagtttttt taatatagga aaatagtttc gattgaaggt agtttttttt 2700
tcgtcgtatt tttcgaaggt tttcgggggt tttgttattt tgagttattt tttatttgtt 2760
tttgtcgatt ttgtaaattg gatatatttt taagggttta gaatagtatt tggtataaaa 2820
taggtgttta aaaatatgta tgtaaaatag tggtttttgg cggaggttgt tagagttggt 2880
tgtggtttta tagagtagga agaagtacgt tttatttgtt tgggtttcgg ttagggtgtc 2940
gagggttagt atggatatta ggattagggc gtagattatt ttgtttttta tggtggttat 3000
tgtttttttt ttgttttaaa ggcgatttcg agttagggat gagaggtcgt tcgagtttcg 3060
gattttatag ggtaggtttt gtttgtttaa agagcgttag ataatatttg tttaaggagg 3120
ttcggggatt ttttgagata ataattttta ttgattttta ttaaaggtgt tttttagata 3180
tggttaagtt atatggaagg atttgttgat agatagagac gatatgtggt gaggttattt 3240
tggttgaggg atttgagatt tagaaagttt ttttttttta tgggagtttt ttttaatttg 3300
attttaattt aggttttatt tatatttgag aggtttttttc gttagggtaa atattgtatt 3360
tattgtagaa gtgatttata gtgagagatg gtcggaaaaa ggtttggtcg tgataatagt 3420
ggtttacggg gtggttatcg tgatttgta gggggaaggg aaggagttta tgaagtttat 3480
ttcgtttagg tttaagttaa tttttttata gtggaattgt tttaataatg aaattgtagg 3540
tttggcgtag tggtttacgt ttgtaatttt aatattttgg gaggttaaag taggcggatt 3600
atttaaagtt aggagtttga gattagtttg gttaatatgg cgaaatttcg tttttataaa 3660
aaaaaaaaat ataaaaatta gttaggtatg gtggcgggtg tttgtaattt tagttatttt 3720
```

```
ggaggttaag ttaggagaat tatttgaatt tgggaggcgg aggttgtagt gagtcgagat 3780
tattttattg tattttagtt tgagcgatag attaagtttt tttttgggag gggttggggg 3840
taggagggag aaaaaaatag tatatacgag ttgttattaa aaattgataa agtgaacgtg 3900
gtttattttt ttgtgtttat ggggtttttt tttttttttt tttatatttt tttagttggt 3960
tttttggatt tttgtatttt agaggatagt tttttgtttt tttgtattat gttttatttt 4020
ttttttttgt tcggagtttt ttttttttata gtttttatta ttttttggtg gataaaataa 4080
gtgattgata tattatgttg gttatttatt tattgtattt ttttttgtag tagacgggag 4140
ggcgtatggg agtagaagtt tttatttttt agtgttttga ggtggtttta gattttagaa 4200
tagtggtttt gattggtatt cgagaaatag ttgttgaaga gttgtatgaa gaaatggata 4260
ataggagaaa tttttttttt tttagtgaga aattaggttt tgaggaaaat gtatgttagt 4320
ttaataaagg tattattttt ttttgggtga tttagttagt atatttttgt agtttttttg 4380
ggaggggagt ttgggttaga atgggggcgg ttatgttttt tttcgtttta ttttaggatt 4440
tagttttttt tttagggttg ttatttttgt atcggggttt ttatttttgg gttagggttt 4500
ttaggagggg tttgatcgta ggatttaggg aggggtcgag gtttggtgtt ttttgtggtt 4560
agtttaagtt tattgaaaat aggacggggg tattaggttt agggtggtgt ttatagtgac 4620
ggtggttttc gttgttttaa tttttcggat taaaatgggg gtttttggaa atgggatgta 4680
aattgtataa ttatttggag aaaaggtggg tagtgttcgg tgaggtttaa gttatataga 4740
ttttatagtt tagtaaattt ttttgttttt aggtttggtt ttttaatttt agaaatatgt 4800
ttatatttgg gtagaagaaa atataggtaa agatattttt tttagtattg tttgtaatag 4860
ttggaaatag ggaaagaagg aaggaaggaa aggagggaaa gaaaagtaaa ataaatattt 4920
attaattagg gaaagggaaa atgaatttaa aatataatgt agtcgttttt tagtattttt 4980
gggggattgg ttttaggatt ttttatttag ggatgttaaa atttataaat gtttaagttt 5040
ttggtataaa atgatatagt gcgtagaaat tacgtgtatt ttttttttata ttttaattat 5100
ttttaggtta gttataatat ttgatgtaat atttatattt tattttattt tgtagatttt 5160
atatagtatt tgatttgcgg tatatttaag ttttgatttt taagatgttg taaataagat 5220
tttaaaatat tttttttttt tgagatagag ttttgttttg tcgtttaggt tggagtgcgg 5280
tggtatgatt ttagtttatt gtaattttta ttttttaggt ttaagtgatt tttttgtttt 5340
agtttttttga gtagttggga ttataggtat gtgttattac gtttagttaa tttttgtgtt 5400
tttagtagag atagggtttt attatgttgg ttaggttggt ttcgaatttt tgattttagg 5460
tgatgtattc gttttagttt tttaaagtgt tgggattata agtgtgagtt attgtgttta 5520
gttggatttt aaaatagttt tgatttgata ttattggaat ttatggatgt agaatttatg 5580
aatatagaga attaattgta tatatgtatt atattttata tatattatat atgtatgtat 5640
gtgagtattt tttagaagat gaaaatgaat aaataatgag aaatattgaa aataaagcga 5700
agtgaaaaaa ttcgttaaaa tggtttgtaa agtaggatat tatttataat gtagattttt 5760
aaattatgtg aaataatgtg atatattgtt ggaattgtat ttagatgaat aaaatgatga 5820
tagtgtagat aggaacgaga tattgatttt agtatgatgg gtttgtttag gaaggaggaa 5880
gtaggaaaag gtggcggggt agggcgtaaa ggggttttcg atgttttcgt aatgtttttt 5940
tttgttgaag aaaatttgag ggaaataagg taaaaaggtt agtatttatt aatgaggggt 6000
agcgtagatt tacggatgtt gttttagtat tttgtatatg tttgtaattg tttttttgga 6060
aagtttagtt tttatttttt tgggagagtt gtgagataat tattaagtgt cggagtttgg 6120
tttttttatat tagggttgga gcggttggag ttgttttcgg gatttttcgt ttttcgggat 6180
aagagtttta tttttgtgag agtgggcgga atttaagatt atattatata taattttatt 6240
tataaaaatt tgttgaagat aattttagtg tggcgaggga gagaatgtag aggtaagcgt 6300
tagtttatta ttagtggggg attttgtgtt agtattttta tagttttggg ttttagtttt 6360
tttatttgaa agacgtagtg gttgggttat aggatatttg aagatttcgt attttagatt 6420
tttttcggta ggtatttatg gtcgtggggg cgtttgtggt tgtgtaggta ttgtttgttt 6480
tttgcgttta gtcgattttt ttgcggtttg gcgagagttt tttatttttta gtggggggtt 6540
tcgtagtcgt ttagattttt tataaggggg acgcgtagtt ggggagttag ttttttttttg 6600
acgtttttta tttgttcgtt gtttttgatt tgtgttttgg taggaggggg ttttgtaggt 6660
ggttttggag ggagggagga gtttgggagg aagtggattc gggttttttt ttaggatttt 6720
ttttgggatt atttgggata ggttacgttt tttttttagt tatagtttag gtgggcgttt 6780
tttttatata ttattttttt taggttttag agtagatttt ttattttttt gttttagttt 6840
gggggggttta ttagtcgtaa ggagattttt atagttttta tttttttcgt attgtttaga 6900
ttcgttgtgg tgaagttcgt tttttgttag gtttttggtt aaaaaagtaa ttgttatttt 6960
agggattttt tataaggata tttagttgag gttttggggt t 7001
```

<210> 89
<211> 7001
<212> DNA
<213> Artificial Sequence

<220>
<223> chemically treated genomic DNA (Homo sapiens)

<400> 89

```
aattttaggg ttttagttgg gtgtttttgt aaagggtttt tggggtggta gttgtttttt     60
tgattagagg tttggtagga aacgggtttt attatagcgg atttgggtaa tgcgaggaga    120
gtgaagattg taaaagtttt tttgcggttg gtaaattttt taggttgggg taggggggtg    180
aggggtttgt tttggggttt ggggaagatg gtgtgtggag aggacgttta tttgggttgt    240
ggttgagagg gggacgtggt ttgttttagg tgattttagg agggattttg gaggagagtt    300
cggatttatt ttttttaaa ttttttttt tttttagggt tatttataga atttttttt        360
gttaaggtat aggttagggg tagcgggtag gtggggagcg ttaggaggag attgattttt    420
taattgcgcg ttttttttgt ggggaatttg ggcggttgcg gggtttttta ttgaaaatga    480
ggggttttcg ttagatcgta gggaggtcgg ttgggcgtag gaaataagta atgtttgtat    540
agttatagac gtttttacgg ttatagatgt ttgtcggaga gggtttgagg tgcggagttt    600
ttagatgttt tgtggtttag ttattgcgtt ttttagataa gaaaattaag atttagagtt    660
gtgaaaatgt tggtataggg tttttttattg atgatggatt ggcgtttgtt tttgtatttt    720
ttttttcgtt atattggaat tgtttttaat aagtttttat ggataaggtt atgtatggtg    780
tggttttggg tttcgtttat ttttataggg ataaagtttt tgtttcgaga aacggagagt    840
ttcgggggta gttttagtcg ttttaatttt aatgtgggag gttaaatttc ggtatttggt    900
agttatttta tagttttttt aaggagatag gagttgggtt ttttaagaaa gtaattgtaa    960
atatgtatag aatgttagaa taatattcgt gaatttacgt tgttttttat taatggatgt   1020
taattttttt attttatttt ttttagattt tttttaataa aagaaaatat tacggagata   1080
tcgaaggttt ttttgcgttt tatttcgtta ttttttttg tttttttttt tttagataga    1140
tttattatgt tgaagttagt gtttcgtttt tgtttatatt gttattattt tgtttatttg   1200
gatgtagttt taatagtata ttatattgtt ttatatagtt tggaaattta tattataaat   1260
gatattttat tttataaatt attttaacgg atttttttat ttcgttttgt ttttaatatt   1320
ttttattatt tatttatttt tattttttga aagatattta tatatatata tatataatat   1380
atgtaaagta taatatatgt atgtaattag tttttttatat ttatgggttt tatatttatg  1440
gattttagta atgttagatt aaaattgttt taaaatttag ttgggtatag tggtttatat   1500
ttgtaatttt agtattttgg gaggttgagg cgggtgtatt atttgaggtt aggagttcga   1560
gattagtttg gttaatatgg tgaagttttg tttttattaa aaatataaaa attagttggg   1620
cgtggtggta tatgtttgta attttagtta tttaggaggt tgaggtagga gaattattta   1680
aatttgggag gtggaggttg tagtaagttg agattatgtt atcgtatttt agtttgggcg   1740
atagagtaag attttgtttt aaaaaaaaaa agtattttaa aattttattt ataatatttt   1800
aaagattaaa atttgggtgt gtcgtaagtt aagtattatg tagaatttgt agaatgaaat   1860
gaggtgtagg tattgtatta ggtattataa ttaatttaga gatggttaaa gtgtaggaga   1920
ggatgtacgt ggtttttgcg tattatgtta ttttatatta gggatttgag tatttgtgga   1980
ttttggtatt tttgggtggg gggtttgaa attagttttt taggggtatt aagggacgat    2040
tgtattgtat tttaagttta tttttttttt ttttggttaa tgaatattta ttttgttttt   2100
tttttttttt tttttttttt tttttttttt tttgttttta gttgttataa ataatgttaa   2160
aaagaatatt tttgtttgtg tttttttttg tttaagtatg aatatgtttt tgggattaag   2220
agattaggtt tggaaataga agaatttgtt gagttgtaaa atttgtatgg tttaaatttt   2280
atcgaatatt gtttattttt tttttaaatg attgtgtaat ttatatttta tttttaagag   2340
tttttatttt gattcgagaa attagagtag cgaaaattat cgttattatg agtattattt   2400
tgggtttggt gttttcgttt tgtttttaat aaatttaagt tgattataga ggatattagg   2460
tttcggtttt tttttgggtt ttgcggttag gttttttttg gagattttgg tttaggagtg   2520
gagatttcgg tgtaggagtg gtagttttgg aggaggggtt gggttttggg atggagcgaa   2580
gaggaatatg gtcgttttta ttttggttta ggtttttttt ttagaggggt tgtagaaatg   2640
tattgattag gttatttaag aaaagatagt atttttgtta ggttagtatg tatttttttt   2700
agggtttaat tttttattga agaagaaaag attttttttg ttgtttattt ttttatgtag   2760
ttttttaata gttgtttttc gaatgttaat taaagttatt gttttagggt ttggggttat   2820
tttaaggtat taggagatga ggattttttgt ttttatgcgt tttttcgttt gttgtaggga  2880
ggagtgtaat gaataaataa ttaatataat gtgttagtta tttgttttat ttattaggag   2940
gtaataagag ttatgaaaga gaaagtttcg agtaggggag gggagtgagg tatggtatag   3000
gagagtagga ggttgttttt taaaatatag gagtttaggg gattaattgg gaaggtgtgg   3060
gagggggagg gagggagttt tatagatata ggggagtgaa ttacgtttat tttgttagtt   3120
tttgatggta gttcgtatat attatttttt tttttttttt gtttttagtt ttttttagaa   3180
ggagatttaa tttgtcgttt aggttggagt gtagtagggt gatttcgatt tattgtaatt   3240
ttcgtttttt aggtttaagt gatttttttg atttaatttt tagagtagtt aggattatag   3300
gtattcgtta ttatgtttgg ttaattttttg tatttttttt ttttgtagag acggggtttc  3360
gttatgttgg ttaggttagt tttaaatttt tgattttaag tgattcgttt gttttggttt   3420
tttaaagtgt tgggattata ggcgtgagtt attgcgttag gtttataatt ttattattaa   3480
aataatttta ttgtaaaaga attagtttag gtttagacgg aatgggtttt atgagttttt   3540
tttttttttt ttgtaaggtt acggtggtta tttcgtgagt tattgttgtt acggttaagt   3600
```

ttttttttcgg ttatttttta ttatgaatta tttttgtagt gagtatagta tttattttgg 3660
cgggagggtt ttttagatat gagtaggatt tggattaagg ttaggttgga ggagattttt 3720
atgggaaaga gggatttttt gaattttaga ttttttagtt aagatgattt tattatatgt 3780
cgtttttgtt tattagtaaa tttttttatg tagtttgatt atgtttagga aatatttttg 3840
ataaaaatta gtggagatta ttgttttaga ggatttcgg gtttttttag gtaaatgtta 3900
tttaacgttt tttaagtaaa tagagtttgt tttataaaat tcggggttcg ggcggttttt 3960
tatttttgat tcggggtcgt ttttggagta gagaggaggt aatggttatt atggagaata 4020
aggtgatttg cgttttggtt ttggtgttta tgttggtttt cggtattttg gtcgaggttt 4080
agataggtaa ggcgtgtttt tttttgtttt gtggggttat agttagtttt ggtagttttc 4140
gttaggagtt attgttttat atatatattt ttgagtattt gttttgtgtt aggtgttgtt 4200
ttaggttttt aaaagtatat ttaatttata ggatcggtaa aagtaggtgg agagtaattt 4260
agggtggtag ggttttcgga gattttcgag aagtgcgacg aggagggggt tgtttttagt 4320
cggggttgtt tttttgtgtt aggaagatta tataattttt ttaagtgtta tgttttaaag 4380
aggaagtgtt ggcgtggggt tttagaatag tgtttttgat tgtttatgtt aatatttttt 4440
ttaggggtag atttttttaa ggtttattta gataggttta aatgtcggtt ttagtgatgg 4500
ttatttggga gatttttttt tataggttcg aatgttcgtt ttagtggtgg ttaattggga 4560
gatttttttt tataggtttt tgggtttttt tgggatttat gttttgggag ttaaagttat 4620
tttttttatg agtgcgtggt tggtaattta tattttttgg tgttgttaag tggatcggtt 4680
gttttgggtt tttttaggga gtggaggagg aggttatttt tgtttttttg ggaagtgttt 4740
gtattttaat tttttttattt gtagaatgga ttaacggttt gttttagggt tgttaggaaa 4800
tgttgtgtgg tagtatttgc gatttgtatt ttgttagttg tggggagttg aataatttat 4860
ttgtcgttat taggtatagt tttaaggtgg gggtaggaga aagggttttt tacgttttta 4920
aagtaagggt ttttagagag gtttgaagag ggagcgttta gtggtgttgt tcgtgttttt 4980
attgtttttt agttattttt tgatttttgt tgtggggtat cgggtttgag gggtgggttt 5040
gggtagcgta gaagagtagt tagtattggg ttgtagtggg aagattttta agtttatggt 5100
agggagcggg ggagttttgg aattcgagag aggaagtggt ttcggtgtat agaacgaatt 5160
gggtgggttt tcgtgttggt tatttttagg tttatttgtt tgcgtttttg tttttatttt 5220
agtttttagt tttgtttttt gtgttgtggg attatagagg tcgtggtaaa tttttttttt 5280
tattttatat atttttggt ttaaggttta gagcgttttt gcgggttatt taggtttatg 5340
atttgttat aattgaaatt tagaaaattg tgattatagt ttagtgtatt cgtgtgtgga 5400
aattatttt atttatttt attatgcgat aaagataaag cgggtgggta agatagagtt 5460
tgtcggaggt agagtatcgg ggttggaaat tttttttttt gaggaggaaa tttttcgat 5520
ttttaggatg atgatttttt tttattacgg ggtttttttt gatttttata gtgtttcggg 5580
ggtgggcgat gattattttt acgtcgcgat ggatttagat tttaggaggg taaggttttt 5640
atggaagttg ttgggtagcg ggagttgaat acggattttt tttagtaagt taggaatatt 5700
ttttttaaag atatttcgag gtagtttttg atagtaaagt agataagaga atagtttttt 5760
tcggtttttt ttggggcgtt tttatttgag ttagtgtggt tagattgagt tttttttttt 5820
ttatgtttta aggtagggat agggatcgga gggtgttttg ggtttttttt ttattttttg 5880
ttgtaggttg ttaattatta gattttaata ggttgttttt tgagattttt gatttcgcgg 5940
agtttagagt ttgaagtttt ggtgttagaa ttttttgtat aagattttgc ggtagttttt 6000
agttagtttt atttgtttac gtgttttttt tttttagatt ttttttttta ttgttttgtt 6060
ttaagttgtt ttatagtttg tattttttgt cggttttttt tagattattt tattcggttt 6120
ttttatttta tttgtaatgg gtttttattt tttgaatata tttgggtttt tggaatggtt 6180
tttgtttatg cggttttatt tttatttggt gaattttttt ttgtagggag tttttttgtt 6240
ttgtttaatt tgtttgttat tggtttttc ggggagtgtt ttattttcgt ggttattttg 6300
ggtattttgg gacgatggtt ttgcgttgtt tcgtatatgt ttttgttttt ttttttttatt 6360
agattttttag attttttttt tttttttttt gagatggagt tttgttttgt tatttaggtt 6420
ggagtgtaat ggtgcgattt tggtttatta taatttttgt tttttgggtt taagtgattt 6480
ttttgtttta gtttttttaag tagttgggat tatagacgtg tgttataatg ttcgtttaat 6540
tttttgtatt tttagtagag atggggtttt attattttgg ttaggttggt tttgaatttt 6600
tgattttaag tgatttattt tttttagttt ttttaaagtgt tgggattata ggtatgagtt 6660
tgggtttaga tatttagatt tttattaatg atttttttgg ttttaatttt tgggtttttt 6720
ttatttggta tagtgtttgg tttttgttat gttagttttt atttttttatg tatataaatg 6780
gtgtttagta aatatttatg tattgagtaa aatttaataa ttatttgttg aaattaaaaa 6840
gtgaataaat aagttattta gaaagatgta aagtttataa atttggggta ttttgtattt 6900
tttttgagcg taatgtttgt atattaggat gtgaggatta cgtttttttt ttatgttttg 6960
agggttttat attcgtttta ttggatagtt gttgatgtta t 7001

<210> 90
<211> 6499
<212> DNA

<213> Artificial Sequence

<220>
<223> chemically treated genomic DNA (Homo sapiens)

<400> 90

```
tattgtatat ttttatttat aattggtagt taattattga atatatatag atataaatgt 60
attttaatat attattattt ttatttagat tttgtgtaat atttaattat ttaattatat 120
taataaataa aagtttttag aaattgatgt atttagtata attaaggggt aatttgttta 180
tgattatagg agttatttta tttatattaa tttgatgtga ggagataatt aaattttttt 240
tttttataa gaaaaattta attttgggag gtttaggtag gagaattttt tgaatttagg 300
aggtagaagt tgtagtgagt tgagattgta ttattgtatt ttagtttggg taataagagt 360
gaaattttgt tttaaaaaaa taaataaata aaataaaata aaaagtaaaa tttaattttt 420
atattttttt tttaaaaata ttatattatt gaaatagaat agaattttgg attttaatgt 480
ttttatgttt tttgataatg taaagattat attaagaata atattgattt tttgtgtata 540
tgtgtgaaat tatgttgagt tgatgtattt agaaaagaaa ttgtagtgat attgggtata 600
gaatttttaa ttttaatttt tttttgttg tggaatagaa atttagtttt atttttttg 660
tttataatat ataagagtag ataattagat atgaggtgat taaatatata aattgatttt 720
atagttatta tggaaagatt ataatatgtt tggatgaggt gggggaaaga gttttttttt 780
gaattgagat ttgaataggt tttagataga ttaaaaattt atggtttgtt tttttaatt 840
ttttattatt gattatttgg gagaattaaa atggtttgga ttataaagtg attataatta 900
gtagggtagg tatattaaat aaaaatgggg tgagtgtgtt tggatgggaa aaattggggt 960
gtagagaaat atttttgtt ttggaggttt tgtttatagt gagtttgagg atgtagatga 1020
tatggaatat gagttttatt aggatatata ttttttgta tagtttttt tgattagtgt 1080
tgtttattgt tttaggtatt atttttagtt attttgtgtt aggtatttag gatagttttg 1140
tttaaagtat gattgttttt tggttagtga agaaatagta aagaaatgag agtttgtttt 1200
tgaagttttt gtagtaattt aatattttg taatgttgtg gtatatgttt tatattttt 1260
aaaagtattt atttgtagat tagaaataaa agagtttttt agtatagata gtttgagaga 1320
tgttattgta gagtgttttg ggaggatggg atttttttag atattgatgg ttttttggag 1380
tggtgttgga gtttgttgag atagggatgt ggggattgtt gtggaatagt atgttgtgaa 1440
aataggtata gatgggttgt tgttatgtat ttagtatttg gtaggtttag gtattttgtt 1500
ttggggagat agagtaatag ttgttatttt taaaaatgtg atataaatat gatttttgtt 1560
ttttattgta tgtgtatgta tgtgtgtatg tgtatgtgtg gttggtgtgt gggtaaataa 1620
tattggtgag ggagttagta gtgtagagat gtagagatga atttaatgaa tttatatttg 1680
gaggtttttg aaaggagttt tgtgtatgta taattaggaa gtagaagtgt agttgtaggt 1740
tagttttatt ttttggtgga aatagtaaat tatagtttta gtatttttta tttgtagtaa 1800
aagaaatgta tgttgtattt gggaggggta ggagaatttt ttagaatttg gtgggtttat 1860
tagttagggt tgtgtagaga aatagaatta atgagataaa tatgttagat agatgataga 1920
tagatagata gatagataga tagatagata gatagataga gatgatagat gatatagata 1980
gatgatagat gatagataat agatgatata tagatagata tagtaaatag atttatagat 2040
atgatagaga gagaatgaga gagatttata ttaaagaatt ggtttatatg attgtggggg 2100
ttgtaaattt tatatttgta gaatttgttg gtaggatgga aatttaggta agagtggata 2160
ttgtagtttt gagttgaaag tttatagggt agtaagttgg aaatttaggt aaggttttta 2220
ggttgtagtt ttaagaagaa ttttatattt tttaggaaat tttagtttgt gtttttaggg 2280
tttttaattg attgaatgaa gtataaatat tatggaagga aatttggttt atttaaagtt 2340
tgttggatta agtattaatt ttaattaaaa aatgttttta tagtaatatt tagattattg 2400
tttagttaag tatttgggta ttatagttta gataagttga tgtataaaat ttattattat 2460
aagggaggaa tttatatata tattttattg tagtaaaatt tttatagttt aatgttttgt 2520
atttttttta gaaggaaatg ttttagtgta gagttgaata ttgtattttt ttgtagttta 2580
tataaatttt atataatttt gatttgttga gtttttttg aaaattggat aatttaagtg 2640
tttatgaaag gttttaattg tttaagaaaa tagattgttt gtgtgaatta taaagaaaaa 2700
gggattttag aaggaatatt ggtattttgg gaagtaggtt ggggtaaggt ttgtataagt 2760
gaattagaag ttttaggtat gaagttagta tttttgttat ggtttatgtt agttgagttt 2820
ttatttttgt tttgtgtttg gtttttagat tttgtgagtt tgttttggga tagggttatg 2880
gttaatttag tagagatttt ggtaaagtat tttgggaatg agagtgagaa aggtttaaag 2940
tagttaaggt ttaaagataa tttttgattt tattttttta aatgttagtt tttaagattt 3000
taggtttttt ggattttaat ttttatgtta ttttaaaggt tttaaatttt agttttagta 3060
gttttagttt atagttttag gtttttagag attattttag taatttttaa atattatttt 3120
agggatttag ttttgatagt taagattatt tttttataa aataattgta gatattaggt 3180
gaagattttt aaagttttaa ggaaatttta aattttattt tgaggatatt gattttatta 3240
aagttttgag gaaattttaa attttatttt gaggatatta gttttattgt taggaattgg 3300
gattttaatt tatagtattt ggatttgag aatagaggtt ttggggttaa atgggttgaa 3360
tttagtattt tatttttatg tttttgggtg gatagtaatt tttttttatt gtgttttttt 3420
gtgggtttta ggttttatat ttgagggatg tggttttttt tttttatatt atgttggtta 3480
```

```
agaatgattt atatagttat tatggaatat tgtatggaga taaggagtgg ttgtgttttt    3540
gtttgaaggg tttattttat ttatgaggta ggatgatttt taaggtagtg ttatttatta    3600
gagttaaagt ttaggtaagg tgtttagggg ttagttgtaa tatgaagtat attagttaag    3660
gtagttttaa agagtattgt tagaggaaga atttatattt ggttgtttaa tgaggttgaa    3720
aggtaaattt aggttaagta ggttaagtaa agaggttagt ttagtatttt gggaggtaga    3780
ggtaggtgga ttatttgagg ttaggagttt gagattagtt tggttaatat ggtgaaattt    3840
tgtttttatt aaaaatataa aaattagttg ggtgtggtgg tatgtgtttg taattttagt    3900
tatttaggag gttgaggtag gagaattgtt tgaattaggg aggtggaggt tgtggtgagt    3960
taagattagg ttattgtatt ttagtttggg tgatagagtg aaattttgtt ttaaaaaata    4020
aataaataaa taaataaaaa taaaaaagaa gttagaaagt ttatgaaaaa tatttggagg    4080
gaagaagtta gtttaagaat aaaagtatta ggttagggtg tttaagattt ttaaggattg    4140
gttttggttg tgtagatatt taaagagatt gtgtaaggtt ggataggagg gtgagttggt    4200
taaaaaggtg atttgttaat tgtggaatta attattgttt aatgttttgg ataattgagt    4260
ttgatttgta atggttaggt ttattattgt aaattagttt tgaagtataa ataagtttat    4320
taaattattt tttttgttta tatttttttt tatttttga tttatgtaat ttttgtagtt    4380
tatatttaaa taaaaaataa taatatggat ttgttattat gtatgttgtg gtttattttt    4440
aattgtaaag tttttgatta tttttttgtt atgttgattt ttatgttaga agaaaatgtt    4500
ttgttattta ttatgagtgt agaaaattgt ttattagaaa tgattttaaa tagtaaagga    4560
tgtatatttg aaataatagg gttgtaatga aaaagtatat atttttaat tttatttaat    4620
atattgtatt tgtagagtaa aagatataga aaagtatttt atgttttaaa aatgtaagtt    4680
tgagagtgaa aaatagagat aataaaaatt tttttttaaa tattattggt ggaaaagttt    4740
taaagtaata gaaagtatat ataaaatgtt tgtgaatttt tgtgtaataa aattatatat    4800
atatatgtgt gtgtgtatgt atatttgttt tttttgtttt gttttgtttt ttttttttt    4860
tttgagatgg agttttgttt tgttgtttag gttggagtgt agtggtggga ttttggttta    4920
ttgtaagttt tgttttttgg gtttatgtta tttttttgtt ttagtttttt aagtagttgg    4980
gattataggt gtttgttatt atgtttggtt aattttttgt atttttagta gagatggggt    5040
tttattgttt tagtggggtt ggttttgatt ttttgatttt gtgatttgtt tgttttggtt    5100
ttttaaagtg ttgggattat aggtgtgagt tattgtgttt ggttaatgta tgtatatttg    5160
taatttagat ttgtaaatgt agttatattt atatttatat attaaatgta ttaaaaagat    5220
ttattaagtt ttttatagtt agtttataaa tttttagttt ttttatatat ttgtattttt    5280
tgttttaata tgatataata gttaaaaaaa tttaaaaatt aataattaat taagtttttt    5340
tttttgtag attatattag ttattattta gaaatattaa tttattgttt taaaaataat    5400
tttttagag atgtttggta tttagtatat gaaattattt ataattgaat aatataaata    5460
aaatagaaaa aagaatttat ttttgggata tttgaagaaa gtgatttata aatttagttt    5520
atttttgagt tttattattt ttaggaaaat ggtttttatg ttattttat gttatgtttg    5580
atttgtttg tttgagtttg ttttttagaa agattgtgat ttttggtttg ttgaatatga    5640
aatttatatg attattttg ggaaaaattt tttttttagt gattgtaagt taggttttgt    5700
ggttgagaag tattagttgt atttggttga ttgattgtat tattttgttt ttatgttgtt    5760
tataaagata tatttgagat ttggtaattt ataaaagagg tttaatagat ttatagtgtt    5820
aagtgggtgg gggtgtttta taattatggt ggaaggtgaa aggtatgttt tatatggtgg    5880
tagataagag aagagagttt gtgtaggaaa atttttttt ataaaattat tagattttat    5940
gagatttatt tattattata agaatagtat gggaaggatt tatttttatg atttaattat    6000
ttttaattag gatttttat aatatgtggg aattatggga attataattt aagataagat    6060
ttgggtgagg ttatagttaa ttatattatt gatttttatt atattataaa gaattatttt    6120
gtagagagaa tttgtttttt atttttaata atttgtttat tttggatatt ttatataaat    6180
agagttatat aatatttgtt gttggttttt ttatttagtg taatgttttt aaggtttatt    6240
tgtgttagag tatgtatttt tgttttattt ttttatatta ttaaataata tttgattgta    6300
tggatgtgtt atattttatt tgatttttta ttagttgata gattgttggg tttttttatt    6360
ttttggttat tatgaatgat ggtattatat gaatatttat atataagttt ttatgggtat    6420
atgtttttat tttttttggg tatatattta ggtgggggat tgttgttata tggtaatttt    6480
atgtttaata ttttgagga                                                 6499
```

<210> 91
<211> 6499
<212> DNA
<213> Artificial Sequence

<220>
<223> chemically treated genomic DNA (Homo sapiens)

<400> 91

tttttaggat gttaaatata aagttattat atgatagtaa ttttttattt aggtatatat 60

```
ttaagaaaga taaaaatata tatttatgaa aatttgtgtg tgaatgttta tatagtatta     120
ttatttgtaa tagttaaaaa gtagaaaaat ttaatagttt gttaattgat aagagattaa     180
ataaaatgtg gtatatttat ataattgaat attatttagt gatgtaaagg aatgaagtaa     240
aggtatatat tttaatatag atgaattttg aaaatattat attaaatgaa aaagttagta     300
ataaatatta tataatttta tttatgtgaa atatttagaa taaataaatt attagagatg     360
gaaagtagat tttttttgta agatagtttt ttataatatg atgaaagtta gtgatatggt     420
tggttgtgat tttatttaaa ttttattttg aattgtagtt tttataattt ttatatgttg     480
tggagggttt tggttggagg taattgaatt atgggggtgg gttttttttg tgttattttt     540
gtgatagtga gtaagtttta tgaggtttga tggttttata agaggaaatt tttttgtgta     600
agtttttttt ttttgtttgt tattatgtga gatatgtttt ttattttttg ttatgattgt     660
gaggtatttt tatttatttg atattgtgag tttgttaaat ttttttttgta aattgttaag    720
ttttaggtat gtttttatga gtagtatgaa aatagagtaa tatagttagt taattaagta     780
tagttgatgt tttttggtta taggatttgg tttgtagtta ttaaaaaagg aatttttttt     840
aagagtgatt atgtaaattt tatatttaat aggttaagga ttataatttt tttggaaaat     900
agatttaagt aagataagtt aaatataata tagagatgat atagagatta ttttttttaaa    960
aataatgaga tttagggata aattgagttt gtaggttatt tttttagat gttttaaaag     1020
taagtttttt tttttgtttt gtttgtatta tttaattata aatgatttta tatattaagt    1080
gttaaatatt tttagagaaa ttatttttaa aataatgaat taatgttttt gaataatggt    1140
tgatatagtt tataagaaaa aaaaatttgg ttaattatta atttttaaat tttttttaatt   1200
attatattat gttaagataa aaaatgtgaa tatgtgaaga agttgggaat ttatggattg    1260
attatgagga atttagtgaa ttttttttggt gtatttgatg tgtggatatg aatgtggttg   1320
tatttataaa tttgagttat aaatatatat atattggttg ggtgtggtgg tttatgtttg    1380
taattttagt attttgggag gttgaggtgg gtagattatg aggttaggag attgagatta    1440
attttgttaa aatggtgaaa ttttgttttt attaaaaata taaaaaatta gttgggtgta    1500
gtggtgggtg tttgtagttt tagttatttg ggaggttgag gtaggagaat ggtgtgaatt    1560
tgggaggtgg agtttgtagt gagttgagat tttgttattg tattttagtt tgggtgatag    1620
agtgagattt tgttttaaaa aaaaaaaaaa aaataaaata aaataaaaaa aataaatata    1680
tatatatata tatatatata tatatgattt tattgtataa aaatttatag gtattttata    1740
tatatttttt attgttttga gatttttttta ttaataatat ttagagaaag gttttttattg  1800
tttttgtttt ttattttttag atttatattt ttaaaatata agatgttttt ttatattttt   1860
tattttatgg gtataatgta ttgaatgaga ttgagaaata tgtatttttt tattataatt    1920
ttattatttt aaatatatat tttttgttgt ttagaattgt ttttagtaag tagtttttttg   1980
tatttatggt gaatagtaaa gtgttttttt ttgatatgaa gattagtatg ataggagaat    2040
aattagaaat tttatagtta gaagtaaatt atgatatgta taataataag tttatgttat    2100
tatttttttat ttagatatga gttatagaga ttatatgaat tagggaataa aagaaaatgt   2160
aaataggagg ggtagtttaa tgaatttgtt tatattttaa gattgattta taatggtaag    2220
tttaattatt gtgagttaga tttagttatt taggatattg ggtggtggtt gattttatag    2280
ttagtagatt atttttttgg ttagtttatt tttttgttta gttttgtata attttttttaa   2340
gtgtttatat agttaggatt aatttttggg gattttagat gttttgattt aatgttttta    2400
tttttggatt aatttttttt ttttaaatgt tttttatggg ttttttgatt tttttttttat   2460
ttttatttat ttatttattt attttttgag gtggagtttt attttgttat ttaggttgga    2520
gtgtagtggt ttgattttgg tttattataa tttttgtttt tttggtttaa gtaatttttt    2580
tgttttagtt ttttgaatag ttgggattat aggtgtgtgt tattatattt agttaatttt    2640
tgtattttta gtaaagataa ggttttatta tgttggttag gttggttttg aatttttgat    2700
tttaggtgat ttatttgttt ttgtttttta aagtgttggg ttgatttttt tatttggttt    2760
gtttgattta gatttgtttt ttaattttat taaatagtta ggtgtagatt ttttttttttgg  2820
taatattttt tgagattgtt ttggttaatg tgttttatgt tgtaattggt ttttaaatat    2880
tttatttgag ttttgatttt agtgagtagt attgttttga gggttatttt gttttatgaa    2940
tgggatgagt tttttaggta ggggtatagt tatttttttat ttttatgtaa tattttatga   3000
tgattgtgta gattattttt ggttaatatg atataaaagg aggaggttat attttttaaa    3060
tataggattt aaaatttatg aggggatgtg gtggaggagg gttgttgttt atttgggggt    3120
gtgggagtga ggtattggat ttagtttatt tggttttgaa gtttttgttt ttggaatttg    3180
ggtgttgtgg gttgaggttt tggtttttaa tggtgggatt ggtgttttttg agatgaaatt   3240
tggggttttt ttggggtttt ggtgggattg gtgtttttag gatgagattt agggtttttt    3300
tggggttttg gggattttta tttaatattt gtgattattt tatgagagga gtggttttgg    3360
ttgttagaat tggattttttg gggtgatatt tgggagttat tggagtgatt tttgaagatt   3420
tagggttatg agttggagtt gttggggttg aaatttgggg tttttgaagt ggtatggaga    3480
ttgaggttta gagagtttga gattttgagg gttgatattt ggagagatgg ggttgagggt    3540
tgtttttggg ttttgattgt tttgggtttt ttttattttt atttttggga tgttttgtta    3600
gaatttttgt tggattggtt gtaattttgt tttggagtgg gtttataggg tttgaaggtt    3660
aggtatgagg taaaggtaaa gatttaattg atataggtta tgatagaggt gttgattttg    3720
tgtttaaaat ttttgattta tttatgtaag ttttgtttta atttgttttt tggagtatta    3780
atgttttttt taaaattttt ttttttttgt aatttatata aatagtttat ttttttaagt    3840
```

```
aattaaaatt ttttatgaat atttaagttg tttagttttt aggagaggtt tagtaaatta 3900
aagttgtatg ggatttgtat gagttatgga aagatgtgat atttaatttt gtattgaaat 3960
gttttttttt gggaaaagta taaaatgtta aattgtaaag attttgttgt aataaagtgt 4020
atatataaat tttttttttg tgatggtgaa ttttatatgt taatttattt gagttatggt 4080
gtttagatat ttggttgaat agtagtttaa atgttgttgt gaaggtgttt tttagttggg 4140
attaatgttt aatttagtag attttgagta aattggattt ttttttataa tgtttgtgtt 4200
ttatttaatt agttgaagat tttaagaata tagattgagg ttttttaaag aatatgaaat 4260
tttttttaag attgtaattt agaaattttg tttgagtttt tagtttgttg ttttgtggat 4320
ttttgattta aggttataat atttattttt atttgaattt ttattttgtt agtaaatttt 4380
atagatgtag gatttgtagt ttttataatt atgtgagtta atttttttaat gtaaattttt 4440
tttatttttt ttttgttata tttatagatt tatttgttat atttatttat gtattattta 4500
ttatttatta tttattattt atttatatta tttattattt ttatttattt atttatttat 4560
ttatttattt atttatttat ttattattta tttaatatat ttattttatt ggttttgttt 4620
ttttatataa ttttgattaa taaatttatt aaattttaag aagttttttt atttttttta 4680
ggtgtagtat atattttttt tgttataggt gagaagtgtt aaggttgtaa tttgttgttt 4740
ttattagagg atgggattgg tttatgattg tatttttgtt ttttgattat gtatgtatag 4800
ggtttttttt aagggttttt aaatataaat ttattagatt tatttttgtg tttttgtatt 4860
gttaattttt ttattaatat tatttattta tatattaatt atatatgtgt atgtatatat 4920
gtatatatat ataataaaaa atgaaagtta tatttgtgtt atatttttgg aaatgatagt 4980
tgttgttttg tttttttggg gtagggtgtt tgggtttgtt aaatattgag tatgtggtaa 5040
tagtttattt gtgtttgttt ttataaatatg ttgttttata gtaatttttg tgtttttatt 5100
ttagtaagtt ttagtattat tttagggagt tgttaatgtt tagaagaatt ttattttttt 5160
agggtatttt atagtagtat tttttaaatt atttgtgttg aaggattttt ttatttttag 5220
tttgtagatg aatattttta aaaaatataa aatatgtatt atgatattgt agaaatgtta 5280
agttgttata aaagttttaa aagtagattt ttattttttt attatttttt tattgattag 5340
aaagtaatta tgttttgaat agaattgttt taagtgttta atatagagtg gttgaggatg 5400
gtgtttaggg taatagatag tattgattag ggaagattgt gtaggaggat gtatattttg 5460
gtggggttta tgttttatat tatttgtatt tttaggtttg ttatgaatga agtttttaaa 5520
ataggaaata ttttttttgta ttttagtttt ttttatttaa gtatatttat tttatttttta 5580
tttagtatgt ttgttttatt aattgtaatt attttataat ttaggttatt ttgatttttt 5640
tagatgatta atgatgggaa gttgaagaag ataggttatg ggtttttagt ttatttaaag 5700
tttatttaaa ttttagttta gagggagatt ttttttttta ttttgtttaa atatattatg 5760
gtttttttat ggtaattgtg aaattagttt atatatttaa ttattttata tttgattatt 5820
tatttttatg tattatgaat agagaaggtg gaattaaatt tttattttat aataagaagg 5880
aggttgagat tggaaatttt atatttaata ttgttgtaat tttttttttg gatatattag 5940
tttgatatag ttttatatat gtgtataagg agttaatgtt atttttgata taatttttat 6000
attgttaaaa agtatagaaa tattgaggtt taaaatttta ttttatttta atagtgtaat 6060
gtttttaaag aaaaaatgtg agggttaagt tttatttttt attttatttt atttatttat 6120
tttttttgaga tagagtttta tttttgttgt ttaggttgga gtgtaatggt gtaattttgg 6180
tttattgtaa tttttgtttt ttgggtttaa gagatttttt tgtttaagtt ttttggaatt 6240
aagttttttt tataggaaaa aaggaatttg attgtttttt tatattaaat tagtgtaaat 6300
gaaatgattt ttgtaattat aaataaatta ttttttaatt gtgttagata tattaatttt 6360
taaaggtttt tatttgttga tgtaattaaa tggttaaata ttatatagaa tttaagtggg 6420
aataataatg tgttaaaata tatttatgtt tatgtgtatt taatgattag ttgttagtta 6480
taagtgagaa tatgtagta 6499
```

<210> 92
<211> 6456
<212> DNA
<213> Artificial Sequence

<220>
<223> chemically treated genomic DNA (Homo sapiens)

<400> 92

```
taagaatttt aagttattat ttttgtgtat gtaggtagta ttagtgttag taataataag      60
ttagtttttt tggtaaattt attaaaatta aattttaaat ttttgaaaaa attaatatgt     120
ttattatgtt tttgttagtt tagaggttta attatttgtg ggggagggtt tataattatg     180
attaattatt tatgatttga aggtatttat aaaagtattt gttttttttaa gaaattatta    240
aagaattagg tttaagattg taaaattaat tttaggggta atagtaaatt ataggtttgg     300
ggatttaatt ttgataattt ttttttggaa agaaaagaat tgttgttaat tgtttttatt     360
ttttgatttt gtttgagtaa atatttgtag tttttaaaat ttagaatttt tagtttggtt     420
```

```
aagtaagtta tggggattaa tggttgtttt aaggtagagg tagaagagaa gaaataggaa     480
ggaaataaaa agggaatgaa gatttgtagt ttatgagggt gatattatta ataatttttt     540
taagtattat tgaattttat ggtttgtgat tgagagtttt ttttgatatt ttataggtta     600
attattttta aggatttttt tggttttaga gtttgtaatg tttgggtttg aatatgattt     660
gttttataaa aaaggttagg tgaattgatt tttttttgtg aagttgtttt atggtaagag     720
attattaatt tgttgaaatg taaataattt tttagatatt tgaagtattt ttaaatttat     780
aaagtgtttt tatatatgtt attttatttt atttttatga ttttaatagg taggtgttat     840
tattatttta atatgtaaaa ataatttag aaaataatgt ttttgttttt agtttataag     900
aatatttttt tgaaatttat agtatagaat ttaagttatg ggttagttag aaatgtttag     960
aaatatatta attttaggag gataaattaa gagaatagga tggattagat aaaatttata    1020
agtataatgg tagaaagtgt gtttttggta taagaatggt gtaagtttg taaataatag    1080
tgttatatag gggtgttttt gtagtatttt tatgtttgga aggatattat ttatttatat    1140
atatgataag tatttttaat tggatatttt ataggtaata gataaaataa aataagaatt    1200
tttgttttaa aattgtagtg gtggaattta ttttttggga tgtattaggt gattttttta    1260
ggatgatagt tgagttatag ggggttaaat atattttttt tttttttttt taatattata    1320
aagagtttta aaaatggatt ttgttgtttt taataaataa aatatatatg aaaaaaagtt    1380
atttataggt ttttatatat aattttattt tttaaaatat ttattttttt ttattagtta    1440
ggttgaggta taatgtggtt ttaattttat aattatttat tgttttattt atatatttat    1500
gatttttga tgtttttttag tttgagttta aattgtgttg ggaataaagt attgtataaa    1560
ttattagttt gggataagag gagataagtt aaaatttttt aagaagattt tgtagatatt    1620
tggttttttt gagtttgtag agagtttggt attttaaagg ttagttttgg gttttttat    1680
tttgatgatt aatgttaaat tgtttgattt tttataaaaa tatttagttg tgtttattta    1740
tattatttta tttttagtgt atatagtagg tatgtatttg ttgaatagaa gttttaaatg    1800
gatgagaaaa aagatatttt tgttatatat tttttaaga ttgagaaagg taaaattata    1860
atttaatttg tagaagtttt taggtatttt tttttaatgt aaagttatgt atttttgttt    1920
aaaaaagaaa aggatgatat aattaaataa tgttaattat ataatgggga aattttagga    1980
ttattaagaa aatattatta ttttaagtta tatattttta tttaatattg gttattatat    2040
aggagagagt aagaattgtt gttttttat ttatttgtat aatttatttg ggaaaagtat    2100
gggtagaata tttatgattt aattatatta tattttggat ttatatttaa tttttttggag   2160
aattagttta aagagtatgt ttgtttagag atataaagta aaatagatat agggataata    2220
gttttggaag aaatttttat gatgttgttt gagattgggt attagtattt aaattttta    2280
gattattagg agttattttt attatgtaat tttaataaag agaaattgag agtatgattg    2340
gtaaaaatat tatgatgttt ttgtattttg ttaatttgat ttatatttgg ttttatttat    2400
aaagttttta ttgattatta tatggtattt ttattttta gttttattta ttttgttttt    2460
taaaaatttt ttaatggtat ggttatgaaa aatatgtggg ggttagtggg gtatgtttta    2520
ggttggttta ttttgttttt aaaatgtatt tatttttttt agattttttt ttagggtggg    2580
tagggagaaa atgtttttaa gttgtatagt tgtagggaag tttgtttagg ttatgttttt    2640
ttgatttgtt ttttggtttg ttgtttggta aggttattgg ttttggtttg tggttttgtt    2700
gataaaattt aatttttatt ttgtaagatt aataggttgt gtaggatttt tttagttgtt    2760
ttttgttttg tattttttg ggtttgagga tttttggttt ggtttttttt gtgtgtgtga    2820
ggttttagat ggtgagggtg tagttttttg gtttatttga ttatgttggg gtgtttgaag    2880
gtttttaggt gttttagtat tgttattttg tggatggtgg agagtggtat gtttttttg     2940
ttggtttgta tttgtatgtg ttttaatgtt atgaaatggt ttttgttttt taagttgtgg    3000
gttttgaata tttttttata ggtgtttttt ttgattttg ttatgtattt gtattgttgg     3060
ttagtgtggt ataggttgtt tttttttatg ttgtttggat gttgtttgtt gtggtgttgt    3120
tggggtgggt ggggggtgtg tttaattagt tggtggttgt tgtttgttta ttttggggtt    3180
ttttggagat tggtttagtt ttatttgttt tttgttggtt taggtgtttg ggtgttgggg    3240
tttttgttgt tgtagaagtt ggatggagag atttttttgt ggggttggtg taattttggt    3300
gttgttgttg tgaaattttg tttgtagagt tgttgttgtt gttgttgttg gaggagtgag    3360
ttgatttttt tttttttttt tttgaagtga agtttttaat atagatatga ttatatagtt    3420
tttgtttaag tgtgttttag tttagaatta ttgtatttaa aggaggagat gggaggataa    3480
gaagaaagtg taattagata gtttagaagt tttttttggg gttttttag atttttttt     3540
ttttttttta tgaagttttt attgttattt tttgtgtgtt tttgtttttt tttaagttgt    3600
ttaattttt ttggtttttt tgagaaaagt gaagttattt tttttttttt gtagggttga    3660
agttgttttt gtgtggagag gttgtagggg tttttgggg atgagtgagt ggtgtgggat     3720
gtagtggaat gggagggggt gtgttgagta gtttagagtg tgttgggagt gtgggggagg    3780
ggaggtgttg ggtatgttaa tgttatggtt taatatttat ttttatatta ttgtgttgtg    3840
ttgttatttt ttttgttttt tgaggtttgg atgtgtaggg agatggggtt taggggtgtt    3900
ggagggtgtt taggggttgt gtataagttg gagtggaagg attgtgggtt tatttgtgtg    3960
gggttgtaga atgtgggtgg gggtttttag gattttgtaa tttgatgttg ggagtgtgtg    4020
agaaggggtg gttagatatt tgtttagaaa ttgttttttt ttttttatt tttagttttt     4080
ttatgaggag atatttaaaa atgatttgta tatataaatg gttttttttgg ggtaaaggag   4140
ttttggttgg aaatggaatt ttttttttgg ttgggaggat tttttggtg gaaattttgg     4200
```

```
gaagattagt tatttggttg gggagggttg gggtttattg ggggtgggtg agtgagtggt     4260
tttgggggag gggagatatt gttttttatg ggattttaag ggtgggagaa aggggtgttt     4320
gttataattt tgtttgttgt ttttttaaag aataatttta ggaaggggat agtataattg     4380
tgttttgttt ttaagtttta aattgatttt gatttgagta aaatttaatt ttttttttaa     4440
agggtggggg gtggggggtt aaattttgtg tttttagggg tgagagagaa ggtttttgtt     4500
tttggggttt ggatttgtga attttttttt atttttagga tttttttgtt gtaggtagta     4560
gaggtggttg ttttattttt tttttggtta aaggtttggt ttagtttgta gttggatgtt     4620
tttggagttt tttgtataat aaagtgttag agtagggtat tttaattttt ttttttttatt    4680
tagtagttat ttgggattta gggggttttt ttttttttagg gttgttgtgg ttttggtagg    4740
atttttatta tgatggttgt atgtttggaa tttttggggt attttggaat tatttgtttt     4800
gtaattgttg gtttgtttgt tttttgttag gaattaaata aatggtgtga tttttatgat     4860
gagtgggtgg agtggattgt ggtgagagta gagtttttgg tatttattat atttagaatt     4920
tttttttaaaa gttgaggaaa gagtttttag ggattgtttg ggggtttgag ttaggttgag    4980
ggttatatag gtagttgggg agttgttgtt ggttgtttaa tttgtttgtt ttgtattttt     5040
ttaatttttt tttgtttgag tttagttttg ggttttgtag tgaaggaagt gttggggatg     5100
ttttattttt gtagggaatt gtgtttgttg tttttgttgg gttttgtatt gttttggttt     5160
ttttttttatt tgtttttttag atggttggtt ttggtggaga gaaatgggag tagtaatgtt   5220
tttttttttt tttttttttta ttttttttttg ttgttgttgt tgtttttttta tgttggttga  5280
atgtgatttg attttatttt aaaaaaagtt tgatatagtg ttttaatatt tttgtatttt     5340
tttgtgatta taaaggttgt agttgttttt ttttgttttt tgtttttgtt ttttgttttt     5400
atatttaatg aaatttttta tgtgtttttt ttgaagtttg ttaagtatta taagggttgt     5460
tattagtata gtgataagag gttatattgg ggattgtgat ttttgtgtgt gtgtatatgt     5520
agatgtgttt atatgtatat ggatgggtgt gttgtgggga ttagggttgt ttattgtggg     5580
gtgtgtgttt aattttaata ttttaaatta taaaagtaaa tttggaaatt atagtaaatg     5640
tgtgtttttg tggggtttta agtttgggta ttggttggtt gtgtgtattt ttttgtgtat     5700
aatatgtttg taggaagttt gtgttaatat ttattttgtg gaggggaagg tggagtttat     5760
atttatattt tagtgagttg gagagggagt ttttgagggg gaaatgtaag tatatttttt     5820
agtatggagt ttttagaggt agtgtatttt aaattttaaa tgtgaattat tatgtgagtg     5880
tttgagtaga gttttagttt tgtttggaga aattgtatga ggtgtgtttt gtgtgggtgt     5940
gtgtgtgtat ggatatgtgg aagtaggatt ttggtgttgt gggtgttttt aggttgggat     6000
tttttttttt tattagataa ggagaatttt ttagtttttt aaaattggtt tttataatta     6060
attttgtttt agtttgtaag ggttaatta aagttgattt taaggaaata taggtttttt      6120
ttattttttg ttattttttta tttattttat ttatagtttt gttttggatt ttggttaatt    6180
ttttagattt tttttgggat tttttatttt gtagtttatt tatttgagtg tatagttttt     6240
tatttgaggg gtttagttgt gttgggtttt ttgagggggt tgtgagtgtt agttggtttt     6300
ttgtatgtgt ttgtggtttt gtggagtagg taattagatt ttggggaagg agttattagt     6360
attttttttg gtgagggggt gggtatagtg gtggagggtg gagggatggt ggagggtgtt     6420
gtttgtgttg tttgttgggg gtggatgggg gttgtt                               6456
```

<210> 93

<211> 6456

<212> DNA

<213> Artificial Sequence

<220>

<223> chemically treated genomic DNA (Homo sapiens)

<400> 93

```
agtggttttt gtttgttttt agtgagtggt gtgggtggtg ttttttgttg tttttttgtt      60
ttttgttgtt gtgtttattt ttttgttgga gagagtgttg gtaatttttt ttttagagtt     120
tgattatttg ttttgtgagg ttgtggatat gtgtggagag ttgattgata tttgtagttt     180
ttttgggagg tttgatgtga ttgggttttt taggtgagga gttgtgtgtt tgggtgggtg     240
ggttgtgggg tgggggggttt tgggggagat ttggggggatt ggttggggtt tgaggtagaa     300
ttgtgggtgg ggtaagtgga gggtggtggg gggtgggggg gatttgtgtt tttttggaat     360
tggttttggt taattttttta tgaattgaga tgggattggt tatgggggtt agttttgggg     420
agttagagga ttttttttat ttgatgagag ggggagattt tagtttaggg atatttgtga     480
tattgagatt ttgtttttat gtatttgtat gtatatatat ttatatggaa tatgttttat     540
atagtttttt taaatggaat tggaatttta tttggatatt tatataataa tttatattta     600
gggtttaaaa tgtattgttt ttagggattt tatgttggag aatgtgtttg tatttttttt     660
ttgaaaattt ttttttagt ttgttgaggt gtgggtgtgg gttttatttt ttttttatg      720
agataaatgt taatgtaggt tttttgtggg tgtgttatat atagaaaagt gtatgtagtt     780
ggttagtgtt taggtttgga gttttgtgaa gatgtatgtt tgttgtagtt tttaagttta     840
```

```
tttttataat ttaaaatatt ggagttaaat atatgttttg tagtgagtag ttttggtttt    900
tgtagtgtgt ttgtttgtgt gtatatgggt gtatttgtgt gtgtgtatat gtgggagttg    960
tggtttttgg tgtggttttt tgttgttgtg ttggtggtga tttttgtggt gtttggtagg   1020
ttttgggaga ggtgtatgaa ggattttatt gagtgtgaag atagagagta gagatagaaa   1080
gtagaaggag gtggttgtag tttttgtagt tgtggaggaa tgtggaaatg ttgaagtatt   1140
atgttaaatt ttttttgaaa tggggttaag ttatatttag ttagtgtggg aaagtaataa   1200
taataataaa aaaaagtgga ggagaggggg ggaaaaggta ttgttgtttt tgtttttttt   1260
tgttgaagtt gattgtttgg ggggtgagtg gggggagagt tagggtagtg tggggtttgg   1320
tgggggtagt aggtgtagtt ttttgtgggg gtgggatgtt tttgatgttt tttttgttgt   1380
ggagtttggg gttagatttg agtgggaggg ggttgggggg atgtaggata ggtgggttgg   1440
gtggttggtg gtggtttttt ggttatttgt gtggtttttg atttggttta gatttttggg   1500
tagtttttgg gggttttttt tttggttttt aaggaaagtt ttgaatgtag tgagtgttag   1560
aagttttgtt tttgttgtgg tttgttttat ttgtttattg tggggggttgt gttgtttgtt   1620
taatttttgg taaaaggtga gtgggttagt ggttgtgagg taggtaattt tgaggtgttt   1680
tgggaatttt ggatatgtgg ttgttgtggt gaaagtttttg ttgaggttgt ggtggttttg   1740
gaggagggag gttttttagg ttttagatag ttgttgaatg gggagggaaa gttagggtat   1800
tttattttgg tgttttgttg tgtagagggt tttgggggtg tttggttgta gattggattg   1860
ggtttttagt tggaggggga atggggtagt tatttttgtt atttatagtg gggaggtttt   1920
gggaatggga aaaggtttgt gagtttgggt tttgaggata gagatttttt tttttatttt   1980
tggaggtgta ggatttaatt ttttattttt tattttttga aagggaggtt gagttttgtt   2040
taggttgaaa ttagtttaaa atttaaggat gaggtgtgat tatgttattt ttttttttgaa   2100
gttatttttt aaggagatga taagtgaaat tgtggtgaat attttttttt tttattttttg   2160
ggattttgtg ggggatggtg tttttttttt tttaggattg tttgtttgtt tatttttgat   2220
aagttttaat tttttttgat tagatggttg gttttttttaa ggtttttatt agggaggttt   2280
ttttagttgg agagagaatt ttgtttttaa ttgagatttt tttattttaa aaagattatt   2340
tgtgtatgtg agttgttttt aaatgttttt ttgtagaaaa attgaaagta gaaagaaagg   2400
aagtaatttt tgagtagatg tttgattatt ttttttttgta tatttttagt attgggttat   2460
agggttttgg gagtttttat ttatatttttg tggttttata tggatggatt tatagttttt   2520
ttgttttagt ttgtgtgtga tttttgaatg ttttttggta tttttggatt ttgttttttt   2580
gtatgtttgg gttttaggag gtggggaggg tagtggtgta atataatgat atagggataa   2640
atattaagtt gtgatattga tgtgtttggt gttttttttt ttttgtgttt ttggtatatt   2700
ttgggttgtt tggtatgttt tttttttgttt tattgtgttt tgtgttgttt gtttattttt   2760
gaggggtttt tgtaattttt ttgtgtgaag atggttttag ttttgtaggg aaagaaaagt   2820
aattttgttt tttttggagg aattaggaag gattaagtgg tttgggagag ggtaggagtg   2880
tgtggagggt agtgatggag gttttgtaaa ggaggaggag gggagtttgg aggaattttg   2940
aggaaggttt ttgggttgtt tgattgtatt ttttttttat tttttttgttt ttttttttag   3000
gtgtaatgat tttggattga gatgtgtttg ggtagaggtt atgtaattgt gtttgtgttg   3060
aggattttgt tttgaggagg gaagaggagg gattggtttg tttttttggt ggtggtggtg   3120
gtggtgattt tgtaggtgga gttttgtggt ggtggtatta gggttatgtt agttttgtgg   3180
ggaggttttt ttatttagtt tttgtagtgg tgaaagtttt agtgtttgag tgtttgagtt   3240
ggtggggagt aagtaaagtt agattgattt ttggggagtt ttggagtagg tgagtggtgg   3300
ttgttagtta gttgagtgta tttttttgttt gttttagtgg tgttgtggtg ggtggtgttt   3360
aggtggtatg gagaaggatg gtttgtgttg tgttgattag tagtatgaat gtgtggtgga   3420
gattggggag ggtgtttatg ggaaggtgtt taaggtttgt gatttgaaga atggaggttg   3480
ttttgtggtg ttgaagtgtg tgtgggtgta gattggtgag gagggtatgt tgttttttat   3540
tatttgtgag gtggtggtgt tgaggtattt ggagattttt gagtatttta atgtggttag   3600
gtgagttagg gagttgtgtt tttgttattt ggggttttgt gtgtgtgggg agggttgagt   3660
tgggaatttt taggtttaga aaggtgtggg gtagagggtg attggggagg ttttgtgtga   3720
tttgttggtt ttatagagtg agagttaagt tttgttgata gagttgtgag ttagagttgg   3780
tgattttgtt agatagtgag ttgaaagata agttaaaaaa atatgatttg agtaggtttt   3840
tttgtagttg tgtaatttag aagtgttttt tttttgttta ttttgaagga gggtttgggg   3900
gaagtgggtg tgttttaggg ataggataag ttaatttgag atatgttttg ttagtttttg   3960
tatatttttt gtggttatgt tgttggggaa tttttgaaag atagagtagg tgagattgga   4020
gaatgaggat gttgtgtgat aattagtggg aattttatgg gtggagttaa gtgtaaatta   4080
ggttaataag atgtagaaat attataatat ttttgttagt tatattttta gttttttttt   4140
attaaagttg tgtagtaaga atgattttttg gtggtttaag gagtttgagt gttgatgttt   4200
aattttaaat agtgttataa aaattttttt taaaattgtt atttttgtgt ttgttttatt   4260
ttatattttt gggtggatat atttttttaga ttaatttttt aaagggttaa atgtgagttt   4320
aagatgtggt atggttagat tataaatgtt ttgtttatgt tttttttaga tggattgtgt   4380
gagtgggtgg ggagatagta gtttttattt ttttttgtgt ggtaattagt attaaatgaa   4440
aatgtgtgat ttaggataat ggtgtttttt tagtgatttt ggaatttttt tattgtgtgg   4500
ttgatattgt ttggttgtgt tatttttttt tttttaaat agagatgtat agttttatat   4560
tgaaaaggaa tgtttagaaa tttttgtaaa ttgggttgtg attttatttt ttttgatttt   4620
```

gagaaaatgt gtagtagaaa tatttttttt tttatttatt tagggttttt gtttaataaa 4680
tatatgttta ttgtgtgtgt tgaggatgaa atgatgtaaa taagtatagt taaatatttt 4740
tgtaaaaagt taaataattt aatattagtt attaaagtgg agagatttaa gattgatttt 4800
tgaaatatta aatttttat agatttagag agattaaata tttatagaat ttttttggga 4860
ggttttaatt tgtttttttt tattttaggt tggtgatttg tgtggtgttt tgtttttaat 4920
gtagtttaga tttaagttag aaggtattaa agaattataa atatataaat aaggtagtaa 4980
atggttataa agttaagatt gtattatatt ttggtttagt tagtgaagga gaatgagtgt 5040
tttagaagat gagattatat ataaaaattt atagatgatt ttttttatg tgtattttat 5100
ttgttaaaaa taataaaatt tgtttttggg gtttttgtg atattggagg gaaaagggga 5160
gggtatattt agttttttgt aatttagttg ttgttttagg ggaattattt aatatatttt 5220
gaaggatgaa ttttattatt ataattttaa gatggaagtt tttgttttat tttgtttgtt 5280
atttatggag tatttagtta agggtgttta ttatgtatat gaatgaatag tgttttttta 5340
aatgtagagg tattatagag gtatttttgt ataatattat tgtttataaa gtttatgtta 5400
tttttatatt agaggtatat tttttgttat tgtgtttgta gattttgttt gatttatttt 5460
attttttga tttattttt tgagattgat gtgtttttag atgtttttaa ttgatttatg 5520
atttggattt tgtattgtga attttaggag gatattttta tgaattgaag ataaaggtat 5580
tgtttttaa aattattttt atgtgttaag gtgatgatgg tatttatttg ttaaagttgt 5640
gagaataaaa tgagatggtg tatgtgaaag tattttgtaa atttgaaagt attttagata 5700
tttaaaggat tgtttgtgtt ttagtagatt gatggttttt tgttatggag tagttttata 5760
gggaggggtt aatttatttg atttttttg tagaataaat tatatttaaa tttagatatt 5820
ataggttttg gaattaagaa gatttttgag gataattagt ttgtaagatg ttagaaaaga 5880
tttttagtta tgagttatga aatttagtga tatttgagga aattattgat gatgttattt 5940
ttatgagtta tagattttta ttttttttt gtttttttt tgttttttt tttttgtttt 6000
tgttttgaaa taattattgg tttttatgat ttgtttggtt agattaagaa ttttggattt 6060
tagaggttgt gaatatttgt ttagataggg ttaggaaata aggatagttg atagtaattt 6120
tttttttttt aagggagagt tgttaaagtt gggtttttaa atttgtgatt tattattgtt 6180
tttgggatta gttttatagt tttaagtttg gtttttaat ggtttttaa aagggtagat 6240
gtttttataa gtatttttaa gttataagta attgattata attataggtt tttttttata 6300
gatagttaag tttttaagtt aataggagta taataaatat attagttttt ttaaaagttt 6360
aagatttaat tttgatggat ttgttagaaa agttgatttg ttattgttgg tattggtatt 6420
gtttgtatgt atagggataa tgatttgaaa tttttg 6456

<210> 94
<211> 6257
<212> DNA
<213> Artificial Sequence

<220>
<223> chemically treated genomic DNA (Homo sapiens)

<400> 94

```
ttatagtagt ttttttgatt ggattaattt ttttggatat aatagtaggg taggggtggg      60
gtttggggag aaaggatatt tttaattttg attttaatat tttgatggtt tttaaggttg     120
tttgtatatt tatttaggtg taagtttttt aaggtgtggt gtgatgaatt agtgattttt     180
ggagttaggt tagtgtattt ttttttgta gggttgtaag ttgtaggatt gagaggtagg      240
ttgattaggt tttgggttgg atgatggggt gagagtaagg ggttagtttt gatatatgtt     300
taattttttt ttttagtttt aagatatttt gggtaaattg tttattttag ttttttttgat    360
ttttatttta attttaatat tagtttaaga gaaaataggg atattgatgg ttatttagaa     420
gggttgttgt gttttatata tagtaatatt ttttgaatgt ttgtgatagt ggtttaagga     480
ataagttaat tttatattat tattttggat atttgtataa aattttattt tatttttatt     540
atatgaatgt gttagggttg ttttttttgtt ttgttttttt tttttttttt tgagatagag    600
ttttgttttt gttgtttagg ttggagtata atggtgtgat tttggtttat tgtaattttt     660
gtttttttagg tttaagtgat ttatttgttt tagttttttt gagtagttgg gattataggt    720
atgtgttatt atgtttggtt aattttgtgt ttttagtaga gatagggttt ttttatgttg     780
gttaggttgg tattaaattt ttgattttag gtgatttatt tgttttggtt ttttaaagtg     840
ttgtaattat aggtatgagt tattgtattt agttgtgtta gggttttttt ttgtttaatt     900
tttttttttt ttttgttttt tttttttttt ttaatggagt tttattttgt tatttaggtt     960
ggagtgtagt ggtaagattt tagtttattg taatttttgt tttttgagtt taagtaattt    1020
ttttgtttta gtttttttgag tagttgggat tataggtgtt tgttattata tttagttaat   1080
ttttgtattt ttagtagaga tggggttttg ttatgttggt taggttggtt ttgaattttt    1140
gattttgtga tttgtttgtt ttggtttttt aaagtgttgg gattataggt atgagttgtt    1200
atatttggtt aattttgtat tatttttttta aagagagttt tttaaattat ataagtttta   1260
```

ggttttataa aatttagatt tgttttagta taattaaatt tgggattatt tattgagtaa 1320
ttattatgtg ttaagtattg tgttgagtgt ttttagagta ttattttttt taattttagt 1380
atagtatgtt agatgttgtt ttatagatga gttaattgag attagagatg tttagttatt 1440
tgtttaaggt gatatgattg atatggaata gagttaagat tttttttttt tttttgata 1500
tggagtttta ttttgttttt taggttggag tgtagaggtg taattttagt ttattgtaag 1560
ttttgttttt taggtttatg ttattttttt gttttagttt tttgagtagt tgggattata 1620
ggtatttgtt attatatttg gttaattttt tgtattttta gtagagatag ggttttattg 1680
tgttagttag gatggttttg atttttttgat tttgtgattt gtttgttttg gtttttaaa 1740
gtgttggaat tataggtgtg agttattgtg attggttaga tttaagattt gaatttaggt 1800
tttttttggtt ttagaggttt ttgtttttta atttttttagg atggtatagt aatttgtttt 1860
ataagaggtg tttgttttaa gtgtgtttag tatatggaag taagtttaga aatgtaagtg 1920
tatatttgta aagaggtgtg ggagatgggg gggagggaag agagaaagag atgttggtgt 1980
tttttatttt ttagttttttg ataggtgttt ttgatttttt tttgattagt atagttgtat 2040
ttttggttgg ggtattttaa ttagaattgt taaatttagt atataaaaat aaggaggttt 2100
agttaaattt gaattttaga taaataatga ataatttgtt agtataaata tgttttatgt 2160
aatattttgt tgaaattaaa aaaaaaaaaa aaagtttttt ttttattttt atttttatta 2220
ttaggtttaa ggaatagggt taggggtttt aaatagaatg tggttgagaa gtggaattaa 2280
gtaggttaat agaaggtaag gggtaaagaa gaaattttga atgtattggg tgttgggtgt 2340
tttttttaaat aagtaagaag ggtgtatttt gaagaattga gatagaagtt tttttgggtt 2400
gggtgtagtt gtttgtggtt gtaatttttag tattttggga ggttgaggtg ggaggattat 2460
ttgaggttgg gagtttaaga ttagttttat taatgtggag aaattttgtt tttattaaaa 2520
atataaaaaa ttagttggtt atggtggtat atgtttgtaa ttttagttgt ttgggaggtt 2580
gaggtaggag aattatttga attagggagg tagaggttgt ggtgagtaga gattgtgtta 2640
ttgtttttta gtttgggtaa taagagtaaa agtttgttta aaaaaaaaaa aaagtttttt 2700
tgatgtgatt gttttttttt aaatttgtag attttttttaa gattatgttt tttagatatt 2760
ttaaagattt tagaagatat gttttggggg ttttggaagt tataaggtaa atataatata 2820
tttttttttt tgattattaa ttttattaga ggatgtggtg ggaaaattat tatttgatat 2880
taaaataaat aggtttggga tggagtagga tgtaagtttt ttaggaaagt ttaagataaa 2940
atttgagatt taaaagggtg ttaagagtgg tagtttaggg aatttatttt ggattttggg 3000
ggaggggggta gagttattag tttttgtatt tagggatttt ttgaggaaaa gtgtgagaat 3060
ggttgtaggt aatttaggtg ttttggtgtt aggagggatg tatttaggtt tgtgtgaaga 3120
gagggagaaa gtgaagttgg gagttgttat ttttagattt gttggaatgt agttggaggg 3180
ggtgagttgg gagtgtgttt gtttttaatt ataggagaag gaggaggtgg aggaggaggg 3240
ttgtttgagg aagtataaga atgaagttgt gaagttgaga ttttttttta ttgggattgg 3300
agaaattagg ggagtttttt gggtagttgt gtgttttttt ttatggggtt ttttattgtg 3360
ttgtgtgttt ggttttttatt ttttgtagta ttttgtgttt tgtgtttttt tagttgggtt 3420
tagttggagt tatggggttg gagttgtagt gagtattatg gagttggtgg ttttgtgttg 3480
ttggggggttt tttttttgttt ttttgttttt tggagttgtg agtatttaag gtgggtttgg 3540
tgtggggagg ggatggagta gtggtgggat tttgttttgt ggatgttttg ttgaggtttt 3600
gtggttggtg gggttagagg ggtttggatg agttttttta ttttgaagtt gtggatagtt 3660
gagatgttta gggtagttgg gttttggggt ttttgggtgg gaggggggtag ttatatggta 3720
gtggtttgag atggtttatt taagagattg gtgttttttta ggttttgagg ggttttggga 3780
atttgttaaa gaagtttttt gaaattgttt agaaagtttt tttgtaaagg gtgtattgtg 3840
tagagtgtgt gtgtgtgttt tttttttttt tgagtttttt taagtttttt taaagttttt 3900
ttagttggta gtttttgttt ttggattggt ttgggttgga tttttttgggg gggttttttg 3960
ttttgttttt tttttagttt tttttttgttt ttttttagat gattttggtt tggttgtttt 4020
tgtttttggt ggggttgggt gtgtgtgtgt gtggtggagt ggagggtggt atagtaattt 4080
gttttaatta gagttgggga ggaaagggtg gtttggaggg tggtttttttg ttggggtttg 4140
ggttgggggt gggggagatg tttgttttga atagattttt ggggttagtt tagggattgt 4200
gttttgtgat ttttggagtg tgtggattat ggagggggtgg gggtgggttt tttggggtgt 4260
aaagtgggag agttttttaga gaaggaagtt aagaaataag gttagatggg agtttaggga 4320
gggttgtgtt gtttgttgt tttttttttg gtgttgtgtg tggggaaggg tgagtggggg 4380
tagtgtgtat tttgatttat ttgtttattt gtgtgtatta attataaaag ttaatatata 4440
gtttgggtta ggtatatttt gttaggaatt gtttgtggtg ttttgtatgt attttttttt 4500
aattttagaa tatttttata gtggaagttt tgttagtatt ttggattgag tagtagttta 4560
gaggttgagt agtagttagt aagtggtggg gttaagatgg gattttaggt agtgtgattt 4620
ttaattatgt atttgaaatt gttatatgga tgagtgtatt tggagtaatg agggatattg 4680
ttttttgagt tattgggttg taggggagat aaaatgaaag tgttttggga gttgtgggtg 4740
gtttttatag gttagagggt ttggggaggg agtgggtgtt attgtggttg tgtgttgttt 4800
gaggggtttt ttgtgagtga gtgtatggtt gtgttatttt tgtaggttta tgttagggtg 4860
ttttttagtt gtgtggtttt tgtatttgtg tgtttgggtt ttgtgttgtt aaatagtagt 4920
ttttttgttg atttggggat ataggttgaa ttttgttttt tgtaggaatt tttttaaggt 4980
gttgggttag atttgttata aatagaggga ggtagttttt tatggttatg ttttttttgtt 5040

gaggaagaag gttttttttt tttagggagt atatttttgt tttttttgtt ttttagataa 5100
gtatttttat tttttatttt ttgatgagaa gggtgaggtt atattgagtt gttaggttga 5160
gttgttgttt ttttttattt tgggttggga gttgattagg gaatggtagt tgttgtagag 5220
ttggatttga gggttgggtt ttttggatgg ggttttttta tgtttttatt ttttaatttg 5280
tattattgat tgtgttgtgt aggagttagt taaaaagtta ttgtatagtt tgggtaataa 5340
ggtaaaattt tgtataaaaa atataaaaat tagttggatg tgattatatg tgtttgtagt 5400
tttagttatt ttggaggttg aggtaggagg attatttgag tttaggaagt tgaggtttgt 5460
agtgagttgt gattgtaaat gtttttttagt ttgggtgata gtgtgagatt ttgttttaga 5520
aaaaaagtat attatttagt tgtttttagt atttagattt tatttaaggg gtgaggtttg 5580
gggtaggaat gtgggggaag gggaggttta gggggagttt tagaggggtt aggatttttt 5640
tgaaattttt ttttagaggt atgggtttta taaattgtag taaatatatt tttttaattt 5700
tgtagaattt ttttatattt taattttagt atgatttttt taatagtttt ttttttttat 5760
tatatttggg gaaagtattt attttatttg ttaagaaaaa aataattgaa aagataggga 5820
ttaaatgtaa aaagaaaaaa tatgtggtat tttaaagtta aatataaagt atgtttaatt 5880
tttttgtggt ttgggattat ttatattttt gttgtatgaa tttgttttgt tttaattttt 5940
aagaaatgta tggtgtattt tttatatgtt aggtttttat ttatgttttt atttaatttt 6000
tgtgatagtt ttgtgaagta ggtgtataga tgagaaaatg gaagtttaga gaaatgaagt 6060
aatttattta aggtttttag ttatttagta atgtttaggg aatttttgga ttttgaagag 6120
gaggtattaa gaggtggtta gagttttatt ttagttaata ataatgggtt gaataaagtt 6180
ttaggggtag gtaggtggtt agatgggagg agaagtgttt tttttgttta ggtgaatgat 6240
tttttttattt atttttt 6257

<210> 95
<211> 6257
<212> DNA
<213> Artificial Sequence

<220>
<223> chemically treated genomic DNA (Homo sapiens)

<400> 95

```
gagaagtgga tggaaaggtt atttgtttga ataagaggag tgtttttttt tttatttggt    60
tatttgtttg tttttaaggt tttgtttaat ttattattgt tggttggaat aagattttaa   120
ttatttttta atgttttttt tttagagttt aaaaattttt tggatattat tgggtagttg   180
ggagttttgg ataagttgtt ttattttttt gaatttttat tttttattt gtgtatttat    240
tttataggat tgttatagag attaaatgaa agtatggata aaaatttagt atataggaag   300
tatattgtat atttttaaa agttaagata agataggttt atatagtagg aatatgggta    360
attttaaatt atgagaaaat taaatatgtt ttgtgtttga ttttggaatg ttatgtattt   420
ttttttttta tatttgattt ttattttttt aattgttttt tttttgataa ataaaatgag   480
tattttttttt aagtatagta aagagaggag gttgttggaa gaattatatt ggaattaaaa   540
tatgaaggag ttttgtaaga ttaaaaggat gtatttgttg taatttgtaa aatttatatt   600
tttaagaaag gattttagaa aaattttgat ttttttgggg tttttttttag gttttttttt   660
tttttatatt tttgttttag attttatttt ttgggtaaaa tttgggtgtt ggaggtagtt   720
gggtggtata tttttttttt gaaatggagt tttatattgt tatttaggtt ggagagtatt   780
tgtaattata gtttattgta agttttaatt ttttgggttt aggtgatttt tttgttttag   840
tttttggagt agttgggatt ataggtatgt gtaattatat ttaattaatt tttgtatttt   900
ttgtatagag ttttgttttg ttgtttaggt tgtgtaatga ttttttaatt aatttttgta   960
taatataatt aatagtgtag gttgaggggt gaggatatga ggaggtttta tttagagaat  1020
ttagttttta aatttagttt tgtagtaatt gttatttttt gattaatttt tagtttaagg  1080
tgaggaaggg tagtagttta gtttgatagt ttagtatggt tttattttttt ttattagaag  1140
atgagaggtg aagatgtttg tttgggaaat agggagggta aggatgtatt ttttggaaga  1200
gaggaatttt ttttttagt aagaaggtgt ggttatagaa ggttattttt ttttgtttat   1260
ggtagatttg gtttagtatt ttaagggagt ttttgtagag gatagagttt agtttgtgtt  1320
tttaagttag tagagagatt gttgtttggt aatataaagt ttagatatat aaatataaag  1380
attatataat tgaggaatat tttggtgtag atttgtagag ataatatggt tatgtattta  1440
tttatagagg gtttttgggg taatatatag ttatgatgat atttattttt tttttagatt  1500
ttttgattta tggagattat ttatgatttt tagaatattt ttattttgtt tttttttgtag  1560
tttagtgatt tagggagtag tgttttttat tgttttaggt gtatttattt atatagtgat  1620
tttgaatgta tggttggggg ttgtattgtt tggggtttta ttttgatttt attatttatt  1680
agttgttatt taatttttgg attgttattt agtttagaat gttggtagaa tttttattat  1740
aggggtgttt taggattaaa ggagaatgta tgtaaaatat tataaatagt ttttggtaga  1800
gtatatttgg tttaggttat atgttagttt ttatgattaa tgtatatagg tggatagatg  1860
```

```
gggtaataag agtgaaattt tgttttaaaa aaaaaaaaaa aaaataaaat aaaaaaataa  5700
ttttagtata tttatatagt aaggataagg tggagttttg tataggtatt tagagtgata  5760
atgtaaaatt aatttatttt ttggattgtt gttataaata tttgagaaat attgttgtgt  5820
atggaatata gtagtttttt tggatggtta ttaatatttt tattttttttt tgagttggtg  5880
ttagggttag ggtgaggatt aggggaattg aggtaagtaa tttgtttagg atgttttagg  5940
gttagagaga aaagttgggt atgtattaaa attgattttt tatttttatt ttattattta  6000
gtttaggatt tggttaattt gtttttagt tttgtagttt atagttttgt gggaagagga   6060
tgtgttgatt tggttttagg agttattggt ttattatatt atattttgga gggtttgtat  6120
ttggatgagt gtgtaggtag ttttagaggt tattgggatg ttaggattag ggttagaggt  6180
gtttttttttt tttaggtttt atttttgttt tgttgttgtg tttggaaagg ttagtttagt  6240
taggaaagtt attgtgg                                                 6257
```

<210> 96
<211> 8467
<212> DNA
<213> Artificial Sequence

<220>
<223> chemically treated genomic DNA (Homo sapiens)

<400> 96

```
atgaattaaa ttttttttg gtattaagaa gggatatata gtattttgag ttttgttttt    60
gttttttat taagttttt ttaatagttg gaagtggttg aagggggttg ttttaaggag    120
attttttgtt ttttttga tagttgatta aaagaaaata ggttatgttt tttaaattta    180
tgtttatttt gtttttaatt attgtttttt taaataaagg taatatttgt gtaattttag    240
ttagtttgaa tttttggagg taaatagagt gtaatttgtt ttggaagaat tttgttatgt    300
ttaaggtttt atgttgggtg ttggtttttt tgtttttaat tatttttta ttttgatttg    360
gattgtagaa attttaatt ttttgtttt tagttttatt tttattttta gatttggggt    420
tgttaaataa tggaaatttt aggaagatgt aggtgtggtt tttaaagttt tggtttgtga    480
gtgtttttag gttggttggg gtgttggttt ttttagaaat aaggtttgaa tatgtaggtg    540
ttagttagga ttttttgttg tttgtttatt gttgtttttt ttttgggatt tgagagaagt    600
gggagtgtgt gtgtgtgtgt gtgtgtgtgt gtgtgtgtgt gtgtgtgttt tgtgttagga    660
gaaaggtttg tggttgttgt tttagttggt ttgtttattt tttgttttta ggagaagttt    720
gtttatttgg gatattagtg attttttta tttttatttt attggtttta tattttttt    780
atttttttt aagttgttga tgtagaaagt atttggttat tttgaaatgg tagtttttgg    840
aattttagtt ttgttttgta atttagtagg gtttttgga agttttataa ttgagattta    900
tgggtttatt gggtaattaa atgatgttat tgtgttaatt ttgtatgtat tattgttgtt    960
tgtgttgttt gtgggttgtt ggttggttgt agtttgttgg tgatgagggt attatagttg    1020
ttttgattgt gtagattatg tatgttttgg ttttgggaat ttattatgta ttagaatata    1080
ttagtgtttg tattttaaaa ggttaaatta ttggttttta gttagggatt tttggtaagt    1140
ggtttgttag tgatgagtgt ttgtttatat tggtatatag tggagttttt tgtttttggt    1200
ttatttgttt ttaggaaagt tttggggtga ggtgaaggtg attgaagtaa tgtttttttt    1260
tttagattgt agttgtttag gggggatata gtatggtatt ttttattgaa tttttttgt    1320
ttgtttgtat tttagttttt tttttttag tgattttttt atttttttt ttttttttt    1380
ttttgatgtt tgattttagt agtaaaggag gtaaaaaagg tattgagttg ttagttaaat    1440
ttgaaaagtg tggttttgtt tttttatag ttattggtag tttttttgtgg aaggtttgtt    1500
ttttggggta gttgtggttt tggagtggtt gtgtttggtg tttgttgggt gtggttttgt    1560
tttaggtttg ggagggtagg ttggttgttt tggtgagtgg tagagttttt ttggatagtt    1620
tttgtttatt taaatagaag atgttggtgt tggagtgggt ttggatatgg tgaggttgtg    1680
agttggtttg agtggtgggg tttggtgatt ttttttttt tttttgtttt ttttttttt    1740
ttgtatgtat gttttgtttg tttttatttt gtttttattt tgggtgagtt tgtttgtagt    1800
ttggggtgta tatttgtatg tgtatttttt tttatttatt tttgtgtttg tttttatttt    1860
tgtagttgag ttttgttatg tgtgttttgt ttgtttgttg gttgtttttg ttgtttttgt    1920
tgtttttggg ttttgatgga ttgaatgaag gttgtttata ttgtttattg atgttttatt    1980
aaagatttag aaggttgtgt tatgaatttg gagttgataa tggaaagttt gggtattttg    2040
tatgggttgg ttggtggtgg tagtggtggg ggtggtggtg ggggtggtgg gggtggtggt    2100
gggggtttgg gttatgagta ggagttgttg gttagtttta gtttttatta tgtgggttgt    2160
ggtgttgttg gtttgttgtg gggttttttg ttgtttttaa ttgtgtatta ggagttgggt    2220
atggtggtag tggtggtagt ggtggtgttg tgtttggtta tggttattag tatggttttg    2280
attttggatg gtggtgatta ttggtttgag ttttttattt tgttgtatta tgttatgagt    2340
atgttttgtg atttgttttt gtttggtatg ggtatgagta atatttatat tatgttgata    2400
ttgttttagt tgttgttatt tatttttatt gtgtttgata agttttatta ttttttatttg    2460
```

ggttaggata tatattgttt ttatttattt tttttatgt atagtattaa ggaaaaggta 1920
gtagaataat gtagtttttt ttaggttttt atttggtttt attttttagt tttttttttt 1980
gggaattttt ttattttata ttttaagaaa tttattttta ttttttatg gtttatgtgt 2040
tttaaaagtt atagagtata gtttttaagt tggttttaag aatttgttta aagtaaatgt 2100
tttttttgtt tttaatttag atttagtaa gaggttattt tttgggttat tttttttttt 2160
ttggttttgg ttgggatagg ttgttatgtt atttttattt ttattatata tatatatatt 2220
tgattttgtt agaagtagga gtaattaaat taaaattgtt tgaaggggag tggggagggg 2280
ttggaggagg ggtagggtag aggatttttt taaggaattt agtttaggtt agtttggagg 2340
tggaggttgt taattggaaa ggttttgaga aagtttgagg gggtttaaga aggggggaaa 2400
tgtgtgtgtg tgttttatgt aatatatttt ttgtgggaaa atttttttgaa taattttaga 2460
gaattttttt gataagtttt tggagttttt tggagtttgg aaagtgttag ttttttggat 2520
gggttatttt gagttgttgt tgtgtaattg ttttttttg tttgagggtt ttagggtttg 2580
gttgttttga gtgttttgat tgtttataat tttgggatag gagagtttgt ttgggttttt 2640
ttggttttgt tggttgtggg atttggtgg ggtatttata gggtagggtt ttgttgttgt 2700
tttgtttttt ttttatatta gatttatttt gggtgtttgt ggttttgggg ggtaagaggg 2760
tgaggaggag tttttagtgg tataaggttg ttagttttat ggtgtttatt gtggttttgg 2820
ttttatggtt ttggttggat ttggttggga gggtgtgggg tgtggggtgt tgtgaggggt 2880
gggggttggg tgtgtggtgt agtaaagggt tttgtgggaa ggggtgtgtg gttgtttggg 2940
gggttttttt ggtttttttg gttttaatgg aggggaattt tagttttata attttatttt 3000
tatatttttt taagtagttt tttttttta tttttttttt tttttgtgat tgggagtaag 3060
tgtgttttta gtttgttttt tttaattgta ttttaataag tttgggagtg gtaattttta 3120
gttttatttt tttttttttt ttgtgtaggt ttgggtgtgt tttttttagt gttgggatgt 3180
ttgggttgtt tgtagttgtt tttatatttt tttttggaga atttttaaat gtagaggttg 3240
gtgattttgt tttttttttt ggagtttggg ataaattttt taggttgtta tttttaatat 3300
ttttttaagt tttaggtttt atttttaaatt tttttgggga gtttgtattt tattttattt 3360
taagtttatt tgttttaata ttaaataatg gttttttttat tatatttttt agtaaaattg 3420
atagttaagg aggggggatgt gttgtgttta ttttgtggtt tttaggattt ttggggtata 3480
tttttttggaa tttttgaagt atttgaaaag tatgatttta agagggttta taaatttggg 3540
aggagatagt tatattgaaa ggattttttt tttttttttaa atgaattttt gtttttgttg 3600
tttaggttgg agagtaatgg tgtgattttt gtttattata atttttgttt ttttggttta 3660
agtgattttt ttgttttagt tttttgagta gttgggatta taggtatgtg ttattatgat 3720
tagttaattt tttgtatttt tagtaaagat agggtttttt tatgttggtg aggttggttt 3780
tgaattttta atttaggtg atttttttgt tttagttttt taaagtgttg gaattataat 3840
tatgagtaat tgtatttagt ttaaaaagat ttttatttta atttttttaaa atgtattttt 3900
tttgtttatt taaggaggta tttagtattt aatgtattta aggttttttt tttgtttttt 3960
gtttttttatt agtttgttta attttatttt ttaattatat tttatttgga gtttttgatt 4020
ttattttta ggtttagtgg taggggtggg gatggaagga agattttttt tttttttttt 4080
aattttaata agatattgta tgggatatat ttatattaat aaattattta ttgtttattt 4140
gaaatttaaa tttaattggg tttttttatt tttatgtgtt aaatttggta gttttagttg 4200
gaatgtttta gttaagaatg tagttatatt ggttaagaag ggattaaagg tatttattag 4260
ggattggaga atgaaggata ttagtatttt tttttttttt tttttttttt tattttttat 4320
atttttttat aggtatatat ttgtattttt aaatttgttt ttatgtgttg agtatattta 4380
aagtaggtat tttttgtggg ataggttgtt atgttatttt agggagttga gaaatagggg 4440
tttttgggat taagaggatt tgggtttaaa ttttgaattt ggttagttgt ggtggtttat 4500
atttgtaatt ttagtatttt gggaggttga ggtaggtaga ttatgaggtt aggagattga 4560
gattattttg gttaatatgg tgaaattttg tttttgttaa aaatataaaa aattagttag 4620
gtgtggtggt gggtgtttgt agttttagtt atttaggagg ttgaggtagg agaatggtgt 4680
gaatttggga ggtagagttt gtagtgagtt gagattgtgt ttttgtattt tagtttggga 4740
gatagagtga gattttgtgt taaaaaaaaa aaaaaaaatt ttgattttat tttatattag 4800
ttatgttatt ttgggtaagt gattgagtat ttttggtttt agttggttta tttgtaaaat 4860
agtatttgat atattatgtt ggggttaaag gagataatgt tttggaagta tttagtgtaa 4920
tatttggtat ataataattg tttaataaat ggttttagat ttagttatat tggggtagat 4980
ttaggttttg tggggtttga agtttatata atttgggaaa ttttttttaa gaaagtaata 5040
taaagttggt tgagtatggt ggtttatgtt tgtaatttta gtattttggg aggttaagat 5100
aggtagatta tgaggttagg agtttgagat tagtttggtt aatatgataa aattttattt 5160
ttattaaaag tataaaaatt aattaggtgt ggtggtaggt atttgtaatt ttagttattt 5220
gggaggttga ggtaggagaa ttgtttgaat ttagagggta gaggttgtag tgagttgaga 5280
ttttgttatt gtattttagt ttgggtgata gagtaagatt ttattgaaag aaagaaagag 5340
agtaagagag agaaaggaat tgaatagaaa aagattttag tatggttggg tgtggtggtt 5400
tatgtttgta attgtagtat tttgggaggt taaggtaggt ggattatttg aggttgggag 5460
tttggtatta gtttgattaa tatggagaaa ttttgttttt attaaaaata taaaattagt 5520
tgggtatggt ggtgtatgtt tgtaatttta gttatttggg aaggttgagg taggtgaatt 5580
gtttgaattt gggaagtgga ggttgtggtg agttaagatt gtgttattgt attttagttt 5640

```
tattattatt tgtattatta ttattattat tattattagt gtttgtttgg taatgttagt     2520
ggtagtttta tttttatgtg tgatgagtgt gggtttttgg ttatgaataa tttttatagt     2580
ttttataagg agatgtttgg tatgagttag agtttgtttt tgttggttgt tatgttgttg     2640
ggtaatgggt taggtggttt ttataatgtg tagtagagtt tgtttaatta tggtttgttg     2700
ggttatgata aaatgtttag ttttaatttt gatgtgtatt atattgttat gttgatttgt     2760
ggtgagtaat atttgttttg tggtttgggt attttatttg tggttatgat gttgtatttg     2820
aatggtttgt attatttggg ttatatttag ttttatgggt tggtgttggt atttagttgt     2880
gagtggttat ttttgttttt attgggtttg taggtggtta tgttgggtta gttggaagaa     2940
attaatatta aagaggtggt ttagtgtatt atagtggagt tgaagtgtta tagtattttt     3000
taggtgattt ttgtgtagag ggtgttgtgt tggttttagg ggattttttt tgatttgttt     3060
tggaatttaa aattgtggag taaatttaaa tttggtaggg agatttttttg taggatgtgg    3120
aagtggtttt aggagtttga gttttagtgt atgtttgttt tatgtttggt aggtaaggtt     3180
ggggttagtt aggggttagg ttgttgggaa gagggttttg ggtttggtgt ttgtggttta     3240
agtttgtgtg ttgagttatt ttttttgatt tttttttttt ttttttttata tatgtttttt    3300
tttttttttgt ttttattttt ttttttattt tttttttttt ttttgttttt tttttatttt    3360
tttttttttt tttttttttt ttttttttatt ttttttttgt ttttgagttt ttattgattg    3420
attttttttt attttatttg tttttttttt aatgtgttaa tttttgtttt atttttgatt    3480
tttttaggta ttgggaggtg ggatgggggt gtgtgttttt ttttaggagt tttgtttttt    3540
taagatttat agaaattagg atttgttttt atttaaaatt ttatgtattt taagtttttt    3600
ttagataata tattttaatt ttttgggttg attagttttt ttgtgtagag gtagttgaga    3660
ggtttttgttt tgtagaggga aaagagtttt ttattttttt atttattata taggtaaatt   3720
tatttggtta ttggttgaag gtatagtttt gtttttgtgg ggaattggtg gttaggatat    3780
aatagtgttt ttggagttta tttttggttt tggtgttggt gtagggattt tttgattggg    3840
tttgaggggt ttgggttagt tttaatgtta ttatttatag tgagggtagg gtgtaaggtt    3900
gagaaggtta tatttattgt tttgggagga tgtgggagaa gagattgagg tggaaagtgt    3960
tttgttttgt ttattggttg tttttgtttt ggttttagtg tttgttggga tttgttagga    4020
tttgttgggg ttttgggaga ttttgagtat ttgtaggaag aggtgttgag aaattaaaaa    4080
tttaggttag ttaatgtatt tttgttgttg gttgtaggtt ttgtttttgt attaagtggg    4140
tgttgattgt gtgtgtttgg tgattgtggg gaggattggt ggtttgtggg aggggatggg    4200
tagaggtgtg ggttatattg ttttggagtt ggtttggttt tttgtgtttt ttttagtggt    4260
taagttgtga ggtatagttt tttattgttt taggagtata gaaatttttt gtgtgggtgg    4320
tgggtgtgtg agttagaggg aaagatgtag tagttattgt gattggtatg tagttgtgtg    4380
tttttgtgtg tatggatttt gtgtggtgtg tgtggtgatt gtgttgtttt taggagtaag    4440
ttatgggttt agaggggtaa aatgtttagg tttttttgttg ggaaggatat attatatttt   4500
atggtaagtt agggtgggtg atttttttatg gattgggtgg agggggtat tttttaggat    4560
tggtgggtgg tttaggggaa taatttgtgg tggtgatgat ttgtatagtg tgggttttgg    4620
gatgtgtgtg gttttgagtt agttttgtat agtttgtttt tggagttgtg agtttaggtt    4680
tttattttttg atttttttggg ttttttttgt attgttgagt ttagtttgtg gggtgtattt  4740
gattaatgtt tgatagggtt ggggaatgtg ataggtagta ggtttatttg ggtttgggga    4800
gggggagttt ttgttttgat agtatttttt tttgttgttt gttggtggat ttttattttt    4860
agttggtaat tgttttgtag tgttgattta agaaggtaaa gaaaattagg ttttttttgta   4920
aagagttttt tttaaattgg tggattttgg atattttgag tggatttaga aatttatgta    4980
attttttttt tttagtttat tttttatttt tttttatagt ttttttttgat ttgttgttgg   5040
tttggggtaa gataaagtag ttagtagaga gtgataataa tagtggtggg aaatgaattg    5100
gagattggtt gatagttttt aatattttgt tatagatttt tttgaatgtt ttaggttgtt    5160
tttggtgggt tttagtattt gttggttttt tgggtattgg ggattagaag gaatttggta    5220
gttggtttta ggggtatagt taaaggtagg atgatagtta tttttttgtt tattttagag    5280
tgttgttgtt tttttatgtt ggttgtgtaa agaatatagt ttttaaaaaa tatgtgtttt    5340
ttgtttatat aggtttgaaa gtgatgagga aagtaatgtt ttgtttatta gtgagtttta    5400
gtttttaaaa tgattttaag tgttgttgag atgagaaagt gtggtatttt ggggggttttt   5460
agttttattt gtgtttatgg tgtaagtttg tagggatagg tttgggatag tattgtttat    5520
gttgttagat tttttgtaga ggattgttga agttgttttt gtgggagata gaatgttttt    5580
tttagtgagt ggaaaaggtt tgttgaggat tttgttttgt ttgagtattt aaatgtgtgt    5640
ttgttttatt attttgggtt gaaaagggat aagagtttta gtttttttat ttggttattt    5700
tattagtaat tataagtgtg ttgagtggtt attattatat aggaggtttt ttagtttggg    5760
gttagtagat tagttttttt agatattgat gtagaagttg ggattggtaa gtaggtatta    5820
tgtgtttgga gtgttagggg ataggagtaa atggagaaga aaagtggagg tttttttttgt   5880
ttggagtatt gattggaatt tttgttggta tgttgtagag ggtttttgtt gttgggtttt    5940
gggggtttaa taagtttagt tgttttgtag gtggtttggt tggattttta gattggtgtt    6000
tggaagatat tgttttttgtt ttttttttgt taaatttgtt tttttttttt tttttatagg   6060
ttataggttt tttttttttt ttattttggt tttgtttttg ggttttgtta aatagttaag    6120
taggttgggg tttagggggt ttagaatgaa gaggtttgat ttggttagtg ttggtaaagt    6180
ttattttttag gtgaggttat aatagaggta ggtttttttt gtttagtttg ttggtgtagt   6240
```

```
tatagttaag ggtggtattt gaaaggaaaa gggagaaaat tttggagaaa tttagattgt 6300
tttaatgtta gattttagag aaattgattt taaatgtatg gatttgtttg gaaagggtgg 6360
ttaagtggta ggtggttgta attttgtttg gttgggtttt tgtagaggtt ttttaagatt 6420
agtttttgta gggtggtttt tagtaatttg ataagaggtg gttaagataa atttttgtgg 6480
gtttgagtat atatttttgg gtgttgggtt ttagagattt ttaaattaag tataaataag 6540
aagggagtga gagaatttag gttagaattt gtatgggtat tttattgagg aaaagtgagg 6600
ttttggtggt aggtatgttt ttttttgatg tttgaaaatt gagttgagtg tttgattata 6660
tttattgtag aggtttttgt ttttagtgag tttggatttt ttagtggttt gtttggagtt 6720
ggtttttagt ttttgttgta gtttgatgta tggttttttt ttggtagtaa gtttttagtg 6780
gttagtttga agttaatttt gtttaggtgg ttgagggttt ttagttaatt tattatgatg 6840
ttgtttgggt tatttgatgt ttgtagtggt gggatatggt ttgggtagtg tgtagtggtt 6900
tttgttaggg gtattgtgtg tgtgtttgtt ttttgttgtg ttggggatgt ttttgggtga 6960
tatgggttgt tgggtatttt ttaagttgag gaaatggatt ttttttgtag agttttgtgt 7020
ttattttta atttttatt ttgtttttg ttgttagggt ttttgattta gtttattttt 7080
tttggtggtt tagttaggga ttagagttgg agaggttgaa tgtaatttgt gttagtatgg 7140
aatagatgat atgtttgttt gttagttgtt tggatgaata attgaaaagt ttgttgtagt 7200
ttgtgttttg ttaagttttg ggtgttggga gaatattttt ttaatatgta ttagggtggg 7260
tgggagtggg tagaggaggt gggatttgag ggaggagagt gaatttgagt aggagaagta 7320
gtttaggtag ttaggtgttt ttgatgtgag aggttgggta tttattttta ttttaggttt 7380
tttattgtgt ggttatgtta tttttttaaa taaatgtgta tatggaggga gattgatgtt 7440
gataatgttt agaagattaa aagagtatta atgttggtaa taataatgta aatgtgtgga 7500
tttagatttt attgatttgg aatttgattt ggtgtgtttt tagtaagttt gatggtgtgt 7560
ttttttagt agagtgttta ttagtgttat ggttttgtgg ttttttagtg gtgttgtttt 7620
gttagttttg tgtgggtttt tttgtttgat tgtagttttt tttttgtgag gttttagttt 7680
gttttatttt tttgaggttt ttttttttt ttgtggggtt ttttgttttt tgtatttttt 7740
tttttgattt ttgtattatt tgtttttgtg tgtatatatt gttatttgtg tttttggtga 7800
tttgtttggg tggttgggtt tgtgaagtta atgtgttgaa tggtgtttga gttttttttaa 7860
ttatttgtg tttggttgtt gttattgggt tttggtgatt aagtttaagt ttgaaaatga 7920
tgtggttaaa gttgtttgtt aatggtagag tttaattagt tgtagatttt tttttatttt 7980
tattggtttt tgtattaaaa aggtttatgt gtagattttt tatgtaggtg tatttgttgg 8040
ataattaaat gtggttttag taataaaagt ttgagtttta ttttttttgag tagtaggttt 8100
tgtgttggat tggttgggtt taatagggag aattttgtgt tttattttgg ttgggatagg 8160
aagtaagaga agtaaattgg ggaattttg ttttattttt ttttattttt tggatgtttt 8220
gggttgaggt ttgggttatt ggtttattgt gggtaaggag gtgttttgtg gaatatttgt 8280
attgattgga aaagaaagaa ttttaaagtt ttttttttt gtttgtgggg gatattatta 8340
tattttgtta attattttt tggttaaatg gtggtttgtt tttttagaag gagtattaag 8400
gtgaatttta tggaaaaaaa ataaaagatt agggatttaa gtgggagtag gatgagaatt 8460
aattttt 8467
```

<210> 97
<211> 8467
<212> DNA
<213> Artificial Sequence

<220>
<223> chemically treated genomic DNA (Homo sapiens)

<400> 97

```
aagaattagt ttttatttta tttttattta ggtttttgat tttttatttt ttttttataa      60
aatttatttt agtgtttttt ttggaaaaat aagttattat ttggttaggg gagtggttgg     120
tagggtgtgg tggtgttttt tataagtggg aaggaagagt tttaaagttt tttttttttt     180
agttaatgta ggtgttttat aggatatttt tttatttgtg atgggttagt gatttgggtt     240
ttggtttagg atgtttaggg ggtgggaagg ggtggggtag gggtttttta gtttgttttt     300
tttatttttt gttttagtta aggtaggata taagattttt tttgttagat ttgattagtt     360
tagtgtagag tttgttattt ggagggatga agtttaggtt tttgttatta gggttatgtt     420
taattgttta atgaatgtat ttgtgtaaaa agtttgtgtg tgagtttttt tagtatgaga     480
attgatgagg ataggagggg atttgtggtt gattaagttt tgttgttagt aggtagtttt     540
agttatgtta tttttaaatt tgggtttagt tattagggtt tagtggtaat ggttaagtat     600
aggatagtta ggaagatttg ggtattgttt agtgtattgg ttttgtggat ttagttgttt     660
aggtggattg ttggaagtgt aagtagtggt gtgtgtgtat aggggtgggt ggtgtagagg     720
ttgggggagg gggtgtagag ggtagagagt tttgtgagag gaggagaaaa ttttggggaa     780
gtaaggtggg ttggggtttt gtgggggagg agttgtggtt agatgggaga atttgtgtag     840
```

```
agttggtgaa gtggtattgt tggaaaattg tggggttgtg gtgttggtga gtgttttgtt    900
gggaagagtg tgttgttggg tttattggaa atgtgttgga ttaagtttta gattaatgaa    960
atttgggttt atatgtttat gttattgttg ttagtattaa tgttttttta atttttttaaa   1020
tattattagt attgattttt ttttatatat atatttgttt gagaaagtga tataattata   1080
tagtggaaag tttggaataa aaataaatgt ttagtttttt gtattgaggg tgtttggttg   1140
tttgggttgt tttttttgtt tgggtttatt tttttttttt gggttttgtt tttttttgttt   1200
gtttttgttt attttgatgt gtgttgggaa ggtgtttttt tggtatttgg gatttgatga   1260
agtatagatt gtagtgaatt ttttaattat ttatttaagt agttagtagg taaatatatt   1320
gtttgttttg tattggtatg ggttgtgttt agttttttta gttttgattt ttaattaaat   1380
tgttaggaga ggtgggttga gttgggagtt ttagtagtga gaaatgaggt gggaggttgg   1440
ggggtgggtg tgagattttg tgaagggggt ttgttttttt ggtttgggag gtgtttagtg   1500
gtttgtgtta tttaaggatg tttttggtgt agtgggagat aagtatgtat gtggtgtttt   1560
tagtaggagt tattgtgtat tgtttgggtt gtgttttgtt gttgtgggta ttaggtggtt   1620
taggtgatat tatggtgggt tggttaaggg tttttggttg tttgaataga attggtttta   1680
gattggttgt tgggagtttg ttgttaggag agggttgtgt gttggattat ggtggggatt   1740
ggagattagt tttgggtagg ttgttggggg atttgggttt attggaggtg gaaatttttg   1800
tagtaaatgt agttgggtgt ttggtttgat ttttgggtgt tggaagaaaa tatgtttgtt   1860
attgaggttt tgtttttttt tagtgggatg tttgtgtaag ttttagtttg ggtttttta    1920
tttttttttt gtttgtgttt ggtttagagg tttttggggt ttaatgtttg agagtgtgtg   1980
tttgaatttg tagaaatttg ttttggttgt tttttgttag gttgttgaaa attgttttgt   2040
aagggttggt tttggggaat ttttgtgaag gtttgattgg gtggggttgt aattatttgt   2100
tatttagttg tttttttga atgaatttgt gtatttggag ttaatttttt tgaaatttaa   2160
tgttggggta atttaaattt ttttgaagtt tttttttttt ttttttaag tgttattttt    2220
ggttgtgatt atattggtag gttgggtagg aaggatttgt ttttgttgtg attttgttta   2280
agggtgagtt ttgttggtgt tggttaaatt agattttttt attttgagtt ttttaaattt   2340
tggtttgttt ggttgtttgg taggatttgg gggtggggtt aaaatgagag agaaagggaa   2400
tttataattt atgagagaaa gagaagaggt aggtttggtg ggaggggggt agggatggtg   2460
ttttttaggt attggtttga gagtttggtt gagttgtttg tggagtggtt gggtttgtta   2520
aatttttggg gtttaatggt gagggttttt tgtggtgtat tggtggggat tttgattgat   2580
atttgggtg gagaaagttt ttgtttttttt tttttatttg tttttgtttt ttagtgtttt   2640
gagtatataa tatttattta ttagttttag tttttgtatt agtgtttgaa gagattgatt   2700
tattgatttt aaattgaaaa gttttttatg taataataat tatttaatat atttgtagtt   2760
gttgataaaa tggttagata aaaaggttaa agtttttgtt ttttttttaat ttagggtaat  2820
aaaatagata tatatttgaa tgtttgagta aagtggggtt tttagtaggt ttttttttatt  2880
tgttggagga ggtattttgt tttttatgaa ggtagtttta gtgatttttt gtggaaaatt   2940
tagtagtgtg ggtagtgttg ttttgggttt gtttttgtag atttgtatta tgggtgtggg   3000
tggggttgag gatttttggg atgttatgtt tttttatttt agtaatgttt gggattattt   3060
taaaagttga aatttgttaa taggtgaagt attatttttt ttattatttt tagatttata   3120
tgggtagaag gtatgtgttt tttaaaagtt gtgtttttttg tgtgattggt atgagggggt  3180
ggtagtgttt tgagatgagt aggagaatag ttgttatttt gtttttaatt gtatttttaa   3240
gattagttgt taagtttttt ttggttttg gtgtttaaga agttggtggg tattaaaatt    3300
tattagagat agtttgggat atttgggggg atttatgata aaatgttaag aattgttagt   3360
tagtttttag tttatttttt gttgttatta ttattgtttt ttattggttg ttttattttg   3420
ttttgaatta atagtaaatt agagaaaatt gtaggagagg atgaaaaata aattaaagga   3480
gaaagattat ataaattttt aaatttattt aaagtatttg gagtttgttg atttggggga   3540
ggtttttgt agggaaattt agttttttt attttttag attaatattg tggggtgatt      3600
attgattggg aataggaatt tattagtaga tggtaaagga aaatgttgtt aaagtggaaa   3660
tttttttttt ttaagtttgg gtgaatttgt tgtttgttat atttttttagt tttgttgggt  3720
gttggttgag tgtattttat aagttgggtt tagtggtgtg aggaaagttt gggggattgg   3780
gggtggaaat ttgagtttgt agttttggaa gtgagttgtg tgaggttggt ttggaattgt   3840
gtgtatttta agatttgtgt tatgtaaatt attgttatta tgaattgttt ttttagattg   3900
tttgttgatt ttgaaagata ttttttttta tttgatttat gggaagttgt ttattttggt   3960
ttgttatagg gtatagtgtg tttttttag tggggatttt ggatatttg tttttttggg     4020
tttgtggttt gtttttaggg gtagtgtagt tgttatgtat attgtgtggg gtttgtgtgt   4080
ataaaagtgt gtaattgtgt gttagttgta gtaattattg tattttttttt tttagtttgt  4140
gtatttgttg tttatatagg aggtttttgt gtttttagaa taatagaggg ttgtattttg   4200
tagtttggtt gttagaggag gtataaagag ttgagttggt tttagaataa tgtaatttgt   4260
gtttttattt gtttttttt gtgggttgtt agtttttttt gtggttgtta ggtgtgtata    4320
attagtgttt gtttaatgta aaggtggagt ttgtagttgg tggtagggat gtattaatta   4380
atttgaattt ttaatttttt agtatttttt tttgtgagtg tttagggttt tttggagttt   4440
tggtaaattt tggtaaattt tagtaaatgt tgggattggg gtaaggatgg ttggtgagta   4500
aggtaaagtg tttttttattt tagttttttt ttttatgttt ttttaaagtg gtgaatgtga  4560
tttttttaat tttatatttt gtttttgttg taggtagtga tattggagtt ggtttgagtt   4620
```

```
ttttaagttt ggttagaaag tttttgtgtt aatgttaagg ttagagatgg gttttaggag    4680
tgttgttgta ttttggttgt tggtttttttg tgggggtaag gttgtgtttt tagttaatga   4740
ttaaataagt ttgtttatat ggtgggtggg agagtagaga gttttttttt ttttgtagag    4800
taaagttttt taattgtttt tgtataggga gattagttag tttggaaaat tgaaatgtgt    4860
tgtttaaaag agatttgaag tgtatggggt tttgaataag ggtaggtttt ggtttttgtg    4920
ggttttggaa agatagggtt tttagaggaa aatgtatatt tttattttgt tttttagtat    4980
ttgggaagat tggaaatagg gtaaaggttg gtatattgag gggagggtga atgaaatggg    5040
gggggggttgg ttaatgaaag tttagggata aggagagagt aagaaagaaa aagaaaaggg   5100
agaagggaaa gtaggggaag agtggaagag aaagagaaaa tggaagaaga aataaaaatg    5160
agaagaaaga ggatgtgtat aggaaagaga aggaaagaat taagagaagt gatttggtgt    5220
gtagatttgg gttataagta ttggatttgg agtttttttt ttagtagttt ggtttttggt    5280
tagttttggt tttatttgtt aggtgtaagg tggatatgtg ttggaatttg ggtttttgaa    5340
gttattttta tattttgtgg aaggtttttt tgttagattt gagtttattt tatggttttg    5400
gattttggag taggttggag agagtttttt gagattggta tagtattttt tgtgtaaaga    5460
ttgtttgggg gatattgtag tgttttagtt ttgttgtgat gtgttgggtt atttttttgg    5520
tgttgatttt ttttagttgg tttgatgtgg ttatttgtga gtttgatgag gatgagggtg    5580
gttgtttgtg attgggtgtt agtattggtt tgtgagattg agtgtggttt gggtggtgta    5640
ggttgtttag gtgtgatatt atggttgtag gtggggtgtt taggttgtgg gataggtgtt    5700
gtttattgtg ggttagtatg gtagtgtggt gtgtgttgaa gttggggttg agtattttgt    5760
tgtggtttgg tggattgtag ttgggtagat tttgttgtgt gttgtggagg ttgtttagtt    5820
tgttgtttag tggtgtggtg gttagtgggg ataggttttg gtttatgttg ggtatttttt    5880
tgtagggatt gtagaggttg tttatggttg ggagtttgtg tttgttgtgt atgagggtga    5940
agttgttgtt gatgttgttg gataggtgtt ggtggtggtg gtggtggtgg tggtgtggat    6000
ggtggtgtgg gtgagggtgg tggaatttgt tagatatggt ggagatgggt ggtagtggtt    6060
ggagtggtgt tagtgtggtg taggtgttgt ttatgtttat gttaggtgga gatgagttgt    6120
aggatatgtt tatggtgtgg tgtagtggga tggagagttt gggttggtag ttgttgttgt    6180
ttaggattga ggttatgttg gtgattatgg ttgagtgtga tgttgttgtt gttgttgttg    6240
ttgttgtgtt tagtttttgg tgtgtggttg gaggtggtgg agggttttgt agtgagttag    6300
tggtgttgtg gtttgtgtgg tgggggttgg ggttggttag tagtttttgt ttatggtttg    6360
ggtttttgtt gttgttttttg ttgtttttgt tgttgttttt gttattgttg ttgttggttg   6420
gtttgtgtaa agtgtttaga tttttttattg ttagttttgg gtttatggtg tagtttttta   6480
ggtttttggt gaggtattga taggtggtgt aggtagtttt tatttagttt attagggttt    6540
gggggtggtg ggggtggtgg gggtggttgg tgggtgggta aggtgtgtgt ggtggggttt    6600
ggttgtggga gtgggggtgg gtgtgggagt gagtggagag gagtgtgtgt gtgggtgtgt    6660
gttttgggtt gtgggtgggt ttgtttgggg tgggggtggg gtgggggtgg atggggtgtg    6720
tgtgtgggag aggggagggg atggggaggg agggagggat tattgggttt tgttgtttgg    6780
gttggtttgt agttttgtta tgtttgagtt tgttttggtg ttgatgtttt ttgtttgggt    6840
gagtgggagt tgtttagaag ggttttgttg tttgttgggg tagttaattt attttttttgg   6900
atttggggtg gggttatgtt tgatgggtgt taagtgtaat tattttgagg ttgtagttgt    6960
tttgggaggt gggtttttta tgggaagtta ttagtggttg tggagggggt ggggttgtat    7020
tttttaggtt tggttgatgg tttggtgttt tttttatttt ttttgttatt ggaattaaat    7080
gttaaggaga gggagggagg agaaggtggg ggggttgttg gggagaggga ggttggagtg    7140
tgagtgagtg agagagattt ggtgaaagat gttgtgttgt gttttttttg agtagttgtg    7200
atttggggga aggggtattg ttttaattgt ttttgtttta ttttaaagtt tttttggagg    7260
tgagtaagtt gggagtaaaa gattttgtta tgtgttagta tagataaata tttgttatta    7320
ataagttatt tattgagagt ttttagttgg gagttaatag tttagttttt taaaatgtag    7380
gtgttaatgt attttaatgt atggtaaatt tttgaggttg ggatatgtat aatttatgtg    7440
gttagagtaa ttgtagtgtt tttgttgtta gtgagttgtg gttggttggt ggtttatggg    7500
tggtgtgggt agtagtaatg tatgtaaagt taatataata atattattta attatttggt    7560
gagtttataa attttagtta tgaggttttt gaagagtttt gttagattgt aaaataaaat    7620
tagaattttg ggagttgttg ttttaaggtg attaagtgtt ttttgtatta gtgatttgga    7680
aagaagtggg gagagtgtag aattggtgag gtggaggtga ggagggttgt tggtgtttta    7740
agtgagtaaa tttttttttgg gggtagagag taagtaaatt agttgagatg gtaattatgg   7800
attttttttt taatatgaga tatatatatg tgtatatata tatatatata tatatatata    7860
tatttttgtt ttttttaaat tttgggagag aaatggtggt gggtagatga taaggaattt    7920
tggttgatat ttgtatattt aaattttgtt tttgaaaaga ttagtgtttt ggttagtttg    7980
agaatgttta taagttaagg ttttaaaaat tgtatttatg ttttttttaga atttttgttg   8040
tttggtaatt ttaggtttgg aggtgggagt ggaattgggg gatagagagt tggagatttt    8100
tgtagtttga gttggggtgg aggggtagtt gaagatagag aggttaatat ttggtataaa    8160
gttttaaatg taataagatt tttttaaagt aggttgtgtt ttgtttgttt ttgaagattt    8220
aagttagttg aggttgtgta gatgttgttt ttgtttaaag agatagtaat taaggatagg    8280
gtggatataa gtttgagaag tgtgatttat ttttttttga ttagttgtta aaagaagagt    8340
agggggtttt tttaagatag tttttttttaa ttatttttag ttgttagaga aaatttgata   8400
```

agaaaataag ggtagagttt ggaatattgt atgttttttt ttggtgttag agaaaggttt 8460
gatttat 8467

<210> 98
<211> 4453
<212> DNA
<213> Artificial Sequence

<220>
<223> chemically treated genomic DNA (Homo sapiens)

<400> 98

```
ttggttttga agtttatagt attgttgatt tagtttgttt tttggaaggt tggtagttta      60
gtaagtatag aagttttttt agaagatagt gggttatttg tttttaaaa gttgaaaggt      120
taatttgtat tttttttagt aggtagttgg tattttgagt ttttggttgg ggtagagtaa      180
aggagttttt ttttttttta ttttttggt atttttttg ttttttttt gttattttta      240
ggtggattta gatttaaggt ttagatttgt aaggtaggaa aatgttgtag gtttaggttg      300
ggaaagggtt taaagttgtt agtggattgt tgggatttag ttttttttt tttattaaga      360
gagtgagttt tattgggttt aaaatgattt taagttttgg tttttgatat taggggaaag      420
agatgggggt gatagaatta tagaattttt gttatgtttt tttaagtgtg tttagagatg      480
tgtgtgtgtg tgtgtgtgta tatataaatg tttgtttatt tttaggtagg aagggtggat      540
gtagttattt atatatggtt tgtttttttg gaggataatt ttatttgata aataattgtt      600
tttatttgaa tagaataaat aaggtttat gatgaagtaa aatattaaat atatatgtat      660
taaaaaatgt ataattattt ttttggaatg ggttatatag agatgtgttt tttaaaatgt      720
taagagtgta aaaggataaa tagtgaaaaa taaatttttt ttttatttg tttttagtt      780
ttttaatttt tttatttaga ggtgagaata gaattttat atttttaga atttttatag      840
ttagaattgt ttatatgttt ttattgtttt tattttatt tttgtttgta taaataaatg      900
aattgtttat tatggaaatt ttttaaaaga tttgttaata ttttaatagg aagtattaat      960
agtttatgtt ttaggatttt gtttttataa ttttgtaata ttatattatg atatttaatt      1020
taatttttat taagttttgt taaaaatgga ttttaaatta agttgtaaat ttttagtaat      1080
ttggttttgt ttttttttt ttgatagtat tattaaataa atttttttat tgttgaaagt      1140
aataagtttg gttttgtttt atttattggt tgtgttggtg atatttgggg attgttattg      1200
aatagatgta tagagggagt ttttataggt aggggttttt ttgtttgtgt ttttgggaga      1260
gtatgttttg tatatttgtt gtgttgatga agattttata gttttattag ttgtgggtaa      1320
gggggtttga ggtagtttta ggtaagttgg ggtttagtgg ggagaagttg tagaagaatt      1380
gattagagga ttttaggagg ttttagagtt gggtgaggta gagagttttt tgtgtgtttt      1440
ttttttttt tgtaatttgg ggattttttg tattggggta ggtttttggt taggtgtatg      1500
ggaggaagta tggagaattt ataagtttt tgattttta gtttagatgt tgttgggttt      1560
tttttgttgg agattgtgtt tttttaaat ttttgtgagt gttgtggaag tatgtggggt      1620
ttgggttgtt gagtgttgta agatagggga gggagttggg tgggagaggg aggggtggtg      1680
ttggggtggg ttttgatata gagtaggtgt tgtgggttgt agtatagtgt ggagattgta      1740
gttttggagt ttgggttagg gtttatttgt ttttgtagtg ttggtttgtg ttttttgtt      1800
gtagttattg gtgagtgttg tggttttgag atttttgggt tggatgtgtg gtggttttag      1860
tttttgagtg tttgtttttt ttgttttggg ttgtttgggt tttttgggtt ttttggtggt      1920
tgtatggagt taaggtgttt tgttttgggt gtttttttgtg ggtgttgatt taggttgttt      1980
ggagtttgga gtttagagag gagagagata gttggggagt ttggttattg tgggtatttt      2040
ttttgtgttg tagttgtttg tttggtttgt ttttttgttt ttttgttttt tgttttgatt      2100
ttttttttt ttgtagagtt gttgtttagt gttttgattt tgttattatg agagttttgt      2160
tggtgtgttt gtttttttgt gttttggttg tgagtgattt taaagtgagt gtgtttttgt      2220
tttgattgat gttgtttaag gatttttgat tagtattagg ggagaggagg ggttgtttag      2280
ggagttgggg ttttttggat tttatttata gtagggttag atttttttta ggaaatggga      2340
tagggtggta gtggaggttt gagaattatg ggggttggta ttggttggta agggaggaag      2400
aggttgttgg gattgtttta gtttgtgggt atttggtaga tgaagtttgt ttgggttaat      2460
ttattttttt tggttggaaa tttatggttt tttatttgag aattagatat gaatagggtg      2520
aggtgagagg gagagggaag agtgggtttt gggattgggg ttagtttatt tttattttgg      2580
agtttttgga gtatgggatt tttgatgaag tttttttttg aatttttttt agggtagtaa      2640
tgaattttat taagttttat gtgagtattt atttttataa tagttggttg tatagataag      2700
ttgggaaggt tttaggggat atttttttt tgttttttgt tgtagggttg tgttattttt      2760
tattattttt atttttttt gtttatttta tttttgtttt ttttagtgaa ttgtgattgt      2820
ttaaatggag gaatatgtgt gtttaataag tattttttta atatttattg gtgtaattgt      2880
ttaaagaaat ttggagggta gtattgtgaa ataggtatgg ggatttttat tgtaattggg      2940
agagaaattt ggggataggg agggatgggt gggaggtaag agtaggtagg agttaggagt      3000
```

```
tggaggtagg gtgggtgata tttttatttt tatgtgataa gtataaatat atatatatgt    3060
ttatgaaata gtggttatat aaatgtgagg tggggttgga aggagatttt gtttagtttt    3120
ttggtaggtt tgaaatgata tttttaaaat gtttgttggt agttgggtat ggtggtttat    3180
gtttgtaatt ttagtatttt gagaggttaa ggtgagtgga ttatttgagg ttaggagttt    3240
aagattagtt tggataatat ggtgtaattt tgtttttatt aaaaatgtaa aaattagttt    3300
ggtatggtag tggatgtttg tagttttagt tatttgggag gttgaggtag gagaattgtt    3360
tgaatttggg aggtagagat tttagtgagt tgagattata ttattgtatt ttaattgggt    3420
gatagagtaa gattttattt taaaaaaaaa aaataaaagt tagttggaat gttttttttt    3480
ttttatatt tttttatttt tttgtttttt tgtagataag ttaaaaattt gttatgaggg    3540
gaatggttat ttttattgag gaaaggttag tattgatatt atgggttggt tttgtttgtt    3600
ttggaatttt gttattgttt tttagtaaat gtattatgtt tatagatttg atgtttttta    3660
gttgggtttg gggaaatata attattgtag gtgaggtggg ggtaataagg attaaaagtt    3720
tttttatag tttttttagaa attttgttat tatttttttt ttttagaggg ttggttatag    3780
tataagagaa gtgtggtttt tggttgagtt tttttgagg ggaggaggta gggaaggttt    3840
tttgggttgg aatgatattt tttatttttt tgtgttgtta ggaatttaga taattggagg    3900
tgattttggt gttatgtgta ggtgggttta aagttgtttg tttaagagtg tatggtgtat    3960
gattgtgtag atggtgagta ttattgattt gttgatgata gtggggtgga aggggataaa    4020
tttatatgtt tttttatttt attataggag gattgaggag gtggggggtg tttgagaggg    4080
atgtttttt ttatttgttt ttttaagata ttttttttgtt tgttttttag gaaaaaagtt    4140
tttttttttt ttagaagaat taaaatttta gtgtggttaa aagattttga ggttttgttt    4200
taagattatt gggggagaat ttattattat tgagaattag ttttggtttg tggttattta    4260
taggaggtat tggggggggtt ttgttattta tgtgtgtgga ggtagtttta ttagtttttg    4320
ttgggtgatt agtgttatat attgttttat gtatggtttt gggttttttt tttttgattt    4380
ttttgtttta ttttaagtat attttttttt ttttttagt aaagtgtttt gttttatttt    4440
tttttattt gtt                                                        4453
```

<210> 99
<211> 4453
<212> DNA
<213> Artificial Sequence

<220>
<223> chemically treated genomic DNA (Homo sapiens)

<400> 99

```
ggtagatgag ggagaaatga ggtggaatat tttgttggga aggagaaagg gatgtgtttg      60
gggtggggta gaagagttga agaggagaaa tttagggttg tatatgaagt agtgtgtggt     120
gttgattatt tagtaagggt tgatgaggtt gttttatat atgtaggtga tagagttttt     180
ttggtgtttt ttgtagatgg ttgtaaatta gggttggttt ttgatggtgg tgaatttttt     240
tttaataatt ttaaagtggg gttttagagt tttttggtta tattgaaatt ttaatttttt     300
tggaggagag gagggttttt tttttggagg ataaatagag ggatgtttta gggaggtagg     360
taggagaaag tatttttttt gggtattttt tattttttta gttttttttgt gatggaataa     420
ggggatatgt aagtttgttt ttttttattt tattgttatt agtaggttag tgatgtttat     480
tatttgtgta gttatgtatt atgtattttt ggataagtag ttttaggttt atttgtatat     540
agtattaggg ttgtttttgg ttgtttgggt ttttggtaat atagaaagat aggggatgtt     600
attttaattt agagggtttt ttttgttttt tttttttagg gaagatttaa ttagaggttg     660
tatttttttt gtgttatggt tagtttttttg ggagaagggg atggtaataa ggtttttggg     720
aagttgtagg gagggttttt ggtttttgtt gtttttattt tatttgtagt aattatgttt     780
ttttaggttt agttgaagag tattagattt gtgggtatgg tatgtttgtt gaaggatagt     840
ggtagagttt tagggtaggt agggttggtt tatggtgtta gtgttggttt tttttttggta     900
aaagtgatta ttttttttat agtaggtttt tgatttattt ataaggggat aggaggatga     960
gagaatatga gaaagagaag aatattttaa ttaatttta ttttttttttt ttgagatgga    1020
gttttgtttt gttgtttagt tggagtgtag tggtgtgatt ttagtttatt gagattttg    1080
tttttttaggt ttaagtaatt tttttgtttt agttttttaa gtagttggga ttataggtat    1140
ttattattat gttaggttga tttttgtatt tttagtagag gtagggttat attatgttgt    1200
ttaggttggt tttgaatttt tgattttaaa tgatttattt attttgattt tttaaaatgt    1260
tgggattata agtgtgagtt attatgtttg gttgttaatg gatattttaa agatgttgtt    1320
ttagatttgt tagaagattg gatagggttt tttttttaatt ttattttata tttgtgtggt    1380
tattgttttg tgagtgtgtg tgtgtgttta tgtttgttat atagggatga agatgttatt    1440
tattttattt ttagttttta atttttgttt gtttttgttt tttatttatt ttttttttgtt    1500
tttaaatttt tttttttagtt gtagtggaga tttttatatt tattttatag tgttgttttt    1560
tgaatttttt tgggtagttg tattagtgaa tgttggagaa gtatttgttg gatatatatg    1620
```

```
tttttttatt tagatagtta tagtttgttg gagagaataa aggtggggta agtgaggggg   1680
agtggaagtg gtaaggggtg gtgtagtttt gtagtagagg gtagggaggg gatgtttttt   1740
gaagtttttt taatttgttt gtgtagttaa ttgttgtagg ggtggatatt tatatggaat   1800
ttgatgaagt ttattgttgt tttggaagag atttgggagg aggttttatt aaaggtttta   1860
tgttttaggg attttagggt gagggtaaat tggttttaat tttaaaattt attttttttt   1920
tttttttttg ttttattttg tttgtattta gtttttaaat ggaagattat gggtttttag   1980
ttaggagaaa tggattgatt taagtaagtt ttatttatta gatgtttgta ggttggggta   2040
gttttggtgg tttttttttt ttttgttagt tagtgttaat ttttgtggtt tttaagtttt   2100
tgttgttatt ttgttttatt ttttggggag agtttggttt tgttgtggat ggaatttgga   2160
ggattttagt tttttgagta gttttttttt tttttggtgg ttgattagag gtttttgggt   2220
agtattagtt aaagtaagag tgtatttatt ttggagttgt ttatgattag gatgtagaga   2280
agtaggtgtg ttagtagggt ttttatggtg gtgaggttgg ggtgttagat ggtggttttg   2340
taaaggaagg agaagttagg gtaagaggtg gaggaatggg aaggtaggtt aggtgggtga   2400
ttgtagtgta ggggagatgt ttgtggtgat taggtttttt agttgttttt tttttttttg   2460
ggttttggat tttgggtagt ttggattggt atttgtgggg gatgtttggg atggggtgtt   2520
ttgattttgt gtagttgttg gggagtttag ggagtttggg tagtttaggg tgggggaggt   2580
agatgtttgg gagttggggt tgttgtgtat ttggtttggg gattttagga ttgtggtatt   2640
tattggtggt tgtggtagga gggtgtgagt tggtgttgtg gggataggtg gattttggtt   2700
tgggttttgg ggttgtggtt tttgtattgt gttgtgattt gtggtgtttg ttttatatta   2760
gggtttgttt tggtgttgtt tttttttttt ttgtttggtt tttttttttg ttttgtagtg   2820
tttagtgatt tggattttgt gtgtttttgt aatgtttata aagatttggg ggaagtgtga   2880
tttttagtgg aggggattta atagtgtttg gattgaggaa ttgagaggtt tgtaaatttt   2940
ttgtgttttt ttttatgtat ttggttgggg gtttgtttta gtgtaaggag tttttgaatt   3000
gtagagagga gagaaggtgt ataggagatt ttttattttg tttagttttg aagtttttttg  3060
gggtttttta attagttttt ttgtaatttt tttttgttgg gttttaattt gtttaagatt   3120
gttttagatt tttttgtttg tagttgatgg agttgtgaag tttttattaa tgtgataaat   3180
gtatgagata tattttttta gaagtataga tagaaaaatt tttgtttgta ggggtttttt   3240
ttgtgtgttt gtttagtggt agtttttaga tattattaat ataattagtg gatggaataa   3300
agttgggttt attgtttttg gtagtaaggg ggtttgtttg atggtgttat tagaggggga   3360
aaggtaaggt tagattattg aaaatttgta gtttggttta aagtttgttt ttgatagggt   3420
ttgataagga ttgggttagg tgttgtgata tgatgttata ggattgtggg aataaagttt   3480
tagggtataa attgttggtg tttttattg aagtgttaat gggtttttttg ggaagttttt   3540
ataatgagta atttatttat ttgtgtaggt aagaataaaa gtaaagataa tggaaatatg   3600
tagatagttt taattgtgga ggttttggag ggtgtggaag ttttgttttt atttttgagt   3660
agaggaattg ggagattgga ggataaaata agaggaagat ttattttttta ttgtttgttt  3720
ttttatattt ttaatatttt aaaaagtata tttttgtata gtttatttta aaaagataat   3780
tatgtatttt ttaatgtatg tgtatttagt gttttatttt attatagagt tttgtttatt   3840
ttatttagat agaaataatt gtttattaaa taaaattgtt ttttagaaaa atagattatg   3900
tgtaaatgat tgtatttatt tttttgtttt gaggataagt agatatttgt gtatatatat   3960
atatatatat atgtattttt gggtatattt ggaggaatat agtagggatt ttgtgatttt   4020
gttattttta tttttttttt ttagtgttag gaattagggt ttggggttat tttgaattta   4080
gtaggatttg tttttttagt gggaaggagg aggttgagtt ttagtaattt attagtggtt   4140
ttgggttttt ttttagttta ggtttgtagt atttttttgt tttgtaaatt tggattttgg   4200
gtttgggttt atttgagagt gatagaagga aggtagggag agtgttagga aggtaggaag   4260
gaggaaggtt tttttgtttt gttttagttg agggtttagg gtgttagttg tttgttgggg   4320
aaagtataag ttagtttttt agtttttggg aggtaggtga tttattgttt tttggagaga   4380
tttttgtgtt tgttgagttg ttagtttttt aggggataga ttgagttagt gatgttatag   4440
gttttagagt taa                                                      4453
```

<210> 100
<211> 10490
<212> DNA
<213> Artificial Sequence

<220>
<223> chemically treated genomic DNA (Homo sapiens)

<400> 100

```
agaaggtgtt tgtttttttt ttgttttttg ttatgattgt aagtttttttg aattgggagt      60
tgattaaatt tttttttttt ataaattatt taggtttaag tatttttttta tagtagtgtg     120
aaaataaatt aatattttttt ttttgaggtg tttttttttt aggtaatttg ttgtttttat     180
gttttttttt tgttttttgt tttttttttt ttttttatga ggtttaagtg ataaatgggg     240
```

ttagttttag ttttagtttt agttttagtt ttattttatt gtaggtttgt gtggttgttg 300
gagaggttgt gttttttttt tttttttgag tttgtttgat atgtttttttg gattttggag 360
gaaattgatt ttttattttt atattggtgt aatatttttt aagattttaa agttgtatta 420
tttgagttag tttttttttt tatttttatt tgttagggtt gttattggga tagttttaga 480
gggtggtgtt aatggatgaa tggatggatg gatagtagtt tagggatgat gtttttgttt 540
gttttgaatt gggttttttt tttaatgaga agtttttttg agtgagtata tatagttatt 600
ttttggtatt tatggaggat tagttttagg gtttttggga atgttaaaat ttatggatgt 660
ttaagttttt gatataatgt ggtttagtat ttatgtataa gttatgtata tttttttgta 720
tatgttagat tgttattaga ttatttatga tgtgtaatat aatgtagatg ttatataaat 780
ggttgtgata ttgtattttt tagggaatga tgataagaat aaagtttgta tatgtttaat 840
agaaatataa ttgtttaatt tattttttga atatttttta tttgttgttg ttgaatttat 900
agatgtagag tttttggata tgagagttaa gtgtgttttg agagtagggt gggtgaggtt 960
gttaatgagt ataggggagt aggtgttgat taggaggatt ttgtattggg gtatttggat 1020
gttttgtttt aggatttgag attttagttg gatggtatag gtagatttag tttaggttaa 1080
agttgttttt ttgaagtttt tttttatttt aagttttttt tggattttgg aatttggtat 1140
tttttaggtt ttgtgtggaa ggatagatga attaggtttt agataatatg tttagaagag 1200
tgagttttta ttgtgtgttt ggtatttttt ttataggatt ttttagttag aatatttaag 1260
ggttatggag agaaatattt agttaaaata ttagaaaaga aaaagtgata ttattagaga 1320
tattaaaaag attattaggt aatagtatta gtttttgtat tttgagattt aatagtagta 1380
gttatttttt tttattgtta tgtgtatttt aggattattt tgggtgggga gggttgtggt 1440
tagggagtag ttatggatgt tttgatgttg gttttgggtt ttgggggtga tagtgatgag 1500
gaattgggtg tatatatgag tggggtagtt gggtttggtt agagaagtag tatatatgtg 1560
tatagatgtg tttatttata tatatatgtg tatgtatgtg tataaatata ttgtaggtag 1620
gtatgttgat gttttaggta gtggaggatt ttgattttgg gtgttgttga tttgggtaag 1680
gttttattgt gatttgtgtt atgattttag aatgttattg gtgtttagta tttgtttgtt 1740
ttttggtttg ttttagtggt ttatagtagt tatatatagg tagtggtatt tgtgagtagt 1800
tttgtggatt taaaggtttt ttttttgaga ggtatgattt aggttagttg atttattaga 1860
attaggtgag tgtgatttgt tttttttttt ttaggtggat ttggggatag tggttatggt 1920
gtgggtggtg ttggtttttg tggggtagtt attgaggagg gttatttttg agtatttatt 1980
aggtgtttgt tttatattgt ttgtgtagat gattggtttt tttgttttta tggtggtttt 2040
gtagagtggg tgttgttttt aaatgttttt atttgataga tgagatgttt ggggttagag 2100
aggtagtaat tggtttggga atttggatat gattttgagt tttgttttta gttttgttgt 2160
gtgttgtgtt ggaatttagg tttgaatttt gtgatttttt tgttttagat tttaaatttg 2220
tttaggtttt ttattttgat ggggtagagt ttggttttgg tagagttatt ggtatagagt 2280
tattggtata gatttattga tggtttttag aatatttttg tgttttaagt tgggttttga 2340
tggttgttgt gggttttatt gaatatatat ggtttttttgt ttaggggagt ttgttgtttt 2400
tgggtagttg tggaaaatga aggagttttg gagggttggt tgaggggaga ttattttttt 2460
ttgtgtttaa aggggtttgg gtattagggt ttttttttagg tatttttttgt tttgtgtggt 2520
tttttttgagg ttttgttttt tttttgtttt gagtattttt aggagggatg gtttatttag 2580
ttgtttttta ggattaagga tttattgttt ttttttagtg atttaggaaa atgaagtttt 2640
tttttgttgg gatggtttag aatggtggat tttatagttt ttttgtgaga gatgtggttt 2700
ttatgtgtat aatagatttt ttttattttt taaatttaat atttttttgt ttaataggtg 2760
ttatttttaa agtggtttta ttgtttagat tgaagagtta tggtagttaa agtgatgagt 2820
ggagtagaat tgagtagttg ggagagattt tgtttttttgt aggaaattgg gtattgttga 2880
ggttttgagt attttaggag gttgattgta tagagatttt tggttgttga ttttagtttg 2940
tttttatatt tttggaatag tttattatgg gtttttttatt tttggtaggt ggaaattatt 3000
taatttgttg gggttggtgt gtttttattt tatggtattg ggggataata ggattttttg 3060
tttaggtttt attgtattta agtttttggg aagatgttta tttttgtttg ggatttgaga 3120
ttttagagat tggagtagtt gtgggttatt gggtttggtt tttttttttt tgggggtggt 3180
ggtggaatgg gggttatgta gttagttagt atttgggagt ttggtgagag tggtttaggt 3240
gtttttttgaa gttgttgtgt atagtgtgat ttttagataa ttttgtttta taggatggat 3300
gtggtagagg ttgtgggtag ttggtgggta taagagtgag aggatattat tatgaaatat 3360
gaaaaggtat aagttggttt gtttttttgga gggaggtttt ttttagtgtg ttttggttaa 3420
agggtttttgg gtttttttagg agtatagggt agggatgggt ggttaatgtt tttaggtttt 3480
tgtatttttt attttggatt ttttattaag gtttttttttg ggttataggg atattgagtt 3540
gggttgttag aggataaggg gtttaagttt ttttgaagtt ataataataa tgttgattat 3600
ttggggattg tatagtgagt tttttgtatt tttttttattt ttaaagtatt tgttttagtt 3660
tagggatggg tttgttttta gaaaggtttt tttgatgtag gatatgtttt attaggttgg 3720
gttaattttt tttttaggga tagaattttt ttttgatttt tttgtaggtt tagtttgagg 3780
ttgttaggtt agaggtgtgg ggtttattta gggagttggt gggaatggag attgggttag 3840
gttaggtttt tgggtgttta gtagttttgt tggtaagtga gtataagagg agtggggtag 3900
tttgagggtt tggttttgtt tatttggaga taattttggt gagatgtaag ggttatggtt 3960
atagggtgag gggatgtttg gtttagtttt agggttgttg tttagtaggt ttttgagggt 4020

ttatttgttt ttgttttttt ttattttttt agagttatag tttttattgt tttgtgaggg 4080
gaaaaggtat ggtgataatg ggggttgtag ttttaggaga atgggggaga agatgggtag 4140
ggttttgttt tgggtatttt atggtgaggt tagggaggta gtagggtttg tggttaaaga 4200
tttgggtttg gtgttgggaa gggatttggg gttgggtaag aggagtttag ttaggagttt 4260
atttttttagg gattatagga tggagagata gaggattttt ggggaggtag ggtgggaggg 4320
agttgatgag ttgtgttatt tttgaaatgt agggtgtgtg gtttgggtgt agggagaggt 4380
aggtggatgt tgggaggtta gaatttgtaa gggttttggg gttgttaagt ggggtgggtt 4440
tttggtgtag ttagagtata ttgggtaggt tttagggtag gtttttttga ttttggtggg 4500
gggatgtggt tatttttttga gggattttttg ttagggtttg gttgtttatt ttgggtggtt 4560
tttattttat tttagggtta atttttttta gttttagtag aaagtattat tttgagttta 4620
ggatgggtag ttttattggg tagtttgatt gtttttttatg ttaggggttt tagtaatttt 4680
ggttaggttg tttttatatt tttttttttt ttaggttttg ttttttttgg gagttagttt 4740
tataggaagg tttttgtttt ttttttttttg tgtttttttt tgggttgagt tttgagttgg 4800
aaagggatag agttagtttt ttttggggggt tggtatttag gttggggttg ttttaggttt 4860
tgtgtagttt tttagttttg tttgggttgt tttatagtga gatggagttg ttttttttga 4920
ttgtgtggga ggtgaaggta agagtttgat gtgtggaggg gttggtttag ggatgtaggg 4980
attgggtggg tggttagtga ggtagaggaa gtagttggtt tgagtggtgg tgggtgaggg 5040
taatatgttg ttattgggag gggtagtagt ttttgttgga tttgatttta ggttgttgtt 5100
tattttggta gtttgataaa attttaaaag gagaattata gttttggttt gggggtggtt 5160
gtgtgtttgt gttaggattt tatttagagg ttgggattta agattggtgt gtttgtggtt 5220
tgaggatggt atattttggg gttttaaagt tagtttattg gtgtttattt gtttaaaggt 5280
ttttagtttt tgaggtttgt ttttttttgg ttttttttag ttggttttta ttagggtttt 5340
agagtttaag atttagtatt tgtgggtggt tttgggaagt ttggtagttt tgttaatttt 5400
aatatgtttt atttgatagt aaatttggtg ggagattagt tgaaagagta agtgggtgga 5460
tatgttggga gattgggaga aatataaaag tagtagaaag gtaatgtgtg gagggaggaa 5520
gtattttttg tagagatagg ggataggtat ttatggttgt ggtttggtat tattagtttt 5580
ttagagggtg ggtggtatat tgtttttgtt tagaggattg taggtttggt tgttagattt 5640
tttgtttatt tgtgtaagtg ttattttgta gggagggaat ttgaatttag ggttgggatt 5700
atttggagtt taaggttagg gatgttttgg tgatttgaag gaaggaaaag gtttagatta 5760
gagttttgat tttgagtgtt tatttatttt tttagttttg ggaagggaga ttttgtttta 5820
gtttgatttt atttttattg aggaattatg gggttaaaat tgataatttt tagaattttt 5880
gggtttggt ttttattggg gttattttgt ggtttgtgat attagattgt tttttgttta 5940
tagtttatag attgagtgta taagggaatg tttatgaata tttggggttt gatgtggtta 6000
gttttttttga atattgagga aatgaagttg aaaaattttg gaagatatta ggtatgttta 6060
gttagagtat aataaatagg ataggttgtg ttggggttta ggtttttagt tggagggaat 6120
gttaagatta ttttggggag ttgggggtga aggttagatg aatattttgg gtatagatgg 6180
tgatatagtt attatagata aatttagttt tggtgatttt ttttggtttt agtaataagt 6240
taaaatgtag ttttttgtag aaggaaattt tttttttgtt tttttttttt gaagtgttga 6300
ttgtgggttg attgttattg gggtagggga gtttttttatt tgttttgaga ttgttttttt 6360
tttttggttt tgttttatag attatgaagg agaagggtaa gaggttattt gagtatattt 6420
agtgtattga ttgggatgta agtgggatat taaggaagta tatatttttt aggattgat 6480
atggaattaa gtaagtttat gggagttata gggttttagt agagatgggg tgaatgagag 6540
ggatgggggt ttttttggag tagaagttag ggttatttag gagggatgat atagttgtta 6600
agagtttttt tggtttaggg agtagttggt attatgaatt gagtattttt ttggttttaa 6660
gttttgggtt agattggaat atgtggggtt agaatttagg aggattttga ggagatggaa 6720
ggtagtaaat aaaattatgt ataatggtga agggtgtttt ttttgattta tggggattta 6780
tggtaggatt tatgggaggg tggtaggata gagggtttat gagttttttt taggtaatag 6840
tgatagtatt aaatgttggg agaattaggg gttttggaaa tttttattta ggtttgttgg 6900
gaatatgata tggtatagtt atgttggtag tttgttgggt agtggtttat aaagtttgat 6960
ggatttgaat tatatatttt taaagtgtta tagatattga atttattgat ttgtaaattg 7020
atatttatat gaaattagta tgttaggttt attgtttgat tttttgttat ttatatatgg 7080
agtttttggg gatggttttt aatatgggga tggggagagt aaggttggtt ttttttttaa 7140
atggaagatt tagtgagaaa agggaatgag ttggtgatgt ttgtatgaat gtgggtggat 7200
tttagatgta ttttgttgag ggatagaagt tagatttaat aagttattat agtaggattt 7260
ttatttttag gttattttgg aaaaggttaa attataggga ttgagaagta gtttgggtgg 7320
ttaggggttg atggattggg gagaggttgg gtgtataggg gttattttgg agatttggag 7380
gatgaaggag ttgttttagg aggggttgga gtggtggttg ggagattttg tatattggtt 7440
tggaattgtg gaggaattgt atatttatag attgaattgg tgtgtgtgta aattgaaaaa 7500
aaaaaaaaaa aattatttag agtgaaaagg attaggtaag ttattgtata attgggttat 7560
ttgtatgtta tagatgtgga ttttattgaa atattttttt aagagtttta ggttttgaag 7620
agtttattgt ttatttggtg aaatatttga atttgaaatg ggatttgttg ttaggttttg 7680
tagataaagt gaaattaata atatttgtat aaaataaatt aaagtttttt ttttttgttt 7740
tttaggtagt gggaattatt ttatattttt ttggtatatg aggagtataa tttggtgagt 7800

```
attttggta gtgaggtttt tgggttatat ttttatattg ataggagtgg gtgtttggtg      7860
ggggtgttgt tgtttttttt aaagttagta tttgtgattt attaggatat aggaggtagg     7920
atgttagttt attgttggta taaattttta aggaaggggg tggttttaag gggttaagtt     7980
gagatataga ggagttaggg tttggatttt tggtgttatt tgggtttgat tattattttt     8040
tagaataaga aatgatgttt tttttttggg gttgttttaa agtttaggag tttggtagta     8100
ttgtatatag gatggtgtta ttagtagata ttttggataa ggtgttgaag tgtttgatgg     8160
atttggtttt tgttatgaaa tgaatgtgta ttttgaggaa gttttttttt tagaggaagt     8220
ttttttttta gaggaagttt ttttagttat ttttgttttt tttaatgata tgagtttttt     8280
taggtgattt tagtttttttt aggtgatgtt tttttatggt gattttggtt tttgtaggag    8340
gtgggttatt gtagggattt gagttatatt gttgttttgt ttttttttta ttttttttgag    8400
gaggatgtat tttgggtatt ggtgtagttg ttggttagtg agaggtattt tttgtagggt     8460
aagtgaatag ttgttttggg gatttttttgt agttagattt ggggatggtt attttggtta    8520
ggtgattata gtttttagtt aaggtatttt ttttgtgttg ttagtttgtt gggagatttt     8580
aggatgtttt tgttgagggt tttataggag tttatggttg atttttaaag tttaaattag     8640
atgtttttta tttttattag tagagggtat tttattttttt ttgtggttat tttttgtgtt    8700
ttggagttat gttttttggt tttgattttg tgtagttgat tttttttttt ttgagagttt     8760
ttttgttttt tagttgtttg ggtttttgtt gttattggtg tttatgaatg ggttgattaa     8820
gtttaggtgg tagtattttt ttatttttttg ttttttggtt tgattttatt attaggagat    8880
gattgggaag tttagtgttt atttagtttt ggttattttg ttgtggtttg aaagttaggt     8940
ttgttttttt tgtattttgg tttaggaggt ttttaggggga atttttagtt aggttttagg    9000
gaatgttttt gttttatttt tttagggtaa aggttgtatg ttggggttat tagatgggag     9060
ggtgggaggt tttggggttt gggggttttt ttagttgttt agtttttgta gttgatggtt     9120
ttatattttg ggggaaggtt ttgattttat gatgggttgg gggttttttta ggattttata    9180
gtttaaatgg tgggattgtt taggggtttt aagattaata ggagtatgtg gtagttatgt     9240
tataatttaa gattatgggg tattaggtga gtttatggtt tttttagttt tttttagagg     9300
ttttgttttt tgtggggttg taggagtagg ggggttggag tttttttgtgg ggttggtgat    9360
tggttgagtt ttagttaggg tttgatttgg gatgttgggt ttttttatggg ttgggagttg    9420
gttttttttt ttgttttgga ggagatagag gtatagggat ggggggtttag tttttgtaga    9480
gtagggtaaa gggtagtgtg tttattggga gtgtgggaag gtgatagtgt tgtggggagt     9540
tttggatatt gtttagtgtt ttgtattagg ggaaggggttt ttagaggttt tggaagaggg    9600
aggtttttag ggtagtttag tggtttgagt atttttgttg tttttattag gataagaaag     9660
atttatgtgg gtagtgtttt ttgttaggtt gttttatttg gatattgatt gatggggtaa     9720
ggaggtatag ggagattttg gtttagggat ttttttgtt ttgtagtgtt ttgttttttt      9780
agtttggggg tttggtttat ttttagttta taggaggttt aggtgggttt ttaaaggata     9840
tataagtaaa atttttttgtt taaggggggt tattttaggg ttatggttgg ggtttaggtt    9900
tagttttatg ggtagattgg gttaggattt gatttgagag ggtttaggga agttttaagt     9960
tttgggtaag tttttttttt taggagttat atttttattt aaatgagtgt tttttatgag    10020
gagttttaag attttgtttg atttagtgtt ttggagggtt taggtgattt ttatggggaa    10080
ggttattgat tttggagatt gaagttttag tgtgtgtagt ttgagttatt agttttagtt    10140
tggaaggatt aggtttttttt atatttgttg tttttataga ttttttttgg gtttattttg   10200
tgtttgtggg atgtgtattt ggtagaaggt gaataggtgt tgatgttgat aataagaatt    10260
gtttttaagg tttagtagag taagtttatg tgtgtttagt ggggtttggg gagttttggg    10320
gttagatttt gattggtttg agggtagttt ttttatattg tttttatgat tttttgtttt    10380
ggtttagagg gaggtttggt taggtgggtt gggtaggata ttgtgatatt gagtttattt    10440
tttatatgat ttagatgaaa gttgagagtg tggtgagtat ttttttgttt               10490
```

<210> 101

<211> 10490

<212> DNA

<213> Artificial Sequence

<220>

<223> chemically treated genomic DNA (Homo sapiens)

<400> 101

```
ggatagggaa gtgtttatta tatttttgat ttttatttgg gttatgtggg ggatgggttt      60
ggtgttatag tgttttgttt agtttatttg gttagatttt tttttgggtt agaatagagg     120
attatgagga tagtgtgagg aagttgtttt tgggttagtt ggggtttgat tttagggttt     180
tttaggtttt gttgggtata tgtagattta ttttgttgaa ttttaaaggt gatttttgtt     240
attggtatta atgtttgttt gtttttttatt agatatatgt tttataggtg tagggtgagt     300
ttgagagaga tttgtgggga tagtaggtgt gaaagaattt ggttttttta ggttggggtt     360
ggtggtttga gttgtgtata ttggggtttt agtttttaga gttagtgatt tttttatga      420
```

```
gggttgtttg agttttttag gatgttgggt tagataaggt tttgaagttt tttatggggg    480
gtatttattt gagtggggat gtggttttg gagagagggg tttgtttagg gtttgaggtt    540
tttttgagtt tttttaagtt gggttttggt ttagtttgtt tatgaggttg ggtttgagtt    600
ttagttatgg ttttgggatg atttttttg ggtagagggt tttgtttgtg tgttttttgg    660
ggatttgttt gagtttttg tgggttggga gtgagttaga tttttgggtt ggggaagtag    720
ggtattgtag ggtaaggaag gtttttgagt tagggttttt ttatgttttt ttattttgtt    780
aattaatatt tggatgaggt agtttaatgg ggaatattgt ttatatagat tttttttgtt    840
ttgatggaag taatagaggt gtttaggtta ttgggttgtt ttaaaaattt ttttttttta    900
gggttttga agattttttt tttagtgtag aatattgggt ggtgtttaga gtttttata    960
atattgttat tttttatat ttttggtgga tatattgttt tttgttttgt tttgtgggag   1020
ttgggtttt atttttgtgt ttttgttttt tttagggtag gaaaggaaat taattttag   1080
tttatggaga atttgatgtt ttaggttagg ttttggttgg gatttagtta gttattagtt   1140
ttatgagggg ttttagtttt tttgttttta tagttttatg ggaggtaggg tttttgggaa   1200
gagttgaggg gattataaat ttatttgatg ttttatggtt ttgggttgtg atgtggttat   1260
tatatgtttt tgttggtttt ggagtttttg gatggtttg ttatttgggt tgtgaaattt   1320
tgagaagttt ttagtttatt atgaaattag agttttttttt taagatgtgg agttattagt   1380
tgtaagagtt gggtagttgg agaggttttt aaattttaag gtttttttatt tttttatttg   1440
gtgattttaa tatgtggttt ttatttttggg gaggtggggt gggaatattt tttggagttt   1500
ggttggaggt ttttttggag gtttttttggg ttagggtgta aaaagggtaa gtttgatttt   1560
taggttatga tagggtggtt ggaattgggt gggtgttggg ttttttggtt attttttggt   1620
agtggggttg ggttagggaa taggggatgg ggagatgttg ttatttgggt ttggttggtt   1680
tatttgtggg tattgatggt agtaggagtt tgggtagttg gagggtagga ggattttttag   1740
ggaggggaga gttagttgta tagaattaga gttggagggt gtggttttag gatatagagg   1800
gtggttatgg ggaggatgag atgttttttg ttgatgggga tgagaggtgt ttgatttggg   1860
ttttgggggt tagttgtgga tttttgtggg atttttagta gagatatttt aaagtttttt   1920
aataagttgg tgatataagg agggtgtttt ggttgaaagt tgtgattatt tggttagggt   1980
ggttattttt aggtttggtt gtaggaggtt tttggggtag ttgtttattt attttgtagg   2040
gagtgttttt tattggttag tagttgtatt agtgtttaga atgtattttt tttaggaaga   2100
tagaggagga ataaggtggt gatgtggttt aggtttttgt agtagtttat tttttgtaag   2160
agttagagtt attatggaag gatattattt gggagggttg aggttatttg ggaggattta   2220
tgttattgga gagggtagag gtgattggag aggtttttttt tgaaggagag gtttttttttg   2280
aaaaagaggt tttttttagga tgtatattta ttttatgata agagttaagt ttattaggta   2340
ttttagtatt ttgtttaaaa tgtttgttga tagtattatt ttgtgtgtga tgttgttaag   2400
tttttgggtt ttggggtagt tttaggagga gggtgttatt ttttgttttg agaagtggtg   2460
gttaggttta ggtgatatta ggagtttagg ttttgatttt tttgtgtttt agtttgattt   2520
tttgagatta ttttttttttt tggaggttta tgttagtggt gagttgatat tttattttttt   2580
atattttggt gggttataaa tattaatttt aaaagaagta atgatatttt tattagatat   2640
ttattttttgt taatatggaa atatggtttg ggaattttat tgttgggaat atttattggg   2700
ttgtattttt tatatgttag gaggatgtgg agtagttttt gttgtttagg aaatagagaa   2760
agggggtttt ggtttgtttt gtgtagatgt tgttaatttt attttgttta taaagtttaa   2820
tagtaaattt tattttaggt ttagatgttt tattagataa gtagtgagtt ttttagggtt   2880
tgagattttt gaagaaatgt tttagtaaaa tttatatttg tgatatgtaa atagtttagt   2940
tgtatagtga tttgtttgat ttttttttatt ttgaatgatt ttttttttttt tttttttagtt   3000
tgtatatatg ttagtttagt ttgtgggtgt atagtttttt tatggtttta aattaatgtg   3060
tagagttttt tggttattgt tttagttttt tttggggtga ttttttttatt ttttaagttt   3120
ttagggtggt ttttatgtat ttagtttttt tttgatttgt tagtttttgg ttatttagat   3180
tgtttttttag tttttgtggt ttggtttttt ttagaatggt ttaggaatgg gaattttatt   3240
gtggtagttt attgggtttg gtttttgttt tttagtaaaa tgtatttagg atttatttat   3300
gtttgtgtgg gtattattgg tttgtttttt tttttttattg ggttttttgt ttgaagggag   3360
gattagtttt gttttttttta tttttgtgtt gaaggttgtt tttgaaggtt ttgtgtgtga   3420
gtgatgagga gttaagtagt gaatttggta tgttggtttt atgtggatgt tagtttgtaa   3480
attagtgggt ttaatatttg tgatattttg gggatgtgtg gtttaagttt attgagtttt   3540
gtgagttatt gtttaatggg ttgttaatgt ggttgtgtta tgttatgttt ttagtggatt   3600
tggatgagag tttttaggat ttttaatttt tttagtattt ggtgttgtta ttgttgtttg   3660
gggggggttt atgggttttt tattttgtta tttttttgtg ggttttatta tgggttttta   3720
tgggttaggg agagtatttt ttattattgt gtatgatttt gtttgttgtt ttttattttt   3780
ttaggatttt tttgggtttt ggttttatat gttttagttt ggtttagggt ttggaattag   3840
ggaggtgttt ggtttatggt gttggttgtt ttttgggttg ggagagtttt tggtagttgt   3900
gttatttttt ttgggtgatt ttggtttttg ttttggggaa gtttttattt tttttattta   3960
ttttattttt gttgggattt tgtggttttt gtaggtttat ttggttttgt attgattttt   4020
gaagaatata tgtttttttta atgttttgtt tatgttttgg ttgatgtgtt ggatgtgttt   4080
agatgatttt ttgttttttt tttttatgat ttgtagggta gggttaagag gaggaagtag   4140
ttttagaata gatggaagat tttttgtttt tagtggtagt tagtttatag ttagtatttt   4200
```

gggaaggaag gatagaagga aggttttttt ttgtagaaag ttgtattttg gtttgttatt 4260
gaagttaggg agggttatta gagttgagtt tgtttgtggt gattgtgtta ttatttgtgt 4320
ttagggtgtt tatttgattt ttatttttag tttttaggg tggttttgat gtttttttta 4380
gttggagatt tgggttttga tatggtttgt tttgtttgtt gtgttttggt tgagtgtatt 4440
tggtattttt tggggttttt taattttatt tttttaatgt ttaggaggat tgattatatt 4500
gggttttgga tgtttatggg tattttttttg tatgtttgat ttatgagttg tgggtagaaa 4560
atgatttggt gttataggtt atggggtgat tttagtgagg attagagttt ggggattttg 4620
gaaattgttg gttttggttt tatgattttt tagtagaggt gagattaagt tgggataggg 4680
tttttttttt taggattgaa agagtggatg gatatttaga gttgaaattt tgatttgaat 4740
tttttttttt ttttaggtta ttagggtatt tttagttttg agttttgggt agttttagtt 4800
ttagatttag attttttttt tgtaaggtga tgtttgtatg aataggtagg aaatttggtg 4860
attaggtttg tagtttttttg ggtgaggata gtgtgttgtt tattttttga gaggttgatg 4920
gtgttaggtt atagttatgg gtgtttgttt tttgttttttg tagagagtgt tttttttttt 4980
tatatgttat ttttttgttg tttttgtatt tttttttagtt ttttagtata tttatttatt 5040
tgtttttttg gttgattttt tgttgaattt gttgttaaat gaggtatgtt ggagttagtg 5100
gagttgttag gtttttttaga gttgtttgtg gatgttgggt tttgggtttt ggagttttgg 5160
tgggagttag ttggaaggag ttagggaagg gtagatttta agggttgaga gtttttgagt 5220
aaatgagtat tagtgggttg gttttgggat tttgggatgt attattttta ggttatagat 5280
atattagttt taggttttag tttttaggtg gggttttgat ataagtgtgt agttattttt 5340
aagttaggat tgtggttttt tttttggaat tttattaaat tgttaaagtg aatagtaatt 5400
tggggttagg tttagtaggg attgttgttt tttttagtga tagtgtgttg tttttatttg 5460
ttattgttta ggttagttgt ttttttttgtt ttattgatta tttgtttagt ttttatgttt 5520
ttggattagt tttttttatgt attaggtttt tatttttgtt ttttgtgtag ttagaggagg 5580
tagttttgtt ttattgtaag gtaatttagg tagagttgag gaattgtatg gggtttggag 5640
tggttttagt ttgggtgttg atttttagaa aggattggtt ttgttttttt ttagtttagg 5700
gtttagttta ggagaaggta tagggaaggg aggataaggg tttttttgtg gggttgattt 5760
ttaggagggg taggatttgg gagaagaagg agtgtaggga tagtttggtt ggggttattg 5820
gggtttttgg tgtgggggt ggttaggttg tttagtgggg ttgtttgttt tggatttgag 5880
gtggtgtttt ttgttggagt tgagaaaggt tagttttgag atgggatggg ggttgtttag 5940
ggtgggtgat tgggttttga taggagtttt ttagggagtg attatatttt tttgttaggg 6000
ttaagggagt ttgttttgag atttgtttgg tgtattttgg ttgtattagg ggtttatttt 6060
atttgatagt tttaaggttt ttgtaggttt tgatttttta gtatttattt gttttttttt 6120
gtatttgagt tatatatttt gtgttttaga agtggtatgg tttgttagtt ttttttttgtt 6180
ttattttttt agggattttt tgtttttttta ttttgtgatt tttgagggat gggtttttgg 6240
ttgggttttt tttatttggt tttagatttt tttttagtat tagatttagg tttttagttg 6300
tgagttttgt tgtttttttg gttttattgt gagatgttta gaatggggtt ttgtttattt 6360
ttttttttgt tttttttaggg ttatagtttt tattgttatt atgttttttt tttttatggg 6420
atagtgaggg ttgtagtttt aggggaatgg gggagaatag gggtaggtgg gtttttagag 6480
atttgttgga taatagtttt gaggttgggt taggtgtttt tttattttgt ggttataatt 6540
tttgtatttt attggggttg tttttaagta gataggggtta gattttagg ttgttttgtt 6600
tttttttgtgt ttatttgttg atagaattgt tgagtgttta ggggtttgat ttagtttagt 6660
ttttattttt attggttttt tagatgggtt ttatattttt ggtttaataa ttttgggttg 6720
gatttgtagg ggagttaggg aggagttttg tttttggaaa ggaggttgat ttgatttggt 6780
gagatatgtt ttgtgttaga aaggtttttt taaaagtaaa tttattttttg agttgagata 6840
ggtgttttag gggtgagggg agtgtagagg atttattgta taatttttaa atgattgatg 6900
ttgttgttgt agttttgaaa aggtttaggt tttttgtttt ttggtagttt agtttggtgt 6960
ttttgtagtt tagagggagt tttggtgagg ggtttaaggt aaagggtgta agggtttggg 7020
ggtattggtt atttgttttt gttttgtgtt tttagggagt ttaggatttt ttgattaggg 7080
tatattggaa gaggtttttt tttaagaagt agattgattt gtattttttt gtattttata 7140
atgatgtttt tttgtttttg tgtttattaa ttgtttgtga tttttattat gtttattttg 7200
tgagatagaa ttgtttaaag gttatattgt atgtggtggt tttggagaat atttgaattg 7260
tttttgttgg gtttttagat gttggttggt tgtgtaattt ttattttatt gttgttttta 7320
gggaaaaagg ggttagattt agtggtttat agttgtttta gtttttggag ttttaagttt 7380
taagtagggg tgggtatttt tttaaggatt tgagtatagt gggatttaga tagagaattt 7440
tgttgttttt taatgttatg aaatggggat atattggttt tagtaggttg aatggttttt 7500
atttgttaag ggtgaagggt ttatgatggg ttattttagg gatgtggagg tagattgggg 7560
ttagtgatta gaggttttttg tgtaattggt tttttgggat gtttagggtt ttagtgatgt 7620
ttagtttttt atagggaata agattttttt tgattgtttg gttttatttt gtttattatt 7680
ttggttattg tggtttttta gtttgaatag tgaagttatt ttaggaataa tgtttgttga 7740
gtaggagggt gttgggtttg ggggatgaga aagatttatt gtatgtatgg aaattatgtt 7800
ttttgtggag ggattgtgga gtttattatt ttgagttgtt ttaataggag gaggttttat 7860
ttttttgggt tattgaggaa gaatagtggg tttttggttt tggagaatag ttggatggat 7920
tgtttttttt gggaatattt gaggtaaaag gagggtgagg ttttaagagg attatgtaga 7980

```
gtaagaaata tttggggaga attttagtgt ttggattttt ttgaatataa gggaagatag     8040
ttttttttta gttagttttt tagggttttt ttatttttta tagttgttta agggtagtag     8100
gtttttttgg ataagggatt atgtgtgttt agtggggttt atagtgatta ttaggattta     8160
gtttagggta tagaggtgtt ttgaggattg ttagtggatt tgtattagtg gttttatatt     8220
agtggttttg ttaggattag gttttgtttt attgggatgg gaaatttggg tagatttggg     8280
atttagggta gggaggttat agggtttagg tttgaatttt agtatagtat atggtagggt     8340
tgagagtaaa atttagggtt atgtttggat ttttaggttg gttattgttt ttttgatttt     8400
agatgtttta tttgttgaat ggggatattt gggaatagta tttattttat gaagttatta     8460
tggagatgaa agagttaatt gtttatatgg gtagtgtaga atgggtgttt ggtgagtgtt     8520
tagggatgat tttttttggt agttgtttta tagaggttaa tattgtttgt attgtagtta     8580
ttgtttttaa gtttgtttgg agggaagaga gtaggttatg tttatttgat tttgatgaat     8640
tagttggttt gggttatgtt ttttagggag aaaatttttg agtttataga gttgtttata     8700
gatattattg tttgtgtgta attgttgtag attattgagg taggttagag agtagatagg     8760
tgttaagtat tagtgatatt ttgaggttat ggtatgaatt atagtggggt tttgtttggg     8820
ttagtagtgt ttagagttag ggtttttttgt tgtttgaggt gttaatatgt ttgtttgtaa     8880
tgtgtttgtg tatgtgtgtg tatatgtgta tgtgggtaaa tatgtttgtg tatgtgtgtg     8940
ttgttttttt ggttaggttt ggttgtttta tttatgtgtg tatttagttt tttattattg     9000
ttatttttga ggtttagggt tagtattaga gtatttatgg ttgtttttta attgtagttt     9060
tttttgttta gggtggtttt gggatatata tagtggtgga gggaagtgat tgttgttatt     9120
ggattttaga atataaaagt taatattatt atttaatggt ttttttagtg tttttaatgg     9180
tattattttt ttttttttga tattttaatt gggtattttt ttttatgatt tttggatatt     9240
ttagttagag gattttgtgg ggaaagtgtt gggtatatag taggggttta ttttttttaga     9300
tatgttattt aaaatttggt ttatttgttt ttttatgtag ggtttagggg atgttgaatt     9360
ttagggttta gaaagagttt gggataaaaa gaaattttaa ggggatggtt ttgatttggg     9420
ttgagtttat ttgtgttatt taattggagt tttaagtttt gaggtaggat gtttagatgt     9480
tttagtgtag ggttttttttg attaatattt gtttttttgt atttattagt aattttattt     9540
attttatttt taaagtatat ttggtttttg tatttaggag ttttgtattt gtagatttag     9600
taatagtaga tggaaaatat ttagaaaata aattggatgg ttatgttttt attgaatatg     9660
tgtagatttt gtttttgtta ttattttttta aagaatatag tattatgatt atttatgtag     9720
tatttgtatt gtattatata ttataaataa tttagtaatg gtttaatgta tatgggagga     9780
tgtgtatagt ttatatgtaa atattaggtt atgttatatt agagatttga gtatttatgg     9840
atttggtatt ttttggggat tttagaatta atttttttatg gatattaagg gatgattgta     9900
tatatttatt taggaaggtt ttttattgga ggaaaggttt ggtttaggat agatagggat     9960
attatttttg gattattgtt tatttattta tttatttatt ggtattattt tttaggattg     10020
ttttaatgat agttttagta agtggagata agaaaaaaga ttggtttaaa tggtatagtt     10080
ttgaggtttt ggaagatgtt gtattagtat gagaataggg ggttagtttt ttttaggatt     10140
tagaaagtat attaggtagg tttggggaga ggaaaggaat atggtttttt tagtagttat     10200
ataggtttgt agtaggatgg ggttggggtt ggggttggga ttggggttgg ttttgtttat     10260
tatttgggtt ttatgagggg aaaagaaaga ataggggggta gaggaggagt atgggggtag     10320
tgggttgttt aaggagaagg tgttttaggg aaggggtatt agtttgtttt tatattgtta     10380
taaagaaata tttgagttta ggtaatttat aaaggaaaga ggtttaattg atttttagtt     10440
tagggaattt atagttatgg tagaaggtga aggggaagta ggtatttttt                10490
```

<210> 102
<211> 7001
<212> DNA
<213> Artificial Sequence

<220>
<223> chemically treated genomic DNA (Homo sapiens)

<400> 102

```
atgatattag taattgttta gtgaggtgga tataaaattt ttaggatatg agagggagat      60
gtggttttta tattttgatg tgtaaatatt atgtttaggg aaaatgtaag gtgttttagg     120
tttgtggatt ttgtattttt ttaggtaatt tatttattta ttttttaatt ttaataaatg     180
attattaaat tttatttaat atataaatat ttattgagta ttatttgtgt gtatgagaag     240
tgggagttag tatggtaaaa gttaggtatt gtgttaggtg agagagattt agaaattaaa     300
attagagaag ttattaataa gagtttaaat atttgggttt aggtttatgt ttgtaatttt     360
agtattttgg gaggttgaag gaggtgaatt atttgaggtt aggagtttaa gattagtttg     420
attaaaatgg tgaagtttta tttttattaa aaatataaaa aattaggtgg gtattgtggt     480
atatgtttgt aattttagtt atttgggagg ttgaggtagg agaattattt gaatttagga     540
ggtagaggtt gtagtgagtt aagattgtat tattgtattt tagtttgagt gatagagtaa     600
```

gattttattt taaaaaaaaa aaaaaaagag tttaaggatt tgatggagga gaaaggtaag 660
aatatgtgtg agataatgta aggttattgt tttagggtgt ttagggtaat tatgggggta 720
gggtattttt tggagaggtt aatgataagt aggttgaata aagtaggggg gtttttgta 780
ggaggaggtt tattaggtga agatggagtt gtatgggtaa aggttatttt agagatttag 840
gtgtgtttag gaggtggaaa tttattgtag gtaaggtgag aggattgggt ggggtggttt 900
aggaggagtt gatagagggt ataagttgtg aaatagtttg aagtagggta gtgaggaaag 960
ggatttagag gaggaagata tgtggataga tggggttggt tgggggttgt tgtaggattt 1020
tatgtaagag gttttaatat tagagtttta ggttttgagt tttgtggaat taaaggtttt 1080
agaaagtaat ttattaggat ttggtggttg atagtttgta gtagggggtg aaagaggagt 1140
ttagagtatt ttttggtttt tgtttttgtt ttggggtata ggagggggagg aatttagttt 1200
ggttatattg gtttaggtga gggtgtttta ggggaggttg agaggggttg ttttttttgtt 1260
tgttttgtta ttagggattg ttttgagatg tttttggaga aagtgttttt ggtttgttgg 1320
gaaggatttg tgtttagttt ttgttgttta gtagttttta tgggaatttt gttttttttgg 1380
ggtttggatt tattgtgatg tgaaggtgat tattgtttat ttttgggata ttgtgggggt 1440
taagagaggt tttgtggtga gggaggatta ttattttggg ggttgggggg gtttttttttt 1500
tagggaggaa gatttttagt tttggtgttt tgtttttggt agattttgtt ttgtttattt 1560
gttttgtttt tgttgtatga tggaaataaa tggaaatggt ttttatatat gaatgtatta 1620
aattgtaatt ataatttttt agattttagt tgtaatagga ttatggattt gagtgatttg 1680
taaagatgtt ttgagttttg agttagaggg tgtgtggggt ggggaggggga gtttgttatg 1740
gtttttgtga ttttatagta taggggggtag agttgggggt tggggtgggg gtaagggtgt 1800
aggtagatgg gtttgggggt ggttagtatg gggatttatt tagtttgttt tgtatattga 1860
ggttattttt tttttgggt tttaaagttt ttttgttttt tgttatgggt ttgggggttt 1920
ttttattgta gtttaatgtt ggttgttttt ttatgttgtt taagtttatt ttttaggttt 1980
ggtattttat agtagagatt aagaggtggt tggagggtag tgggggtatg gatagtatta 2040
ttgggtgttt ttttttttagg tttttttgga aatttttgtt ttggaaatgt agaaagtttt 2100
ttttttttgtt tttattttga aattgtattt aataatggta aataagttat ttagtttttt 2160
atagttggta aagtgtaagt tgtagatgtt gttatatagt atttttttgat agttttaggg 2220
tagattgttg atttattttg taggtaaagg agttgagatg taaatatttt ttaaggaagt 2280
aagaatggtt tttttttttta ttttttagaa ggatttaggg taattgattt atttgataat 2340
attagggaat atgggttgtt agttatgtat ttatgagaga ggtggttttg atttttagag 2400
tatggatttt aggggagttt aggaatttgt aggagagggt tttttagttg gttattattg 2460
ggatgggtat ttgggtttgt gggagagggt tttttaggtg gttattattg ggattggtat 2520
ttgggtttat ttggatgggt tttgggaggg tttgtttttg ggggagatgt tggtatgaat 2580
agttaaaagt attattttga gattttatgt taatattttt tttttgaaat atgatatttg 2640
ggaggattgt atagtttttt taatatagga aaatagtttt gattgaaggt agtttttttt 2700
ttgttgtatt ttttgaaggt ttttgggggt tttgttattt tgagttattt tttatttgtt 2760
tttgttgatt ttgtaaattg gatatatttt taagggttta gaatagtatt tggtataaaa 2820
taggtgttta aaaatatgta tgtaaaatag tggtttttgg tggaggttgt tagagttggt 2880
tgtggtttta tagagtagga agaagtatgt tttatttgtt tgggttttgg ttagggtgtt 2940
gagggttagt atggatatta ggattagggt gtagattatt ttgtttttta tggtggttat 3000
tgtttttttt ttgttttaaa ggtgattttg agttagggat gagaggttgt ttgagttttg 3060
gattttatag ggtaggtttt gtttgtttaa agagtgttag ataatatttg tttaaggagg 3120
tttggggatt ttttgagata ataattttta ttgattttta ttaaaggtgt tttttagata 3180
tggttaagtt atatggaagg atttgttgat agatagagat gatatgtggt gaggttattt 3240
tggttgaggg atttgagatt tagaaagttt ttttttttta tgggagtttt ttttaatttg 3300
attttaattt aggttttatt tatatttgag aggttttttt gttagggtaa atattgtatt 3360
tattgtagaa gtgatttata gtgagagatg gttggaaaaa ggtttggttg tgataatagt 3420
ggtttatggg gtggttattg tgattttgta gggggaaggg aaggagttta tgaagtttat 3480
tttgtttagg tttaagttaa ttttttttata gtggaattgt tttaataatg aaattgtagg 3540
tttggtgtag tggtttatgt ttgtaatttt aatattttgg gaggttaaag taggtggatt 3600
atttaaagtt aggagtttga gattagtttg gttaatatgg tgaaattttg tttttataaa 3660
aaaaaaaaat ataaaaatta gttaggtatg gtggtgggtg tttgtaattt tagttatttt 3720
ggaggttaag ttaggagaat tatttgaatt tgggaggtgg aggttgtagt gagttgagat 3780
tattttattg tattttagtt tgagtgatag attaagtttt tttttgggag gggttggggg 3840
taggagggag aaaaaaatag tatatatgag ttgttattaa aaattgataa agtgaatgtg 3900
gtttattttt ttgtgtttat ggggtttttt tttttttttt tttatatttt tttagttggt 3960
tttttggatt tttgtatttt agaggatagt tttttgtttt tttgtattat gttttatttt 4020
tttttttttgt ttggagtttt ttttttttata gtttttatta ttttttggtg gataaaataa 4080
gtgattgata tattatgttg gttatttatt tattgtattt ttttttgtag tagatgggag 4140
ggtgtatggg agtagaagtt tttatttttt agtgttttga ggtggtttta gattttagaa 4200
tagtggtttt gattggtatt tgagaaatag ttgttgaaga gttgtatgaa gaaatggata 4260
ataggagaaa tttttttttt tttagtgaga aattaggttt tgaggaaaat gtatgttagt 4320
ttaataaagg tattattttt ttttgggtga tttagttagt atatttttgt agtttttttg 4380

ggaggggagt ttgggttaga atgggggtgg ttatgttttt ttttgtttta ttttaggatt 4440
tagttttttt tttagggttg ttatttttgt attggggttt ttatttttgg gttagggttt 4500
ttaggagggg tttgattgta ggatttaggg aggggttgag gtttggtgtt ttttgtggtt 4560
agtttaagtt tattgaaaat aggatggggg tattaggttt agggtggtgt ttatagtgat 4620
ggtggttttt gttgttttaa ttttttggat taaaatgggg gtttttggaa atgggatgta 4680
aattgtataa ttatttggag aaaaggtggg tagtgtttgg tgaggtttaa gttatataga 4740
ttttatagtt tagtaaattt ttttgttttt aggtttggtt ttttaatttt agaaatatgt 4800
ttatatttgg gtagaagaaa atataggtaa agatattttt tttagtattg tttgtaatag 4860
ttggaaatag ggaaagaagg aaggaaggaa aggagggaaa gaaaagtaaa ataaatattt 4920
attaattagg gaaagggaaa atgaatttaa aatataatgt agttgttttt tagtattttt 4980
gggggattgg ttttaggatt ttttatttag ggatgttaaa atttataaat gtttaagttt 5040
ttggtataaa atgatatagt gtgtagaaat tatgtgtatt tttttttata ttttaattat 5100
ttttaggtta gttataatat ttgatgtaat atttatattt tattttattt tgtagatttt 5160
atatagtatt tgatttgtgg tatatttaag ttttgatttt taagatgttg taaataagat 5220
tttaaaatat tttttttttt tgagatagag ttttgttttg ttgtttaggt tggagtgtgg 5280
tggtatgatt ttagtttatt gtaattttta ttttttaggt ttaagtgatt tttttgtttt 5340
agtttttttga gtagttggga ttataggtat gtgttattat gtttagttaa tttttgtgtt 5400
tttagtagag atagggtttt attatgttgg ttaggttggt tttgaatttt tgattttagg 5460
tgatgtattt gtttttagttt tttaaagtgt tgggattata agtgtgagtt attgtgttta 5520
gttggatttt aaaatagttt tgatttgata ttattggaat ttatggatgt agaatttatg 5580
aatatagaga attaattgta tatatgtatt atattttata tatattatat atgtatgtat 5640
gtgagtattt tttagaagat gaaaatgaat aaataatgag aaatattgaa aataaagtga 5700
agtgaaaaaa tttgttaaaa tggtttgtaa agtaggatat tatttataat gtagattttt 5760
aaattatgtg aaataatgtg atatattgtt ggaattgtat ttagatgaat aaaatgatga 5820
tagtgtagat aggaatgaga tattgatttt agtatgatgg gtttgtttag gaaggaggaa 5880
gtaggaaaag gtggtggggt agggtgtaaa ggggttttg atgtttttgt aatgtttttt 5940
tttgttgaag aaaatttgag ggaaataagg taaaaaggtt agtatttatt aatgaggggt 6000
agtgtagatt tatggatgtt gttttagtat tttgtatatg tttgtaattg tttttttgga 6060
aagtttagtt tttatttttt tgggagagtt gtgagataat tattaagtgt tggagtttgg 6120
tttttttatat tagggttgga gtggttggag ttgtttttgg gatttttttgt tttttgggat 6180
aagagtttta tttttgtgag agtgggtgga atttaagatt atattatata taattttatt 6240
tataaaaatt tgttgaagat aattttagtg tggtgaggga gagaatgtag aggtaagtgt 6300
tagtttatta ttagtggggg attttgtgtt agtatttttta tagttttggg ttttagtttt 6360
tttatttgaa agatgtagtg gttgggttat aggatatttg aagatttttgt attttagatt 6420
ttttttggta ggtatttatg gttgtggggg tgtttgtggt tgtgtaggta ttgtttgttt 6480
tttgtgttta gttgattttt ttgtggtttg gtgagagttt tttattttta gtgggggggtt 6540
ttgtagttgt ttagattttt tataaggggg atgtgtagtt ggggagttag tttttttttg 6600
atgtttttta tttgtttgtt gtttttgatt tgtgttttgg taggaggggg ttttgtaggt 6660
ggttttggag ggagggagga gtttgggagg aagtggattt gggttttttt ttaggatttt 6720
ttttgggatt atttgggata ggttatgttt tttttttagt tatagtttag gtgggtgttt 6780
tttttatata ttattttttt taggttttag agtagatttt ttattttttt gttttagttt 6840
ggggggttta ttagttgtaa ggagattttt atagtttttta tttttttttgt attgtttaga 6900
tttgttgtgg tgaagtttgt tttttgttag gtttttggtt aaaaaagtaa ttgttatttt 6960
agggattttt tataaggata tttagttgag gttttggggt t 7001

<210> 103
<211> 7001
<212> DNA
<213> Artificial Sequence

<220>
<223> chemically treated genomic DNA (Homo sapiens)

<400> 103

```
aattttaggg ttttagttgg gtgtttttgt aaagggtttt tggggtggta gttgtttttt      60
tgattagagg tttggtagga aatgggtttt attatagtgg atttgggtaa tgtgaggaga     120
gtgaagattg taaaagtttt tttgtggttg gtaaattttt taggttgggg taggggggtg     180
aggggtttgt tttggggttt ggggaagatg gtgtgtggag aggatgttta tttgggttgt     240
ggttgagagg gggatgtggt ttgttttagg tgattttagg agggattttg gaggagagtt     300
tggatttatt tttttttaaa tttttttttt tttttagggt tatttataga attttttttt     360
gttaaggtat aggttagggg tagtgggtag gtggggagtg ttaggaggag attgattttt     420
taattgtgtg tttttttgt ggggaatttg ggtggttgtg gggtttttta ttgaaaatga     480
```

```
ggggtttttg ttagattgta gggaggttgg ttgggtgtag gaaataagta atgtttgtat     540
agttatagat gtttttatgg ttatagatgt ttgttggaga gggtttgagg tgtggagttt     600
ttagatgttt tgtggtttag ttattgtgtt ttttagataa gaaaattaag atttagagtt     660
gtgaaaatgt tggtataggg ttttttattg atgatggatt ggtgtttgtt tttgtatttt     720
ttttttgtt atattggaat tgtttttaat aagtttttat ggataaggtt atgtatggtg     780
tggttttggg ttttgtttat ttttataggg ataaagtttt tgttttgaga aatggagagt     840
tttgggggta gttttagttg ttttaatttt aatgtgggag gttaaatttt ggtatttggt     900
agttatttta tagttttttt aaggagatag gagttgggtt ttttaagaaa gtaattgtaa     960
atatgtatag aatgttagaa taatatttgt gaatttatgt tgtttttat taatggatgt    1020
taattttttt atttatttt ttttagattt tttttaataa aagaaaatat tatggagata    1080
ttgaaggttt ttttgtgttt tatttgtta tttttttttg ttttttttt tttagataga     1140
tttattatgt tgaagttagt gttttgtttt tgtttatatt gttattattt tgtttatttg    1200
gatgtagttt taatagtata ttatattgtt ttatatagtt tggaaattta tattataaat   1260
gatattttat tttataaatt attttaatgg atttttttat tttgttttgt ttttaatatt   1320
ttttattatt tatttatttt tattttttga aagatattta tatatatata tatataatat   1380
atgtaaagta taatatatgt atgtaattag tttttttatat ttatgggttt tatatttatg  1440
gattttagta atgttagatt aaaattgttt taaaatttag ttgggtatag tggtttatat   1500
ttgtaatttt agtattttgg gaggttgagg tgggtgtatt atttgaggtt aggagtttga   1560
gattagtttg gttaatatgg tgaagttttg tttttattaa aaatataaaa attagttggg   1620
tgtggtggta tatgtttgta attttagtta tttaggaggt tgaggtagga gaattattta   1680
aatttgggag gtggaggttg tagtaagttg agattatgtt attgtatttt agtttgggtg   1740
atagagtaag attttgtttt aaaaaaaaaa agtattttaa aattttattt ataatatttt   1800
aaagattaaa atttgggtgt gttgtaagtt aagtattatg tagaatttgt agaatgaaat   1860
gaggtgtagg tattgtatta ggtattataa ttaatttaga gatggttaaa gtgtaggaga   1920
ggatgtatgt ggtttttgtg tattatgtta ttttatatta gggatttgag tatttgtgga   1980
ttttggtatt tttgggtggg gggttttgaa attagttttt taggggtatt aagggatgat   2040
tgtattgtat tttaagttta tttttttttt ttttggttaa tgaatattta ttttgttttt   2100
ttttttttt ttttttttt ttttttttt tttgttttta gttgttataa ataatgttaa      2160
aaagaatatt tttgtttgtg tttttttttg tttaagtatg aatatgtttt tgggattaag   2220
agattaggtt tggaaataga agaatttgtt gagttgtaaa atttgtatgg tttaaatttt   2280
attgaatatt gtttattttt tttttaaatg attgtgtaat ttatatttta tttttaagag   2340
tttttatttt gatttgagaa attagagtag tgaaaattat tgttattatg agtattattt   2400
tgggtttggt gtttttgttt tgtttttaat aaatttaagt tgattataga ggatattagg   2460
ttttggtttt tttttgggtt ttgtggttag gttttttttg gagattttgg tttaggagtg   2520
gagattttgg tgtaggagtg gtagttttgg aggaggggtt gggttttggg atggagtgaa   2580
gaggaatatg gttgttttta ttttggttta ggttttttt ttagaggggt tgtagaaatg    2640
tattgattag gttatttaag aaaagatagt atttttgtta ggttagtatg tattttttt    2700
agggtttaat tttttattga agaagaaaag attttttttg ttgtttattt ttttatgtag   2760
ttttttaata gttgtttttt gaatgttaat taaagttatt gttttagggt ttggggttat   2820
tttaaggtat taggagatga ggatttttgt ttttatgtgt tttttgttt gttgtaggga    2880
ggagtgtaat gaataaataa ttaatataat gtgttagtta tttgttttat ttattaggag   2940
gtaataagag ttatgaaaga gaaagttttg agtaggggag gggagtgagg tatggtatag   3000
gagagtagga ggttgttttt taaaatatag gagtttaggg gattaattgg gaaggtgtgg   3060
gagggggagg gagggagttt tatagatata ggggagtgaa ttatgtttat tttgttagtt   3120
tttgatggta gtttgtatat attatttttt tttttttttt gttttagtt tttttttagaa   3180
ggagatttaa tttgttgttt aggttggagt gtagtagggt gattttgatt tattgtaatt   3240
tttgtttttt aggtttaagt gattttttg atttaatttt tagagtagtt aggattatag    3300
gtatttgtta ttatgtttgg ttaattttg tattttttt ttttgtagag atggggtttt     3360
gttatgttgg ttaggttagt tttaaatttt tgattttaag tgatttgttt gttttggttt   3420
tttaaagtgt tgggattata ggtgtgagtt attgtgttag gtttataatt ttattattaa   3480
aataatttta ttgtaaaaga attagtttag gtttagatgg aatgggtttt atgagttttt   3540
ttttttttt ttgtaaggtt atggtggtta ttttgtgagt tattgttgtt atggttaagt    3600
tttttttttgg ttatttttta ttatgaatta tttttgtagt gagtatagta tttattttgg  3660
tgggagggtt ttttagatat gagtaggatt tggattaagg ttaggttgga ggagattttt   3720
atgggaaaga gggatttttt gaattttaga ttttttagtt aagatgattt tattatatgt   3780
tgtttttgtt tattagtaaa tttttttatg tagtttgatt atgtttagga aatattttttg  3840
ataaaaatta gtggagatta ttgttttaga ggatttttgg gtttttttag gtaaatgtta   3900
tttaatgttt tttaagtaaa tagagtttgt ttttataaaat ttggggtttg ggtggttttt  3960
tatttttgat ttggggttgt ttttggagta gagaggaggt aatggttatt atggagaata   4020
aggtgatttg tgttttggtt ttggtgttta tgttggtttt tggtattttg gttgaggttt   4080
agataggtaa ggtgtgtttt tttttgtttt gtggggttat agttagtttt ggtagttttt   4140
gttaggagtt attgttttat atatatattt ttgagtattt gttttgtgtt aggtgttgtt   4200
ttaggttttt aaaagtatat ttaatttata ggattggtaa aagtaggtgg agagtaattt   4260
```

```
agggtggtag ggtttttgga gatttttgag aagtgtgatg aggaggggt tgtttttagt 4320
tggggttgtt tttttgtgtt aggaagatta tataattttt ttaagtgtta tgttttaaag 4380
aggaagtgtt ggtgtggggt tttagaatag tgtttttgat tgtttatgtt aatattttt 4440
ttaggggtag atttttttaa ggtttattta gataggttta aatgttggtt ttagtgatgg 4500
ttatttggga gattttttttt tataggtttg aatgtttgtt ttagtggtgg ttaattggga 4560
gattttttttt tataggtttt tgggtttttt tgggatttat gttttgggag ttaaagttat 4620
ttttttatg agtgtgtggt tggtaattta tattttttgg tgttgttaag tggattggtt 4680
gttttgggtt tttttaggga gtggaggagg aggttatttt tgtttttttg ggaagtgttt 4740
gtattttaat tttttattt gtagaatgga ttaatggttt gttttagggt tgttaggaaa 4800
tgttgtgtgg tagtatttgt gatttgtatt ttgttagttg tggggagttg aataatttat 4860
ttgttgttat taggtatagt tttaaggtgg gggtaggaga aagggttttt tatgttttta 4920
aagtaagggt ttttagagag gtttgaagag ggagtgttta gtggtgttgt ttgtgttttt 4980
attgtttttt agttattttt tgatttttgt tgtggggtat tgggtttgag gggtgggttt 5040
gggtagtgta gaagagtagt tagtattggg ttgtagtggg aagattttta agtttatggt 5100
agggagtggg ggagttttgg aatttgagag aggaagtggt tttggtgtat agaatgaatt 5160
gggtgggttt ttgtgttggt tatttttagg tttatttgtt tgtgtttttg tttttatttt 5220
agtttttagt tttgtttttt gtgttgtggg attatagagg ttgtggtaaa ttttttttt 5280
tattttatat attttttggt ttaaggttta gagtgttttt gtgggttatt taggtttatg 5340
atttgttat aattgaaatt tagaaaattg tgattatagt ttagtgtatt tgtgtgtgga 5400
aattattttt atttattttt attatgtgat aaagataaag tgggtgggta agatagagtt 5460
tgttggaggt agagtattgg ggttggaaat tttttttttt gaggaggaaa tttttttgat 5520
ttttaggatg atgattttttt tttattatgg ggttttttttt gattttttata gtgttttggg 5580
ggtgggtgat gattattttt atgttgtgat ggatttagat tttaggaggg taaggttttt 5640
atggaagttg ttgggtagtg ggagttgaat atggattttt tttagtaagt taggaatatt 5700
tttttaaag atattttgag gtagtttttg atagtaaagt agataagaga atagtttttt 5760
ttggtttttt ttggggtgtt tttatttgag ttagtgtggt tagattgagt ttttttttt 5820
ttatgtttta aggtagggat agggattgga gggtgttttg ggtttttttt ttattttttg 5880
ttgtaggttg ttaattatta gattttaata ggttgttttt tgagattttt gattttgtgg 5940
agtttagagt ttgaagtttt ggtgttagaa tttttgtat aagatttgt ggtagttttt 6000
agttagtttt atttgtttat gtgtttttttt tttttagatt ttttttttta ttgttttgtt 6060
ttaagttgtt ttatagtttg tattttttgt tggtttttttt tagattattt tatttggttt 6120
ttttatttta tttgtaatgg gtttttattt tttgaatata tttgggtttt tggaatggtt 6180
tttgtttatg tggttttatt tttatttggt gaattttttt ttgtagggag tttttttgtt 6240
ttgtttaatt tgtttgttat tggttttttt ggggagtgtt ttatttttgt ggttattttg 6300
ggtattttgg gatgatggtt ttgtgttgtt ttgtatatgt ttttgttttt ttttttatt 6360
agatttttag atttttttt tttttttttt gagatggagt tttgttttgt tatttaggtt 6420
ggagtgtaat ggtgtgattt tggtttatta taatttttgt tttttgggtt taagtgattt 6480
ttttgtttta gtttttaag tagttgggat tatagatgtg tgttataatg tttgtttaat 6540
ttttgtatt tttagtagag atggggtttt attattttgg ttaggttggt tttgaatttt 6600
tgattttaag tgatttattt tttttagttt tttaaagtgt tgggattata ggtatgagtt 6660
tgggtttaga tatttagatt tttattaatg atttttttgg ttttaatttt tgggtttttt 6720
ttatttggta tagtgtttgg tttttgttat gttagttttt atttttatg tatataaatg 6780
gtgtttagta aatatttatg tattgagtaa aatttaataa ttatttgttg aaattaaaaa 6840
gtgaataaat aagttattta gaaagatgta aagtttataa atttggggta ttttgtattt 6900
tttttgagtg taatgtttgt atattaggat gtgaggatta tgtttttttt ttatgttttg 6960
agggttttat atttgtttta ttggatagtt gttgatgtta t 7001
```

<210> 104
<211> 20
<212> DNA
<213> Artificial Sequence

<220>
<223> chemically treated genomic DNA (Homo sapiens)

<400> 104
ggagtggagg aaattgagat 20

<210> 105
<211> 22

<212> DNA
<213> Artificial Sequence

<220>
<223> chemically treated genomic DNA (Homo sapiens)

<400> 105
ccacacaaca aatactcaaa ac 22

<210> 106
<211> 33
<212> DNA
<213> Artificial Sequence

<220>
<223> chemically treated genomic DNA (Homo sapiens)

<400> 106
tgggtgtttg taatttttgt tttgtgttag gtt 33

<210> 107
<211> 22
<212> DNA
<213> Artificial Sequence

<220>
<223> chemically treated genomic DNA (Homo sapiens)

<400> 107
gtaggggagg gaagtagatg tt 22

<210> 108
<211> 24
<212> DNA
<213> Artificial Sequence

<220>
<223> chemically treated genomic DNA (Homo sapiens)

<400> 108
ttctaatcct cctttccaca ataa 24

<210> 109
<211> 20
<212> DNA
<213> Artificial Sequence

<220>
<223> chemically treated genomic DNA (Homo sapiens)

<400> 109
agtcggagtc gggagagcga 20

<210> 110
<211> 27
<212> DNA
<213> Artificial Sequence

<220>
<223> chemically treated genomic DNA (Homo sapiens)

<400> 110
agttggagtt gggagagtga aaggaga 27

<210> 111
<211> 144
<212> DNA
<213> Artificial Sequence

<220>
<223> chemically treated genomic DNA (Homo sapiens)

<400> 111

gtaggggagg gaagtagatg ttagcgggtc gaagagtcgg gagtcggagt cgggagagcg 60
aaaggagagg ggatttggcg gggtatttag gagttaatcg aggagtagga gtacggattt 120
ttattgtgga aaggaggatt agaa 144

<210> 112
<211> 22
<212> DNA
<213> Artificial Sequence

<220>
<223> chemically treated genomic DNA (Homo sapiens)

<400> 112
aacatctact tccctcccct ac 22

<210> 113
<211> 32
<212> DNA
<213> Artificial Sequence

<220>
<223> chemically treated genomic DNA (Homo sapiens)

<400> 113
gttagtagag attttattaa attttattgt at 32

<210> 114
<211> 14
<212> DNA
<213> Artificial Sequence

<220>
<223> chemically treated genomic DNA (Homo sapiens)

<400> 114
ttcggttgcg cggt 14

<210> 115
<211> 16
<212> DNA

<213> Artificial Sequence

<220>
<223> chemically treated genomic DNA (Homo sapiens)

<400> 115
tttggttgtg tggttg 16

<210> 116
<211> 164
<212> DNA
<213> Artificial Sequence

<220>
<223> chemically treated genomic DNA (Homo sapiens)

<400> 116

```
gttagtagag attttattaa attttattgt atagtggcgc gcgggcggtc ggtcgagttc      60
ggttgcgcgg ttggcgattt aggagcgagt atagcgttcg ggcgagcgtc gggggggagcg    120
agtaggggcg acgagaaacg aggtagggga gggaagtaga tgtt                      164
```

<210> 117
<211> 22
<212> DNA
<213> Artificial Sequence

<220>
<223> chemically treated genomic DNA (Homo sapiens)

<400> 117
gttaggtgta tgggaggaag ta 22

<210> 118
<211> 19
<212> DNA
<213> Artificial Sequence

<220>
<223> chemically treated genomic DNA (Homo sapiens)

<400> 118
tccctcccct atcttacaa 19

<210> 119
<211> 20
<212> DNA
<213> Artificial Sequence

<220>
<223> chemically treated genomic DNA (Homo sapiens)

<400> 119
acccgaaccc cgcgtacttc 20

<210> 120
<211> 24

<212> DNA
<213> Artificial Sequence

<220>
<223> chemically treated genomic DNA (Homo sapiens)

<400> 120
acccaaaccc cacatacttc caca 24

<210> 121
<211> 166
<212> DNA
<213> Artificial Sequence

<220>
<223> chemically treated genomic DNA (Homo sapiens)

<400> 121

```
gttaggtgta tgggaggaag tacggagaat ttataagttt ttcgattttt tagtttagac      60
gttgttgggt ttttttcgtt ggagatcgcg ttttttttaa atttttgtga gcgttgcgga     120
agtacgcggg gttcgggtcg ttgagcgttg taagataggg gaggga                    166
```

<210> 122
<211> 25
<212> DNA
<213> Artificial Sequence

<220>
<223> chemically treated genomic DNA (Homo sapiens)

<400> 122
gtaggaagag gtgttgagaa attaa 25

<210> 123
<211> 25
<212> DNA
<213> Artificial Sequence

<220>
<223> chemically treated genomic DNA (Homo sapiens)

<400> 123
ccacacaaaa aatttctata ctcct 25

<210> 124
<211> 18
<212> DNA
<213> Artificial Sequence

<220>
<223> chemically treated genomic DNA (Homo sapiens)

<400> 124
acgggtagag gcgcgggt 18

<210> 125

<211> 30
<212> DNA
<213> Artificial Sequence

<220>
<223> chemically treated genomic DNA (Homo sapiens)

<400> 125
atgggtagag gtgtgggtta tattgttttg 30

<210> 126
<211> 264
<212> DNA
<213> Artificial Sequence

<220>
<223> chemically treated genomic DNA (Homo sapiens)

<400> 126

```
gtaggaagag gtgttgagaa attaaaaatt taggttagtt aatgtatttt tgtcgtcggt      60
tgtaggtttc gtttttgtat taagcgggcg ttgattgtgc gcgtttggcg atcgcgggga     120
ggattggcgg ttcgcgggag gggacgggta gaggcgcggg ttatattgtt ttggagtcgg     180
ttcggttttt tgtgtttttt ttagcggtta agttgcgagg tatagttttt tattgtttta     240
ggagtataga aatttttttgt gtgg                                            264
```

<210> 127
<211> 26
<212> DNA
<213> Artificial Sequence

<220>
<223> chemically treated genomic DNA (Homo sapiens)

<400> 127
gtgaggtatt ggatttagtt tatttg 26

<210> 128
<211> 24
<212> DNA
<213> Artificial Sequence

<220>
<223> chemically treated genomic DNA (Homo sapiens)

<400> 128
ccctaaatct catcctaaaa acac 24

<210> 129
<211> 24
<212> DNA
<213> Artificial Sequence

<220>
<223> chemically treated genomic DNA (Homo sapiens)

<400> 129

tgaggtttcg gtttttaacg gtgg 24

<210> 130
<211> 27
<212> DNA
<213> Artificial Sequence

<220>
<223> chemically treated genomic DNA (Homo sapiens)

<400> 130
tgaggttttg gtttttaatg gtgggat 27

<210> 131
<211> 168
<212> DNA
<213> Artificial Sequence

<220>
<223> chemically treated genomic DNA (Homo sapiens)

<400> 131

```
gtgaggtatt ggatttagtt tatttggttt cgaagttttt gttttcggaa ttcgggtgtt      60
gtgggttgag gtttcggttt ttaacggtgg gattggtgtt ttcgagatga aatttggggt     120
tttttcgggg ttttggtggg atcggtgttt ttaggatgag atttaggg                  168
```

<210> 132
<211> 168
<212> DNA
<213> Artificial Sequence

<220>
<223> chemically treated genomic DNA (Homo sapiens)

<400> 132
gtgaggtatt ggatttagtt tatttggttt cgaagttttt gttttcggaa ttcgggtgtt 60 gtgggttgag gtttcggttt ttaacggtgg gattggtgtt ttcgagatga aatttggggt 120 tttttcgggg ttttggtggg atcggtgttt ttaggatgag atttaggg 168

<210> 133
<211> 168
<212> DNA
<213> Artificial Sequence

<220>
<223> chemically treated genomic DNA (Homo sapiens)

<400> 133

```
gtgaggtatt ggatttagtt tatttggttt cgaagttttt gttttcggaa ttcgggtgtt      60
gtgggttgag gtttcggttt ttaacggtgg gattggtgtt ttcgagatga aatttggggt     120
tttttcgggg ttttggtggg atcggtgttt ttaggatgag atttaggg                  168
```

<210> 134
<211> 24

<212> DNA
<213> Artificial Sequence

<220>
<223> chemically treated genomic DNA (Homo sapiens)

<400> 134
ggagggggta gagttattag tttt 24

<210> 135
<211> 21
<212> DNA
<213> Artificial Sequence

<220>
<223> chemically treated genomic DNA (Homo sapiens)

<400> 135
actcccaact tcactttctc c 21

<210> 136
<211> 24
<212> DNA
<213> Artificial Sequence

<220>
<223> chemically treated genomic DNA (Homo sapiens)

<400> 136
taatttaggc gtttcggcgt tagg 24

<210> 137
<211> 29
<212> DNA
<213> Artificial Sequence

<220>
<223> chemically treated genomic DNA (Homo sapiens)

<400> 137
taatttaggt gttttggtgt taggaggga 29

<210> 138
<211> 144
<212> DNA
<213> Artificial Sequence

<220>
<223> chemically treated genomic DNA (Homo sapiens)

<400> 138

```
ggagggggta gagttattag tttttgtatt tagggatttt tcgaggaaaa gtgtgagaac      60
ggttgtaggt aatttaggcg tttcggcgtt aggagggacg tatttaggtt tgcgcgaaga     120
gagggagaaa gtgaagttgg gagt                                            144
```

<210> 139
<211> 23
<212> DNA
<213> Artificial Sequence

<220>
<223> chemically treated genomic DNA (Homo sapiens)

<400> 139
agttggtgat gttgattaga gtt 23

<210> 140
<211> 26
<212> DNA
<213> Artificial Sequence

<220>
<223> chemically treated genomic DNA (Homo sapiens)

<400> 140
ccctcccaat atacaaataa aaacta 26

<210> 141
<211> 15
<212> DNA
<213> Artificial Sequence

<220>
<223> chemically treated genomic DNA (Homo sapiens)

<400> 141
acaccgttcg taaaa 15

<210> 142
<211> 16
<212> DNA
<213> Artificial Sequence

<220>
<223> chemically treated genomic DNA (Homo sapiens)

<400> 142
acaccattca taaaat 16

<210> 143
<211> 189
<212> DNA
<213> Artificial Sequence

<220>
<223> chemically treated genomic DNA (Homo sapiens)

<400> 143

agttggtgat gttgattaga gtttttgtag ttttaaatga tttttttaat taattttaaa 60
tttttagaat ttatcgtata aaaaggttat attttttgga gggacgtcga tggtattagg 120
atagaagtat taggggattt tacgaacggt gtcgtcgaaa tagtagtttt tatttgtata 180
ttgggaggg 189

<210> 144
<211> 27
<212> DNA
<213> Artificial Sequence

<220>
<223> chemically treated genomic DNA (Homo sapiens)

<400> 144
tttttagttg gtttttatta gggtttt 27

<210> 145
<211> 22
<212> DNA
<213> Artificial Sequence

<220>
<223> chemically treated genomic DNA (Homo sapiens)

<400> 145
ccaacatatc cacccactta ct 22

<210> 146
<211> 21
<212> DNA
<213> Artificial Sequence

<220>
<223> chemically treated genomic DNA (Homo sapiens)

<400> 146
tttcgactaa tctcccgccg a 21

<210> 147
<211> 32
<212> DNA
<213> Artificial Sequence

<220>
<223> chemically treated genomic DNA (Homo sapiens)

<400> 147
tttcaactaa tctcccacca aatttactat ca 32

<210> 148
<211> 155
<212> DNA
<213> Artificial Sequence

<220>
<223> chemically treated genomic DNA (Homo sapiens)

<400> 148

```
tttttagttg gtttttatta gggttttaga gtttaagatt tagtattcgc gggcggtttt      60
gggaagtttg gtagtttcgt taattttaat atgttttatt tgatagtaaa ttcggcggga     120
gattagtcga aagagtaagt gggtggatat gttgg                                155
```

<210> 149
<211> 28536
<212> DNA
<213> Homo Sapiens

<400> 149

```
gctctgggag ctaggtttct ctcgctggat gggaaaagga ggtgcggaaa ggggcaggcg      60
gcctgcgggc tagcggggat gagagttgtg gggagaacgg aggagagagt aaaattattc     120
cttttggaag tccaagggaa gggagctgag gtgagagaaa tggcctctct taggtcccaa     180
gtctccgccc ccagcctgca ccactcaagt cggggaagtg acaactcttc ctaaacctcg     240
acttccattt tttgtttcct gcagattgat tgatgtcctc gcgttgttct agttcagcaa     300
ccaaactagg ccgaggtaca cctcttttac ccagagctcc caaatcactc accaacctgc     360
aggagaccta aatcctccgg gcaggtctcg ttagcgcttc cagcccccat ccccaaatcc     420
cctcttctag ctccttcctt cccctccgcc ctttggccct ccccgccccc taccaggctg     480
agaggcggag aggccgggac cgctgggccc tgcggctcca gaagccaaag tccgcccgtg     540
gggaccgttt tcctctccac tgctctcaag tgaacaacat ggtgggaggt gaccctcgtc     600
caaagggctt ttatctgccc cccactgctg cgtgatccct ccgcaagtcg gaggcagagc     660
tttaaaaaga aagaaagtgt gcgttgagca ctgatcttaa atcggggaca ttatctgcga     720
tctctttaag tggcagcagc agtttcttgc agaacaaaga ctcagtgttc gagctccaaa     780
```

```
agcttctagg taaagtttca ttcagatgaa agcaactaag gcgaaaaaac aatgatattg     840
tcggctccag aaaatcggcg gttacttttt gctctaagtt ccccagcgtg gtgtttgttc     900
tcgcccactt tggttgtgcg gggggttgac aagcataaca aaagatctca gcacatccct     960
ccccccacct ccacaacgac cctcctagcg ccatgaggat gaattctctg ggactaagtg    1020
gtatttgttt ctatttgtta tgaaatcaaa tttcacattt ttttaaagct gaaaatcgcg    1080
gataaagaag gatcgggtgc ttaaagttca tttaaacact cacaccgaac tcattccctg    1140
tttagatgtc agaggatggc agggagggcc agggctgtaa ttcatcttga tttgcttcat    1200
tgtcctgcag tttgcaaact ataatcctgt ataatttcag gaacaagcag gtttagaatt    1260
aatgggtcaa tattatttaa aacaaaacac cgggagcatg acctttgcct caactacata    1320
ttgctcgaca agactcagga aactccactc taacctctca accctgagt tctttgagaa     1380
actgaagggc tgtagttatt agaaatcatc tttgtttctt ttaatgtctc caaaggattt    1440
ggaaatagta atgcaatata acatgaagga tctctctact taagcctgaa gataaacgtg    1500
gaagcctaaa tattttgaac tggagatgtt tccctgtaaa tttattatag atgtattcct    1560
cctgagagaa atgcatataa actatacatg tatacatgca caaatatttc tccagaacaa    1620
tttgctagag gtgtaggttt ccccgtaaag gagtaaactt gagagtcatt ttaagctgat    1680
ggacttgcta aatttctttc ttcttttttc tttttcatat tatttgctag ccataatgga    1740
atcctctagg tttaagccaa agaaaaattg gagagacaaa attagatttt gtagcccttt    1800
tccccccccgg gaatgccttt tttttctttt ttagtttctg atgaatggct atcatttatt   1860
tctaccaaat ttaaataagg actgctgcct tgtatgttta actaggcagg cagagggaac    1920
tggtttgttt aggaagcagt gactgagatg tcctggccaa gttagtgaca gaggaggga     1980
gaaagaatcc agaccaattt gtatgcagta tattttactc ccatgaaata aaacacattt    2040
gtttcatatt tgctgaaaag taaaacaata atattgtacg aaatgttata cacagggtag    2100
gttgtacata gcagtttcag aaacatcatt gcatccacca gagaaactat tctaaaactg    2160
atattcacac atttttata ataataataa tatgttagaa acatacagtg tggcatttag     2220
tatatacact cccttgctcg caagcgaaaa atcctaatcg cttctgtata acatgcttta    2280
ttttaaagcc taacctttaa aaacactgtt gtgatattac taacaactgc ttttataaaa    2340
ttaatttgac atttcgatat atatacatcc tttcagtcat ttaaatgtta acaatgctaa    2400
acttaaaaaa taacaagctt atagtaatgt taaaatgtca tatccagtca aacatttgtt    2460
tgtgtatgtg tccttgcaac tgttagaaat acttgtagtg aaagatgtca gacactgagg    2520
acatcccttt gaaatcaaag gagctctctc tttgattcag tggtttcctt ttctctatat    2580
agcttctctt tctctccctt tctttagtgc ccacgacctt ctagcataat tcccagtctt    2640
tcaagggcgg agttgcccca tccggcaagg tcctaggatc ccggcgctgt gggtgcggct    2700
cacacgggcc ggtccactgc atactggcaa gcactcaggt tggaggccgg gttctgcacg    2760
ctggcgtagc cgaagctgga gtgctgcttt gctttcagtc tcaggctggc caggctcgag    2820
ttacacgtgt ccctataaac atacggagga gtcggcggcg cgtaaggaca ggcaggcgtc    2880
ggcaccgcgg aattcagcga cggctactc aggttgttca agttattcag gctgttgaga     2940
ctggagcccg ggacgcctgt cactgctgag ggcaccatgc tggacgacat gctcatggac    3000
gagatagagt tgggtgggga aaacatgctc tgtgatgaca gggggttgac gttcatagag    3060
ttgaagaagg ggaagctctt ggtggatagg gaggcggatg taaggccctt ggcggcccag    3120
ttgttgtagg aatagcctgg gtacatgtcg tcgtagggct gcatgagccc attgaactgc    3180
ggcccgaagc cattcttgca tagctcggcc tgctggttgc gctccctctt tctccatttg    3240
gcccgacgat tcttgaacca aacctggggg cggttggggc aagggagcaa acagatgcca    3300
cagtgcagat tactaaaact tccatcggag gccaaccccc gccttccccc gacacacacg    3360
ctagcgcact cacacaccct ggcctcgctt cactgcaccg ccctgcacac caagatacca    3420
gggccagctt tcagttactg gcccgggtct ccaccaagcg caggagacct ggtctgctct    3480
ggcctgcgag ctgggactcg gagctacgcc acaaacctca gccgaacgca tggagacctg    3540
cggacggttt gatcactcag ccaggcgttt ctccaggtcc aaaaacactt aatgtaaaac    3600
aaacgcgggg cagcaggctt ttccaaccct tcccggggca ccttgcaaac ttgcttccat    3660
tccaaagcca cagacccacg gatgaggaga aggggctgga agggcactag aggatcgctc    3720
tttctcccac gcaattcctc ccttccttcc ctgacctcca ctgtcgtccc ccaccccctg    3780
gtacgtgctc ccttaacagg gactaggccg ccaacactct ttctcgccta gcaaaacaac    3840
caaataaaga gcaaaagacc acctcttcgt cagctcgtta actccaggag cttggcatat    3900
taaactccgg gaacccggaa agggtagttt tggagattcc cccttctttc gctctgcctc    3960
ttctttaccc taagcccacc acaggcctgt ccgcgcgcca ggcccagccg ggtcgtttgg    4020
ctttgcaggc ggccacccag gccggccggc ttccacccgt gtccggtggc ccagccgcaa    4080
ccccgatccc aatccacatc gggcctccct gtcgccccag acggcggctt ttgtgtattg    4140
gagagaggcc tggcctgaga tatccgagct gacaccagtg atgtttcaca ttacacatct    4200
ccgccgggcc cagccgtgta atccgctttt tctcttttc ctttcattct tgatttcctt     4260
tttatccccc ttcctctttg cacccgactg ctataaaaag cacgcctcac tcccacttgg    4320
ctcgacaagc agccgccctg gaaggagagg cagctgcaag gagagcccag cgccgcggct    4380
acaaagcact agggtggagc tgcggaatag cgggcggggt gggagggcgt tttcgaagga    4440
tcccagaaaa cccatagact ctgtctttaa ttacttgcca tttctaccct aggccatcta    4500
aactttgctc aggcgagaag agtacgtgag aggcccgttc ccttgatgtg caagagagct    4560
```

```
aatgaaagac tgaccttgct caaaaccacg ccgcccagga cccagctctg gctctggaca    4620
gttaaactaa aaccattttc aacttcttcc cggcctttta tccaccagca tagcctcatg    4680
ccttgcacaa atgccaccca gagagtgtct tcattccctc tgatttggga gagcattttg    4740
gtctttattc tttttatcgt tgttttcttc tttttgtttg ctctgctcta accgggggct    4800
ttattttttc tacccagagc acttaatttt tttttttaa cagcaaagcc tctggatgcc    4860
gcttgatttg cttgattctg ttttctgctt ccagaatcct aacaaatttg gaatcttcca    4920
ccgaccagca taaaccagga cgttgctatt gggttattta tttgagctca tttttgccaa    4980
tccataaagt acagatttgc tacaaagtta aggtaagccc tttttacaaa actatgatta    5040
taatttagaa gagggggtgt gagtttcaat ttccagagtt caactcctga gagaagataa    5100
ataaaccaag cagaaaagtc tttcttcttt ttttctttct ccttctaaga ggactagtag    5160
ttgtgtatta aaactttgct cccggagatc acaaaactag gaaatagggt gtgtgggaga    5220
gacctgaatg gccgaaacaa ccgtaaagaa ggtgtaagaa gcgcgagccc aggagggaaa    5280
aagctgggcc agggccggga caaaggtttc ccagggaggg ccaactcttc cgtgtctctg    5340
gcgggttttc cttgttaaag gctcacaggt tggagcctgt tcgcggctct tggcctggta    5400
gggattttat tagctctgct ctggcaactg caagccagga acacaatgtc ctgtgcaggg    5460
gattgcccat gcagcccagc tcgtgagatc gcgggatggc ggggcagtga gccggtgccg    5520
ctctgggagc ctgagccagg gcggcagtcc tgtcggcctc ggagagggaa ctgtaatctc    5580
gcaaccaggc cgccgcgagg ccttctgcct ttgcaaagct gcgccccacc ggcgccctcc    5640
caggcggcgc tgccttccac attctctcct ggtctacttg gcctgtacct ccacaacatc    5700
ctccccccat ccctcccaga ctccgtgctg gctcctaccc ggactcgggc ttccgtaagg    5760
ttggtccaca cagcgatttc ttcgcgtgtg gacatgtccg ggtagcggtt cctctggaaa    5820
gtggcctcca gctcctggag ctgctggctg gtaaagtgag tccgctgccg cctttgccgc    5880
ttcttcttag acgggtcctc ggcgcccacg tcctcattct tcccctgctg gcttttatct    5940
ttctctgaaa acgaaacaca cacactttcc cgtcagcatg cccacctgca acgcggacgc    6000
caactggacc ggcggcagaa gccgtggaag agctgggctg cctggcgccg gaggagggtg    6060
cgcgcggcgg ctccgggccg cgaggagcgc tgcgcctgtg gggtgtgcag gcgcaagtgt    6120
gggtgtccgc gccccatttc ctcccctccc ccagcgccgc acgttttatt tacatgttta    6180
tctcactgca gcggcacatt cacttttata gcctgtgctt tcaagtatat ttatacacct    6240
ctgcgcagac acaccaaatc tcctgggacg cgcacacgcg cgtggtttac agaccccccct    6300
ccccctcgca gaaagctcag atttccatgc ggtttgggaa ggctaggaaa agatgtgggg    6360
attcggttgg gcaccgaagt tcgccggccc tttcccaaaa aaaaaaaaaa aatgcctctt    6420
cgcgaagggc atttctgagt ggtttcaggc aatttcctaa cgagtggagc tcctcgggag    6480
ctgaaagccg agaggaaaac agggacagag gtcggcggcc tctgaaggtc ctcgaatcaa    6540
gatgctggga tttttgtgac ccaggaaaca gaagggaggc cagggtacga atagagaggg    6600
cggcagaatt gctcgcgccc ttagcgcccc aggagccggg ccggtcgagg gagaactaaa    6660
gggatgcggg gtagtcaaaa ttccggctcc cggaagttct gcggggagcc aggcgaacga    6720
ccactcccac cacgcctccc cccggagggg ctgacttcct tggggcgaga gggagcgggt    6780
ggcgcagagc agctgagcgg gaatgtctgc agggcggcgc ggcgccttac ctgcggcctc    6840
cgggctggag gtgtcggaga tggtgtgcac ctccagcctg tgcttggagg agtccagcga    6900
ccggggctga ccgggagcca gaaccgaagc catggctaac ggctggggat ggtgacagga    6960
agatgaggag acggccgaca gcttggtccc cgctgctcgg tgctccaagt gaagcgggcc    7020
tttcatgcag ttcatggacg agggagcgcg acgctctact agtccttggc tactgccccg    7080
ccgagccccc gtagccgccg ctgcccgctc cgggtcgcgc tctaggcgcg gagtttcccc    7140
gctgcgggga gagccagggg acgcaacccc cgccgagttc tcaagccaag ctgccccccgt    7200
ctcctccgga aggctcaagc gaaaaagtcc ggagacggaa agtcagcggg caaacgaaga    7260
catgggatgt gggcagaagg gcaccactca gagcgtcttt agggagcagg cttccaagct    7320
ccaaagcgaa acaagagtgg gcaaagaccc ccttcttctc tccctccctc ccccaagaac    7380
ccctccaata aggaaagcta acgccgaccg cgctctgccc gcccccccccc cacgcggcag    7440
ccctgacaga gaagtgtcaa gagtgacagg gacaggtagg tgatattaga tcccctgcgg    7500
cggcagcagc cgctgcagcc acgacgcggc cctctgagcg caccctccgc aacgcgcaca    7560
cgcacacccc tcgggcggtc gaacaggagc cgggccttgc cgcagctcag ctccaggcac    7620
ccaggcgagc gacggaccag atctgcggct ccgcgcttcc ctgttggcct aacatcttaa    7680
aaccagaggc gggcttcctg gtgccgagac gtcactccgc cgcggccctc cccagccctc    7740
tccgcctccg cctcctccca gacccttctc cgggtgcgac tgacgtggct ccgcaccaat    7800
caggacgccc cgagccgcgg tggagggact gtcctgcctg cacctatcag cagtgcgggg    7860
ccgggctact gcctcgccgt gcgcactggg tctacacagg caagctcccg ggaattcagc    7920
tcctgcccag cccaaggcga tccggctttt agtacgaacc caaaggtgaa gagatgaggc    7980
taggagtcga aggcttggga gaagagagtg gaatggtcaa gaagagaaag gtacaaggat    8040
caacaagaca cccactcttt gtgtctcact acatccattt ccaatccccc accccatata    8100
aaaaggagac acgttactta aaactagaaa atttgaaaaa cagcaacaaa tcacctctcc    8160
gatcttaaat tttccaaaca gcctgtcaag tgaatgctgc gctaatctga agaagcttta    8220
attgcaaaga agacagagcc ctgaaaaggc aggctaataa attagaaatc gagaagcaaa    8280
tggacccgtc aaaagaaaat taccttgact ttaaacgaac aactgtttgg tggttcactc    8340
```

tggatttata caagaataaa aagtcgcctc agatcacgtt ctctgtgatg cttattagtc 8400
cccagacaga aaacacacaa tagaagagaa accctaaccc agcgttttca aaatgctgaa 8460
agcttatcca ttctacttaa cgttgattaa gacacatatc ctagatcttt caaattcctt 8520
gtacactgta ttaagctcgt cctaacccga gagagccacg ctttaaattc gactctcttg 8580
tttactttat tatcaatcag atttaaatcc ataaagcctg tagaatcaac aaccttgagc 8640
taattatata tgaaatatgc cttaatgaat ttccatacaa ttaagaatgt tgccaaataa 8700
ccaatttcaa ggataatttt taacagtcat tttcttttcc cagtgagctc aaggctgtct 8760
tgagccatta aagtccaagc aggcagaagg ggtgtgtgtg agctaagggc gaaaagccta 8820
gaactgcgct caactagcaa aagcaaaacc ttatttatat aaaacaaaaa aaatcacctt 8880
tggagacatc aactctttat agcactgttt ccaagcaaat ttaatttcca aagaaattaa 8940
agaaagaaat ccaaacatat tcaaaataat ttttgaaagt ccttttgtcc cccagcatag 9000
gtcagctgga gaggacaaac taatctcctc tgggtttctg catgggcgat tgttttacta 9060
tggagttagt gttatcatct ctgaatgtgt atttgtttga cattacagtc aatgatttgc 9120
aatgccagca tgaagtatct,ttaaaacact ccctccttgt ccttgttcac aagattggga 9180
aacttattcg atgtggaaca aagtggatga agcagactac aaatatattt gcaacttatg 9240
tgtctctcct ttgccctgac cacccccaaa ccctatctgc aactcctccc cattttaaac 9300
ttgcagtcca aagacgcaca tgagaattgt ttttcagtct ttcttcacca gtatcatccc 9360
actttaagaa taatttagct gcaagggagg aatttcttca tagtaagctt taaatcagca 9420
tttctgcttt taacctttta ttccacttta ccccattcca cacatacaga cacctgctca 9480
gagtaaaaca catcctcatg tgacaggtct gcattagctg aggctcatac atccagctat 9540
attaggtcct gcaatcttat cactaaatta tacacattac actagcagcc tgttggtaaa 9600
gaaggttaaa ttaatttaca ttctgctcat tatctggtgc ttaaatgacg cattttatcc 9660
cggagatttg gcggagaatc tccttctcag accccacagc gtttcactga agacaatgct 9720
cttacatttg tagtggtttt taatctgata agactctaat ttgcttaagt cttttaaata 9780
agggttttaa atgtttctag ccgttttctt attgaatttc ctctaattcc cccaagatca 9840
taaagtatat gtgtaaagta aatatttcct cccattgcac tgccagccga tgacctataa 9900
ctaagtcaat aagaatccag ctcttttctg ctgaatgtgt ttactaatca tattccagtt 9960
tcttctttta aacctcagaa tagctgtggt ccccacaata ccatgcccct taaagcttca 10020
tttcatgaag ggactccatc acattaaaga atgaaaaaaa tctccactgt agttagtata 10080
cacagcccct cactccttgt ttttcaagat tcaaacccca gagctgcaaa tatttttgga 10140
agcttgggtg ttaatgcctt attttagaaa gccgagaagc cccacagagc catatagatt 10200
tctaaaccca tctctcataa acccacagaa ttttgataaa agctctggtg gctccactct 10260
accgatggaa ctttcatcac gacaaatata catgtatgaa ggacctcaat cagcctccaa 10320
agtggttgaa aaacccaagg gcacgtgact gctcctcata gtgccaacgt gtgcgagatg 10380
ttggaagcac tggggatcag cagcagccta gatgcctaaa aagataaggt gtcctaattt 10440
gtgtggaccc attgaagtca agtggtgaat aaagacaatt atctagataa ttcagattaa 10500
agtaaaagca aaaccatatc tatttgtata tatatattca catccatttt atattacaga 10560
cacacacacg cacacacaca ctggctctgt aaacaactga ctcaaagtga ggattttctt 10620
tgcatttttc cagcaggagt ttcaacattc tcctaatctc ctaatcactt tacacactca 10680
cagcagcggc gattgggtga cattcttctc aggccccctg tgtggcagga caccaacatg 10740
ataagtctgc atggggaaaa ggaggtatgt ggtgggaact aagaaacact gtccagtgaa 10800
aactctgtgg catggtggtg gttgattttg gagatttaat gtacataaga cttgtgggtg 10860
cacaggcata ggcagcatgg atgagaaagg ggccagaaga aaataaatct tatgcatttt 10920
gtgattccag cattactgtg acctctggct aagttcttct taattggttt tagaaattac 10980
tatgagttca gcctctaaca cagaaatttc caatacggag aacattggtg ggatcctggt 11040
agggaaacta gaggtgctgc atggctcacg tggggcaaag aaggaaagcc cagtgccggc 11100
gtgaggtttt gagtctggga gacatcaggg gttgtctcga ttggggtttc ttgcttattc 11160
tttcaaagaa agaccctaga ggagggaaat gtgtgacatg gggtcagccg tgttttgtgc 11220
tggtatttgc caccgattac cagtcttaaa gtcttattta atttcacact cttcagtgtt 11280
agttgtgcaa agtccctctg gccatggcag tgagcggttg ggctgtgccg ccaaactctc 11340
cgtatcaatc tggcctggga ctcaaccaag tgatctctga cttttggaaa gagtctgtct 11400
tcagagttca cccagaagat ggcttaatta gacatctccc tgagctgtta ggccttagac 11460
gggtgggagt cctgccctgc ccaagctagc tcaaggacga ggcccgcctg gactcagctt 11520
ggagccacgt gatgggcgtg agtgtgtgag ctcctggtaa ggcgcagagg tcagatggag 11580
accttgcatc ctgcccgaga agtgccccac cccctccaat atctggcttt tctctgcata 11640
caaaccaagc tgaaaacagt ccactaccca ccacccctca tagctatgga accaaataac 11700
ccagaaatta aaagcttcac tgtagctgtc cttttcccca tttcctaaat ggaatttaaa 11760
aagctctggc ttgtcaaaag gggaagatta ttttctgaat tggaagtctg tagatatatt 11820
gagcaacagc caccctctct gggtccctgc aaatggtacc cattttttcca acccacagct 11880
ctagctgctc aaccatttga gatttggggt aactacctgg gggaacagtg ttcagatggc 11940
agtgggagtt accacctcac agtggcctgg ggaagagaag agaaagagat tagaggaggg 12000
ggcatttgct aaaatcactc aacgaacatg ctgttaatgc ttcctcacat ttgcatgtta 12060
ctgccacagt tttcctaggt gtcactgagt ctccagaaag caactacttg ccgaactaag 12120

taaaataagg agaatggtat agcacatgtg tttggagaag gggaaggaag ggtggaatat 12180
gaaattgagc atagatatcc aggtcaggaa agaaggaagt ggcaaggggc taaatgaagt 12240
cccacccccc cgccactttt ttaaacaaca gttggattaa acactcatct gtcttcctct 12300
ttttctcttt tcttccctct ccagcttatg tccatctccc ttttctattt cttttgctct 12360
cccttctttc tgtttttctct ctcccagaca tgctggtagc taacattcag cattagttgt 12420
catggtgacc ataaatcacc taaatccaaa aatacccacc tataatctga gatgaagctt 12480
ttatttccct agcgaaataa tattttaaaa gctgtcagct gacaaaaaaa aaggaaccca 12540
cctcattgta gtaacccaag taatatatta cttctaaagg tttaaattaa aatgtcagcc 12600
tgttaaaaac atgctggtag agtcttggac actcttcccg tcagatcctc acaaagaagt 12660
tgactctgtc attcctggtc gctctttaac atacacacac aattctgtat gctctctccc 12720
tttctattaa tttcttcctc ccctaccaac tactttagtt ctcaaagact ctacgcattg 12780
ggttctaaaa gaaaagaatc tcctcctgga tcagaaaaca gcctcattgg ttgctgaagt 12840
gaaagatgtg gggtttaggg ggaaaggtta ttaggaccat aatggcggtg gtggtaggag 12900
gtcatcctag aggagttaag aagaaaaaaa aatgcaggga gaaggactgg aggtggaaag 12960
acagagtaac agaaaactga gctgggtggt caggtgctgc ggtgaagtct agccccgaaa 13020
tgataggtat atattctccc actcctgcct ttttctcctc tgagagaaaa ctttcctagt 13080
cagagaccct ggggggtagg aggcgggcaa acgccgctgc agttgggcct tttgtcttct 13140
accttgggct tgttgtcttt tgcctatctt ggattacagg gtattcgcct agatgaagag 13200
ccattaatta cccattcagt caatattagg aagacgacaa agctttttac ataggattaa 13260
gaagacatca gattgttcca ttagtatatt cctgctcacg aataagcttt cgttatacat 13320
ttccttttcc cccgagccgc tctaacaact gctgcatata ttagcagcgg gcggtgagga 13380
ataacagccg aaccagcctt aagaaacttt gtgtaccgag tcagcagcga ggaacgcgac 13440
ctgtgaagac cacttttgcg ggcagggatc cgcagggact gactactttc ggacaactgg 13500
tacaatttcc cctgggggtg aaaaaccaca acgcggcggg gcacctttca agtgagctgc 13560
agatttgatt ggcgcggggg gtgggggagg ggaggggaga atgggatggc ggaggtcggg 13620
cggaggaaag aaaatggaaa actctcctta cccctatttc gttgcttttt cctcaagcct 13680
catgccccct tcggcaagca cctgtccctt ccgcgctagc cacagctaga gttctccacc 13740
tttctctaca catccccttc tttctgacaa ggctcaggac cttggtcacc accccacgca 13800
ccaccatttt gcgttttgct agagacggtc tgggctgatc tcgctggtgt ccatgttagg 13860
attaaaatcc cttcatgacg gcgaggaaaa ctgtattatt tgctttcagg gggtacatta 13920
ggagtccacg tagtatatgc tccgcaaaca ttcgctgatt gaatgagagg cgcgggggcg 13980
gggcggcgga gaggggtctg cggccgccaa ggccgccagg gttaacccag gtccttcgaa 14040
gaaggttggg accgagctgc tgccgcgtgt gaaggtgtgt gtcgcggctg ggggtgccac 14100
aacgggccat ggagcccacc tcccagaggg aggaagtctg tgcacaccag cgacttgggt 14160
cgaacacttt cagcccatcc actgggccaa agctatttaa caattaatgc gttcctgggg 14220
gaggccgggg gaagtacccg ccccttgtcg ggacgcaaag ccaggcgtca ggtccaaagg 14280
ggctgcagtg cagtccgatt tcagcacgga acccagagcc gccgccctga aactttttaa 14340
gccagtgaca gaggagggtc agtccgtcct cctcctgagg gtgaagacca ttttatgagt 14400
tcctttcagg acccccaaag caagaaaagc caaagaaagg tctctttgct ctaggggggtc 14460
gttctcagct ggcttctaca ccatcttctg ctagctgcgt ccaccctctc tcaaacctct 14520
ggcttttagg ggtctccagt cgctctcgtg ctaccccccgt ttcggggttc tatccaggct 14580
gggcatccgc ccaatggata ccaaggagaa tgggactcac tagggaagga aggcagagcc 14640
tggactgctt agaggtggac tctgcctata cagaacgttt agtctttaac gaggacatgg 14700
tatttttggg cggcgttggg ggcagaggcg gagagggtag cgtaatagac tatcacggcc 14760
ttttgaagaa gtcatatgtc attgtgaact tttttctct ttaaaagcaa agaaaaactt 14820
taaaaaaaca caagaaataa atccttttgt tttacaagca ggctgtggcc aggactttgg 14880
atattccata agttaactta aaatcaggga aggataggtg ctctatcctt cagcagtgct 14940
atagctttgc ccactcgtgt gatttttttt tgtcgtctac tagttcctta aaagccaatt 15000
aaatcaagat tttcagcatt tcttcccatt acatgccttt tcttaattaa tggcattaaa 15060
cgtgtttagg cagcaatttt tcttttcggt caaaaagtag aaaaagacat attgagtgta 15120
ggggaagagc ctttcacgtg cactaaaaca atgcgggcct gaaagtaatg gttaagaaag 15180
caatcattca ttatttctag ttctttatgt gctagttatc aaaagcgaac gaccaggggc 15240
gcctgcgcgg cttgtgactg gcgcaagcag aactcctctg ctcttagccg tttcctgctc 15300
acctacgagg accccatcct accgtaggct tctccacctg tcctcagaat gcattcccca 15360
tgtctaggaa acctggggta gggactgggg gaaggagaca ttttgcgtct ctctggctcc 15420
cagcacaata agaaatgtca gcctcggctt ggcgactgcg agcccgcccc gcgcgaagcg 15480
agattgggga gctcctctag tctggccgga gccagggctg agcccgcgca aagcattctc 15540
cccagaagtc atcgctgctc ttgattttta accatatccc aaacatacta cggtctaata 15600
atttattttt actaccgatt atcaaacaga agaagaattt aacacaatct aaatgacaga 15660
aatcacgcga cgttatctct gcattgcccc catttaaccc catggggtta atcccggaca 15720
agtgagcgtt taactggccc agcagggcga ctggcggtgt agtgcagtgt ccgggcgtga 15780
agcactggat cgctttagac gcttcatttc aacaaatgat cattttctct cagattcacg 15840
gggaaactcc aatgcaagac ctttgttttc ttcttagtaa tacggtttgt ttttttgacc 15900

ggggtcccaa atcgcccccc tccatcccat attacatttg tattcttaca ctttaatccg 15960
gaaagagggg gttaggggcc gagggctgtg gggggggggg ggctttatct gctcacatta 16020
tcaaaggtca atagcctcct aaggctaggc acttatttac tacggagcca ggaaaataga 16080
ggaacagtaa atttgagggg tttttttcca tgtatttgaa aagaaaggca tcttccctcc 16140
tccatccctt acaccccccc ctccgcccca tagaacaagt ttcaattcag gaaaggcctg 16200
tggcgtaggt tggagacttt ttaacttttt acataagcct gtagacctcc tctggcaatg 16260
tccctcgatt cctccagagt gaaaccagcc aatcaagcaa cgacatcgcc aaaacccaag 16320
gcctggcaac cagtacttca ggcaggttcg cgtcgacagg ggcaaacctc cattctaccc 16380
tgggctgcta agcacagtgg ctgccctcca gctcttcagg gatgttgtcg gcccccccgcc 16440
cctcccccaa ccagccaaag caaaccttcg ccaactcaag tttcccttgc ttgcctcccg 16500
cgatgaaccg cgcacccaca agtctgggtg gggcgtggct gagagcctga gtgactgagt 16560
gggccctggt ggtgctgcgc gcagcgggat ataacgagcg acagaggtcg ctgttggacc 16620
acttttccac gccagcctag acgccgaggt ttgctggagc gtgcgcaggg gatcagacca 16680
cagggagcga gcgagaggga gagagaggtg ttgggtctca ggagtgcagt ataatttggg 16740
gaaaggaatt aacgtccctg ggacggctgc ttcccgtccc acccagaggc ggagtgtcta 16800
agttcaagca gcaggcgcgt caggtctggc ggcctcgcct tcttgcgctt cgcccgaggc 16860
ccagagtccc ggaggcgggt gcccagcgcg cggcctgcgc ttccctcccg gccttaccat 16920
tggcgccagg atgctgccgc gggaagaact tgctgctggc tgcctctttt cggctctcag 16980
gagagtccgt gaactcgacc tttttgattt cggagtcttt ggagaagaga cattcaacgg 17040
ccgccggctg cacgcctggg ccacgcgcgc gcccgcccca cgtgcggaga gaggcgtccc 17100
ggaccgcggc cgaaaggagc cggggacggg aggaggggga ggggcgaggc aggccggagg 17160
agaaagaggg acaaagagca aagacccagt tagaggaaag agctgacggc atccccgtcc 17220
cccggaccct ctggcaaccc ggggcaggat ggcgtactct ttctgcgcct ccccggttgc 17280
cggcggttcc agccgggagg agtaggctgg ggggcttcgg tacacagcgc gccgctgctt 17340
cctagtccac cgccccgcca cagggagagg ccactggcga tttggttctg atttccttcc 17400
tgcccaggct ggccccctcgg ggaagcgccc ctcgctgggg cctcgccgca gggccagtgc 17460
cctcttgccg ccctcacgtg gcgcggcctc ccgtccgatg acccgggcag gagaaggggg 17520
ttcttaccta attgcacaca cgccgacacc agtttgcggc agttggtctc cattccccgt 17580
tatctgcaaa acagaagaga aggaaggctg taagaagcgg cggccgtcga gtgagcaggg 17640
ctcagatgag actacgttac actagctgtt aggcgcctac tgtgtgctag gcttcaggcg 17700
tgtttttccg acctgacagc ctctggctgt gcagcagcat ctccagccta gcctcgggcc 17760
caggacattt atttatcaag aggggatctc cctccagagt tgccgcaaaa gtgcccagag 17820
gttagaggac tataaagcca cagtgtgctg gggaggctgt ggacctactt tcaagaatct 17880
cggtgccggg gttaagaact catctgaacg caatggcagc gggagtgggt gggtggagag 17940
gacttttctc tttgggaagc tgtatgcaaa gaccacccctt cagtgcttgt ccattagttg 18000
gagctcggta aacatttgta gaacatcagc gccaacgtgc ccctgtccta gacagcagtt 18060
tccctcggtt ttctgtaatt ctgaaacgaa cgggctcttg gcctagggtg tttcaggagc 18120
gagctgagtt cggcctctc atccatcagg agccactttt ccatatttag ccacactttc 18180
tcctagagat actaacccgg ccatttattt acttactaca aataattacc ctaaagtatg 18240
atttaagatc gcagaggaga gacactgggt ggactgagcg agactgagga gagcagggca 18300
aacgttcttg gagggtttac tgcccgccaa ggacggagaa atagctctgg tataactgct 18360
acccagttct cccccttttct ctcccgggcg agccaaatct ttttcacgtt ttcaaccaca 18420
acgcagcgag ccaagcattt aacgcgctcc cccttctctg ccacaggcaa gccgggagag 18480
gtgggtctcg aggggctcca ccgggtgggt agaagagccg cggttgcttt aaagacaaga 18540
aaagaaggtc cagggctccc taggcccctt cgatctcagc gcctgcctct ctccacgcta 18600
accagggtac gccgacgacc ggagggccca cttcgcgcgg gtgcggggat cggggtggga 18660
gcaagcgttg tcgggttggc ggaggcacag aggcggggca gggagctgcg ggcctgcctc 18720
tggcctgagt accgtttccc tgcgtctcgg cctctcctga agggagctgg gccctgggga 18780
gcctctggcc aaggtcgctg cctacaggag gggctgcccg gcgctgtggc gtggggatcc 18840
agggtgggga cggccaggcg gttcccccac ccgctagcga gaacgcgggc ggggactctg 18900
ccgattcgat ccttgtgggc ccgtgggccc agaagtagca gtttggcggc tccagattta 18960
gtgattctgc agtaaaatca caggattagt ccctgattga gatgtctgtt cgtgagatat 19020
tacaaaattc attatcacag ctttctacta actcgatatg aagtaacaca gatgggattt 19080
tattagtcca gacttcaaat gtttacttat gataatttcg gaggaaattt gcatgtcatc 19140
atcatttcga taatcttttt tttttatatg tctgaactgg ctgcattatt agctggtagc 19200
cggagcactg cagatggtaa ctgcaaatag tttttattta tttatttttt ttaaagaatg 19260
aaatatacaa aagaaaaaga ttgcgttgct tggtgtaaag tcagtcaatt attacacatt 19320
cttcccccca cttccccgtg cttcagtgct gaagaccaaa caaagcaata caaaacaaat 19380
cttcaagaac tcatagagct ccactctaag gactgaaaag aaggtcaagg cgtgttcctt 19440
agctcactcc tacatgtcct tgtgacttgg agatttattt tgcagtcaaa atgagccttg 19500
agacttgcat ttttatgctt catttaatga ccaggcctac tagaagaact gagtctaaat 19560
aactggggaa gataattttt taaaaagaga cctccaattc ccgcctgctg atccttaaac 19620
ttgccctatc aagacaagtc ttctgtgaga aatttggctg ccagacttcg gaactggctt 19680

caatggctaa tctcacaaat tgagatggga gacttttcct gatgggaggt agttctcacc 19740
cccaaagttc atgttctagt tggaatgtat atgccaagga ctcttgtttt ggccaacttg 19800
ggttttatat tgtgagcaca caaaaagcac tacacggcta acggaggacg aggaaccatg 19860
gcaaagcagg caggcaagcc ctaagaaata aaacaatttg ctaaaaaata atttctgatg 19920
actaccgcaa gactgaaagt gcaggaaaaa tacagttcga ataatcccag atcctttcac 19980
atttcccccc ttttcataca ctttgttacc ccataacaaa atctttaatg gaaagtttaa 20040
aaataaacag cacaggaaca tgtgttttaa atgaactaaa ttgtgaaatt agccagtaaa 20100
ttaatttgta gtaagtaatt atttaaggaa attaaaatac tgctcagttc agttctgtat 20160
tttaccatgt gtatgcgttc tttacaacca attaatataa gtgctttagg aacatttgaa 20220
gacaaacacg cttaacttaa ggaacaaagc acctaaataa tttaagtgta attttgctga 20280
gttaaagtaa aacattccac aaatgaagtg gctatttaat tttttaggga aagtttggtt 20340
attgaaatgt tgtatgctca tgttacatca acaaaaatct tcaatttatt ttgcttatgt 20400
gctttgtttt cttgatatta ttggtatttg aattttagat ggatttctgc caaaatgata 20460
ttttgtgtga taaaagcatc tttagttttg attgatagac taaaacaaat gcaaggaaat 20520
ttctttaaat cagattaatt tttcataaaa atattttaga atgtatgaat tctgatattt 20580
acatttataa tggtaaaagt tttttccgtt tagtttagta agacaatact cacacaaaag 20640
agtaaaaaaa aatcacacca ccttatgata gtttgatttc taaattgctt aagaaagtaa 20700
agtggttaaa ctggaaaaga ggaacatatt tcggaggttt agaatcgaaa attttttttct 20760
taatctccag ctggaaaata attctctgca tccatttaaa gtgtatctcc tgaagtgcca 20820
gattggagtt gactggtgat caatttaaag gagttacaat ccaaagaaat ggtgagagct 20880
tggcatccag gcctggctcc caggtaattc gcttgggcct gagaggtcac taactgccag 20940
ttaagatgga atctttttct tttcttttt ttcccaatgg ataacaatgg gaaggggggct 21000
aatcttccag tagctgaaac tttgtaccca gccctttatc ttgagaatgc taatccttgg 21060
cccgaggatt tgttcctgca gtgttggcac cgagatttaa gggaagatac ctcgttttaa 21120
atgccagcca cggtctggct tccctctcga cttcagcacc ctgtagattg ttagtgtctg 21180
tggcggggga cgaaaggaac agggctttgc aaggtctgtt tgccgactgc gttaccttgg 21240
gcgaaactta gccccaaaag ccacaaatca cctacggtga agattctccg aagtggaaca 21300
aatttccaga ctcgcattat ctcacatccc tgcgggatag atggcctcca cttaccggct 21360
accgggagag agctgctgtc tccgcgtccc actgcttccc ggggcgattt ccagcgagcc 21420
gagcctccgg ctgcacggca agcgcccgaa agccgggcct gagaggactg cagggctcct 21480
gagggtgcca agttccgaag gagtccacgg gtgcactggg gcctccgaaa tctagccgcc 21540
actggcagtt tctttctgct cctctccagc tttctcgctc ggtctcgcac tctctctcct 21600
ctccctccct ctcatccctc tctcttccct ctgctcctac tccgtgtggg gagtgacgtg 21660
acgtcagcag agattccacc aaactccact gcacagtggc gcgcgggcgg ccggccgagc 21720
ccggctgcgc ggctggcgat ccaggagcga gcacagcgcc cgggcgagcg ccgggggggag 21780
cgagcagggg cgacgagaaa cgaggcaggg gagggaagca gatgccagcg ggccgaagag 21840
tcgggagccg gagccgggag agcgaaagga gaggggacct ggcggggcac ttaggagcca 21900
accgaggagc aggagcacgg actcccactg tggaaaggag gaccagaagg gaggatggga 21960
tggaagagaa gaaaaagcaa tctgcgccaa cccggcagcc ctaataaatc aaagggggag 22020
cgccagggca gcggggagac agaaacgtac ttttggggag caaatcagga cgggctggga 22080
ggaagcgaca gggaaagtgg cccaagagac ggaacaaagg acaatgttca tggggttgtt 22140
tgggacgagg cgtgtggagt gtgggtgtga gcgtgcgtgt gtgaccttct ttcaggcctg 22200
cagagttgag gaaagaggtc acagcaaaga gggactgcgg agggaggaaa gtgagagacc 22260
ggtagagggc gggagtggag gtgggcgcgg tggggatggg agaggatgag tgaagagaaa 22320
tctagaagaa tggagtgagc tagtgggaga gggtgggagg gccacagccg ggagcgaacg 22380
agctaggctt gtcagctggg gaaggccggg acgctgggcc cagcttagct gggacaccgc 22440
gcccgaggtc aaggcgggtg gaccaggcat gctgagagtg tcggcgcaca ggtgggcacg 22500
gccacgcact gacccagtgt tcacgaaggg tttgcactgg acaaggctca gacgctcata 22560
gagtctagaa tttcctctgc tgtacctaca ttcaacaagt tcaccctggg tcacggatat 22620
ctcatttttt aaaatgacga ggttaaggtt cctggcgagg atggtattaa attgcacggg 22680
atagaagtgg gggtggggga gagagtttcc ctcaagtcca catttgctcc tgcaaagcaa 22740
agagtatgtg aaattacagg gcatattctc actcgaaaag tgtgccttac ttctgaaccc 22800
tgatttctg atttcttgac ttgagcaaag atgtgtattt tggtagtgag cagaatattt 22860
tggctctgtc ctgcctctga gtggaaggac tataaatata attcgcctgg aggaccaggt 22920
gtgaaggctt ctgccaggca tatgggacaa tgttttttca atctcaaggg catcctgtta 22980
atgtatgttt ttggaaagtg ccggaacaca gccattgctc ctggattcgg attttcccac 23040
caatattaat tcctgcttga gagcaaaact caggcccgct attaaaaaga catctctttg 23100
gtccctaatt gagaataaag ttccctctaa aagttgtatt gctcttccta aatcaatata 23160
ccaatactcg caattttaga aatatatagt gactcgggag aatgtgcata aaatagatac 23220
gtttaaaaaa gcttggcgct taaaactaac cctagtcact atataggtgc tgggctttcc 23280
ctacttttgg gggctgtctg gaacatgtta tgtgttttct tgaattactc cgtgttttga 23340
attcatttga gttagcagta aaaacaggca aacaaacttg ctcaatttgt tttgagtgct 23400
aaatcccttc actttgaaat agctaacagt cgacagatgg actcatttta tggaaagggt 23460

tagcctcttc agccacgaag aaaactgatt agagatctac attttaagcc atttctaacc 23520
tccacgtaac atccgtgaaa actcaaactt tctctcttta cccagtggaa actcaaagca 23580
gtgttattta aggggagaga aatgaggggg aaaatgccca cgtgctgttt aattgtattt 23640
cctctctgac tctgagaatt tctatttctg gtttttgaaa tctcgccgag gcaagaaaat 23700
caaatttctt caacaagtcc cacaactgaa ctctagttac aggacaccgg aaagtgcagt 23760
ccgagaaaga catcttcacc tctgcccatc gacgattttt gcagcctccc cattcctctg 23820
agtaatgggc taataatcct ccctttttttt cttttcattt tgtagagatt aagaggcgct 23880
cgtagcagaa cggccttgcc ttcagctggt ggcgaggata ggcaatctca tggaaaagtt 23940
ggaagagaat gagaaaacca aagacagaaa gattcagaga tccgcggaga gacacaggga 24000
gagggaaggg agttgcgctg aaaagacgca aagatacgcg cgtgcaactc cctccccttt 24060
caggtttcag aggtttgcaa accagggctg agaggaaggg gctcgggaag ctcacgttcc 24120
tctcgccccc cttctgtctg gagtctcgcc cgccagaggc tggttaaccc cagtcccggc 24180
cgccgcagac actgcgctga gcttttgggt cctcgccttg cccagcgcca gtgcagctga 24240
agtgagcagc tggtgggaaa tgcaaatggc tcctggagaa atagaagata cagaatgatt 24300
ctcattccct cctcgagtgt gtggaaggag ctggacatac gtttcacgct cctaatcctt 24360
cttttacatt tttagtcata ctcctattaa acaactaatt aatgcccaga atcaccaggg 24420
aatacattag gcatgcaatc gtagaagcag ggtgctgggg ggctacaaac caccgagctg 24480
atttaagacg tggatttcag gtttttccct tgtcaaagca gtaaaggaag agcgggcctt 24540
ggcgactgta tttagattcc gattacttca aattagaagg gggtggaggg agcgtccaag 24600
caaagcaagc aattctctgc cctgcagatg taaacaagat tgtagcatca aaggtactag 24660
ctcctttagg gctagatcgc ctggactggg agcctgggga aggggagaca ctaaccttac 24720
gtatctgtga atttcaagga tgttacattt ttacataaac aactccagtg cggattctct 24780
ggaatggggg gagtaatacc cccattctag aatattaaaa cattttcccc ccaaagcgta 24840
tatccttttt atttcctcaa aattttgaac tatgtctaaa gataatagtc ttccagtaaa 24900
ctggagcatt ggaccatttc cttcacccctt tcctaccgac attttgatga tctgatttta 24960
atgtgtgggg ggcacaggga attaaataca gcccataaaa ctaagcttag atgaaacagt 25020
gctggctaag tgggttcaga taattttttaa tgagaatctc aattacactc ctccccccaa 25080
tatgttgaga caagtgacag aaccgttaga atggtaatca aattggaaag ctcagggaga 25140
acaacaattt cgtgatcaaa ttggggcaaa accgtggaca aatgtggggt gacctccgcc 25200
aactccctgt cacccaagag tcaggatttg ggaaaggtac agtattattt cagagcccgc 25260
tgtgacgggc tgtgtgctac catttacttt cttcactctg gattatgatc tcaaccctgg 25320
caagcaattt ccccagctcc ttatctgaca acaagcgagt atgtaaatac caatggctag 25380
cgatgtttaa ctgcctcaaa cattattgat ttgttggctg ttctaaattg tcttcctagt 25440
ccaggtcttg tttccgaatt gtctattcta gaggtttgat ccatgcctcc gatgctacaa 25500
tacaataatt gtttttttaa aaaaggcatt taagatgaac caattgattt gcatataaat 25560
taaaattact atgcgttgcc gattccggtg ttttataatc atttcgaaat tagtacttaa 25620
ccacttgagc taaaagaata tataaatgcc tgtattgact cactaatgaa ttacccaatt 25680
aaaacgtccg ggcaatgctg ggcgctggaa agattgttaa atcaagacat attacaggag 25740
ggatatgaag attagaaagg taacagacca atatctcgca ctcaaaacgg agtttccggt 25800
gattcccagc tttaattttg gagcaggggt ctttctcctc tgctgttaaa aagattttgt 25860
gcttgtttgt gagtgagtgc attcaagtgg aaggaacgct cccacggcta cggtggctca 25920
ggccctttgc tcggaccggg accttacagt tctaacccag gagcgttaaa ctctggaaga 25980
ctccgggcca gccctggagg tgcgtggccc cgcaagtcgc caggccaagc ttgctttttc 26040
tgtctgcccc tccggcaggc tgggcgcgct atggcagtga gctttccgcg caaacggaga 26100
gctggaacca aagctgacat ttaatagata tgctaactga gcacttacct tcgtcctgag 26160
aataggaata aaaggtagct cttcttaaga gaggcggtgc aaaggcacgc tataggagtt 26220
cagaaaaggc tggcggcggg aaatctgtag cctgggggct agtcaacatc ccctttcatt 26280
tcaagcactt attgatttgc tgttgtcatc tttggcgacg cagaaggaca cttgaaagaa 26340
tttctgatgg ggctctgatc tgagaaagga ggtgacctgc ccaggctccc accaaattct 26400
taattaccac atcaactgct tttttttatc ccccacccga cctccttcct tttgcttatt 26460
cttaactttt taattattca gaaactccct tacctctcag tggcttccct tctgcagcag 26520
ttttctattc gaacctttttc cccgcctttc cgtggtaggg cctgtatatt gatccctctg 26580
acctttggca catctgggcc ctctgaaatc tctcaatctt ttcagatttg aggatggcag 26640
gctccaccct ctccactgtg tgcacacact cagagatatg aaaacttaca cagactgcct 26700
tcaaacccag ggtatctaac agatgttccc tttccagttc gtctcttgat ctgaaatgcc 26760
tgcctgattc caacttggat accactcttt cttgcccttc ctttttcaaa gcagtttgga 26820
catgtgtgca agtgagccca gaacagctcc acccatattc tttaccaaac tgtaaataaa 26880
agaagaacta atgaagtaga ttggcatata gattgcatca agagcccgaa tccccagttt 26940
ctggattccc cattcaactc tggctgtcat ctacattgac agagtcattc taagcagagg 27000
cccagagaaa cctgcattgt gggacaacag gtaaagccac agtaaaaagt ggaataattt 27060
taaagtcatt ttattagaat gtaaattgta tttctgggtt ttgttcgtaa ccacctagtt 27120
ttaatatata cagagttaga caggaaaaaa taggtcaaca cagttattgg tactagagaa 27180
gacaaattcc atgggctcct cagtgaaaag aagatcccca aagtctataa tttttgatca 27240

tttaatttca tttataattg tgggaatgaa taagacacca actgctttat gtatttcatt 27300
tacatcaacc aatttgtgtt tccatcaaaa gcagttatac agaatttctt ttaacttctg 27360
gtagcaagtt cagaaaatga agcttacagc caccctgaac tggatacatc tcttgagctg 27420
accatttctg taagtgcagg aatataatat tgttcttcta tggtcttttt gcactctttt 27480
agggtttgca agtccttatc aggtctgaca tcactgtttg ggtctacatt cattacaagc 27540
aaatttgatt actatgctga ttttaaaaca gcctatttgg ccagcataat cttagttttc 27600
aaattataaa aaccttttaa tatacgaagt tcccagtttt tacctcctct agctccttgc 27660
tcattcaaaa cttctatttt aattggtgta agtaataata atttgtatta ctatttgtac 27720
tccttttact tttttggaga ttgggctgga ttccagagag aacaccagca tcaccaccac 27780
cacaaacaac aaaatctaaa agtaaagccc ttatttgcat gataattggt acttggaatg 27840
cttctgactt actcaatgcc accttataaa ggtaccttgt aaactttctt ggaatttcta 27900
gcaagagctt gtagtaactg gaacaactct ctgggaagat atcctctttg atgggctttc 27960
agtttctgga ggaatagatt gagagcaatt agggagggag gggacattgg aaattggcag 28020
ctacgtcagc tgaaacaagc ctgggttcag taaggtgact gatgttgtgg ttgattccct 28080
accccgagtt tctctttaat tggggcactg actcttccca ctttgggatc ccaaggcact 28140
cggtgtgtat gcagattcct cccttgtggt cttcaccatg tggtttcgta gcaggtctct 28200
ggttcaatga tattttatag tcatagccct catattcatt atcatgatct caatgtttag 28260
gcttttagtg tatttatatt aaacctgctt tattagtaag ctggagcaca caggagagat 28320
gggggcaagt aaggactcag cagagctcaa attcagacat gtttaaatgg ctttgactgt 28380
gtaaagtgtg gcaatgcttc ctgctgccct agctttccac tctaagcttc acatgtcctc 28440
tggctaatga agtgtgatat aggccacatg ctaggaataa tagtatttgt tgagaacaaa 28500
gtgaactcag gaaacctggt atacacaaaa tgcatc 28536

<210> 150 <211> 8252
<212> DNA
<213> Homo Sapiens

<400> 150

```
gccatcgtgg ctgggccata ggctcactgc tctgtggtag atggaagata tgggccttgg     60
taatatactg aaatgccaca ggaaaccaaa ccagaagtga tttaacccac aagcacactt    120
aggtttattt aacatgatgt ctcaaggtgg gtggggggct caaatcagta gctcagggtt    180
attaaggctc taggtcttgg gccttacctc attattacat aatggctgcc tgcaacacca    240
catcacaccc accttccaca acacccaaag gcaggctgca agggctggcc taggaagcca    300
gagggcgctc ttcattcatc aagggagaag cctgccccac cttcaaggag atgcccccta    360
tgtctcatgg gccacataga attgcacggc cacttctggc taaaagaggg gctgggaaag    420
tgaattcttt ctccccccttc cttcgtagag tgcaagttag gtaagaaaga acggatggtg   480
gacagccagg aaacagtcag ctacgagatg ttgtgtggaa agcccacctt tctgacactg    540
gagaactgtg ggaagaacgg acaggccaca caccagctgg gctctgagaa cccccagctt    600
ctgctccaca gtgagccagc aggaatccaa agggcatggg cagaccccag gggctccttc    660
accccaccca ggggtagaga gggcaagacg gagtagagcg aggtaagaga ctgagcaatg    720
cgttcccaaa gactgtgact gacagaagct gtgcaaatta ttatgacaaa ctgaatttta    780
agaggaattg gactaaataa agttagctca tttttacctg taattagcag acagagagtg    840
taagatcaga cataccatag atggaaaaga aaagctaaaa cttccttgca catctgagag    900
gtaagaggag gtgtctgtgg cccccctgac acatacgtga catatgtatt attacggctc    960
atgtgtgtgc tgctgtgtgc agggggcagg gggcttcccg ttcctgatgg ttcttcttgg   1020
tgggtgtctc ccggcctctc aggcctcttt atctgctgaa cgtggtagcc aagggtttgg   1080
gaatgatttg ccagactggt taatgtggaa agagctggcc ccgctcagtt ctgcacctcc   1140
ctcttcctcc ctggaaccgt ctggctgcag agagcaggag ctttccagag ctcccggggg   1200
atcccatctc cagtataact gtgcaacgtg tgcccagcac aagagcacca gatggaggga   1260
caggtgggag gaggaggaac ccagggtgca tttagcttgg ggcttgctga gctgaggtcc   1320
ccagagcaag gacgactagg ttggggggac accctctccc aatccttgcc aagctacaga   1380
ggggccagtg ggaataaagt gacattttgg cttctgtatc cctcctctgc tgttctattt   1440
tctagttcgt tcagatctga ggttggggag gggttttcct ggaggggtca gtccatgcca   1500
ccctctgcaa tttttattac acatgctttc agacattctg ctacaggttt catctcctac   1560
gccaatttct atttgggaaa gcaaataaac ggaaagctaa cttgtgtcac ttggtcgctg   1620
gggcatccgc agtgagtagt taagaaatgc caggggagtc ggtgtccatt ttcatgcctg   1680
gacaagagtg cgtcttggac ttgcgtcctg ctgcacaccc caccgtcccg ctgcacaccc   1740
caccgtccta ctgcacaccc cactgtccta ctgtacaccc caccgtccta ctgcacaccc   1800
cactgtccta ctgcacaccc cactgtccta ctgtacaccc caccatccct gctttctgca   1860
ctcagctgcc cacttgtatc ccagctcagg aagcccagaa gatgcagaac ctctgcgaga   1920
gcccagggtg aaacgctgcg cttcactttc aaagaaagga aatcattca tattctttaa     1980
```

```
aagaatgaac agcacagatt aatatcgatc tcttttaatt tttaggccaa ttttgagtag 2040
tcaaagtcag agcagtcaat ctgtgttgtg agccgaggca cagctgcaga agcgtgtctg 2100
aggtgtccgg tggaggtggc agccgagctc tgggactaat caccgtgctg ggacggcac 2160
cgcgtcagga tgcaggcaga tccctgcaga agtgtctaaa attcacactc ctctacaggg 2220
gtgaggggga gggagaaaga gatgctttag tgaggataaa cattttcttt cacatttaa 2280
caattacaga gtttttactt taaagtatcc ataggcacat tctttagaaa acatgaactg 2340
tcagccgggc acggtggctc acgcctgtaa tcccagctct ctgggaggct gaggcgggca 2400
gatcacctga ggtcaagagt tcaagaccgg tctggccaac atggtgaaac cccgtctata 2460
ctaaaaatac aaaaaaaatt tagctgggcg tagtggcaca cacctgtaat ctcagctact 2520
aggaagctga ggttgaactc aggaggcaga gattgcagtg agccaagatc gcaccattgc 2580
actccagctc aggggcaacg gagcgagact ccatttcaaa aaaaaaaaaa aaaaaagaat 2640
acatgaaatg tcttcagatt tcgtcatgcc ttccccctct atcctaggca agctagaaag 2700
cgttaccaac agtggctctt cccaggcttt tggttagaga tgtgaagaga agccgggggg 2760
aaatcaggtt tcttcccaag tccctttagcc ctgcctttct attcctggac ctgaatgcag 2820
cctgactcag gctaccccat tgcaccacca ctggcggccg tgactctgtg taaaggcata 2880
gctggtgatg ctgatcagag cctctgtagt cttaaatgac ttttctaact aattctaaat 2940
cttcagaacc catcgtataa aaaggccata ccttctggag ggacgtcgat ggtattagga 3000
tagaagcacc aggggacccc acgaacggtg tcgtcgaaac agcagccctt atttgcacac 3060
tgggagggcg tgacaccagg aaaaccacaa ttctgtcttt cacgggggc cactgtacac 3120
gtctctgaaa gtgcacaggt aagaagcaaa gtaagttgtg ggctgaattc cttgatgtta 3180
tcatgcacac acccatccag cttccttctc caatgacatc agcaactgtc cagtgaggcg 3240
gatataaaac cctcaggaca tgagagggag acgtggtcct cacatcctga tgtgcaaaca 3300
ttacgctcag ggaaaatgca aggtgcccca ggtttgtgga ctttgcatct ttctaggtaa 3360
cttatttatt cactttttaa tttcaacaaa tgattattaa attttactca atacataaat 3420
atttactgag caccatttgt gtgcatgaga agtgggagct agcatggcaa aagccaggca 3480
ctgtgccagg tgagagagac ccagaaatta aaaccagaga agtcattaat aagagtctaa 3540
atatctgggc ccaggctcat gcctgtaatc ccagcacttt gggaggctga aggaggtgaa 3600
tcacttgagg tcaggagttc aagaccagcc tgaccaaaat ggtgaagccc catctctact 3660
aaaaatacaa aaaattaggc gggcattgtg gcacacgtct gtaatcccag ctacttggga 3720
ggctgaggca ggagaatcac ttgaacccag gaggcagagg ttgtagtgag ccaagatcgc 3780
accattgcac tccagcctga gtgacagagc aagactccat ctcaaaaaaa aaaaaaaaag 3840
agtctaagga tctgatggag gagaaaggca agaacatgtg cgagacaacg caaggccatc 3900
gtcccagggt gcccagggta accacggggg tagggcactc cccggagagg ccaatgacaa 3960
gcaggttgaa caaagcaggg gggctccctg caggaggagg ttcaccaggt gaagatggag 4020
ccgcatgggc aaaggccatt ccagagaccc aggtgtgttc aggaggtgga aacccattgc 4080
aggtaaggtg agaggaccgg gtggggtggt ctaggaggag ccgacagagg gtacaagctg 4140
tgaaacagct tgaagcaggg cagtgaggaa agggatctag aggaggaaga cacgtggaca 4200
gatggggctg gctgggggct gccgcaggat cttatgcaag aggttctaac accagagctt 4260
caggctctga gctccgcgga atcaaaggtc tcagaaagca acctattagg atctggtggt 4320
tgacagcctg cagcaggggg tgaaagagga gcccagagca ccctccggtc cctgtccctg 4380
ccttggggca taggagggga ggaactcagt ctggccacac tggctcaggt gagggcgccc 4440
caggggaggc cgagaggggc tgttctcttg tctgctttgc tatcagggac tgcctcgaga 4500
tgtctttgga gaaagtgttc ctggcttgct gggaaggatc cgtgttcagc tcccgctgcc 4560
cagcagcttc catgggaacc ttgccctcct ggggtctgga tccatcgcga cgtgaaggtg 4620
atcatcgccc acccccggga cactgtgggg gtcaagagag gccccgtggt gagggaggat 4680
catcatcctg gggtcgggg gggtttcctc ctcagggagg aagatttcca gccccggtgc 4740
tctgcctccg gcagactctg tcttgcccac ccgctttgtc tttgtcgcat gatggaaata 4800
aatggaaatg gtttccacac acgaatgcac taaactgtaa tcacaatttt ctagatttca 4860
gttgtaacag gatcatggac ctgagtgacc cgcaaagacg ctctgagcct tgagccagag 4920
ggtgtgtggg gtggggaggg gagtttgcca cggcctctgt gatcccacag cacaggggc 4980
agagctgggg gctggggtgg gggcaagggc gcaggcagat gggcctgggg gtggccagca 5040
cggggaccca cccagttcgt tctgtacacc gaggccactt cctctctcgg gttccaaagc 5100
tccccgctc cctgccatgg gcttggggt cttcccactg cagcccaatg ctggctgctc 5160
ttctacgctg cccaagccca cccctcaggc ccggtacccc acagcagaga tcaagaggtg 5220
gctggagggc agtgggggca cggacagcac cactgggcgc tccctcttca ggcctctctg 5280
gaaacccttg ctttggaaac gtagaaagcc ctttctcctg cccccacctt gaaactgtac 5340
ctaataacgg caaataagtt attcagctcc ccacagctgg caaagtgcaa gtcgcagatg 5400
ctgccacaca gcatttcctg acagccctag ggcagaccgt tgatccattc tgcaggtaaa 5460
ggagttgaga tgcaaacact tcccaaggaa gcaagaatgg cctcctcctc cactccctag 5520
aaggacccag ggcaaccgat ccacttgaca acaccaggga atatgggttg ccagccacgc 5580
actcatgaga gaggtggctt tgactcccag agcatggatc ccaggggagc ccaggaacct 5640
gtaggagagg gtctcccagt tggccaccac tgggacgggc attcgggcct gtgggagagg 5700
gtctcccagg tggccatcac tgggaccggc atttgggcct atctggatgg gccttgggag 5760
```

ggtctgcccc tgggggagat gttggcatga acagtcaaaa gcactattct gagaccccac 5820
gccaacactt cctctttgaa acatgacact tgggaggatt gtatagtctt cctaacacag 5880
gaaaacagcc ccgactgaag gcagcccccct cctcgtcgca cttctcgaag gtctccgggg 5940
gccctgccac cctgagttac tctccacctg cttttgccga tcctgtaaat tggatatact 6000
tttaagggcc tagaacagca cctggcacaa aacaggtgct caaaaatatg tatgtaaaac 6060
agtggctcct ggcggaggct gccagagctg gctgtggccc cacagagcag gaagaagcac 6120
gccttacctg tctgggcctc ggccagggtg ccgagggcca gcatggacac caggaccagg 6180
gcgcagatca ccttgttctc catggtggcc attgcctcct ctctgctcca aaggcgaccc 6240
cgagtcaggg atgagaggcc gcccgagccc cggattttat agggcaggct ctgtttgctt 6300
aaagagcgtt agataacatt tgcctaagga ggcccgggga tcctctgaga caataatctc 6360
cactgatttt tatcaaaggt gtttcctaga catggtcaag ctacatggaa ggatttgctg 6420
atagacagag acgacatgtg gtgaggtcat cttggctgag ggatctgaga ttcagaaagt 6480
ccctctttcc catgggagtc tcctccaacc tgaccttaat ccaggtccta ctcatatctg 6540
agaggccctc ccgccagggt aaatactgta ctcactgcag aagtgattca tagtgagaga 6600
tggccggaaa aaggcttggc cgtgacaaca gtggctcacg ggtggccac cgtgaccttg 6660
caggggggaag ggaaggagct catgaagccc attccgtcta ggcctaagct aattctttta 6720
cagtggaatt gttttaataa tgaaattgta ggcctggcgc agtggctcac gcctgtaatc 6780
ccaacacttt gggaggccaa agcaggcgga tcacttaaag tcaggagttt gagactagcc 6840
tggccaacat ggcgaaaccc cgtctctaca aaaaaaaaaa atacaaaaat tagccaggca 6900
tggtggcggg tgcctgtaat cctagctact ctggaggtta agtcaggaga atcacttgaa 6960
cctgggaggc ggaggttgca gtgagtcgag atcaccctac tgcactccag cctgagcgac 7020
agattaagtc tccttctggg aggggctggg ggcaggaggg agaaaaaaat agtatatacg 7080
agctgccatc aaaaactgac aaagtgaacg tggttcactc ccctgtgtct atggggctcc 7140
ctccctcccc ctcccacacc ttcccagttg gtcccctgga ctcctgtatt ttagaggaca 7200
gcctcctgct ctcctgtacc atgcctcact cccctcccct gctcggagct ttctctttca 7260
tagctcttat tacctcctgg tggataaaac aagtgactga cacattatgt tggttattta 7320
ttcattgcac tcctccctgc agcagacggg agggcgcatg ggagcagaag tcctcatctc 7380
ctagtgcctt gaggtggccc cagaccctag aacagtggct ttgattggca ttcgagaaac 7440
agctgttgaa gagctgcatg aagaaatgga caacaggaga aatcttttct tcttcagtga 7500
gaaattaggc cctgaggaaa atgcatgcta gcctaacaaa ggtactatct tttcttgggt 7560
gacctagtca gtacatttct gcagcccctc tgggagggga gcctgggcca gaatgggggc 7620
ggccatgttc ctcttcgctc catcccagga cccagcccct cctccagggc tgccactcct 7680
gcaccggggt ctccactcct gggccagggt ctccaggagg ggcctgaccg caggacccag 7740
ggaggggccg aggcctggtg tcctctgtgg tcagcttaag tttattgaaa acaggacggg 7800
ggcaccaggc ccagggtggt gctcatagtg acggtggttt tcgctgctct aatttctcgg 7860
atcaaaatgg gggctcttgg aaatgggatg taaattgcac aatcatttgg agaaaaggtg 7920
ggcagtgttc ggtgaggttt aagccataca gattttacag ctcagcaaat tcttctgttt 7980
ccaggcctgg tctcttaatc ccagaaacat gttcatactt gggcagaaga aaacacaggc 8040
aaagatattc tttttagcat tgtttgtaac agctggaaac agggaaagaa ggaaggaagg 8100
aaaggaggga aagaaaagca aaataaatat tcattaacca gggaaaggga aaatgaactt 8160
aaaatacaat gcagtcgtcc cttagtaccc ctgggggact ggtttcagga ccccccaccc 8220
agggatgcca aaatccacaa atgctcaagt cc 8252

<210> 151
<211> 28536
<212> DNA
<213> Artificial Sequence

<220>
<223> chemically treated genomic DNA (Homo sapiens)

<400> 151

```
gttttgggag ttaggttttt ttcgttggat gggaaaagga ggtgcggaaa ggggtaggcg     60
gtttgcgggt tagcggggat gagagttgtg gggagaacgg aggagagagt aaaattattt    120
tttttggaag tttaagggaa gggagttgag gtgagagaaa tggtttttttt taggttttaa   180
gttttcgttt ttagtttgta ttatttaagt cggggaagtg ataatttttt ttaaatttcg    240
atttttattt tttgtttttt gtagattgat tgatgttttc gcgttgtttt agtttagtaa    300
ttaaattagg tcgaggtata ttttttttat ttagagtttt taaattattt attaatttgt    360
aggagattta aatttttcgg gtaggtttcg ttagcgtttt tagtttttat ttttaaattt    420
tttttttag tttttttttt ttttttcgtt ttttggtttt tttcgttttt tattaggttg     480
agaggcggag aggtcgggat cgttgggttt tgcggtttta gaagttaaag ttcgttcgtg    540
gggatcgttt ttttttttat tgtttttaag tgaataatat ggtgggaggt gattttcgtt    600
```

```
taaagggttt ttatttgttt tttattgttg cgtgattttt tcgtaagtcg gaggtagagt    660
tttaaaaaga aagaaagtgt gcgttgagta ttgattttaa atcggggata ttatttgcga    720
tttttttaag tggtagtagt agtttttttgt agaataaaga tttagtgttc gagttttaaa    780
agtttttagg taaagtttta tttagatgaa agtaattaag gcgaaaaaat aatgatattg    840
tcggttttag aaaatcggcg gttatttttt gttttaagtt ttttagcgtg gtgtttgttt    900
tcgtttattt tggttgtgcg gggggttgat aagtataata aaagatttta gtatattttt    960
tttttttattt ttataacgat ttttttagcg ttatgaggat gaatttttttg ggattaagtg   1020
gtatttgttt ttatttgtta tgaaattaaa ttttatattt ttttaaagtt gaaaatcgcg   1080
gataaagaag gatcgggtgt ttaaagttta tttaaatatt tatatcgaat ttattttttg   1140
tttagatgtt agaggatggt agggagggtt agggttgtaa tttattttga tttgttttat   1200
tgttttgtag tttgtaaatt ataattttgt ataattttag gaataagtag gtttagaatt   1260
aatgggttaa tattatttaa aataaaatat cgggagtatg atttttgttt taattatata   1320
ttgttcgata agatttagga aattttattt taattttta attttgagt tttttgagaa   1380
attgaagggt tgtagttatt agaaattatt tttgtttttt ttaatgtttt taaaggattt   1440
ggaaatagta atgtaatata atatgaagga ttttttttatt taagtttgaa gataaacgtg   1500
gaagtttaaa tattttgaat tggagatgtt tttttgtaaa tttattatag atgtattttt   1560
tttgagagaa atgtatataa attatatatg tatatatgta taaatatttt tttagaataa   1620
tttgttagag gtgtaggttt tttcgtaaag gagtaaattt gagagttatt ttaagttgat   1680
ggatttgtta aattttttttt tttttttttt ttttttatat tatttgttag ttataatgga   1740
attttttagg tttaagttaa agaaaaattg gagagataaa attagatttt gtagtttttt   1800
tttttttcgg gaatgttttt tttttttttt ttagtttttg atgaatggtt attatttatt   1860
tttattaaat ttaaataagg attgttgttt tgtatgttta attaggtagg tagagggaat   1920
tggtttgttt aggaagtagt gattgagatg ttttggttaa gttagtgata gaggaggggga   1980
gaaagaattt agattaattt gtatgtagta tattttattt ttatgaaata aaatatattt   2040
gttttatatt tgttgaaaag taaaataata atattgtacg aaatgttata tatagggtag   2100
gttgtatata gtagttttag aaatattatt gtatttatta gagaaattat tttaaaattg   2160
atatttatat atttttttata ataataataa tatgttagaa atatatagtg tggtatttag   2220
tatatatatt tttttgttcg taagcgaaaa atttaatcg tttttgtata atatgtttta   2280
ttttaaagtt taatttttttaa aaatattgtt gtgatattat taataattgt ttttataaaa   2340
ttaatttgat atttcgatat atatatattt ttttagttat ttaaatgtta ataatgttaa   2400
atttaaaaaa taataagttt atagtaatgt taaaatgtta tatttagtta aatatttgtt   2460
tgtgtatgtg tttttgtaat tgttagaaat atttgtagtg aaagatgtta gatattgagg   2520
atattttttt gaaattaaag gagttttttt tttgatttag tggttttttt tttttttatat   2580
agtttttttt tttttttttt ttttttagtgt ttacgatttt ttagtataat ttttagtttt   2640
ttaagggcgg agttgtttta ttcggtaagg ttttaggatt tcggcgttgt gggtgcggtt   2700
tatacgggtc ggtttattgt atattggtaa gtatttaggt tggaggtcgg gttttgtacg   2760
ttggcgtagt cgaagttgga gtgttgtttt gtttttagtt ttaggttggt taggttcgag   2820
ttatacgtgt ttttataaat atacggagga gtcggcggcg cgtaaggata ggtaggcgtc   2880
ggtatcgcgg aatttagcga cgggttattt aggttgttta agttatttag gttgttgaga   2940
ttggagttcg ggacgtttgt tattgttgag ggtattatgt tggacgatat gtttatggac   3000
gagatagagt tgggtgggga aaatatgttt tgtgatgata gggggttgac gtttatagag   3060
ttgaagaagg ggaagttttt ggtggatagg gaggcggatg taaggttttt ggcggtttag   3120
ttgttgtagg aatagtttgg gtatatgtcg tcgtagggtt gtatgagttt attgaattgc   3180
ggttcgaagt tatttttgta tagttcggtt tgttggttgc gttttttttt tttttatttg   3240
gttcgacgat ttttgaatta aatttggggg cggttggggt aagggagtaa atagatgtta   3300
tagtgtagat tattaaaatt tttatcggag gttaattttc gttttttttc gatatatacg   3360
ttagcgtatt tatatatttt ggtttcgttt tattgtatcg ttttgtatat taagatatta   3420
gggttagttt ttagttattg gttcgggttt ttattaagcg taggagattt ggtttgtttt   3480
ggtttgcgag ttgggattcg gagttacgtt ataaatttta gtcgaacgta tggagatttg   3540
cggacggttt gattatttag ttaggcgttt ttttaggttt aaaaatattt aatgtaaaat   3600
aaacgcgggg tagtaggttt ttttaatttt tttcggggta ttttgtaaat ttgtttttat   3660
tttaaagtta tagatttacg gatgaggaga aggggttgga agggtattag aggatcgttt   3720
ttttttttac gtaatttttt tttttttttt ttgattttta ttgtcgtttt ttattttttg   3780
gtacgtgttt ttttaatagg gattaggtcg ttaatatttt ttttcgttta gtaaaataat   3840
taaataaaga gtaaaagatt atttttttcgt tagttcgtta attttaggag tttggtatat   3900
taaatttcgg gaattcggaa agggtagttt tggagatttt ttttttttttc gttttgtttt   3960
tttttttattt taagtttatt ataggtttgt tcgcgcgtta ggtttagtcg ggtcgtttgg   4020
ttttgtaggc ggttatttag gtcggtcggt ttttattcgt gttcggtggt ttagtcgtaa   4080
tttcgatttt aatttatatc gggttttttt gtcgttttag acggcggttt ttgtgtattg   4140
gagagaggtt tggtttgaga tattcgagtt gatattagtg atgttttata ttatatattt   4200
tcgtcgggtt tagtcgtgta attcgttttt tttttttttt tttttatttt tgattttttt   4260
tttattttt tttttttttg tattcgattg ttataaaaag tacgttttat ttttatttgg   4320
ttcgataagt agtcgttttg gaaggagagg tagttgtaag gagagtttag cgtcgcggtt   4380
```

ataaagtatt agggtggagt tgcggaatag cgggcggggt gggagggcgt tttcgaagga 4440
ttttagaaaa tttatagatt ttgtttttaa ttatttgtta tttttatttt aggttattta 4500
aattttgttt aggcgagaag agtacgtgag aggttcgttt ttttgatgtg taagagagtt 4560
aatgaaagat tgattttgtt taaaattacg tcgtttagga tttagttttg gttttggata 4620
gttaaattaa aattattttt aatttttttt cggtttttta tttattagta tagttttatg 4680
ttttgtataa atgttattta gagagtgttt ttattttttt tgatttggga gagtattttg 4740
gtttttattt tttttatcgt tgtttttttt tttttgtttg ttttgtttta atcgggggtt 4800
ttattttttt tatttagagt atttaatttt tttttttaa tagtaaagtt tttggatgtc 4860
gtttgatttg tttgattttg tttttgttt ttagaatttt aataaatttg gaattttta 4920
tcgattagta taaattagga cgttgttatt gggttattta tttgagttta tttttgttaa 4980
tttataaagt atagatttgt tataaagtta aggtaagttt tttttataaa attatgatta 5040
taatttagaa gagggggtgt gagttttaat ttttagagtt taatttttga gagaagataa 5100
ataaattaag tagaaaagtt tttttttttt tttttttttt ttttttaaga ggattagtag 5160
ttgtgtatta aaattttgtt ttcggagatt ataaaattag gaaatagggt gtgtgggaga 5220
gatttgaatg gtcgaaataa tcgtaaagaa ggtgtaagaa gcgcgagttt aggagggaaa 5280
aagttgggtt agggtcggga taaaggtttt ttagggaggg ttaatttttt cgtgtttttg 5340
gcgggttttt tttgttaaag gtttataggt tggagtttgt tcgcggtttt tggtttggta 5400
gggattttat tagttttgtt ttggtaattg taagttagga atataatgtt ttgtgtaggg 5460
gattgtttat gtagtttagt tcgtgagatc gcgggatggc ggggtagtga gtcggtgtcg 5520
ttttgggagt ttgagttagg gcggtagttt tgtcggtttc ggagagggaa ttgtaatttc 5580
gtaattaggt cgtcgcgagg tttttttgttt ttgtaaagtt gcgttttatc ggcgtttttt 5640
taggcggcgt tgttttttat attttttttt ggtttatttg gtttgtattt ttataatatt 5700
ttttttttat ttttttttaga tttcgtgttg gtttttattc ggattcgggt tttcgtaagg 5760
ttggtttata tagcgatttt ttcgcgtgtg gatatgttcg ggtagcggtt tttttggaaa 5820
gtggtttta gtttttggag ttgttggttg gtaaagtgag ttcgttgtcg tttttgtcgt 5880
ttttttttag acgggttttc ggcgtttacg tttttatttt tttttgttg gtttttattt 5940
ttttttgaaa acgaaatata tatatttttt cgttagtatg tttatttgta acgcggacgt 6000
taattggatc ggcggtagaa gtcgtggaag agttgggttg tttggcgtcg gaggagggtg 6060
cgcgcggcgg tttcgggtcg cgaggagcgt tgcgtttgtg gggtgtgtag gcgtaagtgt 6120
gggtgttcgc gttttatttt tttttttttt ttagcgtcgt acgttttatt tatatgttta 6180
ttttattgta gcggtatatt tatttttata gtttgtgttt ttaagtatat ttatatattt 6240
ttgcgtagat atattaaatt ttttgggacg cgtatacgcg cgtggtttat agattttttt 6300
tttttttcgta gaaagtttag atttttatgc ggtttgggaa ggttaggaaa agatgtgggg 6360
attcggttgg gtatcgaagt tcgtcggttt tttttaaaa aaaaaaaaaa aatgtttttt 6420
cgcgaagggt atttttgagt ggttttaggt aatttttaa cgagtggagt ttttcgggag 6480
ttgaaagtcg agaggaaaat agggatagag gtcggcggtt tttgaaggtt ttcgaattaa 6540
gatgttggga tttttgtgat ttaggaaata gaagggaggt tagggtacga atagagaggg 6600
cggtagaatt gttcgcgttt ttagcgtttt aggagtcggg tcggtcgagg gagaattaaa 6660
gggatgcggg gtagttaaaa tttcggtttt cggaagtttt gcggggagtt aggcgaacga 6720
ttattttat tacgtttttt ttcggagggg ttgatttttt tggggcgaga gggagcgggt 6780
ggcgtagagt agttgagcgg gaatgtttgt agggcggcgc ggcgttttat ttgcggtttt 6840
cgggttggag gtgtcggaga tggtgtgtat ttttagtttg tgtttggagg agtttagcga 6900
tcggggttga tcgggagtta gaatcgaagt tatggttaac ggttggggat ggtgatagga 6960
agatgaggag acggtcgata gtttggtttt cgttgttcgg tgttttaagt gaagcgggtt 7020
ttttatgtag tttatggacg agggagcgcg acgttttatt agtttttggt tattgtttcg 7080
tcgagttttc gtagtcgtcg ttgttcgttt cgggtcgcgt tttaggcgcg gagtttttc 7140
gttgcgggga gagttagggg acgtaatttt cgtcgagttt ttaagttaag ttgttttcgt 7200
tttttttcgga aggtttaagc gaaaaagttc ggagacggaa agttagcggg taaacgaaga 7260
tatgggatgt gggtagaagg gtattattta gagcgttttt agggagtagg tttttaagtt 7320
ttaaagcgaa ataagagtgg gtaaagattt tttttttttt tttttttttt ttttaagaat 7380
tttttttaata aggaaagtta acgtcgatcg cgttttgttc gttttttttt tacgcggtag 7440
ttttgataga gaagtgttaa gagtgatagg gataggtagg tgatattaga tttttttgcgg 7500
cggtagtagt cgttgtagtt acgacgcggt tttttgagcg tatttttcgt aacgcgtata 7560
cgtatatttt tcgggcggtc gaataggagt cgggttttgt cgtagtttag ttttaggtat 7620
ttaggcgagc gacggattag atttgcggtt tcgcgttttt ttgttggttt aatattttaa 7680
aattagaggc gggtttttttg gtgtcgagac gttatttcgt cgcggttttt tttagttttt 7740
ttcgttttcg ttttttttta gatttttttt cgggtgcgat tgacgtggtt tcgtattaat 7800
taggacgttt cgagtcgcgg tggagggatt gttttgtttg tatttattag tagtgcgggg 7860
tcgggttatt gtttcgtcgt gcgtattggg tttatatagg taagttttcg ggaatttagt 7920
ttttgtttag tttaaggcga ttcggttttt agtacgaatt taaaggtgaa gagatgaggt 7980
taggagtcga aggtttggga gaagagagtg gaatggttaa gaagagaaag gtataaggat 8040
taataagata tttatttttt gtgttttatt atatttattt ttaattttt attttatata 8100
aaaaggagat acgttattta aaattagaaa atttgaaaaa tagtaataaa ttattttttc 8160

gattttaaat tttttaaata gtttgttaag tgaatgttgc gttaatttga agaagtttta 8220
attgtaaaga agatagagtt ttgaaaaggt aggttaataa attagaaatc gagaagtaaa 8280
tggattcgtt aaaagaaaat tattttgatt ttaaacgaat aattgtttgg tggtttattt 8340
tggatttata taagaataaa aagtcgtttt agattacgtt ttttgtgatg tttattagtt 8400
tttagataga aaatatataa tagaagagaa attttaattt agcgttttta aaatgttgaa 8460
agtttattta ttttatttaa cgttgattaa gatatatatt ttagattttt taaatttttt 8520
gtatattgta ttaagttcgt tttaattcga gagagttacg ttttaaattc gatttttttg 8580
tttattttat tattaattag atttaaattt ataaagtttg tagaattaat aattttgagt 8640
taattatata tgaaatatgt tttaatgaat ttttatataa ttaagaatgt tgttaaataa 8700
ttaattttaa ggataatttt taatagttat tttttttttt tagtgagttt aaggttgttt 8760
tgagttatta aagtttaagt aggtagaagg ggtgtgtgtg agttaagggc gaaaagttta 8820
gaattgcgtt taattagtaa aagtaaaatt ttatttatat aaaataaaaa aaattatttt 8880
tggagatatt aatttttat agtattgttt ttaagtaaat ttaattttta aagaaattaa 8940
agaaagaaat ttaaatatat ttaaaataat ttttgaaagt tttttttgttt tttagtatag 9000
gttagttgga gaggataaat taattttttt tgggtttttg tatgggcgat tgttttatta 9060
tggagttagt gttattattt ttgaatgtgt atttgtttga tattatagtt aatgatttgt 9120
aatgttagta tgaagtattt ttaaaatatt ttttttttgt ttttgtttat aagattggga 9180
aatttattcg atgtggaata aagtggatga agtagattat aaatatattt gtaatttatg 9240
tgtttttttt ttgttttgat tatttttaaa ttttatttgt aatttttttt tattttaaat 9300
ttgtagttta aagacgtata tgagaattgt tttttagttt tttttttatta gtattatttt 9360
attttaagaa taatttagtt gtaagggagg aatttttttta tagtaagttt taaattagta 9420
tttttgtttt taattttttta ttttatttta ttttatttta tatatataga tatttgttta 9480
gagtaaaata tatttttatg tgataggttt gtattagttg aggtttatat atttagttat 9540
attaggtttt gtaattttat tattaaatta tatatattat attagtagtt tgttggtaaa 9600
gaaggttaaa ttaatttata ttttgtttat tatttggtgt ttaaatgacg tattttattt 9660
cggagatttg gcggagaatt tttttttttag atttatagc gtttttattga agataatgtt 9720
tttatatttg tagtggtttt taatttgata agattttaat ttgtttaagt tttttaaata 9780
agggttttaa atgtttttag tcgtttttttt attgaatttt ttttaatttt tttaagatta 9840
taaagtatat gtgtaaagta aatattttttt tttattgtat tgttagtcga tgatttataa 9900
ttaagttaat aagaatttag ttttttttttg ttgaatgtgt ttattaatta tattttagtt 9960
ttttttttta aattttagaa tagttgtggt ttttataata ttatgttttt taaagtttta 10020
ttttatgaag ggattttatt atattaaaga atgaaaaaaa tttttattgt agttagtata 10080
tatagttttt tattttttgt tttttaagat ttaaatttta gagttgtaaa tatttttgga 10140
agtttgggtg ttaatgtttt attttagaaa gtcgagaagt tttatagagt tatatagatt 10200
tttaaattta tttttttataa atttatagaa ttttgataaa agttttggtg gttttatttt 10260
atcgatggaa tttttattac gataaatata tatgtatgaa ggattttaat tagtttttaa 10320
agtggttgaa aaatttaagg gtacgtgatt gtttttttata gtgttaacgt gtgcgagatg 10380
ttggaagtat tggggattag tagtagttta gatgtttaaa aagataaggt gttttaattt 10440
gtgtggattt attgaagtta agtggtgaat aaagataatt atttagataa tttagattaa 10500
agtaaaagta aaattatatt tatttgtata tatatattta tatttatttt atattataga 10560
tatatatacg tatatatata ttggttttgt aaataattga tttaaagtga ggattttttt 10620
tgtatttttt tagtaggagt tttaatattt ttttaatttt ttaattattt tatatattta 10680
tagtagcggc gattgggtga tatttttttt aggtttttttg tgtggtagga tattaatatg 10740
ataagtttgt atggggaaaa ggaggtatgt ggtgggaatt aagaaatatt gtttagtgaa 10800
aattttgtgg tatggtggtg gttgattttg gagatttaat gtatataaga tttgtgggtg 10860
tataggtata ggtagtatgg atgagaaagg ggttagaaga aaataaattt tatgtatttt 10920
gtgattttag tattattgtg atttttggtt aagttttttt taattggttt tagaaattat 10980
tatgagttta gtttttaata tagaaatttt taatacggag aatattggtg ggattttggt 11040
agggaaatta gaggtgttgt atggtttacg tggggtaaag aaggaaagtt tagtgtcggc 11100
gtgaggtttt gagtttggga gatattaggg gttgtttcga ttggggtttt ttgtttattt 11160
ttttaaagaa agattttaga ggagggaaat gtgtgatatg gggttagtcg tgttttgtgt 11220
tggtatttgt tatcgattat tagttttaaa gttttattta attttatatt ttttagtgtt 11280
agttgtgtaa agtttttttg gttatggtag tgagcggttg ggttgtgtcg ttaaattttt 11340
cgtattaatt tggtttggga tttaattaag tgatttttga tttttggaaa gagtttgttt 11400
ttagagttta tttagaagat ggtttaatta gatatttttt tgagttgtta ggttttagac 11460
gggtgggagt tttgttttgt ttaagttagt ttaaggacga ggttcgtttg gatttagttt 11520
ggagttacgt gatgggcgtg agtgtgtgag tttttggtaa ggcgtagagg ttagatggag 11580
atttgtattt ttgttcgaga agtgttttat ttttttttaat atttggtttt tttttgtata 11640
taaattaagt tgaaaatagt ttattattta ttattttta tagttatgga attaaataat 11700
ttagaaatta aaagttttat tgtagttgtt ttttttttta tttttttaaat ggaatttaaa 11760
aagttttggt ttgttaaaag gggaagatta tttttttgaat tggaagtttg tagatatatt 11820
gagtaatagt tattttttttt gggtttttgt aaatggtatt tatttttttta atttatagtt 11880
ttagttgttt aattatttga gatttggggt aattatttgg gggaatagtg tttagatggt 11940

agtgggagtt attattttat agtggtttgg ggaagagaag agaaagagat tagaggaggg 12000
ggtatttgtt aaaattattt aacgaatatg ttgttaatgt ttttttatat ttgtatgtta 12060
ttgttatagt ttttttaggt gttattgagt ttttagaaag taattatttg tcgaattaag 12120
taaaataagg agaatggtat agtatatgtg tttggagaag gggaaggaag ggtggaatat 12180
gaaattgagt atagatattt aggttaggaa agaaggaagt ggtaaggggt taaatgaagt 12240
tttatttttt cgttattttt ttaaataata gttggattaa atatttattt gttttttttt 12300
tttttttttt tttttttttt ttagtttatg tttatttttt tttttattt tttttgtttt 12360
tttttttttt tgtttttttt tttttagata tgttggtagt taatatttag tattagttgt 12420
tatggtgatt ataaattatt taaatttaaa aatatttatt tataatttga gatgaagttt 12480
ttattttttt agcgaaataa tattttaaaa gttgttagtt gataaaaaaa aaggaattta 12540
ttttattgta gtaatttaag taatatatta tttttaaagg tttaaattaa aatgttagtt 12600
tgttaaaaat atgttggtag agttttggat atttttttcg ttagattttt ataaagaagt 12660
tgattttgtt atttttggtc gtttttttaat atatatatat aattttgtat gttttttttt 12720
tttttattaa tttttttttt ttttattaat tattttagtt tttaaagatt ttacgtattg 12780
ggttttaaaa gaaaagaatt tttttttgga ttagaaaata gttttattgg ttgttgaagt 12840
gaaagatgtg gggtttaggg ggaaaggtta ttaggattat aatggcggtg gtggtaggag 12900
gttattttag aggagttaag aagaaaaaaa aatgtaggga gaaggattgg aggtggaaag 12960
atagagtaat agaaaattga gttgggtggt taggtgttgc ggtgaagttt agtttcgaaa 13020
tgataggtat atattttttt atttttgttt tttttttttt tgagagaaaa tttttttagt 13080
tagagatttt gggggtagg aggcgggtaa acgtcgttgt agttgggttt tttgtttttt 13140
atttgggtt tgttgtttt tgtttatttt ggattatagg gtattcgttt agatgaagag 13200
ttattaatta tttatttagt taatattagg aagacgataa agttttttat ataggattaa 13260
gaagatatta gattgtttta ttagtatatt tttgtttacg aataagtttt cgttatatat 13320
tttttttttt ttcgagtcgt tttaataatt gttgtatata ttagtagcgg gcggtgagga 13380
ataatagtcg aattagtttt aagaaatttt gtgtatcgag ttagtagcga ggaacgcgat 13440
ttgtgaagat tatttttgcg ggtagggatt cgtagggatt gattattttc ggataattgg 13500
tataattttt tttgggggtg aaaaattata acgcggcggg gtatttttta agtgagttgt 13560
agatttgatt ggcgcggggg gtgggggagg ggaggggaga atgggatggc ggaggtcggg 13620
cggaggaaag aaaatggaaa atttttttta tttttatttc gttgtttttt ttttaagttt 13680
tatgtttttt tcggtaagta tttgtttttt tcgcgttagt tatagttaga gttttttatt 13740
tttttttata tatttttttt tttttgataa ggtttaggat tttggttatt attttacgta 13800
ttattatttt gcgttttgtt agagacggtt tgggttgatt tcgttggtgt ttatgttagg 13860
attaaaattt ttttatgacg gcgaggaaaa ttgtattatt tgtttttagg gggtatatta 13920
ggagtttacg tagtatatgt ttcgtaaata ttcgttgatt gaatgagagg cgcgggggcg 13980
gggcggcgga gaggggtttg cggtcgttaa ggtcgttagg gttaatttag gtttttcgaa 14040
gaaggttggg atcgagttgt tgtcgcgtgt gaaggtgtgt gtcgcggttg ggggtgttat 14100
aacgggttat ggagtttatt ttttagaggg aggaagtttg tgtatattag cgatttgggt 14160
cgaatatttt tagtttattt attgggttaa agttatttaa taattaatgc gtttttgggg 14220
gaggtcgggg gaagtattcg tttttgtcg ggacgtaaag ttaggcgtta ggtttaaagg 14280
ggttgtagtg tagttcgatt ttagtacgga atttagagtc gtcgttttga aattttttaa 14340
gttagtgata gaggagggtt agttcgtttt tttttgagg gtgaagatta ttttatgagt 14400
tttttttagg atttttaaag taagaaaagt taaagaaagg ttttttttgtt ttaggggtc 14460
gtttttagtt ggttttttata ttatttttg ttagttgcgt ttattttttt ttaaattttt 14520
ggtttttagg ggtttttagt cgttttcgtg ttattttcgt ttcggggttt tatttaggtt 14580
gggtattcgt ttaatggata ttaaggagaa tgggatttat tagggaagga aggtagagtt 14640
tggattgttt agaggtggat tttgtttata tagaacgttt agtttttaac gaggatatgg 14700
tatttttggg cggcgttggg ggtagaggcg gagagggtag cgtaatagat tattacggtt 14760
ttttgaagaa gttatatgtt attgtgaatt tttttttttt ttaaaagtaa agaaaaattt 14820
taaaaaaata taagaaataa attttttttgt tttataagta ggttgtggtt aggattttgg 14880
atattttata agttaattta aaattaggga aggataggtg ttttattttt tagtagtgtt 14940
atagttttgt ttattcgtgt gatttttttt tgtcgtttat tagtttttta aaagttaatt 15000
aaattaagat ttttagtatt tttttttatt atatgttttt ttttaattaa tggtattaaa 15060
cgtgtttagg tagtaatttt tttttcggt taaaaagtag aaaaagatat attgagtgta 15120
ggggaagagt tttttacgtg tattaaaata atgcgggttt gaaagtaatg gttaagaaag 15180
taattattta ttattttag tttttatgt gttagttatt aaaagcgaac gattaggggc 15240
gtttgcgcgg tttgtgattg gcgtaagtag aatttttttg tttttagtcg ttttttgttt 15300
atttacgagg attttatttt atcgtaggtt tttttatttg tttttagaat gtattttta 15360
tgtttaggaa atttggggta gggattgggg gaaggagata ttttgcgttt ttttggtttt 15420
tagtataata agaaatgtta gtttcggttt ggcgattgcg agttcgtttc gcgcgaagcg 15480
agattgggga gtttttttag tttggtcgga gttagggttg agttcgcgta aagtattttt 15540
tttagaagtt atcgttgttt ttgattttta attatatttt aaatatatta cggtttaata 15600
atttattttt attatcgatt attaaataga agaagaattt aatataattt aaatgataga 15660
aattacgcga cgttattttt gtattgtttt tatttaattt tatggggtta atttcggata 15720

```
agtgagcgtt taattggttt agtagggcga ttggcggtgt agtgtagtgt tcgggcgtga 15780
agtattggat cgttttagac gttttatttt aataaatgat tattttttttt tagatttacg 15840
gggaaatttt aatgtaagat ttttgttttt tttttagtaa tacggtttgt ttttttgatc 15900
ggggttttaa atcgtttttt tttattttat attatatttg tatttttata ttttaattcg 15960
gaaagagggg gttaggggtc gagggttgtg gggggggggg ggttttattt gtttatatta 16020
ttaaaggtta atagtttttt aaggttaggt atttatttat tacggagtta ggaaaataga 16080
ggaatagtaa atttgagggg ttttttttta tgtatttgaa aagaaaggta ttttttttt 16140
tttatttttt atattttttt tttcgtttta tagaataagt tttaatttag gaaaggtttg 16200
tggcgtaggt tggagatttt ttaatttttt atataagttt gtagattttt tttggtaatg 16260
tttttcgatt tttttagagt gaaattagtt aattaagtaa cgatatcgtt aaaatttaag 16320
gtttggtaat tagtatttta ggtaggttcg cgtcgatagg ggtaaatttt tattttattt 16380
tgggttgtta agtatagtgg ttgtttttta gtttttttagg gatgttgtcg gtttttcgtt 16440
tttttttttaa ttagttaaag taaattttcg ttaatttaag ttttttttgt ttgtttttcg 16500
cgatgaatcg cgtatttata agtttgggtg gggcgtggtt gagagtttga gtgattgagt 16560
gggttttggt ggtgttgcgc gtagcgggat ataacgagcg atagaggtcg ttgttggatt 16620
atttttttac gttagtttag acgtcgaggt ttgttggagc gtgcgtaggg gattagatta 16680
tagggagcga gcgagaggga gagagaggtg ttgggtttta ggagtgtagt ataatttggg 16740
gaaaggaatt aacgtttttg ggacggttgt ttttcgtttt atttagaggc ggagtgttta 16800
agtttaagta gtaggcgcgt taggtttggc ggtttcgttt ttttgcgttt cgttcgaggt 16860
ttagagtttc ggaggcgggt gtttagcgcg cggtttgcgt tttttttttcg gttttattat 16920
tggcgttagg atgttgtcgc gggaagaatt tgttgttggt tgtttttttt cggtttttag 16980
gagagttcgt gaattcgatt tttttgattt cggagttttt ggagaagaga tatttaacgg 17040
tcgtcggttg tacgtttggg ttacgcgcgc gttcgtttta cgtgcggaga gaggcgtttc 17100
ggatcgcggt cgaaaggagt cggggacggg aggaggggga ggggcgaggt aggtcggagg 17160
agaaagaggg ataaagagta aagatttagt tagaggaaag agttgacggt attttcgttt 17220
ttcggatttt ttggtaattc ggggtaggat ggcgtatttt ttttgcgttt tttcggttgt 17280
cggcggtttt agtcgggagg agtaggttgg ggggtttcgg tatatagcgc gtcgttgttt 17340
tttagtttat cgtttcgtta tagggagagg ttattggcga tttggttttg attttttttt 17400
tgtttaggtt ggtttttcgg ggaagcgttt ttcgttgggg tttcgtcgta gggttagtgt 17460
ttttttgtcg tttttacgtg gcgcggtttt tcgttcgatg attcgggtag gagaaggggg 17520
tttttattta attgtatata cgtcgatatt agtttgcggt agttggtttt tatttttcgt 17580
tatttgtaaa atagaagaga aggaaggttg taagaagcgg cggtcgtcga gtgagtaggg 17640
tttagatgag attacgttat attagttgtt aggcgtttat tgtgtgttag gttttaggcg 17700
tgtttttttcg atttgatagt ttttggttgt gtagtagtat ttttagttta gtttcgggtt 17760
taggatattt atttattaag aggggatttt tttttagagt tgtcgtaaaa gtgtttagag 17820
gttagaggat tataaagtta tagtgtgttg gggaggttgt ggatttattt ttaagaattt 17880
cggtgtcggg gttaagaatt tatttgaacg taatggtagc gggagtgggt gggtggagag 17940
gattttttttt tttgggaagt tgtatgtaaa gattattttt tagtgtttgt ttattagttg 18000
gagttcggta aatatttgta gaatattagc gttaacgtgt ttttgtttta gatagtagtt 18060
tttttcggtt ttttgtaatt ttgaaacgaa cgggtttttg gtttagggtg ttttaggagc 18120
gagttgagtt cgggtttttt atttattagg agttattttt ttatatttag ttatattttt 18180
ttttagagat attaattcgg ttatttattt atttattata aataattatt ttaaagtatg 18240
atttaagatc gtagaggaga gatattgggt ggattgagcg agattgagga gagtagggta 18300
aacgtttttg gagggtttat tgttcgttaa ggacggagaa atagttttgg tataattgtt 18360
atttagtttt tttttttttt ttttcggcg agttaaattt tttttacgtt tttaattata 18420
acgtagcgag ttaagtattt aacgcgtttt ttttttttttg ttataggtaa gtcgggagag 18480
gtgggtttcg aggggtttta tcgggtgggt agaagagtcg cggttgtttt aaagataaga 18540
aaagaaggtt tagggttttt taggtttttt cgattttagc gtttgttttt ttttacgtta 18600
attagggtac gtcgacgatc ggagggttta tttcgcgcgg gtgcggggat cggggtggga 18660
gtaagcgttg tcgggttggc ggaggtatag aggcggggta gggagttgcg ggtttgtttt 18720
tggtttgagt atcgtttttt tgcgtttcgg ttttttttga agggagttgg gttttgggga 18780
gtttttggtt aaggtcgttg tttataggag gggttgttcg gcgttgtggc gtggggattt 18840
agggtgggga cggttaggcg gttttttttat tcgttagcga gaacgcgggc ggggatttttg 18900
tcgattcgat ttttgtgggt tcgtgggttt agaagtagta gtttggcggt tttagattta 18960
gtgattttgt agtaaaatta taggattagt ttttgattga gatgtttgtt cgtgagatat 19020
tataaaattt attattatag ttttttatta attcgatatg aagtaatata gatgggattt 19080
tattagttta gattttaaat gtttatttat gataatttcg gaggaaattt gtatgttatt 19140
attatttcga taattttttt tttttatatg tttgaattgg ttgtattatt agttggtagt 19200
cggagtattg tagatggtaa ttgtaaatag tttttattta tttatttttt ttaaagaatg 19260
aaatatataa aagaaaaaga ttgcgttgtt tggtgtaaag ttagttaatt attatatatt 19320
tttttttta tttttcgtg ttttagtgtt gaagattaaa taaagtaata taaaataaat 19380
ttttaagaat ttatagagtt ttattttaag gattgaaaag aaggttaagg cgtgtttttt 19440
agtttatttt tatatgtttt tgtgatttgg agatttattt tgtagttaaa atgagttttg 19500
```

agatttgtat ttttatgttt tatttaatga ttaggtttat tagaagaatt gagtttaaat 19560
aattggggaa gataattttt taaaaagaga tttttaattt tcgtttgttg atttttaaat 19620
ttgttttatt aagataagtt ttttgtgaga aatttggttg ttagatttcg gaattggttt 19680
taatggttaa ttttataaat tgagatggga gatttttttt gatgggaggt agtttttatt 19740
tttaaagttt atgttttagt tggaatgtat atgttaagga tttttgtttt ggttaatttg 19800
ggttttatat tgtgagtata taaaaagtat tatacggtta acggaggacg aggaattatg 19860
gtaaagtagg taggtaagtt ttaagaaata aaataatttg ttaaaaaata atttttgatg 19920
attatcgtaa gattgaaagt gtaggaaaaa tatagttcga ataattttag atttttttat 19980
attttttttt tttttatata ttttgttatt ttataataaa atttttaatg gaaagtttaa 20040
aaataaatag tataggaata tgtgttttaa atgaattaaa ttgtgaaatt agttagtaaa 20100
ttaatttgta gtaagtaatt atttaaggaa attaaaatat tgtttagttt agttttgtat 20160
tttattatgt gtatgcgttt tttataatta attaatataa gtgttttagg aatatttgaa 20220
gataaatacg tttaatttaa ggaataaagt atttaaataa tttaagtgta attttgttga 20280
gttaaagtaa aatattttat aaatgaagtg gttatttaat tttttaggga aagtttggtt 20340
attgaaatgt tgtatgttta tgttatatta ataaaaattt ttaatttatt ttgtttatgt 20400
gttttgtttt tttgatatta ttggtatttg aattttagat ggatttttgt taaaatgata 20460
ttttgtgtga taaaagtatt tttagttttg attgatagat taaaataaat gtaaggaaat 20520
ttttttaaat tagattaatt ttttataaaa atattttaga atgtatgaat tttgatattt 20580
atatttataa tggtaaaagt ttttttcgtt tagtttagta agataatatt tatataaaag 20640
agtaaaaaaa aattatatta ttttatgata gtttgatttt taaattgttt aagaaagtaa 20700
agtggttaaa ttggaaaaga ggaatatatt tcggaggttt agaatcgaaa attttttttt 20760
taatttttag ttggaaaata atttttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattc gtttgggttt gagaggttat taattgttag 20940
ttaagatgga attttttttt tttttttttt tttttaatgg ataataatgg gaaggggggtt 21000
aatttttttag tagttgaaat tttgtattta gttttttatt ttgagaatgt taatttttgg 21060
ttcgaggatt tgttttttgta gtgttggtat cgagatttaa gggaagatat ttcgttttaa 21120
atgttagtta cggtttggtt ttttttttcga ttttagtatt ttgtagattg ttagtgtttg 21180
tggcgggggga cgaaaggaat agggttttgt aaggtttgtt tgtcgattgc gttattttgg 21240
gcgaaattta gttttaaaag ttataaatta tttacggtga agattttttcg aagtggaata 21300
aatttttaga ttcgtattat ttttatattt tgcgggatag atggttttta tttatcggtt 21360
atcgggagag agttgttgtt ttcgcgtttt attgtttttc ggggcgattt ttagcgagtc 21420
gagttttcgg ttgtacggta agcgttcgaa agtcgggttt gagaggattg tagggttttt 21480
gagggtgtta agtttcgaag gagtttacgg gtgtattggg gttttcgaaa tttagtcgtt 21540
attggtagtt tttttttgtt tttttttagt tttttcgttc ggtttcgtat ttttttttt 21600
tttttttttt tttattttt ttttttttt ttgtttttat ttcgtgtggg gagtgacgtg 21660
acgttagtag agattttatt aaattttatt gtatagtggc gcgcgggcgg tcggtcgagt 21720
tcggttgcgc ggttggcgat ttaggagcga gtatagcgtt cgggcgagcg tcgggggggag 21780
cgagtagggg cgacgagaaa cgaggtaggg gagggaagta gatgttagcg ggtcgaagag 21840
tcgggagtcg gagtcgggag agcgaaagga gaggggattt ggcggggtat ttaggagtta 21900
atcgaggagt aggagtacgg atttttattg tggaaaggag gattagaagg gaggatggga 21960
tggaagagaa gaaaaagtaa tttgcgttaa ttcggtagtt ttaataaatt aaagggggag 22020
cgttagggta gcggggagat agaaacgtat ttttggggag taaattagga cgggttggga 22080
ggaagcgata gggaaagtgg tttaagagac ggaataaagg ataatgttta tggggttgtt 22140
tgggacgagg cgtgtggagt gtgggtgtga gcgtgcgtgt gtgatttttt tttaggtttg 22200
tagagttgag gaaagaggtt atagtaaaga gggattgcgg agggaggaaa gtgagagatc 22260
ggtagagggc gggagtggag gtgggcgcgg tggggatggg agaggatgag tgaagagaaa 22320
tttagaagaa tggagtgagt tagtgggaga gggtgggagg gttatagtcg ggagcgaacg 22380
agttaggttt gttagttggg gaaggtcggg acgttgggtt tagtttagtt gggatatcgc 22440
gttcgaggtt aaggcgggtg gattaggtat gttgagagtg tcggcgtata ggtgggtacg 22500
gttacgtatt gatttagtgt ttacgaaggg tttgtattgg ataaggttta gacgtttata 22560
gagtttagaa ttttttttgt tgtatttata tttaataagt ttattttggg ttacggatat 22620
tttattttt aaaatgacga ggttaaggtt tttggcgagg atggtattaa attgtacggg 22680
atagaagtgg gggtggggga gagagttttt tttaagttta tatttgtttt tgtaaagtaa 22740
agagtatgtg aaattatagg gtatattttt attcgaaaag tgtgttttat ttttgaattt 22800
tgattttttg attttttgat ttgagtaaag atgtgtattt tggtagtgag tagaatattt 22860
tggttttgtt ttgtttttga gtggaaggat tataaatata attcgtttgg aggattaggt 22920
gtgaaggttt ttgttaggta tatgggataa tgttttttta atttaaggg tattttgtta 22980
atgtatgttt ttggaaagtg tcggaatata gttattgttt ttggattcgg atttttttat 23040
taatattaat ttttgtttga gagtaaaatt taggttcgtt attaaaaaga tattttttttg 23100
gtttttaatt gagaataaag tttttttttaa aagttgtatt gtttttttta aattaatata 23160
ttaatattcg taattttaga aatatatagt gattcgggag aatgtgtata aaatagatac 23220
gtttaaaaaa gtttggcgtt taaaattaat tttagttatt atataggtgt tgggtttttt 23280

ttatttttgg gggttgtttg gaatatgtta tgtgtttttt tgaattattt cgtgttttga 23340
atttatttga gttagtagta aaaataggta aataaatttg tttaatttgt tttgagtgtt 23400
aaattttttt attttgaaat agttaatagt cgatagatgg atttatttta tggaaagggt 23460
tagttttttt agttacgaag aaaattgatt agagatttat attttaagtt atttttaatt 23520
tttacgtaat attcgtgaaa atttaaattt tttttttta tttagtggaa atttaaagta 23580
gtgttattta aggggagaga aatgaggggg aaaatgttta cgtgttgttt aattgtattt 23640
tttttttgat tttgagaatt tttattttg gtttttgaaa tttcgtcgag gtaagaaaat 23700
taaatttttt taataagttt tataattgaa ttttagttat aggatatcgg aaagtgtagt 23760
tcgagaaaga tatttttatt tttgtttatc gacgattttt gtagtttttt tattttttg 23820
agtaatgggt taataatttt tttttttttt tttttatttt tgtagagatt aagaggcgtt 23880
cgtagtagaa cggttttgtt tttagttggt ggcgaggata ggtaatttta tggaaaagtt 23940
ggaagagaat gagaaaatta aagatagaaa gatttagaga ttcgcggaga gatataggga 24000
gagggaaggg agttgcgttg aaaagacgta aagatacgcg cgtgtaattt tttttttttt 24060
taggttttag aggtttgtaa attagggttg agaggaaggg gttcgggaag tttacgtttt 24120
tttcgttttt tttttgtttg gagtttcgtt cgttagaggt tggttaattt tagtttcggt 24180
cgtcgtagat attgcgttga gtttttgggt tttcgttttg tttagcgtta gtgtagttga 24240
agtgagtagt tggtgggaaa tgtaaatggt ttttggagaa atagaagata tagaatgatt 24300
tttatttttt tttcgagtgt gtggaaggag ttggatatac gttttacgtt tttaattttt 24360
tttttatatt tttagttata tttttattaa ataattaatt aatgtttaga attattaggg 24420
aatatattag gtatgtaatc gtagaagtag ggtgttgggg ggttataaat tatcgagttg 24480
atttaagacg tggattttag gttttttttt tgttaaagta gtaaaggaag agcgggtttt 24540
ggcgattgta tttagatttc gattatttta aattagaagg gggtggaggg agcgtttaag 24600
taaagtaagt aatttttgt tttgtagatg taaataagat tgtagtatta aaggtattag 24660
tttttttagg gttagatcgt ttggattggg agtttgggga aggggagata ttaattttac 24720
gtatttgtga attttaagga tgttatattt ttatataaat aattttagtg cggatttttt 24780
ggaatggggg gagtaatatt tttattttag aatattaaaa tatttttttt ttaaagcgta 24840
tatttttttt attttttaa aattttgaat tatgtttaaa gataatagtt ttttagtaaa 24900
ttggagtatt ggattatttt ttttattttt ttttatcgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt tttttttaa 25080
tatgttgaga taagtgatag aatcgttaga atggtaatta aattggaaag tttagggaga 25140
ataataattt cgtgattaaa ttggggtaaa atcgtggata aatgtggggt gattttcgtt 25200
aatttttgt tatttaagag ttaggatttg ggaaaggtat agtattattt tagagttcgt 25260
tgtgacgggt tgtgtgttat tatttatttt ttttattttg gattatgatt ttaattttgg 25320
taagtaattt ttttagtttt ttatttgata ataagcgagt atgtaaatat taatggttag 25380
cgatgtttaa ttgttttaaa tattattgat ttgttggttg ttttaaattg tttttttagt 25440
ttaggttttg ttttcgaatt gtttatttta gaggtttgat ttatgttttc gatgttataa 25500
tataataatt gtttttttaa aaaaggtatt taagatgaat taattgattt gtatataaat 25560
taaaattatt atgcgttgtc gatttcggtg ttttataatt atttcgaaat tagtatttaa 25620
ttatttgagt taaaagaata tataaatgtt tgtattgatt tattaatgaa ttatttaatt 25680
aaaacgttcg ggtaatgttg ggcgttggaa agattgttaa attaagatat attataggag 25740
ggatatgaag attagaaagg taatagatta atatttcgta tttaaaacgg agttttcggt 25800
gatttttagt tttaattttg gagtaggggt tttttttttt tgttgttaaa aagattttgt 25860
gtttgtttgt gagtgagtgt atttaagtgg aaggaacgtt tttacggtta cggtggttta 25920
ggtttttgt tcggatcggg attttatagt tttaatttag gagcgttaaa ttttggaaga 25980
tttcgggtta gttttggagg tgcgtggttt cgtaagtcgt taggttaagt ttgttttttt 26040
tgtttgtttt ttcggtaggt tgggcgcgtt atggtagtga gtttttcgcg taaacggaga 26100
gttggaatta aagttgatat ttaatagata tgttaattga gtatttattt tcgttttgag 26160
aataggaata aaaggtagtt tttttaaga gaggcggtgt aaaggtacgt tataggagtt 26220
tagaaaaggt tggcggcggg aaatttgtag tttgggggtt agttaatatt ttttttttatt 26280
ttaagtattt attgatttgt tgttgttatt tttggcgacg tagaaggata tttgaaagaa 26340
tttttgatgg ggttttgatt tgagaaagga ggtgatttgt ttaggttttt attaaatttt 26400
taattattat attaattgtt tttttttatt ttttattcga tttttttttt tttgtttatt 26460
tttaattttt taattattta gaaatttttt tattttttag tggttttttt tttgtagtag 26520
tttttttattc gaattttttt ttcgtttttt cgtggtaggg tttgtatatt gatttttttg 26580
atttttggta tatttgggtt ttttgaaatt ttttaatttt tttagatttg aggatggtag 26640
gttttatttt ttttattgtg tgtatatatt tagagatatg aaaatttata tagattgttt 26700
ttaaatttag ggtatttaat agatgttttt tttttagttc gtttttttgat ttgaaatgtt 26760
tgtttgattt taatttggat attatttttt tttgtttttt ttttttttaaa gtagtttgga 26820
tatgtgtgta agtgagttta gaatagtttt atttatattt tttattaaat tgtaaataaa 26880
agaagaatta atgaagtaga ttggtatata gattgtatta agagttcgaa tttttagttt 26940
ttggattttt tatttaattt tggttgttat ttatattgat agagttattt taagtagagg 27000
tttagagaaa tttgtattgt gggataatag gtaaagttat agtaaaaagt ggaataattt 27060

taaagttatt ttattagaat gtaaattgta tttttgggtt ttgttcgtaa ttatttagtt 27120
ttaatatata tagagttaga taggaaaaaa taggttaata tagttattgg tattagagaa 27180
gataaatttt atgggttttt tagtgaaaag aagattttta aagtttataa tttttgatta 27240
tttaatttta tttataattg tgggaatgaa taagatatta attgttttat gtattttatt 27300
tatattaatt aatttgtgtt tttattaaaa gtagttatat agaatttttt ttaattttttg 27360
gtagtaagtt tagaaaatga agtttatagt tattttgaat tggatatatt ttttgagttg 27420
attatttttg taagtgtagg aatataatat tgttttttta tggtttttttt gtatttttttt 27480
agggtttgta agtttttatt aggtttgata ttattgtttg ggtttatatt tattataagt 27540
aaatttgatt attatgttga ttttaaaata gtttatttgg ttagtataat tttagttttt 27600
aaattataaa aattttttaa tatacgaagt ttttagtttt tatttttttt agtttttttgt 27660
ttatttaaaa tttttatttt aattggtgta agtaataata atttgtatta ttatttgtat 27720
ttttttttatt tttttggaga ttgggttgga ttttagagag aatattagta ttattattat 27780
tataaataat aaaatttaaa agtaaagttt ttatttgtat gataattggt atttggaatg 27840
tttttgattt atttaatgtt attttataaa ggtattttgt aaattttttt ggaattttta 27900
gtaagagttt gtagtaattg gaataatttt ttgggaagat atttttttttg atgggttttt 27960
agtttttgga ggaatagatt gagagtaatt agggagggag gggatattgg aaattggtag 28020
ttacgttagt tgaaataagt ttgggtttag taaggtgatt gatgttgtgg ttgatttttt 28080
atttcgagtt tttttttaat tggggtattg atttttttta ttttgggatt ttaaggtatt 28140
cggtgtgtat gtagattttt tttttgtggt ttttattatg tggtttcgta gtaggttttt 28200
ggtttaatga tattttatag ttatagtttt tatatttatt attatgattt taatgtttag 28260
gtttttagtg tatttatatt aaatttgttt tattagtaag ttggagtata taggagagat 28320
gggggtaagt aaggatttag tagagtttaa atttagatat gtttaaatgg ttttgattgt 28380
gtaaagtgtg gtaatgtttt ttgttgtttt agttttttat tttaagtttt atatgttttt 28440
tggttaatga agtgtgatat aggttatatg ttaggaataa tagtatttgt tgagaataaa 28500
gtgaatttag gaaatttggt atatataaaa tgtatt 28536

<210> 152
<211> 28536
<212> DNA
<213> Artificial Sequence

<220>
<223> chemically treated genomic DNA (Homo sapiens)

<400> 152

```
gatgtatttt gtgtatatta ggtttttga gtttatttg tttttaataa atattattat      60
ttttagtatg tggtttatat tatattttat tagttagagg atatgtgaag tttagagtgg   120
aaagttaggg tagtaggaag tattgttata ttttatatag ttaaagttat ttaaatatgt   180
ttgaatttga gttttgttga gtttttattt gtttttattt tttttgtgtg ttttagttta   240
ttaataaagt aggtttaata taaatatatt aaaagtttaa atattgagat tatgataatg   300
aatatgaggg ttatgattat aaaatattat tgaattagag atttgttacg aaattatatg   360
gtgaagatta taagggagga atttgtatat atatcgagtg ttttgggatt ttaaagtggg   420
aagagttagt gttttaatta aagagaaatt cggggtaggg aattaattat aatattagtt   480
attttattga atttaggttt gttttagttg acgtagttgt taatttttaa tgtttttttt   540
tttttaatt gtttttaatt tatttttta gaaattgaaa gtttattaaa gaggatattt   600
ttttagagag ttgttttagt tattataagt ttttgttaga aattttaaga aagtttataa   660
ggtattttta taaggtggta ttgagtaagt tagaagtatt ttaagtatta attattatgt   720
aaataagggt tttattttta gattttgttg tttgtggtgg tggtgatgtt ggtgtttttt   780
ttggaattta gtttaatttt taaaaaagta aaaggagtat aaatagtaat ataaattatt   840
attatttata ttaattaaaa tagaagtttt gaatgagtaa ggagttagag gaggtaaaaa   900
ttgggaattt cgtatattaa aaggttttta taatttgaaa attaagatta tgttggttaa   960
ataggttgtt ttaaaattag tatagtaatt aaatttgttt gtaatgaatg tagatttaaa  1020
tagtgatgtt agatttgata aggatttgta aattttaaaa gagtgtaaaa agattataga  1080
agaataatat tatattttg tatttataga aatggttagt ttaagagatg tatttagttt  1140
agggtggttg taagttttat tttttgaatt tgttattaga agttaaaaga aattttgtat  1200
aattgttttt gatggaaata taaattggtt gatgtaaatg aaatatataa agtagttggt  1260
gttttattta tttttataat tataaatgaa attaaatgat taaaaattat agattttggg  1320
gatttttttt ttattgagga gtttatggaa tttgtttttt ttagtattaa taattgtgtt  1380
gatttatttt tttttgttta attttgtata tattaaaatt aggtggttac gaataaaatt  1440
tagaaatata atttatattt taataaaatg attttaaaat tattttattt tttattgtgg  1500
ttttatttgt tgttttataa tgtaggtttt tttgggtttt tgtttagaat gattttgtta  1560
atgtagatga tagttagagt tgaatgggga atttagaaat tggggattcg ggtttttgat  1620
```

gtaatttata tgttaattta ttttattagt ttttttttta tttatagttt ggtaaagaat 1680
atgggtggag ttgttttggg tttatttgta tatatgttta aattgttttg aaaaaggaag 1740
ggtaagaaag agtggtattt aagttggaat taggtaggta ttttagatta agagacgaat 1800
tggaaaggga atatttgtta gatattttgg gtttgaaggt agtttgtgta agtttttata 1860
tttttgagtg tgtgtatata gtggagaggg tggagtttgt tatttttaaa tttgaaaaga 1920
ttgagagatt ttagagggtt tagatgtgtt aaaggttaga gggattaata tataggtttt 1980
attacggaaa ggcggggaaa aggttcgaat agaaaattgt tgtagaaggg aagttattga 2040
gaggtaaggg agtttttgaa taattaaaaa gttaagaata agtaaaagga aggaggtcgg 2100
gtgggggata aaaaaaagta gttgatgtgg taattaagaa tttggtggga gtttgggtag 2160
gttatttttt tttttagatt agagttttat tagaaatttt tttaagtgtt tttttgcgtc 2220
gttaaagatg ataatagtaa attaataagt gtttgaaatg aaagggggatg ttgattagtt 2280
tttaggttat agatttttcg tcgttagttt tttttgaatt tttatagcgt gtttttgtat 2340
cgtttttttt aagaagagtt atttttttatt tttattttta ggacgaaggt aagtgtttag 2400
ttagtatatt tattaaatgt tagttttggt tttagttttt cgtttgcgcg gaaagtttat 2460
tgttatagcg cgtttagttt gtcggagggg tagatagaaa aagtaagttt ggtttggcga 2520
tttgcggggt tacgtatttt tagggttggt tcggagtttt ttagagttta acgtttttgg 2580
gttagaattg taaggtttcg gttcgagtaa agggtttgag ttatcgtagt cgtgggagcg 2640
ttttttttat ttgaatgtat ttatttataa ataagtataa aattttttta atagtagagg 2700
agaaagattt ttgttttaaa attaaagttg ggaattatcg gaaatttcgt tttgagtgcg 2760
agatattggt ttgttatttt tttaattttt atattttttt tgtaatatgt tttgatttaa 2820
taattttttt agcgtttagt attgttcgga cgttttaatt gggtaattta ttagtgagtt 2880
aatataggta tttatatatt tttttagttt aagtggttaa gtattaattt cgaaatgatt 2940
ataaaatatc ggaatcggta acgtatagta attttaattt atatgtaaat taattggttt 3000
attttaaatg tttttttttaa aaaaataatt attgtattgt agtatcggag gtatggatta 3060
aatttttaga atagataatt cggaaataag atttggatta ggaagataat ttagaatagt 3120
taataaatta ataatgtttg aggtagttaa atatcgttag ttattggtat ttatatattc 3180
gtttgttgtt agataaggag ttggggaaat tgtttgttag ggttgagatt ataatttaga 3240
gtgaagaaag taaatggtag tatatagttc gttatagcgg gttttgaaat aatattgtat 3300
ttttttttaaa ttttgatttt tgggtgatag ggagttggcg gaggttattt tatatttgtt 3360
tacggttttg ttttaatttg attacgaaat tgttgttttt tttgagtttt ttaatttgat 3420
tattatttta acggttttgt tatttgtttt aatatattgg ggggaggagt gtaattgaga 3480
tttttattaa aaattatttg aatttattta gttagtattg ttttatttaa gtttagtttt 3540
atgggttgta tttaattttt tgtgtttttt atatattaaa attagattat taaaatgtcg 3600
gtaggaaagg gtgaaggaaa tggtttaatg ttttagttta ttggaagatt attattttta 3660
gatatagttt aaaattttga ggaaataaaa aggatatacg ttttgggggg aaaatgtttt 3720
aatattttag aatgggggta ttattttttt tattttagag aattcgtatt ggagttgttt 3780
atgtaaaaat gtaatatttt tgaaatttat agatacgtaa ggttagtgtt tttttttttt 3840
taggttttta gtttaggcga tttagtttta aaggagttag tatttttgat gttataattt 3900
tgtttatatt tgtagggtag agaattgttt gttttgtttg gacgtttttt ttattttttt 3960
ttaatttgaa gtaatcggaa tttaaatata gtcgttaagg ttcgtttttt ttttattgtt 4020
ttgataaggg aaaaatttga aatttacgtt ttaaattagt tcggtggttt gtagtttttt 4080
agtattttgt ttttacgatt gtatgtttaa tgtatttttt ggtgattttg ggtattaatt 4140
agttgtttaa taggagtatg attaaaaatg taaaagaagg attaggagcg tgaaacgtat 4200
gtttagtttt ttttatatat tcgaggaggg aatgagaatt attttgtatt ttttattttt 4260
ttaggagtta tttgtatttt ttattagttg tttattttag ttgtattggc gttgggtaag 4320
gcgaggattt aaaagtttag cgtagtgttt gcggcggtcg ggattggggt taattagttt 4380
ttggcgggcg agattttaga tagaaggggg gcgagaggaa cgtgagtttt tcgagttttt 4440
tttttttagt tttggtttgt aaatttttga aatttgaaag gggagggagt tgtacgcgcg 4500
tatttttgcg ttttttttagc gtaatttttt tttttttttt tgtgtttttt cgcggatttt 4560
tgaatttttt tgtttttggt tttttatttt ttttttaatt tttttatgag attgtttatt 4620
ttcgttatta gttgaaggta aggtcgtttt gttacgagcg ttttttaatt tttataaaat 4680
gaaaagaaaa aaagggagga ttattagttt attatttaga ggaatgggga ggttgtaaaa 4740
atcgtcgatg ggtagaggtg aagatgtttt tttcggattg tatttttcgg tgttttgtaa 4800
ttagagttta gttgtgggat ttgttgaaga aatttgattt ttttgtttcg gcgagatttt 4860
aaaaattaga aatagaaatt tttagagtta gagaggaaat ataattaaat agtacgtggg 4920
tatttttttt tttatttttt ttttttttaaa taatattgtt ttgagttttt attgggtaaa 4980
gagagaaagt ttgagttttt acggatgtta cgtggaggtt agaaatggtt taaaatgtag 5040
atttttaatt agtttttttc gtggttgaag aggttaattt tttttataaa atgagtttat 5100
ttgtcgattg ttagttattt taaagtgaag ggatttagta tttaaaataa attgagtaag 5160
tttgtttgtt tgtttttatt gttaatttaa atgaatttaa aatacggagt aatttaagaa 5220
aatatataat atgttttaga tagtttttaa aagtagggaa agtttagtat ttatatagtg 5280
attagggtta gttttaagcg ttaagttttt ttaaacgtat ttattttatg tatatttttt 5340
cgagttatta tatattttta aaattgcgag tattggtata ttgatttagg aagagtaata 5400

```
taattttag agggaatttt atttttaatt agggattaaa gagatgtttt tttaatagcg 5460
ggtttgagtt ttgtttttaa gtaggaatta atattggtgg gaaaattcga atttaggagt 5520
aatggttgtg tttcggtatt ttttaaaaat atatattaat aggatgtttt tgagattgaa 5580
aaaatattgt tttatatgtt tggtagaagt ttttatattt ggtttttttag gcgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
tcgagtgaga atatgttttg taattttata tatttttttgt tttgtaggag taaatgtgga 5820
tttgagggaa attttttttt ttattttttat ttttatttcg tgtaatttaa tattattttc 5880
gttaggaatt ttaatttcgt tattttaaaa aatgagatat tcgtgattta gggtgaattt 5940
gttgaatgta ggtatagtag aggaaatttt agattttatg agcgtttgag ttttgtttag 6000
tgtaaatttt tcgtgaatat tgggttagtg cgtggtcgtg tttatttgtg cgtcgatatt 6060
tttagtatgt ttggtttatt cgttttgatt tcgggcgcgg tgttttagtt aagttgggtt 6120
tagcgtttcg gtttttttta gttgataagt ttagttcgtt cgttttcggt tgtggttttt 6180
ttattttttt ttattagttt attttatttt tttagatttt tttttattta ttttttttta 6240
tttttatcgc gtttattttt attttcgttt tttatcggtt ttttattttt ttttttttcgt 6300
agtttttttt tgttgtgatt ttttttttta attttgtagg tttgaaagaa ggttatatac 6360
gtacgtttat atttatattt tatacgtttc gttttaaata attttatgaa tattgttttt 6420
tgtttcgttt tttgggttat ttttttttgtc gttttttttt agttcgtttt gatttgtttt 6480
ttaaaagtac gttttttgttt tttcgttgtt ttggcgtttt tttttttgatt tattagggtt 6540
gtcgggttgg cgtagattgt tttttttttt tttttatttt attttttttt ttggtttttt 6600
tttttatagt gggagttcgt gtttttgttt ttcggttggt ttttaagtgt ttcgttaggt 6660
tttttttttt ttcgtttttt cggtttcggt tttcgatttt tcggttcgtt ggtatttgtt 6720
tttttttttt gtttcgtttt tcgtcgtttt tgttcgtttt tttcggcgtt cgttcgggcg 6780
ttgtgttcgt ttttggatcg ttagtcgcgt agtcgggttc ggtcggtcgt tcgcgcgtta 6840
ttgtgtagtg gagtttggtg gaatttttgt tgacgttacg ttattttttta tacggagtag 6900
gagtagaggg aagagagagg gatgagaggg agggagagga gagagagtgc gagatcgagc 6960
gagaaagttg gagaggagta gaaagaaatt gttagtggcg gttagatttc ggaggtttta 7020
gtgtattcgt ggatttttttc ggaatttggt atttttagga gttttgtagt ttttttaggt 7080
tcggttttcg ggcgtttgtc gtgtagtcgg aggttcggtt cgttggaaat cgtttcggga 7140
agtagtggga cgcggagata gtagtttttt ttcggtagtc ggtaagtgga ggttatttat 7200
ttcgtaggga tgtgagataa tgcgagtttg gaaatttgtt ttatttcgga gaatttttat 7260
cgtaggtgat ttgtggtttt tggggttaag tttcgtttaa ggtaacgtag tcggtaaata 7320
gattttgtaa agttttgttt ttttcgtttt tcgttataga tattaataat ttatagggtg 7380
ttgaagtcga gagggaagtt agatcgtggt tggtatttaa aacgaggtat tttttttttaa 7440
atttcggtgt taatattgta ggaataaatt ttcgggttaa ggattagtat ttttaagata 7500
aagggttggg tataaagttt tagttattgg aagattagtt tttttttttat tgttatttat 7560
tgggaaaaaa aagaaaagaa aaagatttta ttttaattgg tagttagtga tttttttaggt 7620
ttaagcgaat tatttgggag ttaggtttgg atgttaagtt tttattattt ttttggattg 7680
taattttttt aaattgatta ttagttaatt ttaatttggt attttaggag atatatttta 7740
aatggatgta gagaattatt ttttagttgg agattaagaa aaaaatttc gattttaaat 7800
tttcgaaata tgtttttttt ttttagttta attattttat ttttttaagt aatttagaaa 7860
ttaaattatt ataaggtggt gtgatttttt tttatttttt tgtgtgagta ttgttttatt 7920
aaattaaacg gaaaaaattt ttattattat aaatgtaaat attagaattt atatatttta 7980
aaatattttt atgaaaaatt aatttgattt aaagaaattt ttttgtattt gttttagttt 8040
attaattaaa attaaagatg tttttattat ataaaatatt attttggtag aaatttattt 8100
aaaatttaaa tattaataat attaagaaaa taaagtatat aagtaaaata aattgaagat 8160
ttttgttgat gtaatatgag tatataatat tttaataatt aaattttttt taaaaaatta 8220
aatagttatt ttatttgtgg aatgttttat tttaatttag taaaattata tttaaattat 8280
ttaggtgttt tgttttttaa gttaagcgtg tttgtttttta aatgttttta aagtatttat 8340
attaattggt tgtaaagaac gtatatatat ggtaaaatat agaattgaat tgagtagtat 8400
tttaattttt ttaaataatt atttattata aattaattta ttggttaatt ttataattta 8460
gtttatttaa aatatatgtt tttgtgttgt ttatttttaa atttttattt aaagattttg 8520
ttatggggta ataaagtgta tgaaaagggg ggaaatgtga aaggatttgg gattattcga 8580
attgtatttt ttttgtattt ttagttttgc ggtagttatt agaaattatt ttttagtaaa 8640
ttgttttatt ttttagggtt tgtttgtttg ttttgttatg gtttttcgtt tttcgttagt 8700
cgtgtagtgt tttttgtgtg tttataatat aaaatttaag ttggttaaaa taagagtttt 8760
tggtatatat attttaatta gaatatgaat tttgggggtg agaattattt tttattagga 8820
aaagtttttt attttaattt gtgagattag ttattgaagt tagtttcgaa gtttggtagt 8880
taaatttttt atagaagatt tgttttgata gggtaagttt aaggattagt aggcgggaat 8940
tggaggtttt tttttaaaaa attatttttt ttagttattt agatttagtt tttttagtag 9000
gtttggttat taaatgaagt ataaaaatgt aagttttaag gtttattttg attgtaaaat 9060
aaatttttaa gttataagga tatgtaggag tgagttaagg aatacgtttt gatttttttt 9120
ttagtttttta gagtggagtt ttatgagttt ttgaagattt gttttgtatt gttttgtttg 9180
```

gtttttagta ttgaagtacg gggaagtggg gggaagaatg tgtaataatt gattgatttt 9240
atattaagta acgtaatttt tttttttgt atattttatt ttttaaaaaa aataaataaa 9300
taaaaattat ttgtagttat tatttgtagt gtttcggtta ttagttaata atgtagttag 9360
tttagatata taaaaaaaaa agattatcga aatgatgatg atatgtaaat ttttttcgaa 9420
attattataa gtaaatattt gaagtttgga ttaataaaat tttatttgtg ttattttata 9480
tcgagttagt agaaagttgt gataatgaat tttgtaatat tttacgaata gatattttaa 9540
ttagggatta attttgtgat tttattgtag aattattaaa tttggagtcg ttaaattgtt 9600
atttttgggt ttacgggttt ataaggatcg aatcggtaga gttttcgttc gcgttttcgt 9660
tagcgggtgg gggaatcgtt tggtcgtttt tattttggat ttttacgtta tagcgtcggg 9720
tagttttttt tgtaggtagc gattttggtt agaggttttt tagggtttag ttttttttag 9780
gagaggtcga gacgtaggga aacggtattt aggttagagg taggttcgta gtttttttgtt 9840
tcgtttttgt gttttcgtta attcgataac gtttgttttt atttcgattt tcgtattcgc 9900
gcgaagtggg tttttcggtc gtcggcgtat tttggttagc gtggagagag gtaggcgttg 9960
agatcgaagg ggtttaggga gttttggatt tttttttttt gtttttaaag taatcgcggt 10020
tttttttattt attcggtgga gtttttcgag atttattttt ttcggtttgt ttgtggtaga 10080
gaagggggag cgcgttaaat gtttggttcg ttgcgttgtg gttgaaaacg tgaaaaagat 10140
ttggttcgtt cgggagagaa agggggagaa ttgggtagta gttatattag agttattttt 10200
tcgtttttgg cgggtagtaa atttttttaag aacgtttgtt ttgttttttt tagtttcgtt 10260
tagtttattt agtgtttttt ttttgcgatt ttaaattata ttttagggta attatttgta 10320
gtaagtaaat aaatggtcgg gttagtattt ttaggagaaa gtgtggttaa atatggaaaa 10380
gtggtttttg atggatgaga ggttcgaatt tagttcgttt ttgaaatatt ttaggttaag 10440
agttcgttcg ttttagaatt atagaaaatc gagggaaatt gttgtttagg atagggggtac 10500
gttggcgttg atgttttata aatgtttatc gagttttaat taatggataa gtattgaagg 10560
gtggtttttg tatatagttt tttaaagaga aaagtttttt ttatttattt attttcgttg 10620
ttattgcgtt tagatgagtt tttaatttcg gtatcgagat ttttgaaagt aggtttatag 10680
tttttttagt atattgtggt tttatagttt ttttaatttt gggtattttt gcggtaattt 10740
tggagggaga tttttttttg ataaataaat gttttgggtt cgaggttagg ttggagatgt 10800
tgttgtatag ttagaggttg ttaggtcgga aaaatacgtt tgaagtttag tatatagtag 10860
gcgtttaata gttagtgtaa cgtagtttta tttgagtttt gtttattcga cggtcgtcgt 10920
tttttatagt tttttttttt ttttgttttg tagataacgg ggaatggaga ttaattgtcg 10980
taaattggtg tcggcgtgtg tgtaattagg taagaatttt ttttttttgt tcgggttatc 11040
ggacgggagg tcgcgttacg tgagggcggt aagagggtat tggttttgcg gcgaggtttt 11100
agcgaggggc gtttttttcga ggggttagtt tgggtaggaa ggaaattaga attaaatcgt 11160
tagtggtttt tttttgtggc ggggcggtgg attaggaagt agcggcgcgt tgtgtatcga 11220
agttttttag tttatttttt tcggttggaa tcgtcggtaa tcggggaggc gtagaaagag 11280
tacgttattt tgtttcgggt tgttagaggg ttcgggggac ggggatgtcg ttagtttttt 11340
tttttaattg ggtttttgtt ttttgttttt tttttttttt cggtttgttt cgtttttttt 11400
ttttttttcg ttttcggttt ttttcggtcg cggttcggga cgtttttttt cgtacgtggg 11460
gcgggcgcgc gcgtggttta ggcgtgtagt cggcggtcgt tgaatgtttt tttttaaag 11520
atttcgaaat taaaaaggtc gagtttacgg attttttttga gagtcgaaaa gaggtagtta 11580
gtagtaagtt tttttcgcgg tagtattttg gcgttaatgg taaggtcggg agggaagcgt 11640
aggtcgcgcg ttgggtattc gttttcggga ttttgggttt cgggcgaagc gtaagaaggc 11700
gaggtcgtta gatttgacgc gtttgttgtt tgaatttaga tatttcgttt ttgggtggga 11760
cgggaagtag tcgttttagg gacgttaatt ttttttttta aattatattg tatttttgag 11820
atttaatatt tttttttttt tttcgttcgt tttttgtggt ttgatttttt gcgtacgttt 11880
tagtaaattt cggcgtttag gttggcgtgg aaaagtggtt taatagcgat ttttgtcgtt 11940
cgttatattt cgttgcgcgt agtattatta gggtttattt agttatttag gtttttagtt 12000
acgttttatt tagatttgtg ggtgcgcggt ttatcgcggg aggtaagtaa gggaaatttg 12060
agttggcgaa ggtttgtttt ggttggttgg gggagggggcg gggggtcgat aatattttttg 12120
aagagttgga gggtagttat tgtgtttagt agtttagggt agaatggagg tttgtttttg 12180
tcgacgcgaa tttgtttgaa gtattggttg ttaggttttg ggttttggcg atgtcgttgt 12240
ttgattggtt ggttttattt tggaggaatc gagggatatt gttagaggag gtttataggt 12300
ttatgtaaaa agttaaaaag tttttaattt acgttatagg ttttttttga attgaaattt 12360
gttttatggg gcggaggggg gggtgtaagg gatggaggag ggaagatgtt ttttttttta 12420
aatatatgga aaaaaatttt ttaaatttat tgttttttta tttttttggt ttcgtagtaa 12480
ataagtgttt agttttagga ggttattgat ttttgataat gtgagtagat aaagtttttt 12540
ttttttttata gttttcggtt tttaattttt tttttttcgga ttaaagtgta agaatataaa 12600
tgtaatatgg gatggagggg ggcgatttgg gatttcggtt aaaaaaataa atcgtattat 12660
taagaagaaa ataaaggttt tgtattggag ttttttcgtg aatttgagag aaaatgatta 12720
tttgttgaaa tgaagcgttt aaagcgattt agtgttttac gttcggatat tgtattatat 12780
cgttagtcgt tttgttgggt tagttaaacg tttatttgtt cgggattaat tttatggggt 12840
taaatggggg taatgtagag ataacgtcgc gtgatttttg ttatttagat tgtgttaaat 12900
tttttttttg tttgataatc ggtagtaaaa ataaattatt agatcgtagt atgtttggga 12960

tatggttaaa aattaagagt agcgatgatt tttggggaga atgttttgcg cgggtttagt 13020
tttggtttcg gttagattag aggagttttt taatttcgtt tcgcgcgggg cgggttcgta 13080
gtcgttaagt cgaggttgat atttttattt gtgttgggag ttagagagac gtaaaatgtt 13140
tttttttttt agtttttattt ttaggttttt tagatatggg gaatgtattt tgaggatagg 13200
tggagaagtt tacggtagga tggggttttc gtaggtgagt aggaaacggt taagagtaga 13260
ggagttttgt ttgcgttagt tataagtcgc gtaggcgttt ttggtcgttc gtttttgata 13320
attagtatat aaagaattag aaataatgaa tgattgtttt tttaattatt atttttaggt 13380
tcgtattgtt ttagtgtacg tgaaaggttt tttttttata tttaatatgt ttttttttat 13440
tttttgatcg aaaagaaaaa ttgttgttta aatacgttta atgttattaa ttaagaaaag 13500
gtatgtaatg ggaagaaatg ttgaaaattt tgatttaatt ggtttttaag gaattagtag 13560
acgataaaaa aaaattatac gagtgggtaa agttatagta ttgttgaagg atagagtatt 13620
tatttttttt tgattttaag ttaatttatg gaatatttaa agttttggtt atagtttgtt 13680
tgtaaaataa aaggatttat tttttgtgtt tttttaaagt ttttttttgt ttttaaagag 13740
aaaaaaagtt tataatgata tatgattttt ttaaaaggtc gtgatagttt attacgttat 13800
ttttttcgtt tttgttttta acgtcgttta aaaatattat gttttcgtta aagattaaac 13860
gttttgtata ggtagagttt atttttaagt agtttaggtt ttgttttttt tttttagtga 13920
gttttatttt ttttggtatt tattgggcgg atgtttagtt tggatagaat ttcgaaacgg 13980
gggtagtacg agagcgattg gagattttta aaagttagag gtttgagaga gggtggacgt 14040
agttagtaga agatggtgta gaagttagtt gagaacgatt ttttagagta aagagatttt 14100
tttttggttt tttttgtttt ggggggtttg aaaggaattt ataaaatggt ttttattttt 14160
aggaggagga cggattgatt tttttttgtt attggtttaa aaagttttag ggcggcggtt 14220
ttgggtttcg tgttgaaatc ggattgtatt gtagtttttt tggatttgac gtttggtttt 14280
gcgtttcgat aaggggcggg tattttttttc ggtttttttt aggaacgtat taattgttaa 14340
atagttttgg tttagtggat gggttgaaag tgttcgattt aagtcgttgg tgtgtataga 14400
tttttttttt ttgggaggtg ggttttatgg ttcgttgtgg tatttttagt cgcgatatat 14460
atttttatac gcggtagtag ttcggtttta atttttttcg aaggatttgg gttaattttg 14520
gcggttttgg cggtcgtaga tttttttttcg tcgtttcgtt ttcgcgtttt ttatttaatt 14580
agcgaatgtt tgcggagtat atattacgtg gatttttaat gtattttttg aaagtaaata 14640
atatagtttt tttcgtcgtt atgaagggat tttaatttta atatggatat tagcgagatt 14700
agtttagatc gtttttagta aaacgtaaaa tggtggtgcg tggggtggtg attaaggttt 14760
tgagttttgt tagaaagaag gggatgtgta gagaaaggtg gagaatttta gttgtggtta 14820
gcgcggaagg gataggtgtt tgtcgaaggg ggtatgaggt ttgaggaaaa agtaacgaaa 14880
taggggtaag gagagttttt tatttttttt ttttcgttcg attttcgtta ttttattttt 14940
tttttttttt ttttattttt cgcgttaatt aaatttgtag tttatttgaa aggtgtttcg 15000
tcgcgttgtg gtttttttatt tttaggggaa attgtattag ttgttcgaaa gtagttagtt 15060
tttgcggatt tttgttcgta aaagtggttt ttataggtcg cgtttttcgt tgttgattcg 15120
gtatataaag tttttttaagg ttggttcggt tgttattttt tatcgttcgt tgttaatata 15180
tgtagtagtt gttagagcgg ttcgggggaa aaggaaatgt ataacgaaag tttattcgtg 15240
agtaggaata tattaatgga ataatttgat gtttttttaa ttttatgtaa aaagttttgt 15300
cgtttttttta atattgattg aatgggtaat taatggtttt ttatttaggc gaatattttg 15360
taatttaaga taggtaaaag ataataagtt taaggtagaa gataaaaggt ttaattgtag 15420
cggcgtttgt tcgtttttta tttttttaggg tttttgatta ggaaagtttt tttttagagg 15480
agaaaaaggt aggagtggga gaatatatat ttattatttc ggggttagat tttatcgtag 15540
tatttgatta tttagtttag tttttttgtta ttttgttttt ttatttttag tttttttttt 15600
tgtatttttt tttttttttta atttttttag gatgattttt tattattatc gttattatgg 15660
ttttaataat tttttttttt aaattttata tttttttattt tagtaattaa tgaggttgtt 15720
ttttgattta ggaggagatt tttttttttt agaatttaat gcgtagagtt tttgagaatt 15780
aaagtagttg gtaggggagg aagaaattaa tagaaaggga gagagtatat agaattgtgt 15840
gtgtatgtta aagagcgatt aggaatgata gagttaattt ttttgtgagg atttgacggg 15900
aagagtgttt aagattttat tagtatgttt ttaataggtt gatattttaa tttaaatttt 15960
tagaagtaat atattatttg ggttattata atgaggtggg tttttttttt tttgttagtt 16020
gatagttttt aaaatattat ttcgttaggg aaataaaagt tttattttag attataggtg 16080
ggtatttttg gatttaggtg atttatggtt attatgataa ttaatgttga atgttagtta 16140
ttagtatgtt tgggagagag aaaatagaaa gaagggagag taaaagaaat agaaaaggga 16200
gatggatata agttggagag ggaagaaaag agaaaaagag gaagatagat gagtgtttaa 16260
tttaattgtt gtttaaaaaa gtggcggggg ggtgggattt tatttagttt tttgttattt 16320
tttttttttt tgatttggat atttatgttt aattttatat tttatttttt tttttttttt 16380
tttaaatata tgtgttatat tattttttttt attttattta gttcggtaag tagttgtttt 16440
ttggagattt agtgatattt aggaaaattg tggtagtaat atgtaaatgt gaggaagtat 16500
taatagtatg ttcgttgagt gatttagtta aatgtttttt tttttaattt tttttttttt 16560
tttttttttag gttattgtga ggtggtaatt tttattgtta tttgaatatt gttttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740

agaaaataat ttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gagggggtgg ggtatttttc gggtaggatg taaggttttt atttgatttt tgcgttttat 16980
taggagttta tatatttacg tttattacgt ggttttaagt tgagtttagg cgggtttcgt 17040
ttttgagtta gtttgggtag ggtaggattt ttattcgttt aaggtttaat agtttaggga 17100
gatgtttaat taagttattt tttgggtgaa ttttgaagat agattttttt taaaagttag 17160
agattatttg gttgagtttt aggttagatt gatacggaga gtttggcggt atagtttaat 17220
cgtttattgt tatggttaga gggattttgt ataattaata ttgaagagtg tgaaattaaa 17280
taagatttta agattggtaa tcggtggtaa atattagtat aaaatacggt tgattttatg 17340
ttatatattt tttttttta gggtttttt ttgaaagaat aagtaagaaa ttttaatcga 17400
gataattttt gatgtttttt agatttaaaa ttttacgtcg gtattgggtt ttttttttt 17460
gttttacgtg agttatgtag tatttttagt ttttttatta ggattttatt aatgtttttc 17520
gtattggaaa tttttgtgtt agaggttgaa tttatagtaa tttttaaaat taattaagaa 17580
gaatttagtt agaggttata gtaatgttgg aattataaaa tgtataagat ttattttttt 17640
ttggtttttt ttttatttat gttgtttatg tttgtgtatt tataagtttt atgtatatta 17700
aatttttaaa attaattatt attatgttat agagttttta ttggatagtg ttttttagtt 17760
tttattatat atttttttt ttttatgtag atttattatg ttggtgtttt gttatatagg 17820
gggtttgaga agaatgttat ttaatcgtcg ttgttgtgag tgtgtaaagt gattaggaga 17880
ttaggagaat gttgaaattt ttgttggaaa aatgtaaaga aaatttttat tttgagttag 17940
ttgtttatag agttagtgtg tgtgtgcgtg tgtgtgtttg taatataaaa tggatgtgaa 18000
tatatatata taaatagata tggttttgtt tttattttaa tttgaattat ttagataatt 18060
gtttttattt attatttgat tttaatgggt ttatataaat taggatattt tattttttta 18120
ggtatttagg ttgttgttga tttttagtgt ttttaatatt tcgtatacgt tggtattatg 18180
aggagtagtt acgtgttttt gggtttttta attattttgg aggttgattg aggtttttta 18240
tatatgtata tttgtcgtga tgaaagtttt atcggtagag tggagttatt agagttttta 18300
ttaaaatttt gtgggtttat gagagatggg tttagaaatt tatatggttt tgtggggttt 18360
ttcggttttt taaaataagg tattaatatt taagttttta aaaatatttg tagttttggg 18420
gtttgaattt tgaaaaataa ggagtgaggg gttgtgtata ttaattatag tggagatttt 18480
ttttattttt taatgtgatg gagttttttt atgaaatgaa gttttaaggg gtatggtatt 18540
gtggggatta tagttatttt gaggtttaaa agaagaaatt ggaatatgat tagtaaatat 18600
atttagtaga aaagagttgg atttttattg atttagttat aggttatcgg ttggtagtgt 18660
aatgggagga aatatttatt ttatatatat attttatgat tttgggggaa ttagaggaaa 18720
tttaataaga aaacggttag aaatatttaa aatttttatt taaaagattt aagtaaatta 18780
gagttttatt agattaaaaa ttattataaa tgtaagagta ttgtttttag tgaaacgttg 18840
tggggtttga gaaggagatt tttcgttaaa ttttcgggat aaaatgcgtt atttaagtat 18900
tagataatga gtagaatgta aattaattta atttttttta ttaataggtt gttagtgtaa 18960
tgtgtataat ttagtgataa gattgtagga tttaatatag ttggatgtat gagttttagt 19020
taatgtagat ttgttatatg aggatgtgtt ttattttgag taggtgtttg tatgtgtgga 19080
atggggtaaa gtggaataaa aggttaaaag tagaaatgtt gatttaaagt ttattatgaa 19140
gaaatttttt ttttgtagtt aaattatttt taaagtggga tgatattggt gaagaaagat 19200
tgaaaaataa tttttatgtg cgtttttgga ttgtaagttt aaaatgggga ggagttgtag 19260
atagggtttg ggggtggtta gggtaaagga gagatatata agttgtaaat atatttgtag 19320
tttgttttat ttattttgtt ttatatcgaa taagtttttt aattttgtga ataaggataa 19380
ggagggagtg ttttaaagat attttatgtt ggtattgtaa attattgatt gtaatgttaa 19440
ataaatatat atttagagat gataatatta attttatagt aaaataatcg tttatgtaga 19500
aatttagagg agattagttt gtttttttta gttgatttat gttgggggat aaaaggattt 19560
ttaaaaatta ttttgaatat gtttggattt ttttttttaa tttttttgga aattaaattt 19620
gtttggaaat agtgttataa agagttgatg tttttaaagg tgattttttt tgttttatat 19680
aaataaggtt ttgtttttgt tagttgagcg tagttttagg tttttcgttt ttagtttata 19740
tatatttttt ttgtttgttt ggattttaat ggtttaagat agttttgagt ttattgggaa 19800
aagaaaatga ttgttaaaaa ttatttttga aattggttat ttggtaatat ttttaattgt 19860
atggaaattt attaaggtat atttatatata taattagttt aaggttgttg attttatagg 19920
ttttatggat ttaaatttga ttgataataa agtaaataag agagtcgaat ttaaagcgtg 19980
gtttttttcgg gttaggacga gtttaatata gtgtataagg aatttgaaag atttaggata 20040
tgtgttttaa ttaacgttaa gtagaatgga taagttttta gtattttgaa aacgttgggt 20100
tagggttttt tttttattgt gtgtttttttg tttggggatt aataagtatt atagagaacg 20160
tgatttgagg cgatttttta tttttgtata aatttagagt gaattattaa atagttgttc 20220
gtttaaagtt aaggtaattt ttttttgacg ggtttatttg tttttcgatt tttaatttat 20280
tagtttgttt ttttagggtt ttgttttttt tgtaattaaa gtttttttag attagcgtag 20340
tatttatttg ataggttgtt tggaaaattt aagatcggag aggtgatttg ttgttgtttt 20400
ttaaattttt tagttttaag taacgtgttt tttttttata tggggtgggg gattggaaat 20460
ggatgtagtg agatataaag agtgggtgtt ttgttgattt ttgtattttt ttttttttga 20520

ttattttatt ttttttttt aagttttcga tttttagttt tattttttta tttttgggtt 20580
cgtattaaaa gtcggatcgt tttgggttgg gtaggagttg aattttcggg agtttgtttg 20640
tgtagattta gtgcgtacgg cgaggtagta gttcggtttc gtattgttga taggtgtagg 20700
taggatagtt tttttatcgc ggttcggggc gttttgattg gtgcggagtt acgttagtcg 20760
tattcggaga agggtttggg aggaggcgga ggcggagagg gttggggagg gtcgcggcgg 20820
agtgacgttt cggtattagg aagttcgttt ttggttttaa gatgttaggt taatagggaa 20880
gcgcggagtc gtagatttgg ttcgtcgttc gtttgggtgt ttggagttga gttgcggtaa 20940
ggttcggttt ttgttcgatc gttcgagggg tgtgcgtgtg cgcgttgcgg agggtgcgtt 21000
tagagggtcg cgtcgtggtt gtagcggttg ttgtcgtcgt aggggattta atattattta 21060
tttgttttttg ttatttttga tattttttttg ttagggttgt cgcgtggggg gggggcgggt 21120
agagcgcggt cggcgttagt ttttttttatt ggaggggttt ttgggggagg gagggagaga 21180
agaagggggt ttttgtttat ttttgtttcg ttttggagtt tggaagtttg ttttttaaag 21240
acgttttgag tggtgttttt ttgtttatat tttatgtttt cgtttgttcg ttgatttttc 21300
gttttcggat tttttcgttt gagtttttcg gaggagacgg gggtagtttg gtttgagaat 21360
tcggcggggg ttgcgttttt tggtttttttt cgtagcgggg aaatttcgcg tttagagcgc 21420
gattcggagc gggtagcggc ggttacgggg gttcggcggg gtagtagtta aggattagta 21480
gagcgtcgcg ttttttcgtt tatgaattgt atgaaaggtt cgttttattt ggagtatcga 21540
gtagcgggga ttaagttgtc ggtcgttttt ttattttttt gttattattt ttagtcgtta 21600
gttatggttt cggttttggt tttcggttag tttcggtcgt tggatttttt taagtatagg 21660
ttggaggtgt atattatttt cgatattttt agttcggagg tcgtaggtaa ggcgtcgcgt 21720
cgttttgtag atattttcgt ttagttgttt tgcgttattc gttttttttc gttttaagga 21780
agttagtttt ttcgggggga ggcgtggtgg gagtggtcgt tcgtttggtt tttcgtagaa 21840
ttttcgggag tcggaatttt gattatttcg tattttttta gttttttttc gatcggttcg 21900
gtttttgggg cgttaagggc gcgagtaatt ttgtcgtttt ttttattcgt attttggttt 21960
tttttttgtt ttttgggtta taaaaatttt agtattttga ttcgaggatt tttagaggtc 22020
gtcgattttt gtttttgttt ttttttcggt ttttagtttt cgaggagttt tattcgttag 22080
gaaattgttt gaaattattt agaaatgttt ttcgcgaaga ggtatttttt ttttttttt 22140
gggaaagggt cggcgaattt cggtgtttaa tcgaattttt atattttttt ttagtttttt 22200
taaatcgtat ggaaatttga gtttttttgcg agggggaggg gggtttgtaa attacgcgcg 22260
tgtgcgcgtt ttaggagatt tggtgtgttt gcgtagaggt gtataaatat atttgaaagt 22320
ataggttata aaagtgaatg tgtcgttgta gtgagataaa tatgtaaata aaacgtgcgg 22380
cgttgggggga ggggaggaaa tggggcgcgg atatttatat ttgcgtttgt atattttata 22440
ggcgtagcgt ttttcgcggt tcggagtcgt cgcgcgtatt tttttttcggc gttaggtagt 22500
ttagtttttt tacggttttt gtcgtcggtt tagttggcgt tcgcgttgta ggtgggtatg 22560
ttgacgggaa agtgtgtgtg tttcgttttt agagaaagat aaaagttagt aggggaagaa 22620
tgaggacgtg ggcgtcgagg attcgtttaa gaagaagcgg taaaggcggt agcggattta 22680
ttttattagt tagtagtttt aggagttgga ggttattttt tagaggaatc gttattcgga 22740
tatgtttata cgcgaagaaa tcgttgtgtg gattaatttt acggaagttc gagttcgggt 22800
aggagttagt acggagtttg ggagggatgg ggggaggatg ttgtggaggt ataggttaag 22860
tagattagga gagaatgtgg aaggtagcgt cgtttgggag ggcgtcggtg gggcgtagtt 22920
ttgtaaaggt agaaggtttc gcggcggttt ggttgcgaga ttatagtttt tttttcgagg 22980
tcgataggat tgtcgttttg gtttaggttt ttagagcggt atcggtttat tgtttcgtta 23040
tttcgcgatt ttacgagttg ggttgtatgg gtaatttttt gtataggata ttgtgttttt 23100
ggtttgtagt tgttagagta gagttaataa aatttttatt aggttaagag tcgcgaatag 23160
gttttaattt gtgagttttt aataaggaaa attcgttaga gatacggaag agttggtttt 23220
ttttgggaaa tttttgtttc ggttttggtt tagttttttt ttttttgggt tcgcgttttt 23280
tatatttttt ttacggttgt ttcggttatt taggtttttt ttatatattt tatttttttag 23340
ttttgtgatt ttcgggagta aagttttaat atataattat tagttttttt agaaggagaa 23400
agaaaaaaag aagaaagatt tttttgtttg gtttatttat ttttttttag gagttgaatt 23460
ttggaaattg aaatttatat ttttttttt aaattataat tatagtttg taaaaagggt 23520
ttattttaat tttgtagtaa atttgtattt tatggattgg taaaaatgag tttaaataaa 23580
taatttaata gtaacgtttt ggtttatgtt ggtcggtgga agattttaaa tttgttagga 23640
ttttggaagt agaaaataga attaagtaaa ttaagcggta tttagaggtt ttgttgttaa 23700
aaaaaaaaaa ttaagtgttt tgggtagaaa aaataaagtt ttcggttaga gtagagtaaa 23760
taaaaagaag aaaataacga taaaaagaat aaagattaaa atgttttttt aaattagagg 23820
gaatgaagat attttttggg tggtatttgt gtaaggtatg aggttatgtt ggtggataaa 23880
aggtcgggaa gaagttgaaa atggttttag tttaattgtt tagagttaga gttgggtttt 23940
gggcggcgtg gttttgagta aggttagttt tttattagtt tttttgtata ttaagggaac 24000
gggtttttta cgtatttttt tcgtttgagt aaagtttaga tggtttaggg tagaaatggt 24060
aagtaattaa agatagagtt tatgggtttt ttgggatttt tcgaaaacgt ttttttattt 24120
cgttcgttat ttcgtagttt tattttagtg ttttgtagtc gcggcgttgg gtttttttttg 24180
tagttgtttt tttttttagg gcggttgttt gtcgagttaa gtgggagtga ggcgtgtttt 24240
ttatagtagt cgggtgtaaa gaggaagggg gataaaaagg aaattaagaa tgaaaggaaa 24300

```
aagagaaaaa gcggattata cggttgggtt cggcggagat gtgtaatgtg aaatattatt   24360
ggtgttagtt cggatatttt aggttaggtt ttttttaat atataaaagt cgtcgtttgg   24420
ggcgataggg aggttcgatg tggattggga tcggggttgc ggttgggtta tcggatacgg   24480
gtggaagtcg gtcggtttgg gtggtcgttt gtaaagttaa acgattcggt tgggtttggc   24540
gcgcggatag gtttgtggtg ggtttagggt aaagaagagg tagagcgaaa gaagggggaa   24600
tttttaaaat tattttttc gggttttcgg agtttaatat gttaagtttt tggagttaac   24660
gagttgacga agaggtggtt ttttgttttt tatttggttg ttttgttagg cgagaaagag   24720
tgttggcggt ttagtttttg ttaagggagt acgtattagg gggtggggga cgatagtgga   24780
ggttagggaa ggaagggagg aattgcgtgg gagaaagagc gattttttag tgttttttta   24840
gtttttttttt tttattcgtg ggtttgtggt tttggaatgg aagtaagttt gtaaggtgtt   24900
tcgggaaggg ttggaaaagt ttgttgtttc gcgtttgttt tatattaagt gtttttggat   24960
ttggagaaac gtttggttga gtgattaaat cgttcgtagg tttttatgcg ttcggttgag   25020
gtttgtggcg tagtttcgag ttttagttcg taggttagag tagattaggt tttttgcgtt   25080
tggtggagat tcgggttagt aattgaaagt tggttttggt attttggtgt gtagggcggt   25140
gtagtgaagc gaggttaggg tgtgtgagtg cgttagcgtg tgtgtcgggg gaaggcgggg   25200
gttggttttc gatggaagtt ttagtaattt gtattgtggt atttgtttgt ttttttgttt   25260
taatcgtttt taggtttggt ttaagaatcg tcgggttaaa tggagaaaga gggagcgtaa   25320
ttagtaggtc gagttatgta agaatggttt cgggtcgtag tttaatgggt ttatgtagtt   25380
ttacgacgat atgtatttag gttattttta taataattgg gtcgttaagg gttttatatt   25440
cgttttttta tttattaaga gttttttttt ttttaatttt atgaacgtta atttttttgtt   25500
attatagagt atgttttttt tatttaattt tatttcgttt atgagtatgt cgtttagtat   25560
ggtgttttta gtagtgatag gcgtttcggg ttttagtttt aatagtttga ataatttgaa   25620
taatttgagt agttcgtcgt tgaatttcgc ggtgtcgacg tttgtttgtt tttacgcgtc   25680
gtcgattttt tcgtatgttt atagggatac gtgtaattcg agtttggtta gtttgagatt   25740
gaaagtaaag tagtatttta gtttcggtta cgttagcgtg tagaattcgg tttttaattt   25800
gagtgtttgt tagtatgtag tggatcggtt cgtgtgagtc gtatttatag cgtcgggatt   25860
ttaggatttt gtcggatggg gtaatttcgt ttttgaaaga ttgggaatta tgttagaagg   25920
tcgtgggtat taaagaaagg gagagaaaga gaagttatat agagaaaagg aaattattga   25980
attaaagaga gagttttttt gattttaaag ggatgttttt agtgtttgat atttttttatt   26040
ataagtattt ttaatagttg taaggatata tatataaata aatgtttgat tggatatgat   26100
attttaatat tattataagt ttgttatttt ttaagtttag tattgttaat atttaaatga   26160
ttgaaaggat gtatatatat cgaaatgtta aattaatttt ataaaagtag ttgttagtaa   26220
tattataata gtgtttttaa aggttaggtt ttaaaataaa gtatgttata tagaagcgat   26280
taggattttt cgtttgcgag taagggagtg tatatattaa atgttatatt gtatgttttt   26340
aatatattat tattattata aaaaatgtgt gaatattagt tttagaatag ttttttttggt   26400
ggatgtaatg atgtttttga aattgttatg tataatttat tttgtgtata atatttcgta   26460
taatattatt gttttatttt ttagtaaata tgaaataaat gtgttttatt ttatgggagt   26520
aaaatatatt gtatataaat tggtttggat ttttttttt ttttttttgtt attaatttgg   26580
ttaggatatt ttagttattg ttttttaaat aaattagttt tttttgtttg tttagttaaa   26640
tatataaggt agtagttttt atttaaattt ggtagaaata aatgatagtt atttattaga   26700
aattaaaaag aaaaaaaaag gtattttcgg gggggaaaag ggttataaaa tttaattttg   26760
ttttttttaat tttttttttgg tttaaattta gaggatttta ttatggttag taaataatat   26820
gaaaaagaaa aaagaagaaa gaaatttagt aagtttatta gtttaaaatg atttttaagt   26880
ttattttttt acggggaaat ttatattttt agtaaattgt tttggagaaa tatttgtgta   26940
tgtatatatg tatagtttat atgtattttt tttaggagga atatatttat aataaattta   27000
tagggaaata tttttagttt aaaatattta ggtttttacg tttattttta ggtttaagta   27060
gagagatttt ttatgttata ttgtattatt atttttaaat tttttggaga tattaaaaga   27120
aataaagatg atttttaata attatagttt tttagttttt taaagaattt aggggttgag   27180
aggttagagt ggagtttttt gagttttgtc gagtaatatg tagttgaggt aaaggttatg   27240
ttttcggtgt tttgttttaa ataatattga tttattaatt ttaaatttgt ttgtttttga   27300
aattatatag gattatagtt tgtaaattgt aggataatga agtaaattaa gatgaattat   27360
agttttggtt ttttttgtta tttttttgata tttaaatagg gaatgagttc ggtgtgagtg   27420
tttaaatgaa ttttaagtat tcgatttttt tttattcgcg atttttagtt ttaaaaaaat   27480
gtgaaatttg attttataat aaatagaaat aaatattatt tagttttaga gaatttattt   27540
ttatggcgtt aggagggtcg ttgtggaggt gggggaggg atgtgttgag atttttttgtt   27600
atgtttgtta atttttcgta taattaaagt gggcgagaat aaatattacg ttggggaatt   27660
tagagtaaaa agtaatcgtc gattttttgg agtcgataat attattgttt tttcgtttta   27720
gttgttttta tttgaatgaa attttattta gaagttttttg gagttcgaat attgagtttt   27780
tgttttgtaa gaaattgttg ttgttattta aagagatcgt agataatgtt ttcgatttaa   27840
gattagtgtt taacgtatat ttttttttt tttaaagttt tgttttcgat ttgcggaggg   27900
attacgtagt agtgggggggt agataaaagt tttttggacg agggttattt tttattatgt   27960
tgtttatttg agagtagtgg agaggaaaac ggtttttacg ggcggatttt ggtttttgga   28020
gtcgtagggt ttagcggttt cggtttttttc gtttttttagt ttggtagggg gcggggaggg   28080
```

```
ttaaagggcg gaggggaagg aaggagttag aagaggggat ttggggatgg gggttggaag   28140
cgttaacgag atttgttcgg aggatttagg ttttttgtag gttggtgagt gatttgggag   28200
ttttgggtaa aagaggtgta tttcggttta gtttggttgt tgaattagaa taacgcgagg   28260
atattaatta atttgtagga aataaaaaat ggaagtcgag gtttaggaag agttgttatt   28320
ttttcgattt gagtggtgta ggttgggggc ggagatttgg gatttaagag aggttatttt   28380
ttttatttta gttttttttt tttggatttt taaaaggaat aattttattt ttttttttcgt   28440
tttttttata atttttattt tcgttagttc gtaggtcgtt tgtttttttt cgtatttttt   28500
ttttttattt agcgagagaa atttagtttt tagagt                             28536
```

<210> 153
<211> 8252
<212> DNA
<213> Artificial Sequence

<220>
<223> chemically treated genomic DNA (Homo sapiens)

<400> 153

gttatcgtgg ttgggttata ggtttattgt tttgtggtag atggaagata tgggttttgg 60
taatatattg aaatgttata ggaaattaaa ttagaagtga tttaatttat aagtatattt 120
aggtttattt aatatgatgt tttaaggtgg gtgggggtt taaattagta gtttagggtt 180
attaaggttt taggttttgg gtttatttt attattatat aatggttgtt tgtaatatta 240
tattatattt attttttata atatttaaag gtaggttgta agggttggtt taggaagtta 300
gagggcgttt tttatttatt aagggagaag tttgttttat ttttaaggag atgtttttta 360
tgttttatgg gttatataga attgtacggt tatttttggt taaaagaggg gttgggaaag 420
tgaatttttt tttttttttt tttcgtagag tgtaagttag gtaagaaaga acggatggtg 480
gatagttagg aaatagttag ttacgagatg ttgtgtggaa agtttatttt tttgatattg 540
gagaattgtg ggaagaacgg ataggttata tattagttgg gttttgagaa tttttagttt 600
ttgttttata gtgagttagt aggaatttaa agggtatggg tagattttag gggttttttt 660
attttattta ggggtagaga gggtaagacg gagtagagcg aggtaagaga ttgagtaatg 720
cgtttttaaa gattgtgatt gatagaagtt gtgtaaatta ttatgataaa ttgaatttta 780
agaggaattg gattaaataa agttagttta ttttatttg taattagtag atagagagtg 840
taagattaga tatattatag atggaaaaga aaagttaaaa tttttttgta tatttgagag 900
gtaagaggag gtgtttgtgg tttttttgat atatacgtga tatatgtatt attacggttt 960
atgtgtgtgt tgttgtgtgt aggggggtagg gggtttttcg tttttgatgg ttttttttgg 1020
tgggtgtttt tcggtttttt aggtttttttt atttgttgaa cgtggtagtt aagggtttgg 1080
gaatgatttg ttagattggt taatgtggaa agagttggtt tcgtttagtt ttgtattttt 1140
tttttttttt ttggaatcgt ttggttgtag agagtaggag tttttagag ttttcggggg 1200
attttatttt tagtataatt gtgtaacgtg tgtttagtat aagagtatta gatggaggga 1260
taggtgggag gaggaggaat ttagggtgta tttagtttgg ggtttgttga gttgaggttt 1320
ttagagtaag gacgattagg ttggggggat attttttttt aatttttgtt aagttataga 1380
ggggttagtg ggaataaagt gatattttgg tttttgtatt tttttttgt tgttttattt 1440
tttagttcgt ttagatttga ggttggggag gggttttttt ggaggggtta gtttatgtta 1500
tttttgtaa tttttattat atatgtttt agatattttg ttataggttt tattttttac 1560
gttaattttt atttgggaaa gtaaataaac ggaaagttaa tttgtgttat ttggtcgttg 1620
gggtattcgt agtgagtagt taagaaatgt taggggagtc ggtgtttatt tttatgtttg 1680
gataagagtg cgttttggat ttgcgttttg ttgtatattt tatcgtttcg ttgtatattt 1740
tatcgtttta ttgtatattt tattgtttta ttgtatattt tatcgtttta ttgtatattt 1800
tattgtttta ttgtatattt tattgtttta ttgtatattt tattattttt gtttttgta 1860
tttagttgtt tatttgtatt ttagtttagg aagtttagaa gatgtagaat ttttgcgaga 1920
gtttagggtg aaacgttgcg ttttattttt aaagaaagga aaattattta tatttttaa 1980
aagaatgaat agtatagatt aatatcgatt ttttttaatt tttaggttaa ttttgagtag 2040
ttaaagttag agtagttaat ttgtgttgtg agtcgaggta tagttgtaga agcgtgtttg 2100
aggtgttcgg tggaggtggt agtcgagttt tgggattaat tatcgtgttg gggacggtat 2160
cgcgttagga tgtaggtaga tttttgtaga agtgtttaaa atttatattt ttttataggg 2220
gtgaggggga gggagaaaga gatgttttag tgaggataaa tatttttttt tatatttaaa 2280
taattataga gtttttattt taaagtattt ataggtatat ttttagaaa atatgaattg 2340
ttagtcgggt acggtggttt acgtttgtaa ttttagtttt ttgggaggtt gaggcgggta 2400
gattatttga ggttaagagt ttaagatcgg tttggttaat atggtgaaat ttcgtttata 2460
ttaaaaatat aaaaaaaatt tagttgggcg tagtggtata tatttgtaat tttagttatt 2520
aggaagttga ggttgaattt aggaggtaga gattgtagtg agttaagatc gtattattgt 2580
attttagttt aggggtaacg gagcgagatt ttattttaaa aaaaaaaaa aaaaaagaat 2640

atatgaaatg tttttagatt tcgttatgtt tttttttttt attttaggta agttagaaag 2700
cgttattaat agtggttttt tttaggtttt tggttagaga tgtgaagaga agtcgggggg 2760
aaattaggtt ttttttaag ttttttagtt ttgtttttt atttttggat ttgaatgtag 2820
tttgatttag gttattttat tgtattatta ttggcggtcg tgattttgtg taaaggtata 2880
gttggtgatg ttgattagag tttttgtagt tttaaatgat ttttttaatt aattttaaat 2940
ttttagaatt tatcgtataa aaaggttata tttttttggag ggacgtcgat ggtattagga 3000
tagaagtatt aggggatttt acgaacggtg tcgtcgaaat agtagttttt atttgtatat 3060
tgggagggcg tgatattagg aaaattataa ttttgttttt tacgggggggt tattgtatac 3120
gtttttgaaa gtgtataggt aagaagtaaa gtaagttgtg ggttgaattt tttgatgtta 3180
ttatgtatat atttatttag tttttttttt taatgatatt agtaattgtt tagtgaggcg 3240
gatataaaat ttttaggata tgagagggag acgtggtttt tatattttga tgtgtaaata 3300
ttacgtttag ggaaaatgta aggtgtttta ggtttgtgga ttttgtattt ttttaggtaa 3360
tttatttatt tattttttaa ttttaataaa tgattattaa atttatttta atatataaat 3420
atttattgag tattatttgt gtgtatgaga agtgggagtt agtatggtaa aagttaggta 3480
ttgtgttagg tgagagagat ttagaaatta aaattagaga agttattaat aagagtttaa 3540
atatttgggt ttaggtttat gtttgtaatt ttagtatttt gggaggttga aggaggtgaa 3600
ttatttgagg ttaggagttt aagattagtt tgattaaaat ggtgaagttt tatttttatt 3660
aaaaatataa aaaattaggc gggtattgtg gtatacgttt gtaattttag ttatttggga 3720
ggttgaggta ggagaattat ttgaatttag gaggtagagg ttgtagtgag ttaagatcgt 3780
attattgtat tttagtttga gtgatagagt aagattttat tttaaaaaaa aaaaaaaaag 3840
agtttaagga tttgatggag gagaaaggta agaatatgtg cgagataacg taaggttatc 3900
gtttagggt gtttagggta attacggggg tagggtattt ttcggagagg ttaatgataa 3960
gtaggttgaa taaagtaggg gggttttttg taggaggagg tttattaggt gaagatggag 4020
tcgtatgggt aaaggttatt ttagagattt aggtgtgttt aggaggtgga aatttattgt 4080
aggtaaggtg agaggatcgg gtggggtggt ttaggaggag tcgatagagg gtataagttg 4140
tgaaatagtt tgaagtaggg tagtgaggaa agggatttag aggaggaaga tacgtggata 4200
gatggggttg gttgggggtt gtcgtaggat tttatgtaag aggttttaat attagagttt 4260
taggttttga gtttcgcgga attaaaggtt ttagaaagta atttattagg atttggtggt 4320
tgatagtttg tagtaggggg tgaaagagga gtttagagta tttttcggtt tttgtttttg 4380
ttttggggta taggagggga ggaatttagt ttggttatat tggtttaggt gagggcgttt 4440
taggggaggt cgagaggggt tgttttttttg tttgttttgt tattagggat tgtttcgaga 4500
tgtttttgga gaaagtgttt ttggtttgtt gggaaggatt cgtgtttagt tttcgttgtt 4560
tagtagtttt tatgggaatt ttgttttttt ggggtttgga tttatcgcga cgtgaaggtg 4620
attatcgttt atttcggga tattgtgggg gttaagagag gtttcgtggt gagggaggat 4680
tattattttg ggggtcgggg gggttttttt tttagggagg aagattttta gtttcggtgt 4740
tttgttttcg gtagattttg ttttgtttat tcgttttgtt tttgtcgtat gatggaaata 4800
aatggaaatg gtttttatat acgaatgtat taaattgtaa ttataatttt ttagatttta 4860
gttgtaatag gattatggat ttgagtgatt cgtaaagacg ttttgagttt tgagttagag 4920
ggtgtgtggg gtggggaggg gagtttgtta cggtttttgt gattttatag tatagggggt 4980
agagttgggg gttggggtgg gggtaagggc gtaggtagat gggtttgggg gtggttagta 5040
cggggattta tttagttcgt tttgtatatc gaggttattt tttttttcgg gttttaaagt 5100
tttttcgttt tttgttatgg gtttgggggt tttttattg tagtttaatg ttggttgttt 5160
ttttacgttg tttaagttta tttttttaggt tcggtatttt atagtagaga ttaagaggtg 5220
gttggagggt agtgggggta cggatagtat tattgggcgt ttttttttta ggtttttttg 5280
gaaattttg ttttggaaac gtagaaagtt tttttttttg tttttatttt gaaattgtat 5340
ttaataacgg taaataagtt atttagtttt ttatagttgg taaagtgtaa gtcgtagatg 5400
ttgttatata gtatttttttg atagttttag ggtagatcgt tgatttattt tgtaggtaaa 5460
ggagttgaga tgtaaatatt ttttaaggaa gtaagaatgg tttttttttt tatttttttag 5520
aaggatttag ggtaatcgat ttatttgata atattaggga atatgggttg ttagttacgt 5580
atttatgaga gaggtggttt tgatttttag agtatggatt ttaggggagt ttaggaattt 5640
gtaggagagg gtttttttagt tggttattat tgggacgggt attcgggttt gtgggagagg 5700
gtttttttagg tggttattat tgggatcggt atttgggttt atttggatgg gttttgggag 5760
ggtttgtttt tgggggagat gttggtatga atagttaaaa gtattatttt gagattttac 5820
gttaatattt ttttttttgaa atatgatatt tgggaggatt gtatagtttt tttaatatag 5880
gaaaatagtt tcgattgaag gtagtttttt tttcgtcgta tttttcgaag gtttcgggg 5940
gttttgttat tttgagttat ttttatttg tttttgtcga ttttgtaaat tggatatatt 6000
tttaagggtt tagaatagta tttggtataa aataggtgtt taaaaatatg tatgtaaaat 6060
agtggttttt ggcggaggtt gttagagttg gttgtggttt tatagagtag gaagaagtac 6120
gttttatttg tttgggtttc ggttagggtg tcgagggtta gtatggatat taggattagg 6180
gcgtagatta ttttgttttt tatggtggtt attgtttttt ttttgtttta aaggcgattt 6240
cgagttaggg atgagaggtc gttcgagttt cggattttat agggtaggtt ttgtttgttt 6300
aaagagcgtt agataatatt tgtttaagga ggttcgggga ttttttgaga taataatttt 6360
tattgatttt tattaaaggt gtttttttaga tatggttaag ttatatggaa ggatttgttg 6420

```
atagatagag acgatatgtg gtgaggttat tttggttgag ggatttgaga tttagaaagt    6480
ttttttttt tatgggagtt tttttaatt tgattttaat ttaggtttta tttatatttg    6540
agaggttttt tcgttagggt aaatattgta tttattgtag aagtgattta tagtgagaga    6600
tggtcggaaa aaggtttggt cgtgataata gtggtttacg ggtggttat cgtgattttg    6660
tagggggaag ggaaggagtt tatgaagttt atttcgttta ggtttaagtt aattttttta    6720
tagtggaatt gttttaataa tgaaattgta ggtttggcgt agtggtttac gtttgtaatt    6780
ttaatatttt gggaggttaa agtaggcgga ttatttaaag ttaggagttt gagattagtt    6840
tggttaatat ggcgaaattt cgtttttata aaaaaaaaaa atataaaaat tagttaggta    6900
tggtggcggg tgtttgtaat tttagttatt ttggaggtta agttaggaga attatttgaa    6960
tttgggaggc ggaggttgta gtgagtcgag attattttat tgtattttag tttgagcgat    7020
agattaagtt tttttttggg aggggttggg ggtaggaggg agaaaaaaat agtatatacg    7080
agttgttatt aaaaattgat aaagtgaacg tggtttattt ttttgtgttt atggggtttt    7140
ttttttttt tttttatatt tttttagttg gttttttgga tttttgtatt ttagaggata    7200
gtttttttgtt tttttgtatt atgttttatt ttttttttt gttcggagtt ttttttttta    7260
tagtttttat tattttttgg tggataaaat aagtgattga tatattatgt tggttattta    7320
tttattgtat ttttttttgt agtagacggg agggcgtatg ggagtagaag tttttatttt    7380
ttagtgtttt gaggtggttt tagattttag aatagtggtt ttgattggta ttcgagaaat    7440
agttgttgaa gagttgtatg aagaaatgga taataggaga aattttttttt tttttagtga    7500
gaaattaggt tttgaggaaa atgtatgtta gtttaataaa ggtattattt ttttttgggt    7560
gatttagtta gtatattttt gtagtttttt tgggagggga gtttgggtta gaatgggggc    7620
ggttatgttt tttttcgttt tattttagga tttagttttt tttttagggt tgttattttt    7680
gtatcggggt ttttattttt gggttagggt ttttaggagg ggtttgatcg taggatttag    7740
ggaggggtcg aggtttggtg tttttttgtgg ttagtttaag tttattgaaa ataggacggg    7800
ggtattaggt ttagggtggt gtttatagtg acggtggttt tcgttgtttt aatttttcgg    7860
attaaaatgg gggtttttgg aaatgggatg taaattgtat aattatttgg agaaaaggtg    7920
ggtagtgttc ggtgaggttt aagttatata gattttatag tttagtaaat tttttttgttt    7980
ttaggtttgg ttttttaatt ttagaaatat gtttatattt gggtagaaga aaatataggt    8040
aaagatattt tttttagtat tgtttgtaat agttggaaat agggaaagaa ggaaggaagg    8100
aaaggaggga aagaaaagta aaataaatat ttattaatta gggaaaggga aaatgaattt    8160
aaaatataat gtagtcgttt tttagtattt ttgggggatt ggttttagga tttttttattt    8220
agggatgtta aaatttataa atgtttaagt tt                                  8252
```

<210> 154
<211> 8252
<212> DNA
<213> Artificial Sequence

<220>
<223> chemically treated genomic DNA (Homo sapiens)

<400> 154

```
ggatttgagt atttgtggat tttggtattt ttgggtgggg ggttttgaaa ttagtttttt      60
aggggtatta agggacgatt gtattgtatt ttaagtttat tttttttttt tttggttaat     120
gaatatttat tttgtttttt tttttttttt tttttttttt tttttttttt ttgtttttag     180
ttgttataaa taatgttaaa aagaatattt ttgtttgtgt tttttttgt ttaagtatga     240
atatgttttt gggattaaga gattaggttt ggaaatagaa gaatttgttg agttgtaaaa     300
tttgtatggt ttaaatttta tcgaatattg tttatttttt ttttaaatga ttgtgtaatt     360
tatattttat ttttaagagt ttttattttg attcgagaaa ttagagtagc gaaaattatc     420
gttattatga gtattatttt gggtttggtg ttttcgtttt gtttttaata aatttaagtt     480
gattatagag gatattaggt ttcggttttt ttttgggttt tgcggttagg tttttttttgg     540
agattttggt ttaggagtgg agatttcggt gtaggagtgg tagttttgga ggaggggttg     600
ggttttggga tggagcgaag aggaatatgg tcgtttttat tttggtttag gttttttttt     660
tagaggggtt gtagaaatgt attgattagg ttatttaaga aaagatagta tttttgttag     720
gttagtatgt attttttttta gggtttaatt ttttattgaa gaagaaaaga ttttttttgt     780
tgtttatttt tttatgtagt tttttaatag ttgtttttcg aatgttaatt aaagttattg     840
ttttagggtt tggggttatt ttaaggtatt aggagatgag gatttttgtt tttatgcgtt     900
ttttcgtttg ttgtagggag gagtgtaatg aataaataat taatataatg tgttagttat     960
ttgttttatt tattaggagg taataagagt tatgaaagag aaagtttcga gtaggggagg    1020
ggagtgaggt atggtatagg agagtaggag gttgtttttt aaaatatagg agtttagggg    1080
attaattggg aaggtgtggg agggggaggg agggagtttt atagatatag gggagtgaat    1140
tacgtttatt ttgttagttt ttgatggtag ttcgtatata ttattttttt tttttttttg    1200
tttttagttt tttttagaag gagatttaat ttgtcgttta ggttggagtg tagtagggtg    1260
```

atttcgattt attgtaattt tcgtttttta ggtttaagtg attttttga tttaattttt 1320
agagtagtta ggattatagg tattcgttat tatgtttggt taatttttgt atttttttt 1380
tttgtagaga cggggtttcg ttatgttggt taggttagtt ttaaattttt gattttaagt 1440
gattcgtttg ttttggtttt ttaaagtgtt gggattatag gcgtgagtta ttgcgttagg 1500
tttataattt tattattaaa ataattttat tgtaaaagaa ttagtttagg tttagacgga 1560
atgggtttta tgagtttttt ttttttttt tgtaaggtta cggtggttat ttcgtgagtt 1620
attgttgtta cggttaagtt ttttttcggt tatttttat tatgaattat ttttgtagtg 1680
agtatagtat ttattttggc gggagggttt tttagatatg agtaggattt ggattaaggt 1740
taggttggag gagattttta tgggaaagag ggattttttg aattttagat tttttagtta 1800
agatgatttt attatatgtc gtttttgttt attagtaaat tttttatgt agtttgatta 1860
tgtttaggaa atatttttga taaaaattag tggagattat tgttttagag gattttcggg 1920
ttttttagg taaatgttat ttaacgtttt ttaagtaaat agagtttgtt ttataaaatt 1980
cggggttcgg gcggtttttt atttttgatt cggggtcgtt tttggagtag agaggaggta 2040
atggttatta tggagaataa ggtgatttgc gttttggttt tggtgtttat gttggttttc 2100
ggtattttgg tcgaggttta gataggtaag gcgtgttttt ttttgttttg tggggttata 2160
gttagttttg gtagttttcg ttaggagtta ttgttttata tatatatttt tgagtatttg 2220
ttttgtgtta ggtgttgttt taggttttta aaagtatatt taatttatag gatcggtaaa 2280
agtaggtgga gagtaattta gggtggtagg gttttcggag attttcgaga agtgcgacga 2340
ggaggggggtt gtttttagtc ggggttgttt ttttgtgtta ggaagattat ataatttttt 2400
taagtgttat gttttaaaga ggaagtgttg gcgtggggtt ttagaatagt gtttttgatt 2460
gtttatgtta atattttttt taggggtaga ttttttaag gtttatttag ataggtttaa 2520
atgtcggttt tagtgatggt tatttgggag attttttttt ataggttcga atgttcgttt 2580
tagtggtggt taattgggag attttttttt ataggttttt gggtttttt gggatttatg 2640
ttttgggagt taaagttatt tttttatga gtgcgtggtt ggtaatttat atttttttggt 2700
gttgttaagt ggatcggttg ttttgggttt ttttagggag tggaggagga ggttattttt 2760
gtttttttgg gaagtgtttg tattttaatt tttttatttg tagaatggat taacggtttg 2820
ttttagggtt gttaggaaat gttgtgtggt agtatttgcg atttgtattt tgttagttgt 2880
ggggagttga ataatttatt tgtcgttatt aggtatagtt ttaaggtggg ggtaggagaa 2940
agggtttttt acgtttttaa agtaagggtt tttagagagg tttgaagagg gagcgtttag 3000
tggtgttgtt cgtgttttta ttgtttttta gttatttttt gatttttgtt gtggggtatc 3060
gggtttgagg ggtgggtttg ggtagcgtag aagagtagtt agtattgggt tgtagtggga 3120
agatttttaa gtttatggta gggagcgggg gagttttgga attcgagaga ggaagtggtt 3180
tcggtgtata gaacgaattg ggtgggtttt cgtgttggtt atttttaggt ttatttgttt 3240
gcgtttttgt ttttatttta gtttttagtt ttgtttttttg tgttgtggga ttatagaggt 3300
cgtggtaaat ttttttttt atttatata tttttttggtt taaggtttag agcgtttttg 3360
cgggttattt aggtttatga ttttgttata attgaaattt agaaaattgt gattatagtt 3420
tagtgtattc gtgtgtggaa attattttta tttattttta ttatgcgata aagataaagc 3480
gggtgggtaa gatagagttt gtcggaggta gagtatcggg gttggaaatt tttttttttg 3540
aggaggaaat tttttcgatt tttaggatga tgattttttt ttattacggg gttttttttg 3600
atttttatag tgtttcgggg gtgggcgatg attattttta cgtcgcgatg gatttagatt 3660
ttaggagggt aaggttttta tggaagttgt tgggtagcgg gagttgaata cggatttttt 3720
ttagtaagtt aggaatattt tttttaaaga tatttcgagg tagtttttga tagtaaagta 3780
gataagagaa tagtttttttt cggtttttttt tggggcgttt ttatttgagt tagtgtggtt 3840
agattgagtt ttttttttt tatgttttaa ggtagggata gggatcggag ggtgttttgg 3900
gtttttttttt tatttttttgt tgtaggttgt taattattag atttaatag gttgtttttt 3960
gagattttttg atttcgcgga gtttagagtt tgaagttttg gtgttagaat tttttgtata 4020
agattttgcg gtagttttta gttagtttta tttgtttacg tgtttttttt ttttagattt 4080
ttttttttat tgttttgttt taagttgttt tatagtttgt attttttgtc ggtttttttt 4140
agattatttt attcggtttt tttattttat ttgtaatggg tttttatttt ttgaatatat 4200
ttgggttttt ggaatggttt ttgtttatgc ggtttttatt ttatttggtg aatttttttt 4260
tgtagggagt tttttttgttt tgtttaattt gtttgttatt ggtttttcg gggagtgttt 4320
tattttcgtg gttattttgg gtattttggg acgatggttt tgcgttgttt cgtatatgtt 4380
tttgtttttt tttttatta gattttaga ttttttttt ttttttttg agatggagtt 4440
ttgttttgtt atttaggttg gagtgtaatg gtgcgatttt ggtttattat aatttttgtt 4500
ttttgggttt aagtgatttt tttgttttag ttttttaagt agttgggatt atagacgtgt 4560
gttataatgt tcgtttaatt ttttgtattt ttagtagaga tggggtttta ttattttggt 4620
taggttggtt ttgaattttt gattttaagt gatttatttt ttttagtttt ttaaagtgtt 4680
gggattatag gtatgagttt gggtttagat atttagattt ttattaatga ttttttttggt 4740
tttaattttt gggttttttt tatttggtat agtgtttggt ttttgttatg ttagttttta 4800
tttttatgt atataaatgg tgtttagtaa atatttatgt attgagtaaa atttaataat 4860
tatttgttga aattaaaaag tgaataaata agttatttag aaagatgtaa agtttataaa 4920
tttggggtat tttgtatttt ttttgagcgt aatgtttgta tattaggatg tgaggattac 4980
gtttttttttt tatgttttga gggttttata ttcgttttat tggatagttg ttgatgttat 5040

```
tggagaagga agttggatgg gtgtgtgtat gataatatta aggaatttag tttataattt 5100
attttgtttt ttatttgtgt atttttagag acgtgtatag tggtttttcg tgaaagatag 5160
aattgtggtt tttttggtgt tacgtttttt tagtgtgtaa ataagggttg ttgtttcgac 5220
gatatcgttc gtggggtttt ttggtgtttt tattttaata ttatcgacgt tttttttagaa 5280
ggtatggttt ttttatacga tgggttttga agatttagaa ttagttagaa aagttattta 5340
agattataga ggttttgatt agtattatta gttatgtttt tatatagagt tacggtcgtt 5400
agtggtggtg taatggggta gtttgagtta ggttgtattt aggtttagga atagaaaggt 5460
agggttaagg gatttgggaa gaaatttgat ttttttttcgg tttttttttta tatttttaat 5520
taaaagtttg ggaagagtta ttgttggtaa cgtttttttag tttgtttagg atagaggggg 5580
aaggtatgac gaaatttgaa gatattttat gtattttttt tttttttttt tttttgaaat 5640
ggagtttcgt ttcgttgttt ttgagttgga gtgtaatggt gcgattttgg tttattgtaa 5700
tttttgtttt ttgagtttaa ttttagtttt ttagtagttg agattatagg tgtgtgttat 5760
tacgtttagt taaatttttt ttgtattttt agtatagacg ggttttattt atgttggtta 5820
gatcggtttt gaattttttga ttttaggtga tttgttcgtt ttagtttttt agagagttgg 5880
gattataggc gtgagttatc gtgttcggtt gatagtttat gtttttttaaa gaatgtgttt 5940
atggatattt taaagtaaaa atttgtaat tgtttaaatg tgaaagaaaa tgtttatttt 6000
tattaaagta tttttttttt tttttttttt atttttgtag aggagtgtga attttagata 6060
tttttgtagg gatttgtttg tattttgacg cggtgtcgtt tttagtacgg tgattagttt 6120
tagagttcgg ttgttatttt tatcggatat tttagatacg tttttgtagt tgtgtttcgg 6180
tttataatat agattgattg ttttgatttt gattatttaa aattggttta aaaattaaaa 6240
gagatcgata ttaatttgtg ttgtttattt ttttaaagaa tatgaatgat tttttttttt 6300
ttgaaagtga agcgtagcgt tttattttgg gttttcgtag aggttttgta ttttttgggt 6360
tttttgagtt gggatataag tgggtagttg agtgtagaaa gtagggatgg tggggtgtat 6420
agtaggatag tggggtgtgt agtaggatag tggggtgtgt agtaggacgg tggggtgtat 6480
agtaggatag tggggtgtgt agtaggacgg tggggtgtgt agcgggacgg tggggtgtgt 6540
agtaggacgt aagtttaaga cgtattttttg tttaggtatg aaaatggata tcgatttttt 6600
tggtattttt taattattta ttgcggatgt tttagcgatt aagtgatata agttagtttt 6660
tcgtttattt gtttttttaa atagaaattg gcgtaggaga tgaaatttgt agtagaatgt 6720
ttgaaagtat gtgtaataaa aattgtagag ggtggtatgg attgattttt ttaggaaaat 6780
ttttttttaa ttttagattt gaacgaatta gaaaatagaa tagtagagga gggatataga 6840
agttaaaatg ttattttatt tttattggtt tttttgtagt ttggtaagga ttgggagagg 6900
gtgttttttt aatttagtcg tttttgtttt ggggatttta gtttagtaag ttttaagtta 6960
aatgtatttt gggttttttt tttttttatt tgttttttta tttggtgttt ttgtgttggg 7020
tatacgttgt atagttatat tggagatggg atttttcggg agttttggaa agtttttgtt 7080
ttttgtagtt agacggtttt agggaggaag agggaggtgt agaattgagc ggggttagtt 7140
ttttttatat taattagttt ggtaaattat ttttaaattt ttggttatta cgtttagtag 7200
ataaagaggt ttgagaggtc gggagatatt tattaagaag aattattagg aacgggaagt 7260
tttttgtttt ttgtatatag tagtatatat atgagtcgta ataatatata tgttacgtat 7320
gtgttagggg ggttatagat attttttttt atttttttaga tgtgtaagga agttttagtt 7380
tttttttttt atttatggta tgtttgattt tatatttttt gtttgttaat tataggtaaa 7440
aatgagttaa ttttatttag tttaattttt tttaaaattt agtttgttat aataatttgt 7500
atagttttttg ttagttatag tttttgggaa cgtattgttt agttttttat ttcgttttat 7560
ttcgttttgt ttttttttatt tttgggtggg gtgaaggagt ttttggggtt tgtttatgtt 7620
ttttggattt ttgttggttt attgtggagt agaagttggg ggtttttaga gtttagttgg 7680
tgtgtggttt gttcgttttt ttttatagttt tttagtgtta gaaaggtggg ttttttatat 7740
aatatttcgt agttgattgt tttttggttg tttattattc gttttttttt atttaatttg 7800
tattttacga aggaaggggg agaaagaatt tatttttttta gttttttttt tagttagaag 7860
tggtcgtgta attttatgtg gtttatgaga tatagggggt attttttttga aggtggggta 7920
ggtttttttt ttgatgaatg aagagcgttt tttggttttt taggttagtt tttgtagttt 7980
gtttttgggt gttgtggaag gtgggtgtga tgtggtgttg taggtagtta ttatgtaata 8040
atgaggtaag gtttaagatt tagagtttta ataattttga gttattgatt tgagtttttt 8100
atttattttg agatattatg ttaaataaat ttaagtgtgt ttgtgggtta aattattttt 8160
ggtttggttt tttgtggtat tttagtatat tattaaggtt tatatttttt atttattata 8220
gagtagtgag tttatggttt agttacgatg gt 8252
```

<210> 155

<211> 28536

<212> DNA

<213> Artificial Sequence

<220>

<223> chemically treated genomic DNA (Homo sapiens)

<400> 155

```
gttttgggag ttaggttttt tttgttggat gggaaaagga ggtgtggaaa ggggtaggtg      60
gtttgtgggt tagtggggat gagagttgtg gggagaatgg aggagagagt aaaattattt     120
tttttggaag tttaagggaa gggagttgag gtgagagaaa tggtttttt taggttttaa      180
gtttttgttt ttagtttgta ttatttaagt tggggaagtg ataatttttt ttaaattttg     240
atttttattt tttgtttttt gtagattgat tgatgttttt gtgttgtttt agtttagtaa     300
ttaaattagg ttgaggtata ttttttttat ttagagtttt taaattattt attaatttgt     360
aggagattta aatttttttgg gtaggtttttg ttagtgtttt tagtttttat ttttaaattt   420
tttttttttag ttttttttttt tttttttgtt ttttggtttt ttttgttttt tattaggttg   480
agaggtggag aggttgggat tgttgggttt tgtggtttta gaagttaaag tttgtttgtg     540
gggattgttt tttttttttat tgtttttaag tgaataatat ggtgggaggt gatttttgtt    600
taaagggttt ttatttgttt tttattgttg tgtgattttt ttgtaagttg gaggtagagt     660
tttaaaaaga aagaaagtgt gtgttgagta ttgattttaa attggggata ttatttgtga     720
tttttttaag tggtagtagt agtttttgt agaataaaga tttagtgttt gagttttaaa      780
agtttttagg taaagtttta tttagatgaa agtaattaag gtgaaaaaat aatgatattg     840
ttggttttag aaaattggtg gttatttttt gttttaagtt ttttagtgtg gtgtttgttt     900
ttgtttattt tggttgtgtg gggggttgat aagtataata aaagatttta gtatattttt     960
tttttttattt ttataatgat tttttttagtg ttatgaggat gaatttttttg ggattaagtg 1020
gtatttgttt ttatttgtta tgaaattaaa ttttatattt ttttaaagtt gaaaattgtg    1080
gataaagaag gattgggtgt ttaaagttta tttaaatatt tatattgaat ttattttttg    1140
tttagatgtt agaggatggt agggagggtt agggttgtaa tttattttga tttgttttat    1200
tgttttgtag tttgtaaatt ataattttgt ataattttag gaataagtag gtttagaatt    1260
aatgggttaa tattatttaa aataaaatat tgggagtatg atttttgttt taattatata    1320
ttgtttgata agatttagga aattttattt taatttttta atttttgagt tttttgagaa    1380
attgaagggt tgtagttatt agaaattatt tttgtttttt ttaatgtttt taaaggattt    1440
ggaaatagta atgtaatata atatgaagga ttttttttatt taagtttgaa gataaatgtg   1500
gaagtttaaa tattttgaat tggagatgtt tttttgtaaa tttattatag atgtattttt    1560
tttgagagaa atgtatataa attatatatg tatatatgta taaatatttt tttagaataa    1620
tttgttagag gtgtaggttt ttttgtaaag gagtaaattt gagagttatt ttaagttgat    1680
ggatttgtta aattttttttt tttttttttt tttttttatat tatttgttag ttataatgga   1740
atttttttagg tttaagttaa agaaaaattg gagagataaa attagatttt gtagtttttt   1800
ttttttttgg gaatgttttt tttttttttt ttagttttttg atgaatggtt attatttatt   1860
tttattaaat ttaaataagg attgttgttt tgtatgttta attaggtagg tagagggaat    1920
tggtttgttt aggaagtagt gattgagatg ttttggttaa gttagtgata gaggaggggga   1980
gaaagaattt agattaattt gtatgtagta tattttattt ttatgaaata aaatatattt    2040
gttttatatt tgttgaaaag taaaataata atattgtatg aaatgttata tatagggtag    2100
gttgtatata gtagttttag aaatattatt gtatttatta gagaaattat tttaaaattg    2160
atatttatat atttttttata ataataataa tatgttagaa atatatagtg tggtatttag   2220
tatatatatt tttttgtttg taagtgaaaa attttaattg tttttgtata atatgtttta    2280
ttttaaagtt taatttttaa aaatattgtt gtgatattat taataattgt ttttataaaa    2340
ttaatttgat attttgatat atatatattt ttttagttat ttaaatgtta ataatgttaa    2400
atttaaaaaa taataagttt atagtaatgt taaaatgtta tatttagtta aatatttgtt    2460
tgtgtatgtg tttttgtaat tgttagaaat atttgtagtg aaagatgtta gatattgagg    2520
atattttttt gaaattaaag gagttttttt tttgatttag tggttttttt ttttttatat    2580
agtttttttt tttttttttt tttttagtgt ttatgatttt ttagtataat ttttagtttt    2640
ttaagggtgg agttgtttta tttggtaagg ttttaggatt ttggtgttgt gggtgtggtt    2700
tatatgggtt ggtttattgt atattggtaa gtatttaggt tggaggttgg gttttgtatg    2760
ttggtgtagt tgaagttgga gtgttgtttt gtttttagtt ttaggttggt taggtttgag    2820
ttatatgtgt ttttataaat atatggagga gttggtggtg tgtaaggata ggtaggtgtt    2880
ggtattgtgg aatttagtga tgggttattt aggttgttta agttatttag gttgttgaga    2940
ttggagtttg ggatgtttgt tattgttgag ggtattatgt tggatgatat gtttatggat    3000
gagatagagt tgggtgggga aaatatgttt tgtgatgata gggggttgat gtttatagag    3060
ttgaagaagg ggaagttttt ggtggatagg gaggtggatg taaggttttt ggtggtttag    3120
ttgttgtagg aatagtttgg gtatatgttg ttgtagggtt gtatgagttt attgaattgt    3180
ggtttgaagt tatttttgta tagtttggtt tgttggttgt gtttttttttt tttttatttg   3240
gtttgatgat ttttgaatta aatttggggg tggttggggt aagggagtaa atagatgtta    3300
tagtgtagat tattaaaatt tttattggag gttaattttt gtttttttttt gatatatatg   3360
ttagtgtatt tatatatttt ggttttgttt tattgtattg ttttgtatat taagatatta    3420
gggttagttt ttagttattg gtttgggttt ttattaagtg taggagattt ggtttgtttt    3480
ggtttgtgag ttgggatttg gagttatgtt ataaatttta gttgaatgta tggagatttg    3540
tggatggttt gattatttag ttaggtgttt ttttaggttt aaaaatattt aatgtaaaat    3600
aaatgtgggg tagtaggttt ttttaatttt ttttggggta ttttgtaaat ttgtttttat    3660
```

```
tttaaagtta tagatttatg gatgaggaga aggggttgga agggtattag aggattgttt    3720
ttttttttat gtaatttttt tttttttttt ttgattttta ttgttgtttt ttattttttg    3780
gtatgtgttt ttttaatagg gattaggttg ttaatatttt tttttgttta gtaaaataat    3840
taaataaaga gtaaaagatt atttttttgt tagtttgtta attttaggag tttggtatat    3900
taaattttgg gaatttggaa agggtagttt tggagatttt tttttttttt gttttgtttt    3960
tttttatttt taagtttatt ataggtttgt ttgtgtgtta ggtttagttg ggttgtttgg    4020
ttttgtaggt ggttatttag gttggttggt ttttatttgt gtttggtggt ttagttgtaa    4080
ttttgatttt aatttatatt gggttttttt gttgttttag atggtggttt ttgtgtattg    4140
gagagaggtt tggtttgaga tatttgagtt gatattagtg atgttttata ttatatattt    4200
ttgttgggtt tagttgtgta atttgttttt tttttttttt tttttatttt tgattttttt    4260
tttatttttt tttttttttg tatttgattg ttataaaaag tatgttttat ttttatttgg    4320
tttgataagt agttgttttg gaaggagagg tagttgtaag gagagtttag tgttgtggtt    4380
ataaagtatt agggtggagt tgtggaatag tgggtggggt gggagggtgt ttttgaagga    4440
ttttagaaaa tttatagatt ttgtttttaa ttatttgtta tttttatttt aggttattta    4500
aattttgttt aggtgagaag agtatgtgag aggtttgttt ttttgatgtg taagagagtt    4560
aatgaaagat tgattttgtt taaaattatg ttgtttagga tttagttttg gttttggata    4620
gttaaattaa aattattttt aatttttttt tggtttttta tttattagta tagttttatg    4680
ttttgtataa atgttattta gagagtgttt ttattttttt tgatttggga gagtattttg    4740
gtttttattt tttttattgt tgtttttttt tttttgtttg ttttgtttta attggggggtt    4800
ttattttttt tatttagagt atttaatttt ttttttttaa tagtaaagtt tttggatgtt    4860
gtttgatttg tttgattttg tttttttgttt ttagaatttt aataaatttg gaatttttta    4920
ttgattagta taaattagga tgttgttatt gggttattta tttgagttta tttttgttaa    4980
tttataaagt atagatttgt tataaagtta aggtaagttt tttttataaa attatgatta    5040
taatttagaa gagggggtgt gagttttaat ttttagagtt taatttttga gagaagataa    5100
ataaattaag tagaaaagtt tttttttttt tttttttttt tttttaaga ggattagtag    5160
ttgtgtatta aaattttgtt tttggagatt ataaaattag gaaatagggt gtgtgggaga    5220
gatttgaatg gttgaaataa ttgtaaagaa ggtgtaagaa gtgtgagttt aggagggaaa    5280
aagttgggtt agggttggga taaaggtttt ttagggaggg ttaatttttt tgtgtttttg    5340
gtgggttttt tttgttaaag gtttataggt tggagtttgt ttgtggtttt tggtttggta    5400
gggattttat tagttttgtt ttggtaattg taagttagga atataatgtt ttgtgtaggg    5460
gattgtttat gtagtttagt ttgtgagatt gtgggatggt ggggtagtga gttggtgttg    5520
ttttgggagt ttgagttagg gtggtagttt tgttggtttt ggagagggaa ttgtaatttt    5580
gtaattaggt tgttgtgagg tttttttgttt ttgtaaagtt gtgttttatt ggtgtttttt    5640
taggtggtgt tgtttttttat attttttttt ggtttatttg gtttgtattt ttataatatt    5700
ttttttttat tttttttaga ttttgtgttg gtttttattt ggatttgggt ttttgtaagg    5760
ttggtttata tagtgatttt tttgtgtgtg gatatgtttg ggtagtggtt tttttggaaa    5820
gtggtttta gtttttggag ttgttggttg gtaaagtgag tttgttgttg tttttgttgt    5880
ttttttttag atgggttttt ggtgtttatg tttttatttt tttttttgttg gtttttattt    5940
ttttttgaaa atgaaatata tatatttttt tgttagtatg tttatttgta atgtggatgt    6000
taattggatt ggtggtagaa gttgtggaag agttgggttg tttggtgttg gaggagggtg    6060
tgtgtggtgg ttttgggttg tgaggagtgt tgtgtttgtg gggtgtgtag gtgtaagtgt    6120
gggtgtttgt gttttatttt tttttttttt ttagtgttgt atgttttatt tatatgttta    6180
ttttattgta gtggtatatt tatttttata gtttgtgttt ttaagtatat ttatatattt    6240
ttgtgtagat atattaaatt ttttgggatg tgtatatgtg tgtggtttat agattttttt    6300
ttttttgta gaaagtttag atttttatgt ggtttgggaa ggttaggaaa agatgtgggg    6360
atttggttgg gtattgaagt ttgttggttt tttttaaaa aaaaaaaaaa aatgtttttt    6420
tgtgaagggt atttttgagt ggttttaggt aatttttaa tgagtggagt tttttgggag    6480
ttgaaagttg agaggaaaat agggatagag gttggtggtt tttgaaggtt tttgaattaa    6540
gatgttggga tttttgtgat ttaggaaata gaagggaggt tagggtatga atagagaggg    6600
tggtagaatt gtttgtgttt ttagtgtttt aggagttggg ttggttgagg gagaattaaa    6660
gggatgtggg gtagttaaaa ttttggtttt tggaagtttt gtggggagtt aggtgaatga    6720
ttatttttat tatgtttttt tttggagggg ttgatttttt tggggtgaga gggagtgggt    6780
ggtgtagagt agttgagtgg gaatgtttgt agggtggtgt ggtgttttat ttgtggtttt    6840
tgggttggag gtgttggaga tggtgtgtat ttttagtttg tgtttggagg agtttagtga    6900
ttggggttga ttgggagtta gaattgaagt tatggttaat ggttggggat ggtgatagga    6960
agatgaggag atggttgata gtttggtttt tgttgtttgg tgttttaagt gaagtgggtt    7020
ttttatgtag tttatggatg agggagtgtg atgttttatt agtttttggt tattgttttg    7080
ttgagttttt gtagttgttg ttgtttgttt tgggttgtgt tttaggtgtg gagttttttt    7140
gttgtgggga gagttagggg atgtaatttt tgttgagttt ttaagttaag ttgtttttgt    7200
ttttttttgga aggtttaagt gaaaaagttt ggagatggaa agttagtggg taaatgaaga    7260
tatgggatgt gggtagaagg gtattattta gagtgttttt agggagtagg tttttaagtt    7320
ttaaagtgaa ataagagtgg gtaaagattt tttttttttt tttttttttt ttttaagaat    7380
ttttttaata aggaaagtta atgttgattg tgttttgttt gtttttttttt tatgtggtag    7440
```

ttttgataga gaagtgttaa gagtgatagg gataggtagg tgatattaga ttttttgtgg 7500
tggtagtagt tgttgtagtt atgatgtggt tttttgagtg tattttttgt aatgtgtata 7560
tgtatatttt ttgggtggtt gaataggagt tgggttttgt tgtagtttag ttttaggtat 7620
ttaggtgagt gatggattag atttgtggtt ttgtgttttt ttgttggttt aatattttaa 7680
aattagaggt gggtttttttg gtgttgagat gttattttgt tgtggttttt tttagttttt 7740
tttgtttttg ttttttttta gatttttttt tgggtgtgat tgatgtggtt ttgtattaat 7800
taggatgttt tgagttgtgg tggagggatt gttttgtttg tatttattag tagtgtgggg 7860
ttgggttatt gttttgttgt gtgtattggg tttatatagg taagtttttg ggaatttagt 7920
ttttgtttag tttaaggtga tttggttttt agtatgaatt taaaggtgaa gagatgaggt 7980
taggagttga aggtttggga gaagagagtg gaatggttaa gaagagaaag gtataaggat 8040
taataagata tttatttttt gtgtttttatt atatttattt ttaatttttt attttatata 8100
aaaaggagat atgttattta aaattagaaa atttgaaaaa tagtaataaa ttattttttt 8160
gattttaaat tttttaaata gtttgttaag tgaatgttgt gttaatttga agaagtttta 8220
attgtaaaga agatagagtt ttgaaaaggt aggttaataa attagaaatt gagaagtaaa 8280
tggatttgtt aaaagaaaat tattttgatt ttaaatgaat aattgtttgg tggtttattt 8340
tggatttata taagaataaa aagttgtttt agattatgtt ttttgtgatg tttattagtt 8400
tttagataga aaatatataa tagaagagaa attttaattt agtgttttta aaatgttgaa 8460
agtttattta ttttatttaa tgttgattaa gatatatatt ttagattttt taaatttttt 8520
gtatattgta ttaagtttgt tttaatttga gagagttatg ttttaaattt gatttttttg 8580
tttattttat tattaattag atttaaattt ataaagtttg tagaattaat aattttgagt 8640
taattatata tgaaatatgt tttaatgaat ttttatataa ttaagaatgt tgttaaataa 8700
ttaattttaa ggataatttt taatagttat tttttttttt tagtgagttt aaggttgttt 8760
tgagttatta aagtttaagt aggtagaagg ggtgtgtgtg agttaagggt gaaaagttta 8820
gaattgtgtt taattagtaa aagtaaaatt ttatttatat aaaataaaaa aaattatttt 8880
tggagatatt aatttttttat agtattgttt ttaagtaaat ttaattttta aagaaattaa 8940
agaaagaaat ttaaatatat ttaaaataat ttttgaaagt ttttttgttt tttagtatag 9000
gttagttgga gaggataaat taattttttt tgggttttttg tatgggtgat tgttttatta 9060
tggagttagt gttattattt ttgaatgtgt atttgtttga tattatagtt aatgatttgt 9120
aatgttagta tgaagtattt ttaaaatatt ttttttttgt ttttgtttat aagattggga 9180
aatttatttg atgtggaata aagtggatga agtagattat aaatatattt gtaatttatg 9240
tgtttttttt ttgttttgat tatttttaaa ttttatttgt aatttttttt tattttaaat 9300
ttgtagttta aagatgtata tgagaattgt tttttagttt ttttttatta gtattatttt 9360
attttaagaa taatttagtt gtaagggagg aatttttttta tagtaagttt taaattagta 9420
tttttgtttt taattttttta ttttatttta ttttatttta tatatataga tatttgttta 9480
gagtaaaata tatttttatg tgataggttt gtattagttg aggtttatat atttagttat 9540
attaggtttt gtaattttat tattaaatta tatatattat attagtagtt tgttggtaaa 9600
gaaggttaaa ttaatttata ttttgtttat tatttggtgt ttaaatgatg tattttattt 9660
tggagatttg gtggagaatt ttttttttag attttatagt gttttattga agataatgtt 9720
tttatatttg tagtggtttt taatttgata agattttaat ttgtttaagt tttttaaata 9780
agggttttaa atgtttttag ttgttttttt attgaatttt ttttaatttt tttaagatta 9840
taaagtatat gtgtaaagta aatatttttt tttattgtat tgttagttga tgatttataa 9900
ttaagttaat aagaatttag ttttttttttg ttgaatgtgt ttattaatta tattttagtt 9960
ttttttttta aattttagaa tagttgtggt ttttataata ttatgttttt taaagtttta 10020
ttttatgaag ggattttatt atattaaaga atgaaaaaaa tttttattgt agttagtata 10080
tatagttttt tattttttgt tttttaagat ttaaatttta gagttgtaaa tatttttgga 10140
agtttgggtg ttaatgtttt attttagaaa gttgagaagt tttatagagt tatatagatt 10200
tttaaattta ttttttataa atttatagaa ttttgataaa agttttggtg gttttatttt 10260
attgatggaa tttttattat gataaatata tatgtatgaa ggattttaat tagtttttaa 10320
agtggttgaa aaatttaagg gtatgtgatt gtttttttata gtgttaatgt gtgtgagatg 10380
ttggaagtat tggggattag tagtagttta gatgtttaaa aagataaggt gttttaattt 10440
gtgtggattt attgaagtta agtggtgaat aaagataatt atttagataa tttagattaa 10500
agtaaaagta aaattatatt tatttgtata tatatattta tatttatttt atattataga 10560
tatatatatg tatatatata ttggttttgt aaataattga tttaaagtga ggattttttt 10620
tgtatttttt tagtaggagt tttaatattt ttttaatttt ttaattattt tatatattta 10680
tagtagtggt gattgggtga tattttttttt aggtttttttg tgtggtagga tattaatatg 10740
ataagtttgt atggggaaaa ggaggtatgt ggtgggaatt aagaaatatt gtttagtgaa 10800
aattttgtgg tatggtggtg gttgattttg gagatttaat gtatataaga tttgtgggtg 10860
tataggtata ggtagtatgg atgagaaagg ggttagaaga aaataaattt tatgtatttt 10920
gtgattttag tattattgtg atttttggtt aagttttttt taattggttt tagaaattat 10980
tatgagttta gtttttaata tagaaatttt taatatggag aatattggtg ggattttggt 11040
agggaaatta gaggtgttgt atggtttatg tggggtaaag aaggaaagtt tagtgttggt 11100
gtgaggtttt gagtttggga gatattaggg gttgttttga ttggggtttt ttgtttattt 11160
ttttaaagaa agattttaga ggagggaaat gtgtgatatg gggttagttg tgttttgtgt 11220

tggtatttgt tattgattat tagttttaaa gtttattta attttatatt ttttagtgtt 11280
agttgtgtaa agttttttg gttatggtag tgagtggttg ggttgtgttg ttaaattttt 11340
tgtattaatt tggtttggga tttaattaag tgatttttga tttttggaaa gagtttgttt 11400
ttagagttta tttagaagat ggtttaatta gatatttttt tgagttgtta ggttttagat 11460
gggtgggagt tttgttttgt ttaagttagt ttaaggatga ggtttgtttg gatttagttt 11520
ggagttatgt gatgggtgtg agtgtgtgag tttttggtaa ggtgtagagg ttagatggag 11580
attttgtatt ttgtttgaga agtgttttat ttttttaat atttggtttt tttttgtata 11640
taaattaagt tgaaaatagt ttattattta ttatttttta tagttatgga attaaataat 11700
ttagaaatta aaagttttat tgtagttgtt tttttttta tttttaaat ggaatttaaa 11760
aagttttggt ttgttaaaag gggaagatta ttttttgaat tggaagtttg tagatatatt 11820
gagtaatagt tatttttttt gggtttttgt aaatggtatt tattttttta atttatagtt 11880
ttagttgttt aattatttga gatttggggt aattatttgg gggaatagtg tttagatggt 11940
agtgggagtt attattttat agtggtttgg ggaagagaag agaaagagat tagaggaggg 12000
ggtatttgtt aaaattattt aatgaatatg ttgttaatgt tttttatat ttgtatgtta 12060
ttgttatagt tttttaggt gttattgagt ttttagaaag taattatttg ttgaattaag 12120
taaaataagg agaatggtat agtatatgtg tttggagaag gggaaggaag ggtggaatat 12180
gaaattgagt atagatattt aggttaggaa agaaggaagt ggtaaggggt taaatgaagt 12240
tttatttttt tgttattttt ttaaataata gttggattaa atatttattt gttttttttt 12300
tttttttttt ttttttttt ttagtttatg tttatttttt ttttttattt tttttgtttt 12360
tttttttttt tgttttttt tttttagata tgttggtagt taatatttag tattagttgt 12420
tatggtgatt ataaattatt taaatttaaa aatatttatt tataatttga gatgaagttt 12480
ttattttttt agtgaaataa tattttaaaa gttgttagtt gataaaaaaa aaggaattta 12540
ttttattgta gtaatttaag taatatatta tttttaaagg tttaaattaa aatgttagtt 12600
tgttaaaaat atgttggtag agttttggat attttttttg ttagattttt ataaagaagt 12660
tgatttttgtt attttggtt gtttttaat atatatatat aattttgtat gttttttttt 12720
tttttattaa tttttttttt ttttattaat tattttagtt tttaaagatt ttatgtattg 12780
ggttttaaaa gaaaagaatt tttttttgga ttagaaaata gttttattgg ttgttgaagt 12840
gaaagatgtg gggtttaggg ggaaaggtta ttaggattat aatggtggtg gtggtaggag 12900
gttattttag aggagttaag aagaaaaaaa aatgtaggga gaaggattgg aggtggaaag 12960
atagagtaat agaaaattga gttgggtggt taggtgttgt ggtgaagttt agttttgaaa 13020
tgataggtat atattttttt attttttgttt tttttttttt tgagagaaaa ttttttttagt 13080
tagagatttt ggggggtagg aggtgggtaa atgttgttgt agttgggttt tttgtttttt 13140
attttgggtt tgttgttttt tgtttatttt ggattatagg gtatttgttt agatgaagag 13200
ttattaatta tttatttagt taatattagg aagatgataa agttttttat ataggattaa 13260
gaagatatta gattgtttta ttagtatatt tttgtttatg aataagtttt tgttatatat 13320
tttttttttt tttgagttgt tttaataatt gttgtatata ttagtagtgg gtggtgagga 13380
ataatagttg aattagtttt aagaaatttt gtgtattgag ttagtagtga ggaatgtgat 13440
ttgtgaagat tatttttgtg ggtagggatt tgtagggatt gattattttt ggataattgg 13500
tataattttt tttgggggtg aaaaattata atgtggtggg gtattttttta agtgagttgt 13560
agatttgatt ggtgtggggg gtgggggagg ggaggggaga atgggatggt ggaggttggg 13620
tggaggaaag aaaatggaaa atttttttta tttttatttt gttgtttttt ttttaagttt 13680
tatgtttttt ttggtaagta tttgtttttt ttgtgttagt tatagttaga gtttttttatt 13740
tttttttata tatttttttt tttttgataa ggtttaggat tttggttatt attttatgta 13800
ttattatttt gtgttttgtt agagatggtt tgggttgatt ttgttggtgt ttatgttagg 13860
attaaaattt ttttatgatg gtgaggaaaa ttgtattatt tgtttttagg gggtatatta 13920
ggagtttatg tagtatatgt tttgtaaata tttgttgatt gaatgagagg tgtgggggtg 13980
gggtggtgga gaggggtttg tggttgttaa ggttgttagg gttaatttag gtttttttgaa 14040
gaaggttggg attgagttgt tgttgtgtgt gaaggtgtgt gttgtggttg ggggtgttat 14100
aatgggttat ggagtttatt ttttagaggg aggaagtttg tgtatattag tgatttgggt 14160
tgaatatttt tagtttattt attgggttaa agttatttaa taattaatgt gtttttgggg 14220
gaggttgggg gaagtatttg tttttttgttg ggatgtaaag ttaggtgtta ggtttaaagg 14280
ggttgtagtg tagtttgatt ttagtatgga atttagagtt gttgttttga aatttttttaa 14340
gttagtgata gaggagggtt agtttgtttt tttttgagg gtgaagatta ttttatgagt 14400
ttttttttagg atttttaaag taagaaaagt taaagaaagg tttttttgtt ttaggggggtt 14460
gtttttagtt ggtttttata ttattttttg ttagttgtgt ttattttttt ttaaattttt 14520
ggtttttagg ggtttttagt tgtttttgtg ttattttttgt tttggggttt tatttaggtt 14580
gggtatttgt ttaatggata ttaaggagaa tgggatttat tagggaagga aggtagagtt 14640
tggattgttt agaggtggat tttgtttata tagaatgttt agtttttaat gaggatatgg 14700
tatttttggg tggtgttggg ggtagaggtg gagagggtag tgtaatagat tattatggtt 14760
ttttgaagaa gttatatgtt attgtgaatt tttttttttt ttaaaagtaa agaaaaattt 14820
taaaaaaata taagaaataa atttttttgt tttataagta ggttgtggtt aggattttgg 14880
atattttata agttaattta aaattaggga aggataggtg ttttattttt tagtagtgtt 14940
atagttttgt ttatttgtgt gatttttttt tgttgtttat tagtttttta aaagttaatt 15000

```
aaattaagat ttttagtatt tttttttatt atatgttttt ttttaattaa tggtattaaa 15060
tgtgtttagg tagtaatttt tttttttggt taaaaagtag aaaaagatat attgagtgta 15120
ggggaagagt tttttatgtg tattaaaata atgtgggttt gaaagtaatg gttaagaaag 15180
taattattta ttatttttag tttttatgt gttagttatt aaaagtgaat gattaggggt 15240
gtttgtgtgg tttgtgattg gtgtaagtag aattttttg tttttagttg ttttttgttt 15300
atttatgagg atttatttt attgtaggtt tttttatttg tttttagaat gtatttttta 15360
tgtttaggaa atttggggta gggattgggg gaaggagata ttttgtgttt ttttggtttt 15420
tagtataata agaaatgtta gttttggttt ggtgattgtg agtttgtttt gtgtgaagtg 15480
agattgggga gtttttttag tttggttgga gttagggttg agtttgtgta aagtattttt 15540
tttagaagtt attgttgttt ttgattttta attatatttt aaatatatta tggtttaata 15600
atttattttt attattgatt attaaataga agaagaattt aatataattt aaatgataga 15660
aattatgtga tgttattttt gtattgtttt tatttaattt tatggggtta attttggata 15720
agtgagtgtt taattggttt agtagggtga ttggtggtgt agtgtagtgt ttgggtgtga 15780
agtattggat tgttttagat gttttatttt aataaatgat tattttttt tagatttatg 15840
gggaaatttt aatgtaagat ttttgttttt tttttagtaa tatggtttgt tttttgatt 15900
ggggttttaa attgtttttt tttattttat attatatttg tatttttata ttttaatttg 15960
gaaagagggg gttaggggtt gagggttgtg gggggggggg ggttttattt gtttatatta 16020
ttaaaggtta atagtttttt aaggttaggt atttatttat tatggagtta ggaaaataga 16080
ggaatagtaa atttgagggg ttttttttta tgtatttgaa aagaaaggta ttttttttt 16140
tttattttt atatttttt ttttgtttta tagaataagt tttaatttag gaaaggtttg 16200
tggtgtaggt tggagatttt ttaatttttt atataagttt gtagattttt tttggtaatg 16260
tttttgatt tttttagagt gaaattagtt aattaagtaa tgatattgtt aaaatttaag 16320
gtttggtaat tagtatttta ggtaggtttg tgttgatagg ggtaaatttt tattttattt 16380
tgggttgtta agtatagtgg ttgtttttta gtttttttagg gatgttgttg gttttttgtt 16440
tttttttaa ttagttaaag taaatttttg ttaatttaag tttttttgt ttgtttttttg 16500
tgatgaattg tgtatttata agtttgggtg gggtgtggtt gagagtttga gtgattgagt 16560
gggttttggt ggtgttgtgt gtagtgggat ataatgagtg atagaggttg ttgttggatt 16620
atttttttat gttagtttag atgttgaggt ttgttggagt gtgtgtaggg gattagatta 16680
tagggagtga gtgagaggga gagagaggtg ttgggtttta ggagtgtagt ataatttggg 16740
gaaaggaatt aatgtttttg ggatggttgt tttttgtttt atttagaggt ggagtgttta 16800
agtttaagta gtaggtgtgt taggtttggt ggttttgttt ttttgtgttt tgtttgaggt 16860
ttagagtttt ggaggtgggt gtttagtgtg tggtttgtgt ttttttttg gttttattat 16920
tggtgttagg atgttgttgt gggaagaatt tgttgttggt tgttttttt tggtttttag 16980
gagagtttgt gaatttgatt tttttgattt tggagttttt ggagaagaga tatttaatgg 17040
ttgttggttg tatgtttggg ttatgtgtgt gtttgtttta tgtgtggaga gaggtgtttt 17100
ggattgtggt tgaaaggagt tggggatggg aggaggggga ggggtgaggt aggttggagg 17160
agaaagaggg ataaagagta aagatttagt tagaggaaag agttgatggt atttttgttt 17220
tttggatttt ttggtaattt ggggtaggat ggtgtatttt ttttgtgttt ttttggttgt 17280
tggtggtttt agttgggagg agtaggttgg ggggttttgg tatatagtgt gttgttgttt 17340
tttagtttat tgttttgtta tagggagagg ttattggtga tttggttttg atttttttt 17400
tgtttaggtt ggtttttttgg ggaagtgttt tttgttgggg ttttgttgta gggttagtgt 17460
tttttgttg tttttatgtg gtgtggtttt ttgtttgatg atttgggtag gagaaggggg 17520
tttttattta attgtatata tgttgatatt agtttgtggt agttggtttt tattttttgt 17580
tatttgtaaa atagaagaga aggaaggttg taagaagtgg tggttgttga gtgagtaggg 17640
tttagatgag attatgttat attagttgtt aggtgtttat tgtgtgttag gttttaggtg 17700
tgtttttttg atttgatagt ttttggttgt gtagtagtat ttttagttta gttttgggtt 17760
taggatattt atttattaag aggggatttt tttttagagt tgttgtaaaa gtgtttagag 17820
gttagaggat tataaagtta tagtgtgttg gggaggttgt ggatttattt ttaagaattt 17880
tggtgttggg gttaagaatt tatttgaatg taatggtagt gggagtgggt gggtggagag 17940
gatttttttt tttgggaagt tgtatgtaaa gattattttt tagtgtttgt ttattagttg 18000
gagtttggta aatatttgta gaatattagt gttaatgtgt ttttgtttta gatagtagtt 18060
tttttggtt ttttgtaatt ttgaaatgaa tgggtttttg gtttagggtg ttttaggagt 18120
gagttgagtt tgggtttttt atttattagg agttattttt ttatatttag ttatattttt 18180
ttttagagat attaatttgg ttatttattt atttattata aataattatt ttaaagtatg 18240
atttaagatt gtagaggaga gatattgggt ggattgagtg agattgagga gagtagggta 18300
aatgtttttg gagggtttat tgtttgttaa ggatggagaa atagttttgg tataattgtt 18360
atttagtttt tttttttttt tttttgggtg agttaaattt tttttatgtt tttaattata 18420
atgtagtgag ttaagtattt aatgtgtttt tttttttttg ttataggtaa gttgggagag 18480
gtgggttttg aggggtttta ttgggtgggt agaagagttg tggttgtttt aaagataaga 18540
aaagaaggtt tagggttttt taggtttttt tgattttagt gtttgttttt ttttatgtta 18600
attagggtat gttgatgatt ggagggttta ttttgtgtgg gtgtggggat tggggtggga 18660
gtaagtgttg ttgggttggt ggaggtatag aggtggggta gggagttgtg ggtttgtttt 18720
tggtttgagt attgtttttt tgtgttttgg ttttttttga agggagttgg gttttgggga 18780
```

gtttttggtt aaggttgttg tttataggag gggttgtttg gtgttgtggt gtggggattt 18840
agggtgggga tggttaggtg gtttttttat ttgttagtga gaatgtgggt ggggattttg 18900
ttgatttgat ttttgtgggt ttgtgggttt agaagtagta gtttggtggt tttagattta 18960
gtgattttgt agtaaaatta taggattagt ttttgattga gatgtttgtt tgtgagatat 19020
tataaaattt attattatag ttttttatta atttgatatg aagtaatata gatgggattt 19080
tattagttta gattttaaat gtttatttat gataattttg gaggaaattt gtatgttatt 19140
attattttga taattttttt tttttatatg tttgaattgg ttgtattatt agttggtagt 19200
tggagtattg tagatggtaa ttgtaaatag tttttattta tttatttttt ttaaagaatg 19260
aaatatataa aagaaaaaga ttgtgttgtt tggtgtaaag ttagttaatt attatatatt 19320
ttttttttta ttttttttgtg ttttagtgtt gaagattaaa taaagtaata taaaataaat 19380
ttttaagaat ttatagagtt ttattttaag gattgaaaag aaggttaagg tgtgtttttt 19440
agtttatttt tatatgtttt tgtgatttgg agatttattt tgtagttaaa atgagttttg 19500
agatttgtat ttttatgttt tatttaatga ttaggtttat tagaagaatt gagtttaaat 19560
aattggggaa gataattttt taaaaagaga tttttaattt ttgtttgttg atttttaaat 19620
ttgttttatt aagataagtt ttttgtgaga aatttggttg ttagattttg gaattggttt 19680
taatggttaa ttttataaat tgagatggga gatttttttt gatgggaggt agtttttatt 19740
tttaaagttt atgttttagt tggaatgtat atgttaagga tttttgtttt ggttaatttg 19800
ggttttatat tgtgagtata taaaaagtat tatatggtta atggaggatg aggaattatg 19860
gtaaagtagg taggtaagtt ttaagaaata aaataatttg ttaaaaaata atttttgatg 19920
attattgtaa gattgaaagt gtaggaaaaa tatagtttga ataattttag atttttttat 19980
attttttttt tttttatata ttttgttatt ttataataaa atttttaatg gaaagtttaa 20040
aaataaatag tataggaata tgtgttttaa atgaattaaa ttgtgaaatt agttagtaaa 20100
ttaatttgta gtaagtaatt atttaaggaa attaaaatat tgtttagttt agttttgtat 20160
tttattatgt gtatgtgttt tttataatta attaatataa gtgttttagg aatatttgaa 20220
gataaatatg tttaatttaa ggaataaagt atttaaataa tttaagtgta attttgttga 20280
gttaaagtaa aatattttat aaatgaagtg gttatttaat tttttaggga aagtttggtt 20340
attgaaatgt tgtatgttta tgttatatta ataaaaattt ttaatttatt ttgtttatgt 20400
gttttgtttt tttgatatta ttggtatttg aattttagat ggattttttgt taaaatgata 20460
ttttgtgtga taaaagtatt tttagttttg attgatagat taaaataaat gtaaggaaat 20520
tttttttaaat tagattaatt ttttataaaa atattttaga atgtatgaat tttgatattt 20580
atatttataa tggtaaaagt ttttttttgtt tagtttagta agataatatt tatataaaag 20640
agtaaaaaaa aattatatta ttttatgata gtttgatttt taaattgttt aagaaagtaa 20700
agtggttaaa ttggaaaaga ggaatatatt ttggaggttt agaattgaaa attttttttt 20760
taatttttag ttggaaaata atttttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattt gtttgggttt gagaggttat taattgttag 20940
ttaagatgga attttttttt tttttttttt tttttaatgg ataataatgg gaaggggggtt 21000
aatttttttag tagttgaaat tttgtattta gttttttatt ttgagaatgt taatttttgg 21060
tttgaggatt tgtttttgta gtgttggtat tgagatttaa gggaagatat tttgttttaa 21120
atgttagtta tggtttggtt tttttttttga ttttagtatt ttgtagattg ttagtgtttg 21180
tggtggggga tgaaaggaat agggttttgt aaggtttgtt tgttgattgt gttattttgg 21240
gtgaaattta gttttaaaag ttataaatta tttatggtga agattttttg aagtggaata 21300
aatttttaga tttgtattat tttatatttt tgtgggatag atggttttta tttattggtt 21360
attgggagag agttgttgtt tttgtgtttt attgtttttt ggggtgattt ttagtgagtt 21420
gagtttttgg ttgtatggta agtgtttgaa agttgggttt gagaggattg tagggttttt 21480
gagggtgtta agttttgaag gagtttatgg gtgtattggg gtttttgaaa tttagttgtt 21540
attggtagtt ttttttttgtt ttttttttagt ttttttgttt ggttttgtat ttttttttttt 21600
tttttttttt tttatttttt tttttttttt ttgtttttat tttgtgtggg gagtgatgtg 21660
atgttagtag agatttttatt aaattttatt gtatagtggt gtgtgggtgg ttggttgagt 21720
ttggttgtgt ggttggtgat ttaggagtga gtatagtgtt tgggtgagtg ttgggggggag 21780
tgagtagggg tgatgagaaa tgaggtaggg gagggaagta gatgttagtg ggttgaagag 21840
ttgggagttg gagttgggag agtgaaagga gaggggattt ggtggggtat ttaggagtta 21900
attgaggagt aggagtatgg atttttattg tggaaaggag gattagaagg gaggatggga 21960
tggaagagaa gaaaaagtaa tttgtgttaa tttggtagtt ttaataaatt aaagggggag 22020
tgttagggta gtggggagat agaaatgtat ttttggggag taaattagga tgggttggga 22080
ggaagtgata gggaaagtgg tttaagagat ggaataaagg ataatgttta tggggttgtt 22140
tgggatgagg tgtgtggagt gtgggtgtga gtgtgtgtgt gtgatttttt tttaggtttg 22200
tagagttgag gaaagaggtt atagtaaaga gggattgtgg agggaggaaa gtgagagatt 22260
ggtagagggt gggagtggag gtgggtgtgg tggggatggg agaggatgag tgaagagaaa 22320
tttagaagaa tggagtgagt tagtgggaga gggtgggagg gttatagttg ggagtgaatg 22380
agttaggttt gttagttggg gaaggttggg atgttgggtt tagtttagtt gggatattgt 22440
gtttgaggtt aaggtgggtg gattaggtat gttgagagtg ttggtgtata ggtgggtatg 22500
gttatgtatt gatttagtgt ttatgaaggg tttgtattgg ataaggttta gatgtttata 22560

gagtttagaa ttttttttgt tgtatttata tttaataagt ttattttggg ttatggatat 22620
tttatttttt aaaatgatga ggttaaggtt tttggtgagg atggtattaa attgtatggg 22680
atagaagtgg gggtggggga gagagttttt tttaagttta tatttgtttt tgtaaagtaa 22740
agagtatgtg aaattatagg gtatattttt atttgaaaag tgtgttttat ttttgaattt 22800
tgattttttg attttttgat ttgagtaaag atgtgtattt tggtagtgag tagaatattt 22860
tggttttgtt ttgtttttga gtggaaggat tataaatata atttgtttgg aggattaggt 22920
gtgaaggttt ttgttaggta tatgggataa tgttttttta attttaaggg tattttgtta 22980
atgtatgttt ttggaaagtg ttggaatata gttattgttt ttggatttgg atttttttat 23040
taatattaat ttttgtttga gagtaaaatt taggtttgtt attaaaaaga tattttttttg 23100
gtttttaatt gagaataaag ttttttttaa aagttgtatt gtttttttta aattaatata 23160
ttaatatttg taattttaga aatatatagt gatttgggag aatgtgtata aaatagatat 23220
gtttaaaaaa gtttggtgtt taaaattaat tttagttatt atataggtgt tgggtttttt 23280
ttatttttgg gggttgtttg gaatatgtta tgtgtttttt tgaattattt tgtgttttga 23340
atttatttga gttagtagta aaaataggta aataaatttg tttaatttgt tttgagtgtt 23400
aaattttttt attttgaaat agttaatagt tgatagatgg atttatttta tggaaagggt 23460
tagttttttt agttatgaag aaaattgatt agagatttat attttaagtt atttttaatt 23520
tttatgtaat atttgtgaaa atttaaattt ttttttttta tttagtggaa atttaaagta 23580
gtgttattta aggggagaga aatgaggggg aaaatgttta tgtgttgttt aattgtattt 23640
tttttttgat tttgagaatt tttattttttg gtttttgaaa ttttgttgag gtaagaaaat 23700
taaatttttt taataagttt tataattgaa ttttagttat aggatattgg aaagtgtagt 23760
ttgagaaaga tatttttatt tttgtttatt gatgattttt gtagtttttt tatttttttg 23820
agtaatgggt taataatttt tttttttttt ttttttattt tgtagagatt aagaggtgtt 23880
tgtagtagaa tggttttgtt tttagttggt ggtgaggata ggtaatttta tggaaaagtt 23940
ggaagagaat gagaaaatta aagatagaaa gatttagaga tttgtggaga gatataggga 24000
gagggaaggg agttgtgttg aaaagatgta aagatatgtg tgtgtaattt tttttttttt 24060
taggttttag aggtttgtaa attagggttg agaggaaggg gtttgggaag tttatgtttt 24120
ttttgttttt tttttgtttg gagttttgtt tgttagaggt tggttaattt tagttttggt 24180
tgttgtagat attgtgttga gtttttgggt ttttgttttg tttagtgtta gtgtagttga 24240
agtgagtagt tggtgggaaa tgtaaatggt ttttggagaa atagaagata tagaatgatt 24300
tttatttttt ttttgagtgt gtggaaggag ttggatatat gttttatgtt tttaattttt 24360
tttttatatt tttagttata tttttattaa ataattaatt aatgtttaga attattaggg 24420
aatatattag gtatgtaatt gtagaagtag ggtgttgggg ggttataaat tattgagttg 24480
atttaagatg tggattttag gttttttttt tgttaaagta gtaaaggaag agtgggtttt 24540
ggtgattgta tttagatttt gattatttta aattagaagg gggtggaggg agtgtttaag 24600
taaagtaagt aatttttgt tttgtagatg taaataagat tgtagtatta aaggtattag 24660
tttttttagg gttagattgt ttggattggg agtttgggga aggggagata ttaattttat 24720
gtatttgtga attttaagga tgttatattt ttatataaat aattttagtg tggatttttt 24780
ggaatggggg gagtaatatt tttattttag aatattaaaa tatttttttt ttaaagtgta 24840
tatttttttt atttttttaa aattttgaat tatgtttaaa gataatagtt ttttagtaaa 24900
ttggagtatt ggattatttt ttttattttt ttttattgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt tttttttttaa 25080
tatgttgaga taagtgatag aattgttaga atggtaatta aattggaaag tttagggaga 25140
ataataattt tgtgattaaa ttggggtaaa attgtggata aatgtggggt gatttttgtt 25200
aatttttgt tatttaagag ttaggatttg ggaaaggtat agtattattt tagagtttgt 25260
tgtgatgggt tgtgtgttat tatttatttt ttttattttg gattatgatt ttaattttgg 25320
taagtaattt ttttagtttt ttatttgata ataagtgagt atgtaaatat taatggttag 25380
tgatgtttaa ttgttttaaa tattattgat ttgttggttg ttttaaattg ttttttttagt 25440
ttaggttttg tttttgaatt gtttatttta gaggtttgat ttatgttttt gatgttataa 25500
tataataatt gttttttttaa aaaaggtatt taagatgaat taattgattt gtatataaat 25560
taaaattatt atgtgttgtt gattttggtg ttttataatt attttgaaat tagtatttaa 25620
ttatttgagt taaaagaata tataaatgtt tgtattgatt tattaatgaa ttatttaatt 25680
aaaatgtttg ggtaatgttg ggtgttggaa agattgttaa attaagatat attataggag 25740
ggatatgaag attagaaagg taatagatta atattttgta tttaaaatgg agtttttggt 25800
gatttttagt tttaattttg gagtaggggt ttttttttt tgttgttaaa aagattttgt 25860
gtttgtttgt gagtgagtgt atttaagtgg aaggaatgtt tttatggtta tggtggttta 25920
ggttttttgt ttggattggg attttatagt tttaatttag gagtgttaaa ttttggaaga 25980
ttttgggtta gttttggagg tgtgtggttt tgtaagttgt taggttaagt ttgttttttt 26040
tgtttgtttt tttggtaggt tgggtgtgtt atggtagtga gttttttgtg taaatggaga 26100
gttggaatta aagttgatat ttaatagata tgttaattga gtatttattt ttgttttgag 26160
aataggaata aaaggtagtt ttttttaaga gaggtggtgt aaaggtatgt tataggagtt 26220
tagaaaaggt tggtggtggg aaatttgtag tttgggggtt agttaatatt ttttttttatt 26280
ttaagtattt attgatttgt tgttgttatt tttggtgatg tagaaggata tttgaaagaa 26340

```
tttttgatgg ggttttgatt tgagaaagga ggtgatttgt ttaggttttt attaaatttt   26400
taattattat attaattgtt tttttttatt ttttatttga tttttttttt tttgtttatt   26460
tttaattttt taattattta gaaatttttt tattttttag tggttttttt tttgtagtag   26520
tttttttattt gaattttttt tttgtttttt tgtggtaggg tttgtatatt gatttttttg   26580
atttttggta tatttgggtt ttttgaaatt ttttaatttt tttagatttg aggatggtag   26640
gttttatttt ttttattgtg tgtatatatt tagagatatg aaaatttata tagattgttt   26700
ttaaatttag ggtatttaat agatgttttt tttttagttt gtttttttgat ttgaaatgtt   26760
tgtttgattt taatttggat attatttttt tttgtttttt tttttttaaa gtagtttgga   26820
tatgtgtgta agtgagttta gaatagtttt atttatattt tttattaaat tgtaaataaa   26880
agaagaatta atgaagtaga ttggtatata gattgtatta agagtttgaa tttttagttt   26940
ttggattttt tatttaattt tggttgttat ttatattgat agagttattt taagtagagg   27000
tttagagaaa tttgtattgt gggataatag gtaaagttat agtaaaaagt ggaataattt   27060
taaagttatt ttattagaat gtaaattgta tttttgggtt ttgtttgtaa ttatttagtt   27120
ttaatatata tagagttaga taggaaaaaa taggttaata tagttattgg tattagagaa   27180
gataaatttt atgggttttt tagtgaaaag aagattttta aagtttataa tttttgatta   27240
tttaatttta tttataattg tgggaatgaa taagatatta attgttttat gtattttatt   27300
tatattaatt aatttgtgtt tttattaaaa gtagttatat agaatttttt ttaattttttg   27360
gtagtaagtt tagaaaatga agtttatagt tattttgaat tggatatatt ttttgagttg   27420
attattttg taagtgtagg aatataatat tgttttttta tggttttttt gtattttttt   27480
agggtttgta agttttatt aggtttgata ttattgtttg ggtttatatt tattataagt   27540
aaatttgatt attatgttga ttttaaaata gtttatttgg ttagtataat tttagttttt   27600
aaattataaa aatttttttaa tatatgaagt ttttagtttt tatttttttt agtttttttgt   27660
ttatttaaaa tttttatttt aattggtgta agtaataata atttgtatta ttatttgtat   27720
ttttttttatt tttttggaga ttgggttgga ttttagagag aatattagta ttattattat   27780
tataaataat aaaatttaaa agtaaagttt ttatttgtat gataattggt atttggaatg   27840
tttttgattt atttaatgtt attttataaa ggtattttgt aaattttttt ggaattttta   27900
gtaagagttt gtagtaattg gaataatttt ttgggaagat atttttttttg atgggttttt   27960
agtttttgga ggaatagatt gagagtaatt agggagggag gggatattgg aaattggtag   28020
ttatgttagt tgaaataagt ttgggtttag taaggtgatt gatgttgtgg ttgatttttt   28080
attttgagtt ttttttttaat tggggtattg atttttttta ttttgggatt ttaaggtatt   28140
tggtgtgtat gtagattttt tttttgtggt ttttattatg tggttttgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agttttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                             28536
```

<210> 156
<211> 28536
<212> DNA
<213> Artificial Sequence

<220>
<223> chemically treated genomic DNA (Homo sapiens)

<400> 156

```
gatgtatttt gtgtatatta ggttttttga gtttattttg tttttaataa atattattat     60
ttttagtatg tggtttatat tatattttat tagttagagg atatgtgaag tttagagtgg    120
aaagttaggg tagtaggaag tattgttata ttttatatag ttaaagttat ttaaatatgt    180
ttgaatttga gttttgttga gtttttattt gtttttattt tttttgtgtg ttttagttta    240
ttaataaagt aggtttaata taaatatatt aaaagtttaa atattgagat tatgataatg    300
aatatgaggg ttatgattat aaaatattat tgaattagag atttgttatg aaattatatg    360
gtgaagatta taagggagga atttgtatat atattgagtg ttttgggatt ttaaagtggg    420
aagagttagt gttttaatta aagagaaatt tggggtaggg aattaattat aatattagtt    480
attttattga atttaggttt gttttagttg atgtagttgt taatttttaa tgtttttttt    540
tttttaatt gttttaatt tattttttta gaaattgaaa gtttattaaa gaggatattt    600
ttttagagag ttgttttagt tattataagt ttttgttaga aattttaaga aagtttataa    660
ggtattttta taaggtggta ttgagtaagt tagaagtatt ttaagtatta attattatgt    720
aaataagggt tttattttta gattttgttg tttgtggtgg tggtgatgtt ggtgtttttt    780
ttggaattta gtttaatttt taaaaaagta aaaggagtat aaatagtaat ataaattatt    840
attatttata ttaattaaaa tagaagtttt gaatgagtaa ggagttagag gaggtaaaaa    900
```

```
ttgggaattt tgtatattaa aaggttttta taatttgaaa attaagatta tgttggttaa     960
ataggttgtt ttaaaattag tatagtaatt aaatttgttt gtaatgaatg tagatttaaa    1020
tagtgatgtt agatttgata aggatttgta aattttaaaa gagtgtaaaa agattataga    1080
agaataatat tatatttttg tatttataga aatggttagt ttaagagatg tatttagttt    1140
agggtggttg taagttttat tttttgaatt tgttattaga agttaaaaga aattttgtat    1200
aattgttttt gatggaaata taaattggtt gatgtaaatg aaatatataa agtagttggt    1260
gttttattta tttttataat tataaatgaa attaaatgat taaaaattat agattttggg    1320
gatttttttt ttattgagga gtttatggaa tttgtttttt ttagtattaa taattgtgtt    1380
gatttatttt tttttgttta attttgtata tattaaaatt aggtggttat gaataaaatt    1440
tagaaatata atttatattt taataaaatg attttaaaat tattttattt tttattgtgg    1500
ttttatttgt tgttttataa tgtaggtttt tttgggtttt tgtttagaat gattttgtta    1560
atgtagatga tagttagagt tgaatgggga atttagaaat tggggatttg ggtttttgat    1620
gtaatttata tgttaattta ttttattagt tttttttttta tttatagttt ggtaaagaat    1680
atgggtggag ttgttttggg tttatttgta tatatgttta aattgttttg aaaaaggaag    1740
ggtaagaaag agtggtattt aagttggaat taggtaggta ttttagatta agagatgaat    1800
tggaaaggga atatttgtta gatattttgg gtttgaaggt agtttgtgta agtttttata    1860
tttttgagtg tgtgtatata gtggagaggg tggagtttgt tatttttaaa tttgaaaaga    1920
ttgagagatt ttagagggtt tagatgtgtt aaaggttaga gggattaata tataggtttt    1980
attatggaaa ggtggggaaa aggtttgaat agaaaattgt tgtagaaggg aagttattga    2040
gaggtaaggg agtttttgaa taattaaaaa gttaagaata agtaaaagga aggaggttgg    2100
gtgggggata aaaaaaagta gttgatgtgg taattaagaa tttggtggga gtttgggtag    2160
gttatttttt tttttagatt agagttttat tagaaatttt tttaagtgtt tttttgtgtt    2220
gttaaagatg ataatagtaa attaataagt gtttgaaatg aaaggggatg ttgattagtt    2280
tttaggttat agattttttg ttgttagttt tttttgaatt tttatagtgt gtttttgtat    2340
tgtttttttt aagaagagtt atttttttatt tttattttta ggatgaaggt aagtgtttag    2400
ttagtatatt tattaaatgt tagttttggt tttagttttt tgtttgtgtg gaaagtttat    2460
tgttatagtg tgtttagttt gttggagggg tagatagaaa aagtaagttt ggtttggtga    2520
tttgtggggt tatgtatttt tagggttggt ttggagtttt ttagagttta atgtttttgg    2580
gttagaattg taaggttttg gtttgagtaa agggtttgag ttattgtagt tgtgggagtg    2640
ttttttttat ttgaatgtat ttatttataa ataagtataa aattttttta atagtagagg    2700
agaaagattt ttgttttaaa attaaagttg ggaattattg gaaattttgt tttgagtgtg    2760
agatattggt ttgttatttt tttaattttt atattttttt tgtaatatgt tttgatttaa    2820
taattttttt agtgtttagt attgtttgga tgttttaatt gggtaattta ttagtgagtt    2880
aatataggta tttatatatt tttttagttt aagtggttaa gtattaattt tgaaatgatt    2940
ataaaatatt ggaattggta atgtatagta atttttaattt atatgtaaat taattggttt    3000
attttaaatg tttttttttaa aaaaataatt attgtattgt agtattggag gtatggatta    3060
aatttttaga atagataatt tggaaataag atttggatta ggaagataat ttagaatagt    3120
taataaatta ataatgtttg aggtagttaa atattgttag ttattggtat ttatatattt    3180
gtttgttgtt agataaggag ttggggaaat tgtttgttag ggttgagatt ataatttaga    3240
gtgaagaaag taaatggtag tatatagttt gttatagtgg gttttgaaat aatattgtat    3300
tttttttaaa ttttgatttt tgggtgatag ggagttggtg gaggttattt tatatttgtt    3360
tatggttttg ttttaatttg attatgaaat tgttgttttt tttgagtttt ttaatttgat    3420
tattatttta atggttttgt tatttgtttt aatatattgg ggggaggagt gtaattgaga    3480
tttttattaa aaattatttg aatttattta gttagtattg ttttatttaa gtttagtttt    3540
atgggttgta tttaattttt tgtgtttttt atatattaaa attagattat taaaatgttg    3600
gtaggaaagg gtgaaggaaa tggtttaatg ttttagttta ttggaagatt attattttta    3660
gatatagttt aaaattttga ggaaataaaa aggatatatg ttttgggggg aaaatgtttt    3720
aatattttag aatgggggta ttattttttt tattttagag aatttgtatt ggagttgttt    3780
atgtaaaaat gtaatatttt tgaaatttat agatatgtaa ggttagtgtt tttttttttt    3840
taggttttta gtttaggtga tttagtttta aaggagttag tatttttgat gttataattt    3900
tgtttatatt tgtagggtag agaattgttt gttttgtttg gatgtttttt ttattttttt    3960
ttaatttgaa gtaattggaa tttaaatata gttgttaagg tttgtttttt ttttattgtt    4020
ttgataaggg aaaaatttga aatttatgtt ttaaattagt ttggtggttt gtagtttttt    4080
agtattttgt ttttatgatt gtatgtttaa tgtatttttt ggtgattttg ggtattaatt    4140
agttgtttaa taggagtatg attaaaaatg taaaagaagg attaggagtg tgaaatgtat    4200
gtttagtttt ttttatatat ttgaggaggg aatgagaatt attttgtatt ttttattttt    4260
ttaggagtta tttgtatttt ttattagttg tttattttag ttgtattggt gttgggtaag    4320
gtgaggattt aaaagtttag tgtagtgttt gtggtggttg ggattggggt taattagttt    4380
ttggtgggtg agattttaga tagaaggggg gtgagaggaa tgtgagtttt ttgagttttt    4440
tttttttagt tttggtttgt aaatttttga aatttgaaag gggagggagt tgtatgtgtg    4500
tatttttgtg tttttttagt gtaatttttt tttttttttt tgtgtttttt tgtggatttt    4560
tgaatttttt tgtttttggt ttttttattt ttttttaatt tttttatgag attgtttatt    4620
tttgttatta gttgaaggta aggttgtttt gttatgagtg ttttttaatt tttataaaat    4680
```

gaaaagaaaa aaagggagga ttattagttt attatttaga ggaatgggga ggttgtaaaa 4740
attgttgatg ggtagaggtg aagatgtttt ttttggattg tattttttgg tgttttgtaa 4800
ttagagttta gttgtgggat ttgttgaaga aatttgattt ttttgttttg gtgagatttt 4860
aaaaattaga aatagaaatt tttagagtta gagaggaaat ataattaaat agtatgtggg 4920
tattttttttt tttattttt tttttttaaa taatattgtt ttgagttttt attgggtaaa 4980
gagagaaagt ttgagttttt atggatgtta tgtggaggtt agaaatggtt taaaatgtag 5040
atttttaatt agtttttttt gtggttgaag aggttaattt tttttataaa atgagtttat 5100
ttgttgattg ttagttattt taaagtgaag ggatttagta tttaaaataa attgagtaag 5160
tttgtttgtt tgtttttatt gttaatttaa atgaatttaa aatatggagt aatttaagaa 5220
aatatataat atgttttaga tagtttttaa aagtagggaa agtttagtat ttatatagtg 5280
attagggtta gttttaagtg ttaagttttt ttaaatgtat ttattttatg tatatttttt 5340
tgagttatta tatattttta aaattgtgag tattggtata ttgatttagg aagagtaata 5400
taatttttag agggaatttt atttttaatt agggattaaa gagatgtttt tttaatagtg 5460
ggtttgagtt ttgtttttaa gtaggaatta atattggtgg gaaaatttga atttaggagt 5520
aatggttgtg ttttggtatt ttttaaaaat atatattaat aggatgtttt tgagattgaa 5580
aaaatattgt tttatatgtt tggtagaagt ttttatattt ggttttttag gtgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
ttgagtgaga atatgttttg taattttata tattttttgt tttgtaggag taaatgtgga 5820
tttgagggaa attttttttt ttatttttat ttttattttg tgtaatttaa tattattttt 5880
gttaggaatt ttaattttgt tattttaaaa aatgagatat ttgtgattta gggtgaattt 5940
gttgaatgta ggtatagtag aggaaatttt agattttatg agtgtttgag ttttgtttag 6000
tgtaaatttt ttgtgaatat tgggttagtg tgtggttgtg tttatttgtg tgttgatatt 6060
tttagtatgt ttggtttatt tgttttgatt ttgggtgtgg tgttttagtt aagttgggtt 6120
tagtgttttg gtttttttta gttgataagt ttagtttgtt tgtttttggt tgtggttttt 6180
ttattttttt ttattagttt atttatttt tttagatttt tttttattta ttttttttta 6240
tttttattgt gtttattttt atttttgttt tttattggtt ttttattttt ttttttttgt 6300
agtttttttt tgttgtgatt ttttttttta attttgtagg tttgaaagaa ggttatatat 6360
gtatgtttat atttatattt tatatgtttt gttttaaata atttatgaa tattgttttt 6420
tgttttgttt tttgggttat ttttttgtt gtttttttttt agtttgtttt gatttgtttt 6480
ttaaaagtat gtttttgttt ttttgttgtt ttggtgtttt tttttgatt tattagggtt 6540
gttgggttgg tgtagattgt tttttttttt tttttatttt attttttttt ttggtttttt 6600
tttttatagt gggagtttgt gtttttgttt tttggttggt ttttaagtgt tttgttaggt 6660
tttttttttt tttgtttttt tggttttggt ttttgatttt ttggtttgtt ggtatttgtt 6720
tttttttttt gttttgtttt ttgttgtttt tgtttgtttt ttttggtgtt tgtttgggtg 6780
ttgtgtttgt ttttggattg ttagttgtgt agttgggttt ggttggttgt ttgtgtgtta 6840
ttgtgtagtg gagtttggtg gaatttttgt tgatgttatg ttattttta tatggagtag 6900
gagtagaggg aagagagagg gatgagaggg agggagagga gagagagtgt gagattgagt 6960
gagaaagttg gagaggagta gaaagaaatt gttagtggtg gttagatttt ggaggtttta 7020
gtgtatttgt ggatttttttt ggaatttggt atttttagga gttttgtagt ttttttaggt 7080
ttggtttttg ggtgtttgtt gtgtagttgg aggtttggtt tgttggaaat tgttttggga 7140
agtagtggga tgtggagata gtagtttttt tttggtagtt ggtaagtgga ggttatttat 7200
tttgtaggga tgtgagataa tgtgagtttg gaaatttgtt ttattttgga gaatttttat 7260
tgtaggtgat ttgtggtttt tggggttaag ttttgtttaa ggtaatgtag ttggtaaata 7320
gatttgtaa agttttgttt tttttgtttt ttgttataga tattaataat ttatagggtg 7380
ttgaagttga gagggaagtt agattgtggt tggtatttaa aatgaggtat tttttttttaa 7440
attttggtgt taatattgta ggaataaatt tttgggttaa ggattagtat ttttaagata 7500
aagggttggg tataaagttt tagttattgg aagattagtt ttttttttat tgttatttat 7560
tgggaaaaaa aagaaaagaa aaagatttta ttttaattgg tagttagtga ttttttaggt 7620
ttaagtgaat tatttgggag ttaggtttgg atgttaagtt tttattattt ttttggattg 7680
taattttttt aaattgatta ttagttaatt ttaatttggt attttaggag atatatttta 7740
aatggatgta gagaattatt ttttagttgg agattaagaa aaaaattttt gattttaaat 7800
ttttgaaata tgtttttttt ttttagttta attattttat ttttttaagt aatttagaaa 7860
ttaaattatt ataaggtggt gtgatttttt tttatttttt tgtgtgagta ttgttttatt 7920
aaattaaatg gaaaaaattt ttattattat aaatgtaaat attagaattt atatatttta 7980
aaatattttt atgaaaaatt aatttgattt aaagaaattt ttttgtattt gttttagttt 8040
attaattaaa attaaagatg tttttattat ataaaatatt attttggtag aaatttattt 8100
aaaatttaaa tattaataat attaagaaaa taaagtatat aagtaaaata aattgaagat 8160
ttttgttgat gtaatatgag tatataatat tttaataatt aaattttttt taaaaaatta 8220
aatagttatt ttatttgtgg aatgttttat tttaatttag taaaattata tttaaattat 8280
ttaggtgttt tgtttttttaa gttaagtgtg tttgttttta aatgttttta aagtatttat 8340
attaattggt tgtaaagaat gtatatatat ggtaaaatat agaattgaat tgagtagtat 8400
tttaattttt ttaaataatt atttattata aattaattta ttggttaatt ttataattta 8460

gtttatttaa aatatatgtt tttgtgttgt ttatttttaa attttttatt aaagattttg 8520
ttatggggta ataaagtgta tgaaaagggg ggaaatgtga aaggatttgg gattatttga 8580
attgtatttt ttttgtattt ttagttttgt ggtagttatt agaaattatt ttttagtaaa 8640
ttgttttatt ttttagggtt tgtttgtttg ttttgttatg gtttttttgtt ttttgttagt 8700
tgtgtagtgt tttttgtgtg tttataatat aaaatttaag ttggttaaaa taagagtttt 8760
tggtatatat attttaatta gaatatgaat tttgggggtg agaattattt tttattagga 8820
aaagtttttt attttaattt gtgagattag ttattgaagt tagttttgaa gtttggtagt 8880
taaatttttt atagaagatt tgttttgata gggtaagttt aaggattagt aggtgggaat 8940
tggaggtttt tttttaaaaa attatttttt ttagttattt agatttagtt tttttagtag 9000
gtttggttat taaatgaagt ataaaaatgt aagttttaag gtttattttg attgtaaaat 9060
aaatttttaa gttataagga tatgtaggag tgagttaagg aatatgtttt gatttttttt 9120
ttagttttta gagtggagtt ttatgagttt ttgaagattt gttttgtatt gttttgtttg 9180
gtttttagta ttgaagtatg gggaagtggg gggaagaatg tgtaataatt gattgatttt 9240
atattaagta atgtaatttt ttttttttgt atattttatt ttttaaaaaa aataaataaa 9300
taaaaattat ttgtagttat tatttgtagt gttttggtta ttagttaata atgtagttag 9360
tttagatata taaaaaaaaa agattattga aatgatgatg atatgtaaat tttttttgaa 9420
attattataa gtaaatattt gaagtttgga ttaataaaat tttatttgtg ttattttata 9480
ttgagttagt agaaagttgt gataatgaat tttgtaatat tttatgaata gatattttaa 9540
ttagggatta attttgtgat tttattgtag aattattaaa tttggagttg ttaaattgtt 9600
atttttgggt ttatgggttt ataaggattg aattggtaga gtttttgttt gtgtttttgt 9660
tagtgggtgg gggaattgtt tggttgtttt tattttggat ttttatgtta tagtgttggg 9720
tagttttttt tgtaggtagt gattttggtt agaggttttt tagggtttag tttttttttag 9780
gagaggttga gatgtaggga aatggtattt aggttagagg taggtttgta gtttttttgtt 9840
ttgtttttgt gtttttgtta atttgataat gtttgttttt attttgattt ttgtatttgt 9900
gtgaagtggg ttttttggtt gttggtgtat tttggttagt gtggagagag gtaggtgttg 9960
agattgaagg ggtttaggga gttttggatt tttttttttt gtttttaaag taattgtggt 10020
tttttttattt atttggtgga gttttttgag atttattttt tttggtttgt ttgtggtaga 10080
gaagggggag tgtgttaaat gtttggtttg ttgtgttgtg gttgaaaatg tgaaaaagat 10140
ttggtttgtt tgggagagaa agggggagaa ttgggtagta gttatattag agttattttt 10200
ttgtttttgg tgggtagtaa attttttaag aatgtttgtt ttgttttttt tagttttgtt 10260
tagtttattt agtgtttttt ttttgtgatt ttaaattata ttttagggta attatttgta 10320
gtaagtaaat aaatggttgg gttagtattt ttaggagaaa gtgtggttaa atatggaaaa 10380
gtggttttttg atggatgaga ggtttgaatt tagtttgttt ttgaaatatt ttaggttaag 10440
agtttgtttg ttttagaatt atagaaaatt gagggaaatt gttgtttagg atagggggtat 10500
gttggtgttg atgttttata aatgtttatt gagttttaat taatggataa gtattgaagg 10560
gtggttttttg tatatagttt tttaaagaga aaagtttttt ttatttattt atttttgttg 10620
ttattgtgtt tagatgagtt tttaattttg gtattgagat ttttgaaagt aggtttatag 10680
ttttttttagt atattgtggt tttatagttt tttaattttt gggtattttt gtggtaattt 10740
tggagggaga ttttttttttg ataaataaat gttttgggtt tgaggttagg ttggagatgt 10800
tgttgtatag ttagaggttg ttaggttgga aaaatatgtt tgaagtttag tatatagtag 10860
gtgtttaata gttagtgtaa tgtagtttta tttgagtttt gtttatttga tggttgttgt 10920
tttttatagt tttttttttt ttttgttttg tagataatgg ggaatggaga ttaattgttg 10980
taaattggtg ttggtgtgtg tgtaattagg taagaatttt tttttttttgt ttgggttatt 11040
ggatgggagg ttgtgttatg tgagggtggt aagagggtat tggttttgtg gtgaggtttt 11100
agtgaggggt gttttttttga ggggttagtt tgggtaggaa ggaaattaga attaaattgt 11160
tagtggtttt tttttgtggt ggggtggtgg attaggaagt agtggtgtgt tgtgtattga 11220
agtttttttag tttatttttt ttggttggaa ttgttggtaa ttggggaggt gtagaaagag 11280
tatgttattt tgttttgggt tgttagaggg tttgggggat ggggatgttg ttagtttttt 11340
tttttaattg ggttttttgtt ttttgttttt tttttttttt tggtttgttt tgtttttttt 11400
tttttttttg tttttggttt tttttggttg tggtttggga tgtttttttt tgtatgtggg 11460
gtgggtgtgt gtgtggttta ggtgtgtagt tggtggttgt tgaatgtttt ttttttaaag 11520
attttgaaat taaaaaggtt gagtttatgg atttttttga gagttgaaaa gaggtagtta 11580
gtagtaagtt ttttttgtgg tagtatttttg gtgttaatgg taaggttggg agggaagtgt 11640
aggttgtgtg ttgggtattt gtttttggga ttttgggttt tgggtgaagt gtaagaaggt 11700
gaggttgtta gatttgatgt gtttgttgtt tgaatttaga tattttgttt ttgggtggga 11760
tgggaagtag ttgttttagg gatgttaatt tttttttttta aattatattg tatttttgag 11820
atttaatatt tttttttttt ttttgtttgt tttttgtggt ttgatttttt gtgtatgttt 11880
tagtaaattt tggtgtttag gttggtgtgg aaaagtggtt taatagtgat ttttgttgtt 11940
tgttatattt tgttgtgtgt agtattatta gggtttattt agttatttag gtttttagtt 12000
atgttttatt tagatttgtg ggtgtgtggt ttattgtggg aggtaagtaa gggaaatttg 12060
agttggtgaa ggtttgtttt ggttggttgg gggaggggtg gggggttgat aatatttttg 12120
aagagttgga gggtagttat tgtgtttagt agtttagggt agaatggagg tttgtttttg 12180
ttgatgtgaa tttgtttgaa gtattggttg ttaggttttg ggttttggtg atgttgttgt 12240

ttgattggtt ggttttattt tggaggaatt gagggatatt gttagaggag gtttataggt 12300
ttatgtaaaa agttaaaaag tttttaattt atgttatagg ttttttttga attgaaattt 12360
gttttatggg gtggaggggg gggtgtaagg gatggaggag ggaagatgtt ttttttttta 12420
aatatatgga aaaaaatttt ttaaatttat tgttttttta ttttttttggt tttgtagtaa 12480
ataagtgttt agttttagga ggttattgat ttttgataat gtgagtagat aaagtttttt 12540
tttttttata gtttttggtt tttaattttt tttttttgga ttaaagtgta agaatataaa 12600
tgtaatatgg gatggagggg ggtgatttgg gattttggtt aaaaaaataa attgtattat 12660
taagaagaaa ataaaggttt tgtattggag tttttttgtg aatttgagag aaaatgatta 12720
tttgttgaaa tgaagtgttt aaagtgattt agtgttttat gtttggatat tgtattatat 12780
tgttagttgt tttgttgggt tagttaaatg tttatttgtt tgggattaat tttatggggt 12840
taaatggggg taatgtagag ataatgttgt gtgatttttg ttatttagat tgtgttaaat 12900
tttttttttg tttgataatt ggtagtaaaa ataaattatt agattgtagt atgtttggga 12960
tatggttaaa aattaagagt agtgatgatt tttggggaga atgttttgtg tgggtttagt 13020
tttggttttg gttagattag aggagttttt taattttgtt ttgtgtgggg tgggtttgta 13080
gttgttaagt tgaggttgat atttttttatt gtgttgggag ttagagagat gtaaaatgtt 13140
tttttttttt agtttttatt ttaggttttt tagatatggg gaatgtattt tgaggatagg 13200
tggagaagtt tatggtagga tggggttttt gtaggtgagt aggaaatggt taagagtaga 13260
ggagttttgt ttgtgttagt tataagttgt gtaggtgttt ttggttgttt gtttttgata 13320
attagtatat aaagaattag aaataatgaa tgattgtttt tttaattatt atttttaggt 13380
ttgtattgtt ttagtgtatg tgaaaggttt ttttttttata tttaatatgt ttttttttat 13440
tttttgattg aaaagaaaaa ttgttgttta aatatgttta atgttattaa ttaagaaaag 13500
gtatgtaatg ggaagaaatg ttgaaaattt tgatttaatt ggtttttaag gaattagtag 13560
atgataaaaa aaaattatat gagtgggtaa agttatagta ttgttgaagg atagagtatt 13620
tatttttttt tgattttaag ttaatttatg gaatatttaa agttttggtt atagtttgtt 13680
tgtaaaataa aaggatttat tttttgtgtt tttttaaagt tttttttttgt ttttaaagag 13740
aaaaaaagtt tataatgata tatgattttt ttaaaaggtt gtgatagttt attatgttat 13800
tttttttgtt tttgttttta atgttgttta aaaatattat gtttttgtta aagattaaat 13860
gttttgtata ggtagagttt atttttaagt agtttaggtt ttgttttttt tttttagtga 13920
gttttatttt ttttggtatt tattgggtgg atgtttagtt tggatagaat tttgaaatgg 13980
gggtagtatg agagtgattg gagattttta aaagttagag gtttgagaga gggtggatgt 14040
agttagtaga agatggtgta gaagttagtt gagaatgatt ttttagagta aagagatttt 14100
tttttggttt tttttgtttt gggggtttttg aaaggaattt ataaaatggt ttttattttt 14160
aggaggagga tggattgatt tttttttgtt attggtttaa aaagttttag ggtggtggtt 14220
ttgggttttg tgttgaaatt ggattgtatt gtagtttttt tggatttgat gtttggtttt 14280
gtgttttgat aaggggtggg tatttttttt ggtttttttt aggaatgtat taattgttaa 14340
atagttttgg tttagtggat gggttgaaag tgtttgattt aagttgttgg tgtgtataga 14400
tttttttttt ttgggaggtg ggttttatgg tttgttgtgg tatttttagt tgtgatatat 14460
atttttatat gtggtagtag tttggtttta atttttttttg aaggatttgg gttaattttg 14520
gtggttttgg tggttgtaga ttttttttttg ttgttttgtt tttgtgtttt ttatttaatt 14580
agtgaatgtt tgtggagtat atattatgtg gatttttaat gtattttttg aaagtaaata 14640
atatagtttt ttttgttgtt atgaagggat tttaatttta atatggatat tagtgagatt 14700
agtttagatt gtttttagta aaatgtaaaa tggtggtgtg tggggtggtg attaaggttt 14760
tgagttttgt tagaaagaag gggatgtgta gagaaaggtg gagaatttta gttgtggtta 14820
gtgtggaagg gataggtgtt tgttgaaggg ggtatgaggt ttgaggaaaa agtaatgaaa 14880
taggggtaag gagagttttt tatttttttt tttttgtttg atttttgtta ttttattttt 14940
tttttttttt ttttattttt tgtgttaatt aaatttgtag tttatttgaa aggtgttttg 15000
ttgtgttgtg gtttttttatt tttaggggaa attgtattag ttgtttgaaa gtagttagtt 15060
tttgtggatt tttgtttgta aaagtggttt ttataggttg tgttttttttgt tgttgatttg 15120
gtatataaag ttttttaagg ttggtttggt tgttattttt tattgtttgt tgttaatata 15180
tgtagtagtt gttagagtgg tttgggggaa aaggaaatgt ataatgaaag tttatttgtg 15240
agtaggaata tattaatgga ataatttgat gttttttttaa ttttatgtaa aaagttttgt 15300
tgtttttttta atattgattg aatgggtaat taatggtttt ttatttaggt gaatattttg 15360
taatttaaga taggtaaaag ataataagtt taaggtagaa gataaaaggt ttaattgtag 15420
tggtgtttgt ttgttttttta tttttttaggg tttttgatta ggaaagtttt tttttagagg 15480
agaaaaaggt aggagtggga gaatatatat ttattatttt ggggttagat tttattgtag 15540
tatttgatta tttagtttag ttttttgtta ttttgttttt ttatttttag tttttttttt 15600
tgtatttttt tttttttttta attttttttag gatgattttt tattattatt gttattatgg 15660
ttttaataat tttttttttt aaattttata ttttttattt tagtaattaa tgaggttgtt 15720
ttttgattta ggaggagatt tttttttttt agaatttaat gtgtagagtt tttgagaatt 15780
aaagtagttg gtaggggagg aagaaattaa tagaaaggga gagagtatat agaattgtgt 15840
gtgtatgtta aagagtgatt aggaatgata gagttaattt ttttgtgagg atttgatggg 15900
aagagtgttt aagattttat tagtatgttt ttaataggtt gatattttaa tttaaatttt 15960
tagaagtaat atattatttg ggttattata atgaggtggg tttttttttt tttgttagtt 16020

gatagttttt aaaatattat tttgttaggg aaataaaagt tttattttag attataggtg 16080
ggtatttttg gatttaggtg atttatggtt attatgataa ttaatgttga atgttagtta 16140
ttagtatgtt tgggagagag aaaatagaaa gaagggagag taaaagaaat agaaaaggga 16200
gatggatata agttggagag ggaagaaaag agaaaaagag gaagatagat gagtgtttaa 16260
tttaattgtt gtttaaaaaa gtggtggggg ggtgggattt tatttagttt tttgttattt 16320
tttttttttt tgatttggat atttatgttt aattttatat tttatttttt tttttttttt 16380
tttaaatata tgtgttatat tatttttttt attttattta gtttggtaag tagttgtttt 16440
ttggagattt agtgatattt aggaaaattg tggtagtaat atgtaaatgt gaggaagtat 16500
taatagtatg tttgttgagt gattttagta aatgtttttt tttttaattt tttttttttt 16560
tttttttag gttattgtga ggtggtaatt tttattgtta tttgaatatt gtttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740
agaaaataat tttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gaggggggtgg ggtatttttt gggtaggatg taaggttttt atttgatttt tgtgttttat 16980
taggagttta tatatttatg tttattatgt ggttttaagt tgagtttagg tgggttttgt 17040
ttttgagtta gtttgggtag ggtaggattt ttatttgttt aaggtttaat agtttaggga 17100
gatgtttaat taagttattt tttgggtgaa ttttgaagat agattttttt taaaagttag 17160
agattatttg gttgagtttt aggttagatt gatatggaga gtttggtggt atagtttaat 17220
tgtttattgt tatggttaga gggattttgt ataattaata ttgaagagtg tgaaattaaa 17280
taagatttta agattggtaa ttggtggtaa atattagtat aaaatatggt tgattttatg 17340
ttatatattt ttttttttta gggttttttt ttgaaagaat aagtaagaaa ttttaattga 17400
gataattttt gatgtttttt agatttaaaa ttttatgttg gtattgggtt tttttttttt 17460
gttttatgtg agttatgtag tatttttagt tttttttatta ggattttatt aatgtttttt 17520
gtattggaaa tttttgtgtt agaggttgaa tttatagtaa tttttaaaat taattaagaa 17580
gaatttagtt agaggttata gtaatgttgg aattataaaa tgtataagat ttattttttt 17640
ttggtttttt ttttatttat gttgtttatg tttgtgtatt tataagtttt atgtatatta 17700
aatttttaaa attaattatt attatgttat agagttttta ttggatagtg ttttttagtt 17760
tttattatat attttttttt ttttatgtag atttattatg ttggtgtttt gttatatagg 17820
gggtttgaga agaatgttat ttaattgttg ttgttgtgag tgtgtaaagt gattaggaga 17880
ttaggagaat gttgaaattt ttgttggaaa aatgtaaaga aaatttttat tttgagttag 17940
ttgtttatag agttagtgtg tgtgtgtgtg tgtgtgtttg taatataaaa tggatgtgaa 18000
tatatatata taaatagata tggttttgtt tttattttaa tttgaattat ttagataatt 18060
gtttttattt attatttgat tttaatgggt ttatataaat taggatattt tatttttttta 18120
ggtatttagg ttgttgttga tttttagtgt ttttaatatt ttgtatatgt tggtattatg 18180
aggagtagtt atgtgttttt gggtttttta attattttgg aggttgattg aggtttttta 18240
tatatgtata tttgttgtga tgaaagtttt attggtagag tggagttatt agagttttta 18300
ttaaaatttt gtgggtttat gagagatggg tttagaaatt tatatggttt tgtggggttt 18360
tttggttttt taaaataagg tattaatatt taagttttta aaaatatttg tagttttggg 18420
gtttgaattt tgaaaaataa ggagtgaggg gttgtgtata ttaattatag tggagatttt 18480
ttttattttt taatgtgatg gagttttttt atgaaatgaa gttttaaggg gtatggtatt 18540
gtggggatta tagttatttt gaggtttaaa agaagaaatt ggaatatgat tagtaaatat 18600
atttagtaga aaagagttgg atttttattg atttagttat aggttattgg ttggtagtgt 18660
aatgggagga aatatttatt ttatatatat attttatgat tttgggggaa ttagaggaaa 18720
tttaataaga aaatggttag aaatatttaa aatttttatt taaaagattt aagtaaatta 18780
gagttttatt agattaaaaa ttattataaa tgtaagagta ttgtttttag tgaaatgttg 18840
tggggtttga gaaggagatt ttttgttaaa tttttgggat aaaatgtgtt atttaagtat 18900
tagataatga gtagaatgta aattaattta attttttttta ttaataggtt gttagtgtaa 18960
tgtgtataat ttagtgataa gattgtagga tttaatatag ttggatgtat gagttttagt 19020
taatgtagat ttgttatatg aggatgtgtt ttattttgag taggtgtttg tatgtgtgga 19080
atggggtaaa gtggaataaa aggttaaaag tagaaatgtt gatttaaagt ttattatgaa 19140
gaaatttttt ttttgtagtt aaattatttt taaagtggga tgatattggt gaagaaagat 19200
tgaaaaataa tttttatgtg tgtttttgga ttgtaagttt aaaatgggga ggagttgtag 19260
atagggtttg ggggtggtta gggtaaagga gagatatata agttgtaaat atatttgtag 19320
tttgttttat ttattttgtt ttatattgaa taagtttttt aattttgtga ataaggataa 19380
ggagggagtg ttttaaagat attttatgtt ggtattgtaa attattgatt gtaatgttaa 19440
ataaatatat atttagagat gataatatta attttatagt aaaataattg tttatgtaga 19500
aatttagagg agattagttt gttttttttta gttgatttat gttgggggat aaaaggattt 19560
ttaaaaatta ttttgaatat gtttggattt ttttttttaa tttttttgga aattaaattt 19620
gtttggaaat agtgttataa agagttgatg tttttaaagg tgattttttt tgttttatat 19680
aaataaggtt ttgtttttgt tagttgagtg tagttttagg ttttttgttt ttagtttata 19740
tatatttttt ttgtttgttt ggattttaat ggtttaagat agttttgagt ttattgggaa 19800

```
aagaaaatga ttgttaaaaa ttatttttga aattggttat ttggtaatat ttttaattgt   19860
atggaaattt attaaggtat attttatata taattagttt aaggttgttg attttatagg   19920
ttttatggat ttaaatttga ttgataataa agtaaataag agagttgaat ttaaagtgtg   19980
gtttttttgg gttaggatga gtttaatata gtgtataagg aatttgaaag atttaggata   20040
tgtgttttaa ttaatgttaa gtagaatgga taagttttta gtattttgaa aatgttgggt   20100
tagggttttt tttttattgt gtgtttttttg tttggggatt aataagtatt atagagaatg   20160
tgatttgagg tgattttttta tttttgtata aatttagagt gaattattaa atagttgttt   20220
gtttaaagtt aaggtaattt ttttttgatg ggtttatttg tttttttgatt tttaatttat   20280
tagtttgttt ttttagggtt ttgttttttt tgtaattaaa gtttttttag attagtgtag   20340
tatttatttg ataggttgtt tggaaaattt aagattggag aggtgatttg ttgttgtttt   20400
ttaaattttt tagttttaag taatgtgttt tttttttata tggggtgggg gattggaaat   20460
ggatgtagtg agatataaag agtgggtgtt ttgttgattt ttgtattttt tttttttga   20520
ttattttatt tttttttttt aagtttttga tttttagttt tattttttta tttttgggtt   20580
tgtattaaaa gttggattgt tttgggttgg gtaggagttg aatttttggg agtttgtttg   20640
tgtagattta gtgtgtatgg tgaggtagta gtttggtttt gtattgttga taggtgtagg   20700
taggatagtt tttttattgt ggtttggggt gttttgattg gtgtggagtt atgttagttg   20760
tatttggaga agggtttggg aggaggtgga ggtggagagg gttggggagg gttgtggtgg   20820
agtgatgttt tggtattagg aagtttgttt ttggttttaa gatgttaggt taatagggaa   20880
gtgtggagtt gtagatttgg tttgttgttt gtttgggtgt ttggagttga gttgtggtaa   20940
ggtttggttt ttgtttgatt gtttgagggg tgtgtgtgtg tgtgttgtgg agggtgtgtt   21000
tagagggttg tgttgtggtt gtagtggttg ttgttgttgt aggggattta atattattta   21060
tttgtttttg ttatttttga tatttttttg ttagggttgt tgtgtggggg gggggtgggt   21120
agagtgtggt tggtgttagt ttttttttatt ggaggggttt ttgggggagg gagggagaga   21180
agaagggggt ttttgtttat ttttgttttg ttttggagtt tggaagtttg ttttttaaag   21240
atgttttgag tggtgttttt ttgtttatat tttatgtttt tgtttgtttg ttgatttttt   21300
gtttttggat tttttttgttt gagtttttttg gaggagatgg gggtagtttg gtttgagaat   21360
ttggtggggg ttgtgttttt tggttttttt tgtagtgggg aaattttgtg tttagagtgt   21420
gatttggagt gggtagtggt ggttatgggg gtttggtggg gtagtagtta aggattagta   21480
gagtgttgtg tttttttttgtt tatgaattgt atgaaaggtt tgttttattt ggagtattga   21540
gtagtgggga ttaagttgtt ggttgttttt ttattttttt gttattattt ttagttgtta   21600
gttatggttt tggttttggt ttttggttag ttttggttgt tggatttttt taagtatagg   21660
ttggaggtgt atattatttt tgatattttt agtttggagg ttgtaggtaa ggtgttgtgt   21720
tgttttgtag atatttttgt ttagttgttt tgtgttattt gtttttttttt gttttaagga   21780
agttagtttt tttgggggga ggtgtggtgg gagtggttgt ttgtttggtt ttttgtagaa   21840
tttttgggag ttggaatttt gattatttttg tattttttta gtttttttttt gattggtttg   21900
gtttttgggg tgttaagggt gtgagtaatt ttgttgtttt ttttatttgt attttggttt   21960
ttttttttgtt ttttgggtta taaaaatttt agtatttttga tttgaggatt tttagaggtt   22020
gttgattttt gtttttgttt ttttttttggt ttttagtttt tgaggagttt tatttgttag   22080
gaaattgttt gaaattattt agaaatgttt tttgtgaaga ggtatttttt tttttttttt   22140
gggaaagggt tggtgaattt tggtgtttaa ttgaattttt atattttttt ttagtttttt   22200
taaattgtat ggaaatttga gtttttttgtg agggggaggg gggtttgtaa attatgtgtg   22260
tgtgtgtgtt ttaggagatt tggtgtgttt gtgtagaggt gtataaatat atttgaaagt   22320
ataggttata aaagtgaatg tgttgttgta gtgagataaa tatgtaaata aaatgtgtgg   22380
tgttgggggga ggggaggaaa tggggtgtgg atatttatat ttgtgtttgt atattttata   22440
ggtgtagtgt tttttgtggt ttggagttgt tgtgtgtatt tttttttggt gttaggtagt   22500
ttagtttttt tatggttttt gttgttggtt tagttggtgt ttgtgttgta ggtgggtatg   22560
ttgatgggaa agtgtgtgtg ttttgttttt agagaaagat aaaagttagt aggggaagaa   22620
tgaggatgtg ggtgttgagg atttgtttaa gaagaagtgg taaaggtggt agtggattta   22680
ttttattagt tagtagtttt aggagttgga ggttattttt tagaggaatt gttatttgga   22740
tatgtttata tgtgaagaaa ttgttgtgtg gattaatttt atggaagttt gagtttgggt   22800
aggagttagt atggagtttg ggagggatgg ggggaggatg ttgtggaggt ataggttaag   22860
tagattagga gagaatgtgg aaggtagtgt tgtttgggag ggtgttggtg gggtgtagtt   22920
ttgtaaaggt agaaggtttt gtggtggttt ggttgtgaga ttatagtttt tttttttgagg   22980
ttgataggat tgttgttttg gtttaggttt ttagagtggt attggtttat tgttttgtta   23040
ttttgtgatt ttatgagttg ggttgtatgg gtaatttttt gtataggata ttgtgttttt   23100
ggtttgtagt tgttagagta gagttaataa aatttttatt aggttaagag ttgtgaatag   23160
gttttaattt gtgagttttt aataaggaaa atttgttaga gatatggaag agttggtttt   23220
ttttgggaaa tttttgtttt ggttttggtt tagttttttt tttttttgggt ttgtgttttt   23280
tatatttttt ttatggttgt tttggttatt taggtttttt ttatatattt tattttttag   23340
ttttgtgatt tttgggagta aagttttaat atataattat tagttttttt agaaggagaa   23400
agaaaaaaag aagaaagatt tttttgtttg gtttatttat tttttttttag gagttgaatt   23460
ttggaaattg aaatttatat tttttttttt aaattataat tatagttttg taaaaagggt   23520
ttattttaat tttgtagtaa atttgtattt tatggattgg taaaaatgag tttaaataaa   23580
```

taatttaata gtaatgtttt ggtttatgtt ggttggtgga agattttaaa tttgttagga 23640
ttttggaagt agaaaataga attaagtaaa ttaagtggta tttagaggtt ttgttgttaa 23700
aaaaaaaaaa ttaagtgttt tgggtagaaa aaataaagtt tttggttaga gtagagtaaa 23760
taaaaagaag aaaataatga taaaaagaat aaagattaaa atgtttttt aaattagagg 23820
gaatgaagat attttttggg tggtatttgt gtaaggtatg aggttatgtt ggtggataaa 23880
aggttgggaa gaagttgaaa atggttttag tttaattgtt tagagttaga gttgggtttt 23940
gggtggtgtg gttttgagta aggttagttt tttattagtt tttttgtata ttaagggaat 24000
gggtttttta tgtatttttt ttgtttgagt aaagtttaga tggtttaggg tagaaatggt 24060
aagtaattaa agatagagtt tatgggtttt ttgggatttt ttgaaaatgt ttttttattt 24120
tgtttgttat tttgtagttt tattttagtg ttttgtagtt gtggtgttgg gtttttttttg 24180
tagttgtttt ttttttttagg gtggttgttt gttgagttaa gtgggagtga ggtgtgtttt 24240
ttatagtagt tgggtgtaaa gaggaagggg gataaaaagg aaattaagaa tgaaaggaaa 24300
aagagaaaaa gtggattata tggttgggtt tggtggagat gtgtaatgtg aaatattatt 24360
ggtgttagtt tggatatttt aggttaggtt tttttttaat atataaaagt tgttgtttgg 24420
ggtgataggg aggtttgatg tggattggga ttggggttgt ggttgggtta ttggatatgg 24480
gtggaagttg gttggtttgg gtggttgttt gtaaagttaa atgatttggt tgggtttggt 24540
gtgtggatag gtttgtggtg ggtttagggt aaagaagagg tagagtgaaa gaagggggaa 24600
tttttaaaat tatttttttt gggtttttgg agtttaatat gttaagtttt tggagttaat 24660
gagttgatga agaggtggtt ttttgttttt tatttggttg ttttgttagg tgagaaagag 24720
tgttggtggt ttagtttttg ttaagggagt atgtattagg gggtggggga tgatagtgga 24780
ggttagggaa ggaagggagg aattgtgtgg gagaaagagt gatttttttag tgttttttta 24840
gttttttttt tttatttgtg ggtttgtggt tttggaatgg aagtaagttt gtaaggtgtt 24900
ttgggaaggg ttggaaaagt ttgttgtttt gtgtttgttt tatattaagt gtttttggat 24960
ttggagaaat gtttggttga gtgattaaat tgtttgtagg tttttatgtg tttggttgag 25020
gtttgtggtg tagttttgag ttttagtttg taggttagag tagattaggt tttttgtgtt 25080
tggtggagat ttgggttagt aattgaaagt tggttttggt attttggtgt gtagggtggt 25140
gtagtgaagt gaggttaggg tgtgtgagtg tgttagtgtg tgtgttgggg gaaggtgggg 25200
gttggttttt gatggaagtt ttagtaattt gtattgtggt atttgtttgt tttttttgttt 25260
taattgtttt taggtttggt ttaagaattg ttgggttaaa tggagaaaga gggagtgtaa 25320
ttagtaggtt gagttatgta agaatggttt tgggttgtag tttaatgggt ttatgtagtt 25380
ttatgatgat atgtatttag gttattttta taataattgg gttgttaagg gttttatatt 25440
tgttttttta tttattaaga gttttttttt ttttaatttt atgaatgtta atttttttgtt 25500
attatagagt atgttttttt tatttaattt tattttgttt atgagtatgt tgtttagtat 25560
ggtgttttta gtagtgatag gtgttttggg ttttagtttt aatagtttga ataatttgaa 25620
taatttgagt agtttgttgt tgaattttgt ggtgttgatg tttgtttgtt tttatgtgtt 25680
gttgattttt ttgtatgttt atagggatat gtgtaatttg agtttggtta gtttgagatt 25740
gaaagtaaag tagtatttta gttttggtta tgttagtgtg tagaatttgg tttttaattt 25800
gagtgtttgt tagtatgtag tggattggtt tgtgtgagtt gtatttatag tgttgggatt 25860
ttaggatttt gttggatggg gtaattttgt ttttgaaaga ttgggaatta tgttagaagg 25920
ttgtgggtat taaagaaagg gagagaaaga gaagttatat agagaaaagg aaattattga 25980
attaaagaga gagttttttt gattttaaag ggatgttttt agtgtttgat atttttttatt 26040
ataagtattt ttaatagttg taaggatata tatataaata aatgtttgat tggatatgat 26100
attttaatat tattataagt ttgttatttt ttaagtttag tattgttaat atttaaatga 26160
ttgaaaggat gtatatatat tgaaatgtta aattaatttt ataaaagtag ttgttagtaa 26220
tattataata gtgtttttaa aggttaggtt ttaaaataaa gtatgttata tagaagtgat 26280
taggattttt tgtttgtgag taagggagtg tatatattaa atgttatatt gtatgttttt 26340
aatatattat tattattata aaaaatgtgt gaatattagt tttagaatag ttttttttggt 26400
ggatgtaatg atgtttttga aattgttatg tataatttat tttgtgtata atattttgta 26460
taatattatt gttttatttt ttagtaaata tgaaataaat gtgttttatt ttatgggagt 26520
aaaatatatt gtatataaat tggtttggat tttttttttt ttttttttgtt attaatttgg 26580
ttaggatatt ttagttattg tttttttaaat aaattagttt tttttgtttg tttagttaaa 26640
tatataaggt agtagttttt atttaaattt ggtagaaata aatgatagtt atttattaga 26700
aattaaaaag aaaaaaaaag gtatttttgg gggggaaaag ggttataaaa tttaattttg 26760
tttttttaat ttttttttgg tttaaattta gaggatttta ttatggttag taaataatat 26820
gaaaaagaaa aaagaagaaa gaaatttagt aagtttatta gtttaaaatg atttttaagt 26880
ttattttttt atggggaaat tttatatttt agtaaattgt tttggagaaa tatttgtgta 26940
tgtatatatg tatagtttat atgtattttt tttaggagga atatatttat aataaattta 27000
tagggaaata tttttagttt aaaatattta ggtttttatg tttattttta ggtttaagta 27060
gagagatttt ttatgttata ttgtattatt atttttaaat tttttggaga tattaaaaga 27120
aataaagatg atttttaata attatagttt tttagttttt taaagaattt aggggttgag 27180
aggttagagt ggagtttttt gagttttgtt gagtaatatg tagttgaggt aaaggttatg 27240
tttttggtgt tttgttttaa ataatattga tttattaatt ttaaatttgt ttgtttttga 27300
aattatatag gattatagtt tgtaaattgt aggataatga agtaaattaa gatgaattat 27360

```
agttttggtt ttttttgtta ttttttgata tttaaatagg gaatgagttt ggtgtgagtg    27420
tttaaatgaa ttttaagtat ttgatttttt tttatttgtg atttttagtt ttaaaaaaat    27480
gtgaaatttg attttataat aaatagaaat aaatattatt tagttttaga gaatttattt    27540
ttatggtgtt aggagggttg ttgtggaggt ggggggaggg atgtgttgag atttttttgtt    27600
atgtttgtta atttttttgta taattaaagt gggtgagaat aaatattatg ttggggaatt    27660
tagagtaaaa agtaattgtt gattttttgg agttgataat attattgttt ttttgtttta    27720
gttgttttta tttgaatgaa attttattta gaagtttttg gagtttgaat attgagtttt    27780
tgttttgtaa gaaattgttg ttgttattta aagagattgt agataatgtt tttgatttaa    27840
gattagtgtt taatgtatat tttttttttt tttaaagttt tgtttttgat ttgtggaggg    27900
attatgtagt agtggggggt agataaaagt tttttggatg agggttattt tttattatgt    27960
tgtttatttg agagtagtgg agaggaaaat ggtttttatg ggtggatttt ggtttttgga    28020
gttgtagggt ttagtggttt tggttttttt gtttttttagt ttggtagggg gtggggaggg    28080
ttaaagggtg gaggggaagg aaggagttag aagaggggat ttggggatgg gggttggaag    28140
tgttaatgag atttgtttgg aggatttagg ttttttgtag gttggtgagt gatttgggag    28200
ttttgggtaa aagaggtgta ttttggttta gtttggttgt tgaattagaa taatgtgagg    28260
atattaatta atttgtagga aataaaaaat ggaagttgag gtttaggaag agttgttatt    28320
tttttgattt gagtggtgta ggttgggggt ggagatttgg gatttaagag aggttatttt    28380
ttttatttta gttttttttt tttggatttt taaaaggaat aattttattt tttttttgt     28440
ttttttata attttattt ttgttagttt gtaggttgtt tgtttttttt tgtattttt       28500
tttttattt agtgagagaa atttagtttt tagagt                               28536
```

<210> 157
<211> 8252
<212> DNA
<213> Artificial Sequence

<220>
<223> chemically treated genomic DNA (Homo sapiens)

<400> 157

```
gttattgtgg ttgggttata ggtttattgt tttgtggtag atggaagata tgggttttgg      60
taatatattg aaatgttata ggaaattaaa ttagaagtga tttaatttat aagtatattt     120
aggtttattt aatatgatgt tttaaggtgg gtggggggtt taaattagta gtttagggtt     180
attaaggttt taggttttgg gttttatttt attattatat aatggttgtt tgtaatatta     240
tattatattt atttttata atatttaaag gtaggttgta agggttggtt taggaagtta      300
gagggtgttt tttatttatt aagggagaag tttgttttat ttttaaggag atgtttttta     360
tgttttatgg gttatataga attgtatggt tatttttggt taaaagaggg gttgggaaag     420
tgaatttttt tttttttttt ttttgtagag tgtaagttag gtaagaaaga atggatggtg     480
gatagttagg aaatagttag ttatgagatg ttgtgtggaa agtttatttt tttgatattg     540
gagaattgtg ggaagaatgg ataggttata tattagttgg gttttgagaa tttttagttt     600
ttgttttata gtgagttagt aggaatttaa agggtatggg tagattttag gggttttttt     660
attttattta ggggtagaga gggtaagatg gagtagagtg aggtaagaga ttgagtaatg     720
tgtttttaaa gattgtgatt gatagaagtt gtgtaaatta ttatgataaa ttgaatttta     780
agaggaattg gattaaataa agttagttta tttttatttg taattagtag atagagagtg     840
taagattaga tatattatag atggaaaaga aaagttaaaa tttttttgta tatttgagag     900
gtaagaggag gtgtttgtgg ttttttgat atatatgtga tatatgtatt attatggttt      960
atgtgtgtgt tgttgtgtgt agggggtagg gggtttttg tttttgatgg tttttttgg      1020
tgggtgtttt ttggtttttt aggtttttt atttgttgaa tgtggtagtt aagggtttgg     1080
gaatgatttg ttagattggt taatgtggaa agagttggtt ttgtttagtt ttgtattttt    1140
tttttttttt ttggaattgt ttggttgtag agagtaggag ttttttagag tttttggggg    1200
attttatttt tagtataatt gtgtaatgtg tgtttagtat aagagtatta gatggaggga    1260
taggtgggag gaggaggaat ttagggtgta tttagtttgg ggtttgttga gttgaggttt    1320
ttagagtaag gatgattagg ttgggggat attttttttt aatttttgtt aagttataga     1380
ggggttagtg ggaataaagt gatattttgg tttttgtatt ttttttttgt tgttttattt    1440
tttagtttgt ttagatttga ggttggggag gggtttttt ggaggggtta gtttatgtta     1500
tttttgtaa tttttattat atatgttttt agatattttg ttataggttt tattttttat     1560
gttaattttt atttgggaaa gtaaataaat ggaaagttaa tttgtgttat ttggttgttg    1620
gggtatttgt agtgagtagt taagaaatgt taggggagtt ggtgtttatt tttatgtttg    1680
gataagagtg tgttttggat ttgtgttttg ttgtatattt tattgttttg ttgtatattt    1740
tattgtttta ttgtatattt tattgtttta ttgtatattt tattgtttta ttgtatattt    1800
tattgtttta ttgtatattt tattgtttta ttgtatattt tattattttt gtttttttgta   1860
tttagttgtt tatttgtatt ttagtttagg aagtttagaa gatgtagaat ttttgtgaga    1920
```

```
gtttagggtg aaatgttgtg ttttattttt aaagaaagga aaattattta tattttttaa    1980
aagaatgaat agtatagatt aatattgatt ttttttaatt tttaggttaa ttttgagtag    2040
ttaaagttag agtagttaat ttgtgttgtg agttgaggta tagttgtaga agtgtgtttg    2100
aggtgtttgg tggaggtggt agttgagttt tgggattaat tattgtgttg gggatggtat    2160
tgtgttagga tgtaggtaga tttttgtaga agtgtttaaa atttatattt ttttataggg    2220
gtgaggggga gggagaaaga gatgttttag tgaggataaa tatttttttt tatatttaaa    2280
taattataga gtttttattt taaagtattt ataggtatat tttttagaaa atatgaattg    2340
ttagttgggt atggtggttt atgtttgtaa ttttagtttt ttgggaggtt gaggtgggta    2400
gattatttga ggttaagagt ttaagattgg tttggttaat atggtgaaat tttgtttata    2460
ttaaaaatat aaaaaaaatt tagttgggtg tagtggtata tatttgtaat tttagttatt    2520
aggaagttga ggttgaattt aggaggtaga gattgtagtg agttaagatt gtattattgt    2580
atttagttt aggggtaatg gagtgagatt ttattttaaa aaaaaaaaaa aaaaaagaat    2640
atatgaaatg tttttagatt ttgttatgtt tttttttttt attttaggta agttagaaag    2700
tgttattaat agtggttttt tttaggtttt tggttagaga tgtgaagaga agttgggggg    2760
aaattaggtt ttttttaag tttttagtt ttgttttttt atttttggat ttgaatgtag    2820
tttgatttag gttattttat tgtattatta ttggtggttg tgattttgtg taaaggtata    2880
gttggtgatg ttgattagag tttttgtagt tttaaatgat ttttttaatt aattttaaat    2940
ttttagaatt tattgtataa aaaggttata ttttttggag ggatgttgat ggtattagga    3000
tagaagtatt aggggatttt atgaatggtg ttgttgaaat agtagttttt atttgtatat    3060
tgggagggtg tgatattagg aaaattataa ttttgttttt tatggggggt tattgtatat    3120
gtttttgaaa gtgtataggt aagaagtaaa gtaagttgtg ggttgaattt tttgatgtta    3180
ttatgtatat atttatttag tttttttttt taatgatatt agtaattgtt tagtgaggtg    3240
gatataaaat ttttaggata tgagagggag atgtggtttt tatattttga tgtgtaaata    3300
ttatgtttag ggaaaatgta aggtgtttta ggtttgtgga ttttgtattt ttttaggtaa    3360
tttatttatt tattttttaa ttttaataaa tgattattaa attttattta atatataaat    3420
atttattgag tattatttgt gtgtatgaga agtgggagtt agtatggtaa aagttaggta    3480
ttgtgttagg tgagagagat ttagaaatta aaattagaga agttattaat aagagtttaa    3540
atatttgggt ttaggtttat gtttgtaatt ttagtatttt gggaggttga aggaggtgaa    3600
ttatttgagg ttaggagttt aagattagtt tgattaaaat ggtgaagttt tatttttatt    3660
aaaaatataa aaaattaggt gggtattgtg gtatatgttt gtaattttag ttatttggga    3720
ggttgaggta ggagaattat ttgaatttag gaggtagagg ttgtagtgag ttaagattgt    3780
attattgtat tttagtttga gtgatagagt aagattttat tttaaaaaaa aaaaaaaaag    3840
agtttaagga tttgatggag gagaaaggta agaatatgtg tgagataatg taaggttatt    3900
gttttagggt gtttagggta attatggggg tagggtattt tttggagagg ttaatgataa    3960
gtaggttgaa taaagtaggg gggttttttg taggaggagg tttattaggt gaagatggag    4020
ttgtatgggt aaaggttatt ttagagattt aggtgtgttt aggaggtgga aatttattgt    4080
aggtaaggtg agaggattgg gtggggtggt ttaggaggag ttgatagagg gtataagttg    4140
tgaaatagtt tgaagtaggg tagtgaggaa agggatttag aggaggaaga tatgtggata    4200
gatggggttg gttgggggtt gttgtaggat tttatgtaag aggttttaat attagagttt    4260
taggttttga gttttgtgga attaaaggtt ttagaaagta atttattagg atttggtggt    4320
tgatagtttg tagtaggggg tgaaagagga gtttagagta ttttttggtt tttgtttttg    4380
ttttggggta taggagggga ggaatttagt ttggttatat tggtttaggt gagggtgttt    4440
taggggaggt tgagaggggt tgtttttttg tttgttttgt tattagggat tgttttgaga    4500
tgtttttgga gaaagtgttt ttggtttgtt gggaaggatt tgtgtttagt ttttgttgtt    4560
tagtagtttt tatgggaatt ttgttttttt ggggtttgga tttattgtga tgtgaaggtg    4620
attattgttt atttttggga tattgtgggg gttaagagag gttttgtggt gagggaggat    4680
tattattttg ggggttgggg gggttttttt tttagggagg aagattttta gttttggtgt    4740
tttgttttg gtagattttg ttttgtttat ttgttttgtt tttgttgtat gatggaaata    4800
aatggaaatg gtttttatat atgaatgtat taaattgtaa ttataatttt ttagatttta    4860
gttgtaatag gattatggat ttgagtgatt tgtaaagatg ttttgagttt tgagttagag    4920
ggtgtgtggg gtggggaggg gagtttgtta tggtttttgt gattttatag tatagggggt    4980
agagttgggg gttggggtgg gggtaagggt gtaggtagat gggtttgggg gtggttagta    5040
tggggattta tttagtttgt tttgtatatt gaggttattt tttttttgg gttttaaagt    5100
tttttgttt tttgttatgg gtttgggggt ttttttattg tagtttaatg ttggttgttt    5160
ttttatgttg tttaagttta tttttaggt ttggtatttt atagtagaga ttaagaggtg    5220
gttggagggt agtgggggta tggatagtat tattgggtgt tttttttta ggtttttttg    5280
gaaatttttg ttttggaaat gtagaaagtt tttttttttg tttttatttt gaaattgtat    5340
ttaataatgg taaataagtt atttagtttt ttatagttgg taaagtgtaa gttgtagatg    5400
ttgttatata gtattttttg atagttttag ggtagattgt tgatttattt tgtaggtaaa    5460
ggagttgaga tgtaaatatt ttttaaggaa gtaagaatgg tttttttttt tattttttag    5520
aaggatttag ggtaattgat ttatttgata atattaggga atatgggttg ttagttatgt    5580
atttatgaga gaggtggttt tgatttttag agtatggatt ttaggggagt ttaggaattt    5640
gtaggagagg gtttttagt tggttattat tgggatgggt atttgggttt gtgggagagg    5700
```

```
gtttttttagg tggttattat tgggattggt atttgggttt atttggatgg gttttgggag 5760
ggtttgtttt tgggggagat gttggtatga atagttaaaa gtattatttt gagattttat 5820
gttaatattt tttttttgaa atatgatatt tgggaggatt gtatagtttt tttaatatag 5880
gaaaatagtt ttgattgaag gtagtttttt ttttgttgta ttttttgaag gtttttgggg 5940
gttttgttat tttgagttat tttttatttg tttttgttga ttttgtaaat tggatatatt 6000
tttaagggtt tagaatagta tttggtataa aataggtgtt taaaaatatg tatgtaaaat 6060
agtggttttt ggtggaggtt gttagagttg gttgtggttt tatagagtag gaagaagtat 6120
gttttatttg tttgggtttt ggttagggtg ttgagggtta gtatggatat taggattagg 6180
gtgtagatta ttttgttttt tatggtggtt attgtttttt ttttgtttta aaggtgattt 6240
tgagttaggg atgagaggtt gtttgagttt tggattttat agggtaggtt ttgtttgttt 6300
aaagagtgtt agataatatt tgtttaagga ggtttgggga ttttttgaga taataatttt 6360
tattgatttt tattaaaggt gtttttttaga tatggttaag ttatatggaa ggatttgttg 6420
atagatagag atgatatgtg gtgaggttat tttggttgag ggatttgaga tttagaaagt 6480
tttttttttt tatgggagtt ttttttaatt tgattttaat ttaggtttta tttatatttg 6540
agaggttttt ttgttagggt aaatattgta tttattgtag aagtgattta tagtgagaga 6600
tggttggaaa aaggtttggt tgtgataata gtggtttatg gggtggttat tgtgattttg 6660
tagggggaag ggaaggagtt tatgaagttt attttgttta ggtttaagtt aattttttta 6720
tagtggaatt gttttaataa tgaaattgta ggtttggtgt agtggtttat gtttgtaatt 6780
ttaatatttt gggaggttaa agtaggtgga ttatttaaag ttaggagttt gagattagtt 6840
tggttaatat ggtgaaattt tgtttttata aaaaaaaaaa atataaaaat tagttaggta 6900
tggtggtggg tgtttgtaat tttagttatt ttggaggtta agttaggaga attatttgaa 6960
tttgggaggt ggaggttgta gtgagttgag attattttat tgtattttag tttgagtgat 7020
agattaagtt tttttttggg aggggttggg ggtaggaggg agaaaaaaat agtatatatg 7080
agttgttatt aaaaattgat aaagtgaatg tggtttattt ttttgtgttt atggggtttt 7140
tttttttttt tttttatatt tttttagttg gttttttgga tttttgtatt ttagaggata 7200
gtttttttgtt tttttgtatt atgttttatt tttttttttt gtttggagtt ttttttttta 7260
tagtttttat tatttttttgg tggataaaat aagtgattga tatattatgt tggttattta 7320
tttattgtat ttttttttgt agtagatggg agggtgtatg ggagtagaag tttttatttt 7380
ttagtgtttt gaggtggttt tagattttag aatagtggtt ttgattggta tttgagaaat 7440
agttgttgaa gagttgtatg aagaaatgga taataggaga aatttttttt tttttagtga 7500
gaaattaggt tttgaggaaa atgtatgtta gtttaataaa ggtattattt ttttttgggt 7560
gatttagtta gtatattttt gtagtttttt tgggaggggga gtttgggtta gaatgggggt 7620
ggttatgttt ttttttgttt tattttagga tttagttttt tttttagggt tgttattttt 7680
gtattggggt ttttattttt gggttagggt ttttaggagg ggtttgattg taggatttag 7740
ggagggggttg aggtttggtg ttttttgtgg ttagtttaag tttattgaaa ataggatggg 7800
ggtattaggt ttagggtggt gtttatagtg atggtggttt ttgttgtttt aattttttgg 7860
attaaaatgg gggtttttgg aaatgggatg taaattgtat aattatttgg agaaaaggtg 7920
ggtagtgttt ggtgaggttt aagttatata gattttatag tttagtaaat tttttttgttt 7980
ttaggtttgg ttttttaatt tttagaaatat gtttatattt gggtagaaga aaatataggt 8040
aaagatattt tttttagtat tgtttgtaat agttggaaat agggaaagaa ggaaggaagg 8100
aaaggaggga aagaaaagta aaataaatat ttattaatta gggaaaggga aaatgaattt 8160
aaaatataat gtagttgttt tttagtattt ttgggggatt ggttttagga ttttttattt 8220
agggatgtta aaatttataa atgtttaagt tt 8252
```

<210> 158
<211> 8252
<212> DNA
<213> Artificial Sequence

<220>
<223> chemically treated genomic DNA (Homo sapiens)

<400> 158

```
ggatttgagt atttgtggat tttggtattt ttgggtgggg ggttttgaaa ttagtttttt      60
aggggtatta agggatgatt gtattgtatt ttaagtttat tttttttttt tttggttaat     120
gaatatttat tttgtttttt tttttttttt tttttttttt tttttttttt ttgtttttag     180
ttgttataaa taatgttaaa aagaatattt ttgtttgtgt tttttttgt ttaagtatga      240
atatgttttt gggattaaga gattaggttt ggaaatagaa gaatttgttg agttgtaaaa     300
tttgtatggt ttaaatttta ttgaatattg tttatttttt ttttaaatga ttgtgtaatt     360
tatattttat ttttaagagt ttttattttg atttgagaaa ttagagtagt gaaaattatt     420
gttattatga gtattatttt gggtttggtg tttttgtttt gtttttaata aatttaagtt     480
gattatagag gatattaggt tttggttttt ttttgggttt tgtggttagg tttttttttgg    540
```

agattttggt ttaggagtgg agattttggt gtaggagtgg tagttttgga ggaggggttg 600
ggttttggga tggagtgaag aggaatatgg ttgtttttat tttggtttag gttttttttt 660
tagaggggtt gtagaaatgt attgattagg ttatttaaga aaagatagta tttttgttag 720
gttagtatgt attttttta gggtttaatt ttttattgaa gaagaaaaga ttttttttgt 780
tgtttatttt tttatgtagt tttttaatag ttgtttttttg aatgttaatt aaagttattg 840
ttttagggtt tggggttatt ttaaggtatt aggagatgag gatttttgtt tttatgtgtt 900
tttttgtttg ttgtagggag gagtgtaatg aataaataat taatataatg tgttagttat 960
ttgttttatt tattaggagg taataagagt tatgaaagag aaagttttga gtaggggagg 1020
ggagtgaggt atggtatagg agagtaggag gttgtttttt aaaatatagg agtttagggg 1080
attaattggg aaggtgtggg agggggaggg agggagtttt atagatatag gggagtgaat 1140
tatgtttatt ttgttagttt ttgatggtag tttgtatata ttattttttt tttttttttg 1200
tttttagttt tttttagaag gagatttaat ttgttgttta ggttggagtg tagtagggtg 1260
attttgattt attgtaattt ttgtttttta ggtttaagtg atttttttga tttaattttt 1320
agagtagtta ggattatagg tatttgttat tatgtttggt taatttttgt attttttttt 1380
tttgtagaga tggggttttg ttatgttggt taggttagtt ttaaattttt gattttaagt 1440
gatttgtttg ttttggtttt ttaaagtgtt gggattatag gtgtgagtta ttgtgttagg 1500
tttataattt tattattaaa ataattttat tgtaaaagaa ttagtttagg tttagatgga 1560
atgggtttta tgagtttttt tttttttttt tgtaaggtta tggtggttat tttgtgagtt 1620
attgttgtta tggttaagtt tttttttggt tattttttat tatgaattat ttttgtagtg 1680
agtatagtat ttattttggt gggagggttt tttagatatg agtaggattt ggattaaggt 1740
taggttggag gagattttta tgggaaagag ggatttttttg aattttagat tttttagtta 1800
agatgatttt attatatgtt gtttttgttt attagtaaat tttttatgt agtttgatta 1860
tgtttaggaa atatttttga taaaaattag tggagattat tgttttagag gatttttggg 1920
tttttttagg taaatgttat ttaatgtttt ttaagtaaat agagtttgtt ttataaaatt 1980
tggggtttgg gtggtttttt atttttgatt tggggttgtt tttggagtag agaggaggta 2040
atggttatta tggagaataa ggtgatttgt gttttggttt tggtgtttat gttggttttt 2100
ggtattttgg ttgaggttta gataggtaag gtgtgttttt ttttgttttg tggggttata 2160
gttagttttg gtagttttttg ttaggagtta ttgttttata tatatatttt tgagtatttg 2220
ttttgtgtta ggtgttgttt taggttttta aaagtatatt taatttatag gattggtaaa 2280
agtaggtgga gagtaattta gggtggtagg gtttttggag atttttgaga agtgtgatga 2340
ggaggggggtt gtttttagtt ggggttgttt ttttgtgtta ggaagattat ataattttt 2400
taagtgttat gttttaaaga ggaagtgttg gtgtggggtt ttagaatagt gtttttgatt 2460
gtttatgtta atattttttt taggggtaga ttttttttaag gtttatttag ataggtttaa 2520
atgttggttt tagtgatggt tatttgggag attttttttt ataggtttga atgtttgttt 2580
tagtggtggt taattgggag attttttttt ataggttttt gggttttttt gggatttatg 2640
ttttgggagt taaagttatt tttttatga gtgtgtggtt ggtaatttat atttttttggt 2700
gttgttaagt ggattggttg ttttgggttt ttttagggag tggaggagga ggttattttt 2760
gttttttttgg gaagtgtttg tattttaatt tttttatttg tagaatggat taatggtttg 2820
ttttagggtt gttaggaaat gttgtgtggt agtatttgtg atttgtattt tgttagttgt 2880
ggggagttga ataatttatt tgttgttatt aggtatagtt ttaaggtggg ggtaggagaa 2940
agggtttttt atgtttttaa agtaagggtt tttagagagg tttgaagagg gagtgtttag 3000
tggtgttgtt tgtgttttta ttgtttttta gttatttttt gatttttgtt gtgggggtatt 3060
gggtttgagg ggtgggtttg ggtagtgtag aagagtagtt agtattgggt tgtagtggga 3120
agatttttaa gtttatggta gggagtgggg gagttttgga atttgagaga ggaagtggtt 3180
ttggtgtata gaatgaattg ggtgggtttt tgtgttggtt atttttaggt ttatttgttt 3240
gtgtttttgt ttttatttta gtttttagtt ttgtttttttg tgttgtggga ttatagaggt 3300
tgtggtaaat tttttttttt attttatata ttttttggtt taaggtttag agtgtttttg 3360
tgggttattt aggtttatga ttttgttata attgaaattt agaaaattgt gattatagtt 3420
tagtgtattt gtgtgtggaa attattttta ttttattttta ttatgtgata aagataaagt 3480
gggtgggtaa gatagagttt gttggaggta gagtattggg gttggaaatt tttttttttg 3540
aggaggaaat tttttttgatt tttaggatga tgattttttt ttattatggg gttttttttg 3600
atttttatag tgttttgggg gtgggtgatg attattttta tgttgtgatg gatttagatt 3660
ttaggagggt aaggttttta tggaagttgt tgggtagtgg gagttgaata tggatttttt 3720
ttagtaagtt aggaatattt tttttaaaga tattttgagg tagtttttga tagtaaagta 3780
gataagagaa tagttttttt tggttttttt tggggtgttt ttatttgagt tagtgtggtt 3840
agattgagtt tttttttttt tatgttttaa ggtagggata gggattggag ggtgttttgg 3900
gttttttttt tatttttttgt tgtaggttgt taattattag attttaatag gttgtttttt 3960
gagattttttg atttgtgga gtttagagtt tgaagttttg gtgttagaat tttttgtata 4020
agatttgtg gtagtttta gttagtttta tttgtttatg tgtttttttt ttttagattt 4080
ttttttttat tgttttgttt taagttgttt tatagtttgt atttttttgtt ggtttttttt 4140
agattatttt atttggtttt tttattttat ttgtaatggg tttttatttt ttgaatatat 4200
ttgggttttt ggaatggttt ttgtttatgt ggttttattt ttatttggtg aatttttttt 4260
tgtagggagt ttttttgttt tgtttaattt gtttgttatt ggtttttttg gggagtgttt 4320

tatttttgtg gttattttgg gtattttggg atgatggttt tgtgttgttt tgtatatgtt 4380
tttgtttttt tttttatta gattttaga ttttttttt ttttttttg agatggagtt 4440
ttgttttgtt atttaggttg gagtgtaatg gtgtgatttt ggtttattat aatttttgtt 4500
ttttgggttt aagtgatttt tttgttttag tttttaagt agttgggatt atagatgtgt 4560
gttataatgt ttgtttaatt ttttgtattt ttagtagaga tggggtttta ttattttggt 4620
taggttggtt ttgaattttt gattttaagt gatttatttt ttttagtttt ttaaagtgtt 4680
gggattatag gtatgagttt gggtttagat atttagattt ttattaatga tttttttggt 4740
tttaattttt gggttttttt tatttggtat agtgtttggt ttttgttatg ttagttttta 4800
tttttatgt atataaatgg tgtttagtaa atatttatgt attgagtaaa atttaataat 4860
tatttgttga aattaaaaag tgaataaata agttatttag aaagatgtaa agtttataaa 4920
tttggggtat tttgtatttt ttttgagtgt aatgtttgta tattaggatg tgaggattat 4980
gtttttttt tatgttttga gggttttata tttgttttat tggatagttg ttgatgttat 5040
tggagaagga agttggatgg gtgtgtgtat gataatatta aggaatttag tttataattt 5100
atttgtttt ttatttgtgt atttttagag atgtgtatag tggtttttg tgaaagatag 5160
aattgtggtt tttttggtgt tatgtttttt tagtgtgtaa ataagggttg ttgttttgat 5220
gatattgttt gtggggtttt ttggtgtttt tattttaata ttattgatgt ttttttagaa 5280
ggtatggttt ttttatatga tgggttttga agatttagaa ttagttagaa aagttattta 5340
agattataga ggttttgatt agtattatta gttatgtttt tatatagagt tatggttgtt 5400
agtggtggtg taatggggta gtttgagtta ggttgtattt aggtttagga atagaaaggt 5460
agggttaagg gatttgggaa gaaatttgat tttttttgg ttttttttta tatttttaat 5520
taaaagtttg ggaagagtta ttgttggtaa tgtttttag tttgtttagg atagaggggg 5580
aaggtatgat gaaatttgaa gatattttat gtattttttt tttttttttt tttttgaaat 5640
ggagttttgt tttgttgttt ttgagttgga gtgtaatggt gtgattttgg tttattgtaa 5700
tttttgtttt ttgagtttaa ttttagtttt ttagtagttg agattatagg tgtgtgttat 5760
tatgtttagt taaatttttt ttgtattttt agtatagatg gggttttatt atgttggtta 5820
gattggtttt gaatttttga ttttaggtga tttgtttgtt ttagtttttt agagagttgg 5880
gattataggt gtgagttatt gtgtttggtt gatagtttat gttttttaaa gaatgtgttt 5940
atggatattt taaagtaaaa atttgtaat tgtttaaatg tgaaagaaaa tgtttatttt 6000
tattaaagta ttttttttt ttttttttt atttttgtag aggagtgtga atttagata 6060
tttttgtagg gatttgtttg tattttgatg tggtgttgtt tttagtatgg tgattagttt 6120
tagagtttgg ttgttatttt tattggatat tttagatatg tttttgtagt tgtgttttgg 6180
tttataatat agattgattg ttttgatttt gattatttaa aattggttta aaaattaaaa 6240
gagattgata ttaatttgtg ttgtttattt ttttaaagaa tatgaatgat ttttttttt 6300
ttgaaagtga agtgtagtgt tttattttgg gtttttgtag aggttttgta ttttttgggt 6360
tttttgagtt gggatataag tgggtagttg agtgtagaaa gtagggatgg tggggtgtat 6420
agtaggatag tggggtgtgt agtaggatag tggggtgtgt agtaggatgg tggggtgtat 6480
agtaggatag tggggtgtgt agtaggatgg tggggtgtgt agtgggatgg tggggtgtgt 6540
agtaggatgt aagtttaaga tgtattttg tttaggtatg aaaatggata ttgatttttt 6600
tggtattttt taattattta ttgtggatgt tttagtgatt aagtgatata agttagtttt 6660
ttgtttattt gtttttttaa atagaaattg gtgtaggaga tgaaatttgt agtagaatgt 6720
ttgaaagtat gtgtaataaa aattgtagag ggtggtatgg attgattttt ttaggaaaat 6780
tttttttaa ttttagattt gaatgaatta gaaaatagaa tagtagagga gggatataga 6840
agttaaaatg ttattttatt tttattggtt tttttgtagt ttggtaagga ttgggagagg 6900
gtgtttttt aatttagttg tttttgtttt ggggatttta gtttagtaag ttttaagtta 6960
aatgtatttt gggttttttt tttttttatt tgttttttta tttggtgttt ttgtgttggg 7020
tatatgttgt atagttatat tggagatggg attttttggg agttttggaa agtttttgtt 7080
ttttgtagtt agatggtttt agggaggaag agggaggtgt agaattgagt ggggttagtt 7140
tttttatat taattagttt ggtaaattat ttttaaattt ttggttatta tgtttagtag 7200
ataaagaggt ttgagaggtt gggagatatt tattaagaag aattattagg aatgggaagt 7260
tttttgtttt ttgtatatag tagtatatat atgagttgta ataaatatata tgttatgtat 7320
gtgttagggg ggttatagat atttttttt atttttaga tgtgtaagga agttttagtt 7380
tttttttttt atttatggta tgtttgattt tatattttt gtttgttaat tataggtaaa 7440
aatgagttaa ttttatttag tttaattttt tttaaaattt agtttgttat aataatttgt 7500
atagttttg ttagttatag tttttgggaa tgtattgttt agtttttat tttgttttat 7560
tttgtttgt ttttttatt tttgggtggg gtgaaggagt ttttggggtt tgtttatgtt 7620
ttttggattt ttgttggttt attgtggagt agaagttggg ggtttttaga gtttagttgg 7680
tgtgtggttt gtttgttttt tttatagttt tttagtgtta gaaaggtggg tttttttatat 7740
aatatttgt agttgattgt ttttggttg tttattattt gttttttttt atttaatttg 7800
tattttatga aggaaggggg agaaagaatt tattttttta gtttttttt tagttagaag 7860
tggttgtgta atttatgtg gtttatgaga tatagggggt atttttttga aggtggggta 7920
ggttttttt ttgatgaatg aagagtgttt tttggttttt taggttagtt tttgtagttt 7980
gtttttgggt gttgtggaag gtgggtgtga tgtggtgttg taggtagtta ttatgtaata 8040
atgaggtaag gtttaagatt tagagtttta ataattttga gttattgatt tgagtttttt 8100

```
atttattttg agatattatg ttaaataaat ttaagtgtgt ttgtgggtta aattattttt    8160
ggtttggttt tttgtggtat tttagtatat tattaaggtt tatatttttt atttattata    8220
gagtagtgag tttatggttt agttatgatg gt                                  8252
```

## Claims

1. An in-vitro method for providing a prognosis but not predicting the outcome of endocrine treatment of a subject with a cell proliferative disorder of the breast tissue comprising

   a) determining the expression of the gene PITX2 and/or regulatory sequences thereof within a biological sample from the subject, and
   b) determining therefrom the prognosis but not predicting the outcome of endocrine treatment of said subject

2. A method according to Claim 1, wherein, a) comprises determining the expression of the genes PITX2, TFF1 and PLAU and/or regulatory sequences thereof.

3. A method according to Claim 1 further **characterized in that** in step b) the expression of the genes PITX2 and PLAU and/or regulatory sequences thereof is determined.

4. A method according to Claim 1, wherein, a) comprises determining the expression of the genes PITX2 and TFF1 and/or regulatory sequences thereof.

5. The method according to claims 1 to 4 wherein said-cells from said breast cell proliferative disorder tissue are estrogen receptor positive.

6. The method according to claims 1 to 5, wherein said cell proliferative disorder of the breast tissue is selected from the group consisting of ductal carcinoma *in situ,* invasive ductal carcinoma, invasive lobular carcinoma, lobular carcinoma *in situ,* comedocarcinoma, inflammatory carcinoma, mucinous carcinoma, scirrhous carcinoma, colloid carcinoma, tubular carcinoma, medullary carcinoma, metaplastic carcinoma, and papillary carcinoma and papillary carcinoma *in situ,* undifferentiated or anaplastic carcinoma and Paget's disease of the breast.

7. A method according to claims 1 to 6 wherein said expression is determined by analysis of at least one of mRNA expression, LOH, protein expression.

8. A method according to claims 1 to 7 wherein said expression is determined by determining the methylation status of one or more CpG positions within said genes and/or regulatory regions thereof.

9. A method for providing a prognosis but not predicting the outcome of endocrine treatment of a subject with a cell proliferative disorder of the breast tissue comprising,

   a. isolating genomic DNA from a biological sample taken from said sbject;
   b. treating the genomic DNA, or a fragment thereof, with one or more reagents to convert 5-position unmethylated cytosine bases to uracil or to another base that is detectably dissimilar to cytosine in terms of hybridization properties;
   c. contacting the treated genomic DNA, or the treated fragment thereof, with an amplification enzyme and at least two primers comprising, in each case a contiguous sequence at least 18 nucleotides in length that is complementary to, or hybridizes under moderately stringent or stringent conditions to a sequence selected from the group consisting of SEQ ID NO: 150, 151, 155 and 156 and complements thereof, wherein the treated DNA or a fragment thereof is either amplified to produce one or more amplificates, or is not amplified;
   d. determining, based on the presence or absence of, or on the quantity or on a property of said amplificate, the methylation state of at least one CpG dinucleotide sequence of SEQ ID NO: 149, or an average, or a value reflecting an average methylation state of a plurality of CpG dinucleotide sequences of SEQ ID NO: 149 and
   e. determining from said methylation state the prognosis but not predicting the outcome of endocrine treatment of said subject

10. A method according to claim 9 further comprising in step c) at least two primers comprising, in each case a contiguous sequence at least 18 nucleotides in length that is complementary to, or hybridizes under moderately stringent or stringent conditions to a sequence selected from the group consisting of SEQ ID NO: 76 to 103 and SEQ ID NO: 153, 154, 157 and 158 and complements thereof.

11. A method according to claims 9 and 10 wherein said one or more reagents comprises a solution selected from the group consisting of bisulfite, hydrogen sulfite, disulfite, and combinations thereof.

12. The method according to claims 9 and 10 wherein d) is carried out by means of one or more methods taken from the group consisting oligonucleotide hybridization analysis, Ms-SnuPE, sequencing, Real Time detection probes and oligonucleotide array analysis.

13. The use of a nucleic acid molecule consisting of a sequence at least 18 bases in length according to one of the sequences taken from the group consisting of SEQ ID NO: 2-5, SEQ ID NO: 151 to SEQ ID NO: 158 and SEQ ID NO: 76 to SEQ ID NO: 103 in providing a prognosis but not predicting the outcome of endocrine treatment of a subject with a cell proliferative disorder of the breast tissue.

14. The use of an oligomer, in particular an oligonucleotide or peptide nucleic acid (PNA)-oligomer, said oligomer consisting essentially of at least one base sequence having a length of at least 10 nucleotides which hybridizes to or is identical to one of the nucleic acid sequences according to SEQ ID NO: 2-5, SEQ ID NO: 151 to SEQ ID NO: 158 and SEQ ID NO: 76 to SEQ ID NO: 103 in providing a prognosis but not predicting the outcome of endocrine treatment of a subject with a cell proliferative disorder of the breast tissue.

15. The use of a composition comprising the following:

- a nucleic acid comprising a sequence at least 18 bases in length of a segment of the chemically pretreated genomic DNA according to one of the sequences taken from the group comprising SEQ ID NO: 2 to SEQ ID NO: 5 SEQ ID NO: 151, 152, 155 and 156 and sequences complementary thereto, and
- a buffer comprising at least one of the following substances: magnesium chloride, dNTP, of taq polymerase, an oligomer, in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pre-treated genomic DNA according to one of the SEQ ID NO: 2 to SEQ ID NO: 5, SEQ ID NO: 151, 152, 155 and 156 and sequences complementary thereto in providing a prognosis but not predicting the outcome of endocrine treatment of a subject with a cell proliferative disorder of the breast tissue.

**Patentansprüche**

1. In-Vitro-Verfahren zur Bereitstellung einer Prognose, jedoch keiner Vorhersage des Ergebnisses einer endokrinen Behandlung eines Subjekts mit einer zellproliferativen Störung des Brustgewebes, umfassend

a) Ermittlung der Expression des Gens PITX2 und/oder regulatorischer Sequenzen davon in einer biologischen Probe des Subjekts und
b) Ermittlung der Prognose, jedoch nicht der Vorhersage des Ergebnisses der endokrinen Behandlung des Subjekts daraus.

2. Verfahren nach Anspruch 1, wobei a) die Ermittlung der Expression der Gene PITX2, TFFI und PLAU und/oder regulatorischer Sequenzen davon umfasst.

3. Verfahren nach Anspruch 1, ferner **dadurch gekennzeichnet, dass** in Schritt b) die Expression der Gene PITX2 und PLAU und/oder regulatorischer Sequenzen davon ermittelt wird.

4. Verfahren nach Anspruch 1, wobei a) die Ermittlung der Expression der Gene PITX2 und TFFI und/oder regulatorischer Sequenzen davon umfasst.

5. Verfahren nach den Ansprüchen 1 bis 4, wobei Zellen des Brustgewebes mit der zellproliferativen Störung Östrogenrezeptor-positiv sind.

**6.** Verfahren nach den Ansprüchen 1 bis 5, wobei die zellproliferative Störung des Brustgewebes ausgewählt ist aus der Gruppe bestehend aus duktalem Karzinom *in situ,* invasivem duktalem Karzinom, invasivem lobulärem Karzinom, lobulärem Karzinom *in situ,* Komedokarzinom, inflammatorischem Karzinom, muzinösem Karzinom, Carcinoma scirrhosum, Kolloidkarzinom, tubulärem Karzinom, medullärem Karzinom, metaplastischem Karzinom und papillärem Karzinom und papillärem Karzinom *in situ,* undifferenziertem oder anaplastischem Karzinom und Morbus Paget der Brust.

**7.** Verfahren nach den Ansprüchen 1 bis 6, wobei die Expression durch Analyse mindestens eines der Folgenden ermittelt wird: mRNA-Expression, LOH, Proteinexpression.

**8.** Verfahren nach den Ansprüchen 1 bis 7, wobei die Expression durch Ermittlung des Methylierungsstatus einer oder mehrerer CpG-Stellen innerhalb der Gene und/oder regulatorischer Regionen von diesen ermittelt wird.

**9.** Verfahren zur Bereitstellung einer Prognose, jedoch keiner Vorhersage des Ergebnisses einer endokrinen Behandlung eines Subjekts mit einer zellproliferativen Störung des Brustgewebes, umfassend

a. Isolierung genomischer DNA aus einer biologischen Probe, die dem Subjekt entnommen wurde;
b. Behandeln der genomischen DNA oder eines Fragments davon mit einem oder mehreren Reagenzien, um an der 5-Position unmethylierte Cytosinbasen in Uracil oder eine andere Base umzuwandeln, die Cytosin im Hinblick auf Hybridisierungseigenschaften nachweisbar unähnlich ist;
c. In-Kontakt-Bringen der behandelten genomischen DNA oder des behandelten Fragments davon mit einem Amplifizierungsenzym und mindestens zwei Primern, umfassend in jedem Fall eine angrenzende Sequenz mit einer Länge von mindestens 18 Nukleotiden, die komplementär ist zu, oder die unter mäßig stringenten oder stringenten Bedingungen mit einer Sequenz hybridisiert, die ausgewählt ist aus der Gruppe bestehend aus SEQ ID NO: 150, 151, 155 und 156 und Komplementen davon, wobei die behandelte DNA oder ein Fragment davon entweder amplifiziert ist, um ein oder mehrere Amplifikate zu erzeugen, oder nicht amplifiziert ist;
d. Ermittlung des Methylierungsstatus mindestens einer CpG-Dinukleotidsequenz der SEQ ID NO: 149 oder eines Durchschnitts oder eines Werts, der einen durchschnittlichen Methylierungsstatus einer Mehrzahl von CpG-Dinukleotidsequenzen der SEQ ID NO: 149 wiedergibt, auf Basis der An- oder Abwesenheit oder der Quantität oder einer Eigenschaft des Amplifikats und
e. Ermittlung der Prognose, jedoch nicht der Vorhersage des Ergebnisses der endokrinen Behandlung des Subjekts aufgrund des Methylierungsstatus.

**10.** Verfahren nach Anspruch 9, ferner umfassend mindestens zwei Primer in Schritt c), umfassend in jedem Fall eine angrenzende Sequenz mit einer Länge von 18 Nukleotiden, die komplementär ist zu, oder die unter mäßig stringenten oder stringenten Bedingungen mit einer Sequenz hybridisiert, die ausgewählt ist aus der Gruppe bestehend aus SEQ ID NO: 76 bis 103 und SEQ ID NO: 153, 154, 157 und 158 und Komplementen davon.

**11.** Verfahren nach den Ansprüchen 9 und 10, wobei das eine oder die mehreren Reagenzien eine Lösung umfassen, ausgewählt aus der Gruppe bestehend aus Bisulfit, Hydrogensulfit, Disulfit und Kombinationen davon.

**12.** Verfahren nach den Ansprüchen 9 und 10, wobei d) mittels eines oder mehrerer der Verfahren durchgeführt wird aus der Gruppe bestehend aus Oligonukleotid-Hybridisierungsanalyse, Ms-SNuPE, Sequenzierung, Echtzeit-Detektionssonden und Oligonukleotid-Array-Analyse.

**13.** Verwendung eines Nukleinsäuremoleküls, bestehend aus einer Sequenz mit einer Länge von mindestens 18 Basen nach einer der Sequenzen aus der Gruppe bestehend aus SEQ ID NO: 2 bis 5, SEQ ID NO: 151 bis SEQ ID NO: 158 und SEQ ID NO: 76 bis SEQ ID NO: 103 bei der Bereitstellung einer Prognose, jedoch nicht zur Vorhersage des Ergebnisses einer endokrinen Behandlung eines Subjekts mit einer zellproliferativen Störung des Brustgewebes.

**14.** Verwendung eines Oligomers, insbesondere eines Oligonukleotids oder Peptid-Nukleinsäure- (PNA-)Oligomers, wobei das Oligomer im Wesentlichen aus mindestens einer Basensequenz mit einer Länge von mindestens 10 Nukleotiden besteht, welche hybridisiert mit, oder welche identisch ist mit einer der Nukleinsäuresequenzen nach SEQ ID NO: 2 bis 5, SEQ ID NO: 151 bis SEQ ID NO: 158 und SEQ ID NO: 76 bis SEQ ID NO: 103 bei der Bereitstellung einer Prognose, jedoch nicht zur Vorhersage des Ergebnisses einer endokrinen Behandlung eines Subjekts mit einer zellproliferativen Störung des Brustgewebes.

**15.** Verwendung einer Zusammensetzung, die Folgendes umfasst:

- eine Nukleinsäure, umfassend eine mindestens 18 Basen lange Sequenz eines Abschnitts der chemisch vorbehandelten genomischen DNA nach einer der Sequenzen aus der Gruppe, umfassend SEQ ID NO: 2 bis SEQ ID NO: 5, SEQ ID NO: 151, 152, 155 und 156 und dazu komplementären Sequenzen und
- einen Puffer, umfassend mindestens eine der folgenden Substanzen: Magnesiumchlorid, dNTP, Taq-Polymerase, ein Oligomer, insbesondere ein Oligonukleotid oder Peptid-Nukleinsäure- (PNA-)Oligomer, wobei das Oligomer in jedem Fall mindestens eine Basensequenz mit einer Länge von mindestens 9 Nukleotiden umfasst, welche komplementär ist zu, oder unter mäßig stringenten oder stringenten Bedingungen hybridisiert mit einer vorbehandelten genomischen DNA nach einer aus SEQ ID NO: 2 bis SEQ ID NO: 5, SEQ ID NO: 151, 152, 155 und 156 und Sequenzen, die dazu komplementär sind, bei der Bereitstellung einer Prognose, jedoch nicht zur Vorhersage des Ergebnisses einer endokrinen Behandlung eines Subjekts mit einer zellproliferativen Störung des Brustgewebes.

## Revendications

**1.** Méthode in vitro pour la fourniture d'un pronostic, et non la prédiction du résultat du traitement endocrine d'un sujet présentant un trouble prolifératif du tissu mammaire, comprenant :

a) la détermination de l'expression du gène PITX2 et/ou des séquences de régulation de ce dernier au sein d'un échantillon biologique du sujet, et
b) la détermination à partir de cela du pronostic, sans pour autant prédire le résultat du traitement endocrine dudit sujet.

**2.** Méthode selon la revendication 1, dans laquelle a) comprend la détermination de l'expression des gènes PITX2, TFFI et PLAU et/ou des séquences de régulation de ces derniers.

**3.** Méthode selon la revendication 1, en outre **caractérisée en ce que**, à l'étape b), l'expression des gènes PITX2 et PLAU et/ou des séquences de régulation de ces derniers est déterminée.

**4.** Méthode selon la revendication 1, dans laquelle a) comprend la détermination de l'expression des gènes PITX2 et TFFI et/ou des séquences de régulation de ces derniers.

**5.** Méthode selon les revendications 1 à 4, dans laquelle des cellules dudit tissu à troubles prolifératifs de cellules mammaires sont positives pour les récepteurs aux estrogènes.

**6.** Méthode selon les revendications 1 à 5, dans laquelle ledit trouble prolifératif de cellules du tissu mammaire est sélectionné dans le groupe constitué de carcinome ductal *in situ,* carcinome ductal invasif, carcinome lobulaire invasif, carcinome lobulaire *in situ,* comédocarcinome, carcinome inflammatoire, carcinome mucineux, carcinome squirrheux, carcinome colloïde, carcinome tubulaire, carcinome médullaire, carcinome métaplastique, et carcinome papillaire et carcinome papillaire *in situ,* carcinome indifférencié ou anaplastique et maladie de Paget du sein.

**7.** Méthode selon les revendications 1 à 6, dans laquelle ladite expression est déterminée par analyse d'au moins une parmi l'expression de mRNA, LOH, expression de protéine.

**8.** Méthode selon les revendications 1 à 7, dans laquelle ladite expression est déterminée par détermination de l'état de méthylation d'une ou plusieurs positions CpG au sein desdits gènes et/ou régions de régulation de ces derniers.

**9.** Méthode pour la fourniture d'un pronostic, et non la prédiction du résultat du traitement endocrine d'un sujet présentant un trouble prolifératif du tissu mammaire, comprenant :

a. l'isolation de l'ADN génomique d'un échantillon biologique prélevé dudit sujet ;
b. le traitement de l'ADN génomique, ou d'un fragment de ce dernier, avec un ou plusieurs réactifs afin de convertir des bases de cytosine non méthylées en position 5 en uracil ou en une autre base qui est détectable de façon dissemblable à la cytosine en ce qui concerne les propriétés d'hybridation ;
c. la mise en contact de l'ADN génomique, ou du fragment traité de ce dernier, avec un enzyme d'amplification et au moins deux amorces comprenant, dans chaque cas une séquence contiguë d'une longueur d'au moins 18 nucléotides qui est complémentaire à, ou s'hybride dans des conditions modérément rigoureuses ou des conditions rigoureuses à une séquence sélectionnée dans le groupe constitué de SEQ ID NO 150, 151, 155 et 156 et des compléments de ces dernières, où l'ADN traité ou un fragment de ce dernier est soit amplifié afin de produire un ou plusieurs amplificats, soit pas amplifié ;

d. la détermination, sur la base de la présence ou l'absence de, ou sur la base de la quantité ou d'une propriété dudit amplificat, de l'état de méthylation d'au moins une séquence dinucléotidique CpG de SEQ ID NO: 149, ou une moyenne, ou une valeur reflétant un état de méthylation moyen d'une pluralité de séquences dinucléotidiques CpG de SEQ ID NO: 149 et

e. la détermination à partir dudit état de méthylation du pronostic, sans pour autant prédire le résultat du traitement endocrine dudit sujet.

**10.** Méthode selon la revendication 9, comprenant en outre à l'étape c) d'au moins deux amorces comprenant, dans chaque cas, une séquence contiguë d'une longueur d'au moins 9 nucléotides qui est complémentaire à ou s'hybride dans des conditions modérément rigoureuses ou des conditions rigoureuses à une séquence sélectionnée dans le groupe constitué de SEQ ID NO: 76 à 103 et SEQ ID NO: 153, 154, 157 et 158 et des compléments à ces dernières.

**11.** Méthode selon les revendications 9 et 10, dans laquelle ledit ou lesdits réactif(s) comprend (comprennent) une solution sélectionnée dans le groupe constitué de bisulfite, sulfite d'hydrogène, disulfite et de combinaisons de ces derniers.

**12.** Méthode selon les revendications 9 et 10, dans laquelle d) est réalisée au moyen d'une ou plusieurs méthodes choisies dans le groupe constitué de l'analyse d'hybridation oligonucléotidique, Ms-SnuPE, le séquençage, sondes de détection en temps réel et analyse de matrice oligonucléotidique.

**13.** Utilisation d'une molécule d'acide nucléique constituée d'une séquence d'une longueur d'au moins 18 bases conformément à l'une des séquences choisies dans le groupe constitué des SEQ ID NO: 2-5, SEQ ID NO: 151 à SEQ ID NO: 158 et SEQ ID NO: 76 à SEQ ID NO: 103 afin de fournir un pronostic, et non prédire le résultat du traitement endocrine d'un sujet présentant un trouble prolifératif du tissu mammaire.

**14.** Utilisation d'un oligomère, en particulier d'un oligonucléotide ou oligomère d'acide nucléique peptidique (PNA), ledit oligomère étant constitué essentiellement d'au moins une séquence de base d'une longueur d'au moins 10 nucléotides qui s'hybride à ou est identique à une des séquences d'acide nucléique conformément à SEQ ID NO: 2-5, SEQ ID NO: 151 à SEQ ID NO: 158 et SEQ ID NO: 76 à SEQ ID NO: 103 afin de fournir un pronostic, et non prédire le résultat du traitement endocrine d'un sujet présentant un trouble prolifératif du tissu mammaire.

**15.** Utilisation d'une composition comprenant :

- un acide nucléique comprenant une séquence d'une longueur d'au moins 18 bases d'un segment de l'ADN génomique chimiquement prétraité conformément à une des séquences prises dans le groupe comprenant SEQ ID NO 2 à SEQ ID NO 5, SEQ ID NO: 151, 152, 155 et 156 et des séquences complémentaires à ces dernières, et
- un tampon comprenant au moins l'une des substances suivantes : chlorure de magnésium, dNTP, polymérase de Taq, un oligomère, en particulier d'un oligonucléotide ou oligomère d'acide nucléique peptidique (PNA), ledit oligomère comprenant dans chaque cas au moins une séquence de base d'une longueur d'au moins 9 nucléotides qui est complémentaire à ou s'hybride dans des conditions modérément rigoureuses ou des conditions rigoureuses à un ADN génomique prétraité conformément à une séquence parmi SEQ ID NO: 2 à SEQ ID NO: 5, SEQ ID NO: 151, 152, 155 et 156 et les séquences complémentaires à ces dernières afin de fournir un pronostic, et non prédire le résultat du traitement endocrine d'un sujet présentant un trouble prolifératif du tissu mammaire.

Figure 1

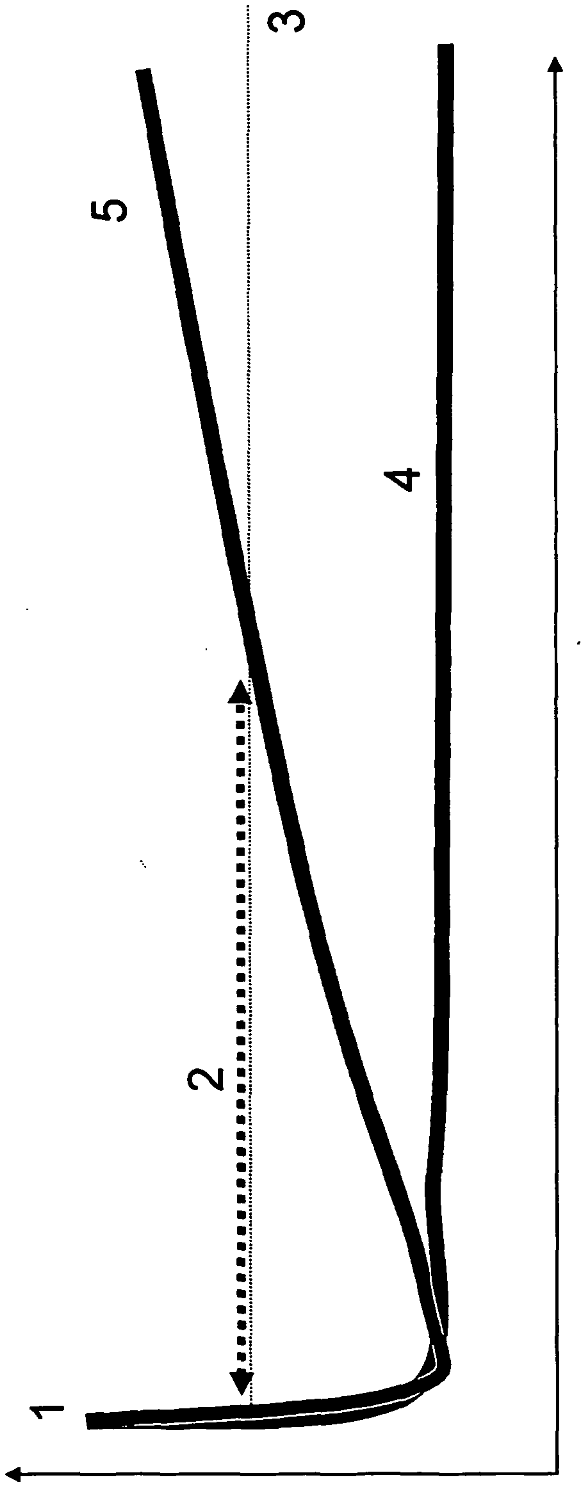

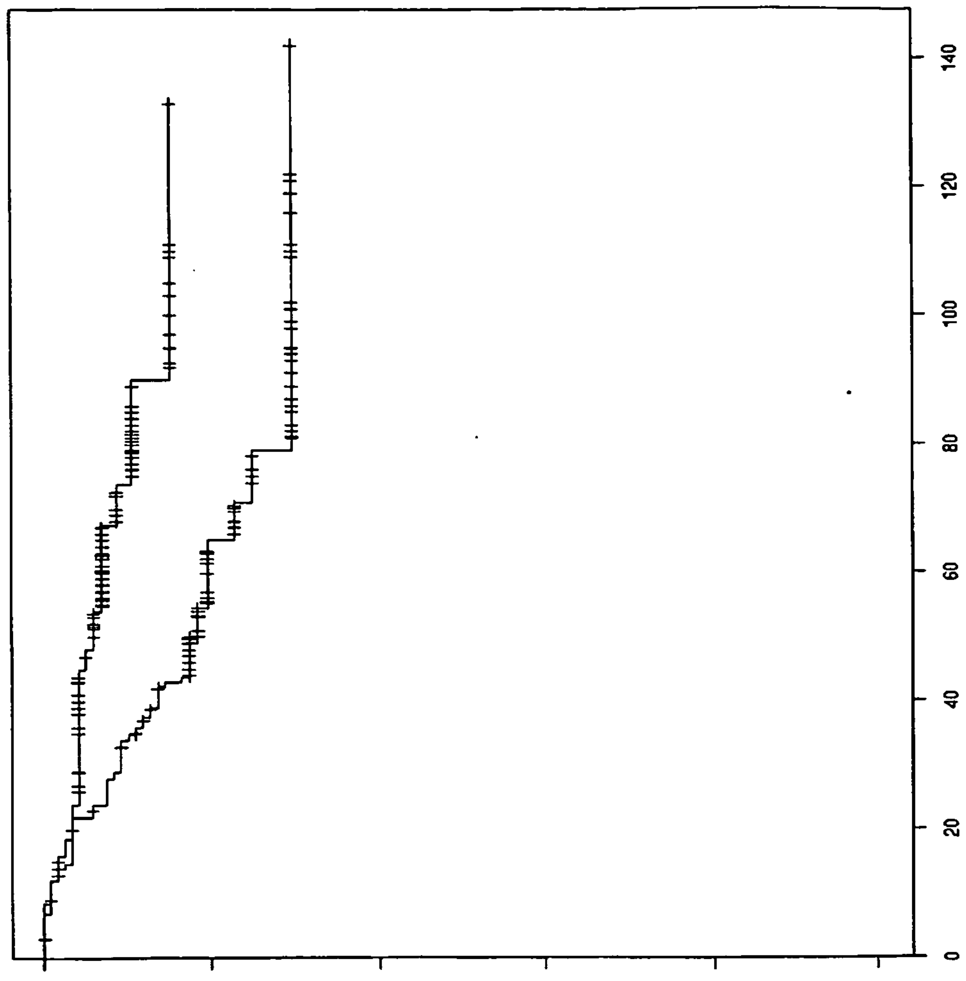


Figure 2

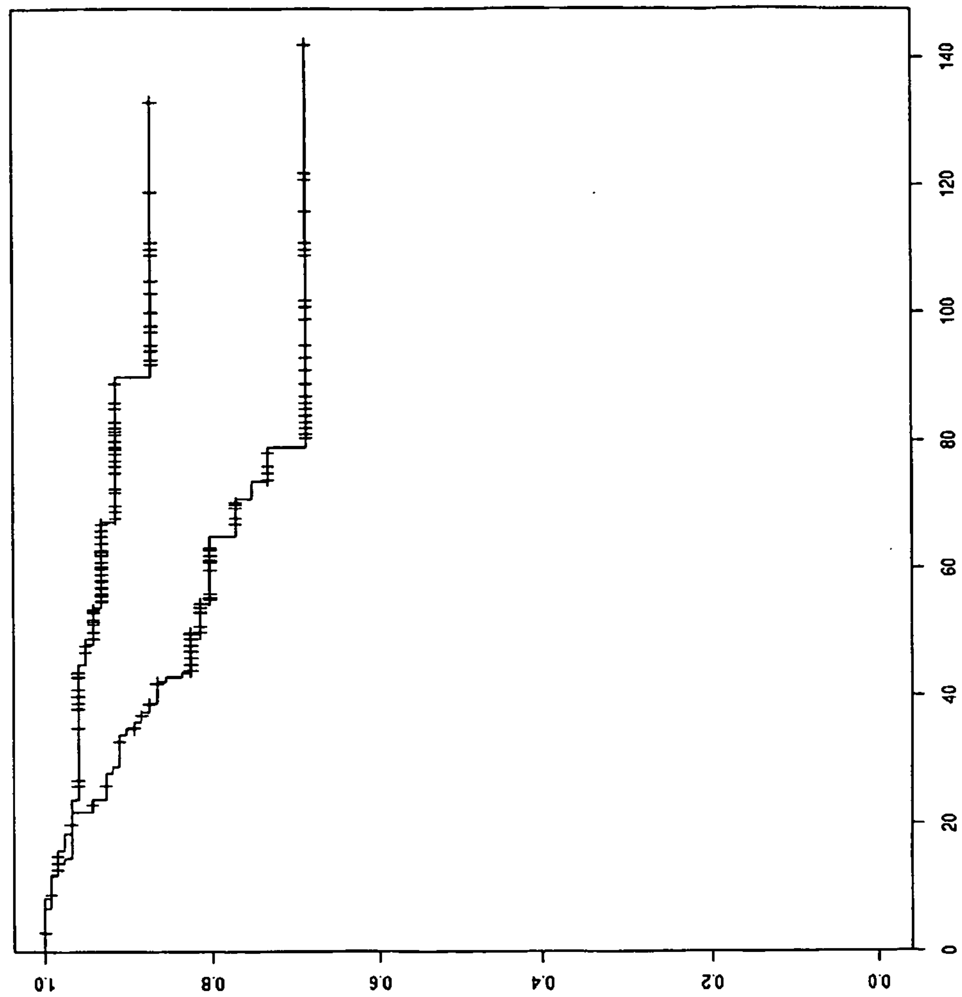


Figure 3

Figure 4

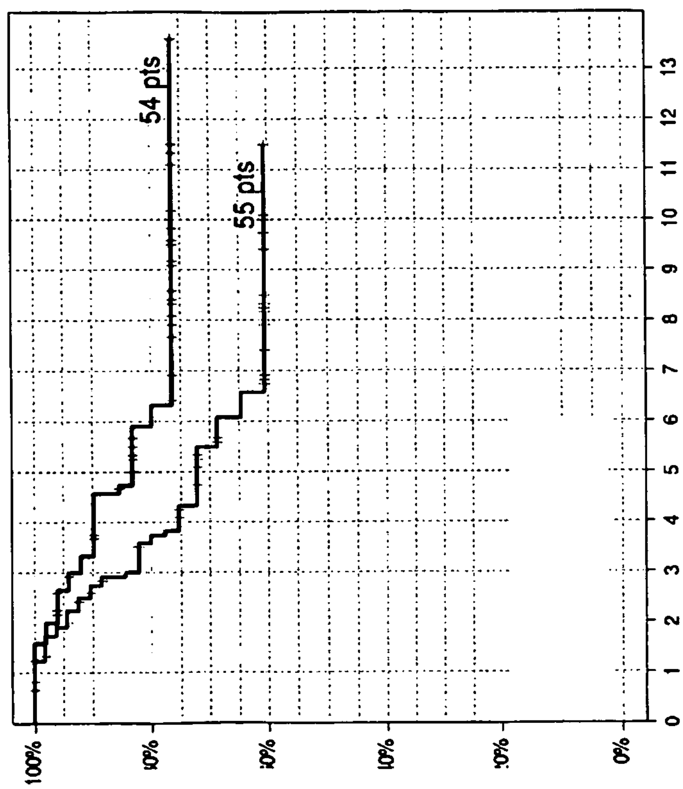

Figure 5

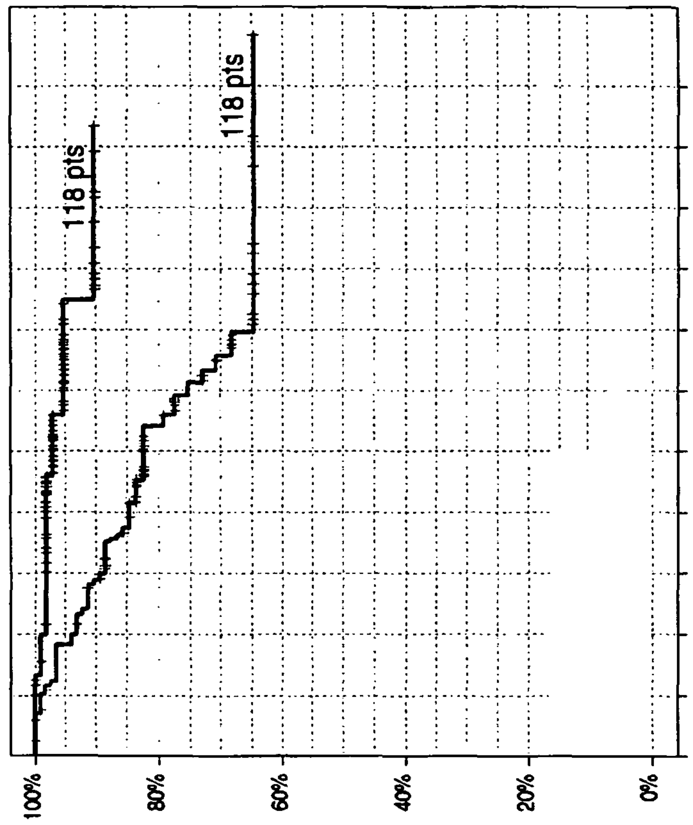

Figure 6

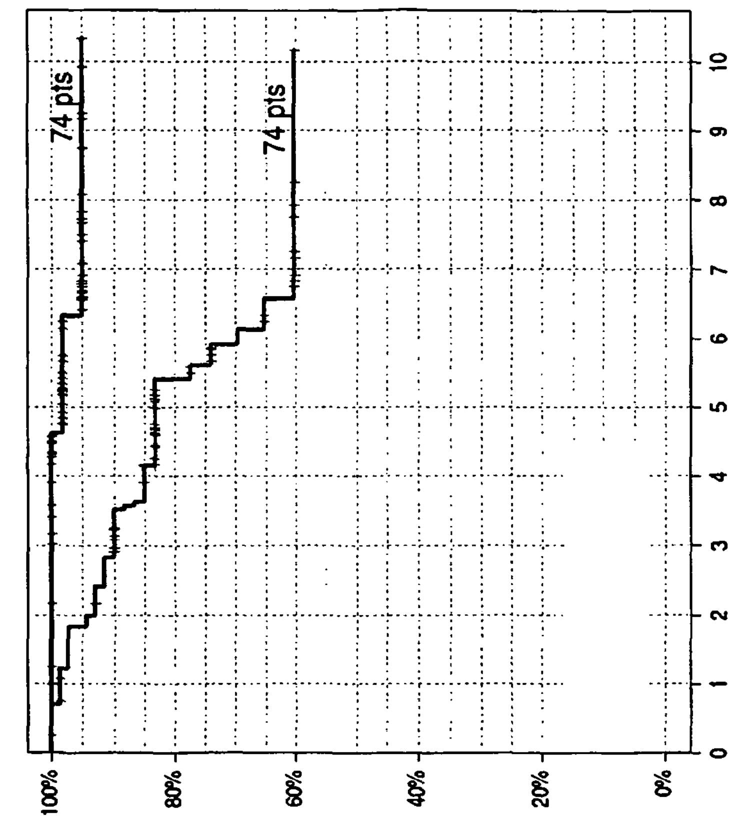

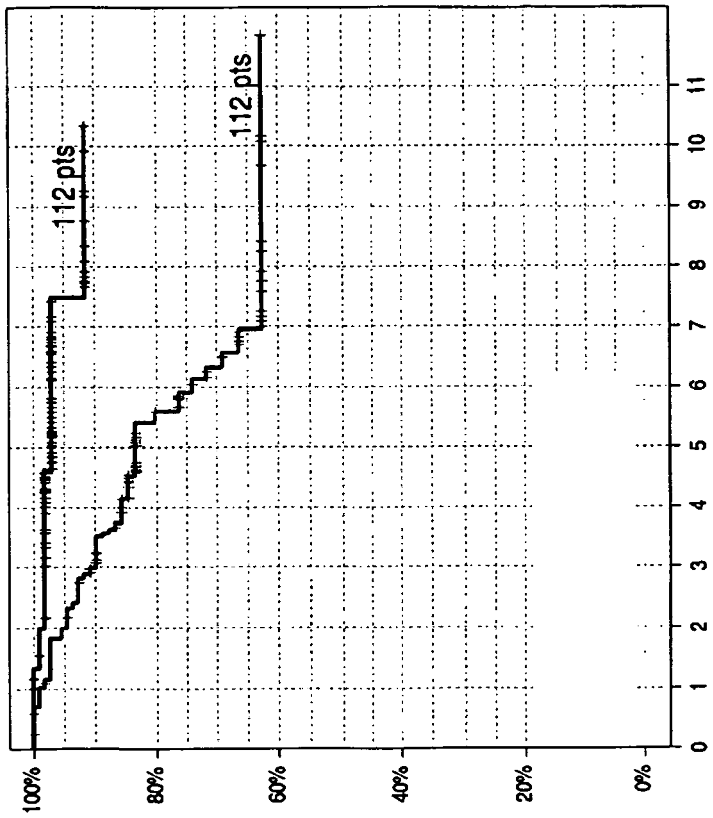


Figure 7

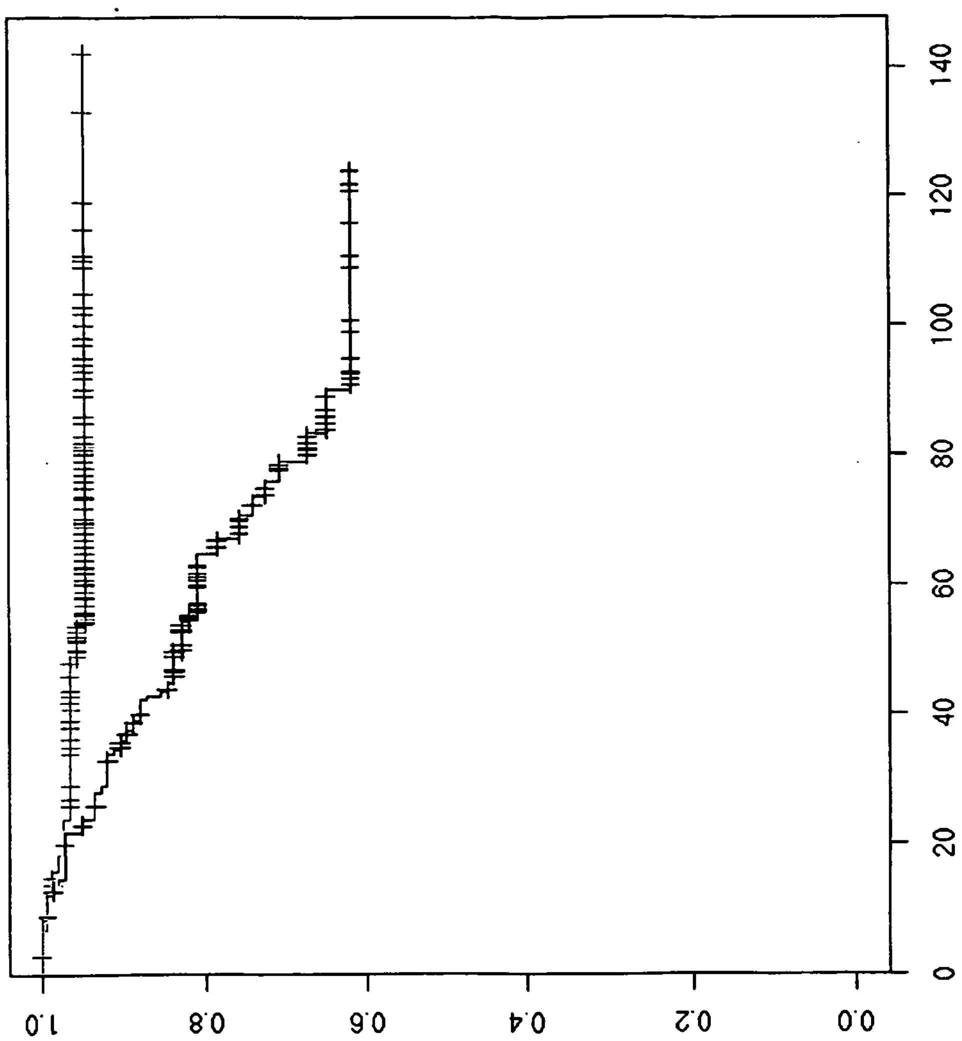


Figure 8

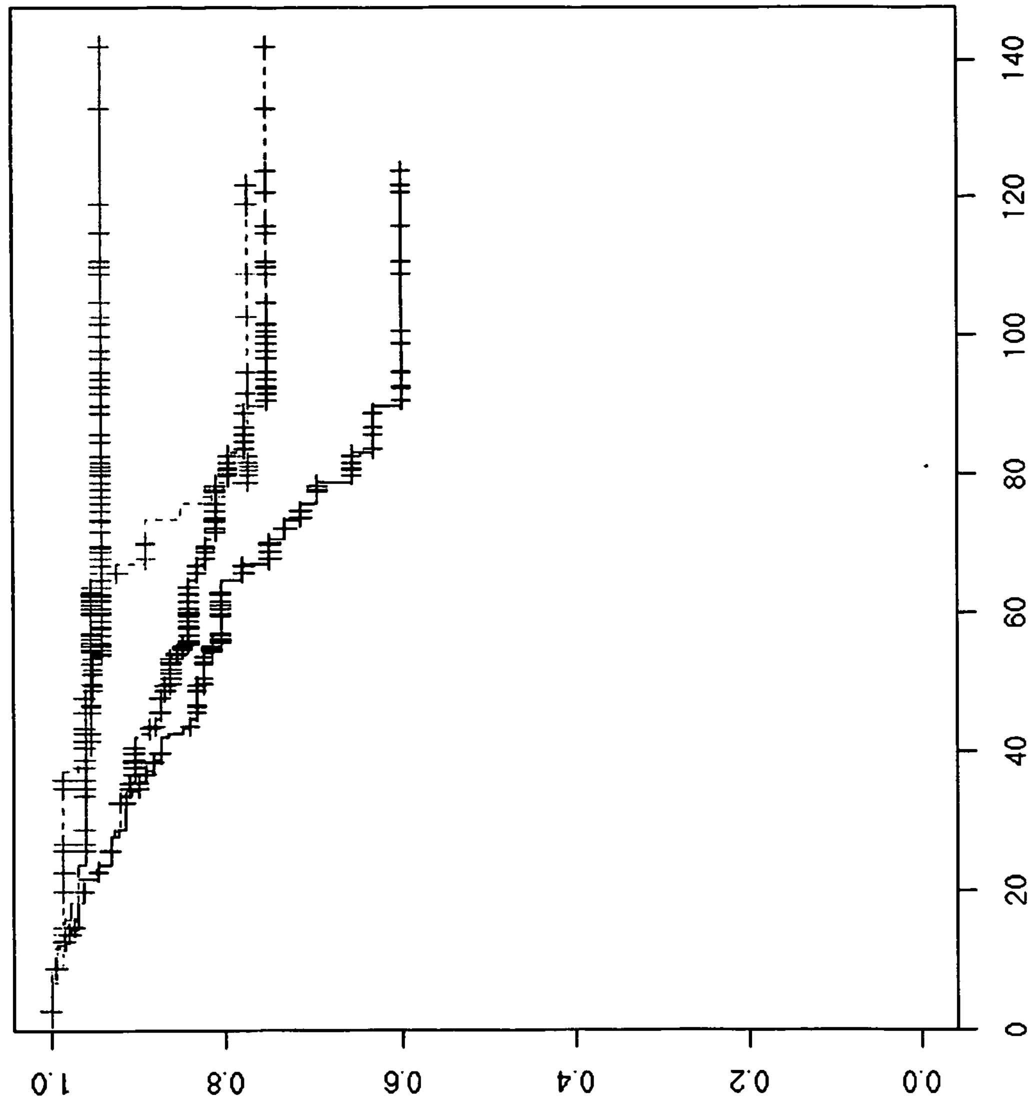


Figure 9

Figure 10

METNCRKLVSACVQLGVQPAAVECLFSKDSEIKKVEFTDSPESRKEAASSKFFPRQ
HPGANEKDKSQQGKNEDVGAEDPSKKKRQRRQRTHFTSQQLQELEATFQRNRYP
DMSTREEIAVWTNLTEARVRVWFKNRRAKWRKRERNQQAELCKNGFGPQFNGL
MQPYDDMYPGYSYNNWAAKGLTSASLSTKSFPFFNSMNVNPLSSQSMFSPPNSISS
MSMSSSMVPSAVTGVPGSSLNSLNNLNNLSSPSLNSAVPTPACPYAPPTPPYVYRDT
CNSSLASLRLKAKQHSSFGYASVQNPASNLSACQYAVDRPV

Figure 11

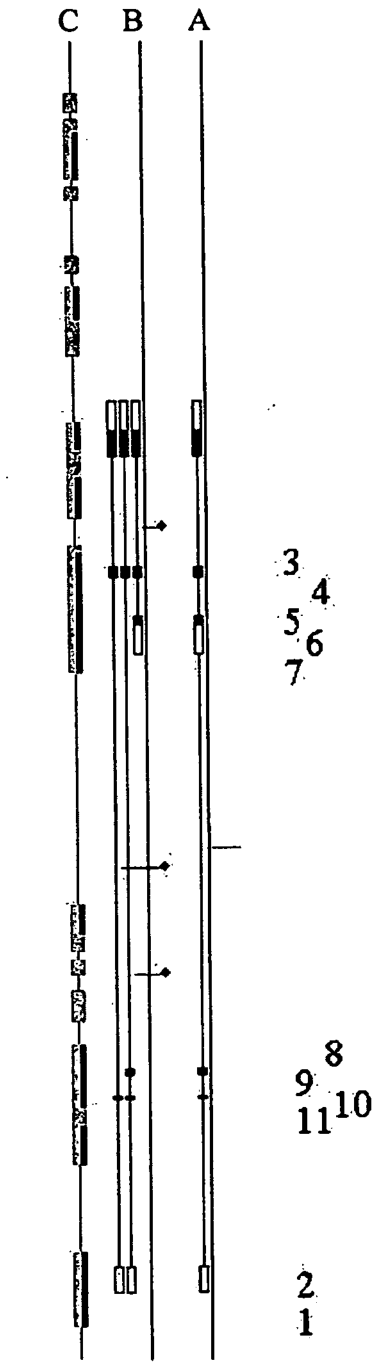

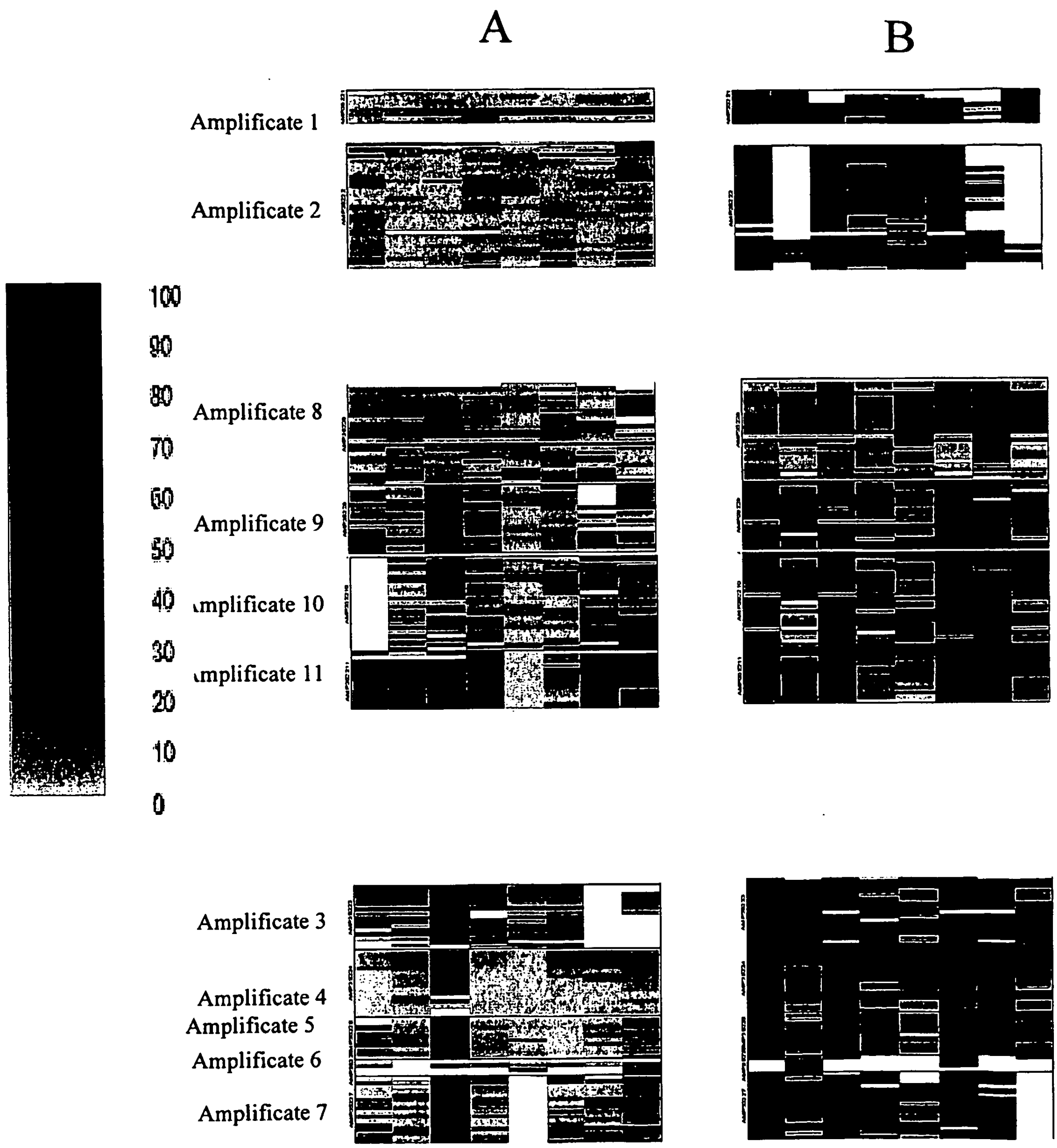
A
B
Amplificate 1
Amplificate 2
100
90
80
70
60
50
40
30
20
10
0
Amplificate 8
Amplificate 9
mplificate 10
mplificate 11
Amplificate 3
Amplificate 4
Amplificate 5
Amplificate 6
Amplificate 7

Figure 12

Figure 13

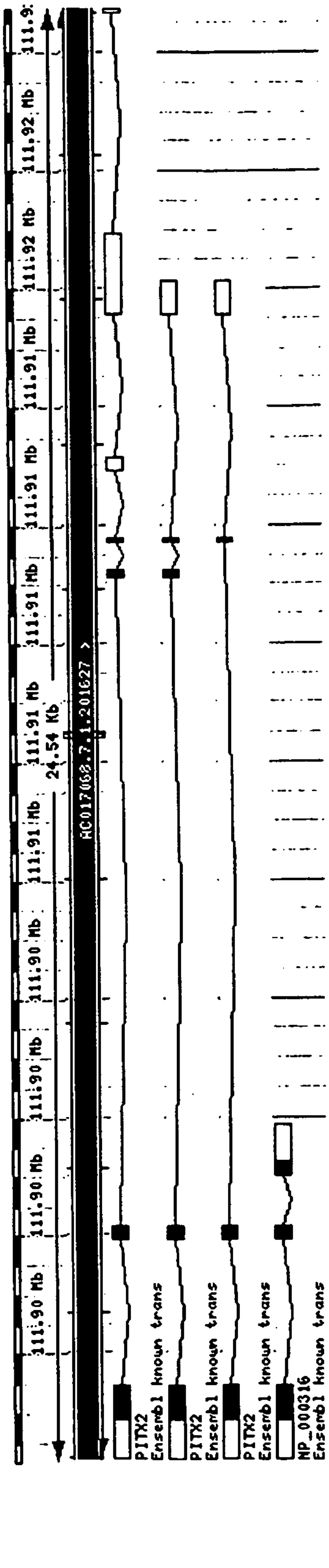
111.90 Mb
111.91 Mb
111.92 Mb
24.54 Kb
AC017068.7.1.201627 >
PITX2
Ensembl known trans
PITX2
Ensembl known trans
PITX2
Ensembl known trans
NP_000316
Ensembl known trans

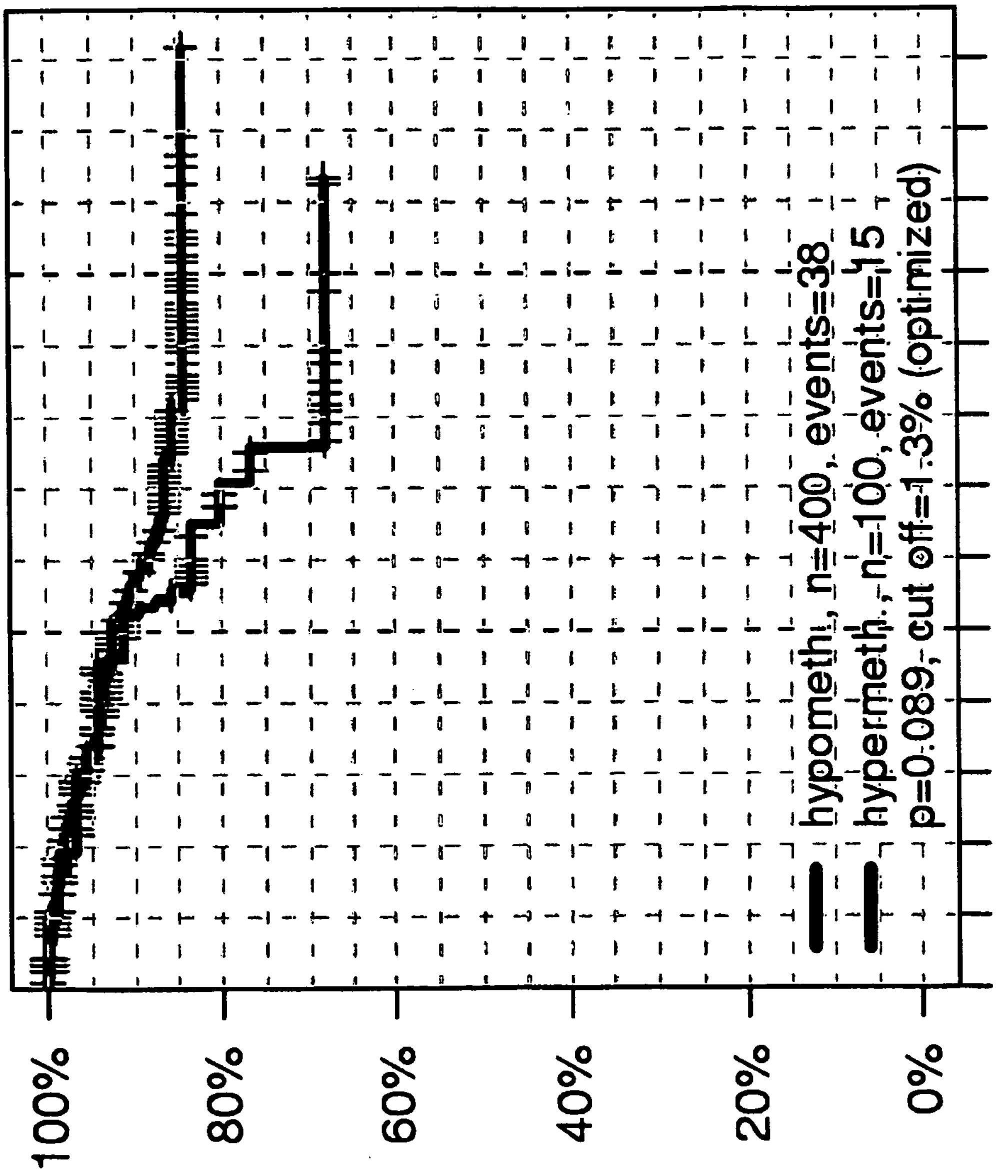
100%
80%
60%
40%
20%
0%
0
1
2
3
4
5
6
7
8
9
11
13
hypometh., n=400, events=38
hypermeth., n=100, events=15
p=0.089, cut off=1.3% (optimized)

**Figure 14**

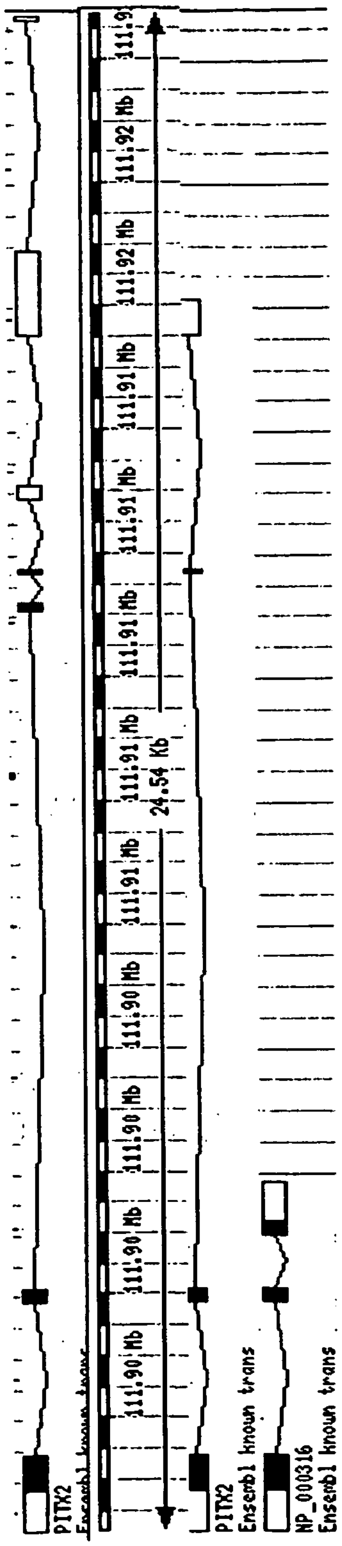
PITX2
Ensembl known trans
111.90 Mb
111.91 Mb
111.92 Mb
24.54 Kb
PITX2
Ensembl known trans
NP_000316
Ensembl known trans

Figure 13

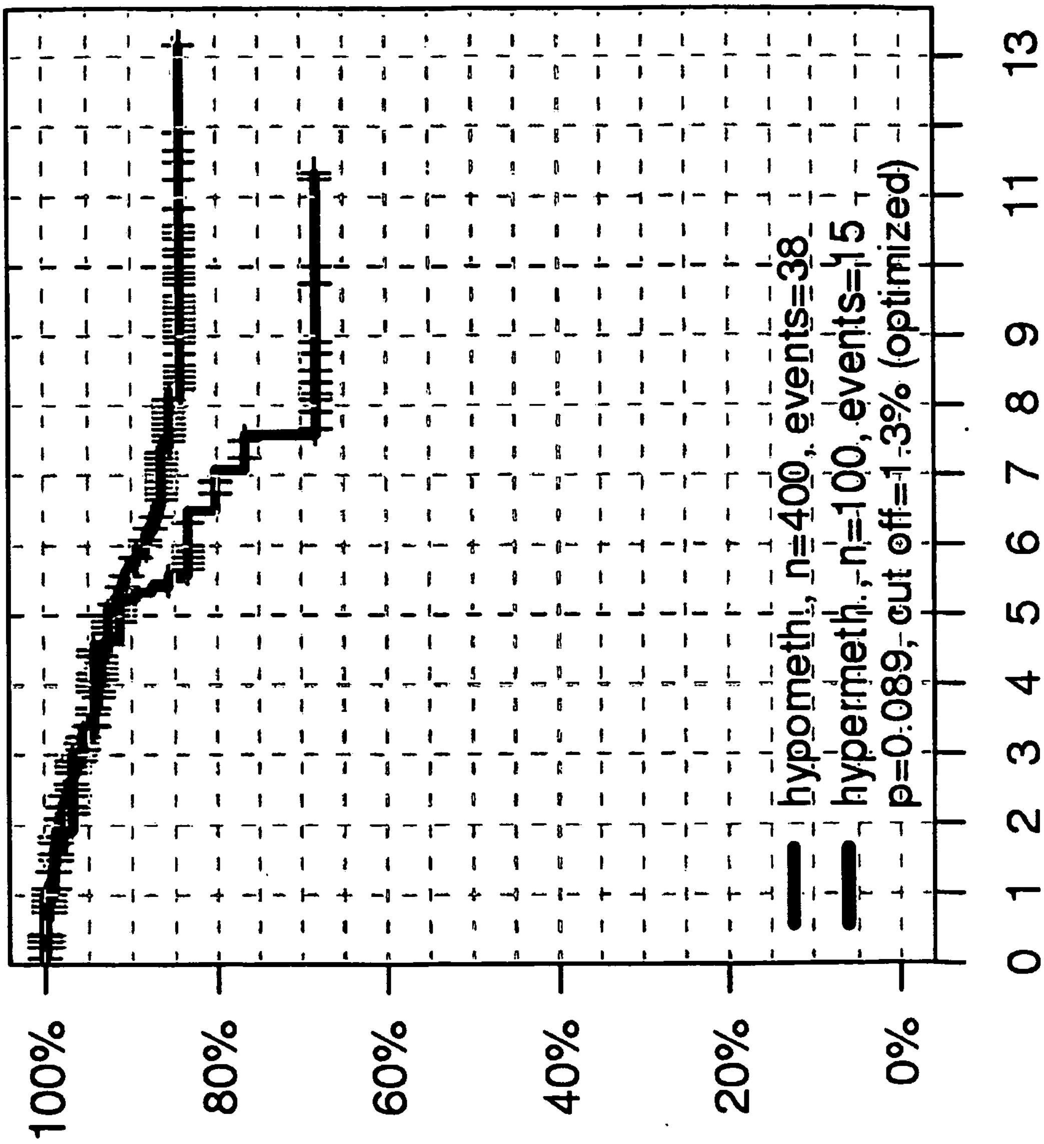
100%
80%
60%
40%
20%
0%
0 1 2 3 4 5 6 7 8 9 11 13
hypometh., n=400, events=38
hypermeth., n=100, events=15
p=0.089, cut off=1.3% (optimized)

Figure 15

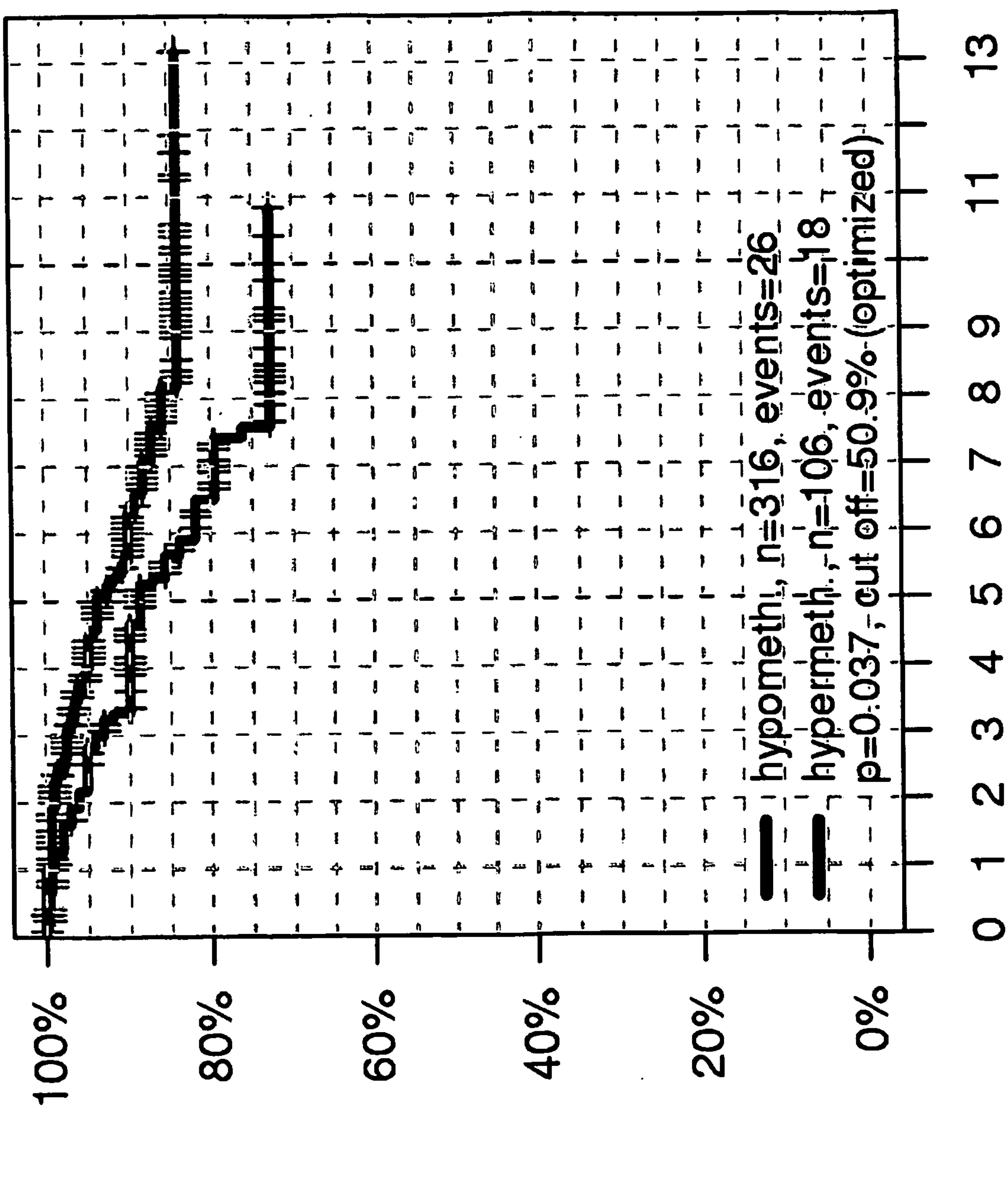

100%
80%
60%
40%
20%
0%
0 1 2 3 4 5 6 7 8 9 11 13
hypometh., n=316, events=26
hypermeth., n=106, events=18
p=0.037, cut off=50.9% (optimized)


Figure 16

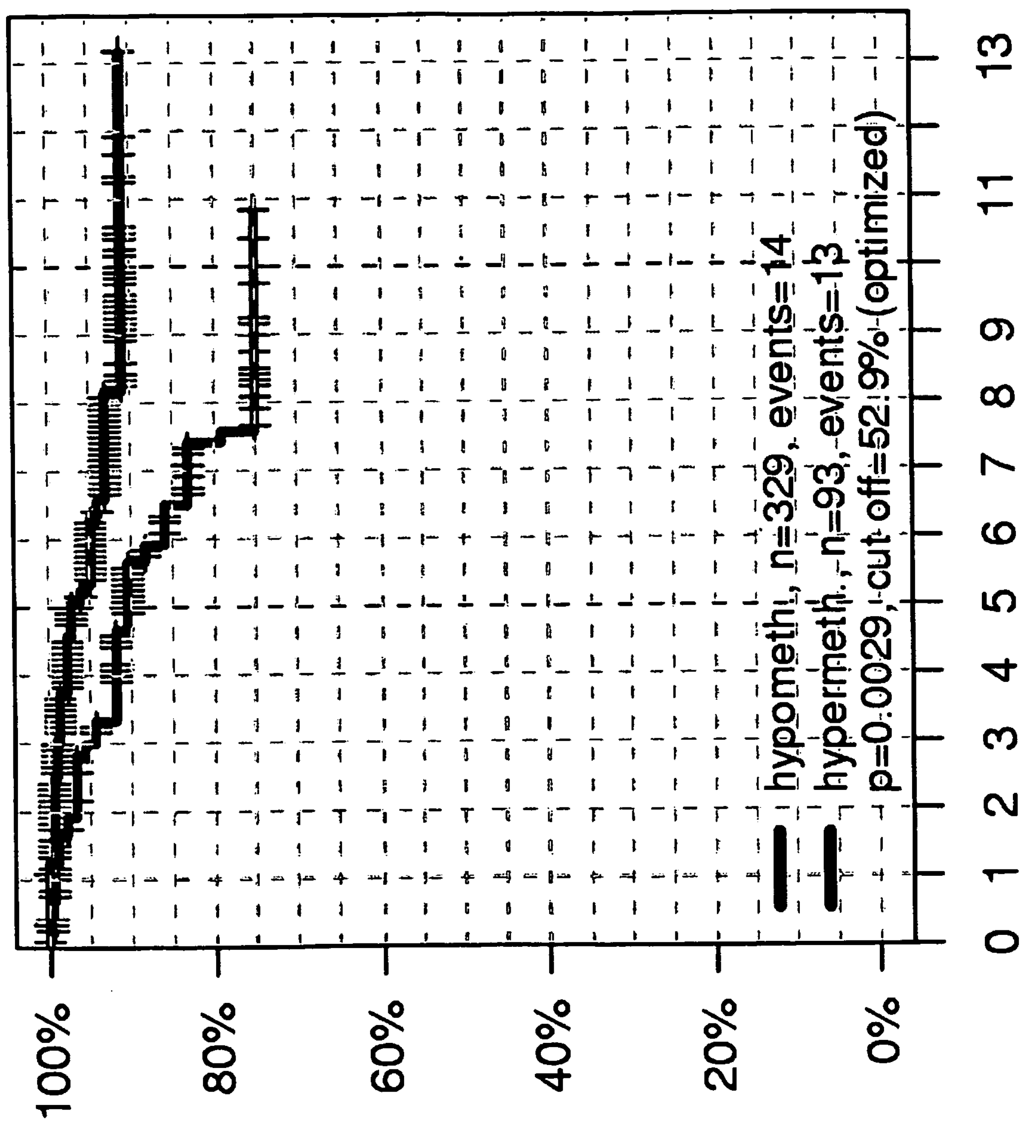
hypometh., n=329, events=14
hypermeth., n=93, events=13
p=0.0029, cut off=52.9% (optimized)
100%
80%
60%
40%
20%
0%
0 1 2 3 4 5 6 7 8 9 11 13

**Figure 17**

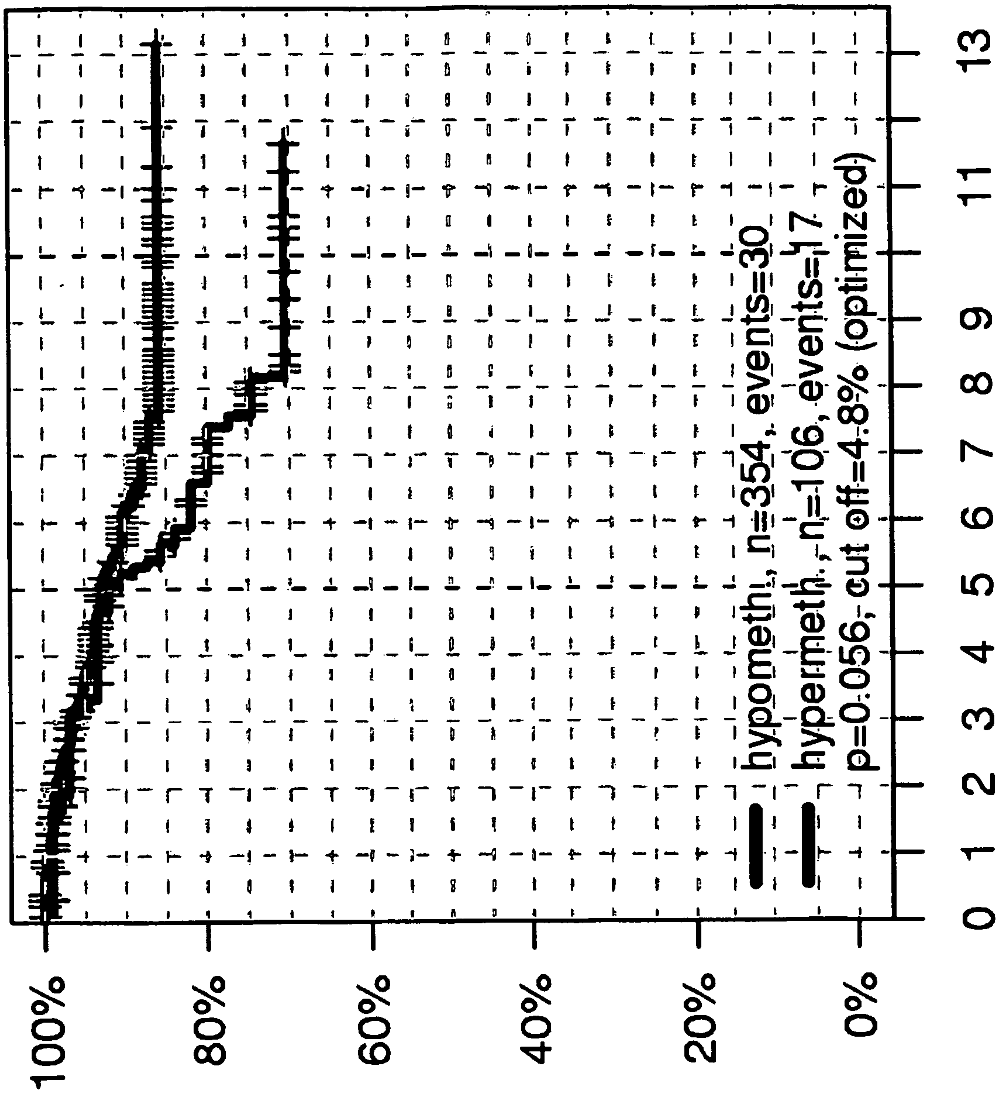
100%
80%
60%
40%
20%
0%
0 1 2 3 4 5 6 7 8 9 11 13
hypometh., n=354, events=30
hypermeth., n=106, events=17
p=0.056, cut off=4.8% (optimized)

**Figure 18**

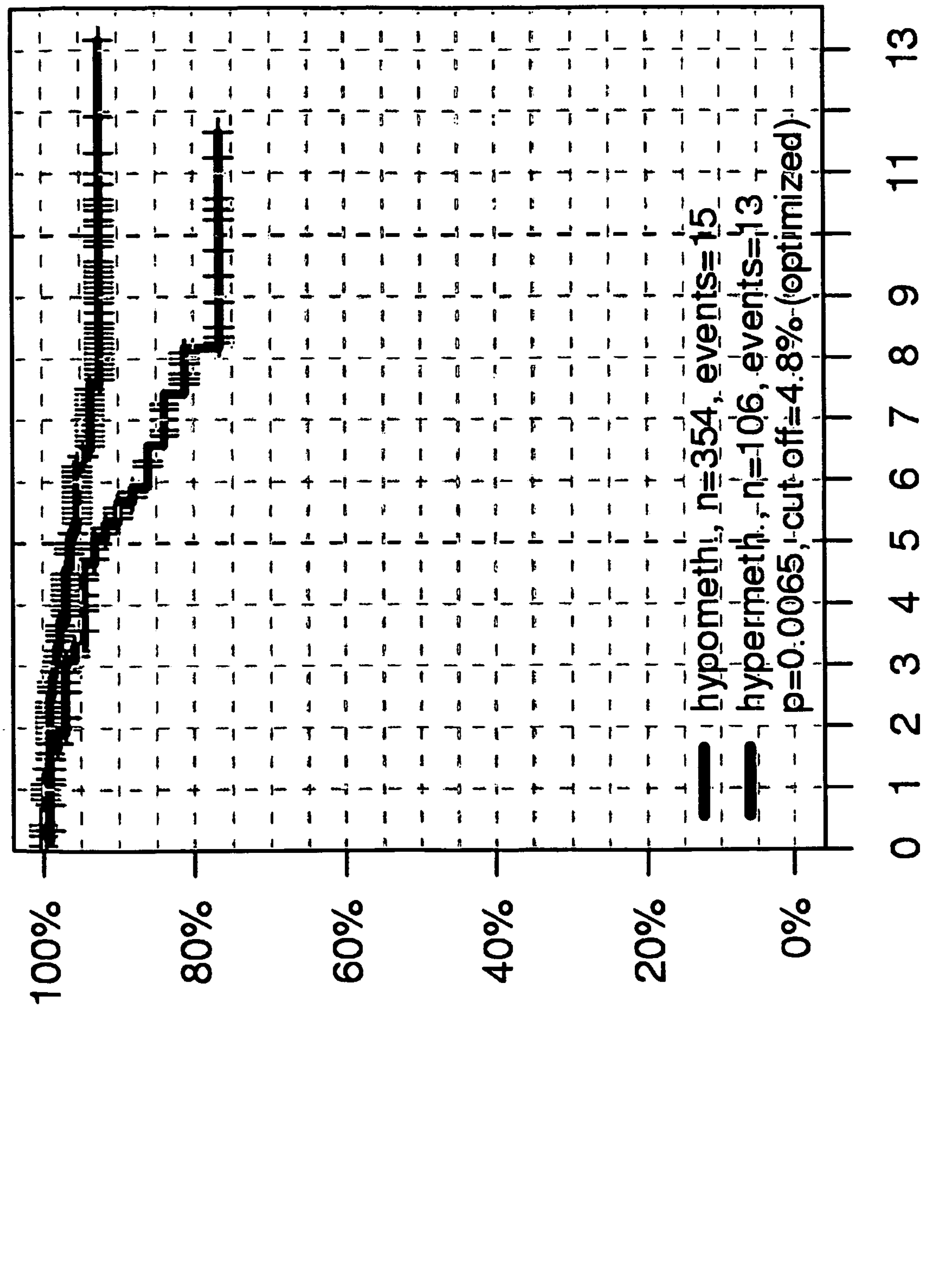
100%
80%
60%
40%
20%
0%
0 1 2 3 4 5 6 7 8 9 11 13
hypometh., n=354, events=15
hypermeth., n=106, events=13
p=0.0065, cut off=4.8% (optimized)

**Figure 19**

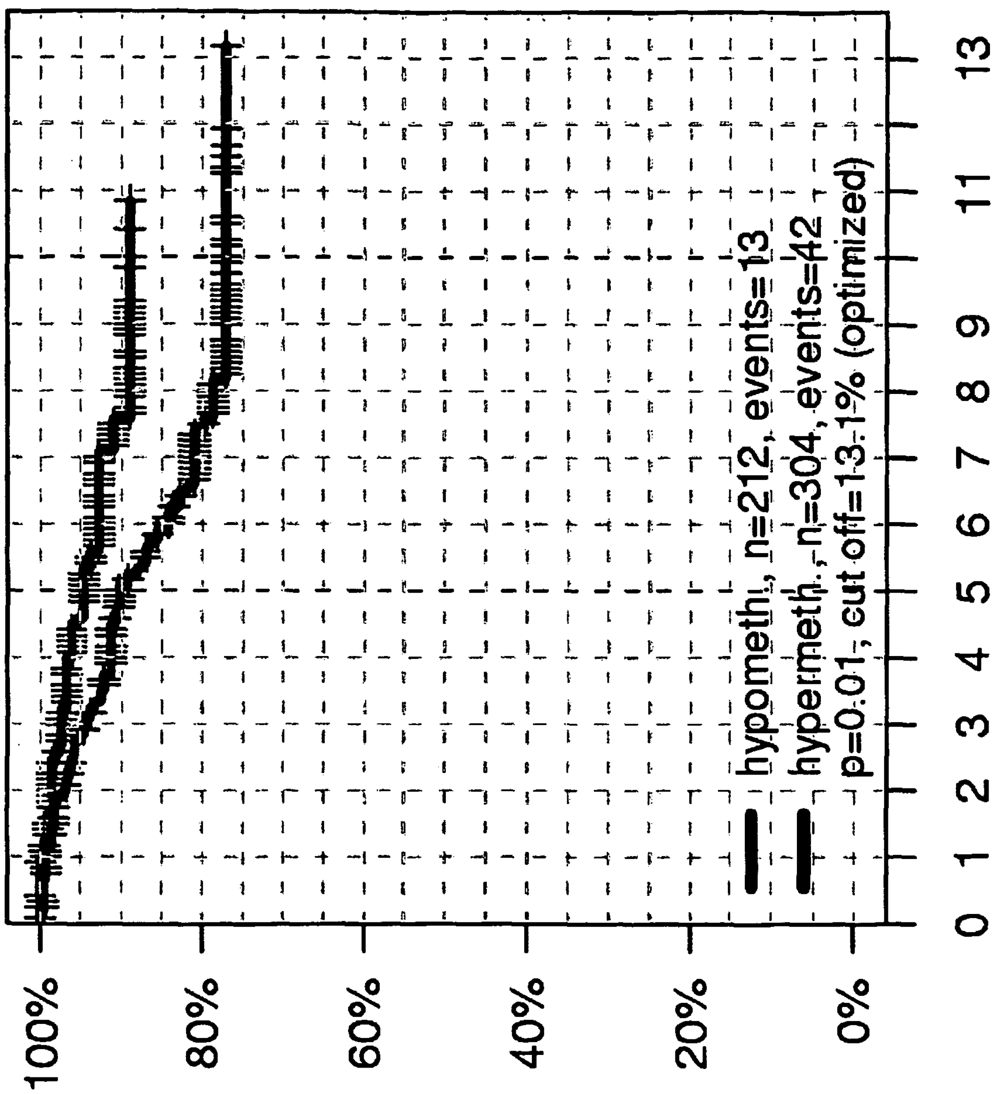

100%
80%
60%
40%
20%
0%
0 1 2 3 4 5 6 7 8 9 11 13
hypometh., n=212, events=13
hypermeth., n=304, events=42
p=0.01, cut off=13.1% (optimized)


**Figure 20**

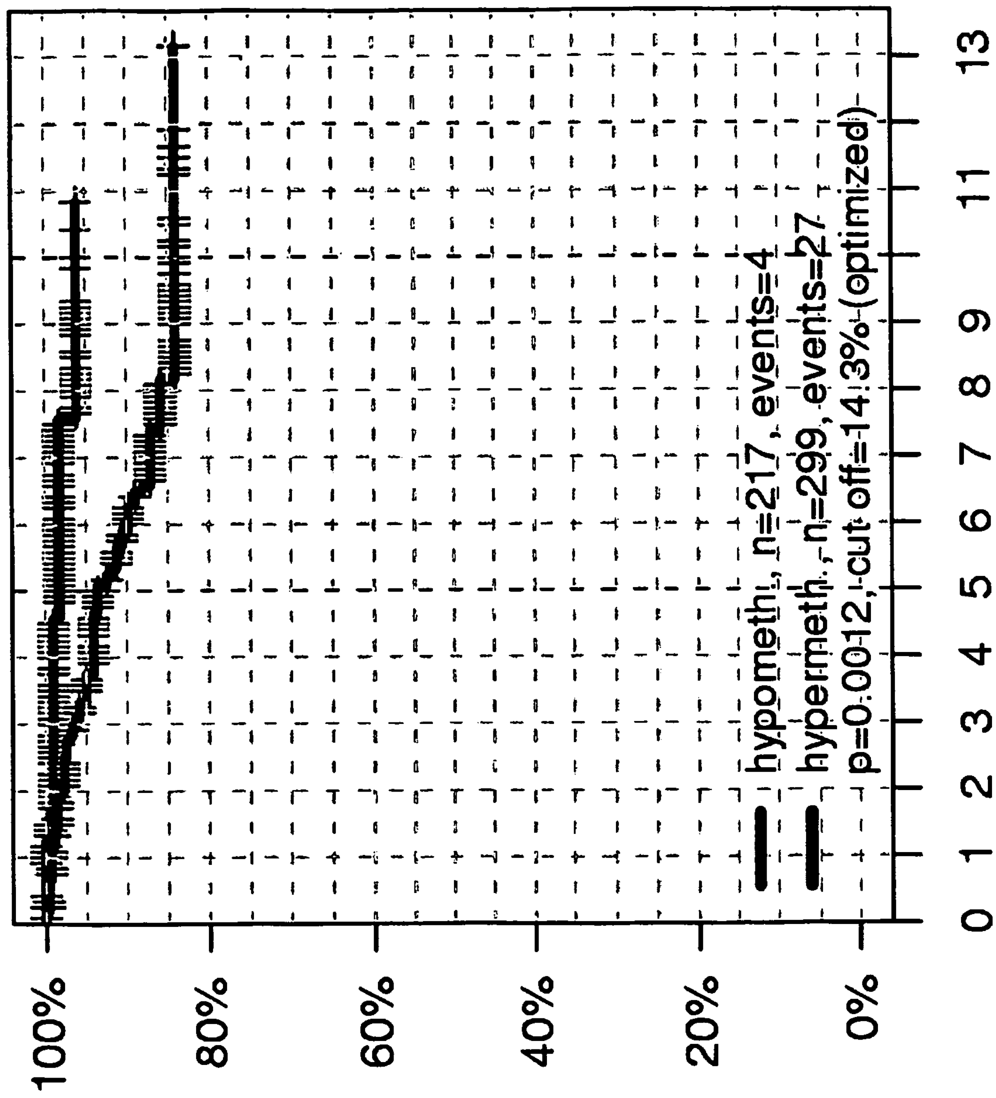

100%
80%
60%
40%
20%
0%
0
1
2
3
4
5
6
7
8
9
11
13
hypometh., n=217, events=4
hypermeth., n=299, events=27
p=0.0012, cut off=14.3% (optimized)


Figure 21

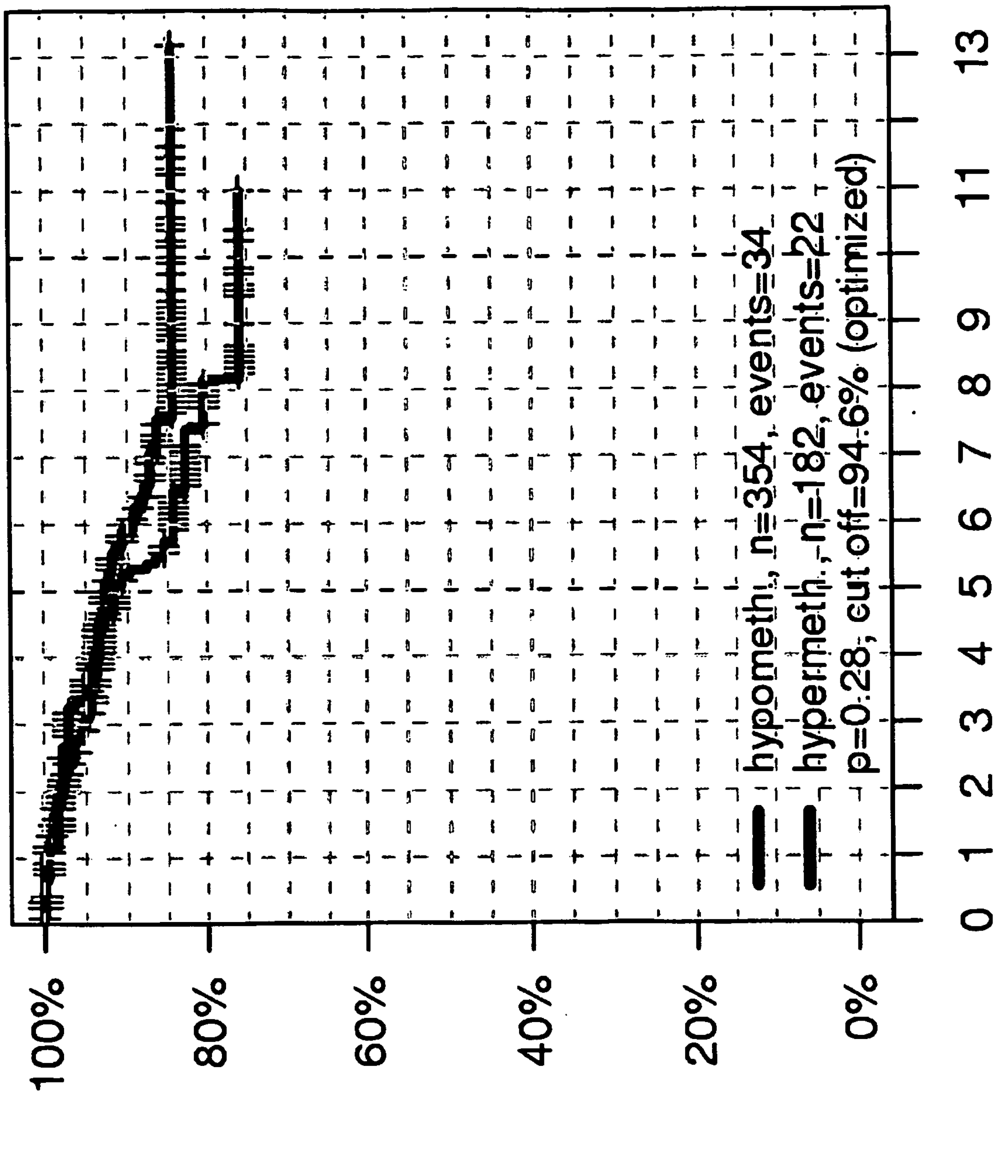
100%
80%
60%
40%
20%
0%
0 1 2 3 4 5 6 7 8 9 11 13
hypometh., n=354, events=34
hypermeth., n=182, events=22
p=0.28, cut off=94.6% (optimized)

**Figure 22**

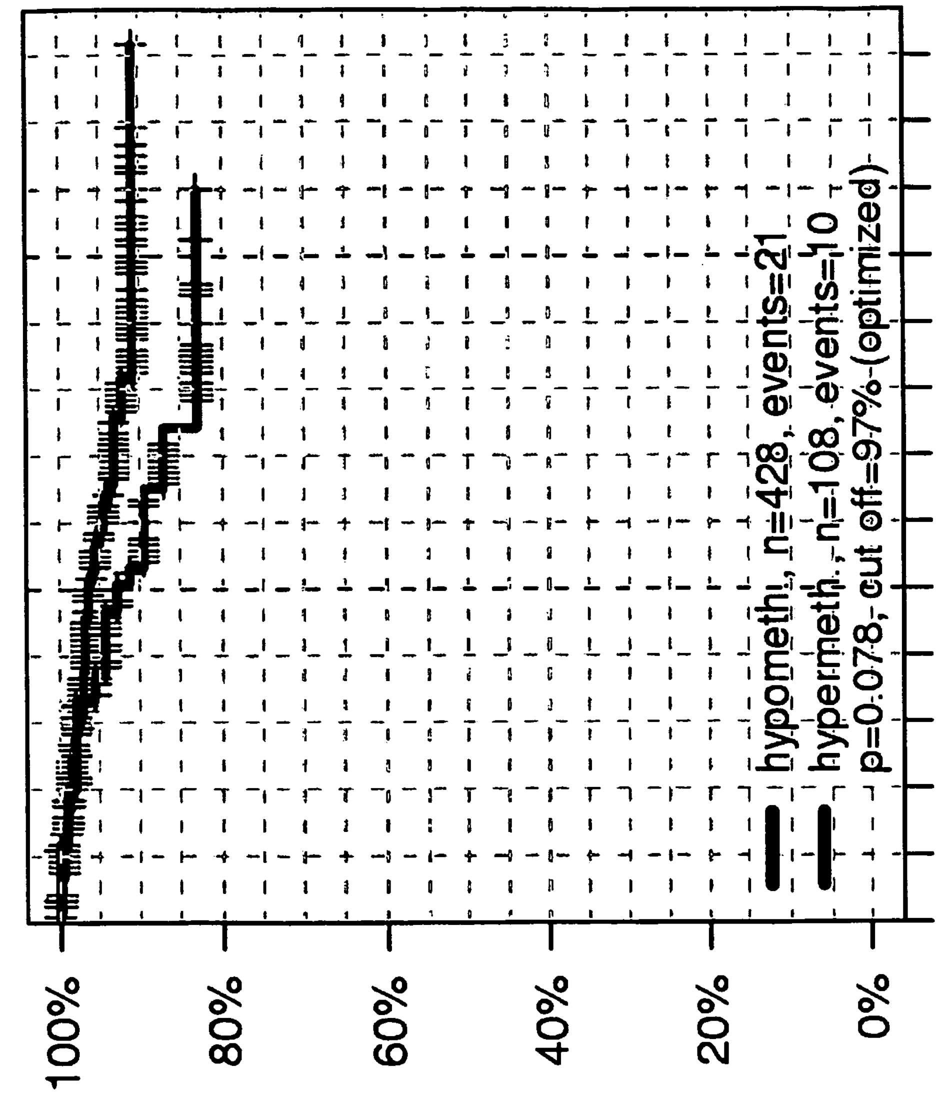
100%
80%
60%
40%
20%
0%
0 1 2 3 4 5 6 7 8 9 11 13
hypometh., n=428, events=21
hypermeth., n=108, events=10
p=0.078, cut off=97% (optimized)

Figure 23

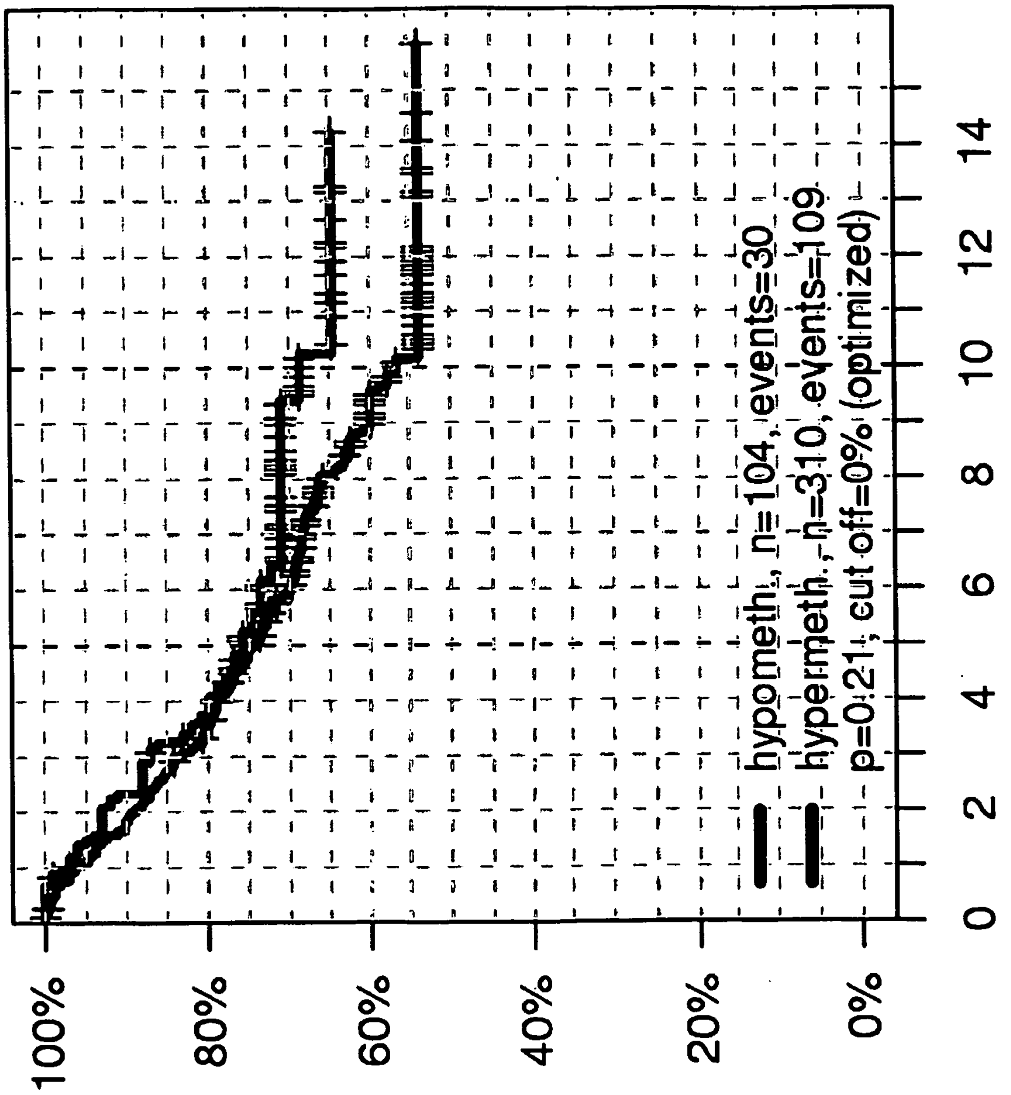
100%
80%
60%
40%
20%
0%
0
2
4
6
8
10
12
14
hypometh., n=104, events=30
hypermeth., n=310, events=109
p=0.21, cut off=0% (optimized)

**Figure 24**

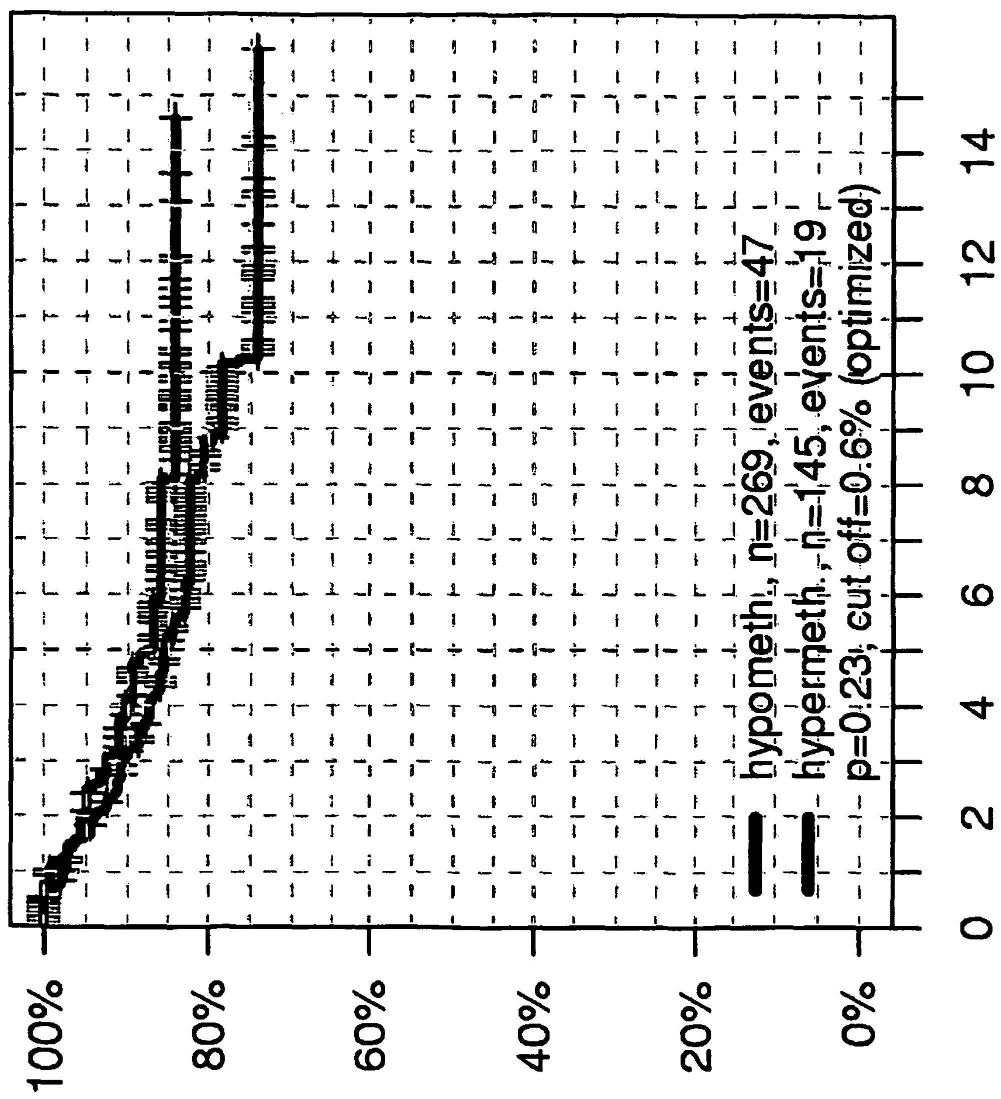

hypometh., n=269, events=47
hypermeth., n=145, events=19
p=0.23, cut off=0.6% (optimized)
100%
80%
60%
40%
20%
0%
0
2
4
6
8
10
12
14


**Figure 25**

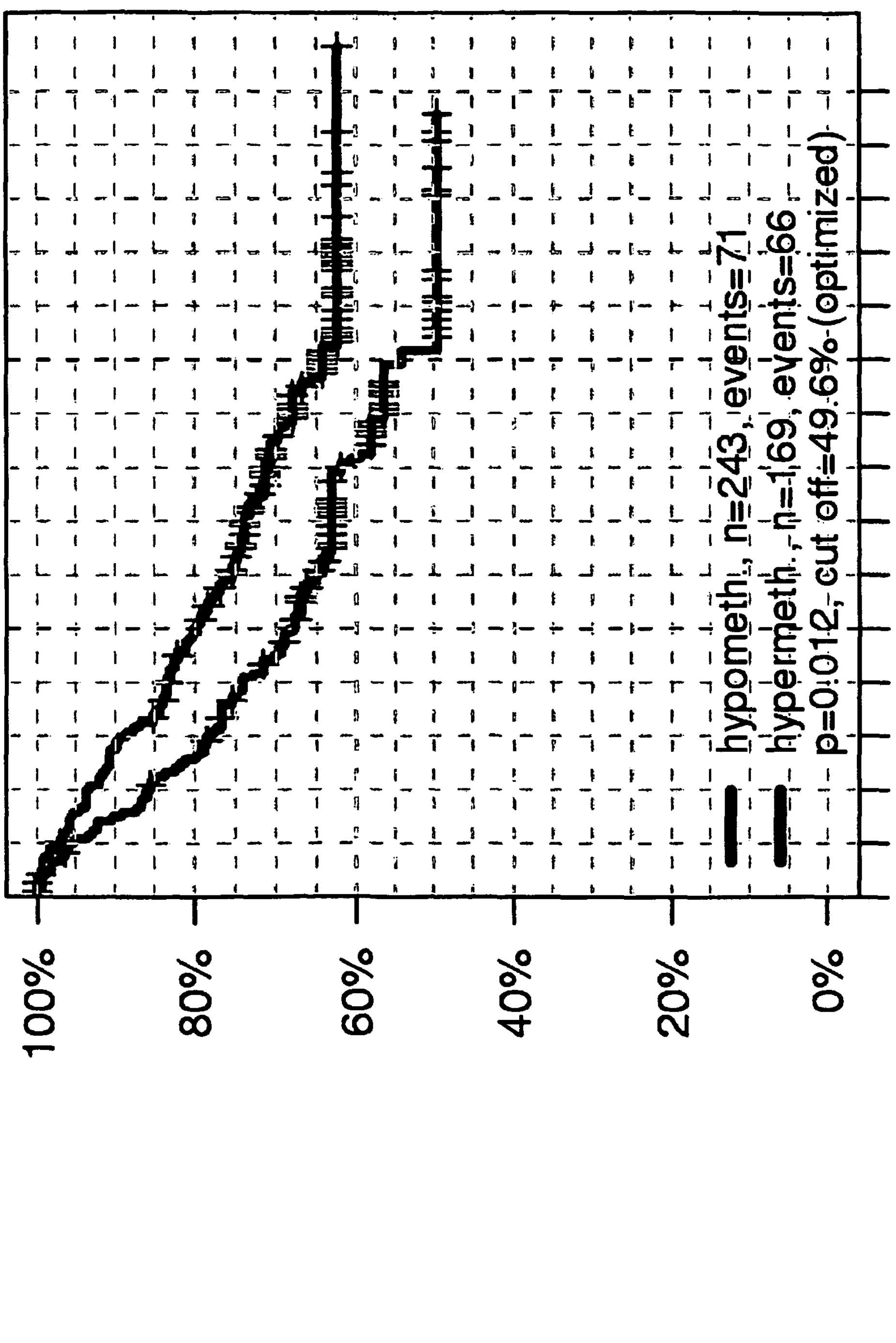
100%
80%
60%
40%
20%
0%
0
2
4
6
8
10
12
14
hypometh., n=243, events=71
hypermeth., n=169, events=66
p=0.012, cut off=49.6% (optimized)

**Figure 26**

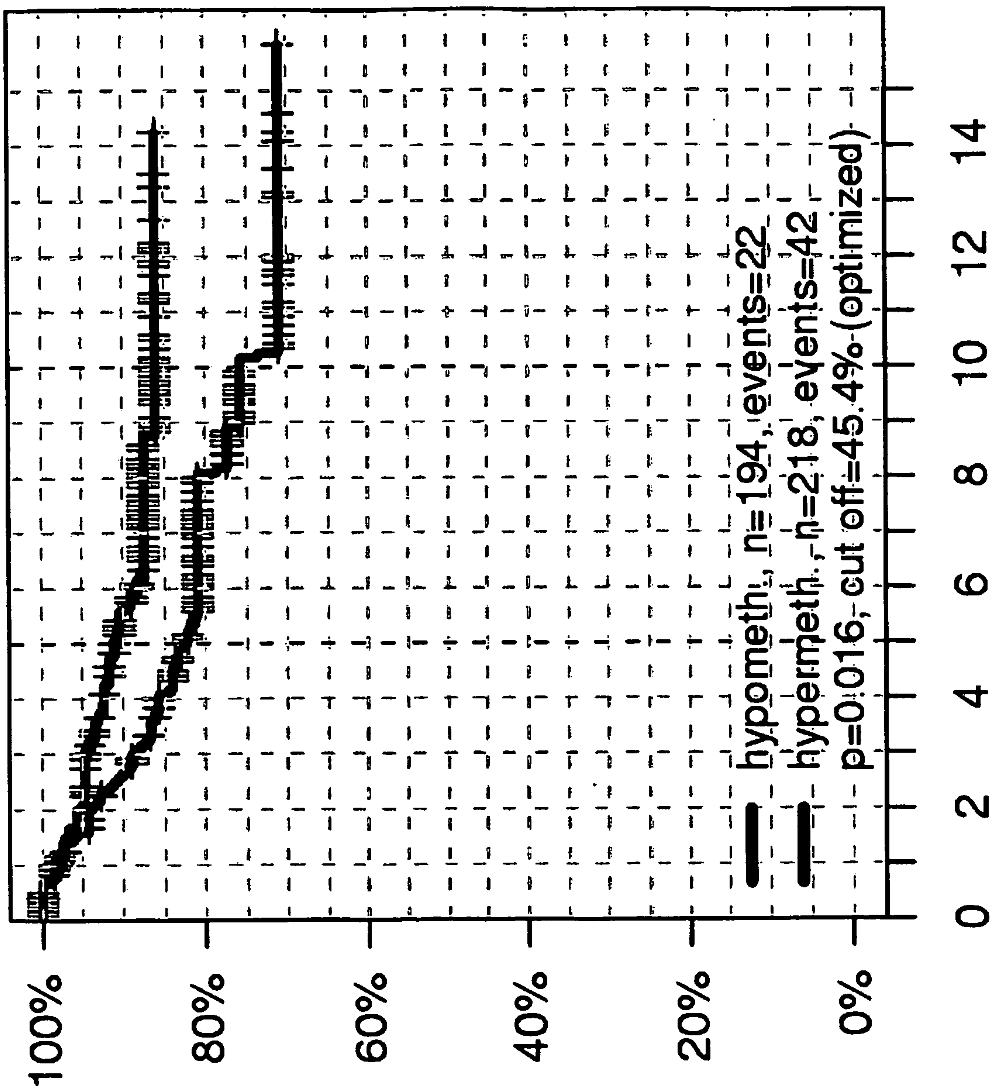

hypometh., n=194, events=22
hypermeth., n=218, events=42
p=0.016, cut off=45.4% (optimized)
100%
80%
60%
40%
20%
0%
0
2
4
6
8
10
12
14


Figure 27

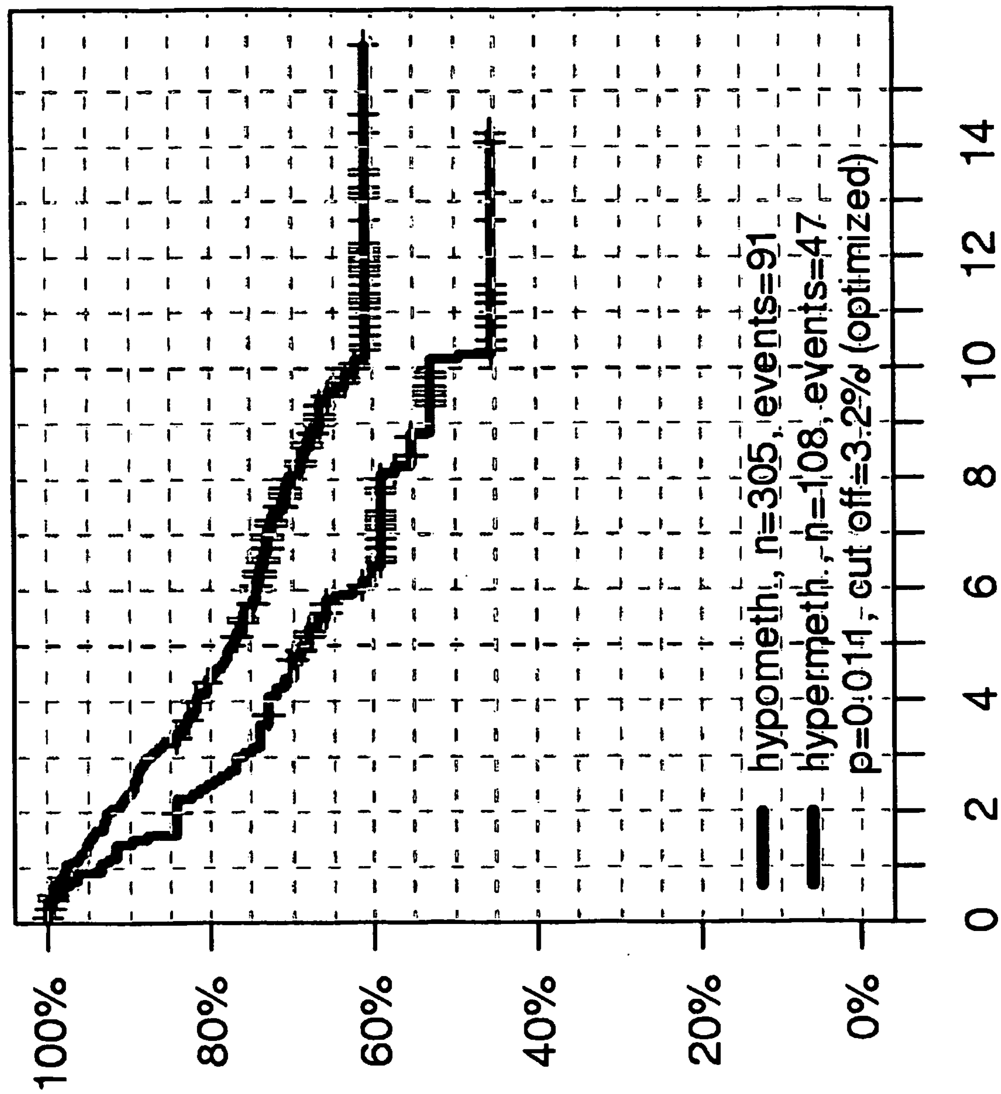
100%
80%
60%
40%
20%
0%
0
2
4
6
8
10
12
14
hypometh., n=305, events=91
hypermeth., n=108, events=47
p=0.011, cut off=3.2% (optimized)

**Figure 28**

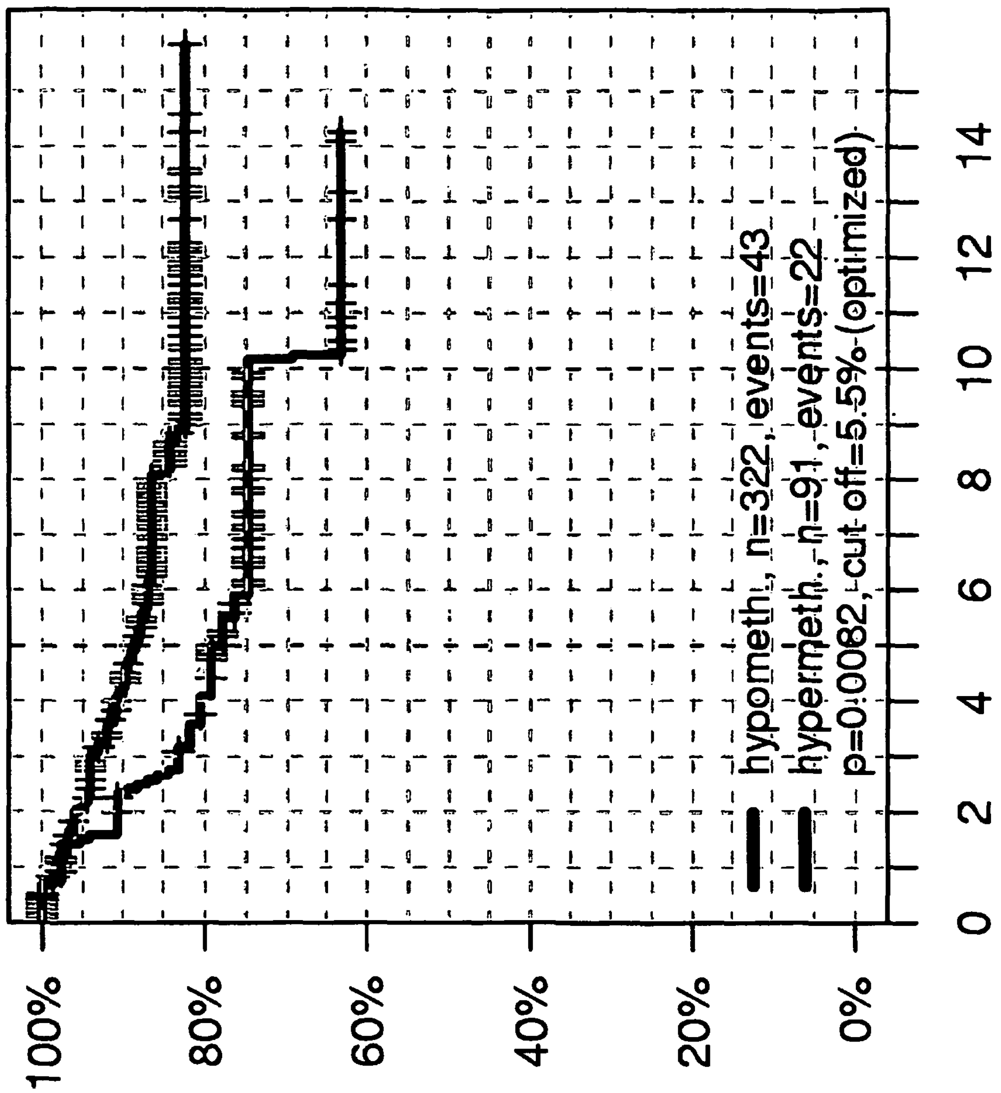
100%
80%
60%
40%
20%
0%
0
2
4
6
8
10
12
14
hypometh., n=322, events=43
hypermeth., n=91, events=22
p=0.0082, cut off=5.5% (optimized)

**Figure 29**

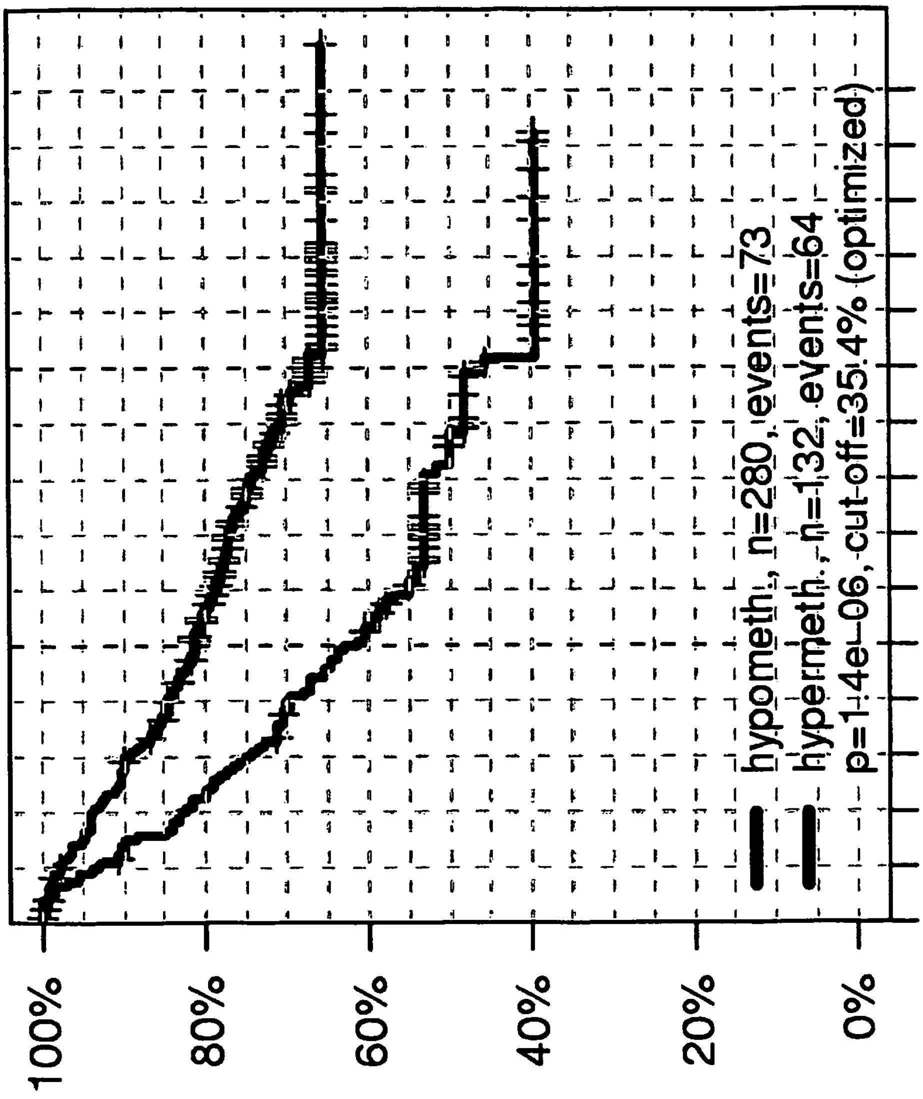
hypometh., n=280, events=73
hypermeth., n=132, events=64
p=1.4e-06, cut-off=35.4% (optimized)
100%
80%
60%
40%
20%
0%
0
2
4
6
8
10
12
14

**Figure 30**

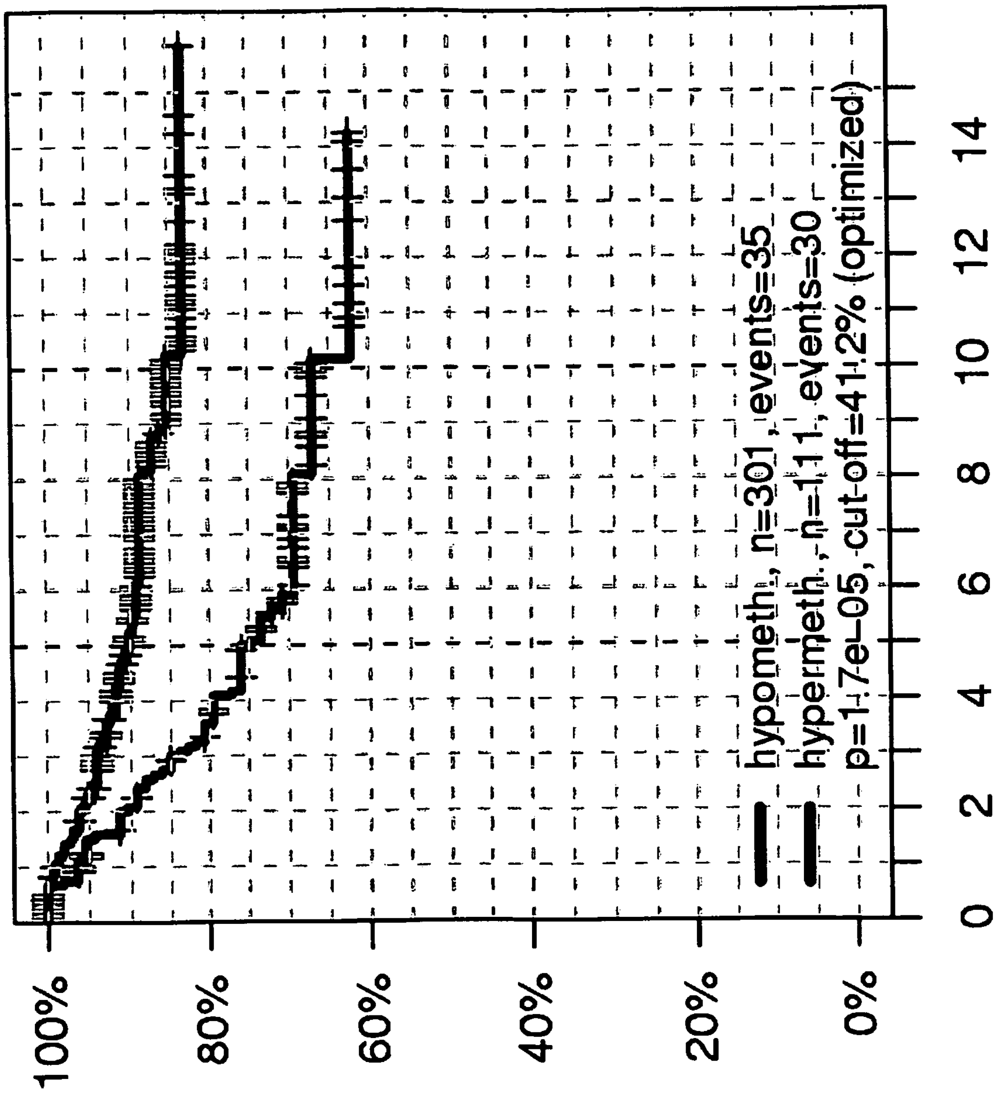
100%
80%
60%
40%
20%
0%
0
2
4
6
8
10
12
14
hypometh., n=301, events=35
hypermeth., n=111, events=30
p=1.7e-05, cut-off=41.2% (optimized)

**Figure 31**

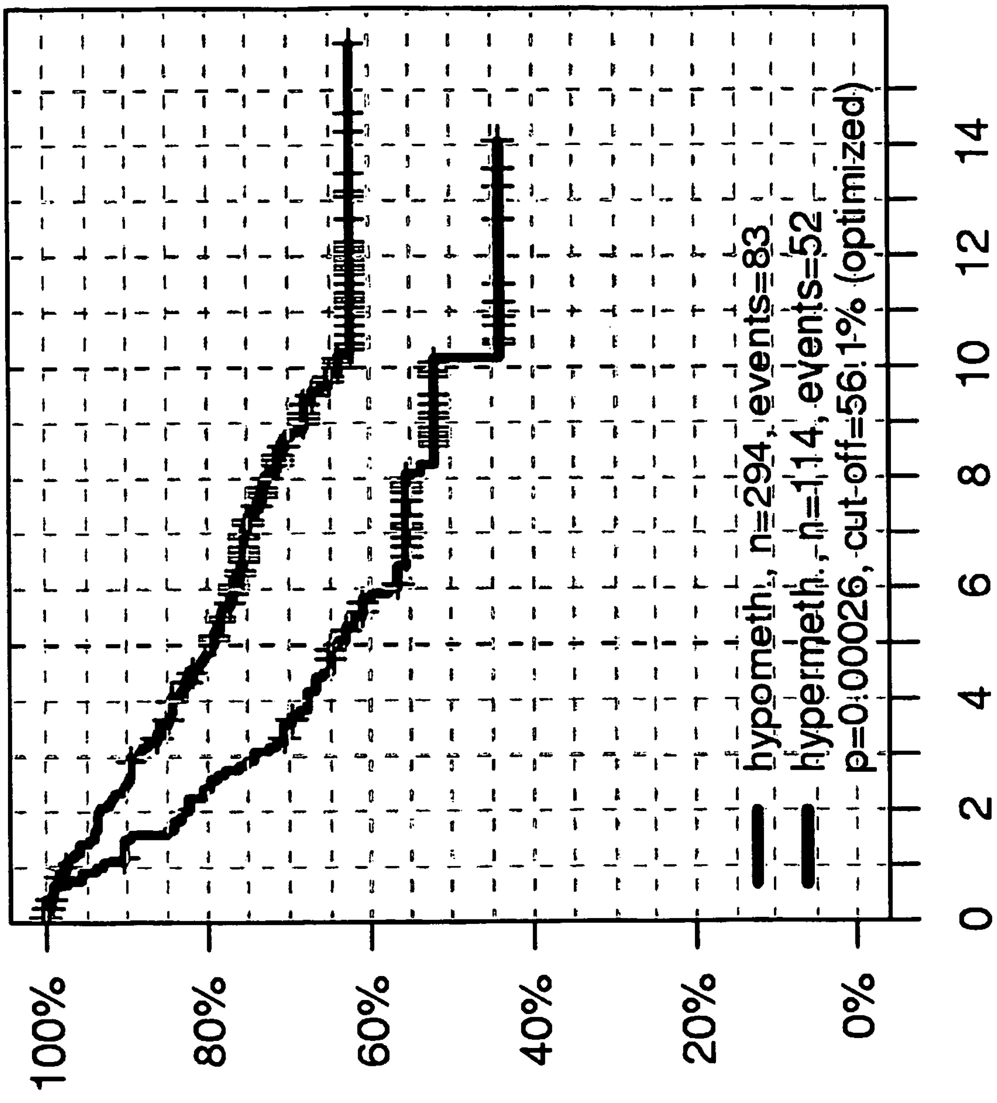
100%
80%
60%
40%
20%
0%
0
2
4
6
8
10
12
14
hypometh., n=294, events=83
hypermeth., n=114, events=52
p=0.00026, cut-off=56.1% (optimized)

**Figure 32**

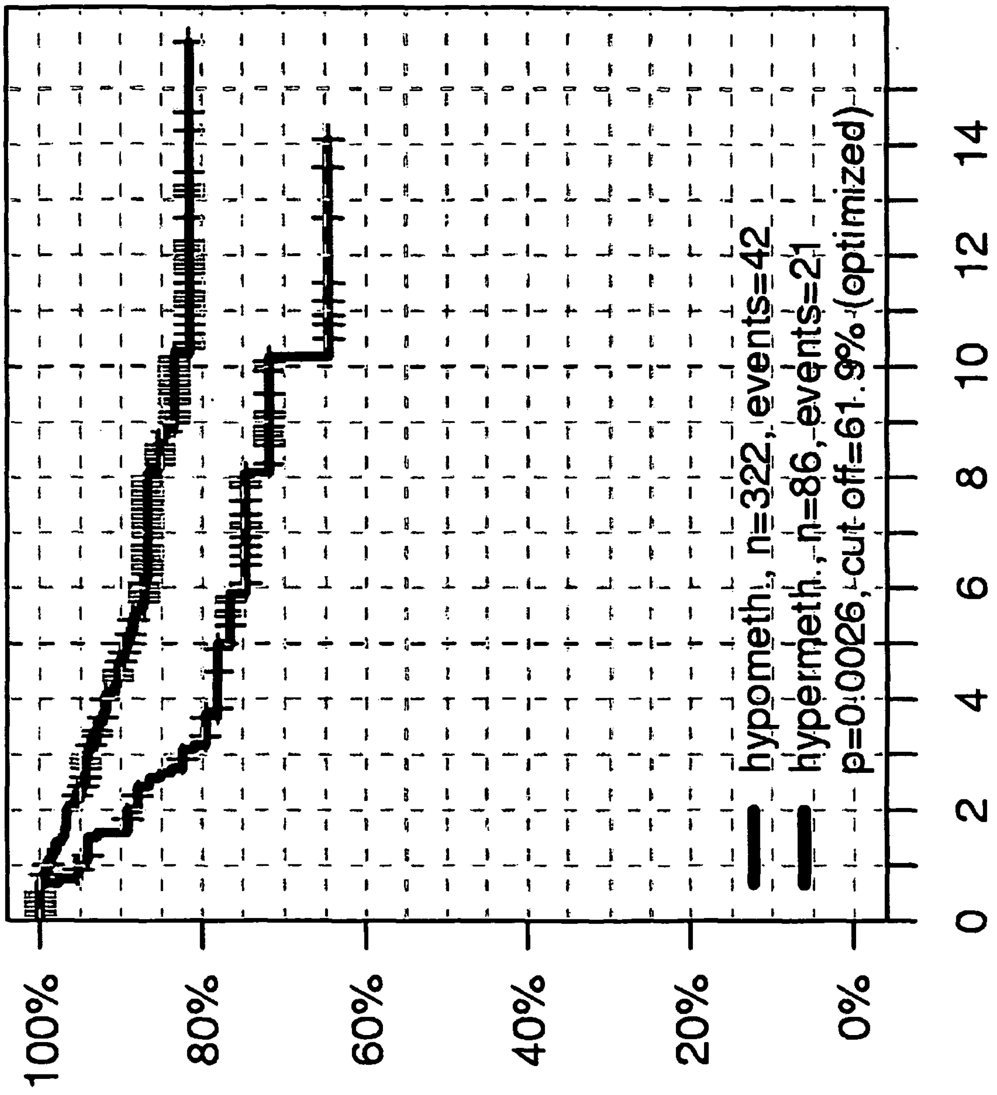
100%
80%
60%
40%
20%
0%
0
2
4
6
8
10
12
14
hypometh., n=322, events=42
hypermeth., n=86, events=21
p=0.0026, cut off=61.9% (optimized)

**Figure 33**

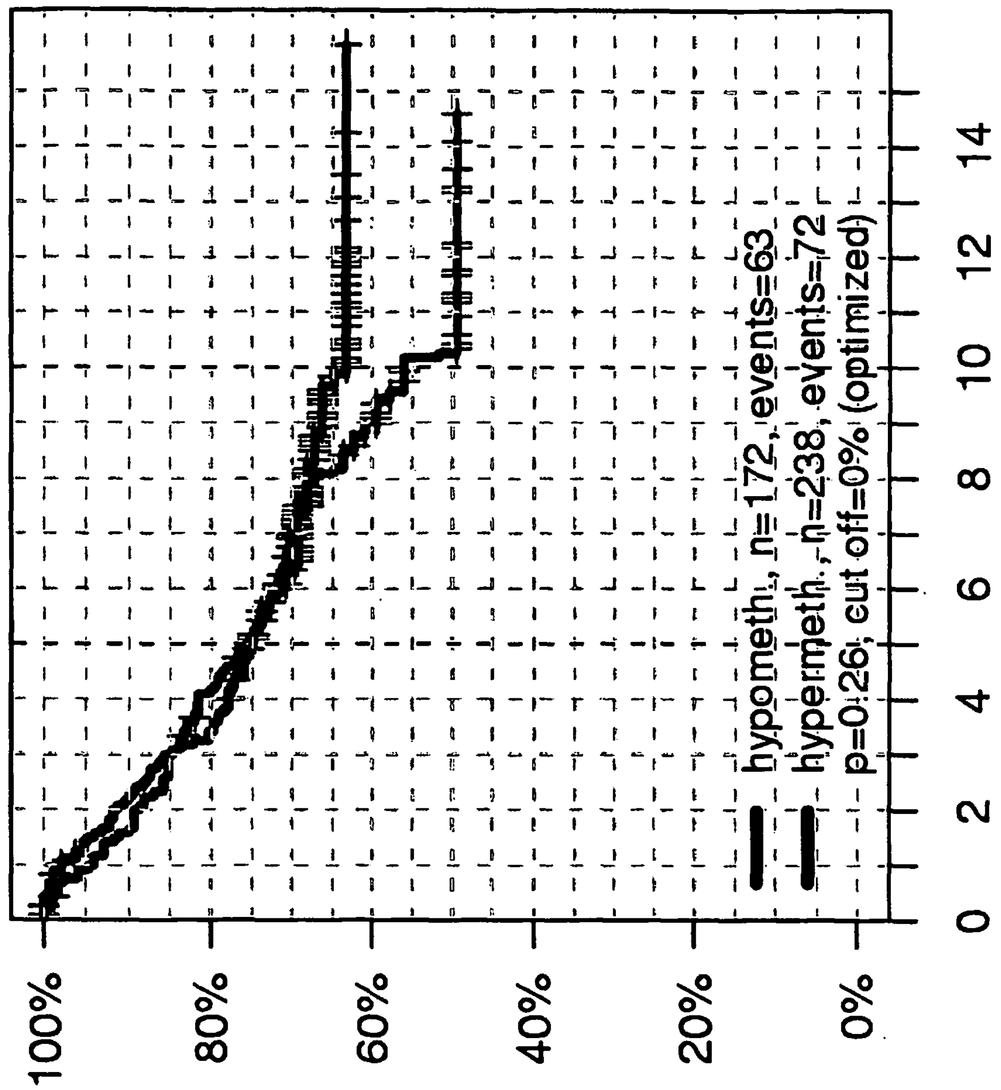
100%
80%
60%
40%
20%
0%
0
2
4
6
8
10
12
14
hypometh., n=172, events=63
hypermeth., n=238, events=72
p=0.26, cut off=0%, (optimized)

**Figure 34**

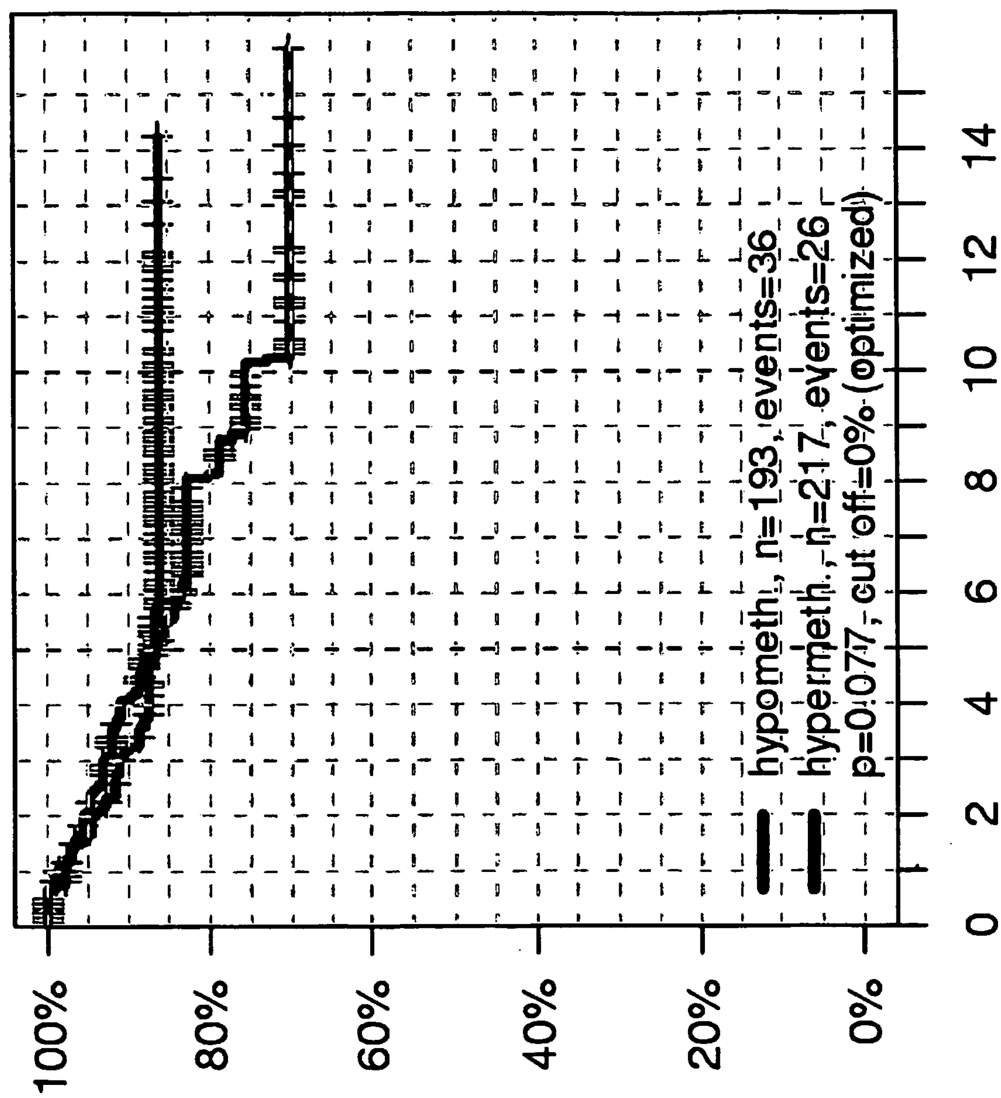
100%
80%
60%
40%
20%
0%
0
2
4
6
8
10
12
14
hypometh., n=193, events=36
hypermeth., n=217, events=26
p=0.077, cut off=0% (optimized)

**Figure 35**

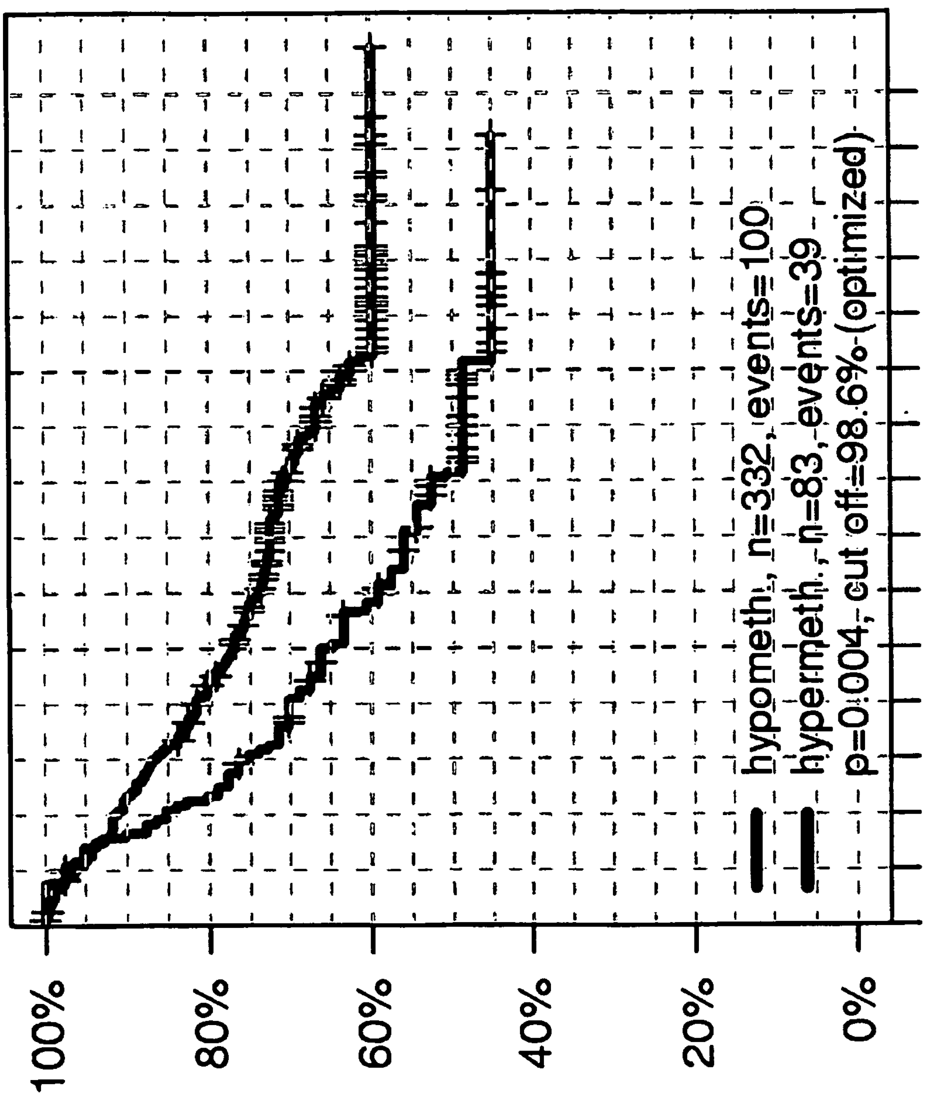
100%
80%
60%
40%
20%
0%
0
2
4
6
8
10
12
14
hypometh., n=332, events=100
hypermeth., n=83, events=39
p=0.004, cut off=98.6% (optimized)

**Figure 36**

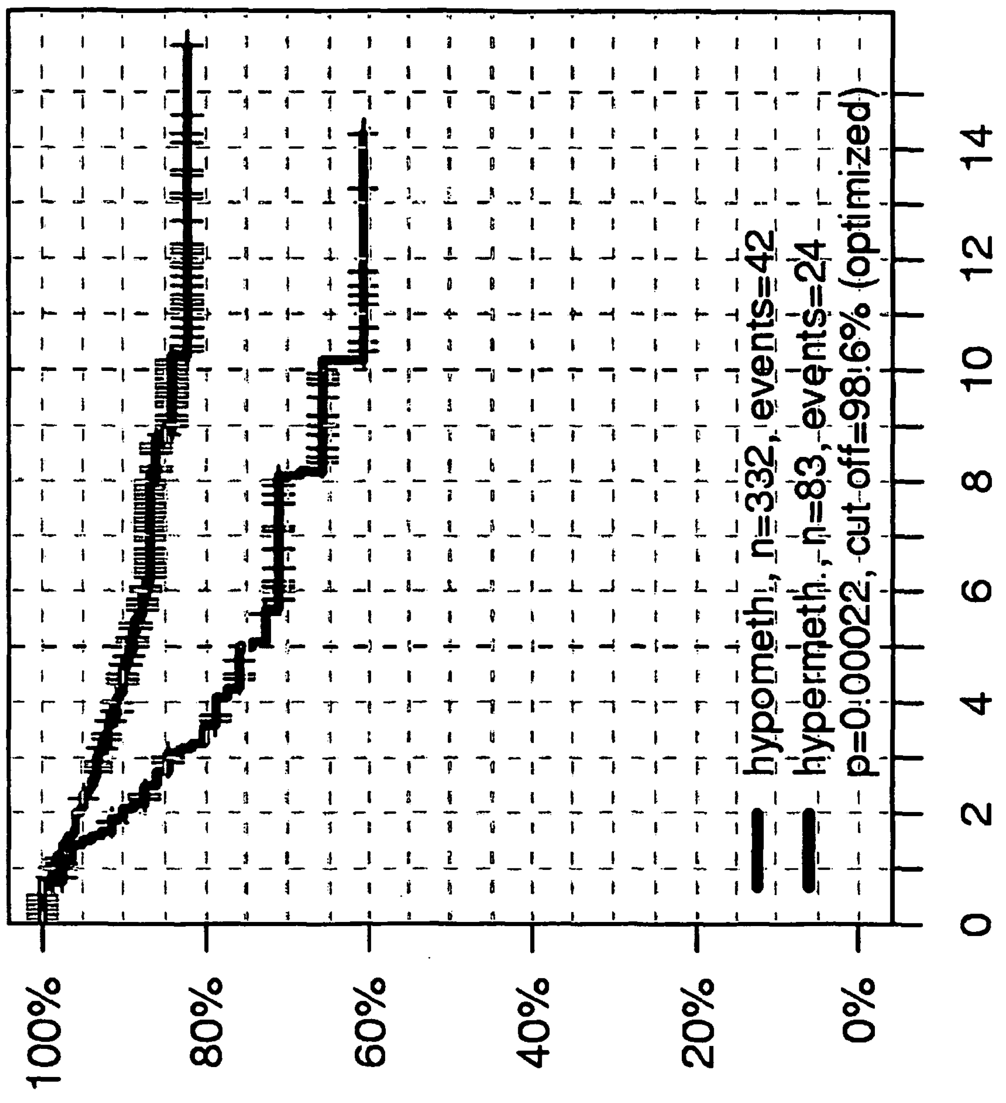
hypometh., n=332, events=42
hypermeth., n=83, events=24
p=0.00022, cut-off=98.6% (optimized)
100%
80%
60%
40%
20%
0%
0
2
4
6
8
10
12
14

**Figure 37**

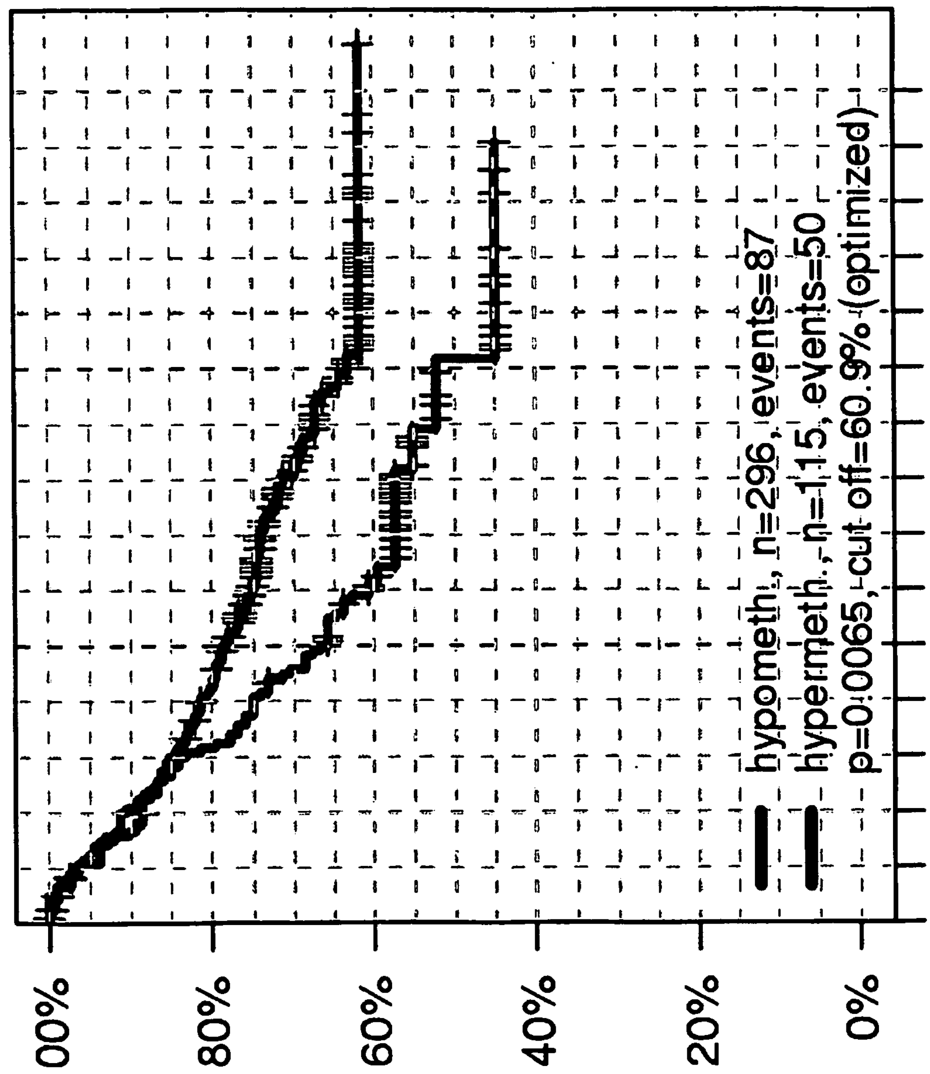
100%
80%
60%
40%
20%
0%
0
2
4
6
8
10
12
14
hypometh., n=296, events=87
hypermeth., n=115, events=50
p=0.0065, cut off=60.9% (optimized)

**Figure 38**

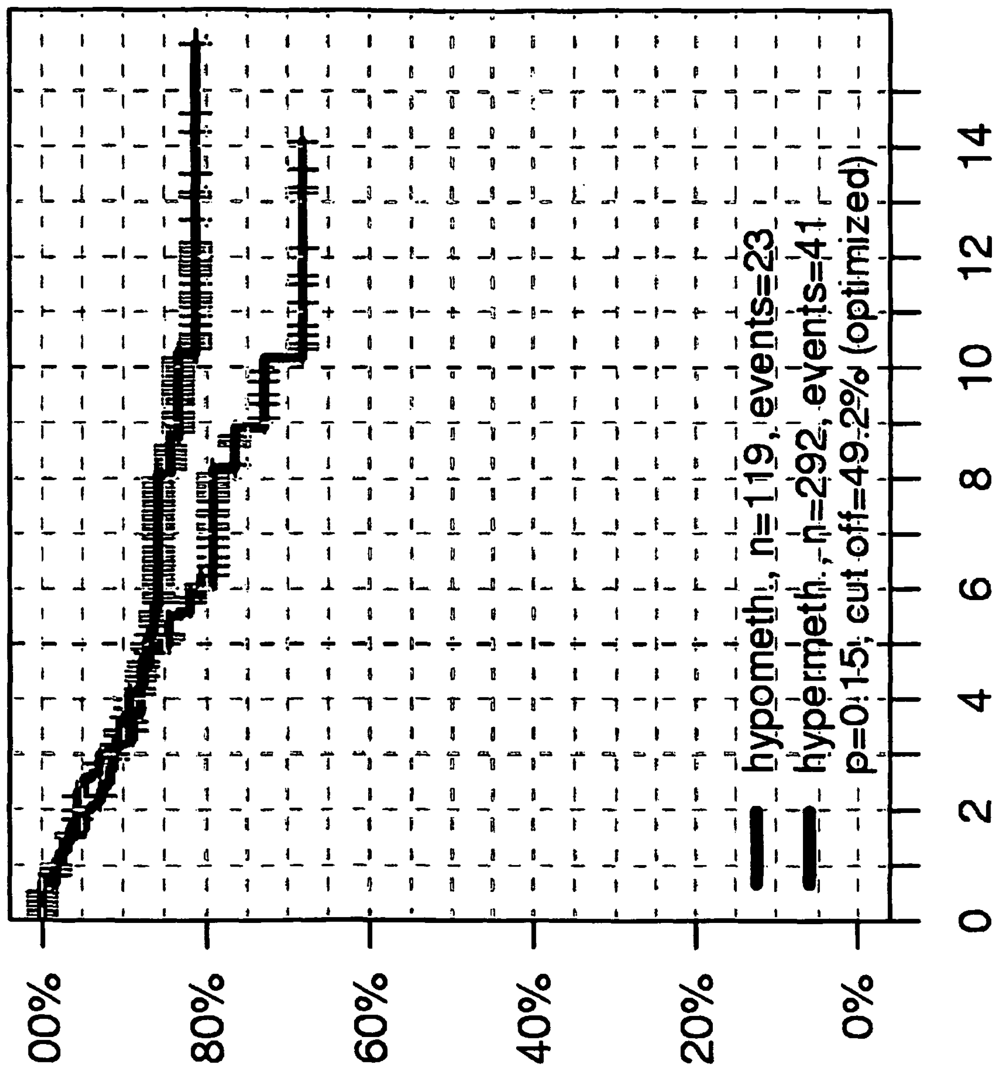
100%
80%
60%
40%
20%
0%
0
2
4
6
8
10
12
14
hypometh., n=119, events=23
hypermeth., n=292, events=41
p=0.15, cut off=49.2% (optimized)

**Figure 39**

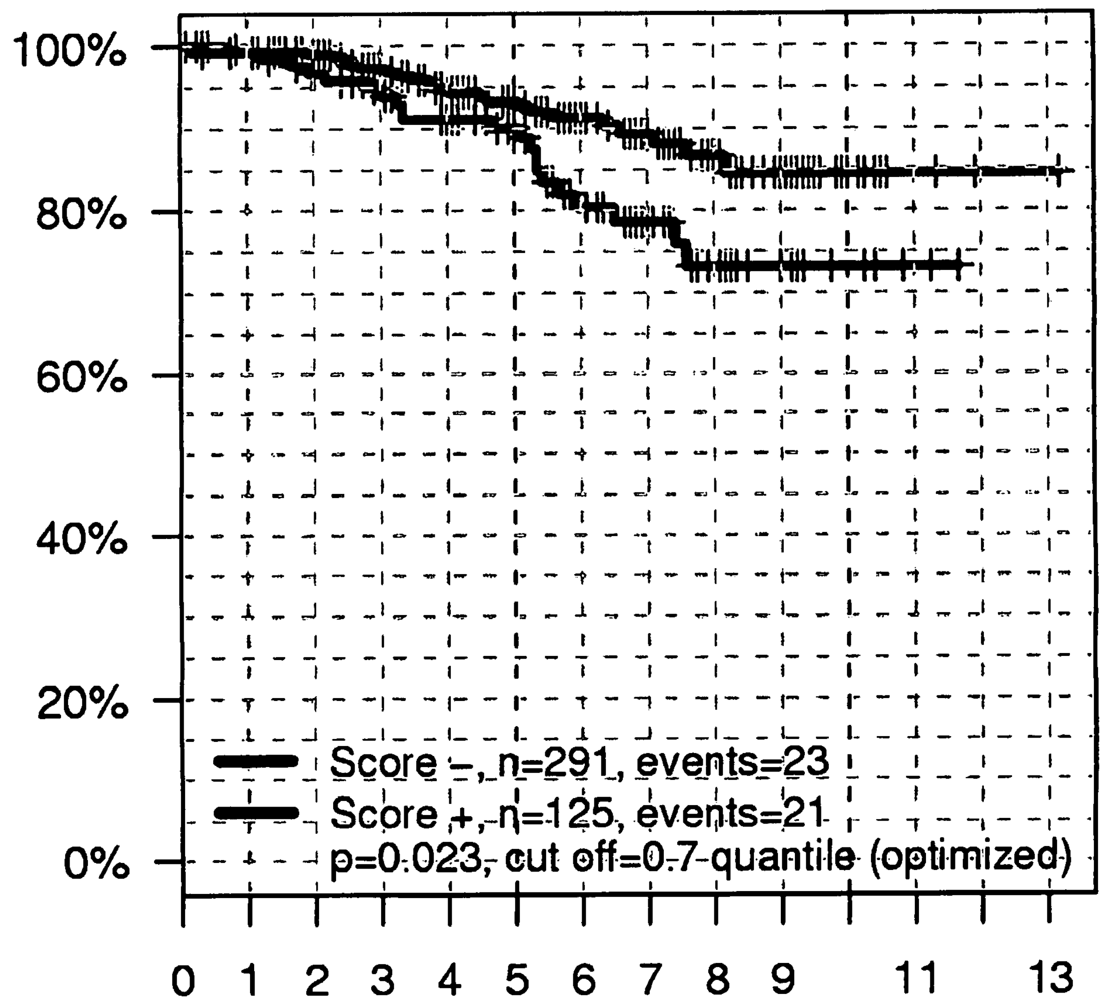
100%
80%
60%
40%
20%
0%
0 1 2 3 4 5 6 7 8 9 11 13
Score -, n=291, events=23
Score +, n=125, events=21
p=0.023, cut off=0.7-quantile (optimized)

**Figure 40**

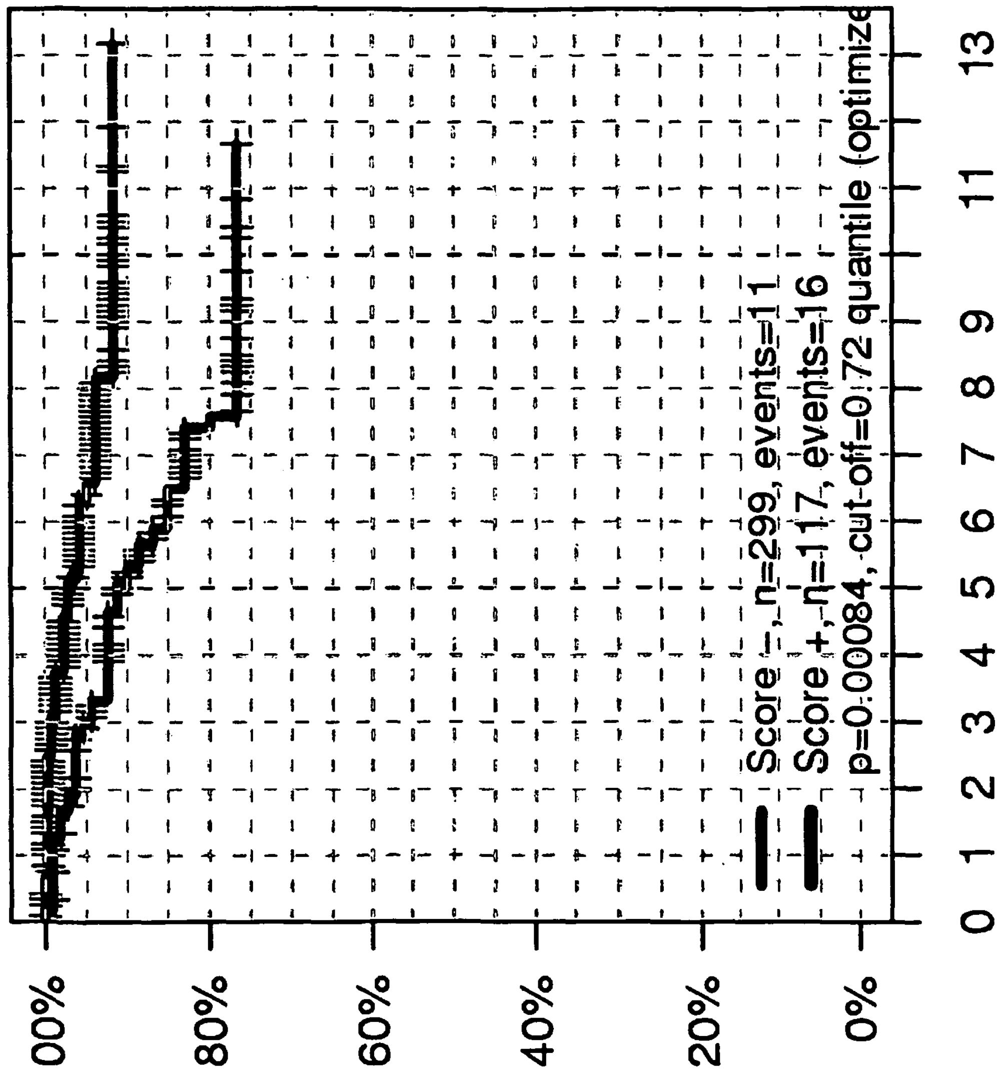
Score -, n=299, events=11
Score +, n=117, events=16
p=0.00084, cut-off=0.72 quantile (optimize
100%
80%
60%
40%
20%
0%
0 1 2 3 4 5 6 7 8 9 11 13

**Figure 41**

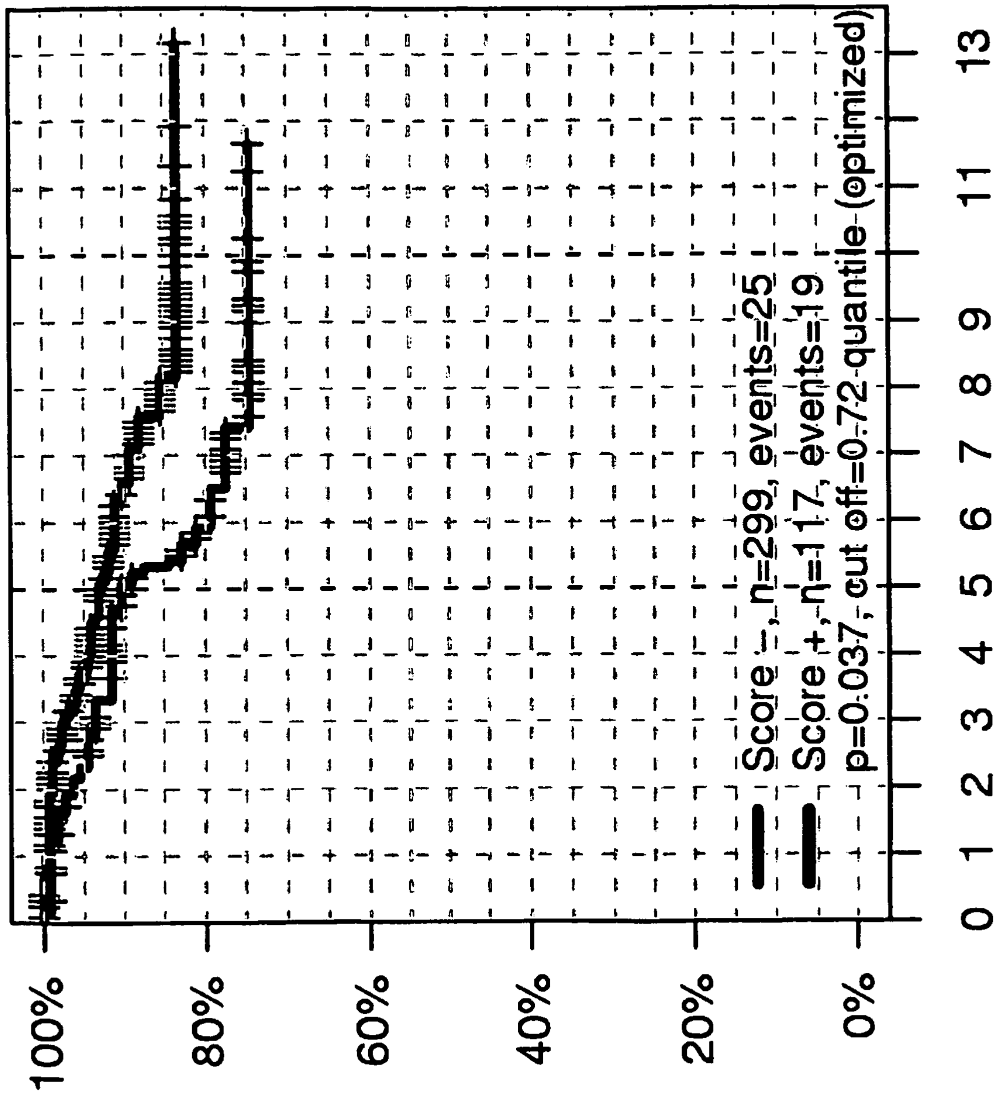
100%
80%
60%
40%
20%
0%
0
1
2
3
4
5
6
7
8
9
11
13
Score –, n=299, events=25
Score +, n=117, events=19
p=0.037, cut off=0.72-quantile (optimized)

**Figure 42**

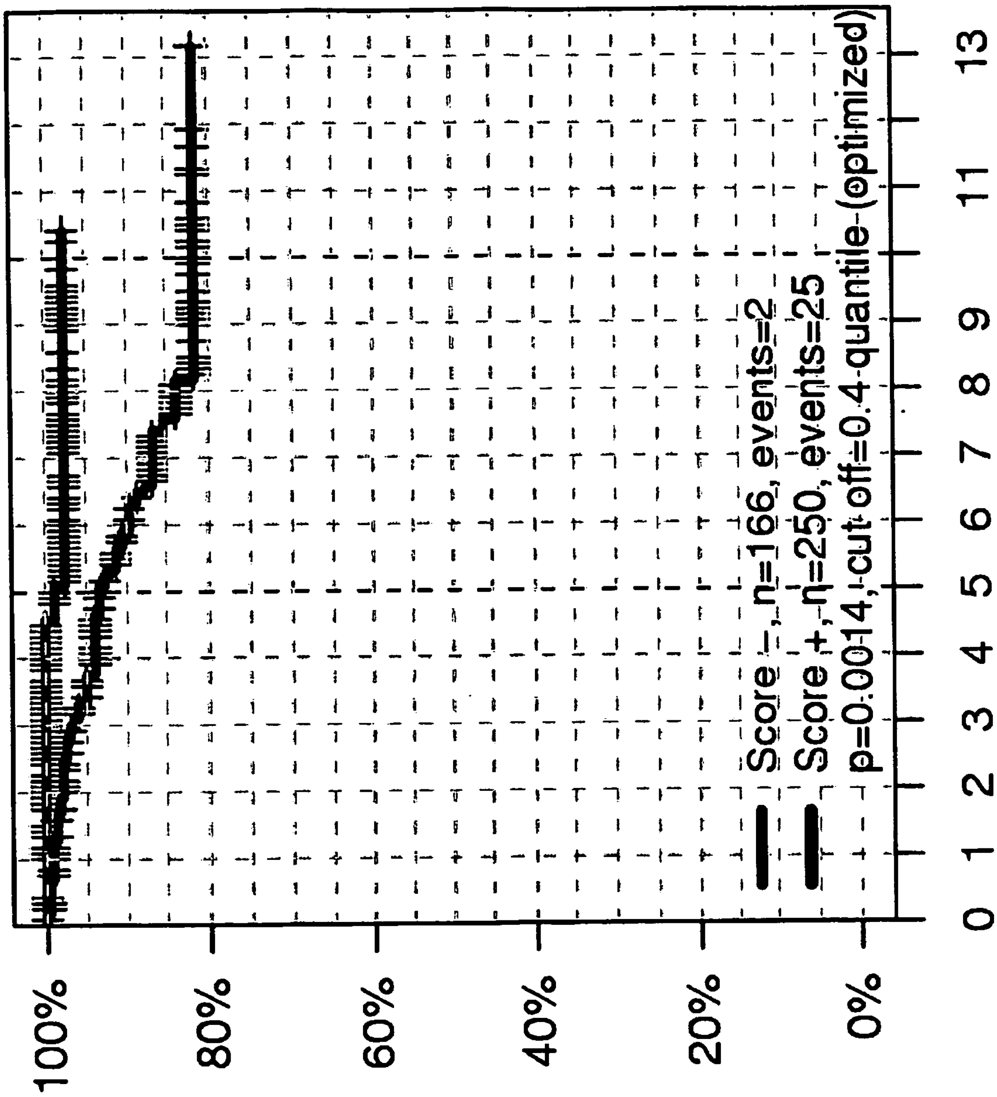
100%
80%
60%
40%
20%
0%
0
1
2
3
4
5
6
7
8
9
11
13
Score -, n=166, events=2
Score +, n=250, events=25
p=0.0014, cut off=0.4 quantile (optimized)

**Figure 43**

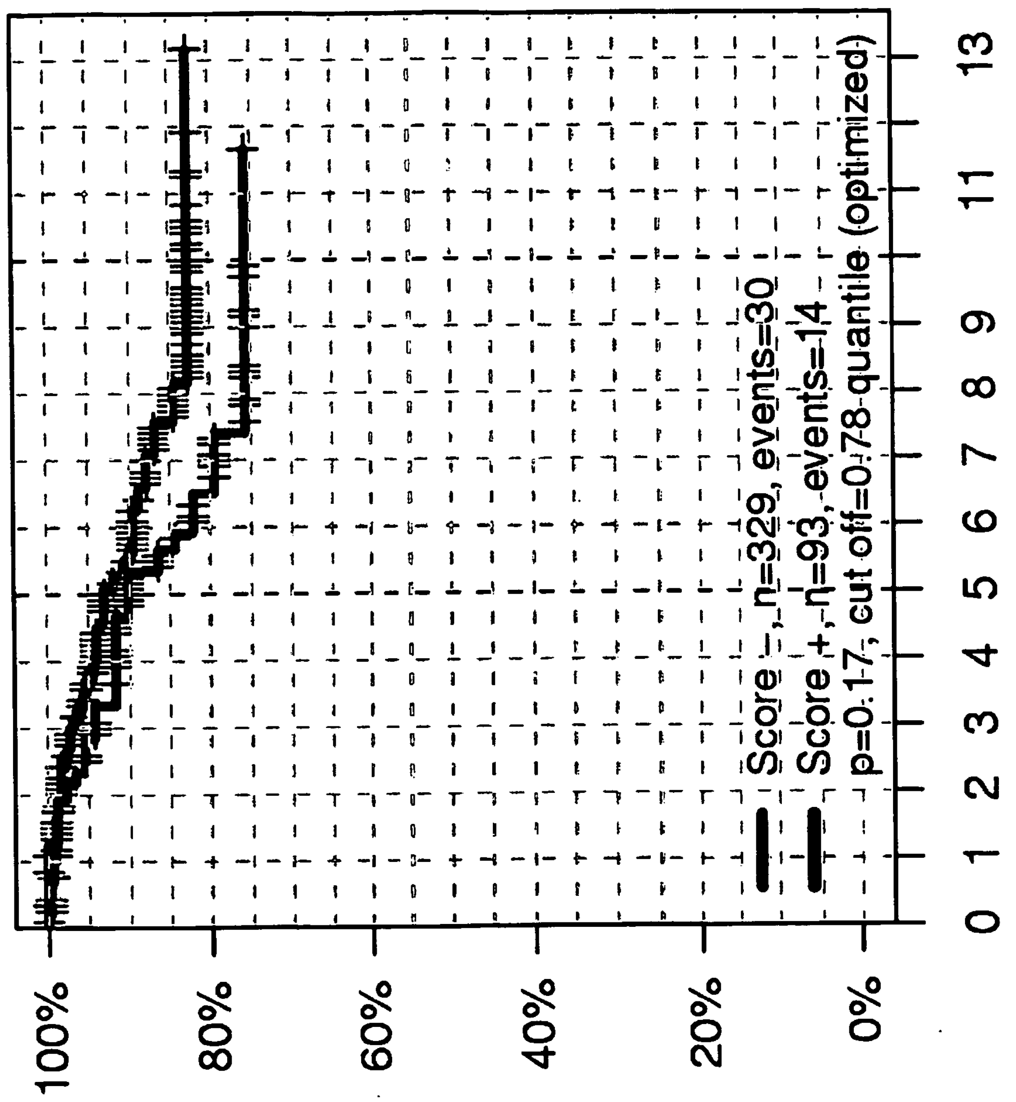
Score –, n=329, events=30
Score +, n=93, events=14
p=0.17, cut off=0.78-quantile (optimized)
100%
80%
60%
40%
20%
0%
0 1 2 3 4 5 6 7 8 9 11 13

**Figure 44**

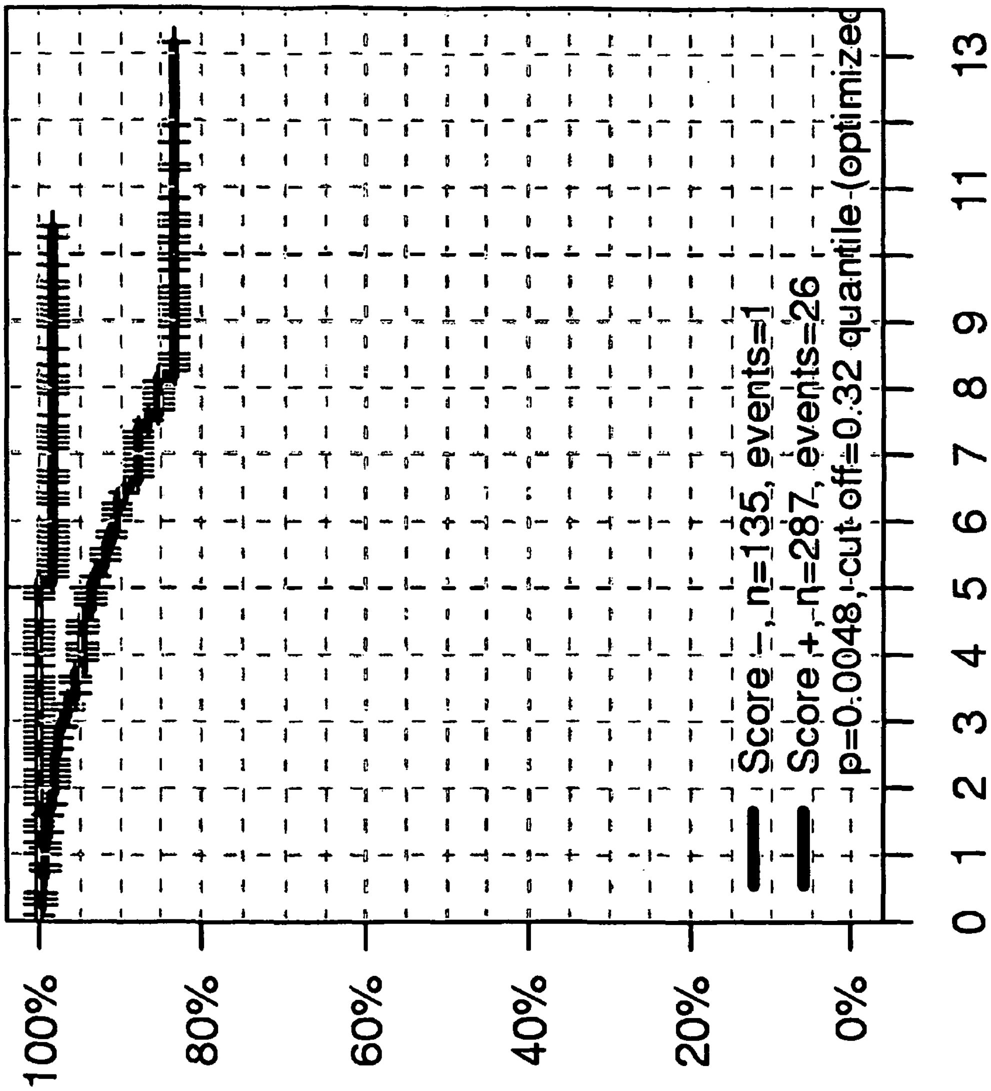
100%
80%
60%
40%
20%
0%
0 1 2 3 4 5 6 7 8 9 11 13
Score -, n=135, events=1
Score +, n=287, events=26
p=0.0048, cut off=0.32 quantile (optimized

**Figure 45**

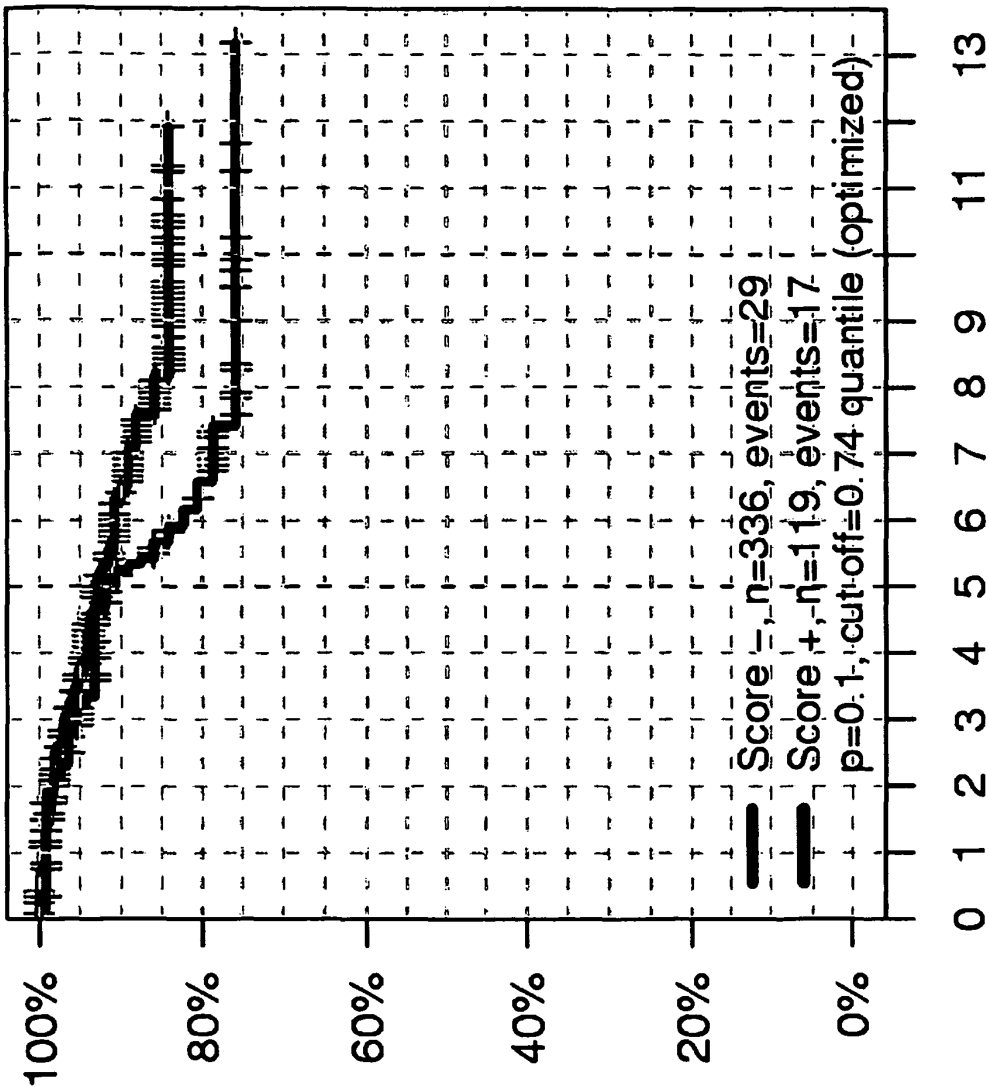

100%
80%
60%
40%
20%
0%
0 1 2 3 4 5 6 7 8 9 11 13
Score -, n=336, events=29
Score +, n=119, events=17
p=0.1, cut-off=0.74 quantile (optimized)


**Figure 46**

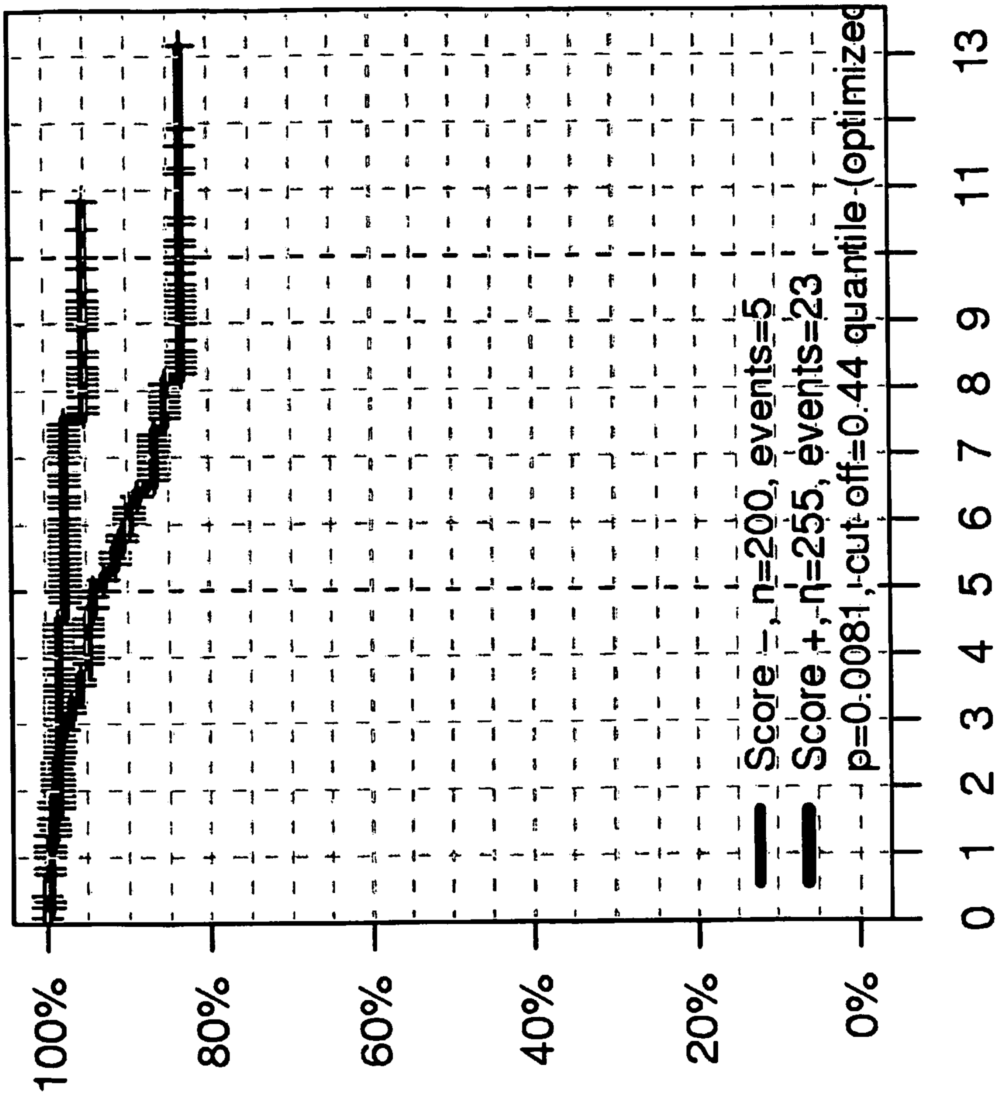
Score –, n=200, events=5
Score +, n=255, events=23
p=0.0081, cut off=0.44 quantile (optimized
100%
80%
60%
40%
20%
0%
0 1 2 3 4 5 6 7 8 9 11 13

**Figure 47**

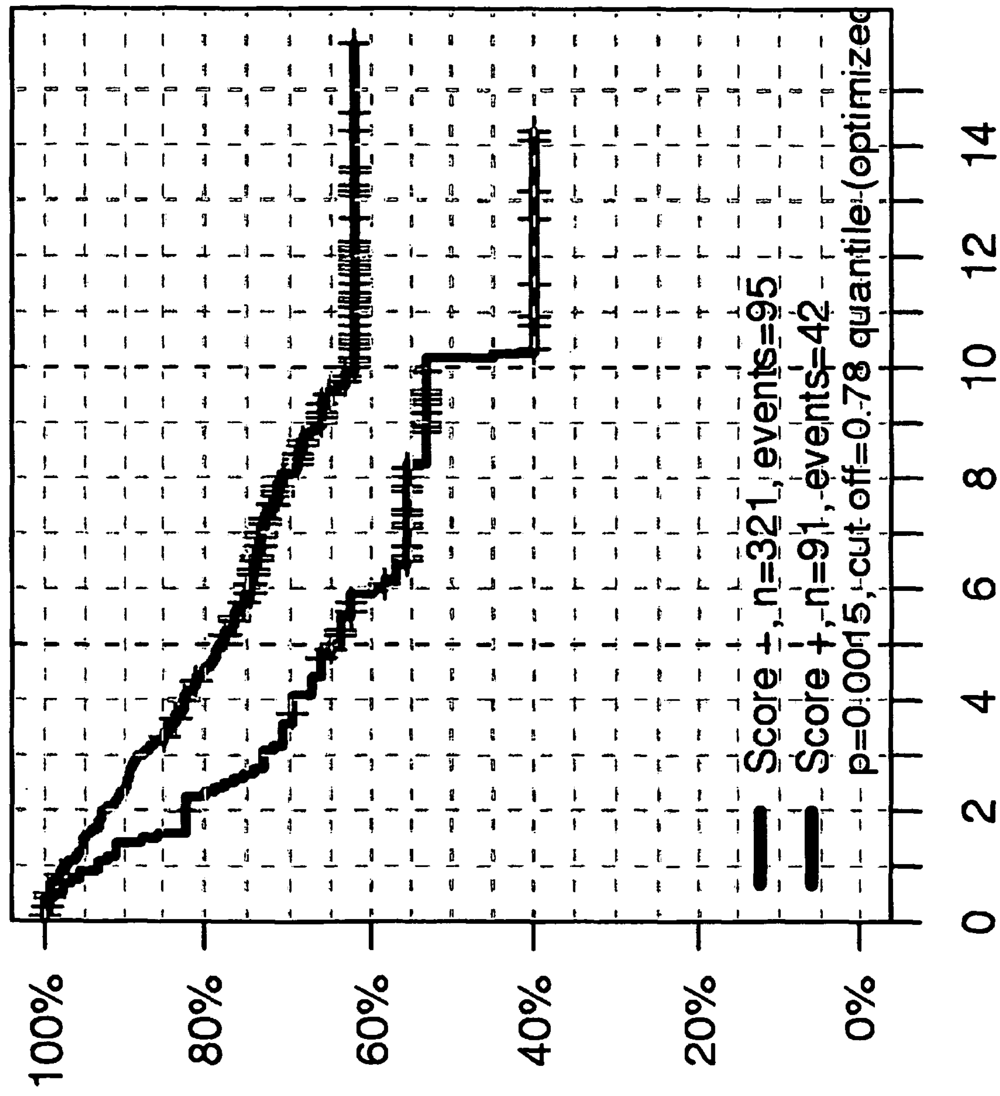
Score -, n=321, events=95
Score +, n=91, events=42
p=0.0015, cut off=0.78 quantile (optimized
100%
80%
60%
40%
20%
0%
0
2
4
6
8
10
12
14

**Figure 48**

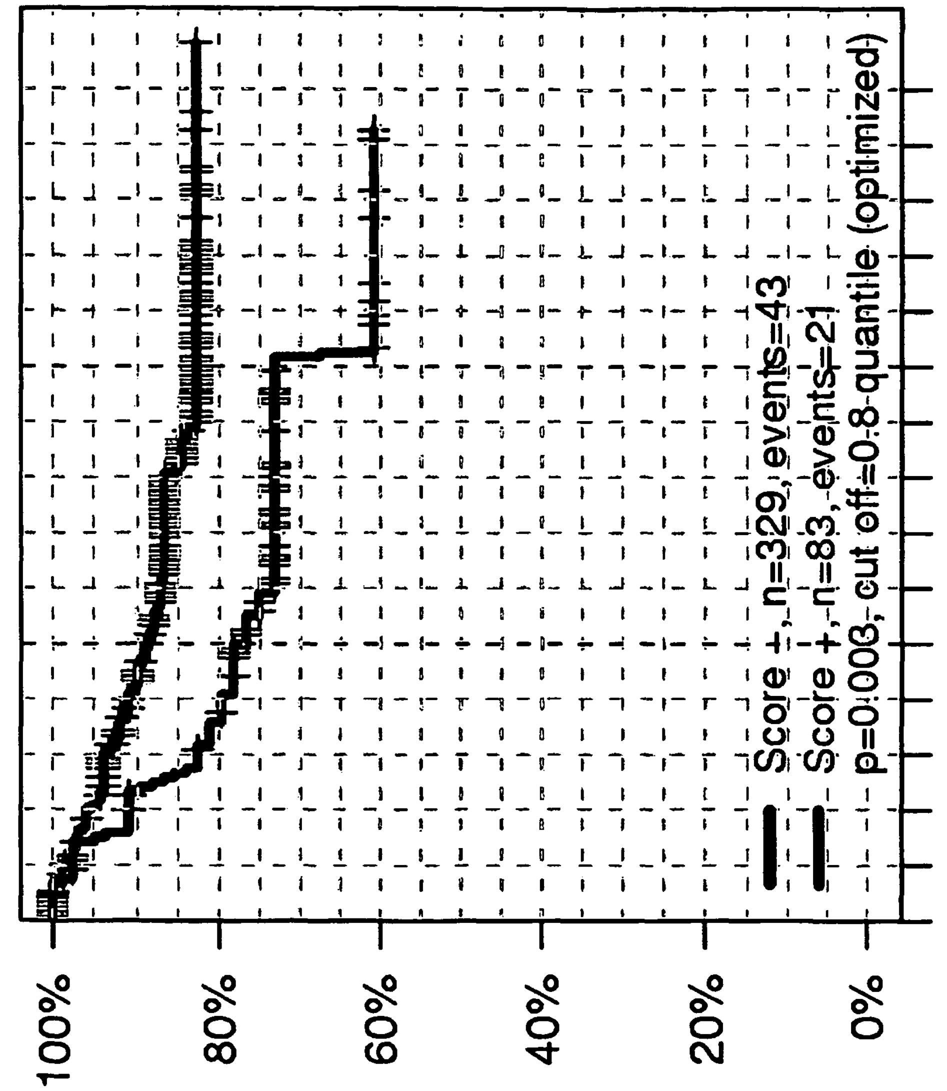

100%
80%
60%
40%
20%
0%
0
2
4
6
8
10
12
14
Score -, n=329, events=43
Score +, n=83, events=21
p=0.003, cut off=0.8 quantile (optimized)


Figure 49

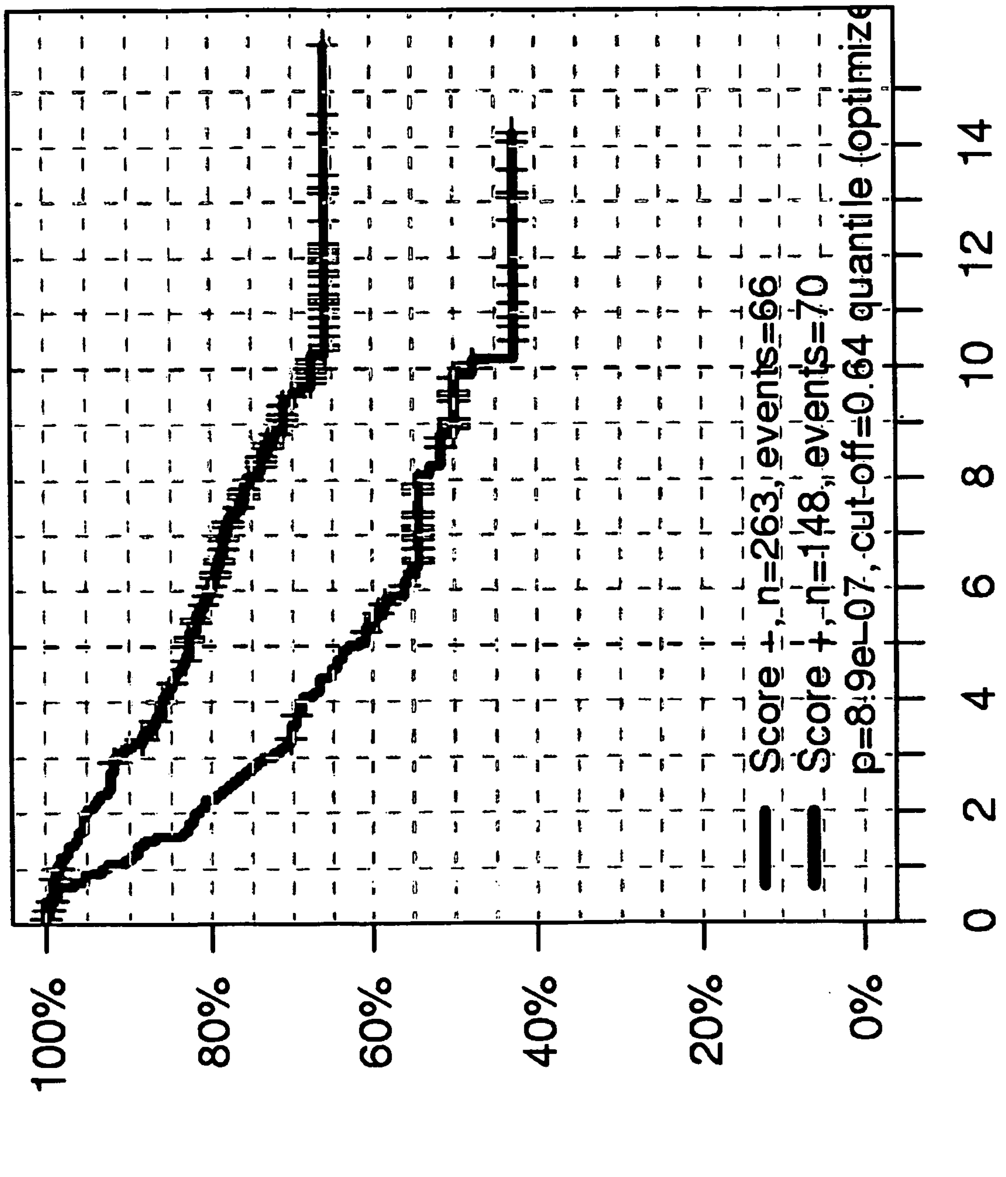
Score -, n=263, events=66
Score +, n=148, events=70
p=8.9e-07, cut-off=0.64 quantile (optimize
100%
80%
60%
40%
20%
0%
0
2
4
6
8
10
12
14

Figure 50

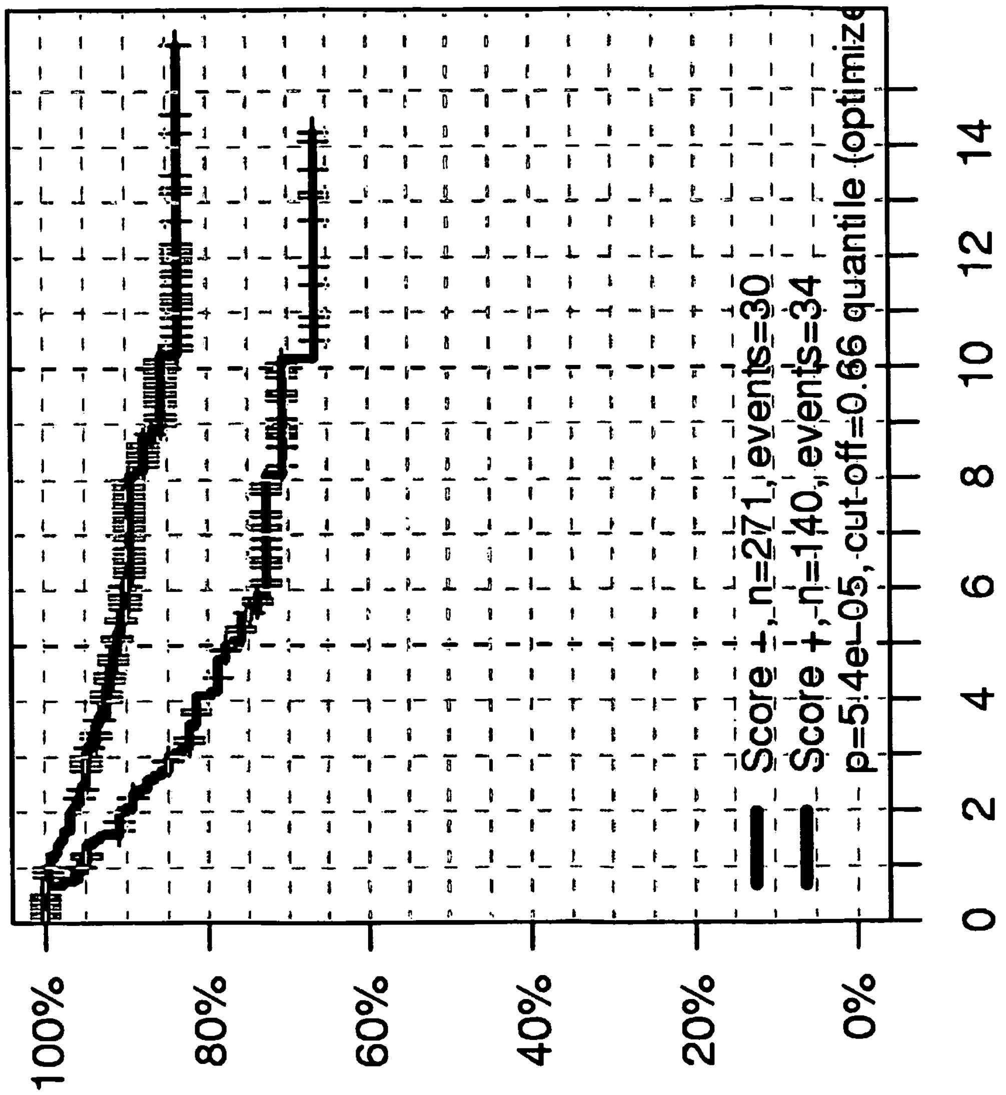
Score -, n=271, events=30
Score +, n=140, events=34
p=5.4e-05, cut-off=0.66 quantile (optimize
100%
80%
60%
40%
20%
0%
0
2
4
6
8
10
12
14

**Figure 51**

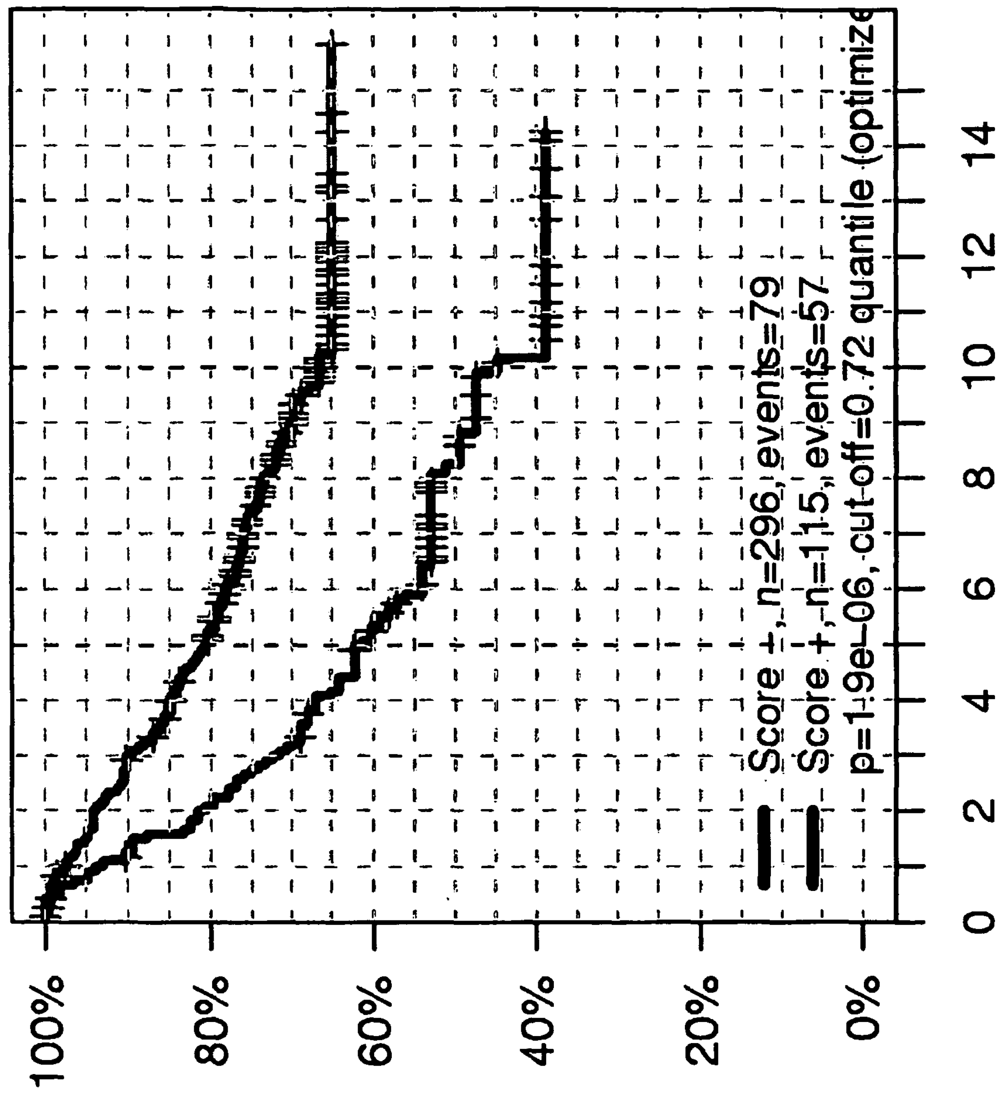
100%
80%
60%
40%
20%
0%
0
2
4
6
8
10
12
14
Score -, n=296, events=79
Score +, n=115, events=57
p=1.9e-06, cut-off=0.72 quantile (optimize

Figure 52

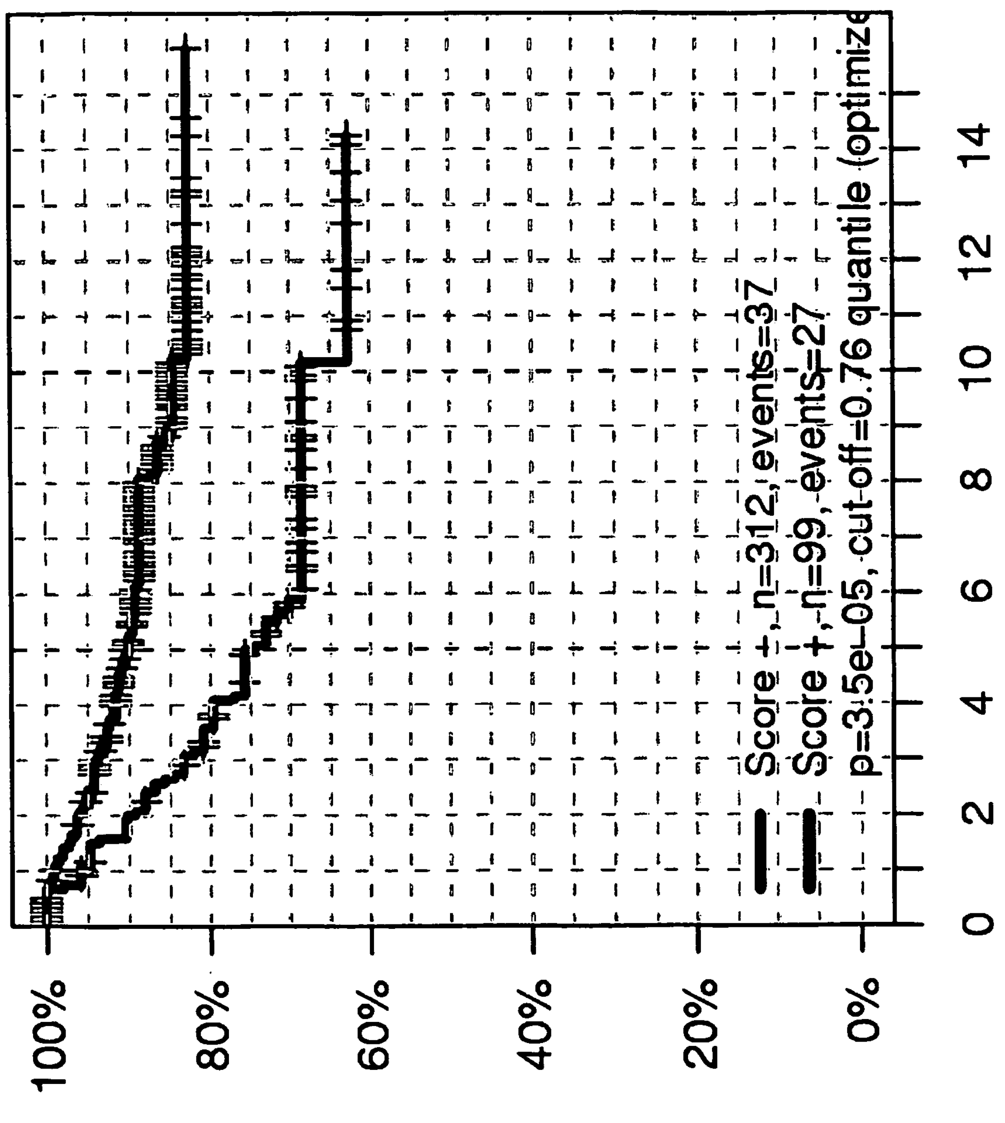

100%
80%
60%
40%
20%
0%
0
2
4
6
8
10
12
14
Score +, n=312, events=37
Score +, -n=99, events=27
p=3.5e-05, cut-off=0.76 quantile (optimiz


Figure 53

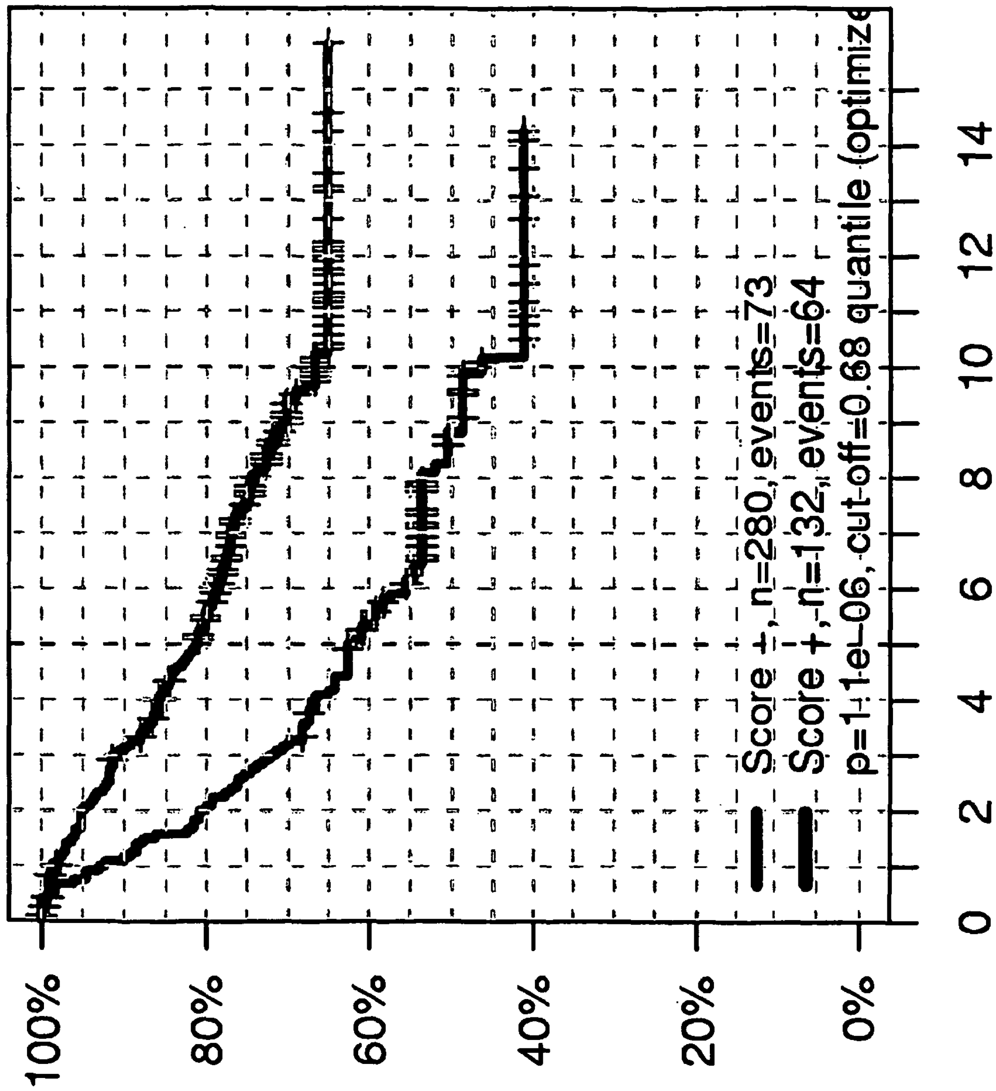
100%
80%
60%
40%
20%
0%
0
2
4
6
8
10
12
14
Score -, n=280, events=73
Score +, n=132, events=64
p=1.1e-06, cut-off=0.68 quantile (optimize

**Figure 54**

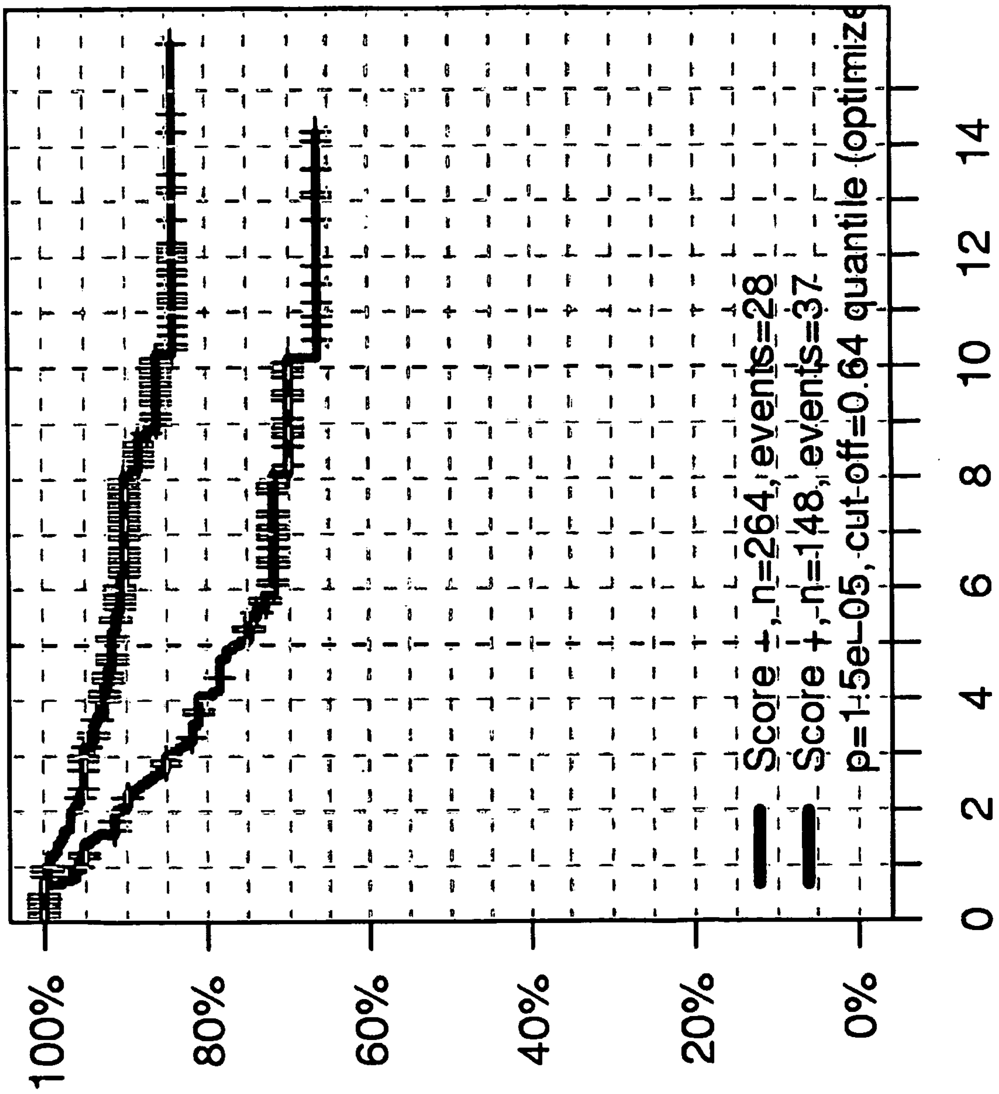
Score +, n=264, events=28
Score +, n=148, events=37
p=1.5e-05, cut-off=0.64 quantile (optimize
100%
80%
60%
40%
20%
0%
0
2
4
6
8
10
12
14

**Figure 55**

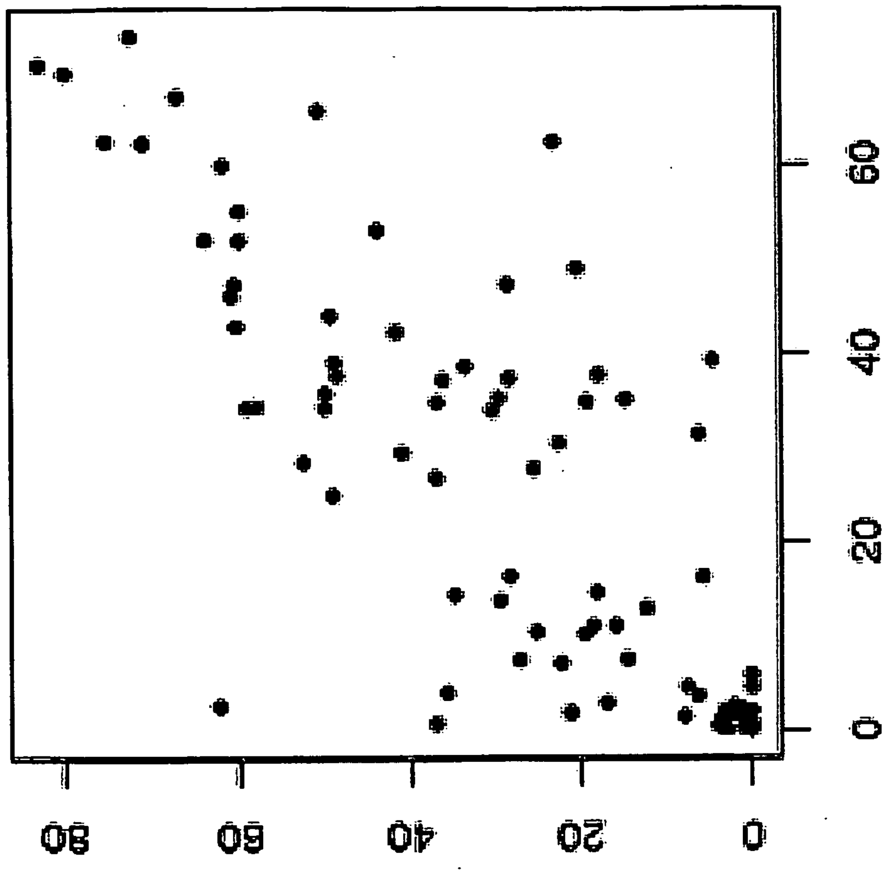


Figure 56

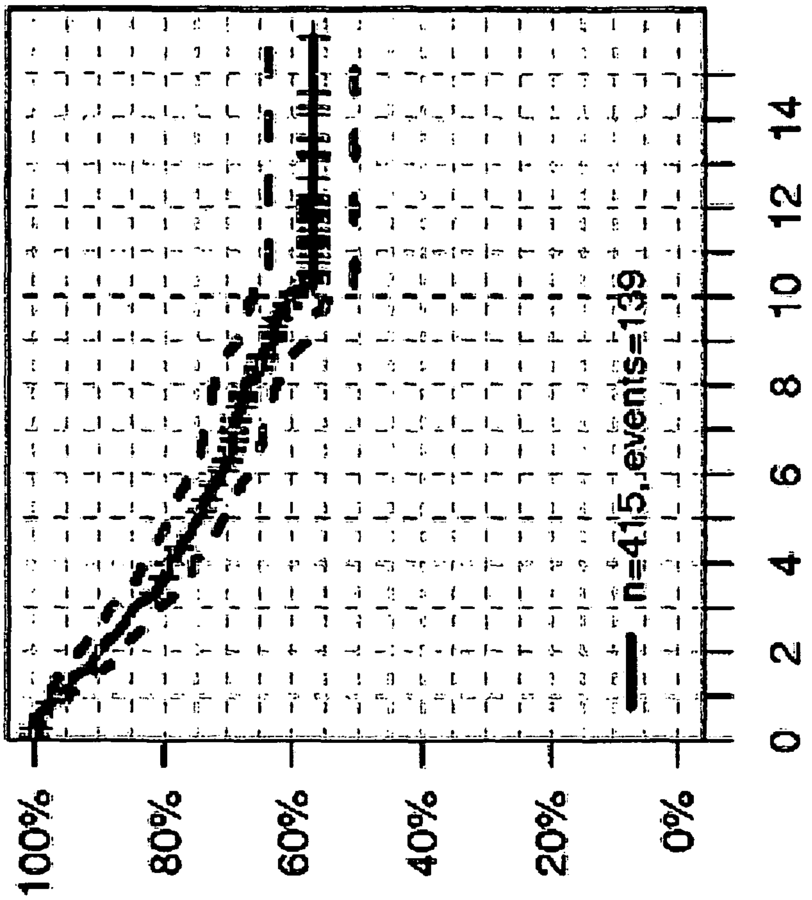


Figure 57

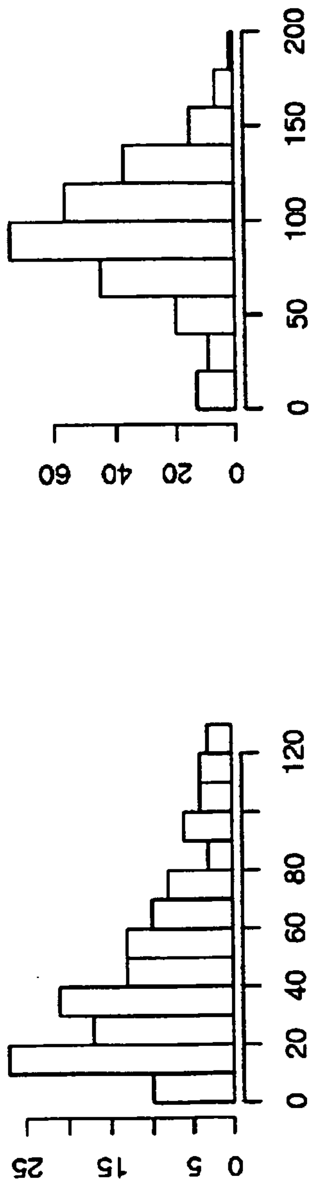


**Figure 58**

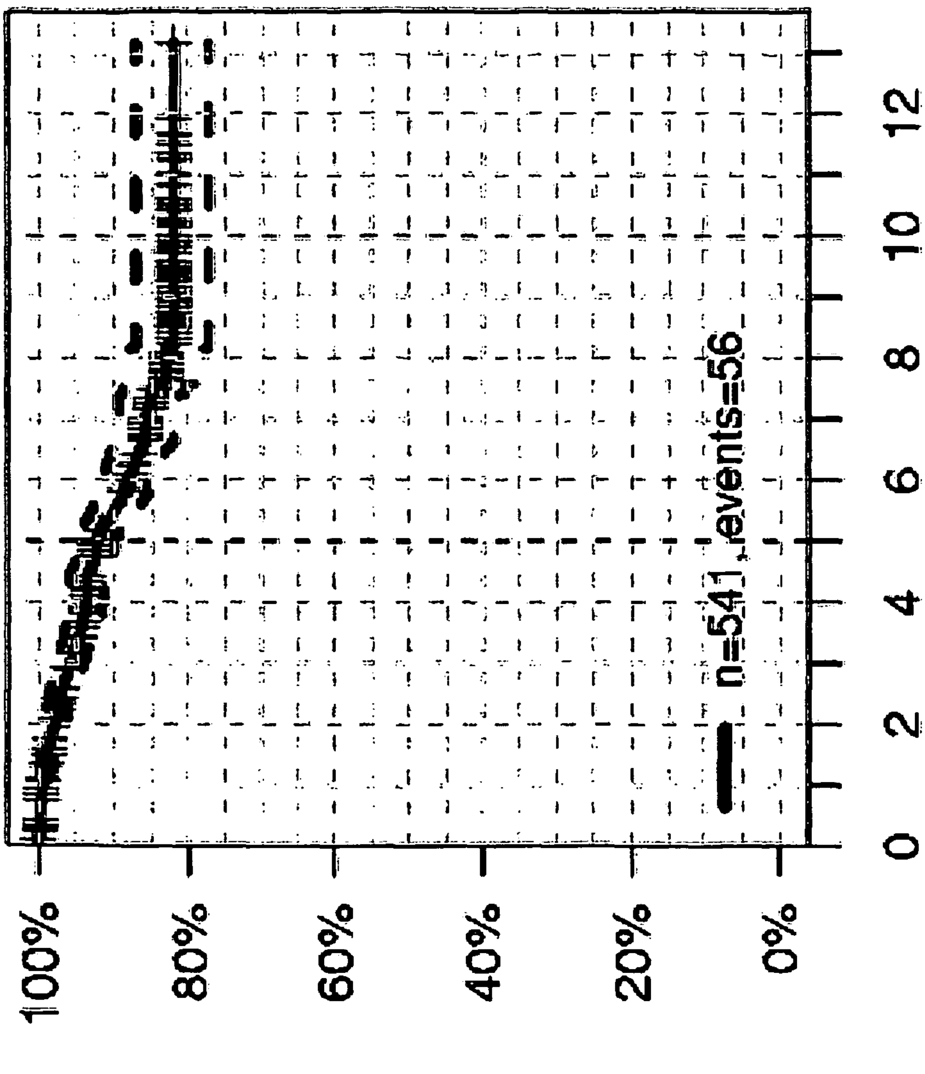


Figure 59

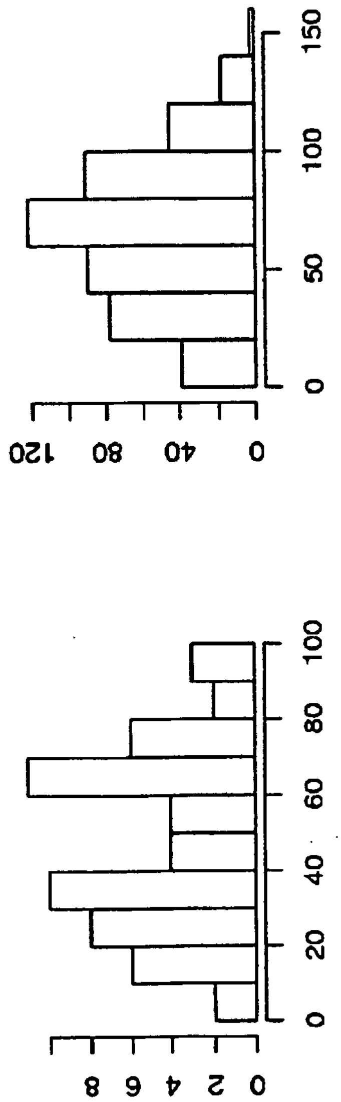


**Figure 60**

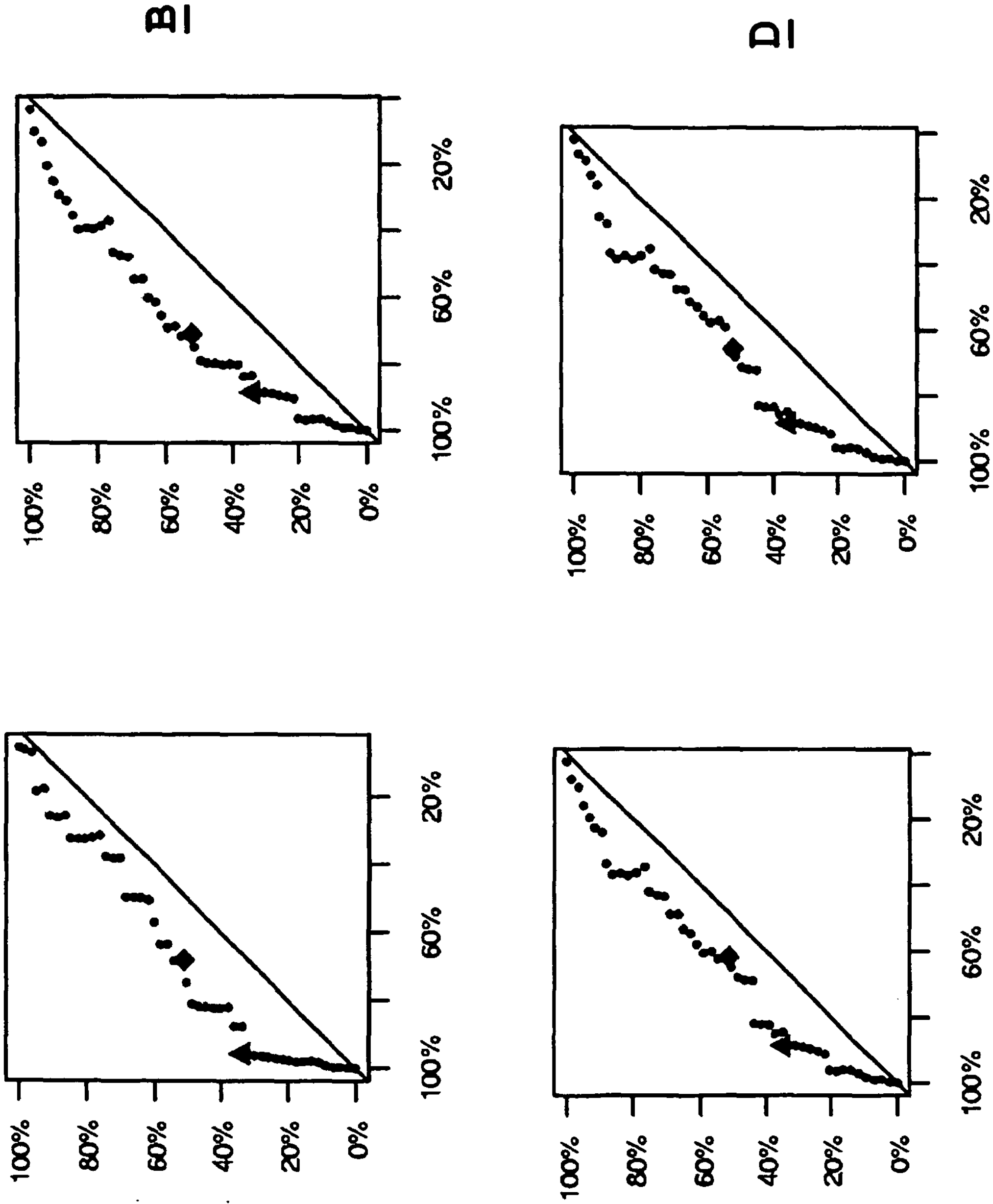


Figure 61

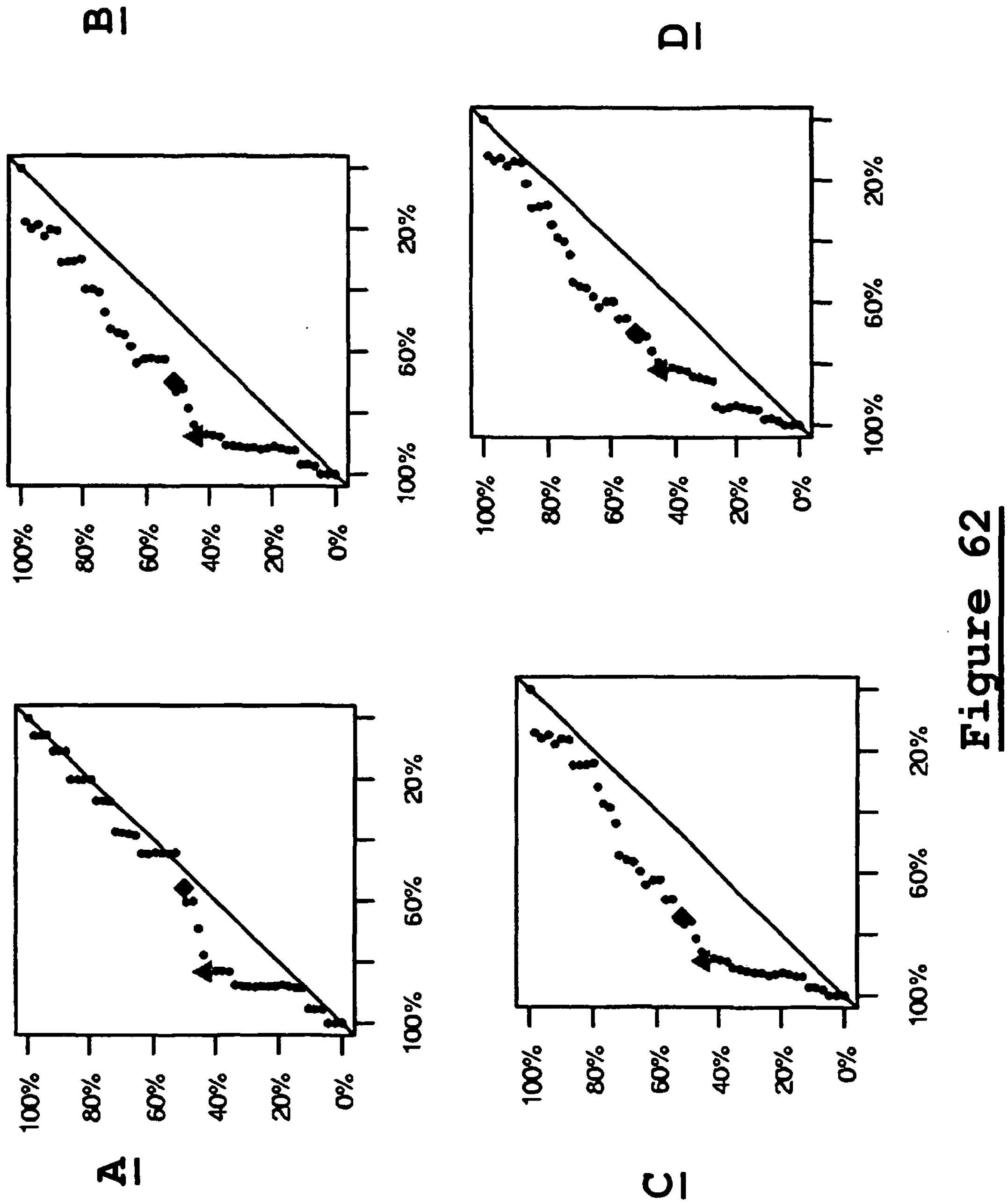


Figure 62

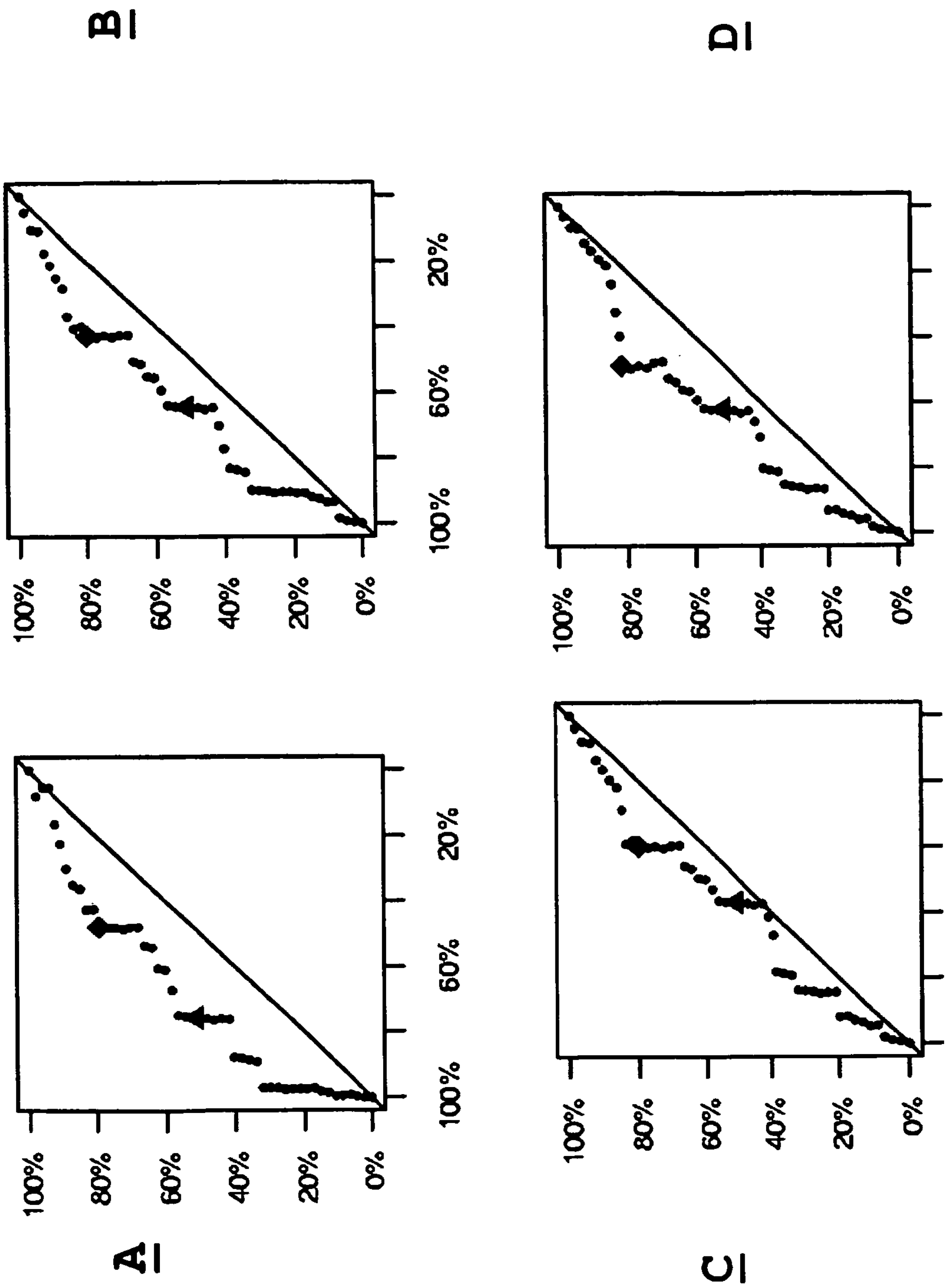


**Figure 63**

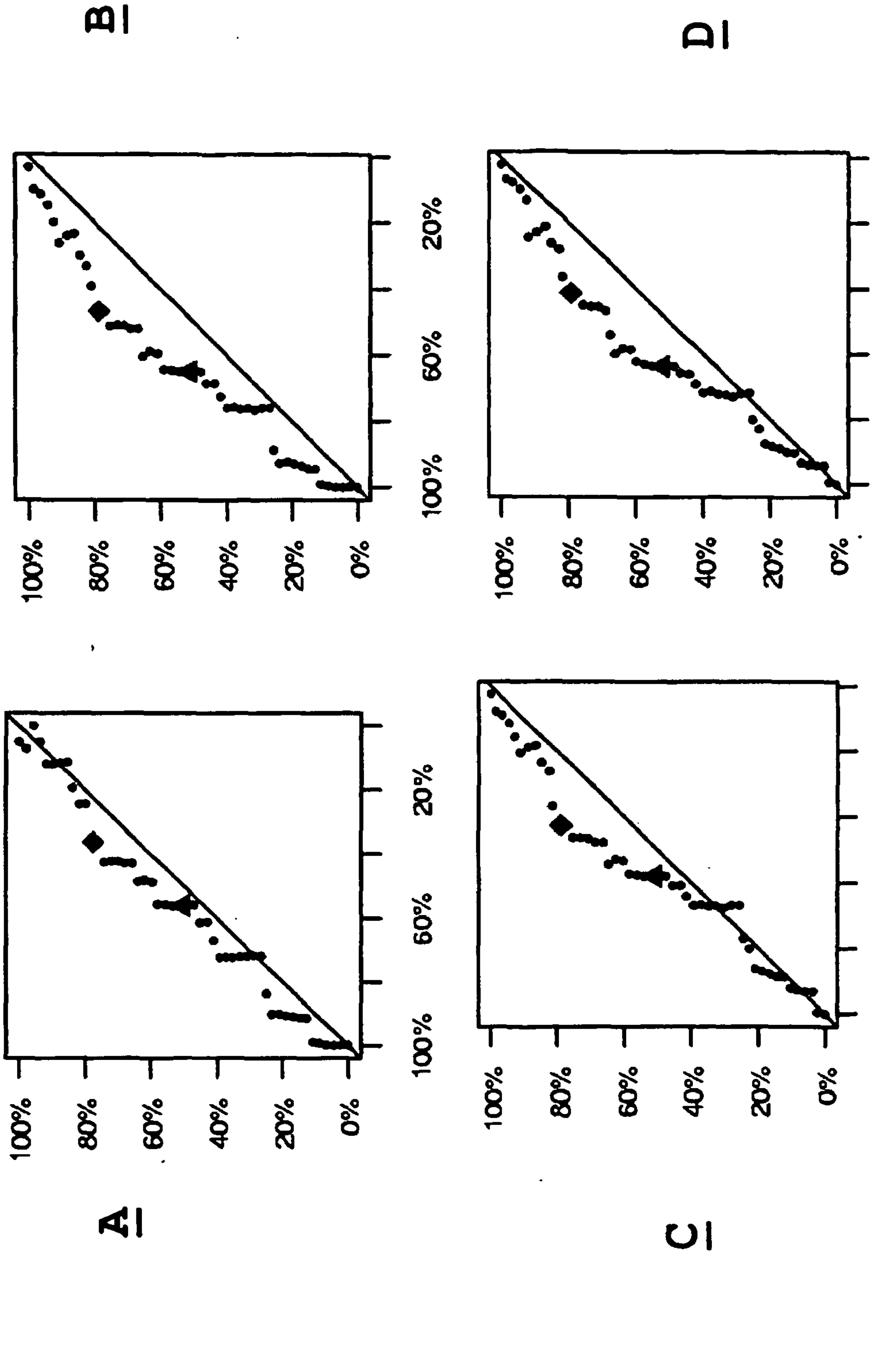


Figure 64

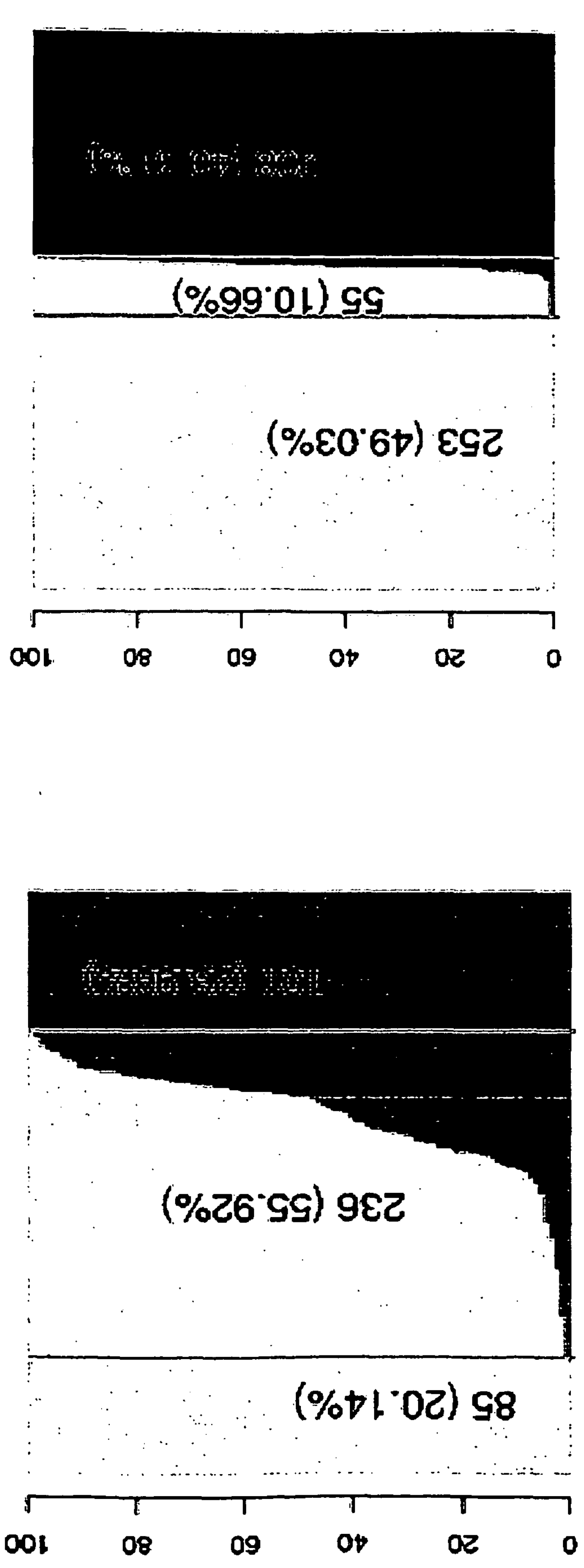
55 (10.66%)
253 (49.03%)
0
20
40
60
80
100
236 (55.92%)
85 (20.14%)
0
20
40
60
80
100

Figure 65

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02077272 A
- WO 0119845 A
- WO 0200927 A
- WO 01092565 A
- US 20030198970 A
- US 5786146 A
- US 6265171 B, Herman
- US 6331393 B
- US 5514758 A
- US 5574142 A
- US 5585481 A
- US 5587371 A
- US 5597696 A
- US 5958773 A
- US 20030013091 A
- US 09898743 B
- WO 04035803 A
- EP 03090432 A
- WO 2004000463 A


### Non-patent literature cited in the description


- *Lancet,* 16 May 1998, vol. 351 (9114), 1451-67
- **Toyota et al.** *Blood,* 2001, vol. 97, 2823-9
- **Shivapurkar et al.** *Cancer Research,* 01 August 1999, vol. 59, 3576-3580
- **Momparler ; Bovenzi.** *J. Cell Physiol.,* 2000, vol. 183, 145-54
- **Muller HM ; Widschwendter A ; Fiegl H ; Ivarsson L ; Goebel G ; Perkmann E ; Marth C ; Widschwendter M.** DNA methylation in serum of breast cancer patients: an independent prognostic marker. *Cancer Res.,* 15 November 2003, vol. 63 (22), 7641-5
- **Gonzalgo et al.** *Cancer Research,* 1997, vol. 57, 594-599
- **Eads et al.** *Cancer Res.,* 1999, vol. 59, 2302-2306
- **Gonzalgo ; Jones.** *Nucleic Acids Res.,* 1997, vol. 25, 2529-2531
- **Herman et al.** *Proc. Natl. Acad. Sci. USA,* 1996, vol. 93, 9821-9826
- **Xiong ; Laird.** *Nucleic Acids Res.,* 1997, vol. 25, 2532-2534
- **Sambrook et al.** Molecular Cloning, A Laboratory Manual. Cold Spring Harbor Press, 1989
- **Ausubel et al.** Current Protocols in Molecular Biology. John Wiley & Sons, 1995
- **Belyavsky et al.** *Nucl Acid Res,* 1989, vol. 17, 2919-2932
- **Krug ; Berger.** Methods in Enzymology. Academic Press, 1987, vol. 152, 316-325
- Basic and Clinical Immunology. Appleton & Lange, 1991, 217-262
- **Milstein ; Kohler.** *Nature,* 1975, vol. 256, 495-497
- **Gulfre ; Milstein.** Methods in Enzymology: Immunochemical Techniques. Academic Press, 1981, vol. 73, 1-46
- **Olek A et al.** A modified and improved method for bisulfite based cytosine methylation analysis. *Nucleic Acids Res.,* 1996, vol. 24, 5064-6
- **Karas ; Hillenkamp.** *Anal Chem.,* 1988, vol. 60, 2299-301
- **Gut ; Beck.** *Current Innovations and Future Trends,* 1995, vol. 1, 147-57
- **Gut ; Beck.** *Nucleic Acids Res.,* 1995, vol. 23, 1367-73
- **Yu et al.** *BioTechniques,* 1997, vol. 23, 714-720
- **Heid et al.** *Genome Res.,* 1996, vol. 6, 986-994
- **Sanger F. et al.** *Proc Natl Acad Sci USA,* 1977, vol. 74, 5463-5467
- **Krol et al.** *BioTechniques,* 1988, vol. 6, 958-976
- **Zon.** *Pharm. Res.,* 1988, vol. 5, 539-549
- *Nature Genetics Supplement,* January 1999, vol. 21
- *Cancer Research,* 01 August 1999, vol. 59, 3576-3580
- *Clin. Cancer Res.,* 1999, vol. 5, 17-23
- **Olek et al.** *Nucleic Acids Res.,* 15 December 1996, vol. 24 (24), 5064-6